# EUROPEAN PATENT APPLICATION

(11) **EP 1 367 124 A1**
(43) Date of publication of application: **03.12.2003**
(21) Application number: 03016881.9
(22) Date of filing: 31.07.1998
(51) Int. Cl.: C12N 15/12, C07K 14/47

(54) **5' ests for secreted proteins expressed in muscle and other mesodermal tissues**

(30) Priority: 01.08.1997 US 905134
(62) Divisional of application: 98936596.0
(71) Applicant: Genset, 91000 Evry (FR)
(72) Inventor: Dumas Milne Edwards, Jean-Baptiste, 75006 Paris (FR); Duclert, Aymeric, 94100 Saint-Maur (FR); Lacroix, Bruno, 69230 Saint-Genis Laval (FR)
(74) Representative: Brasnett, Adrian Hugh

(57) **Abstract**

The sequences of 5' ESTs derived from mRNAs encoding secreted proteins are disclosed. The 5' ESTs may be used to obtain cDNAs and genomic DNAs corresponding to the 5' ESTs. The 5' ESTs may also be used in diagnostic, forensic, gene therapy, and chromosome mapping procedures. Upstream regulatory sequences may also be obtained using the 5' ESTs. The 5' ESTs may also be used to design expression vectors and secretion vectors.

## Description

### Background of the Invention

The estimated 50,000-100,000 genes scattered along the human chromosomes offer tremendous promise for the understanding, diagnosis, and treatment of human diseases. In addition, probes capable of specifically hybridizing to loci distributed throughout the human genome find applications in the construction of high resolution chromosome maps and in the identification of individuals.

In the past, the characterization of even a single human gene was a painstaking process, requiring years of effort. Recent developments in the areas of cloning vectors, DNA sequencing, and computer technology have merged to greatly accelerate the rate at which human genes can be isolated, sequenced, mapped, and characterized. Cloning vectors such as yeast artificial chromosomes (YACs) and bacterial artificial chromosomes (BACs) are able to accept DNA inserts ranging from 300 to 1000 kilobases (kb) or 100-400 kb in length respectively, thereby facilitating the manipulation and ordering of DNA sequences distributed over great distances on the human chromosomes. Automated DNA sequencing machines permit the rapid sequencing of human genes. Bioinformatics software enables the comparison of nucleic acid and protein sequences, thereby assisting in the characterization of human gene products.

Currently, two different approaches are being pursued for identifying and characterizing the genes distributed along the human genome. In one approach, large fragments of genomic DNA are isolated, cloned, and sequenced. Potential open reading frames in these genomic sequences are identified using bioinformatics software. However, this approach entails sequencing large stretches of human DNA which do not encode proteins in order to find the protein encoding sequences scattered throughout the genome. In addition to requiring extensive sequencing, the bioinformatics software may mischaracterize the genomic sequences obtained. Thus, the software may produce false positives in which non-coding DNA is mischaracterized as coding DNA or false negatives in which coding DNA is mislabeled as non-coding DNA.

An alternative approach takes a more direct route to identifying and characterizing human genes. In this approach, complementary DNAs (cDNAs) are synthesized from isolated messenger RNAs (mRNAs) which encode human proteins. Using this approach, sequencing is only performed on DNA which is derived from protein coding portions of the genome. Often, only short stretches of the cDNAs are sequenced to obtain sequences called expressed sequence tags (ESTs). The ESTs may then be used to isolate or purify extended cDNAs which include sequences adjacent to the EST sequences. The extended cDNAs may contain all of the sequence of the EST which was used to obtain them or only a portion of the sequence of the EST which was used to obtain them. In addition, the extended cDNAs may contain the full coding sequence of the gene from which the EST was derived or, alternatively, the extended cDNAs may include portions of the coding sequence of the gene from which the EST was derived. It will be appreciated that there may be several extended cDNAs which include the EST sequence as a result of alternate splicing or the activity of alternative promoters.

In the past, these short EST sequences were often obtained from oligo-dT primed cDNA libraries. Accordingly, they mainly corresponded to the 3' untranslated region of the mRNA. In part, the prevalence of EST sequences derived from the 3' end of the mRNA is a result of the fact that typical techniques for obtaining cDNAs are not well suited for isolating cDNA sequences derived from the 5' ends of mRNAs. (Adams *et al., Nature* **377**:3-174, 1996; Hillier *et al., Genome Res.* **6**:807-828, 1996).

In addition, in those reported instances where longer cDNA sequences have been obtained, the reported sequences typically correspond to coding sequences and do not include the full 5' untranslated region of the mRNA from which the cDNA is derived. Such incomplete sequences may not include the first exon of the mRNA, particularly in situations where the first exon is short. Furthermore, they may not include some exons, often short ones, which are located upstream of splicing sites. Thus, there is a need to obtain sequences derived from the 5' ends of mRNAs.

While many sequences derived from human chromosomes have practical applications, approaches based on the identification and characterization of those chromosomal sequences which encode a protein product are particularly relevant to diagnostic and therapeutic uses. Of the 50,000-100,000 protein coding genes, those genes encoding proteins which are secreted from the cell in which they are synthesized, as well as the secreted proteins themselves, are particularly valuable as potential therapeutic agents. Such proteins are often involved in cell to cell communication and may be responsible for producing a clinically relevant response in their target cells.

In fact, several secretory proteins, including tissue plasminogen activator, G-CSF, GM-CSF, erythropoietin, human growth hormone, insulin, interferon-α, interferon-β, interferon-γ, and interleukin-2, are currently in clinical use. These proteins are used to treat a wide range of conditions, including acute myocardial infarction, acute ischemic stroke, anemia, diabetes, growth hormone deficiency, hepatitis, kidney carcinoma, chemotherapy induced neutropenia and multiple sclerosis. For these reasons, extended cDNAs encoding secreted proteins or portions thereof represent a particularly valuable source of therapeutic agents. Thus, there is a need for the identification and characterization of secreted proteins and the nucleic acids encoding them.

In addition to being therapeutically useful themselves, secretory proteins include short peptides, called signal peptides, at their amino termini which direct their secretion. These signal peptides are encoded by the signal sequences located at the 5' ends of the coding sequences of genes encoding secreted proteins. Because these signal peptides will direct the extracellular secretion of any protein to which they are operably linked, the signal sequences may be exploited to direct the efficient secretion of any protein by operably linking the signal sequences to a gene encoding the protein for which secretion is desired. In addition, portions of signal sequences may also be used to direct the intracellular import of a peptide or protein of interest. This may prove beneficial in gene therapy strategies in which it is desired to deliver a particular gene product to cells other than the cell in which it is produced. Signal sequences encoding signal peptides also find application in simplifying protein purification techniques. In such applications, the extracellular secretion of the desired protein greatly facilitates purification by reducing the number of undesired proteins from which the desired protein must be selected. Thus, there exists a need to identify and characterize the 5' portions of the genes for secretory proteins which encode signal peptides.

Public information on the number of human genes for which the promoters and upstream regulatory regions have been identified and characterized is quite limited. In part, this may be due to the difficulty of isolating such regulatory sequences. Upstream regulatory sequences such as transcription factor binding sites are typically too short to be utilized as probes for isolating promoters from human genomic libraries. Recently, some approaches have been developed to isolate human promoters. One of them consists of making a CpG island library (Cross, *et al.. Nature Genetics* **6**: 236-244, 1994). The second consists of isolating human genomic DNA sequences containing SpeI binding sites by the use of SpeI binding protein. (Mortlock *et al., Genome Res.* **6**:327-335, 1996). Both of these approaches have their limits due to a lack of specificity or of comprehensiveness.

The present 5' ESTs may be used to efficiently identify and isolate upstream regulatory regions which control the location, developmental stage, rate, and quantity of protein synthesis, as well as the stability of the mRNA. (Theil, *BioFactors* **4**:87-93, 1993). Once identified and characterized, these regulatory regions may be utilized in gene therapy or protein purification schemes to obtain the desired amount and locations of protein synthesis or to inhibit, reduce, or prevent the synthesis of undesirable gene products.

In addition, ESTs containing the 5' ends of secretory protein genes may include sequences useful as probes for chromosome mapping and the identification of individuals. Thus, there is a need to identify and characterize the sequences upstream of the 5' coding sequences of genes encoding secretory proteins.

### Summary of the Invention

The present invention relates to purified, isolated, or recombinant ESTs which include sequences derived from the authentic 5' ends of their corresponding mRNAs. The term "corresponding mRNA" refers to the mRNA which was the template for the cDNA synthesis which produced the 5' EST. These sequences will be referred to hereinafter as "5' ESTs." As used herein, the term "purified" does not require absolute purity, rather, it is intended as a relative definition. Individual 5' EST clones isolated from a cDNA library have been conventionally purified to electrophoretic homogeneity. The sequences obtained from these clones could not be obtained directly either from the library or from total human DNA. The cDNA clones are not naturally occurring as such, but rather are obtained via manipulation of a partially purified naturally occurring substance (messenger RNA). The conversion of mRNA into a cDNA library involves the creation of a synthetic substance (cDNA) and pure individual cDNA clones can be isolated from the synthetic library by clonal selection. Thus, creating a cDNA library from messenger RNA and subsequently isolating individual clones from that library results in an approximately 10⁴-10⁶ fold purification of the native message. Purification of starting material or natural material to at least one order of magnitude, preferably two or three orders, and more preferably four or five orders of magnitude is expressly contemplated.

As used herein, the term "isolated" requires that the material be removed from its original environment (e.g., the natural environment if it is naturally occurring). For example, a naturally-occurring polynucleotide present in a living animal is not isolated, but the same polynucleotide, separated from some or all of the coexisting materials in the natural system, is isolated.

As used herein, the term "recombinant" means that the 5' EST is adjacent to "backbone" nucleic acid to which it is not adjacent in its natural environment. Additionally, to be "enriched" the 5' ESTs will represent 5% or more of the number of nucleic acid inserts in a population of nucleic acid backbone molecules. Backbone molecules according to the present invention include nucleic acids such as expression vectors, self-replicating nucleic acids, viruses, integrating nucleic acids, and other vectors or nucleic acids used to maintain or manipulate a nucleic acid insert of interest. Preferably, the enriched 5' ESTs represent 15% or more of the number of nucleic acid inserts in the population of recombinant backbone molecules. More preferably, the enriched 5' ESTs represent 50% or more of the number of nucleic acid inserts in the population of recombinant backbone molecules. In a highly preferred embodiment, the enriched 5' ESTs represent 90% or more of the number of nucleic acid inserts in the population of recombinant backbone molecules.

"Stringent", moderate," and "low" hybridization conditions are as defined in Example 29.

Unless otherwise indicated, a "complementary" sequence is fully complementary.

Thus, 5' ESTs in cDNA libraries in which one or more 5' ESTs make up 5% or more of the number of nucleic acid inserts in the backbone molecules are "enriched recombinant 5' ESTs" as defined herein. Likewise, 5' ESTs in a population of plasmids in which one or more 5' EST of the present invention have been inserted such that they represent 5% or more of the number of inserts in the plasmid backbone are " enriched recombinant 5' ESTs" as defined herein. However, 5' ESTs in cDNA libraries in which 5' ESTs constitute less than 5% of the number of nucleic acid inserts in the population of backbone molecules, such as libraries in which backbone molecules having a 5' EST insert are extremely rare, are not "enriched recombinant 5' ESTs."

In particular, the present invention relates to 5' ESTs which are derived from genes encoding secreted proteins. As used herein, a "secreted" protein is one which, when expressed in a suitable host cell, is transported across or through a membrane, including transport as a result of signal peptides in its amino acid sequence. "Secreted" proteins include without limitation proteins secreted wholly (e.g. soluble proteins), or partially (e.g. receptors) from the cell in which they are expressed. "Secreted" proteins also include without limitation proteins which are transported across the membrane of the endoplasmic reticulum.

Such 5' ESTs include nucleic acid sequences, called signal sequences, which encode signal peptides which direct the extracellular secretion of the proteins encoded by the genes from which the 5' ESTs are derived. Generally, the signal peptides are located at the amino termini of secreted proteins.

Secreted proteins are translated by ribosomes associated with the "rough" endoplasmic reticulum. Generally, secreted proteins are co-translationally transferred to the membrane of the endoplasmic reticulum. Association of the ribosome with the endoplasmic reticulum during translation of secreted proteins is mediated by the signal peptide. The signal peptide is typically cleaved following its co-translational entry into the endoplasmic reticulum. After delivery to the endoplasmic reticulum, secreted proteins may proceed through the Golgi apparatus. In the Golgi apparatus, the proteins may undergo post-translational modification before entering secretory vesicles which transport them across the cell membrane.

The 5' ESTs of the present invention have several important applications. For example, they may be used to obtain and express cDNA clones which include the full protein coding sequences of the corresponding gene products, including the authentic translation start sites derived from the 5' ends of the coding sequences ofthe mRNAs from which the 5' ESTs are derived. These cDNAs will be referred to hereinafter as "full length cDNAs." These cDNAs may also include DNA derived from mRNA sequences upstream of the translation start site. The full length cDNA sequences may be used to express the proteins corresponding to the 5' ESTs. As discussed above, secreted proteins are therapeutically important. Thus, the proteins expressed from the cDNAs may be useful in treating or controlling a variety of human conditions. The 5' ESTs may also be used to obtain the corresponding genomic DNA. The term "corresponding genomic DNA" refers to the genomic DNA which encodes the mRNA from which the 5' EST was derived.

Alternatively, the 5' ESTs may be used to obtain and express extended cDNAs encoding portions of the secreted protein. The portions may comprise the signal peptides of the secreted proteins or the mature proteins generated when the signal peptide is cleaved off The portions may also comprise polypeptides having at least 10 consecutive amino acids encoded by the extended cDNAs or full length cDNAs. Alternatively, the portions may comprise at least 15 consecutive amino acids encoded by the extended cDNAs or full length cDNAs. In some embodiments, the portions may comprise at least 25 consecutive amino acids encoded by the extended cDNAs or full length cDNAs. In other embodiments, the portions may comprise at least 40 amino acids encoded by the extended cDNAs or full length cDNAs.

Antibodies which specifically recognize the entire secreted proteins encoded by the extended cDNAs, full length cDNAs, or fragments thereof having at least 10 consecutive amino acids, at least 15 consecutive amino acids, at least 25 consecutive amino acids, or at least 40 consecutive amino acids may also be obtained as described below. Antibodies which specifically recognize the mature protein generated when the signal peptide is cleaved may also be obtained as described below. Similarly, antibodies which specifically recognize the signal peptides encoded by the extended cDNAs or full length cDNAs may also be obtained.

In some embodiments, the extended cDNAs obtained using the 5' ESTs include the signal sequence. In other embodiments, the extended cDNAs obtained using the 5' ESTs may include the full coding sequence for the mature protein (*i.e.* the protein generated when the signal polypeptide is cleaved off). In addition, the extended cDNAs obtained using the 5' ESTs may include regulatory regions upstream of the translation start site or downstream of the stop codon which control the amount, location, or developmental stage of gene expression.

As discussed above, secreted proteins are therapeutically important. Thus, the proteins expressed from the extended cDNAs or full length cDNAs obtained using the 5' ESTs may be useful in treating or controlling a variety of human conditions.

The 5' ESTs (or cDNAs or genomic DNAs obtained therefrom) may be used in forensic procedures to identify individuals or in diagnostic procedures to identify individuals having genetic diseases resulting from abnormal expression of the genes corresponding to the 5' ESTs. In addition, the present invention is useful for constructing a high resolution map of the human chromosomes.

The present invention also relates to secretion vectors capable of directing the secretion of a protein of interest. Such vectors may be used in gene therapy strategies in which it is desired to produce a gene product in one cell which is to be delivered to another location in the body. Secretion vectors may also facilitate the purification of desired proteins.

The present invention also relates to expression vectors capable of directing the expression of an inserted gene in a desired spatial or temporal manner or at a desired level. Such vectors may include sequences upstream of the 5' ESTs, such as promoters or upstream regulatory sequences.

Finally, the present invention may also be used for gene therapy to control or treat genetic diseases. Signal peptides may also be fused to heterologous proteins to direct their extracellular secretion.

Bacterial clones containing Bluescript plasmids having inserts containing the 5' ESTs of the present invention (SEQ ID NOs: 38-305 are presently stored at 80°C in 4% (v/v) glycerol in the inventor's laboratories under the designations listed next to the SEQ ID NOs in II). The inserts may be recovered from the deposited materials by growing the appropriate clones on a suitable medium. The Bluescript DNA can then be isolated using plasmid isolation procedures familiar to those skilled in the art such as alkaline lysis minipreps or large scale alkaline lysis plasmid isolation procedures. If desired the plasmid DNA may be further enriched by centrifugation on a cesium chloride gradient, size exclusion chromatography, or anion exchange chromatography. The plasmid DNA obtained using these procedures may then be manipulated using standard cloning techniques familiar to those skilled in the art. Alternatively, a PCR can be done with primers designed at both ends of the EST insertion. The PCR product which corresponds to the 5' EST can then be manipulated using standard cloning techniques familiar to those skilled in the art.

One aspect of the present invention is a purified or isolated nucleic acid having the sequence of one of SEQ ID NOs: 38-305 or having a sequence complementary thereto. In one embodiment, the nucleic acid is recombinant.

Another aspect of the present invention is a purified or isolated nucleic acid comprising at least 10 consecutive bases of the sequence of one of SEQ ID NOs: 38-305 or one of the sequences complementary thereto.

Yet another aspect of the present invention is a purified or isolated nucleic acid comprising at least 15 consecutive bases of one of the sequences of SEQ ID NOs: 38-305 or one of the sequences complementary thereto. In one embodiment, the nucleic acid is recombinant.

A further aspect of the present invention is a purified or isolated nucleic acid of at least 15 bases capable of hybridizing under stringent conditions to the sequence of one of SEQ ID NOs: 38-305 or one of the sequences complementary to the sequences of SEQ ID NOs: 38-305. In one embodiment, the nucleic acid is recombinant.

Another aspect of the present invention is a purified or isolated nucleic acid encoding a human gene product, said human gene product having a sequence partially encoded by one of the sequences of SEQ ID NO: 38-305.

Still another aspect of the present invention is a method of making a cDNA encoding a human secretory protein, said human secretory protein being partially encoded by one of SEQ ID NOs 38-305, comprising the steps of contacting a collection of mRNA molecules from human cells with a primer comprising at least 15 consecutive nucleotides of a sequence complementary to one of SEQ ID NOs: 38-305; hybridizing said primer to an mRNA in said collection that encodes said protein; reverse transcribing said hybridized primer to make a first cDNA strand from said mRNA; making a second cDNA strand complementary to said first cDNA strand; and isolating the resulting cDNA encoding said protein comprising said first cDNA strand and said second cDNA strand.

Another aspect of the invention is an isolated or purified cDNA encoding a human secretory protein, said human secretory protein comprising the protein encoded by one of SEQ ID NOs 38-305 or a fragment thereof of at least 10 amino acids, said cDNA being obtainable by the method described in the preceding paragraph. In one embodiment, the cDNA comprises the full protein coding sequence of said protein which sequence is partially included in one of the sequences of SEQ ID NOs: 38-305.

Another aspect of the present invention is a method of making a cDNA encoding a human secretory protein that is partially encoded by one of SEQ ID NOs 38-305, comprising the steps of obtaining a cDNA comprising one of the sequences of SEQ ID NOs: 38-305; contacting said cDNA with a detectable probe comprising at least 15 consecutive nucleotides of said sequence of SEQ ID NO: 38-305 or a sequence complementary thereto under conditions which permit said probe to hybridize to said cDNA; identifying a cDNA which hybridizes to said detectable probe; and isolating said cDNA which hybridizes to said probe.

Another aspect of the present invention is an isolated or purified cDNA encoding a human secretory protein, said human secretory protein comprising the protein encoded by one of SEQ ID NOs 38-305 or a fragment thereof of at least 10 amino acids, said cDNA being obtainable by the method described in the preceding paragraph. In one embodiment, the cDNA comprises the full protein coding sequence partially included in one of the sequences of SEQ ID NOs: 38-305.

Another aspect of the present invention is a method of making a cDNA comprising one of the sequence of SEQ ID NOs: 38-305, comprising the steps of contacting a collection of mRNA molecules from human cells with a first primer capable of hybridizing to the polyA tail of said mRNA; hybridizing said first primer to said polyA tail; reverse transcribing said mRNA to make a first cDNA strand; making a second cDNA strand complementary to said first cDNA strand using at least one primer comprising at least 15 nucleotides of one of the sequences of SEQ ID NOs 38-305; and isolating the resulting cDNA comprising said first cDNA strand and said second cDNA strand.

Another aspect of the present invention is an isolated or purified cDNA encoding a human secretory protein, said human secretory protein comprising the protein encoded by one of SEQ ID NOs 38-305 or a fragment thereof of at least 10 amino acids, said cDNA being obtainable by the method described in the preceding paragraph. In one embodiment, the cDNA comprises the full protein coding sequence partially included in one of the sequences of SEQ ID NOs: 38-305.

In one embodiment of the method described in the two paragraphs above, the second cDNA strand is made by contacting said first cDNA strand with a first pair of primers, said first pair of primers comprising a second primer comprising at least 15 consecutive nucleotides of one of the sequences of SEQ ID NOs 38-305 and a third primer having a sequence therein which is included within the sequence of said first primer; performing a first polymerase chain reaction with said first pair of nested primers to generate a first PCR product; contacting said first PCR product with a second pair of primers, said second pair of primers comprising a fourth primer, said fourth primer comprising at least 15 consecutive nucleotides of said sequence of one of SEQ ID NOs: 38-305 , and a fifth primer, said fourth and fifth primers being capable of hybridizing to sequences within said first PCR product; and performing a second polymerase chain reaction, thereby generating a second PCR product.

One aspect of the present invention is an isolated or purified cDNA encoding a human secretory protein, said human secretory protein comprising the protein encoded by one of SEQ ID NOs 38-305, or a fragment thereof of at least 10 amino acids, said cDNA being obtainable by the method of the preceding paragraph. In one embodiment, the cDNA comprises the full protein coding sequence partially included in one of the sequences of SEQ ID NOs: 38-305.

Another aspect of the present invention is the method described four paragraphs above in which the second cDNA strand is made by contacting said first cDNA strand with a second primer comprising at least 15 consecutive nucleotides of the sequences of SEQ ID NOs: 38-305; hybridizing said second primer to said first strand cDNA; and extending said hybridized second primer to generate said second cDNA strand.

Another aspect of the present invention is an isolated or purified cDNA encoding a human secretory protein, said human secretory protein comprising the protein partially encoded by one of SEQ ID NOs 38-305 or comprising a fragment thereof of at least 10 amino acids, said cDNA being obtainable by the method described in the preceding paragraph. In one embodiment, the cDNA comprises the full protein coding sequence partially included in of one of the sequences of SEQ ID NOs: 38-305.

Another aspect of the present invention is a method of making a protein comprising one of the sequences of SEQ ID NOs: 306-573, comprising the steps of obtaining a cDNA encoding the full protein sequence partially included in one of the sequences of sequence of SEQ ID NOs: 38-305; inserting said cDNA in an expression vector such that said cDNA is operably linked to a promoter; introducing said expression vector into a host cell whereby said host cell produces the protein encoded by said cDNA; and isolating said protein.

Another aspect of the present invention is an isolated protein obtainable by the method described in the preceding paragraph.

Another aspect of the present invention is a method of obtaining a promoter DNA comprising the steps of obtaining DNAs located upstream of the nucleic acids of SEQ ID NOs: 38-305 or the sequences complementary thereto; screening said upstream DNAs to identify a promoter capable of directing transcription initiation; and isolating said DNA comprising said identified promoter. In one embodiment, the obtaining step comprises chromosome walking from said nucleic acids of SEQ ID NOs: 38-305 or sequences complementary thereto. In another embodiment, the screening step comprises inserting said upstream sequences into a promoter reporter vector. In another embodiment, the screening step comprises identifying motifs in said upstream DNAs which are transcription factor binding sites or transcription start sites.

Another aspect of the present invention is an isolated promoter obtainable by the method described above.

Another aspect of the present invention is an isolated or purified protein comprising one of the sequences of SEQ ID NOs: 306-573.

Another aspect of the present invention is the inclusion of at least one of the sequences of SEQ ID NOs: 38-305, or one of the sequences complementary to the sequences of SEQ ID NOs: 38-305, or a fragment thereof of at least 15 consecutive nucleotides in an array of discrete ESTs or fragments thereof of at least 15 nucleotides in length. In one embodiment, the array includes at least two of the sequences of SEQ ID NOs: 38-305, the sequences complementary to the sequences of SEQ ID NOs: 38-305, or fragments thereof of at least 15 consecutive nucleotides. In another embodiment, the array includes at least five of the sequences of SEQ ID NOs: 38-305, the sequences complementary to the sequences of SEQ ID NOs: 38-305, or fragments thereof of at least 15 consecutive nucleotides.

Another aspect of the present invention is a promoter having a sequence selected from the group consisting of SEQ ID NOs: 31, 34, and 37.

### Brief Description of the Drawings

Figure 1 is a summary of a procedure for obtaining cDNAs which have been selected to include the 5' ends of the mRNAs from which they derived.
Figure 2 shows the distribution of Von Heijne scores for 5' ESTs in each of the categories described herein and the probability that these 5' ESTs encode a signal peptide.
Figure 3 summarizes a general method used to clone and sequence extended cDNAs containing sequences adjacent to 5' ESTs.
Figure 4 (description of promoters structure isolated from SignalTag 5' ESTs) provides a schematic description of promoters isolated and the way they are assembled with the corresponding 5' tags.

### Detailed Description of the Preferred Embodiment

Table IV is an analysis of the 43 amino acids located at the N terminus of all human SwissProt proteins to determine the frequency of false positives and false negatives using the techniques for signal peptide identification described herein.

Table V shows the distribution of 5' ESTs in each category described herein and the number of 5' ESTs in each category having a given minimum Von Heijne's score.

Table VI shows the distribution of 5' ESTs in each category described herein with respect to the tissue from which the 5' ESTs of the corresponding mRNA were obtained.

Table VII describes the transcription factor binding sites present in each of these promoters.

### I. General Methods for Obtaining 5' ESTs derived from mRNAs with intact 5' ends

In order to obtain the 5' ESTs of the present invention, mRNAs with intact 5' ends must be obtained. Currently, there are two approaches for obtaining such mRNAs with intact 5' ends as described below: either chemical (1) or enzymatic (2).

### 1. Chemical Methods for Obtaining mRNAs having Intact 5' Ends

One of these approaches is a chemical modification method involving derivatization of the 5' ends of the mRNAs and selection of the derivatized mRNAs. The 5' ends of eukaryotic mRNAs possess a structure referred to as a "cap" which comprises a guanosine methylated at the 7 position. The cap is joined to the first transcribed base of the mRNA by a 5', 5'-triphosphate bond. In some instances, the 5' guanosine is methylated in both the 2 and 7 positions. Rarely, the 5' guanosine is trimethylated at the 2, 7 and 7 positions. In the chemical method for obtaining mRNAs having intact 5' ends, the 5' cap is specifically derivatized and coupled to a reactive group on an immobilizing substrate. This specific derivatization is based on the fact that only the ribose linked to the methylated guanosine at the 5' end of the mRNA and the ribose linked to the base at the 3' terminus of the mRNA, possess 2', 3'-cis diols.

Optionally, the 2', 3'-cis diol of the 3' terminal ribose may be chemically modified, substituted, converted, or eliminated, leaving only the ribose linked to the methylated guanosine at the 5' end of the mRNA with a 2', 3'-cis diol. A variety of techniques are available for eliminating the 2', 3'-cis diol on the 3' terminal ribose. For example, controlled alkaline hydrolysis may be used to generate mRNA fragments in which the 3' terminal ribose is a 3'-phosphate, 2'-phosphate or (2', 3')-cyclophosphate. Thereafter, the fragment which includes the original 3' ribose may be eliminated from the mixture through chromatography on an oligodT column. Alternatively, a base which lacks the 2', 3'-cis diol may be added to the 3' end of the mRNA using an RNA ligase such as T4 RNA ligase. Example 1 below describes a method for ligation of a nucleoside diphosphate to the 3' end of messenger RNA.

### EXAMPLE 1

### Ligation of the Nucleoside Diphosphate pCp to the 3' End of mRNA

One µg of RNA was incubated in a final reaction medium of 10 µl in the presence of 5 U of T₄ phage RNA ligase in the buffer provided by the manufacturer (Gibco - BRL), 40 U of the RNase inhibitor RNasin (Promega) and, 2 µl of ³²pCp (Amersham #PB 10208). The incubation was performed at 37°C for 2 hours or overnight at 7-8°C.

Following modification or elimination of the 2', 3'-cis diol at the 3' ribose, the 2', 3'-cis diol present at the 5' end of the mRNA may be oxidized using reagents such as NaBH₄, NaBH₃CN, or sodium periodate, thereby converting the 2', 3'-cis diol to a dialdehyde. Example 2 describes the oxidation of the 2', 3'-cis diol at the 5' end of the mRNA with sodium periodate.

### EXAMPLE 2

### Oxidation of 2', 3'-cis diol at the 5' End of the mRNA with Sodium Periodate

0.1 OD unit of either a capped oligoribonucleotide of 47 nucleotides (including the cap) or an uncapped oligoribonucleotide of 46 nucleotides were treated as follows. The oligoribonucleotides were produced by *in vitro* transcription using the transcription kit "AmpliScribe T7" (Epicentre Technologies). As indicated below, the DNA template for the RNA transcript contained a single cytosine. To synthesize the uncapped RNA, all four NTPs were included in the *in vitro* transcription reaction. To obtain the capped RNA, GTP was replaced by an analogue of the cap, m7G(5')ppp(5')G. This compound, recognized by the polymerase, was incorporated into the 5' end of the nascent transcript during the initiation of transcription but was not incorporated during the extension step. Consequently, the resulting RNA contained a cap at its 5' end. The sequences of the oligoribonucleotides produced by the *in vitro* transcription reaction were:

+Cap:
5′m7GpppGCAUCCUACUCCCAUCCAAUUCCACCCUAACUCCUCCCAUCUCCAC-
3′ (SEQ ID NO:1)

-Cap:
5′-pppGCAUCCUACUCCCAUCCAAUUCCACCCUAACUCCUCCCAUCUCCAC-3′
(SEQ ID NO:2)

The oligoribonucleotides were dissolved in 9 µl of acetate buffer (0.1 M sodium acetate, pH 5.2) and 3 µl of freshly prepared 0.1 M sodium periodate solution. The mixture was incubated for 1 hour in the dark at 4°C or room temperature. Thereafter, the reaction was stopped by adding 4 µl of 10% ethylene glycol. The product was ethanol precipitated, resuspended in at least 10 µl of water or appropriate buffer and dialyzed against water.

The resulting aldehyde groups may then be coupled to molecules having a reactive amine group, such as hydrazine, carbazide, thiocarbazide or semicarbazide groups, in order to facilitate enrichment of the 5' ends of the mRNAs. Molecules having reactive amine groups which are suitable for use in selecting mRNAs having intact 5' ends include avidin, proteins, antibodies, vitamins, ligands capable of specifically binding to receptor molecules, or oligonucleotides. Example 3 below describes the coupling of the resulting dialdehyde to biotin.

### EXAMPLE 3

### Coupling of the Dialdehyde at the 5' End of Transcripts with Biotin

The oxidation product obtained in Example 2 was dissolved in 50 µl of sodium acetate at a pH between 5 and 5.2 and 50 µl of freshly prepared 0.02 M solution of biotin hydrazide in a methoxyethanol/water mixture (1:1) of formula:

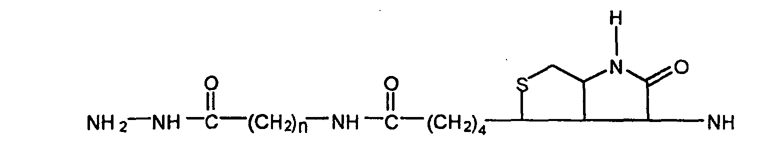

In the compound used in these experiments, n=5. However, it will be appreciated that other commercially available hydrazides may also be used, such as molecules of the above formula in which n varies from 0 to 5. The mixture was then incubated for 2 hours at 37°C, precipitated with ethanol and dialyzed against distilled water. Example 4 demonstrates the specificity of the biotinylation reaction.

### EXAMPLE 4

### Specificity of Biotinylation of Capped Transcripts

The specificity of the biotinylation for capped mRNAs was evaluated by gel electrophoresis of the following samples:
Sample 1. The 46 nucleotide uncapped *in vitro* transcript prepared as in Example 2 and labeled with ³²pCp as described in Example 1.
Sample 2. The 46 nucleotide uncapped *in vitro* transcript prepared as in Example 2, labeled with ³²pCp as described in Example 1, treated with the oxidation reaction of Example 2, and subjected to the biotinylation conditions of Example 3.
Sample 3. The 47 nucleotide capped *in vitro* transcript prepared as in Example 2 and labeled with ³²pCp as described in Example 1.
Sample 4. The 47 nucleotide capped *in vitro* transcript prepared as in Example 2, labeled with ³²pCp as described in Example 1, treated with the oxidation reaction of Example 2, and subjected to the biotinylation conditions of Example 3.

Samples 1 and 2 had identical migration rates, demonstrating that the uncapped RNAs were not oxidized and biotinylated. Sample 3 migrated more slowly than Samples 1 and 2, while Sample 4 exhibited the slowest migration. The difference in migration of the RNAs in Samples 3 and 4 demonstrates that the capped RNAs were specifically biotinylated.

In some cases, mRNAs having intact 5' ends may be enriched by binding the molecule containing a reactive amine group to a suitable solid phase substrate such as the inside of the vessel containing the mRNAs, magnetic beads, chromatography matrices, or nylon or nitrocellulose membranes. For example, where the molecule having a reactive amine group is biotin, the solid phase substrate may be coupled to avidin or streptavidin. Alternatively, where the molecule having the reactive amine group is an antibody or receptor ligand, the solid phase substrate may be coupled to the cognate antigen or receptor. Finally, where the molecule having a reactive amine group comprises an oligonucleotide, the solid phase substrate may comprise a complementary oligonucleotide.

The mRNAs having intact 5' ends may be released from the solid phase following the enrichment procedure. For example, where the dialdehyde is coupled to biotin hydrazide and the solid phase comprises streptavidin, the mRNAs may be released from the solid phase by simply heating to 95 degrees Celsius in 2% SDS. In some methods, the molecule having a reactive amine group may also be cleaved from the mRNAs having intact 5' ends following enrichment. Example 5 describes the capture of biotinylated mRNAs with streptavidin coated beads and the release of the biotinylated mRNAs from the beads following enrichment.

### EXAMPLE 5

### Capture and Release of Biotinylated mRNAs Using Streptavidin Coated Beads

The streptavidin coated magnetic beads were prepared according to the manufacturer's instructions (CPG Inc., USA). The biotinylated mRNAs were added to a hybridization buffer (1.5 M NaCI, pH 5 - 6). After incubating for 30 minutes, the unbound and nonbiotinylated material was removed. The beads were then washed several times in water with 1% SDS. The beads thus obtained were incubated for 15 minutes at 95°C in water containing 2% SDS.

Example 6 demonstrates the efficiency with which biotinylated mRNAs were recovered from the streptavidin coated beads.

### EXAMPLE 6

### Efficiency of Recovery of Biotinylated mRNAs

The efficiency of the recovery procedure was evaluated as follows. Capped RNAs were labeled with ³²pCp, oxidized, biotinylated and bound to streptavidin coated beads as described above. Subsequently, the bound RNAs were incubated for 5, 15 or 30 minutes at 95°C in the presence of 2% SDS.

The products of the reaction were analyzed by electrophoresis on 12% polyacrylamide gels under denaturing conditions (7 M urea). The gels were subjected to autoradiography. During this manipulation, the hydrazone bonds were not reduced.

Increasing amounts of nucleic acids were recovered as incubation times in 2% SDS increased, demonstrating that biotinylated mRNAs were efficiently recovered.

In an alternative method for obtaining mRNAs having intact 5' ends, an oligonucleotide which has been derivatized to contain a reactive amine group is specifically coupled to mRNAs having an intact cap. Preferably, the 3' end of the mRNA is blocked prior to the step in which the aldehyde groups are joined to the derivatized oligonucleotide, as described above, so as to prevent the derivatized oligonucleotide from being joined to the 3' end of the mRNA. For example, pCp may be attached to the 3' end of the mRNA using T4 RNA ligase as described in example 1. However, as discussed above, blocking the 3' end of the mRNA is an optional step. Derivatized oligonucleotides may be prepared as described in Example 7.

### EXAMPLE 7

### Derivatization of Oligonucleotides

An oligonucleotide phosphorylated at its 3' end was converted to a 3' hydrazide in 3' by treatment with an aqueous solution of hydrazine or of dihydrazide of the formula H₂N(R1)NH₂ at about 1 to 3 M, and at pH 4.5 at a temperature of 8°C overnight. This incubation was performed in the presence of a carbodiimide type agent soluble in water such as 1-ethyl-3-(3-dimethylaminopropyl)carbodiimide at a final concentration of 0.3 M.

The derivatized oligonucleotide was then separated from the other agents and products using a standard technique for isolating oligonucleotides.

As discussed above, the mRNAs to be enriched may be treated to eliminate the 3' OH groups which may be present thereon. This may be accomplished by enzymatic ligation of sequences lacking a 3' OH, such as pCp, as described in Example 1. Alternatively, the 3' OH groups may be eliminated by alkaline hydrolysis as described in Example 8 below.

### EXAMPLE 8

### Elimination of 3' OH Groups of mRNA Using Alkaline Hydrolvsis

In a total volume of 100 µl of 0.1 N sodium hydroxide, 1.5 µg mRNA is incubated for 40 to 60 minutes at 4°C. The solution is neutralized with acetic acid and precipitated with ethanol.

Following the optional elimination of the 3' OH groups, the diol groups at the 5' ends of the mRNAs are oxidized as described below in Example 9.

### EXAMPLE 9

### Oxidation of Diols of mRNA

Up to 1 OD unit of RNA was dissolved in 9 µl of buffer (0.1 M sodium acetate, pH 6-7) or water and 3 µl of freshly prepared 0.1 M sodium periodate solution. The reaction was incubated for 1 h in the dark at 4°C or room temperature. Following the incubation, the reaction was stopped by adding 4 µl of 10% ethylene glycol. Thereafter the mixture was incubated at room temperature for 15 minutes. After ethanol precipitation, the product was resuspended in at least 10 µl of water or appropriate buffer and dialyzed against water.

Following oxidation of the diol groups at the 5' ends of the mRNAs, the derivatized oligonucleotide was joined to the resulting aldehydes as described in Example 10.

### EXAMPLE 10

### Ligature of Aldehydes of mRNA to Derivatized Oligonucleotides

The oxidized mRNA was dissolved in an acidic medium such as 50 µl of sodium acetate pH 4-6. Fifty µl of a solution of the derivatized oligonucleotide were added in order to obtain an mRNA:derivatized oligonucleotide ratio of 1:20. The mixture was reduced with a borohydride and incubated for 2 h at 37°C or overnight (14 h) at 10°C. The mixture was then ethanol precipitated, resuspended in 10 µl or more of water or appropriate buffer and dialyzed against distilled water. If desired, the resulting product may be analyzed using acrylamide gel electrophoresis, HPLC analysis, or other conventional techniques.

Following the attachment of the derivatized oligonucleotide to the mRNAs, a reverse transcription reaction may be performed as described in Example 11 below.

### EXAMPLE 11

### Reverse Transcription of mRNAs Ligatured to Derivatized Oligonucleotides

An oligodeoxyribonucleotide was derivatized as follows. Three OD units of an oligodeoxyribonucleotide of sequence 5'ATCAAGAATTCGCACGAGACCATTAT (SEQ ID NO:3) having 5'-OH and 3'-P ends were dissolved in 70 µl of a 1.5 M hydroxybenzotriazole solution, pH 5.3, prepared in dimethylformamide'water (75:25) containing 2 µg of 1-ethyl-3-(3-dimethylaminopropyl)carbodiimide. The mixture was incubated for 2 h 30 min at 22°C and then precipitated twice in LiClO₄/acetone The pellet was resuspended in 200 µl of 0.25 M hydrazine and incubated at 8°C from 3 to 14 h. Following the hydrazine reaction, the mixture was precipitated twice in LiC1O₄/acetone.

The messenger RNAs to be reverse transcribed were extracted from blocks of placenta having sides of 2 cm which had been stored at -80°C. The total RNA was extracted using conventional acidic phenol techniques. Oligo-dT chromatography was used to purify the mRNAs. The integrity of the mRNAs was checked by Northern-blotting.

The diol groups on 7 µg of the placental mRNAs were oxidized as described above in Example 9. The derivatized oligonucleotide was joined to the mRNAs as described in Example 10 above except that the precipitation step was replaced by an exclusion chromatography step to remove derivatized oligodeoxyribonucleotides which were not joined to mRNAs. Exclusion chromatography was performed as follows:

Ten ml of Ultrogel AcA34 (BioSepra#230151) gel, a mix of agarose and acrylamide, were equilibrated in 50 ml of a solution of 10 mM Tris pH 8.0, 300 mM NaCl, 1 mM EDTA, and 0.05% SDS. The mixture was allowed to sediment. The supernatant was eliminated and the gel was resuspended in 50 ml of buffer. This procedure was repeated 2 or 3 times.

A glass bead (diameter 3 mm) was introduced into a 2 ml disposable pipette (length 25 cm). The pipette was filled with the gel suspension until the height of the gel stabilized at 1 cm from the top of the pipette. The column was then equilibrated with 20 ml of equilibration buffer (10 mM Tris HCI pH 7.4, 20 mM NaCl).

Ten µl of the mRNA which had reacted with the derivatized oligonucleotide were mixed in 39 µl of 10 mM urea and 2 µl of blue-glycerol buffer, which had been prepared by dissolving 5 mg of bromophenol blue in 60% glycerol (v/v), and passing the mixture through a 0.45 µm diameter filter.

The column was then loaded with the mRNAs coupled to the oligonucleotide. As soon as the sample had penetrated, equilibration buffer was added. Hundred µl fractions were then collected. Derivatized oligonucleotide which had not been attached to mRNA appeared in fraction 16 and later fractions. Thus, fractions 3 to 15 were combined and precipitated with ethanol.

To determine whether the derivatized oligonucleotide was actually linked to mRNA, one tenth of the combined fractions were spotted twice on a nylon membrane and hybridized to a radioactive probe using conventional techniques. The ³²P labeled probe used in these hybridizations was an oligodeoxyribonucleotide of sequence 5'TAATGGTCTCGTGCGAATTCTTGAT3' (SEQ ID NO:4) anticomplementary to the derivatized oligonucleotide. A signal observed after autoradiography, indicated that the derivatized oligonucleotide had been truly joined to the mRNA

The remaining nine tenth of the mRNAs which had reacted with the derivatized oligonucleotide was reverse transcribed as follows. A reverse transcription reaction was carried out with reverse transcriptase following the manufacturer's instructions and 50 pmol of nonamers with random sequence as primers.

To ensure that reverse transcription had been carried out through the cap structure, two types of experiments were performed.

In the first approach, after elimination of RNA of the cDNA:RNA heteroduplexes obtained from the reverse transcription reaction by an alkaline hydrolysis, a portion of the resulting single stranded cDNAs was spotted on a positively charged membrane and hybridized, using conventional methods, to a ³²P labeled probe having a sequence identical to that of the derivatized oligonucleotide. Control spots containing, 1 pmol, 100 fmol, 50 fmol, 10 fmol and 1 fmol of a control oligodeoxyribonucleotide of sequence identical to that of the derivatized oligonucleotide were included. The signal observed in the spots containing the cDNA indicated that approximately 15 fmol of the derivatized oligonucleotide had been reverse transcribed. These results demonstrate that the reverse transcription can be performed through the cap and, in particular, that reverse transcriptase crosses the 5'-P-P-P-5' bond of the cap of eukaryotic messenger RNAs.

In the second type of experiment, the single stranded cDNAs obtained from the above first strand synthesis were used as template for PCR reactions. Two types of reactions were carried out. First, specific amplification of the mRNAs for alpha globin, dehydrogenase, pp15 and elongation factor E4 were carried out using the following pairs of oligodeoxyribonucleotide primers.

### alpha-globin

GLO-S: 5'CCG ACA AGA CCA ACG TCA AGG CCG C3' (SEQ ID NO:5)

GLO-As: 5'TCA CCA GCA GGC AGT GGC TTA GGA G 3' (SEQ ID NO:6)

### dehydrogenase

3 DH-S: 5'AGT GAT TCC TGC TAC TTT GGA TGG C3' (SEQ ID NO:7)

3 DH-As: 5'GCT TGG TCT TGT TCT GGA GTT TAG A3' (SEQ ID NO:8)

### PP15

PP15-S: 5'TCC AGA ATG GGA GAC AAG CCA ATT T3' (SEQ ID NO:9)

PP15-As: 5'AGG GAG GAG GAA ACA GCG TGA GTC C3' (SEQ ID NO:10)

### Elongation factor E4

EFA1-S: 5'ATG GGA AAG GAA AAG ACT CAT ATC A3' (SEQ ID NO:11)

EF1A-As: 5'AGC AGC AAC AAT CAG GAC AGC ACA G3' (SEQ ID NO:12)

Second, non specific amplifications were also carried out with the antisense oligodeoxyribonucleotides of the pairs described above and with a primer derived from the sequence of the derivatized oligodeoxyribonucleotide

(5'ATCAAGAATTCGCACGAGACCATTA3') (SEQ ID NO:13).

One twentieth of the following RT-PCR product samples were run on a 1.5% agarose gel and stained with ethidium bromide.
Sample 1: The products of a PCR reaction using the globin primers of SEQ ID NOs 5 and 6 in the presence of cDNA
Sample 2: The products of a PCR reaction using the globin primers of SEQ ID NOs 5 and 6 in the absence of added cDNA.
Sample 3: The products of a PCR reaction using the dehydrogenase primers of SEQ ID NOs 7 and 8 in the presence ofcDNA.
Sample 4: The products of a PCR reaction using the dehydrogenase primers of SEQ ID NOs 7 and 8 in the absence of added cDNA.
Sample 5: The products of a PCR reaction using the pp15 primers of SEQ ID NOs 9 and 10 in the presence of cDNA.
Sample 6: The products of a PCR reaction using the pp15 primers of SEQ ID NOs 9 and 10 in the absence of added cDNA
Sample 7: The products of a PCR reaction using the EIF4 primers of SEQ ID NOs 11 and 12 in the presence of added cDNA.
Sample 8: The products of a PCR reaction using the EIF4 primers of SEQ ID NOs 11 and 12 in the absence of added cDNA.

A band of the size expected for the PCR product was observed only in samples 1, 3, 5 and 7, thus indicating the presence of the corresponding sequence in the cDNA population.

PCR reactions were also carried out with the antisense oligonucleotides of the globin and dehydrogenase primers (SEQ ID NOs 6 and 8) and an oligonucleotide whose sequence corresponds to that of the derivatized oligonucleotide. The presence of PCR products of the expected size in the samples equivalent to above samples 1 and 3 indicated that the derivatized oligonucleotide had been linked to mRNA.

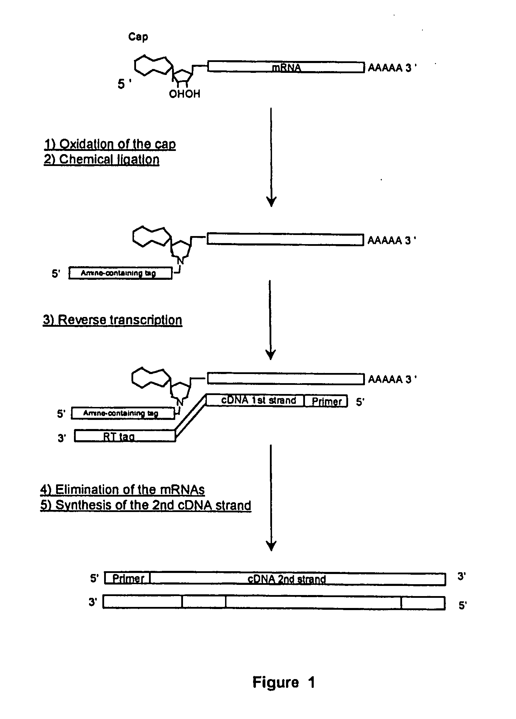

The above examples summarize the chemical procedure for enriching mRNAs for those having intact 5' ends as illustrated in Figure 1. Further detail regarding the chemical approaches for obtaining such mRNAs are disclosed in International Application No. WO96/34981, published November 7, 1996, which is incorporated herein by reference. Strategies based on the above chemical modifications to the 5' cap structure may be utilized to generate cDNAs selected to include the 5' ends of the mRNAs from which they derived. In one version of such procedures, the 5' ends of the mRNAs are modified as described above. Thereafter, a reverse transcription reaction is conducted to extend a primer complementary to the 5' end of the mRNA. Single stranded RNAs are eliminated to obtain a population of cDNA/mRNA heteroduplexes in which the mRNA includes an intact 5' end. The resulting heteroduplexes may be captured on a solid phase coated with a molecule capable of interacting with the molecule used to derivatize the 5' end of the mRNA. Thereafter, the strands of the heteroduplexes are separated to recover single stranded first cDNA strands which include the 5' end of the mRNA. Second strand cDNA synthesis may then proceed using conventional techniques. For example, the procedures disclosed in WO 96/34981 or in Carninci. *et al., Genomics* **37**:327-336, 1996, the disclosures of which are incorporated herein by reference, may be employed to select cDNAs which include the sequence derived from the 5' end of the coding sequence of the mRNA.

Following ligation of the oligonucleotide tag to the 5' cap of the mRNA, a reverse transcription reaction is conducted to extend a primer complementary to the mRNA to the 5' end of the mRNA. Following elimination of the RNA component of the resulting heteroduplex using standard techniques, second strand cDNA synthesis is conducted with a primer complementary to the oligonucleotide tag.

### 2. Enzymatic Methods for Obtaining mRNAs having Intact 5' Ends

Other techniques for selecting cDNAs extending to the 5' end of the mRNA from which they are derived are fully enzymatic. Some versions of these techniques are disclosed in Dumas Milne Edwards J.B. (Doctoral Thesis of Paris VI University, Le clonage des ADNc complets: difficultes et perspectives nouvelles. Apports pour l'etude de la regulation de l'expression de la tryptophane hydroxylase de rat, 20 Dec. 1993 EP0 625572 and Kato *et al.. Gene* **150**:243-250, 1994, the disclosures of which are incorporated herein by reference.

Briefly, in such approaches, isolated mRNA is treated with alkaline phosphatase to remove the phosphate groups present on the 5' ends of uncapped incomplete mRNAs. Following this procedure, the cap present on full length mRNAs is enzymatically removed with a decapping enzyme such as T4 polynucleotide kinase or tobacco acid pyrophosphatase. An oligonucleotide, which may be either a DNA oligonucleotide or a DNA-RNA hybrid oligonucleotide having RNA at its 3' end, is then ligated to the phosphate present at the 5' end of the decapped mRNA using T4 RNA ligase. The oligonucleotide may include a restriction site to facilitate cloning of the cDNAs following their synthesis. Example 12 below describes one enzymatic method based on the doctoral thesis of Dumas.

### EXAMPLE 12

### Enzymatic Approach for Obtaining 5' ESTs

Twenty micrograms of PolyA+ RNA were dephosphorylated using Calf Intestinal Phosphatase (Biolabs). After a phenol chloroform extraction, the cap structure of mRNA was hydrolysed using the Tobacco Acid Pyrophosphatase (purified as described by Shinshi *et al.., Biochemistry* **15**: 2185-2190, 1976) and a hemi 5'DNA/RNA-3' oligonucleotide having an unphosphorylated 5' end, a stretch of adenosine ribophosphate at the 3' end, and an EcoRI site near the 5' end was ligated to the 5'P ends of mRNA using the T4 RNA ligase (Biolabs). Oligonucleotides suitable for use in this procedure are preferably 30 to 50 bases in length. Oligonucleotides having an unphosphorylated 5' end may be synthesized by adding a fluorochrome at the 5' end. The inclusion of a stretch of adenosine ribophosphates at the 3' end of the oligonucleotide increases ligation efficiency. It will be appreciated that the oligonucleotide may contain cloning sites other than EcoRI.

Following ligation of the oligonucleotide to the phosphate present at the 5' end of the decapped mRNA, first and second strand cDNA synthesis is carried out using conventional methods or those specified in EP0 625,572 and Kato *et al. supra,* and Dumas Milne Edwards, *supra,* the disclosures of which are incorporated herein by reference. The resulting cDNA may then be ligated into vectors such as those disclosed in Kato *et al., supra* or other nucleic acid vectors known to those skilled in the art using techniques such as those described in Sambrook *et al.*, Molecular Cloning: A Laboratory Manual 2d Ed., Cold Spring Harbor Laboratory Press, 1989, the disclosure of which is incorporated herein by reference.

### II. Obtention and Characterization of the 5' ESTs of the Present Invention

The 5' ESTs of the present invention were obtained using the aforementioned chemical and enzymatic approaches for enriching mRNAs for those having intact 5' ends as decribed below.

### 1. Obtention of 5' ESTS Using mRNAs with Intact 5' Ends

First, mRNAs were prepared as described in Example 13 below.

### EXAMPLE 13

### Preparation of mRNA With Intact 5' Ends

Total human RNAs or polyA⁺ RNAs derived from 29 different tissues were respectively purchased from LABIMO and CLONTECH and used to generate 44 cDNA libraries as follows. The purchased RNA had been isolated from cells or tissues using acid guanidium thiocyanate-phenol-chloroform extraction (Chomczyniski and Sacchi, *Analytical Biochemistry* **162**:156-159, 1987). PolyA⁺ RNA was isolated from total RNA (LABIMO) by two passes of oligo dT chromatography, as described by Aviv and Leder, *Proc. Natl. Acad Sci. USA* **69**:1408-1412, 1972 in order to eliminate ribosomal RNA.

The quality and the integrity of the polyA+ RNAs were checked. Northern blots hybridized with a globin probe were used to confirm that the mRNAs were not degraded. Contamination of the polyA⁺ mRNAs by ribosomal sequences was checked using Northern blots and a probe derived from the sequence of the 28S rRNA. Preparations of mRNAs with less than 5% of rRNAs were used in library construction. To avoid constructing libraries with RNAs contaminated by exogenous sequences (prokaiyotic or fungal), the presence of bacterial 16S ribosomal sequences or of two highly expressed fungal mRNAs was examined using PCR.

Following preparation of the mRNAs, the above described chemical and/or the enzymatic procedures for enriching mRNAs for thoses having intact 5' ends were employed to obtain 5' ESTs from various tissues. In both approaches, an oligonucleotide tag was attached to the 5' ends of the mRNAs. The oligonucleotide tag had an EcoRI site therein to facilitate later cloning procedures. To facilitate the processing of single stranded and double stranded cDNA obtained in the construction of the librairies, the same nucleotidic sequence was used to design the ligated oligonucleotide in both chemical and enzymatic approaches. Nevertheless, in the chemical procedure, the tag used was an oligodeoxyribonucleotide which was linked to the cap of the mRNA whereas in the enzymatic ligation, the tag was a chimeric hemi 5'DNA/RNA3' oligonucleotide which was ligated to the 5' end of decapped mRNA as described in example 12.

Following attachment of the oligonucleotide tag to the mRNA by either the chemical or enzymatic methods, the integrity of the mRNA was examined by performing a Northern blot with 200 to 500 ng of mRNA using a probe complementary to the oligonucleotide tag before performing the first strand synthesis as described in example 14.

### EXAMPLE 14

### cDNA Synthesis Using mRNA Templates Having Intact 5' Ends

For the mRNAs joined to oligonucleotide tags using both the chemical and enzymatic methods, first strand cDNA synthesis was performed using the Superscript II (Gibco BRL) or the Rnase H Minus M-MLV (Promega) reverse transcriptase with random nonamers as primers. In order to protect internal EcoRI sites in the cDNA from digestion at later steps in the procedure, methylated dCTP was used for first strand synthesis. After removal of RNA by an alkaline hydrolysis, the first strand of cDNA was precipitated using isopropanol in order to eliminate residual primers.

For both the chemical and the enzymatic methods, the second strand of the cDNA was synthesized with a Klenow fragment using a primer corresponding to the 5' end of the ligated oligonucleotide described in Example 12. Preferably, the primer is 20-25 bases in length. Methylated dCTP was also used for second strand synthesis in order to protect internal EcoRI sites in the cDNA from digestion during the cloning process.

Following cDNA synthesis, the cDNAs were cloned into pBlueScript as described in Example 15 below.

### EXAMPLE 15

### Cloning of cDNAsderived from mRNA with intact 5' ends into Bluescript

Following second strand synthesis, the ends of the cDNA were blunted with T4 DNA polymerase (Biolabs) and the cDNA was digested with EcoRI. Since methylated dCTP was used during cDNA synthesis, the EcoRI site present in the tag was the only hemi-methylated site, hence the only site susceptible to EcoRI digestion. The cDNA was then size fractionated using exclusion chromatography (AcA, Biosepra) and fractions corresponding to cDNAs of more than 150 bp were pooled and ethanol precipitated. The cDNA was directionally cloned into the SmaI and EcoRI ends of the phagemid pBlueScript vector (Stratagene). The ligation mixture was electroporated into bacteria and propagated under appropriate antibiotic selection.

Clones containing the oligonucleotide tag attached were then selected as described in Example 16 below.

### EXAMPLE 16

### Selection of Clones Having the Oligonucleotide Tag Attached Thereto

The plasmid DNAs containing 5' EST libraries made as described above were purified (Qiagen). A positive selection of the tagged clones was performed as follows. Briefly, in this selection procedure, the plasmid DNA was converted to single stranded DNA using gene II endonuclease of the phage F1 in combination with an exonuclease (Chang *et al., Gene* **127**:95-8, 1993) such as exonuclease III or T7 gene 6 exonuclease. The resulting single stranded DNA was then purified using paramagnetic beads as described by Fry *et al., Biotechniques,* **13:** 124-131, 1992. In this procedure, the single stranded DNA was hybridized with a biotinylated oligonucleotide having a sequence corresponding to the 3' end of the oligonucleotide described in Example 13. Preferably, the primer has a length of 20-25 bases. Clones including a sequence complementary to the biotinylated oligonucleotide were captured by incubation with streptavidin coated magnetic beads followed by magnetic selection. After capture of the positive clones, the plasmid DNA was released from the magnetic beads and converted into double stranded DNA using a DNA polymerase such as the ThermoSequenase obtained from Amersham Pharmacia Biotech. Alternatively, protocoles such as the one described in the Gene Trapper kit available from Gibco BRL may be used. The double stranded DNA was then electroporated into bacteria. The percentage of positive clones having the 5' tag oligonucleotide was estimated to typically rank between 90 and 98% using dot blot analysis.

Following electroporation, the libraries were ordered in 384-microtiter plates (MTP). A copy of the MTP was stored for future needs. Then the libraries were transferred into 96 MTP and sequenced as described below.

### EXAMPLE 17

### Sequencing of Inserts in Selected Clones

Plasmid inserts were first amplified by PCR on PE 9600 thermocyclers (Perkin-Elmer, Applied Biosystems Division, Foster City, CA), using standard SETA-A and SETA-B primers (Genset SA), AmpliTaqGold (Perkin-Elmer), dNTPs (Boehringer), buffer and cycling conditions as recommended by the Perkin-Elmer Corporation.

PCR products were then sequenced using automatic ABI Prism 377 sequencers (Perkin Elmer). Sequencing reactions were performed using PE 9600 thermocyclers with standard dye-primer chemistry and ThermoSequenase (Amersham Pharmacia Biotech). The primers used were either T7 or 21M13 (available from Genset SA) as appropriate. The primers were labeled with the JOE, FAM, ROX and TAMRA dyes. The dNTPs and ddNTPs used in the sequencing reactions were purchased from Boehringer. Sequencing buffer, reagent concentrations and cycling conditions were as recommended by Amersham.

Following the sequencing reaction, the samples were precipitated with ethanol, resuspended in formamide loading buffer, and loaded on a standard 4% acrylamide gel. Electrophoresis was performed for 2.5 hours at 3000V on an ABI 377 sequencer, and the sequence data were collected and analyzed using the ABI Prism DNA Sequencing Analysis Software, version 2.1.2.

### 2. Computer analysis of the Obtained 5' ESTs: Construction of NetGene and SignalTag databases

The sequence data from the 44 cDNA libraries made as described above were transferred to a proprietary database, where quality control and validation steps were performed. A proprietary base-caller, working using a Unix system, automatically flagged suspect peaks, taking into account the shape of the peaks, the inter-peak resolution, and the noise level. The proprietary base-caller also performed an automatic trimming. Any stretch of 25 or fewer bases having more than 4 suspect peaks was considered unreliable and was discarded. Sequences corresponding to cloning vector or ligation oligonucleotides were automatically removed from the EST sequences. However, the resulting EST sequences may contain 1 to 5 bases belonging to the above mentioned sequences at their 5' end. If needed, these can easily be removed on a case to case basis.

Following sequencing as described above, the sequences of the 5' ESTs were entered in NetGene™, a proprietary database called for storage and manipulation as described below. It will be appreciated by those skilled in the art that the data could be stored and manipulated on any medium which can be read and accessed by a computer. Computer readable media include magnetically, optically, or electronically readable media. For example, the computer readable media may be a hard disc, a floppy disc, a magnetic tape, CD-ROM, RAM, or ROM as well as other types of other media known to those skilled in the art.

In addition, the sequence data may be stored and manipulated in a variety of data processor programs in a diversity of formats. For instance, the sequence data may be stored as text in a word processing file, such as Microsoft WORD or WORDPERFECT or as an ASCII file in a variety of database programs familiar to those of skill in the art, such as DB2, SYBASE, or ORACLE.

The computer readable media on which the sequence information is stored may be in a personal computer, a network, a server or other computer systems known to those skilled in the art. The computer or other system preferably includes the storage media described above, and a processor for accessing and manipulating the sequence data. Once the sequence data has been stored, it may be manipulated and searched to locate those stored sequences which contain a desired nucleic acid sequence or which encode a protein having a particular functional domain. For example, the stored sequence information may be compared to other known sequences to identify homologies, motifs implicated in biological function, or structural motifs.

Programs which may be used to search or compare the stored sequences include the MacPattern (EMBL), BLAST, and BLAST2 program series (NCBI), basic local alignment search tool programs for nucleotide (BLASTN) and peptide (BLASTX) comparisons (Altschul *et al. J. Mol. Biol.* **215**: 403, 1990) and FASTA (Pearson and Lipman, *Proc. Natl. Acad. Sci. USA* **85**: 2444, 1988). The BLAST programs then extend the alignments on the basis of defined match and mismatch criteria.

Motifs which may be detected using the above programs and those described in Example 28 include sequences encoding leucine zippers, helix-turn-helix motifs, glycosylation sites, ubiquitination sites, alpha helices, and beta sheets, signal sequences encoding signal peptides which direct the secretion of the encoded proteins, sequences implicated in transcription regulation such as homeoboxes, acidic stretches, enzymatic active sites, substrate binding sites, and enzymatic cleavage sites.

Before searching the cDNAs in the NetGene™ database for sequence motifs of interest, cDNAs derived from mRNAs which were not of interest were identified and eliminated from further consideration as described in Example 18 below.

### EXAMPLE 18

### Elimination of Undesired Sequences from Further Consideration

5' ESTs in the NetGene™ database which were derived from undesired sequences such as transfer RNAs, ribosomal RNAs, mitochondrial RNAs, prokaryotic RNAs, fungal RNAs, Alu sequences, L1 sequences, or repeat sequences were identified using the FASTA and BLASTN programs with the parameters listed in Table I.

To eliminate 5' ESTs encoding tRNAs from further consideration, the 5' EST sequences were compared to the sequences of 1119 known tRNAs obtained from EMBL release 38, of which 100 were human. The comparison was performed using FASTA on both strands of the 5' ESTs. Sequences having more than 80% homology over more than 60 nucleotides were identified as tRNA. Of the 144,341 sequences screened, 26 were identified as tRNAs and eliminated from further consideration.

To eliminate 5' ESTs encoding rRNAs from further consideration, the 5' EST sequences were compared to the sequences of 2497 known rRNAs obtained from EMBL release 38, of which 73 were human. The comparison was performed using BLASTN on both strands of the 5' ESTs with the parameter S=108. Sequences having more than 80% homology over stretches longer than 40 nucleotides were identified as rRNAs. Of the 144,341 sequences screened, 3,312 were identified as rRNAs and eliminated from further consideration.

To eliminate 5' ESTs encoding mtRNAs from further consideration, the 5' EST sequences were compared to the sequences of the two known mitochondrial genomes for which the entire genomic sequences are available and all sequences transcribed from these mitochondrial genomes including tRNAs, rRNAs, and mRNAs for a total of 38 sequences. The comparison was performed using BLASTN on both strands of the 5' ESTs with the parameter S=108. Sequences having more than 80% homology over stretches longer than 40 nucleotides were identified as mtRNAs. Of the 144,341 sequences screened, 6,110 were identified as mtRNAs and eliminated from further consideration.

Sequences which might have resulted from exogenous contaminants were eliminated from further consideration by comparing the 5' EST sequences to release 46 of the EMBL bacterial and fungal divisions using BLASTN with the parameter S=144. All sequences having more than 90% homology over at least 40 nucleotides were identified as exogenous contaminants. Of the 42 cDNA libraries examined, the average percentages of prokaryotic and fungal sequences contained therein were 0.2% and 0.5% respectively. Among these sequences, only one could be identified as a sequence specific to fungi. The others were either fungal or prokaryotic sequences having homologies with vertebrate sequences or including repeat sequences which had not been masked during the electronic comparison.

In addition, the 5' ESTs were compared to 6093 Alu sequences and 1115 L1 sequences to mask 5' ESTs containing such repeat sequences. 5' ESTs including THE and MER repeats, SSTR sequences or satellite, micro-satellite, or telomeric repeats were also eliminated from further consideration. On average, 11.5% of the sequences in the libraries contained repeat sequences. Of this 11.5%, 7% contained Alu repeats, 3.3% contained L1 repeats and the remaining 1.2% were derived from the other screened types of repetitive sequences. These percentages are consistent with those found in cDNA libraries prepared by other groups. For example, the cDNA libraries of Adams *et al.* contained between 0% and 7.4% Alu repeats depending on the source of the RNA which was used to prepare the cDNA library (Adams *et al., Nature* **377**:174, *1996).*

The sequences of those 5' ESTs remaining after the elimination of undesirable sequences were compared with the sequences of known human mRNAs to determine the accuracy of the sequencing procedures described above.

### EXAMPLE 19

### Measurement of Sequencing Accuracy by Comparison to Known Sequences

To further determine the accuracy of the sequencing procedure described above, the sequences of 5' ESTs derived from known sequences were identified and compared to the original known sequences. First, a FASTA analysis with overhangs shorter than 5 bp on both ends was conducted on the 5' ESTs to identify those matching an entry in the public human mRNA database. The 6655 5' ESTs which matched a known human mRNA were then realigned with their cognate mRNA and dynamic programming was used to include substitutions, insertions, and deletions in the list of "errors" which would be recognized. Errors occurring in the last 10 bases of the 5' EST sequences were ignored to avoid the inclusion of spurious cloning sites in the analysis of sequencing accuracy.

This analysis revealed that the sequences incorporated in the NetGene^{TM} database had an accuracy of more than 99.5%.

To determine the efficiency with which the above selection procedures select cDNAs which include the 5' ends of their corresponding mRNAs, the following analysis was performed.

### EXAMPLE 20

### Determination of Efficiency of 5' EST Selection

To determine the efficiency at which the above selection procedures isolated 5' ESTs which included sequences close to the 5' end of the mRNAs from which they derived, the sequences of the ends of the 5' ESTs derived from the elongation factor 1 subunit α and ferritin heavy chain genes were compared to the known cDNA sequences of these genes. Since the transcription start sites of both genes are well characterized, they may be used to determine the percentage of derived 5' ESTs which included the authentic transcription start sites.

For both genes, more than 95% of the obtained 5' ESTs actually included sequences close to or upstream of the 5' end of the corresponding mRNAs.

To extend the analysis of the reliability of the procedures for isolating 5' ESTs from ESTs in the NetGene™ database, a similar analysis was conducted using a database composed of human mRNA sequences extracted from GenBank database release 97 for comparison. The 5' ends of more than 85% of 5' ESTs derived from mRNAs included in the GeneBank database were located close to the 5' ends of the known sequence. As some of the mRNA sequences available in the GenBank database are deduced from genomic sequences, a 5' end matching with these sequences will be counted as an internal match. Thus, the method used here underestimates the yield of ESTs including the authentic 5' ends of their corresponding mRNAs.

The EST libraries made above included multiple 5' ESTs derived from the same mRNA. The sequences of such 5' ESTs were compared to one another and the longest 5' ESTs for each mRNA were identified. Overlapping cDNAs were assembled into continuous sequences (contigs). The resulting continuous sequences were then compared to public databases to gauge their similarity to known sequences, as described in Example 21 below.

### EXAMPLE 21

### Clustering of the 5' ESTs and Calculation of Novelty Indices for cDNA Libraries

For each sequenced EST library, the sequences were clustered by the 5' end. Each sequence in the library was compared to the others with BLASTN2 (direct strand, parameters S=107). ESTs with High Scoring Segment Pairs (HSPs) at least 25 bp long, having 95% identical bases and beginning closer than 10 bp from each EST 5' end were grouped. The longest sequence found in the cluster was used as representative of the group. A global clustering between libraries was then performed leading to the definition of super-contigs.

To assess the yield of new sequences within the EST libraries, a novelty rate (NR) was defined as: NR= 100 X (Number of new unique sequences found in the library/Total number of sequences from the library). Typically, novelty rating ranged between 10% and 41% depending on the tissue from which the EST library was obtained. For most of the libraries, the random sequencing of 5' EST libraries was pursued until the novelty rate reached 20%.

Following characterization as described above, the collection of 5' ESTs in NetGene™ was screened to identify those 5' ESTs bearing potential signal sequences as described in Example 22 below.

### EXAMPLE 22

### Identification of Potential Signal Sequences in 5' ESTs

The 5' ESTs in the NetGene^{TM} database were screened to identify those having an uninterrupted open reading frame (ORF) longer than 45 nucleotides beginning with an ATG codon and extending to the end of the EST. Approximately half of the cDNA sequences in NetGene™ contained such an ORF. The ORFs of these 5' ESTs were then searched to identify potential signal motifs using slight modifications of the procedures disclosed in Von Heijne, *Nucleic Acids Res*. **14**:4683-4690, 1986, the disclosure of which is incorporated herein by reference. Those 5' EST sequences encoding a stretch of at least 15 amino acid long with a score of at least 3.5 in the Von Heijne signal peptide identification matrix were considered to possess a signal sequence. Those 5' ESTs which matched a known human mRNA or EST sequence and had a 5' end more than 20 nucleotides downstream of the known 5' end were excluded from further analysis. The remaining cDNAs having signal sequences therein were included in a database called SignalTag™.

To confirm the accuracy of the above method for identifying signal sequences, the analysis of Example 23 was performed.

### EXAMPLE 23

### Confirmation of Accuracy of Identification of Potential Signal Sequences in 5' ESTs

The accuracy of the above procedure for identifying signal sequences encoding signal peptides was evaluated by applying the method to the 43 amino acids located at the N terminus of all human SwissProt proteins. The computed Von Heijne score for each protein was compared with the known characterization of the protein as being a secreted protein or a non-secreted protein. In this manner, the number of non-secreted proteins having a score higher than 3.5 (false positives) and the number of secreted proteins having a score lower than 3.5 (false negatives) could be calculated.

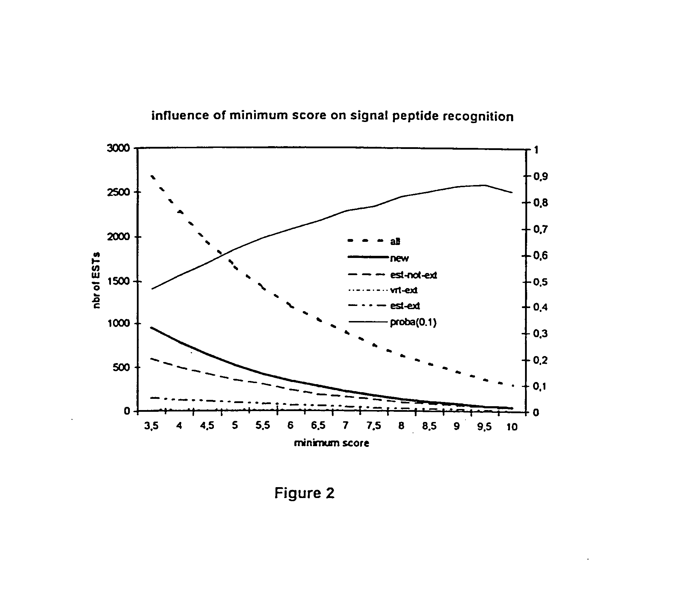

Using the results of the above analysis, the probability that a peptide encoded by the 5' region of the mRNA is in fact a genuine signal peptide based on its Von Heijne's score was calculated based on either the assumption that 10% of human proteins are secreted or the assumption that 20% of human proteins are secreted. The results of this analysis are shown in Figure 2 and table IV.

Using the above method of identification of secretory proteins, 5' ESTs of the following polypeptides known to be secreted were obtained: human glucagon, gamma interferon induced monokine precursor, secreted cyclophilin-like protein, human pleiotropin, and human biotinidase precursor. Thus, the above method successfully identified those 5' ESTs which encode a signal peptide.

To confirm that the signal peptide encoded by the 5' ESTs actually functions as a signal peptide, the signal sequences from the 5' ESTs may be cloned into a vector designed for the identification of signal peptides. Such vectors are designed to confer the ability to grow in selective medium only to host cells containing a vector with an operably linked signal sequence. For example, to confirm that a 5' EST encodes a genuine signal peptide, the signal sequence of the 5' EST may be inserted upstream and in frame with a non-secreted form of the yeast invertase gene in signal peptide selection vectors such as those described in U.S. Patent No. 5,536,637, the disclosure of which is incorporated herein by reference. Growth of host cells containing signal sequence selection vectors with the correctly inserted 5' EST signal sequence confirms that the 5' EST encodes a genuine signal peptide.

Alternatively, the presence of a signal peptide may be confirmed by cloning the extended cDNAs obtained using the ESTs into expression vectors such as pXT1 (as described below in example 30), or by constructing promoter-signal sequence-reporter gene vectors which encode fusion proteins between the signal peptide and an assayable reporter protein. After introduction of these vectors into a suitable host cell, such as COS cells or NIH 3T3 cells, the growth medium may be harvested and analyzed for the presence of the secreted protein. The medium from these cells is compared to the medium from control cells containing vectors lacking the signal sequence or extended cDNA insert to identify vectors which encode a functional signal peptide or an authentic secreted protein.

Those 5' ESTs which encoded a signal peptide, as determined by the method of Example 22 above, were further grouped into four categories based on their homology to known sequences as described in Example 24 below.

### EXAMPLE 24

### Categorization of 5' ESTs Encoding a Signal Peptide

Those 5' ESTs having a sequence not matching any known vertebrate sequence nor any publicly available EST sequence were designated "new." Of the sequences in the SignalTag^{TM} database, 947 of the 5' ESTs having a Von Heijne's score of at least 3.5 fell into this category.

Those 5' ESTs having a sequence not matching any vertebrate sequence but matching a publicly known EST were designated "EST-ext", provided that the known EST sequence was extended by at least 40 nucleotides in the 5' direction. Of the sequences in the SignalTag™ database, 150 of the 5' ESTs having a Von Heijne's score of at least 3.5 fell into this category.

Those ESTs not matching any vertebrate sequence but matching a publicly known EST without extending the known EST by at least 40 nucleotides in the 5' direction were designated "EST." Of the sequences in the SignalTag^{TM} database, 599 of the 5' ESTs having a Von Heijne's score of at least 3.5 fell into this category.

Those 5' ESTs matching a human mRNA sequence but extending the known sequence by at least 40 nucleotides in the 5' direction were designated "VERT-ext." Of the sequences in the SignalTag™ database, 23 of the 5' ESTs having a Von Heijne's score of at least 3.5 fell into this category. Included in this category was a 5' EST which extended the known sequence of the human translocase mRNA by more than 200 bases in the 5' direction. A 5' EST which extended the sequence of a human tumor suppressor gene in the 5' direction was also identified.

Table V shows the distribution of 5' ESTs in each category and the number of 5' ESTs in each category having a given minimum von Heijne's score.

### 3. Evaluation of Spatial and Temporal Expression of mRNAs Corresponding to the 5'ESTs or Extended cDNAs

Each of the 5' ESTs was also categorized based on the tissue from which its corresponding mRNA was obtained, as described below in Example 25.

### EXAMPLE 25

### Categorization of Expression Patterns

Table VI shows the distribution of 5' ESTs in each of the above defined category with respect to the tissue from which the 5'ESTs of the corresponding mRNA were obtained.

Table II provides the sequence identification numbers of 5' EST sequences derived from muscle and other mesodermal tissues, the categories in which these sequences fall, and the von Heijne's score of the signal peptides which they encode. The 5' EST sequences and the amino acid sequences they encode are provided in the appended sequence listings. Table III provides the sequence ID numbers of the 5' ESTs and the sequences of the signal peptides which they encode. The sequences of the 5' ESTs and the polypeptides they encode are provided in the sequence listing appended hereto.

The sequences of DNA SEQ ID NOs: 38-305 can readily be screened for any errors therein and any sequence ambiguities can be resolved by resequencing a fragment containing such errors or amibiguities on both strands. Such fragments may be obtained from the plasmids stored in the inventors' laboratory or can be isolated using the techniques described herein. Resolution of any such ambiguities or errors may be facilitated by using primers which hybridize to sequences located close to the ambiguous or erroneous sequences. For example, the primers may hybridize to sequences within 50-75 bases of the amibiguity or error. Upon resolution of an error or ambiguity, the corresponding corrections can be made in the protein sequences encoded by the DNA containing the error or amibiguity.

In addition to categorizing the 5' ESTs with respect to their tissue of origin, the spatial and temporal expression patterns of the mRNAs corresponding to the 5' ESTs, as well as their expression levels, may be determined as described in Example 26 below. Characterization of the spatial and temporal expression patterns and expression levels of these mRNAs is useful for constructing expression vectors capable of producing a desired level of gene product in a desired spatial or temporal manner, as will be discussed in more detail below.

Furthermore, 5' ESTs whose corresponding mRNAs are associated with disease states may also be identified. For example, a particular disease may result from the lack of expression, over expression, or under expression of an mRNA corresponding to a 5' EST. By comparing mRNA expression patterns and quantities in samples taken from healthy individuals with those from individuals suffering from a particular disease, 5' ESTs responsible for the disease may be identified.

It will be appreciated that the results of the above characterization procedures for 5' ESTs also apply to extended cDNAs (obtainable as described below) which contain sequences adjacent to the 5' ESTs. It will also be appreciated that if desired, characterization may be delayed until extended cDNAs have been obtained rather than characterizing the ESTs themselves.

### EXAMPLE 26

### Evaluation of Expression Levels and Patterns of mRNAs Corresponding to 5' ESTs or Extended cDNAs

Expression levels and patterns of mRNAs corresponding to 5' ESTs or extended cDNAs (obtainable as described below in example 27) may be analyzed by solution hybridization with long probes as described in International Patent Application No. WO 97/05277, the entire contents of which are hereby incorporated by reference. Briefly, a 5' EST, extended cDNA, or fragment thereof corresponding to the gene encoding the mRNA to be characterized is inserted at a cloning site immediately downstream of a bacteriophage (T3, T7 or SP6) RNA polymerase promoter to produce antisense RNA. Preferably, the 5' EST or extended cDNA has 100 or more nucleotides. The plasmid is linearized and transcribed in the presence of ribonucleotides comprising modified ribonucleotides (*i.e.* biotin-UTP and DIG-UTP). An excess of this doubly labeled RNA is hybridized in solution with mRNA isolated from cells or tissues of interest. The hybridizations are performed under standard stringent conditions (40-50°C for 16 hours in an 80% formamide, 0.4 M NaCI buffer, pH 7-8). The unhybridized probe is removed by digestion with ribonucleases specific for single-stranded RNA *(i.e.* RNases CL3, T1, Phy M, U2 or A). The presence of the biotin-UTP modification enables capture of the hybrid on a microtitration plate coated with streptavidin. The presence of the DIG modification enables the hybrid to be detected and quantified by ELISA using an anti-DIG antibody coupled to alkaline phosphatase.

The 5' ESTs, extended cDNAs, or fragments thereof may also be tagged with nucleotide sequences for the serial analysis of gene expression (SAGE) as disclosed in UK Patent Application No. 2 305 241 A, the entire contents of which are incorporated by reference. In this method, cDNAs are prepared from a cell, tissue, organism or other source of nucleic acid for which gene expression patterns must be determined. The resulting cDNAs are separated into two pools. The cDNAs in each pool are cleaved with a first restriction endonuclease, called an anchoring enzyme, having a recognition site which is likely to be present at least once in most cDNAs. The fragments which contain the 5' or 3' most region of the cleaved cDNA are isolated by binding to a capture medium such as streptavidin coated beads. A first oligonucleotide linker having a first sequence for hybridization of an amplification primer and an internal restriction site for a so-called tagging endonuclease is ligated to the digested cDNAs in the first pool. Digestion with the second endonuclease produces short tag fragments from the cDNAs.

A second oligonucleotide having a second sequence for hybridization of an amplification primer and an internal restriction site is ligated to the digested cDNAs in the second pool. The cDNA fragments in the second pool are also digested with the tagging endonuclease to generate short tag fragments derived from the cDNAs in the second pool. The tags resulting from digestion of the first and second pools with the anchoring enzyme and the tagging endonuclease are ligated to one another to produce so-called ditags. In some embodiments, the ditags are concatamerized to produce ligation products containing from 2 to 200 ditags. The tag sequences are then determined and compared to the sequences of the 5' ESTs or extended cDNAs to determine which 5' ESTs or extended cDNAs are expressed in the cell, tissue, organism, or other source of nucleic acids from which the tags were derived. In this way, the expression pattern of the 5' ESTs or extended cDNAs in the cell, tissue, organism, or other source of nucleic acids is obtained.

Quantitative analysis of gene expression may also be performed using arrays. As used herein, the term array means a one dimensional, two dimensional, or multidimensional arrangement of full length cDNAs *(i.e.* extended cDNAs which include the coding sequence for the signal peptide, the coding sequence for the mature protein, and a stop codon), extended cDNAs, 5' ESTs or fragments thereof of sufficient length to permit specific detection of gene expression. Preferably, the fragments are at least 15 nucleotides in length. More preferably, the fragments are at least 100 nucleotide long. More preferably, the fragments are more than 100 nucleotides in length. In some embodiments, the fragments may be more than 500 nucleotide long.

For example, quantitative analysis of gene expression may be performed with full length cDNAs as defined below, extended cDNAs, 5' ESTs, or fragments thereof in a complementary DNA microarray as described by Schena *et al.* (*Science* **270**:467-470, 1995; *Proc. Natl. Acad Sci. U.S.A.* **93**:10614-10619, 1996). Full length cDNAs, extended cDNAs, 5' ESTs or fragments thereof are amplified by PCR and arrayed from 96-well microtiter plates onto silylated microscope slides using high-speed robotics. Printed arrays are incubated in a humid chamber to allow rehydration of the array elements and rinsed, once in 0.2% SDS for 1 min, twice in water for 1 min and once for 5 min in sodium borohydride solution. The arrays are submerged in water for 2 min at 95°C, transferred into 0.2% SDS for 1 min, rinsed twice with water, air dried and stored in the dark at 25°C.

Cell or tissue mRNA is isolated or commercially obtained and probes are prepared by a single round of reverse transcription. Probes are hybridized to 1 cm² microarrays under a 14 x 14 mm glass coverslip for 6-12 hours at 60°C. Arrays are washed for 5 min at 25°C in low stringency wash buffer (1 x SSC/0.2% SDS), then for 10 min at room temperature in high stringency wash buffer (0.1 x SSC/0.2% SDS). Arrays are scanned in 0.1 x SSC using a fluorescence laser scanning device fitted with a custom filter set. Accurate differential expression measurements are obtained by taking the average of the ratios of two independent hybridizations.

Quantitative analysis of the expression of genes may also be performed with full length cDNAs, extended cDNAs, 5' ESTs, or fragments thereof in complementary DNA arrays as described by Pietu *et al..* (*Genome Research* **6**:492-503, 1996). The full length cDNAs, extended cDNAs, 5' ESTs or fragments thereof are PCR amplified and spotted on membranes. Then, mRNAs originating from various tissues or cells are labeled with radioactive nucleotides. After hybridization and washing in controlled conditions, the hybridized mRNAs are detected by phospho-imaging or autoradiography. Duplicate experiments are performed and a quantitative analysis of differentially expressed mRNAs is then performed.

Alternatively, expression analysis of the 5' ESTs or extended cDNAs can be done through high density nucleotide arrays as described by Lockhart *et al.* (*Nature Biotechnology* **14**: 1675-1680, 1996) and Sosnowsky *et al.* (*Proc. Natl. Acad. Sci.* **94**:1119-1123, 1997). Oligonucleotides of 15-50 nucleotides corresponding to sequences of the 5' ESTs or extended cDNAs are synthesized directly on the chip (Lockhart *el al., supra*) or synthesized and then addressed to the chip (Sosnowsky *et al., supra*)*.* Preferably, the oligonucleotides are about 20 nucleotides in length.

cDNA probes labeled with an appropriate compound, such as biotin, digoxigenin or fluorescent dye, are synthesized from the appropriate mRNA population and then randomly fragmented to an average size of 50 to 100 nucleotides. The said probes are then hybridized to the chip. After washing as described in Lockhart *et al, supra* and application of different electric fields (Sonowsky et *al, supra.*)*,* the dyes or labeling compounds are detected and quantified. Duplicate hybridizations are performed. Comparative analysis of the intensity of the signal originating from cDNA probes on the same target oligonucleotide in different cDNA samples indicates a differential expression of the mRNA corresponding to the 5' EST or extended cDNA from which the oligonucleotide sequence has been designed.

### III. Use of 5' ESTs to Clone Extended cDNAs and to Clone the Corresponding Genomic DNAs

Once 5' ESTs which include the 5' end of the corresponding mRNAs have been selected using the procedures described above, they can be utilized to isolate extended cDNAs which contain sequences adjacent to the 5' ESTs. The extended cDNAs may include the entire coding sequence of the protein encoded by the corresponding mRNA, including the authentic translation start site, the signal sequence, and the sequence encoding the mature protein remaining after cleavage of the signal peptide. Such extended cDNAs are referred to herein as "full length cDNAs." Alternatively, the extended cDNAs may include only the sequence encoding the mature protein remaining after cleavage of the signal peptide, or only the sequence encoding the signal peptide.

Example 27 below describes a general method for obtaining extended cDNAs using 5' ESTs. Example 28 below provides experimental results, using the method explained in example 27, describing several extended cDNAs including the entire coding sequence and authentic 5' end of the corresponding mRNA for several secreted proteins.

The methods of Examples 27, 28, and 29 can also be used to obtain extended cDNAs which encode less than the entire coding sequence of the secreted proteins encoded by the genes corresponding to the 5' ESTs. In some embodiments, the extended cDNAs isolated using these methods encode at least 10 amino acids of one of the proteins encoded by the sequences of SEQ ID NOs: 38-305. In further embodiments, the extended cDNAs encode at least 20 amino acids of the proteins encoded by the sequences of SEQ ID NOs: 38-305. In further embodiments, the extended cDNAs encode at least 30 amino amino acids of the sequences of SEQ ID NOs: 38-305. In a preferred embodiment, the extended cDNAs encode a full length protein sequence, which includes the protein coding sequences of SEQ ID NOs: 38-305.

### EXAMPLE 27

### General Method for Using 5' ESTs to Clone and Sequence cDNAs which Include the Entire Coding Region and the Authentic 5' End of the Corresponding mRNA

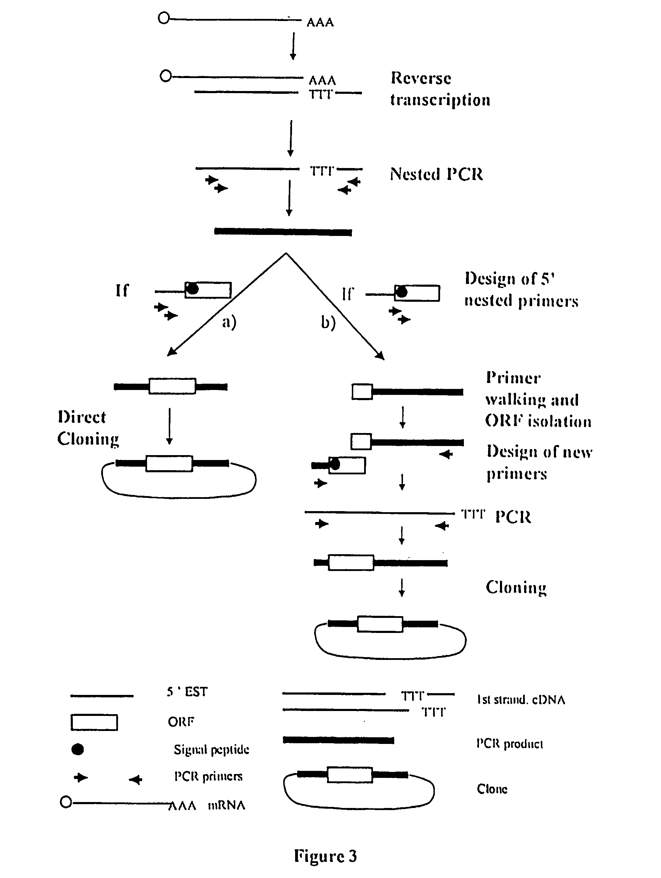

The following general method has been used to quickly and efficiently isolate extended cDNAs having the authentic 5' ends of their corresponding mRNAs as well as the full protein coding sequence and including sequence adjacent to the sequences of the 5' ESTs used to obtain them. This method may be applied to obtain extended cDNAs for any 5' EST in the NetGeneTM database, including those 5' ESTs encoding polypeptides belonging to secreted proteins. The method is summarized in figure 3.

### 1. Obtention of Extended cDNAs

### a) First strand synthesis

The method takes advantage of the known 5' sequence of the mRNA. A reverse transcription reaction is conducted on purified mRNA with a poly 14dT primer containing a 49 nucleotide sequence at its 5' end allowing the addition of a known sequence at the end of the cDNA which corresponds to the 3' end of the mRNA. For example, the primer may have the following sequence: 5'-ATC GTT GAG ACT CGT ACC AGC AGA GTC ACG AGA GAG ACT ACA CGG TAC TGG TTT TTT TTT TTT TTVN -3' (SEQ ID NO: 14). Those skilled in the art will appreciate that other sequences may also be added to the poly dT sequence and used to prime the first strand synthesis. Using this primer and a reverse transcriptase such as the Superscript II (Gibco BRL) or Rnase H Minus M-MLV (Promega) enzyme, a reverse transcript anchored at the 3' polyA site of the RNAs is generated.

After removal of the mRNA hybridized to the first cDNA strand by alkaline hydrolysis, the products of the alkaline hydrolysis and the residual poly dT primer are eliminated with an exclusion column such as an AcA34 (Biosepra) matrix as explained in Example 11.

### b) Second strand synthesis

A pair of nested primers on each end is designed based on the known 5' sequence from the 5' EST and the known 3' end added by the poly dT primer used in the first strand synthesis. Softwares used to design primers are either based on GC content and melting temperatures of oligonucleotides, such as OSP (Illier and Green, *PCR Meth. Appl.* **1**:124-128, 1991), or based on the octamer frequency disparity method (Griffais *et al., Nucleic Acids Res.* **19**: 3887-3891, 1991) such as PC-Rare (http://bioinformatics.weizmann.ac.il/software/PC-Rare/doc/manuel.html).

Preferably, the nested primers at the 5' end are separated from one another by four to nine bases. The 5' primer sequences may be selected to have melting temperatures and specificities suitable for use in PCR.

Preferably, the nested primers at the 3' end are separated from one another by four to nine bases. For example, the nested 3' primers may have the following sequences: (5'- CCA GCA GAG TCA CGA GAG AGA CTA CAC GG -3'(SEQ ID NO: 15), and 5'- CAC GAG AGA GAC TAC ACG GTA CTG G -3' (SEQ ID NO:16). These primers were selected because they have melting temperatures and specificities compatible with their use in PCR. However, those skilled in the art will appreciate that other sequences may also be used as primers.

The first PCR run of 25 cycles is performed using the Advantage Tth Polymerase Mix (Clontech) and the outer primer from each of the nested pairs. A second 20 cycle PCR using the same enzyme and the inner primer from each of the nested pairs is then performed on 1/2500 of the first PCR product. Thereafter, the primers and nucleotides are removed.

### 2. Sequencing of Full Length Extended cDNAs or Fragments Thereof

Due to the lack of position constraints on the design of 5' nested primers compatible for PCR use using the OSP software, amplicons of two types are obtained. Preferably, the second 5' primer is located upstream of the translation initiation codon thus yielding a nested PCR product containing the whole coding sequence. Such a full length extended cDNA undergoes a direct cloning procedure as described in section a. However, in some cases, the second 5' primer is located downstream of the translation initiation codon, thereby yielding a PCR product containing only part of the ORF. Such incomplete PCR products are submitted to a modified procedure described in section b.

### a) Nested PCR products containing complete ORFs

When the resulting nested PCR product contains the complete coding sequence, as predicted from the 5'EST sequence, it is cloned in an appropriate vector such as pED6dpc2, as described in section 3.

### b) Nested PCR products containing incomplete ORFS

When the amplicon does not contain the complete coding sequence, intermediate steps are necessary to obtain both the complete coding sequence and a PCR product containing the full coding sequence. The complete coding sequence can be assembled from several partial sequences determined directly from different PCR products as described in the following section.

Once the full coding sequence has been completely determined, new primers compatible for PCR use are designed to obtain amplicons containing the whole coding region. However, in such cases, 3' primers compatible for PCR use are located inside the 3' UTR of the corresponding mRNA, thus yielding amplicons which lack part of this region, *i.e.* the polyA tract and sometimes the polyadenylation signal, as illustrated in figure 3. Such full length extended cDNAs are then cloned into an appropriate vector as described in section 3.

### c) Sequencing extended cDNAs

Sequencing of extended cDNAs is performed using a Die Terminator approach with the AmpliTaq DNA polymerase FS kit available from Perkin Elmer.

In order to sequence PCR fragments, primer walking is performed using software such as OSP to choose primers and automated computer software such as ASMG (Sutton *et al., Genome Science Technol.* **1**: 9-19, 1995) to construct contigs of walking sequences including the initial 5' tag using minimum overlaps of 32 nucleotides. Preferably, primer walking is performed until the sequences of full length cDNAs are obtained.

Completion of the sequencing of a given extended cDNA fragment is assessed as follows. Since sequences located after a polyA tract are difficult to determine precisely in the case of uncloned products, sequencing and primer walking processes for PCR products are interrupted when a polyA tract is identified in extended cDNAs obtained as described in case b. The sequence length is compared to the size of the nested PCR product obtained as described above. Due to the limited accuracy of the determination of the PCR product size by gel electrophoresis, a sequence is considered complete if the size of the obtained sequence is at least 70 % the size of the first nested PCR product. If the length of the sequence determined from the computer analysis is not at least 70% of the length of the nested PCR product, these PCR products are cloned and the sequence of the insertion is determined. When Northern blot data are available, the size of the mRNA detected for a given PCR product is used to finally assess that the sequence is complete. Sequences which do not fulfill the above criteria are discarded and will undergo a new isolation procedure.

Sequence data of all extended cDNAs are then transferred to a proprietary database, where quality controls and validation steps are carried out as described in example 15.

### 3. Cloning of Full Length Extended cDNAs

The PCR product containing the full coding sequence is then cloned in an appropriate vector. For example, the extended cDNAs can be cloned into the expression vector pED6dpc2 (DiscoverEase, Genetics Institute, Cambridge, MA) as follows. pED6dpc2 vector DNA is prepared with blunt ends by performing an EcoRI digestion followed by a fill in reaction. The blunt ended vector is dephosphorylated. After removal of PCR primers and ethanol precipitation, the PCR product containing the full coding sequence or the extended cDNA obtained as described above is phosphorylated with a kinase subsequently removed by phenol-Sevag extraction and precipitation. The double stranded extended cDNA is then ligated to the vector and the resulting expression plasmid introduced into appropriate host cells.

Since the PCR products obtained as described above are blunt ended molecules that can be cloned in either direction, the orientation of several clones for each PCR product is determined. Then, 4 to 10 clones are ordered in microtiter plates and subjected to a PCR reaction using a first primer located in the vector close to the cloning site and a second primer located in the portion of the extended cDNA corresponding to the 3' end of the mRNA. This second primer may be the antisense primer used in anchored PCR in the case of direct cloning (case a) or the antisense primer located inside the 3'UTR in the case of indirect cloning (case b). Clones in which the start codon of the extended cDNA is operably linked to the promoter in the vector so as to permit expression of the protein encoded by the extended cDNA are conserved and sequenced. In addition to the ends of cDNA inserts, approximately 50 bp of vector DNA on each side of the cDNA insert are also sequenced.

The cloned PCR products are then entirely sequenced according to the aforementioned procedure. In this case, contigation of long fragments is then performed on walking sequences that have already contigated for uncloned PCR products during primer walking. Sequencing of cloned amplicons is complete when the resulting contigs include the whole coding region as well as overlapping sequences with vector DNA on both ends.

### 4. Computer analysis of Full Length Extended cDNA

Sequences of all full length extended cDNAs are then submitted to further analysis as described below. Before searching the extended full length cDNAs for sequences of interest, extended cDNAs which are not of interest (vector RNAs, transfer RNAs, ribosomal RNAs, mitochondrial RNAs, prokaryotic RNAs and fungal RNAs) are discarded using methods essentially similar to those described for 5'ESTs in Example 18.

### a) Identification of structural features

Structural features, e.g. polyA tail and polyadenylation signal, of the sequences of full length extended cDNAs are subsequently determined as follows.

A polyA tail is defined as a homopolymeric stretch of at least 11 A with at most one alternative base within it. The polyA tail search is restricted to the last 100 nt of the sequence and limited to stretches of 11 consecutive A's because sequencing reactions are often not readable after such a polyA stretch. Stretches having more than 90% homology over 8 nucleotides are identified as polyA tails using BLAST2N.

To search for a polyadenylation signal, the polyA tail is clipped from the full-length sequence. The 50 bp preceding the polyA tail are first searched for the canonic polyadenylation AAUAAA signal and, if the canonic signal is not detected, for the alternative AUUAAA signal (Sheets *et al., Nuc. Acids Res.* **18**: 5799-5805, 1990). If neither of these consensus polyadenylation signals is found, the canonic motif is searched again allowing one mismatch to account for possible sequencing errors. More than 85 % of identified polyadenylation signals of either type actually ends 10 to 30 bp from the polyA tail. Alternative AUUAAA signals represents approximately 15 % of the total number of identified polyadenylation signals.

### b) Identification of functional features

Functional features, e.g. ORFs and signal sequences, of the sequences of full length extended cDNAs were subsequently determined as follows.

The 3 upper strand frames of extended cDNAs are searched for ORFs defined as the maximum length fragments beginning with a translation intiation codon and ending with a stop codon. ORFs encoding at least 20 amino acids are preferred.

Each found ORF is then scanned for the presence of a signal peptide in the first 50 amino-acids or, where appropriate, within shorter regions down to 20 amino acids or less in the ORF, using the matrix method of von Heijne (*Nuc. Acids Res.* **14**: 4683-4690, 1986), the disclosure of which is incorporated herein by reference as described in Example 22.

### c) Homology to either nucleotidic or proteic sequences

Categorization of full-length sequences may be achieved using procedures essentially similar to those described for 5'ESTs in Example 24.

Extended cDNAs prepared as described above may be subsequently engineered to obtain nucleic acids which include desired portions of the extended cDNA using conventional techniques such as subcloning, PCR, or *in vitro* oligonucleotide synthesis. For example, nucleic acids which include only the full coding sequences *(i.e.* the sequences encoding the signal peptide and the mature protein remaining after the signal peptide is cleaved off may be obtained using techniques known to those skilled in the art. Alternatively, conventional techniques may be applied to obtain nucleic acids which contain only the coding sequences for the mature protein remaining after the signal peptide is cleaved off or nucleic acids which contain only the coding sequences for the signal peptides.

Similarly, nucleic acids containing any other desired portion of the coding sequences for the secreted protein may be obtained. For example, the nucleic acid may contain at least 10 consecutive bases of an extended cDNA such as one of the extended cDNAs described below. In another embodiment, the nucleic acid may contain at least 15 consecutive bases of an extended cDNA such as one of the extended cDNAs described below. Alternatively, the nucleic acid may contain at least 20 consecutive bases of an extended cDNA such as one of the extended cDNAs described below. In another embodiment, the nucleic acid may contain at least 25 consecutive bases of an extended cDNA such as one of the extended cDNAs described below. In yet another embodiment, the nucleic acid may contain at least 40 consecutive bases of an extended cDNA such as one of the extended cDNAs described below.

Once an extended cDNA has been obtained, it can be sequenced to determine the amino acid sequence it encodes. Once the encoded amino acid sequence has been determined, one can create and identify any of the many conceivable cDNAs that will encode that protein by simply using the degeneracy of the genetic code. For example, allelic variants or other homologous nucleic acids can be identified as described below. Alternatively, nucleic acids encoding the desired amino acid sequence can be synthesized *in vitro.*

In a preferred embodiment, the coding sequence may be selected using the known codon or codon pair preferences for the host organism in which the cDNA is to be expressed.

The extended cDNAs derived from the 5' ESTS of the present invention were obtained as described in Example 28 below.

### EXAMPLE 28

### Characterization of cloned extended cDNAs obtained using 5' ESTs

The procedure described in Example 27 above was used to obtain the extended cDNAs derived from the 5' ESTs of the present invention in a variety of tissues. The following list provides a few examples of thus obtained extended cDNAs.

Using this approach, the fall length cDNA of SEQ ID NO: 17 (internal identification number 48-19-3-G1-FL1) was obtained. This cDNA falls into the "EST-ext" category descn'bed above and encodes the signal peptide MKKVLLLITAILAVAVG (SEQ ID NO: 18) having a von Heijne score of 8.2.

The fall length cDNA of SEQ ID NO: 19 (internal identification number 58-34-2-E7-FL2) was also obtained using this procedure. This cDNA falls into the "EST-ext" category described above and encodes the signal peptide MWWFQQGLSFLPSALVIWTSA (SEQ ID NO:20) having a von Heijne score of 5.5.

Another full length cDNA obtained using the procedure described above has the sequence of SEQ ID NO:21 (internal identification number 51-27-1-E8-FL1). This cDNA, falls into the "EST-ext" category described above and encodes the signal peptide MVLTTLPSANSANSPVNMPTTGPNSLSYASSALSPCLT (SEQ ID NO:22) having a von Heijne score of 5.9.

The above procedure was also used to obtain a full length cDNA having the sequence of SEQ ID NO:23 (internal identification number 76-4-1-G5-FL1). This cDNA falls into the "EST-ext" category described above and encodes the signal peptide ILSTVTALTFAXA (SEQ ID NO:24) having a von Heijne score of 5.5.

The full length cDNA of SEQ ID NO:25 (internal identification number 51-3-3-B10-FL3) was also obtained using this procedure. This cDNA falls into the "new" category described above and encodes a signal peptide LVLTLCTLPLAVA (SEQ ID NO:26) having a von Heijne score of 10.1.

The full length cDNA of SEQ ID NO:27 (internal identification number 58-35-2-F10-FL2) was also obtained using this procedure. This cDNA falls into the "new" category described above and encodes a signal peptide LWLLFFLVTAIHA (SEQ ID NO:28) having a von Heijne score of 10.7.

Bacterial clones containing plasmids containing the full length cDNAs described above are presently stored in the inventor's laboratories under the internal identification numbers provided above. The inserts may be recovered from the stored materials by growing an aliquot of the appropriate bacterial clone in the appropriate medium. The plasmid DNA can then be isolated using plasmid isolation procedures familiar to those skilled in the art such as alkaline lysis minipreps or large scale alkaline lysis plasmid isolation procedures. If desired the plasmid DNA may be further enriched by centrifugation on a cesium chloride gradient, size exclusion chromatography, or anion exchange chromatography. The plasmid DNA obtained using these procedures may then be manipulated using standard cloning techniques familiar to those skilled in the art. Alternatively, a PCR can be done with primers designed at both ends of the cDNA insertion. The PCR product which corresponds to the cDNA can then be manipulated using standard cloning techniques familiar to those skilled in the art.

The polypeptides encoded by the extended cDNAs may be screened for the presence of known structural or functional motifs or for the presence of signatures, small amino acid sequences which are well conserved amongst the members of a protein family. The conserved regions have been used to derive consensus patterns or matrices included in the PROSITE data bank, in particular in the file prosite.dat (Release 13.0 of November 1995, located at http://expasy.hcuge.ch/sprot/prosite.html. Prosite_convert and prosite_scan programs (http://ulrec3.unil.ch/ftpserveur/prosite_scan) may be used to find signatures on the extended cDNAs.

For each pattern obtained with the prosite_convert program from the prosite.dat file, the accuracy of the detection on a new protein sequence may be assessed by evaluating the frequency of irrelevant hits on the population of human secreted proteins included in the data bank SWISSPROT. The ratio between the number of hits on shuffled proteins (with a window size of 20 amino acids) and the number of hits on native (unshuffled) proteins may be used as an index. Every pattern for which the ratio is greater than 20% (one hit on shuffled proteins for 5 hits on native proteins) may be skipped during the search with prosite_scan. The program used to shuffle protein sequences (db_shuffled) and the program used to determine the statistics for each pattern in the protein data banks (prosite_statistics) are available on the ftp site http://ulrec3.unil.ch/ftpserveur/prosite_scan.

In addition to PCR based methods for obtaining extended cDNAs, traditional hybridization based methods may also be employed. These methods may also be used to obtain the genomic DNAs which encode the mRNAs from which the 5' ESTs were derived, mRNAs corresponding to the extended cDNAs, or nucleic acids which are homologous to extended cDNAs or 5' ESTs. Example 29 below provides examples of such methods.

### EXAMPLE 29

### Methods for Obtaining cDNAs which include the Entire Coding Region and the Authentic 5'End ofthe Corresponding mRNA

A full length cDNA library can be made using the strategies described in Examples 13, 14, 15, and 16 above by replacing the random nonamer used in Example 14 with an oligo-dT primer. For instance, the oligonucleotide of SEQ ID NO: 14 may be used.

Alternatively, a cDNA library or genomic DNA library may be obtained from a commercial source or made using techniques familiar to those skilled in the art. Such cDNA or genomic DNA librairies may be used to isolate extended cDNAs obtained from 5' EST or nucleic acids homologous to extended cDNAs or 5' EST as follows. The cDNA library or genomic DNA library is hybridized to a detectable probe comprising at least 10 consecutive nucleotides from the 5' EST or extended cDNA using conventional techniques. Preferably, the probe comprises at least 12, 15, or 17 consecutive nucleotides from the 5' EST or extended cDNA More preferably, the probe comprises at least 20 to 30 consecutive nucleotides from the 5' EST or extended cDNA. In some embodiments, the probe comprises more than 30 nucleotides from the 5' EST or extended cDNA.

Techniques for identifying cDNA clones in a cDNA library which hybridize to a given probe sequence are disclosed in Sambrook *et al., Molecular Cloning: A Laboratory Manual 2d Ed.,* Cold Spring Harbor Laboratory Press, 1989, the disclosure of which is incorporated herein by reference. The same techniques may be used to isolate genomic DNAs.

Briefly, cDNA or genomic DNA clones which hybridize to the detectable probe are identified and isolated for further manipulation as follows. A probe comprising at least 10 consecutive nucleotides from the 5' EST or extended cDNA is labeled with a detectable label such as a radioisotope or a fluorescent molecule. Preferably, the probe comprises at least 12, 15, or 17 consecutive nucleotides from the 5' EST or extended cDNA. More preferably, the probe comprises 20 to 30 consecutive nucleotides from the 5' EST or extended cDNA. In some embodiments, the probe comprises more than 30 nucleotides from the 5' EST or extended cDNA.

Techniques for labeling the probe are well known and include phosphorylation with polynucleotide kinase, nick translation, *in vitro* transcription, and non radioactive techniques. The cDNAs or genomic DNAs in the library are transferred to a nitrocellulose or nylon filter and denatured. After blocking of non specific sites, the filter is incubated with the labeled probe for an amount of time sufficient to allow binding of the probe to cDNAs or genomic DNAs containing a sequence capable of hybridizing thereto.

By varying the stringency of the hybridization conditions used to identify extended cDNAs or genomic DNAs which hybridize to the detectable probe, extended cDNAS having different levels of homology to the probe can be identified and isolated as described below.

### 1. Identification of Extended cDNA or Genomic cDNA Sequences Having a High Degree of Homology to the Labeled Probe

To identify extended cDNAs or genomic DNAs having a high degree of homology to the probe sequence, the melting temperature of the probe may be calculated using the following formulas:

For probes between 14 and 70 nucleotides in length the melting temperature (Tm) is calculated using the formula: Tm=81.5+16.6(log [Na+])+0.41(fraction G+C)-(600/N) where N is the length of the probe.

If the hybridization is carried out in a solution containing formamide, the melting temperature may be calculated using the equation Tm=81.5+16.6(log [Na+))+0.41 (fraction G+C)-(0.63% formamide)-(600/N) where N is the length ofthe probe.

Prehybridization may be carried out in 6X SSC, 5X Denhardt's reagent, 0.5% SDS, 100 µg denatured fragmented salmon sperm DNA or 6X SSC, 5X Denhardt's reagent, 0.5% SDS, 100 µg denatured fragmented salmon sperm DNA, 50% formamide. The formulas for SSC and Denhardt's solutions are listed in Sambrook *et al., supra.*

Hybridization is conducted by adding the detectable probe to the prehybridization solutions listed above. Where the probe comprises double stranded DNA, it is denatured before addition to the hybridization solution. The filter is contacted with the hybridization solution for a sufficient period of time to allow the probe to hybridize to extended cDNAs or genomic DNAs containing sequences complementary thereto or homologous thereto. For probes over 200 nucleotides in length, the hybridization may be carried out at 15-25°C below the Tm. For shorter probes, such as oligonucleotide probes, the hybridization may be conducted at 15-25°C below the Tm. Preferably, for hybridizations in 6X SSC, the hybridization is conducted at approximately 68°C. Preferably, for hybridizations in 50% formamide containing solutions, the hybridization is conducted at approximately 42°C.

All of the foregoing hybridizations would be considered to be under "stringent" conditions.

Following hybridization, the filter is washed in 2X SSC, 0.1% SDS at room temperature for 15 minutes. The filter is then washed with 0.1X SSC, 0.5% SDS at room temperature for 30 minutes to 1 hour. Thereafter, the solution is washed at the hybridization temperature in 0.1X SSC, 0.5% SDS. A final wash is conducted in 0.1X SSC at room temperature.

Extended cDNAs, nucleic acids homologous to extended cDNAs or 5' ESTs, or genomic DNAs which have hybridized to the probe are identified by autoradiography or other conventional techniques.

### 2. Obtention of Extended cDNA or Genomic cDNA Sequences Having Lower Degrees of Homology to the Labeled Probe

The above procedure may be modified to identify extended cDNAs, nucleic acids homologous to extended cDNAs, or genomic DNAs having decreasing levels of homology to the probe sequence. For example, to obtain extended cDNAs, nucleic acids homologous to extended cDNAs, or genomic DNAs of decreasing homology to the detectable probe, less stringent conditions may be used. For example, the hybridization temperature may be decreased in increments of 5°C from 68°C to 42°C in a hybridization buffer having a sodium concentration of approximately 1M. Following hybridization, the filter may be washed with 2X SSC, 0.5% SDS at the temperature of hybridization. These conditions are considered to be "moderate" conditions above 50°C and "low" conditions below 50°C.

Alternatively, the hybridization may be carried out in buffers, such as 6X SSC, containing formamide at a temperature of 42°C. In this case, the concentration of formamide in the hybridization buffer may be reduced in 5% increments from 50% to 0% to identify clones having decreasing levels of homology to the probe. Following hybridization, the filter may be washed with 6X SSC, 0.5% SDS at 50°C. These conditions are considered to be "moderate" conditions above 25% formamide and "low" conditions below 25% formamide.

Extended cDNAs, nucleic acids homologous to extended cDNAs, or genomic DNAs which have hybridized to the probe are identified by autoradiography.

### 3. Determination of the Degree of Homology Between the Obtained Extended cDNAs and the Labeled Probe

If it is desired to obtain nucleic acids homologous to extended cDNAs, such as allelic variants thereof or nucleic acids encoding proteins related to the proteins encoded by the extended cDNAs, the level of homology between the hybridized nucleic acid and the extended cDNA or 5' EST used as the probe may be further determined using BLAST2N; parameters may be adapted depending on the sequence length and degree of homology studied. To determine the level of homology between the hybridized nucleic acid and the extended cDNA or 5'EST from which the probe was derived, the nucleotide sequences of the hybridized nucleic acid and the extended cDNA or 5'EST from which the probe was derived are compared. For example, using the above methods, nucleic acids having at least 95% nucleic acid homology to the extended cDNA or 5'EST from which the probe was derived may be obtained and identified. Similarly, by using progressively less stringent hybridization conditions one can obtain and identify nucleic acids having at least 90%, at least 85%, at least 80% or at least 75% homology to the extended cDNA or 5'EST from which the probe was derived.

To determine whether a clone encodes a protein having a given amount of homology to the protein encoded by the extended cDNA or 5' EST, the amino acid sequence encoded by the extended cDNA or 5' EST is compared to the amino acid sequence encoded by the hybridizing nucleic acid. Homology is determined to exist when an amino acid sequence in the extended cDNA or 5' EST is closely related to an amino acid sequence in the hybridizing nucleic acid. A sequence is closely related when it is identical to that of the extended cDNA or 5' EST or when it contains one or more amino acid substitutions therein in which amino acids having similar characteristics have been substituted for one another. Using the above methods and algorithms such as FASTA with parameters depending on the sequence length and degree of homology studied, one can obtain nucleic acids encoding proteins having at least 95%, at least 90%, at least 85%, at least 80% or at least 75% homology to the proteins encoded by the extended cDNA or 5'EST from which the probe was derived.

In addition to the above described methods, other protocols are available to obtain extended cDNAs using 5' ESTs as outlined in the following paragraphs.

Extended cDNAs may be prepared by obtaining mRNA from the tissue, cell, or organism of interest using mRNA preparation procedures utilizing polyA selection procedures or other techniques known to those skilled in the art. A first primer capable of hybridizing to the polyA tail of the mRNA is hybridized to the mRNA and a reverse transcription reaction is performed to generate a first cDNA strand.

The first cDNA strand is hybridized to a second primer containing at least 10 consecutive nucleotides of the sequences of SEQ ID NOs 38-305. Preferably, the primer comprises at least 12, 15, or 17 consecutive nucleotides from the sequences of SEQ ID NOs 38-305. More preferably, the primer comprises 20 to 30 consecutive nucleotides from the sequences of SEQ ID NOs 38-305. In some embodiments, the primer comprises more than 30 nucleotides from the sequences of SEQ ID NOs 38-305. If it is desired to obtain extended cDNAs containing the full protein coding sequence, including the authentic translation initiation site, the second primer used contains sequences located upstream of the translation initiation site. The second primer is extended to generate a second cDNA strand complementary to the first cDNA strand. Alternatively, RT-PCR may be performed as described above using primers from both ends of the cDNA to be obtained.

Extended cDNAs containing 5' fragments of the mRNA may be prepared by hybridizing an mRNA comprising the sequence of the 5'EST for which an extended cDNA is desired with a primer comprising at least 10 consecutive nucleotides of the sequences complementary to the 5'EST and reverse transcribing the hybridized primer to make a first cDNA strand from the mRNAs. Preferably, the primer comprises at least 12, 15, or 17 consecutive nucleotides from the 5'EST. More preferably, the primer comprises 20 to 30 consecutive nucleotides from the 5'EST.

Thereafter, a second cDNA strand complementary to the first cDNA strand is synthesized. The second cDNA strand may be made by hybridizing a primer complementary to sequences in the first cDNA strand to the first cDNA strand and extending the primer to generate the second cDNA strand.

The double stranded extended cDNAs made using the methods described above are isolated and cloned. The extended cDNAs may be cloned into vectors such as plasmids or viral vectors capable of replicating in an appropriate host cell. For example, the host cell may be a bacterial, mammalian, avian, or insect cell.

Techniques for isolating mRNA, reverse transcribing a primer hybridized to mRNA to generate a first cDNA strand, extending a primer to make a second cDNA strand complementary to the first cDNA strand, isolating the double stranded cDNA and cloning the double stranded cDNA are well known to those skilled in the art and are described *in Current Protocols in Molecular Biology,* John Wiley and Sons, Inc. 1997 and Sambrook *et al., Molecular Cloning: A Laboratory Manual,* Second Edition, Cold Spring Harbor Laboratory Press, 1989, the entire disclosures of which are incorporated herein by reference.

Alternatively, procedures such as the one described in Example 29 may be used for obtaining full length cDNAs or extended cDNAs. In this approach, full length or extended cDNAs are prepared from mRNA and cloned into double stranded phagemids as follows. The cDNA library in the double stranded phagemids is then rendered single stranded by treatment.with an endonuclease, such as the Gene II product of the phage F1, and an exonuclease (Chang *et al., Gene* **127**:95-8*,* 1993). A biotinylated oligonucleotide comprising the sequence of a 5' EST, or a fragment containing at least 10 nucleotides thereof, is hybridized to the single stranded phagemids. Preferably, the fragment comprises at least 12, 15, or 17 consecutive nucleotides from the 5' EST. More preferably, the fragment comprises 20-30 consecutive nucleotides from the 5' EST. In some procedures, the fragment may comprise more than 30 consecutive nucleotides from the 5' EST.

Hybrids between the biotinylated oligonucleotide and phagemids having inserts containing the 5' EST sequence are isolated by incubating the hybrids with streptavidin coated paramagnetic beads and retrieving the beads with a magnet (Fry *et al., Biotechniques,* **13**: 124-131, 1992). Therafter, the resulting phagemids containing the 5' EST sequence are released from the beads and converted into double stranded DNA using a primer specific for the 5' EST sequence. Alternatively, protocoles such as the Gene Trapper kit (Gibco BRL) may be used. The resulting double stranded DNA is transformed into bacteria. Extended cDNAs containing the 5' EST sequence are identified by colony PCR or colony hybridization.

Using any of the above described methods in section III, a plurality of extended cDNAs containing full length protein coding sequences or sequences encoding only the mature protein remaining after the signal peptide is cleaved off may be provided as cDNA libraries for subsequent evaluation of the encoded proteins or use in diagnostic assays as described below.

### IV. Expression of Proteins Encoded by Extended cDNAs Isolated Using 5' ESTs

Extended cDNAs containing the full protein coding sequences of their corresponding mRNAs or portions thereof, such as cDNAs encoding the mature protein, may be used to express the encoded secreted proteins or portions thereof as described in Example 30 below. If desired, the extended cDNAs may contain the sequences encoding the signal peptide to facilitate secretion of the expressed protein. It will be appreciated that a plurality of extended cDNAs containing the fall protein coding sequences or portions thereof may be simultaneously cloned into expression vectors to create an expression library for analysis of the encoded proteins as described below.

### EXAMPLE 30

### Expression of the Proteins Encoded by the Genes Corresponding to 5'ESTS or Portions Thereof

To express the proteins encoded by the genes corresponding to 5' ESTs (or portions thereof), full length cDNAs containing the entire protein coding region or extended cDNAs containing sequences adjacent to the 5' ESTs (or portions thereof) are obtained as described in Examples 27-29 and cloned into a suitable expression vector. If desired, the nucleic acids may contain the sequences encoding the signal peptide to facilitate secretion of the expressed protein. The nucleic acids inserted into the expression vectors may also contain sequences upstream of the sequences encoding the signal peptide, such as sequences which regulate expression levels or sequences which confer tissue specific expression.

The nucleic acid encoding the protein or polypeptide to be expressed is operably linked to a promoter in an expression vector using conventional cloning technology. The expression vector may be any of the mammalian, yeast, insect or bacterial expression systems known in the art. Commercially available vectors and expression systems are available from a variety of suppliers including Genetics Institute (Cambridge, MA), Stratagene (La Jolla, California), Promega (Madison, Wisconsin), and Invitrogen (San Diego, California). If desired, to enhance expression and facilitate proper protein folding, the codon context and codon pairing of the sequence may be optimized for the particular expression organism in which the expression vector is introduced, as explained by Hatfield, *et al.,* U.S. Patent No. 5,082,767, incorporated herein by this reference.

The cDNA cloned into the expression vector may encode the entire protein *(i.e.* the signal peptide and the mature protein), the mature protein *(i.e.* the protein created by cleaving the signal peptide off), only the signal peptide or any other portion thereof.

The following is provided as one exemplary method to express the proteins encoded by the extended cDNAs corresponding to the 5' ESTs or the nucleic acids described above. First, the methionine initiation codon for the gene and the polyA signal of the gene are identified. If the nucleic acid encoding the polypeptide to be expressed lacks a methionine to serve as the initiation site, an initiating methionine can be introduced next to the first codon of the nucleic acid using conventional techniques. Similarly, if the extended cDNA lacks a polyA signal, this sequence can be added to the construct by, for example, splicing out the polyA signal from pSG5 (Stratagene) using BglII and SalI restriction endonuclease enzymes and incorporating it into the mammalian expression vector pXT1 (Stratagene). pXT1 contains the LTRs and a portion of the *gag* gene from Moloney Murine Leukemia Virus. The position of the LTRs in the construct allow efficient stable transfection. The vector includes the Herpes Simplex thymidine kinase promoter and the selectable neomycin gene. The extended cDNA or portion thereof encoding the polypeptide to be expressed is obtained by PCR from the bacterial vector using oligonucleotide primers complementary to the extended cDNA or portion thereof and containing restriction endonuclease sequences for Pst I incorporated into the 5'primer and BglII at the 5' end of the corresponding cDNA 3' primer, taking care to ensure that the extended cDNA is positioned with the poly A signal. The purified fragment obtained from the resulting PCR reaction is digested with PstI, blunt ended with an exonuclease, digested with Bgl II, purified and ligated to pXT1 containing a poly A signal and prepared for this ligation (blunt/BglII).

The ligated product is transfected into mouse NIH 3T3 cells using Lipofectin (Life Technologies, Inc., Grand Island, New York) under conditions outlined in the product specification. Positive transfectants are selected after growing the transfected cells in 600 µg/ml G418 (Sigma, St. Louis, Missouri). Preferably the expressed protein is released into the culture medium, thereby facilitating purification.

Alternatively, the extended cDNAs may be cloned into pED6dpc2 as described above. The resulting pED6dpc2 constructs may be transfected into a suitable host cell, such as COS 1 cells. Methotrexate resistant cells are selected and expanded. Preferably, the protein expressed from the extended cDNA is released into the culture medium thereby facilitating purification.

Proteins in the culture medium are separated by gel electrophoresis. If desired, the proteins may be ammonium sulfate precipitated or separated based on size or charge prior to electrophoresis.

As a control, the expression vector lacking a cDNA insert is introduced into host cells or organisms and the proteins in the medium are harvested. The secreted proteins present in the medium are detected using techniques familiar to those skilled in the art such as Coomassie blue or silver staining or using antibodies against the protein encoded by the extended cDNA.

Antibodies capable of specifically recognizing the protein of interest may be generated using synthetic 15-mer peptides having a sequence encoded by the appropriate 5' EST, extended cDNA, or portion thereof. The synthetic peptides are injected into mice to generate antibody to the polypeptide encoded by the 5' EST, extended cDNA, or portion thereof.

Secreted proteins from the host cells or organisms containing an expression vector which contains the extended cDNA derived from a 5' EST or a portion thereof are compared to those from the control cells or organism. The presence of a band in the medium from the cells containing the expression vector which is absent in the medium from the control cells indicates that the extended cDNA encodes a secreted protein. Generally, the band corresponding to the protein encoded by the extended cDNA will have a mobility near that expected based on the number of amino acids in the open reading frame of the extended cDNA. However, the band may have a mobility different than that expected as a result of modifications such as glycosylation, ubiquitination, or enzymatic cleavage.

Alternatively, if the protein expressed from the above expression vectors does not contain sequences directing its secretion, the proteins expressed from host cells containing an expression vector with an insert encoding a secreted protein or portion thereof can be compared to the proteins expressed in control host cells containing the expression vector without an insert. The presence of a band in samples from cells containing the expression vector with an insert which is absent in samples from cells containing the expression vector without an insert indicates that the desired protein or portion thereof is being expressed. Generally, the band will have the mobility expected for the secreted protein or portion thereof. However, the band may have a mobility different than that expected as a result of modifications such as glycosylation, ubiquitination, or enzymatic cleavage.

The protein encoded by the extended cDNA may be purified using standard immunochromatography techniques. In such procedures, a solution containing the secreted protein, such as the culture medium or a cell extract, is applied to a column having antibodies against the secreted protein attached to the chromatography matrix. The secreted protein is allowed to bind the immunochromatography column. Thereafter, the column is washed to remove non-specifically bound proteins. The specifically bound secreted protein is then released from the column and recovered using standard techniques.

If antibody production is not possible, the extended cDNA sequence or portion thereof may be incorporated into expression vectors designed for use in purification schemes employing chimeric polypeptides. In such strategies, the coding sequence of the extended cDNA or portion thereof is inserted in frame with the gene encoding the other half of the chimera. The other half of the chimera may be β-globin or a nickel binding polypeptide. A chromatography matrix having antibody to β-globin or nickel attached thereto is then used to purify the chimeric protein. Protease cleavage sites may be engineered between the β-globin gene or the nickel binding polypeptide and the extended cDNA or portion thereof. Thus, the two polypeptides of the chimera may be separated from one another by protease digestion.

One useful expression vector for generating β-globin chimerics is pSG5 (Stratagene), which encodes rabbit β-globin. Intron II of the rabbit β-globin gene facilitates splicing of the expressed transcript, and the polyadenylation signal incorporated into the construct increases the level of expression. These techniques as described are well known to those skilled in the art of molecular biology. Standard methods are published in methods texts such as Davis *et al.*, (*Basic Methods in Molecular Biology,* Davis, Dibner, and Battey, ed., Elsevier Press, NY, 1986) and many of the methods are available from Stratagene, Life Technologies, Inc., or Promega. Polypeptide may additionally be produced from the construct using *in vitro* translation systems such as the *In vitro* Express™ Translation Kit (Stratagene).

Following expression and purification of the secreted proteins encoded by the 5' ESTs, extended cDNAs, or fragments thereof; the purified proteins may be tested for the ability to bind to the surface of various cell types as described in Example 31 below. It will be appreciated that a plurality of proteins expressed from these cDNAs may be included in a panel of proteins to be simultaneously evaluated for the activities specifically described below, as well as other biological roles for which assays for determining activity are available.

### EXAMPLE 31

### Analysis of Secreted Proteins to Determine Whether they Bind to the Cell Surface

The proteins encoded by the 5' ESTs, extended cDNAs, or fragments thereof are cloned into expression vectors such as those described in Example 30. The proteins are purified by size, charge, immunochromatography or other techniques familiar to those skilled in the art. Following purification, the proteins are labeled using techniques known to those skilled in the art. The labeled proteins are incubated with cells or cell lines derived from a variety of organs or tissues to allow the proteins to bind to any receptor present on the cell surface. Following the incubation, the cells are washed to remove non-specifically bound protein. The labeled proteins are detected by autoradiography. Alternatively, unlabeled proteins may be incubated with the cells and detected with antibodies having a detectable label, such as a fluorescent molecule, attached thereto.

Specificity of cell surface binding may be analyzed by conducting a competition analysis in which various amounts of unlabeled protein are incubated along with the labeled protein. The amount of labeled protein bound to the cell surface decreases as the amount of competitive unlabeled protein increases. As a control, various amounts of an unlabeled protein unrelated to the labeled protein is included in some binding reactions. The amount of labeled protein bound to the cell surface does not decrease in binding reactions containing increasing amounts of unrelated unlabeled protein, indicating that the protein encoded by the cDNA binds specifically to the cel surface.

As discussed above, secreted proteins have been shown to have a number of important physiological effects and, consequently, represent a valuable therapeutic resource. The secreted proteins encoded by the extended cDNAs or portions thereof made according to Examples 27-29 may be evaluated to determine their physiological activities as described below.

### EXAMPLE 32

### Assaying the Proteins Expressed from Extended cDNAs or Portions Thereof for Cytoldne, Cell Proliferation or Cell Differentiation Activity

As discussed above, secreted proteins may act as cytokines or may affect cellular proliferation or differentiation. Many protein factors discovered to date, including all known cytoldnes, have exhibited activity in one or more factor dependent cell proliferation assays, and hence the assays serve as a convenient confirmation of cytokine activity. The activity of a protein encoded by the extended cDNAs is evidenced by any one of a number of routine factor dependent cell proliferation assays for cell lines including, without limitation, 32D, DA2, DA1G, T10, B9, B9/11, BaF3, MC9/G, M⁺ (preB M⁺), 2E8, RB5, DA1, 123, T1165, HT2, CTLL2, TF-1, Mo7c and CMK. The proteins encoded by the above extended cDNAs or portions thereof may be evaluated for their ability to regulate T cell or thymocyte proliferation in assays such as those described above or in the following references, which are incorporated herein by reference: *Current Protocols in Immunology,* Ed. by Coligan *et al.*, Greene Publishing Associates and Wiley-Interscience; Takai *et al. J Immunol.* **137**:3494-3500, 1986., Bertagnolli *et al., J. Immunol.* **145**:1706-1712, 1990., Bertagnolli *et al.. Cell. Immunol.* **133**:327-341, 1991; Bertagnolli, *et al., J. Immunol.* **149**:3778-3783, 1992; Bowman *et al., J. Immunol* **152**:1756-1761,1994.

In addition, numerous assays for cytokine production and/or the proliferation of spleen cells, lymph node cells and thymocytes are known. These include the techniques disclosed in *Current Protocols in Immunology, supra* **1**:3.12.1-3.12.14; and Schreiber In *Current Protocols in Immunology, supra* **1**: 6.8.1-6.8.8.

The proteins encoded by the cDNAs may also be assayed for the ability to regulate the proliferation and differentiation of hematopoietic or lymphopoietic cells. Many assays for such activity are familiar to those skilled in the art, including the assays in the following references, which are incorporated herein by reference: Bottomly *et al.,* In *Current Protocols in Immunology., supra.* **1** : 6.3.1-6.3.12,; deVries *et al., J. Exp. Med* **173**:1205-1211, 1991; Moreau *et al.. Nature* **36**:690-692, 1988; Greenberger *et al., Proc. Natl. Acad. Sci. U.S.A.* **80**:2931-2938, 1983; Nordan, R., In *Current Protocols in Immunology., supra.* **1** : 6.6.1-6.6.5; Smith et al., *Proc. Natl. Acad. Sci. U.S.A.* **83**:1857-1861. 1986; Bennett *et al.,* in *Current Protocols in Immunology supra* **1** : 6.15.1; Ciarletta *et al..* In *Current Protocols in Immunology, supra* **1**:6.13.1.

The proteins encoded by the cDNAs may also be assayed for their ability to regulate T-cell responses to antigens. Many assays for such activity are familiar to those skilled in the art, including the assays described in the following references, which are incorporated herein by reference: Chapter 3 (*In Vitro* Assays for Mouse Lymphocyte Function), Chapter 6 (Cytokines and Their Cellular Receptors) and Chapter 7, (Immunologic Studies in Humans) in *Current Protocols in Immunology supra;* Weinberger *et al., Proc. Natl. Acad. Sci. USA* **77**:6091-6095, 1980; Weinberger *et al., Eur. J. Immun.* **11**:405-411, 1981; Takai *et al., J. Immunol.* **137**:3494-3500, 1986; Takai *et al., J. Immunol.* **140**:508-512_{,} 1988.

Those proteins which exhibit cytokine, cell proliferation, or cell differentiation activity may then be formulated as pharmaceuticals and used to treat clinical conditions in which induction of cell proliferation or differentiation is beneficial. Alternatively, as described in more detail below, genes encoding these proteins or nucleic acids regulating the expression of these proteins may be introduced into appropriate host cells to increase or decrease the expression of the proteins as desired.

### EXAMPLE 33

### Assaying the Proteins Expressed from Extended cDNAs or Portions Thereof for Activity as immure System Regulators

The proteins encoded by the cDNAs may also be evaluated for their effects as immune regulators. For example, the proteins may be evaluated for their activity to influence thymocyte or splenocyte cytotoxicity. Numerous assays for such activity are familiar to those skilled in the art including the assays described in the following references, which are incorporated herein by reference: Chapter 3 (*In Vitro* Assays for Mouse Lymphocyte Function 3.1-3.19) and Chapter 7 (Immunologic studies in Humans) in *Current Protocols in Immunology ,* Coligan *et al.,* Eds, Greene Publishing Associates and Wiley-Interscience; Herrmann *et al., Proc. Natl*. *Acad. Sci. USA* **78**:2488-2492, 1981; Herrmann *et al., J. Immunol.* **128**:1968-1974, 1982; Handa *et al., J. Immunol.* **135**:1564-1572, 1985; Takai *et al., J. Immunol.* **137**:3494-3500, 1986; Takai *et al., J. Immunol.* **140**:508-512, 1988; Bowman *et al., J. Virology* **61**:1992-1998; Bertagnolli *et al.*, *Cell. Immunol.* **133**:327-341, 1991; Brown *et al., J. Immunol.* **153**:3079-3092, 1994.

The proteins encoded by the cDNAs may also be evaluated for their effects on T-cell dependent immunoglobulin responses and isotype switching. Numerous assays for such activity are familiar to those skilled in the art, including the assays disclosed in the following references, which are incorporated herein by reference: Maliszewski, *J. Immunol.* **144**:3028-3033, 1990; Mond *et al.* in *Current Protocols in Immunology,* **1** : 3.8.1-3.8.16, *supra.*

The proteins encoded by the cDNAs may also be evaluated for their effect on immune effector cells, including their effect on Thl cells and cytotoxic lymphocytes. Numerous assays for such activity are familiar to those skilled in the art, including the assays disclosed in the following references, which are incorporated herein by reference: Chapter 3 (*In Vitro* Assays for Mouse Lymphocyte Function 3.1-3.19) and Chapter 7 (Immunologic Studies in Humans) in *Current Protocols in Immunology, supra;* Takai *et al., J. Immunol.* **137**:3494-3500, 1986; Takai *et al., J. Immunol.* **140**:508-512, 1988; Bertagnolli *et al., J. Immunol.* **149**:3778-3783, 1992.

The proteins encoded by the cDNAs may also be evaluated for their effect on dendritic cell mediated activation of naive T-cells. Numerous assays for such activity are familiar to those skilled in the art, including the assays disclosed in the following references, which are incorporated herein by reference: Guery *et al., J. Immunol.* **134**:536-544, 1995; Inaba *et al., J. Exp. Med* **173**:549-559, 1991; Macatonia *et al*., *J. Immunol.* **154**:5071-5079, 1995; Porgador *et al.J Exp*. *Med* **182**:255-260, 1995; Nair *et al., J. Virol.* **67**:4062-4069, 1993; Huang *et al., Science* **264**:961-965, 1994; Macatonia *et al.J. Exp. Med* **169**:1255-1264, 1989; Bhardwaj *et al., Journal of Clinical Investigation* **94**:797-807, 1994; and Inaba *et al., J. Exp. Med* **172**:631-640, 1990.

The proteins encoded by the cDNAs may also be evaluated for their influence on the lifetime of lymphocytes. Numerous assays for such activity are familiar to those skilled in the art, including the assays disclosed in the following references, which are incorporated herein by reference: Darzynkiewicz *et al., Cytometry* **13**:795-808, 1992; Gorczyca *et al., Leukemia* **7**:659-670, 1993; *Gorczyca et al., Cancer Res.* **53**:1945-1951, 1993; Itoh *et al., Cell* **66**:233-243, 1991; Zacharchuk, *J*. *Immunol.* **145**:4037-4045, 1990; Zamai *et al., Cytometry* **14**:891-897, 1993; Gorczyca *et al., Int. J. Oncol.* **1**:639-648, 1992.

The proteins encoded by the cDNAs may also be evaluated for their influence on early steps of T-cell commitment and development. Numerous assays for such activity are familiar to those skilled in the art, including without limitation the assays disclosed in the following references, which are incorporated herein by references: Antica *et al., Blood* **84**:111-117, 1994; Fine *et al.. Cell. Immunol.* **155**:111-122, 1994; Galy *et al.. Blood* **85**:2770-2778_{,} 1995; Toki *et al., Proc. Nat. Acad Sci. USA* **88**:7548-7551, 1991

Those proteins which exhibit activity as immune system regulators activity may then be formulated as pharmaceuticals and used to treat clinical conditions in which regulation of immune activity is beneficial. For example, the protein may be useful in the treatment of various immune deficiencies and disorders (including severe combined immunodeficiency), e.g., in regulating (up or down) growth and proliferation of T and/or B lymphocytes, as well as effecting the cytolytic activity of NK cells and other cell populations. These immune deficiencies may be genetic or be caused by viral (e.g., HIV) as well as bacterial or fungal infections, or may result from autoimmune disorders. More specifically, infectious diseases caused by viral, bacterial, fungal or other infection may be treatable using a protein encoded by extended cDNAs derived from the 5' ESTs of the present invention, including infections by HIV, hepatitis viruses, herpesviruses, mycobacteria, Leishmania spp., plamodium and various fungal infections such as candidiasis. Of course, in this regard, a protein encoded by extended cDNAs derived from the 5' ESTs of the present invention may also be useful where a boost to the immune system generally may be desirable, *i.e.,* in the treatment of cancer.

Alternatively, proteins encoded by extended cDNAs derived from the 5' ESTs of the present invention may be used in treatment of autoimmune disorders including, for example, connective tissue disease, multiple sclerosis, systemic lupus erythematosus, rheumatoid arthritis, autoimmune pulmonary inflammation, Guillain-Barre syndrome, autoimmune thyroiditis, insulin dependent diabetes mellitis, myasthenia gravis, graft-versus-host disease and autoimmune inflammatory eye disease. Such a protein encoded by extended cDNAs derived from the 5' ESTs of the present invention may also to be useful in the treatment of allergic reactions and conditions, such as asthma (particularly allergic asthma) or other respiratory problems. Other conditions, in which immune suppression is desired (including, for example, organ transplantation), may also be treatable using a protein encoded by extended cDNAs derived from the 5' ESTs of the present invention.

Using the proteins of the invention it may also be possible to regulate immune responses either up or down.

Down regulation may involve inhibiting or blocking an immune response already in progress or may involve preventing the induction of an immune response. The functions of activated T-cells may be inhibited by suppressing T cell responses or by inducing specific tolerance in T cells, or both. Immunosuppression of T cell responses is generally an active non-antigen-specific process which requires continuous exposure of the T cells to the suppressive agent. Tolerance, which involves inducing non-responsiveness or anergy in T cells, is distinguishable from immunosuppression in that it is generally antigen-specific and persists after the end of exposure to the tolerizing agent. Operationally, tolerance can be demonstrated by the lack of a T cell response upon reexposure to specific antigen in the absence of the tolerizing agent.

Down regulating or preventing one or more antigen functions (including without limitation B lymphocyte antigen functions, such as, for example, B7 costimulation), e.g., preventing high level lymphokine synthesis by activated T cells, will be useful in situations of tissue, skin and organ transplantation and in graft-versus-host disease (GVHD). For example, blockage of T cell function should result in reduced tissue destruction in tissue transplantation. Typically, in tissue transplants, rejection of the transplant is initiated through its recognition as foreign by T cells, followed by an immune reaction that destroys the transplant. The administration of a molecule which inhibits or blocks interaction of a B7 lymphocyte antigen with its natural ligand(s) on immune cells (such as a soluble, monomeric form of a peptide having B7-2 activity alone or in conjunction with a monomeric form of a peptide having an activity of another B lymphocyte antigen (e.g., B7-1, B7-3) or blocking antibody), prior to transplantation, can lead to the binding of the molecule to the natural ligand(s) on the immune cells without transmitting the corresponding costimulatory signal. Blocking B lymphocyte antigen function in this matter prevents cytokine synthesis by immune cells, such as T cells, and thus acts as an immunosuppressant. Moreover, the lack of costimulation may also be sufficient to anergize the T cells, thereby inducing tolerance in a subject. Induction of long-term tolerance by B lymphocyte antigen-blocking reagents may avoid the necessity of repeated administration of these blocking reagents. To achieve sufficient immunosuppression or tolerance in a subject, it may also be necessary to block the function of a combination of B lymphocyte antigens.

The efficacy of particular blocking reagents in preventing organ transplant rejection or GVHD can be assessed using animal models that are predictive of efficacy in humans. Examples of appropriate systems which can be used include allogeneic cardiac grafts in rats and xenogeneic pancreatic islet cell grafts in mice, both of which have been used to examine the immunosuppressive effects of CTLA4Ig fusion proteins *in vivo* as described in Lenschow *et al.*, *Science* **257**:789-792, 1992 and Turka *et al., Proc. Natl. Acad. Sci USA,* **89**:11102-11105, 1992. In addition, murine models of GVHD (see Paul ed., *Fundamental Immunology,* Raven Press, New York, 1989, pp. 846-847) can be used to determine the effect of blocking B lymphocyte antigen function *in vivo* on the development of that disease.

Blocking antigen function may also be therapeutically useful for treating autoimmune diseases. Many autoimmuno disorders are the result of inappropriate activation of T cells that are reactive against self tissue and which promote the production of cytokines and autoantibodies involved in the pathology of the diseases. Preventing the activation of autoreactive T cells may reduce or eliminate disease symptoms. Administration of reagents which block costimulation of T cells by disrupting receptor/ligand interactions of B lymphocyte antigens can be used to inhibit T cell activation and prevent production of autoantibodies or T cell-derived cytokines which potentially involved in the disease process. Additionally, blocking reagents may induce antigen-specific tolerance of autoreactive T cells which could lead to long-term relief from the disease. The efficacy of blocking reagents in preventing or alleviating autoimmune disorders can be determined using a number of well-characterized animal models of human autoimmune diseases. Examples include murine experimental autoimmune encephalitis, systemic lupus erythmatosis in MRL/pr/pr mice or NZB hybrid mice, murine autoimmuno collagen arthritis, diabetes mellitus in OD mice and BB rats, and murine experimental myasthenia gravis (see Paul ed., *supra,* pp. 840-856).

Upregulation of an antigen function (preferably a B lymphocyte antigen function), as a means of up regulating immune responses, may also be useful in therapy. Upregulation of immune responses may involve either enhancing an existing immune response or eliciting an initial immune response as shown by the following examples. For instance, enhancing an immune response through stimulating B lymphocyte antigen function may be useful in cases of viral infection. In addition, systemic viral diseases such as influenza, the common cold, and encephalitis might be alleviated by the administration of stimulatory form of B lymphocyte antigens systemically.

Alternatively, antiviral immune responses may be enhanced in an infected patient by removing T cells from the patient, costimulating the T cells *in vitro* with viral antigen-pulsed APCs either expressing a peptide encoded by extended cDNAs derived from the 5' ESTs of the present invention or together with a stimulatory form of a soluble peptide encoded by extended cDNAs derived from the 5' ESTs of the present invention and reintroducing the *in vitro* primed T cells into the patient. The infected cells would now be capable of delivering a costimulatory signal to T cells *in vivo,* thereby activating the T cells.

In another application, upregulation or enhancement of antigen function (preferably B lymphocyte antigen function) may be useful in the induction of tumor immunity. Tumor cells (e.g., sarcoma, melanoma, lymphoma, leukemia, neuroblastoma, carcinoma) transfected with a nucleic acid encoding at least one peptide encoded by extended cDNAs derived from the 5' ESTs of the present invention can be administered to a subject to overcome tumor-specific tolerance in the subject. If desired, the tumor cell can be transfected to express a combination of peptides. For example, tumor cells obtained from a patient can be transfected *ex vivo* with an expression vector directing the expression of a peptide having B7-2-like activity alone, or in conjunction with a peptide having B7-1-like activity and/or B7-3-like activity. The transfected tumor cells are returned to the patient to result in expression of the peptides on the surface of the transfected cell. Alternatively, gene therapy techniques can be used to target a tumor cell for transfection *in vivo.*

The presence of the peptide encoded by extended cDNAs derived from the 5' ESTs of the present invention having the activity of a B lymphocyte antigen(s) on the surface of the tumor cell provides the necessary costimulation signal to T cells to induce a T cell mediated immune response against the transfected tumor cells. In addition, tumor cells which lack or which fail to reexpress sufficient amounts of MHC class I or MHC class II molecules can be transfected with nucleic acids encoding all or a portion of (e.g., a cytoplasmic-domain truncated portion) of an MHC class I α chain and **β**₂ microglobulin or an MHC class II α chain and an MHC class II β chain to thereby express MHC class I or MHC class II proteins on the cell surface, respectively. Expression of the appropriate MHC class I or class II molecules in conjunction with a peptide having the activity of a B lymphocyte antigen (e.g., B7-1, B7-2, B7-3) induces a T cell mediated immune response against the transfected tumor cell. Optionally, a gene encoding an antisense construct which blocks expression of an MHC class II associated protein, such as the invariant chain, can also be cotransfected with a DNA encoding a peptide having the activity of a B lymphocyte antigen to promote presentation of tumor associated antigens and induce tumor specific immunity. Thus, the induction of a T cell mediated immune response in a human subject may be sufficient to overcome tumor-specific tolerance in the subject. Alternatively, as described in more detail below, genes encoding these immune system regulator proteins or nucleic acids regulating the expression of such proteins may be introduced into appropriate host cells to increase or decrease the expression of the proteins as desired.

### EXAMPLE 34

### Assaying the Proteins Expressed from Extended cDNAs or Portions Thereof for Hematopoiesis Regulating Activity

The proteins encoded by the extended cDNAs or portions thereof may also be evaluated for their hematopoiesis regulating activity. For example, the effect of the proteins on embryonic stem cell differentiation may be evaluated. Numerous assays for such activity are familiar to those skilled in the art, including the assays disclosed in the following references, which are incorporated herein by reference: Johansson *et al. Cell. Biol.* **15**:141-151, 1995; Keller *et al., Mol. Cell. Biol.* **13**:473-486, 1993; McClanahan *et al., Blood* **81**:2903-2915, 1993.

The proteins encoded by the extended cDNAs or portions thereof may also be evaluated for their influence on the lifetime of stem cells and stem cell differentiation. Numerous assays for such activity are familiar to those skilled in the art, including the assays disclosed in the following references, which are incorporated herein by reference: Freshney, Methylcellulose Colony Forming Assays, in *Culture of Hematopoietic Cells.,* Freshney, *et al..* Eds. pp. 265-268, Wiley-Liss, Inc., New York, NY. 1994; Hirayama *et al., Proc. Natl. Acad*. *Sci. USA* **89**:5907-5911, 1992; McNiece and Briddell, in *Culture of Hematopoietic Cells, supra,* Neben *et al., Exp. Hematol.* **22**:353-359, 1994; Ploemacher and Cobblestone In *Culture of Hematopoietic Cells, supra*1-21, Spooncer *et al,* in *Culture of Hematopoietic Cells, supra*163-179 and Sutherland in *Culture of Hematopoietic Cells, supra.* 139-162.

Those proteins which exhibit hematopoiesis regulatory activity may then be formulated as pharmaceuticals and used to treat clinical conditions in which regulation of hematopoeisis is beneficial, such as in the treatment of myeloid or lymphoid cell deficiencies. Involvement in regulating hematopoiesis is indicated even by marginal biological activity in support of colony forming cells or of factor-dependent cell lines. For example, proteins supporting the growth and proliferation of erythroid progenitor cells alone or in combination with other cytokines, indicates utility, for example, in treating various anemias or for use in conjunction with irradiation/chemotherapy to stimulate the production of erythroid precursors and/or erythroid cells. Proteins supporting the growth and proliferation of myeloid cells such as granulocytes and monocytes/macrophages *(i.e.,* traditional CSF activity) may be useful, for example, in conjunction with chemotherapy to prevent or treat consequent myelo-suppression. Proteins supporting the growth and proliferation of megakaryocytes and consequently of platelets allows prevention or treatment of various platelet disorders such as thrombocytopenia, and generally may be used in place of or complementary to platelet transfusions. Proteins supporting the growth and proliferation of hematopoietic stem cells which are capable of maturing to any and all of the above-mentioned hematopoietic cells may therefore find therapeutic utility in various stem cell disorders (such as those usually treated with transplantion, including, without limitation, aplastic anemia and paroxysmal nocturnal hemoglobinuria), as well as in repopulating the stem cell compartment post irradiation/chemotherapy, either *in vivo* or *ex vivo (i.e.,* in conjunction with bone marrow transplantation or with peripheral progenitor cell transplantation (homologous or heterologous)) as normal cells or genetically manipulated for gene therapy. Alternatively, as described in more detail below, genes encoding hematopoiesis regulating activity proteins or nucleic acids regulating the expression of such proteins may be introduced into appropriate host cells to increase or decrease the expression of the proteins as desired.

### EXAMPLE 35

### Assaying the Proteins Expressed from Extended cDNAs or Portions Thereof for Regulation of Tissue Growth

The proteins encoded by the extended cDNAs or portions thereof may also be evaluated for their effect on tissue growth. Numerous assays for such activity are familiar to those skilled in the art, including the assays disclosed in International Patent Publication No. WO95/16035, International Patent Publication No. WO95/05846 and International Patent Publication No. WO91/07491, which are incorporated herein by reference.

Assays for wound healing activity include, without limitation, those described in: Winter, *Epidermal Wound Healing,* pps. 71-112, Maibach and Rovee, eds., Year Book Medical Publishers, Inc., Chicago, as modified by Eaglstein and Mertz, *J. Invest. Dermatol.* **71**:382-84, 1978, which are incorporated herein by reference.

Those proteins which are involved in the regulation of tissue growth may then be formulated as pharmaceuticals and used to treat clinical conditions in which regulation of tissue growth is beneficial. For example, a protein encoded by extended cDNAs derived from the 5' ESTs of the present invention also may have utility in compositions used for bone, cartilage, tendon, ligament and/or nerve tissue growth or regeneration, as well as for wound healing and tissue repair and replacement, and in the treatment of burns, incisions and ulcers.

A protein encoded by extended cDNAs derived from the 5' ESTs of the present invention, which induces cartilage and/or bone growth in circumstances where bone is not normally formed, has application in the healing of bone fractures and cartilage damage or defects in humans and other animals. Such a preparation employing a protein of the invention may have prophylactic use in closed as well as open fracture reduction and also in the improved fixation of artificial joints *De novo* bone synthesis induced by an osteogenic agent contributes to the repair of congenital, trauma induced, or oncologic resection induced craniofacial defects, and also is useful in cosmetic plastic surgery.

A protein of this invention may also be used in the treatment of periodontal disease, and in other tooth repair processes. Such agents may provide an environment to attract bone-forming cells, stimulate growth of bone-forming cells or induce differentiation of bone-forming cell progenitors. A protein of the invention may also be useful in the treatment of osteoporosis or osteoarthritis, such as through stimulation of bone and/or cartilage repair or by blocking inflammation or processes of tissue destruction (collagenase activity, osteoclast activity, etc.) mediated by inflammatory processes.

Another category of tissue regeneration activity that may be attributable to the protein encoded by extended cDNAs derived from the 5' ESTs of the present invention is tendon/ligament formation. A protein encoded by extended cDNAs derived from the 5' ESTs of the present invention, which induces tendon/ligament-like tissue or other tissue formation in circumstances where such tissue is not normally formed, has application in the healing of tendon or ligament tears, deformities and other tendon or ligament defects in humans and other animals. Such a preparation employing a tendon/ligament-like tissue inducing protein may have prophylactic use in preventing damage to tendon or ligament tissue, as well as use in the improved fixation of tendon or ligament to bone or other tissues, and in repairing defects to tendon or ligament tissue. *De novo* tendon/ligament-like tissue formation induced by a composition encoded by extended cDNAs derived from the 5' ESTs of the present invention contributes to the repair of tendon or ligaments defects of congenital, traumatic or other origin and is also useful in cosmetic plastic surgery for attachment or repair of tendons or ligaments. The compositions encoded by extended cDNAs derived from the 5' ESTs of the present invention may provide an environment to attract tendon- or ligament-forming cells, stimulate growth of tendon- or ligament-forming cells, induce differentiation of progenitors of tendon- or ligament-forming cells, or induce growth of tendon/ligament cells or progenitors *ex vivo* for return *in vivo* to effect tissue repair. The compositions of the invention may also be useful in the treatment of tendinitis, carpal tunnel syndrome and other tendon or ligament defects. The compositions may also include an appropriate matrix and/or sequestering agent as a carrier as is well known in the art.

The protein encoded by extended cDNAs derived from the 5' ESTs of the present invention may also be useful for proliferation of neural cells and for regeneration of nerve and brain tissue, *i.e.,* for the treatment of central and peripheral nervous system diseases and neuropathies, as well as mechanical and traumatic disorders, which involve degeneration, death or trauma to neural cells or nerve tissue. More specifically, a protein may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotrophic lateral sclerosis, and Shy-Drager syndrome. Further conditions which may be treated in accordance with the present invention include mechanical and traumatic disorders, such as spinal cord disorders, head trauma and cerebrovascular diseases such as stroke. Peripheral neuropathies resulting from chemotherapy or other medical therapies may also be treatable using a protein of the invention.

Proteins of the invention may also be useful to promote better or faster closure of non-healing wounds, including without limitation pressure ulcers, ulcers associated with vascular insufficiency, surgical and traumatic wounds, and the like.

It is expected that a protein encoded by extended cDNAs derived from the 5' ESTs of the present invention may also exhibit activity for generation or regeneration of other tissues, such as organs (including, for example, pancreas, liver, intestine, kidney, skin, endothelium) muscle (smooth, skeletal or cardiac) and vascular (including vascular endothelium) tissue, or for promoting the growth of cells comprising such tissues. Part of the desired effects may be by inhibition or modulation of fibrotic scarring to allow normal tissue to generate. A protein of the invention may also exhibit angiogenic activity.

A protein encoded by extended cDNAs derived from the 5' ESTs of the present invention may also be useful for gut protection or regeneration and treatment of lung or liver fibrosis, reperfusion injury in various tissues, and conditions resulting from systemic cytokinc damage.

A protein encoded by extended cDNAs derived from the 5' ESTs of the present invention may also be useful for promoting or inhibiting differentiation of tissues described above from precursor tissues or cells; or for inhibiting the growth of tissues described above.

Alternatively, as described in more detail below, genes encoding tissue growth regulating activity proteins or nucleic acids regulating the expression of such proteins may be introduced into appropriate host cells to increase or decrease the expression of the proteins as desired.

### EXAMPLE 36

### Assaying the Proteins Expressed from Extended cDNAs or Portions Thereof for Regulation of Reproductive Hormones

The proteins encoded by the extended cDNAs or portions thereof may also be evaluated for their ability to regulate reproductive hormones, such as follicle stimulating hormone. Numerous assays for such activity are familiar to those skilled in the art, including the assays disclosed in the following references, which are incorporated herein by reference: Vale *et al., Endocrinol.* **91**:562-572, 1972; Ling *et al., Nature* **321**:779-782, 1986; Vale *et al., Nature* **321**:776-779, 1986; Mason *et al.., Nature* **318**:659-663, 1985; Forage *et al., Proc. Natl. Acad. Sci. USA* **83**:3091-3095, 1986, Chapter 6.12 in *Current Protocols in Immunology,* Coligan *et al.* Eds. Greene Publishing Associates and Wiley-Intersciece ; Taub *et al., J. Clin. Invest.* **95**:1370-1376, 1995; Lind *et al., APMIS* **103**:140-146, 1995; Muller *et al., Eur. J Immunol.* **25**:1744-1748; Gruber *et al.. J. Immunol.* **152**:5860-5867, 1994; Johnston *et al., J Immunol*. **153**:1762-1768, 1994.

Those proteins which exhibit activity as reproductive hormones or regulators of cell movement may then be formulated as pharmaceuticals and used to treat clinical conditions in which regulation of reproductive hormones are beneficial. For example, a protein encoded by extended cDNAs derived from the 5' ESTs of the present invention may also exhibit activin-or inhibin-related activities. Inhibins are characterized by their ability to inhibit the release of follicle stimulating hormone (FSH), while activins are characterized by their ability to stimulate the release of FSH. Thus, a protein encoded by extended cDNAs derived from the 5' ESTs of the present invention, alone or in heterodimers with a member of the inhibin a family, may be useful as a contraceptive based on the ability of inhibins to decrease fertility in female mammals and decrease spermatogenesis in male mammals. Administration of sufficient amounts of other inhibins can induce infertility in these mammals. Alternatively, the protein of the invention, as a homodimer or as a heterodimer with other protein subunits of the inhibin-B group, may be useful as a fertility inducing therapeutic, based upon the ability of activin molecules in stimulating FSH release from cells of the anterior pituitary. See, for example, United States Patent 4,798,885, the disclosure of which is incorporated herein by reference. A protein of the invention may also be useful for advancement of the onset of fertility in sexually immature mammals, so as to increase the lifetime reproductive performance of domestic animals such as cows, sheep and pigs.

Alternatively, as described in more detail below, genes encoding reproductive hormone regulating activity proteins or nucleic acids regulating the expression of such proteins may be introduced into appropriate host cells to increase or decrease the expression of the proteins as desired.

### EXAMPLE 37

### Assaying the Proteins Expressed from Extended cDNAs or Portions Thereof for Chemotactic/Chemokinetic Activity

The proteins encoded by the extended cDNAs or portions thereof may also be evaluated for chemotactic/chemoldnetic activity. For example, a protein encoded by extended cDNAs derived from the 5' ESTs of the present invention may have chemotactic or chemokinetic activity (e.g., act as a chemokine) for mammalian cells, including, for example, monocytes, fibroblasts, neutrophils, T-cells, mast cells, eosinophils, epithelial and/or endothelial cells. Chemotactic and chemokinetic proteins can be used to mobilize or attract a desired cell population to a desired site of action. Chemotactic or chemokinetic proteins provide particular advantages in treatment of wounds and other trauma to tissues, as well as in treatment of localized infections. For example, attraction of lymphocytes, monocytes or neutrophils to tumors or sites of infection may result in improved immune responses against the tumor or infecting agent.

A protein or peptide has chemotactic activity for a particular cell population if it can stimulate, directly or indirectly, the directed orientation or movement of such cell population. Preferably, the protein or peptide has the ability to directly stimulate directed movement of cells. Whether a particular protein has chemotactic activity for a population of cells can be readily determined by employing such protein or peptide in any known assay for cell chemotaxis.

The activity of a protein of the invention may, among other means, be measured by the following methods:

Assays for chemotactic activity (which will identify proteins that induce or prevent chemotaxis) consist of assays that measure the ability of a protein to induce the migration of cells across a membrane as well as the ability of a protein to induce the adhesion of one cell population to another cell population. Suitable assays for movement and adhesion include, without limitation, those described in: *Current Protocols in Immunology,* Ed by Coligan, Kruisbeek, Margulies, Shevach and Strober, Pub. Greene Publishing Associates and Wiley-Interscience, Chapter **6.12**: 6.12.1-6.12.28; Taub *et al., J. Clin. Invest.* **95**:1370-1376, 1995; Lind *et al., APMIS* **103**:140-146, 1995; Mueller *et al*., *Eur. J. Immunol.* **25**:1744-1748; Gruber *et al., J. Immunol.* **152**:5860-5867, 1994; Johnston *et al. J. Immunol.,* **153**:1762-1768, 1994.

### EXAMPLE 38

### Assaying the Proteins Expressed from Extended cDNAs or Portions Thereof for Regulation of Blood Clotting

The proteins encoded by the extended cDNAs or portions thereof may also be evaluated for their effects on blood clotting. Numerous assays for such activity are familiar to those skilled in the art, including the assays disclosed in the following references, which are incorporated herein by reference: Linet *et al., J. Clin. Pharmacol.* **26**:13l-140, 1986; Burdick *et al.*, *Thrombosis Res.* **45:**413-419, 1987; Humphrey *et* al., *Fibrinolysis* **5**:71-79, 1991; Schaub, *Prostaglandins* **35**:467-474, 1988.

Those proteins which are involved in the regulation of blood clotting may then be formulated as pharmaceuticals and used to treat clinical conditions in which regulation of blood clotting is beneficial. For example, a protein of the invention may also exhibit hemostatic or thrombolytic activity. As a result, such a protein is expected to be useful in treatment of various coagulations disorders (including hereditary disorders, such as hemophilias) or to enhance coagulation and other hemostatic events in treating wounds resulting from trauma, surgery or other causes. A protein of the invention may also be useful for dissolving or inhibiting formation of thromboses and for treatment and prevention of conditions resulting therefrom (such as infarction of cardiac and central nervous system vessels (e.g., stroke)). Alternatively, as described in more detail below, genes encoding blood clotting activity proteins or nucleic acids regulating the expression of such proteins may be introduced into appropriate host cells to increase or decrease the expression of the proteins as desired.

### EXAMPLE 39

### Assaying the Proteins Expressed from Extended cDNAs or Portions Thereof for Involvement in Receptor/Ligand Interactions

The proteins encoded by the extended cDNAs or a portion thereof may also be evaluated for their involvement in receptor/ligand interactions. Numerous assays for such involvement are familiar to those skilled in the art, including the assays disclosed in the following references, which are incorporated herein by reference: Chapter 7. 7.28.1-7.28.22 in *Current Protocols in Immunology,* Coligan *et al.* Eds. Greene Publishing Associates and Wiley-Interscience; Takai *et al., Proc. Natl. Acad. Sci. USA* **84**:6864-6868, 1987; Bierer *et al., J. Exp. Med* **168**:1145-1156, 1988; Rosenstein *et al., J. Exp. Med* **169**:149-160, 1989; Stoltenborg *et al., J. Immunol. Methods* **175**:59-68, 1994; Stitt *et al.. Cell* **80**:661-670, 1995; Gyuris *et al., Cell* **75:**791-803, 1993.

For example, the proteins encoded by extended cDNAs derived from the 5' ESTs of the present invention may also demonstrate activity as receptors, receptor ligands or inhibitors or agonists of receptor/ligand interactions. Examples of such receptors and ligands include, without limitation, cytokine receptors and their ligands, receptor kinases and their ligands, receptor phosphatases and their ligands, receptors involved in cell-cell interactions and their ligands (including without limitation, cellular adhesion molecules (such as selectins, integrins and their ligands) and receptor/ligand pairs involved in antigen presentation, antigen recognition and development of cellular and humoral immune responses). Receptors and ligands are also useful for screening of potential peptide or small molecule inhibitors of the relevant receptor/ligand interaction. A protein encoded by extended cDNAs derived from the 5' ESTs of the present invention (including, without limitation, fragments of receptors and ligands) may themselves be useful as inhibitors of receptor/ligand interactions. Alternatively, as described in more detail below, genes encoding proteins involved in receptor/ligand interactions or nucleic acids regulating the expression of such proteins may be introduced into appropriate host cells to increase or decrease the expression of the proteins as desired.

### EXAMPLE 40

### Assaying the Proteins Expressed from Extended cDNAs or Portions Thereof for Anti-Inflammatory Activity

The proteins encoded by the extended cDNAs or a portion thereof may also be evaluated for anti-inflammatory activity. The anti-inflammatory activity may be achieved by providing a stimulus to cells involved in the inflammatory response, by inhibiting or promoting cell-cell interactions (such as, for example, cell adhesion), by inhibiting or promoting chemotaxis of cells involved in the inflammatory process, inhibiting or promoting cell extravasation, or by stimulating or suppressing production of other factors which more directly inhibit or promote an inflammatory response. Proteins exhibiting such activities can be used to treat inflammatory conditions including chronic or acute conditions, including without limitation inflammation associated with infection (such as septic shock, sepsis or systemic inflammatory response syndrome), ischemia-reperfusioninury, endotoxin lethality, arthritis, complement-mediated hyperacute rejection, nephritis, cytokine- or chemokine-induced lung injury, inflammatory bowel disease, Crohn's disease or resulting from over production of cytokines such as TNF or IL-1. Proteins of the invention may also be useful to treat anaphylaxis and hypersensitivity to an antigenic substance or material. Alternatively, as described in more detail below, genes encoding anti-inflammatory activity proteins or nucleic acids regulating the expression of such proteins may be introduced into appropriate host cells to increase or decrease the expression of the proteins as desired.

### EXAMPLE 41

### Assaying the Proteins Expressed from Extended cDNAs or Portions Thereof for Tumor Inhibition Activity

The proteins encoded by the extended cDNAs or a portion thereof may also be evaluated for tumor inhibition activity. In addition to the activities described above for immunological treatment or prevention of tumors, a protein of the invention may exhibit other anti-tumor activities. A protein may inhibit tumor growth directly or indirectly (such as, for example, via ADCC). A protein may exhibit its tumor inhibitory activity by acting on tumor tissue or tumor precursor tissue, by inhibiting formation of tissues necessary to support tumor growth (such as, for example, by inhibiting angiogenesis), by causing production of other factors, agents or cell types which inhibit tumor growth, or by suppressing, eliminating or inhibiting factors, agents or cell types which promote tumor growth. Alternatively, as described in more detail below, genes tumor inhibition activity proteins or nucleic acids regulating the expression of such proteins may be introduced into appropriate host cells to increase or decrease the expression of the proteins as desired.

A protein of the invention may also exhibit one or more of the following additional activities or effects: inhibiting the growth, infection or function of, or killing, infectious agents, including, without limitation, bacteria, viruses, fungi and other parasites; effecting (suppressing or enhancing) bodily characteristics, including, without limitation, height, weight, hair color, eye color, skin, fat to lean ratio or other tissue pigmentation, or organ or body part size or shape (such as, for example, breast augmentation or diminution, change in bone form or shape); effecting biorhythms or circadian cycles or rhythms; effecting the fertility of male or female subjects; effecting the metabolism, catabolism, anabolism, processing, utilization, storage or elimination of dietary fat, lipid, protein, carbohydrate, vitamins, minerals, cofactors or other nutritional factors or component(s); effecting behavioral characteristics, including, without limitation, appetite, libido, stress, cognition (including cognitive disorders), depression (including depressive disorders) and violent behaviors; providing analgesic effects or other pain reducing effects; promoting differentiation and growth of embryonic stem cells in lineages other than hematopoietic lineages; hormonal or endocrine activity; in the case of enzymes, correcting deficiencies of the enzyme and treating deficiency-related diseases; treatment of hyperproliferative disorders (such as, for example, psoriasis); immunoglobulin-like activity (such as, for example, the ability to bind antigens or complement); and the ability to act as an antigen in a vaccine composition to raise an immune response against such protein or another material or entity which is cross-reactive with such protein. Alternatively, as described in more detail below, genes encoding proteins involved in any of the above mentioned activities or nucleic acids regulating the expression of such proteins may be introduced into appropriate host cells to increase or decrease the expression of the proteins as desired.

### EXAMPLE 42

### Identification of Proteins which Interact with Polypeptides Encoded by Extended cDNAs

Proteins which interact with the polypeptides encoded by cDNAs derived from the 5' ESTs or fragments thereof such as receptor proteins, may be identified using two hybrid systems such as the Matchmaker Two Hybrid System 2 (Catalog No. K1604-1, Clontech). As described in the manual accompanying the kit which is incorporated herein by reference, the the cDNAs derived from 5' ESTs, or fragments thereof, are inserted into an expression vector such that they are in frame with DNA encoding the DNA binding domain of the yeast transcriptional activator GAL4. cDNAs in a cDNA library which encode proteins which might interact with the polypeptides encoded by the extended cDNAs or portions thereof are inserted into a second expression vector such that they are in frame with DNA encoding the activation domain of GAL4. The two expression plasmids are transformed into yeast and the yeast are plated on selection medium which selects for expression of selectable markers on each of the expression vectors as well as GAL4 dependent expression of the HIS3 gene. Transformants capable of growing on medium lacking histidine are screened for GAL4 dependent lacZ expression. Those cells which are positive in both the histidine selection and the lacZ assay contain plasmids encoding proteins which interact with the polypeptide encoded by the extended cDNAs or portions thereof.

Alternatively, the system described in Lustig *et al., Methods in Enzymology* **283**: 83-99, 1997, and in U.S. Patent No. 5,654,150, the disclosure of which is incorporated herein by reference, may be used for identifying molecules which interact with the polypeptides encoded by extended cDNAs. In such systems, *in vitro* transcription reactions are performed on a pool of vectors containing extended cDNA inserts cloned downstream of a promoter which drives *in vitro* transcription. The resulting pools of mRNAs are introduced into *Xenopus laevis* oocytes. The oocytes are then assayed for a desired activity.

Alternatively, the pooled *in vitro* transcription products produced as described above may be translated *in vitro.* The pooled *in vitro* translation products can be assayed for a desired activity or for interaction with a known polypeptide.

Proteins or other molecules interacting with polypeptides encoded by extended cDNAs can be found by a variety of additional techniques. In one method, affinity columns containing the polypeptide encoded by the extended cDNA or a portion thereof can be constructed. In some versions, of this method the affinity column contains chimeric proteins in which the protein encoded by the extended cDNA or a portion thereof is fused to glutathione S-transferase. A mixture of cellular proteins or pool of expressed proteins as described above and is applied to the affinity column. Proteins interacting with the polypeptide attached to the column can then be isolated and analyzed on 2-D electrophoresis gel as described in Ramunsen *et al., Electrophoresis* **18**:588-598, 1997, the disclosure of which is incorporated herein by reference. Alternatively, the proteins retained on the affinity column can be purified by electrophoresis based methods and sequenced. The same method can be used to isolate antibodies, to screen phage display products, or to screen phage display human antibodies.

Proteins interacting with polypeptides encoded by extended cDNAs or portions thereof can also be screened by using an Optical Biosensor as described in Edwards and Leatherbarrow, *Analytical Biochemistry* **246**:1-6, 1997, the disclosure of which is incorporated herein by reference. The main advantage of the method is that it allows the determination of the association rate between the protein and other interacting molecules. Thus, it is possible to specifically select interacting molecules with a high or low association rate. Typically a target molecule is linked to the sensor surface (through a carboxymethl dextran matrix) and a sample of test molecules is placed in contact with the target molecules. The binding of a test molecule to the target molecule causes a change in the refractive index and/ or thickness. This change is detected by the Biosensor provided it occurs in the evanescent field (which extend a few hundred nanometers from the sensor surface). In these screening assays, the target molecule can be one of the polypeptides encoded by extended cDNAs or a portion thereof and the test sample can be a collection of proteins extracted from tissues or cells, a pool of expressed proteins, combinatorial peptide and/ or chemical libraries, or phage displayed peptides. The tissues or cells from which the test proteins are extracted can originate from any species.

In other methods, a target protein is immobilized and the test population is a collection of unique polypeptides encoded by the extended cDNAs or portions thereof.

To study the interaction of the proteins encoded by the extended cDNAs or portions thereof with drugs, the microdialysis coupled to HPLC method described by Wang *et al., Chromatographia* **44**:205-208, 1997 or the affinity capillary electrophoresis method described by Busch *et al., J. Chromatogr.* **777**:311-328, 1997, the disclosures of which are incorporated herein by reference can be used.

It will be appreciated by those skilled in the art that the proteins expressed from the extended cDNAs or portions may be assayed for numerous activities in addition to those specifically enumerated above. For example, the expressed proteins may be evaluated for applications involving control and regulation of inflammation, tumor proliferation or metastasis, infection, or other clinical conditions. In addition, the proteins expressed from the extended cDNAs or portions thereof may be useful as nutritional agents or cosmetic agents.

The proteins expressed from the cDNAs or portions thereof may be used to generate antibodies capable of specifically binding to the expressed protein or fragments thereof as described in Example 40 below. The antibodies may capable of binding a full length protein encoded by a cDNA derived from a 5' EST, a mature protein (*i.e.* the protein generated by cleavage of the signal peptide) encoded by a cDNA derived from a 5' EST, or a signal peptide encoded by a cDNA derived from a 5' EST. Alternatively, the antibodies may be capable of binding fragments of at least 10 amino acids of the proteins encoded by the above cDNAs. In some embodiments, the antibodies may be capable of binding fragments of at least 15 amino acids of the proteins encoded by the above cDNAs. In other embodiments, the antibodies may be capable of binding fragments of at least 25 amino acids of the proteins expressed from the extended cDNAs which comprise at least 25 amino acids of the proteins encoded by the above cDNAs. In further embodiments, the antibodies may be capable of binding fragments of at least 40 amino acids of the proteins encoded by the above cDNAs.

### EXAMPLE 43

### Production of an Antibody to a Human Protein

Substantially pure protein or polypeptide is isolated from the transfected or transformed cells as described in Example 30. The concentration of protein in the final preparation is adjusted, for example, by concentration on an Amicon filter device, to the level of a few µg/ml. Monoclonal or polyclonal antibody to the protein can then be prepared as follows:

### 1. Monoclonal Antibody Production by Hybridoma Fusion

Monoclonal antibody to epitopes of any of the peptides identified and isolated as described can be prepared from murine hybridomas according to the classical method of Kohler, and Milstein, *Nature* **256**:495, 1975 or derivative methods thereof. Briefly, a mouse is repetitively inoculated with a few micrograms of the selected protein or peptides derived therefrom over a period of a few weeks. The mouse is then sacrificed, and the antibody producing cells of the spleen isolated. The spleen cells are fused by means of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall, *Meth. Enzymol.* **70**:419, 1980, the disclosure of which is incorporated herein by reference and derivative methods thereof. Selected positive clones can be expanded and their monoclonal antibody product harvested for use. Detailed procedures for monoclonal antibody production are described in Davis *et al.* in *Basic Methods in Molecular Biology* Elsevier, New York. Section 21-2, the disclosure of which is incorporated herein by reference.

### 2. Polyclonal Antibody Production by Immunization

Polyclonal antiserum containing antibodies to heterogenous epitopes of a single protein can be prepared by immunizing suitable animals with the expressed protein or peptides derived therefrom, which can be unmodified or modified to enhance immunogenicity. Effective polyclonal antibody production is affected by many factors related both to the antigen and the host species. For example, small molecules tend to be less immunogenic than others and may require the use of carriers and adjuvant. Also, host animals response vary depending on site of inoculations and doses, with both inadequate or excessive doses of antigen resulting in low titer antisera. Small doses (ng level) of antigen administered at multiple intradermal sites appears to be most reliable. An effective immunization protocol for rabbits can be found in Vaitukaitis. *et al, J. Clin. Endocrinol. Metab.* **33**:988-991 (1971), the disclosure of which is incorporated herein by reference.

Booster injections can be given at regular intervals, and antiserum harvested when antibody titer thereof, as determined semi-quantitatively, for example, by double immunodifiusion in agar against known concentrations of the antigen, begins to fall. See, for example, Ouchterlony, *et al.,* Chap. 19 in: *Handbook of Experimental Immunology D.* Wier (ed) Blackwell (1973), the disclosure of which is incorporated herein by reference. Plateau concentration of antibody is usually in the range of 0.1 to 0.2 mg/ml of serum (about 12 µM). Affinity of the antisera for the antigen is determined by preparing competitive binding curves, as described, for example, by Fisher, D., Chap. 42 in: *Manual of Clinical Immunology,* 2d Ed. (Rose and Friedman, Eds.) Amer. Soc. For Microbiol., Washington, D.C. (1980), the disclosure of which is incorporated herein by reference.

Antibody preparations prepared according to either protocol are useful in quantitative immunoassays which determine concentrations of antigen-bearing substances in biological samples; they are also used semi-quantitatively or qualitatively to identify the presence of antigen in a biological sample. The antibodies may also be used in therapeutic compositions for killing cells expressing the protein or reducing the levels of the protein in the body.

### V. Use of 5' ESTs or Sequences Obtainable Therefrom or Portions Thereof as Reagents

The 5' ESTs of the present invention (or cDNAs or genomic DNAs obtainable therefrom) may be used as reagents in isolation procedures, diagnostic assays, and forensic procedures. For example, sequences from the 5' ESTs (or cDNAs or genomic DNAs obtainable therefrom) may be detectably labeled and used as probes to isolate other sequences capable of hybridizing to them. In addition, sequences from 5' ESTs (or cDNAs or genomic DNAs obtainable therefrom) may be used to design PCR primers to be used in isolation, diagnostic, or forensic procedures.

### 1. Use of 5' ESTs or Sequences Obtainable Therefrom or Portions Thereof in Isolation, Diagnostic and Forensic Procedures

### EXAMPLE 44

### Preparation of PCR Primers and Amplification of DNA

The 5' EST sequences (or cDNAs or genomic DNAs obtainable therefrom) may be used to prepare PCR primers for a variety of applications, including isolation procedures for cloning nucleic acids capable of hybridizing to such sequences, diagnostic techniques and forensic techniques. The PCR primers are at least 10 bases, and preferably at least 12, 15, or 17 bases in length. More preferably, the PCR primers are at least 20-30 bases in length. In some embodiments, the PCR primers may be more than 30 bases in length. It is preferred that the primer pairs have approximately the same G/C ratio, so that melting temperatures are approximately the same. A variety of PCR techniques are familiar to those skilled in the art. For a review of PCR technology, see Molecular Cloning to Genetic Engineering, White Ed. *in Methods in Molecular Biology* **67**: Humana Press, Totowa 1997, the disclosure of which is incorporated herein by reference. In each of these PCR procedures, PCR primers on either side of the nucleic acid sequences to be amplified are added to a suitably prepared nucleic acid sample along with dNTPs and a thermostable polymerase such as Taq polymerase, Pfu polymerase, or Vent polymerase. The nucleic acid in the sample is denatured and the PCR primers are specifically hybridized to complementary nucleic acid sequences in the sample. The hybridized primers are extended. Thereafter, another cycle of denaturation, hybridization, and extension is initiated. The cycles are repeated multiple times to produce an amplified fragment containing the nucleic acid sequence between the primer sites.

### EXAMPLE 45

### Use of 5'ESTs as Probes

Probes derived from 5' ESTs (or cDNAs or genomic DNAs obtainable therefrom), including full length cDNAs or genomic sequences, may be labeled with detectable labels familiar to those skilled in the art, including radioisotopes and non-radioactive labels, to provide a detectable probe. The detectable probe may be single stranded or double stranded and may be made using techniques known in the art, including *in vitro* transcription, nick translation, or kinase reactions. A nucleic acid sample containing a sequence capable of hybridizing to the labeled probe is contacted with the labeled probe. If the nucleic acid in the sample is double stranded, it may be denatured prior to contacting the probe. In some applications, the nucleic acid sample may be immobilized on a surface such as a nitrocellulose or nylon membrane. The nucleic acid sample may comprise nucleic acids obtained from a variety of sources, including genomic DNA, cDNA libraries, RNA, or tissue samples.

Procedures used to detect the presence of nucleic acids capable of hybridizing to the detectable probe include well known techniques such as Southern blotting, Northern blotting, dot blotting, colony hybridization, and plaque hybridization. In some applications, the nucleic acid capable of hybridizing to the labeled probe may be cloned into vectors such as expression vectors, sequencing vectors, or *in vitro* transcription vectors to facilitate the characterization and expression of the hybridizing nucleic acids in the sample. For example, such techniques may be used to isolate and clone sequences in a genomic library or cDNA library which are capable of hybridizing to the detectable probe as described in Example 30 above.

PCR primers made as described in Example 44 above may be used in forensic analyses, such as the DNA fingerprinting techniques described in Examples 46-50 below. Such analyses may utilize detectable probes or primers based on the sequences of the the 5' ESTs or of cDNAs or genomic DNAs isolated using the 5' ESTs.

### EXAMPLE 46

### Forensic Matching by DNA Sequencing

In one exemplary method, DNA samples are isolated from forensic specimens of, for example, hair, semen, blood or skin cells by conventional methods. A panel of PCR primers based on a number of the 5' ESTs of Example 25, or cDNAs or genomic DNAs isolated therefrom as described above, is then utilized in accordance with Example 44 to amplify DNA of approximately 100-200 bases in length from the forensic specimen. Corresponding sequences are obtained from a test subject. Each of these identification DNAs is then sequenced using standard techniques, and a simple database comparison determines the differences, if any, between the sequences from the subject and those from the sample. Statistically significant differences between the suspect's DNA sequences and those from the sample conclusively prove a lack of identity. This lack of identity can be proven, for example, with only one sequence. Identity, on the other hand, should be demonstrated with a large number of sequences, all matching. Preferably, a minimum of 50 statistically identical sequences of 100 bases in length are used to prove identity between the suspect and the sample.

### EXAMPLE 47

### Positive Identification by DNA Sequencing

The technique outlined in the previous example may also be used on a larger scale to provide a unique fingerprint-type identification of any individual. In this technique, primers are prepared from a large number of 5'EST sequences from Example 25, or cDNA or genomic DNA sequences obtainable therefrom. Preferably, 20 to 50 different primers are used. These primers are used to obtain a corresponding number of PCR-generated DNA segments from the individual in question in accordance with Example 44. Each of these DNA segments is sequenced, using the methods set forth in Example 46. The database of sequences generated through this procedure uniquely identifies the individual from whom the sequences were obtained. The same panel of primers may then be used at any later time to absolutely correlate tissue or other biological specimen with that individual.

### EXAMPLE 48

### Southern Blot Forensic Identification

The procedure of Example 47 is repeated to obtain a panel of at least 10 amplified sequences from an individual and a specimen. Preferably, the panel contains at least 50 amplified sequences. More preferably, the panel contains 100 amplified sequences. In some embodiments, the panel contains 200 amplified sequences. This PCR-generated DNA is then digested with one or a combination of, preferably, four base specific restriction enzymes. Such enzymes are commercially available and known to those of skill in the art. After digestion, the resultant gene fragments are size separated in multiple duplicate wells on an agarose gel and transferred to nitrocellulose using Southern blotting techniques well known to those with skill in the art. For a review of Southern blotting see Davis *et al.* (Basic Methods in Molecular Biology, 1986, Elsevier Press. pp 62-65), the disclosure of which is incorporated herein by reference..

A panel of probes based on the sequences of 5' ESTs (or cDNAs or genomic DNAs obtainable therefrom), or fragments thereof of at least 10 bases, are radioactively or colorimetrically labeled using methods known in the art, such as nick translation or end labeling, and hybridized to the Southern blot using techniques known in the art (Davis *et al.,* supra). Preferably, the probe comprises at least 12, 15, or 17 consecutive nucleotides from the 5' EST (or cDNAs or genomic DNAs obtainable therefrom). More preferably, the probe comprises at least 20-30 consecutive nucleotides from the 5' EST (or cDNAs or genomic DNAs obtainable therefrom). In some embodiments, the probe comprises more than 30 nucleotides from the 5' EST (or cDNAs or genomic DNAs obtainable therefrom).

Preferably, at least 5 to 10 of these labeled probes are used, and more preferably at least about 20 or 30 are used to provide a unique pattern. The resultant bands appearing from the hybridization of a large sample of 5' EST (or cDNAs or genomic DNAs obtainable therefrom) will be a unique identifier. Since the restriction enzyme cleavage will be different for every individual, the band pattern on the Southern blot will also be unique. Increasing the number of 5' EST (or cDNAs or genomic DNAs obtainable therefrom) probes will provide a statistically higher level of confidence in the identification since there will be an increased number of sets of bands used for identification.

### EXAMPLE 49

### Dot Blot Identification Procedure

Another technique for identifying individuals using the 5' EST sequences disclosed herein utilizes a dot blot hybridization technique.

Genomic DNA is isolated from nuclei of subject to be identified. Oligonucleotide probes of approximately 30 bp in length are synthesized that correspond to at least 10, preferably 50 sequences from the 5' ESTs or cDNAs or genomic DNAs obtainable therefrom. The probes are used to hybridize to the genomic DNA through conditions known to those in the art. The oligonucleotides are end labeled with P³² using polynucleotide kinase (Pharmacia). Dot Blots are created by spotting the genomic DNA onto nitrocellulose or the like using a vacuum dot blot manifold (BioRad, Richmond California). The nitrocellulose filter containing the genomic sequences is baked or UV linked to the filter, prehybridized and hybridized with labeled probe using techniques known in the art (Davis et *at., supra).* The ³²P labeled DNA fragments are sequentially hybridized with successively stringent conditions to detect minimal differences between the 30 bp sequence and the DNA. Tetramethylammonium chloride is useful for identifying clones containing small numbers of nucleotide mismatches (Wood *et al., Proc. Natl*. *Acad Sci. USA* **82(6)**:1585-1588, 1985) which is hereby incorporated by reference. A unique pattern of dots distinguishes one individual from another individual.

5' EST sequences (or cDNAs or genomic DNAs obtainable therefrom) or oligonucleotides containing at least 10 consecutive bases from these sequences can be used as probes in the following alternative fingerprinting technique. Preferably, the probe comprises at least 12, 15, or 17 consecutive nucleotides from the 5' EST sequences (or cDNAs or genomic DNAs obtainable therefrom). More preferably, the probe comprises at least 20-30 consecutive nucleotides from the 5' EST sequences (or cDNAs or genomic DNAs obtainable therefrom). In some embodiments, the probe comprises more than 30 nucleotides from the 5' EST sequences (or cDNAs or genomic DNAs obtainable therefrom).

Preferably, a plurality of probes having sequences from different genes are used in the alternative fingerprinting technique. Example 50 below provides a representative alternative fingerprinting procedure in which the probes are derived from 5'EST

### EXAMPLE 50

### Alternative "Fingerprint" Identification Technique

20-mer oligonucleotides are prepared from a large number, e.g. 50, 100, or 200, of 5'EST using commercially available oligonucleotide services such as Genset, Paris, France. Cell samples from the test subject are processed for DNA using techniques well known to those with skill in the art. The nucleic acid is digested with restriction enzymes such as EcoRI and XbaI. Following digestion, samples are applied to wells for electrophoresis. The procedure, as known in the art, may be modified to accommodate polyacrylamide electrophoresis, however in this example, samples containing 5 ug of DNA are loaded into wells and separated on 0.8% agarose gels. The gels are transferred onto nitrocellulose using standard Southern blotting techniques.

10 ng of each of the oligonucleotides are pooled and end-labeled with ³²p The nitrocellulose is prehybridized with blocking solution and hybridized with the labeled probes. Following hybridization and washing, the nitrocellulose filter is exposed to X-Omat AR X-ray film. The resulting hybridization pattern will be unique for each individual.

It is additionally contemplated within this example that the number of probe sequences used can be varied for additional accuracy or clarity.

The proteins encoded by the extended cDNAs may also be used to generate antibodies as explained in Examples 30 and 43 in order to identify the tissue type or cell species from which a sample is derived as described in example 51.

### EXAMPLE 51

### Identification of Tissue Types or Cell Species by Means of Labeled Tissue Specific Antibodies

Identification of specific tissues is accomplished by the visualization of tissue specific antigens by means of antibody preparations according to Examples 30 and 43 which are conjugated, directly or indirectly to a detectable marker. Selected labeled antibody species bind to their specific antigen binding partner in tissue sections, cell suspensions, or in extracts of soluble proteins from a tissue sample to provide a pattern for qualitative or semi-qualitative interpretation.

Antisera for these procedures must have a potency exceeding that of the native preparation, and for that reason, antibodies are concentrated to a mg/ml level by isolation of the gamma globulin fraction, for example, by ion-exchange chromatography or by ammonium sulfate fractionation. Also, to provide the most specific antisera, unwanted antibodies, for example to common proteins, must be removed from the gamma globulin fraction, for example by means of insoluble immunoabsorbents, before the antibodies are labeled with the marker. Either monoclonal or heterologous antisera is suitable for either procedure.

### A. Immunohistochemical techniques

Purified, high-titer antibodies, prepared as described above, are conjugated to a detectable marker, as described, for example, by Fudenberg, Chap. 26 in: *Basic and Clinical Immunology,* 3rd Ed. Lange, Los Altos, California, 1980, or Rose,. *et al.,* Chap. **12** in: *Methods in Immunodiagnosis,* 2d Ed. John Wiley and Sons, New York (1980), the disclosures of which are incorporated herein by reference.

A fluorescent marker, either fluorescein or rhodamine, is preferred, but antibodies can also be labeled with an enzyme that supports a color producing reaction with a substrate, such as horseradish peroxidase. Markers can be added to tissue-bound antibody in a second step, as described below. Alternatively, the specific antitissue antibodies can be labeled with ferritin or other electron dense particles, and localization of the ferritin coupled antigen-antibody complexes achieved by means of an electron microscope. In yet another approach, the antibodies are radiolabeled, with, for example ¹²⁵I, and detected by overlaying the antibody treated preparation with photographic emulsion.

Preparations to carry out the procedures can comprise monoclonal or polyclonal antibodies to a single protein or peptide identified as specific to a tissue type, for example, brain tissue, or antibody preparations to several antigenically distinct tissue specific antigens can be used in panels, independently or in mixtures, as required.

Tissue sections and cell suspensions are prepared for immunohistochemical examination according to common histological techniques. Multiple cryostat sections (about 4 µm, unfixed) of the unknown tissue and known control, are mounted and each slide covered with different dilutions of the antibody preparation. Sections of known and unknown tissues should also be treated with preparations to provide a positive control, a negative control, for example, pre-immune sera, and a control for non-specific staining, for example, buffer.

Treated sections are incubated in a humid chamber for 30 min at room temperature, rinsed, then washed in buffer for 30-45 min. Excess fluid is blotted away, and the marker developed.

If the tissue specific antibody was not labeled in the first incubation, it can be labeled at this time in a second antibody-antibody reaction, for example, by adding fluorescein- or enzyme-conjugated antibody against the immunoglobulin class of the antiserum-producing species, for example, fluorescein labeled antibody to mouse IgG. Such labeled sera are commercially available.

The antigen found in the tissues by the above procedure can be quantified by measuring the intensity of color or fluorescence on the tissue section, and calibrating that signal using appropriate standards.

### B. Identification of tissue specific soluble proteins

The visualization of tissue specific proteins and identification of unknown tissues from that procedure is carried out using the labeled antibody reagents and detection strategy as described for immunohistochemistry; however the sample is prepared according to an electrophoretic technique to distribute the proteins extracted from the tissue in an orderly array on the basis of molecular weight for detection.

A tissue sample is homogenized using a Virtis apparatus; cell suspensions are disrupted by Dounce homogenization or osmotic lysis, using detergents in either case as required to disrupt cell membranes, as is the practice in the art. Insoluble cell components such as nuclei, microsomes, and membrane fragments are removed by ultracentrifugation, and the soluble protein-containing fraction concentrated if necessary and reserved for analysis.

A sample of the soluble protein solution is resolved into individual protein species by conventional SDS polyacrylamide electrophoresis as described, for example, by Davis, *et al.,* Section **19-2** in: *Basic Methods in Molecular Biology,* Leder ed., Elsevier, New York, 1986, the disclosure of which is incorporated herein by reference, using a range of amounts of polyacrylamide in a set of gels to resolve the entire molecular weight range of proteins to be detected in the sample. A size marker is run in parallel for purposes of estimating molecular weights of the constituent proteins. Sample size for analysis is a convenient volume of from 5 to 55 µl, and containing from about 1 to 100 µg protein. An aliquot of each of the resolved proteins is transferred by blotting to a nitrocellulose filter paper, a process that maintains the pattern of resolution. Multiple copies are prepared. The procedure, known as Western Blot Analysis, is well described in Davis, L. *et al., supra* Section 19-3. One set of nitrocellulose blots is stained with Coomassie blue dye to visualize the entire set of proteins for comparison with the antibody bound proteins. The remaining nitrocellulose filters are then incubated with a solution of one or more specific antisera to tissue specific proteins prepared as described in Examples 30 and 43. In this procedure, as in procedure A above, appropriate positive and negative sample and reagent controls are run.

In either procedure A or B, a detectable label can be attached to the primary tissue antigen-primary antibody complex according to various strategies and permutations thereof. In a straightforward approach, the primary specific antibody can be labeled; alternatively, the unlabeled complex can be bound by a labeled secondary anti-IgG antibody. In other approaches, either the primary or secondary antibody is conjugated to a biotin molecule, which can, in a subsequent step, bind an avidin conjugated marker. According to yet another strategy, enzyme labeled or radioactive protein A, which has the property of binding to any IgG, is bound in a final step to either the primary or secondary antibody.

The visualization of tissue specific antigen binding at levels above those seen in control tissues to one or more tissue specific antibodies, prepared from the gene sequences identified from extended cDNA sequences, can identify tissues of unknown origin, for example, forensic samples, or differentiated tumor tissue that has metastasized to foreign bodily sites.

In addition to their applications in forensics and identification, 5' ESTs (or cDNAs or genomic DNAs obtainable therefrom) may be mapped to their chromosomal locations. Example 52 below describes radiation hybrid (RH) mapping of human chromosomal regions using 5'ESTs. Example 53 below describes a representative procedure for mapping an 5' EST to its location on a human chromosome. Example 54 below describes mapping of 5' ESTs on metaphase chromosomes by Fluorescence In Situ Hybridization (FISH). Those skilled in the art will appreciate that the method of Examples 52-54 may also be used to map cDNAs or genomic DNAs obtainable from the 5' ESTs to their chromosomal locations.

### 2. Use of 5' ESTs or Sequences Obtainable Therefrom or Portions Thereof in Chromosome Mapping

### EXAMPLE 52

### Radiation hybrid mapping of 5'ESTs to the human genome

Radiation hybrid (RH) mapping is a somatic cell genetic approach that can be used for high resolution mapping of the human genome. In this approach, cell lines containing one or more human chromosomes are lethally irradiated, breaking each chromosome into fragments whose size depends on the radiation dose. These fragments are rescued by fusion with cultured rodent cells, yielding subclones containing different portions of the human genome. This technique is described by Benham *et al., Genomics* **4**:509-517, 1989; and Cox *et al.. Science* **250**:245-250, 1990, the entire contents of which are hereby incorporated by reference. The random and independent nature of the subclones permits efficient mapping of any human genome marker. Human DNA isolated from a panel of 80-100 cell lines provides a mapping reagent for ordering 5'EST. In this approach, the frequency of breakage between markers is used to measure distance, allowing construction of fine resolution maps as has been done using conventional ESTs (Schuler *et al.. Science* **274**:540-546, 1996, hereby incorporated by reference).

RH mapping has been used to generate a high-resolution whole genome radiation hybrid map of human chromosome 17q22-q25.3 across the genes for growth hormone (GH) and thymidine kinase (TK) (Foster *et al., Genomics* **33**:185-192, 1996), the region surrounding the Gorlin syndrome gene (Obermayr *et al., Eur. J. Hum. Genet.* **4:**242-245, 1996), 60 loci covering the entire short arm of chromosome 12 (Raeymaekers *et al., Genomics* **29:**170-178, 1995), the region of human chromosome 22 containing the neurofibromatosis type 2 locus (Frazer *et al., Genomics* **14**:574-584, 1992) and 13 loci on the long arm of chromosome 5 (Warrington *et al., Genomics* **11**:701-708, 1991).

### EXAMPLE 53

### Mapping of 5'ESTs to HumanChromosomes using PCR techniques

5' ESTs (or cDNAs or genomic DNAs obtainable therefrom) may be assigned to human chromosomes using PCR based methodologies. In such approaches, oligonucleotide primer pairs are designed from the 5' ESTs (or cDNAs or genomic DNAs obtainable therefrom) to minimize the chance of amplifying through an intron. Preferably, the oligonucleotide primers are 18-23 bp in length and are designed for PCR amplification. The creation of PCR primers from known sequences is well known to those with skill in the art. For a review of PCR technology see Erlich in *PCR Technology, Principles and Applications for DNA Amplification,* Freeman and Co., New York, 1992, the disclosure of which is incorporated herein by reference.

The primers are used in polymerase chain reactions (PCR) to amplify templates from total human genomic DNA PCR conditions are as follows: 60 ng of genomic DNA is used as a template for PCR with 80 ng of each oligonucleotide primer, 0.6 unit of Taq polymerase, and 1 µCu of a ³²P-labeled deoxycytidine triphosphate. The PCR is performed in a microplate thermocycler (Techne) under the following conditions: 30 cycles of 94°C, 1.4 min; 55°C, 2 min; and 72°C, 2 min; with a final extension at 72°C for 10 min. The amplified products are analyzed on a 6% polyacrylamide sequencing gel and visualized by autoradiography. If the length of the resulting PCR product is identical to the distance between the ends of the primer sequences in the extended cDNA from which the primers are derived, then the PCR reaction is repeated with DNA templates from two panels of human-rodent somatic cell hybrids, BIOS PCRable DNA (BIOS Corporation) and NIGMS Human-Rodent Somatic Cell Hybrid Mapping Panel Number 1 (NIGMS, Camden, NJ).

PCR is used to screen a series of somatic cell hybrid cell lines containing defined sets of human chromosomes for the presence of a given 5' EST (or cDNA or genomic DNA obtainable therefrom). DNA is isolated from the somatic hybrids and used as starting templates for PCR_reactions using the primer pairs from the 5' EST (or cDNA or genomic DNA obtainable therefrom). Only those somatic cell hybrids with chromosomes containing the human gene corresponding to the 5' EST (or cDNA or genomic DNA obtainable therefrom) will yield an amplified fragment. The 5' EST (or cDNA or genomic DNA obtainable therefrom) are assigned to a chromosome by analysis of the segregation pattern of PCR products from the somatic hybrid DNA templates. The single human chromosome present in all cell hybrids that give rise to an amplified fragment is the chromosome containing that 5'EST (or cDNA or genomic DNA obtainable therefrom). For a review of techniques and analysis of results from somatic cell gene mapping experiments, see Ledbetter *et al., Genomics* **6**:475-481, 1990, the disclosure of which is incorporated herein by reference.

### EXAMPLE 54

### Mapping of Extended 5' ESTs to Chromosomes Using Fluorescence In Situ Hybridization

Fluorescence in situ hybridization allows the 5'EST (or cDNA or genomic DNA obtainable therefrom) to be mapped to a particular location on a given chromosome. The chromosomes to be used for fluorescence in situ hybridization techniques may be obtained from a variety of sources including cell cultures, tissues, or whole blood.

In a preferred embodiment, chromosomal localization of an 5'EST (or cDNA or genomic DNA obtainable therefrom) is obtained by FISH as described by *Cherif et al.* (*Proc. Natl. Acad. Sci. U.S.A*., **87**:6639-6643, 1990) , the disclosure of which is incorporated herein by reference.; Metaphase chromosomes are prepared from phytohemagglutinin (PHA)-stimulated blood cell donors. PHA-stimulated lymphocytes from healthy males are cultured for 72 h in RPMI-1640 medium. For synchronization, methotrexate (10 µM) is added for 17 h, followed by addition of 5-bromodeoxyuridine (5-BrdU, 0.1 mM) for 6 h. Colcemid (1 ug/ml) is added for the last 15 min before harvesting the cells. Cells are collected, washed in RPMI, incubated with a hypotonic solution of KCI (75 mM) at 37°C for 15 min and fixed in three changes ofmethanol:acetic acid (3:1). The cell suspension is dropped onto a glass slide and air dried. The 5'EST (or cDNA or genomic DNA obtainable therefrom) is labeled with biotin-16 dUTP by nick translation according to the manufacturer's instructions (Bethesda Research Laboratories, Bethesda, MD), purified using a Sephadex G-50 column (Phannacia, Upsala, Sweden) and precipitated. Just prior to hybridization, the DNA pellet is dissolved in hybridization buffer (50% formamide, 2 X SSC, 10% dextran sulfate, 1 mg/ml sonicated salmon sperm DNA, pH 7) and the probe is denatured at 70°C for 5-10 min.

Slides kept at -20°C are treated for 1 h at 37°C with RNase A (100 µg/ml), rinsed three times in 2 X SSC and dehydrated in an ethanol series. Chromosome preparations are denatured in 70% formamide, 2 X SSC for 2 min at 70°C, then dehydrated at 4°C. The slides are treated with proteinase K (10 µg/100 ml in 20 mM Tris-HCl, 2 mM CaCl₂) at 37°C for 8 min and dehydrated. The hybridization mixture containing the probe is placed on the slide, covered with a coverslip, sealed with rubber cement and incubated overnight in a humid chamber at 37°C. After hybridization and post-hybridization washes, the biotinylated probe is detected by avidin-FITC and amplified with additional layers of biotinylated goat anti-avidin and avidin-FlTC. For chromosomal localization, fluorescent R-bands are obtained as previously described (Cherif *et al., supra.).* The slides are observed under a LEICA fluorescence microscope (DMRXA). Chromosomes are counterstained with propidium iodide and the fluorescent signal of the probe appears as two symmetrical yellow-green spots on both chromatids of the fluorescent R-band chromosome (red). Thus, a particular 5'EST (or cDNA or genomic DNA obtainable therefrom) may be localized to a particular cytogenetic R-band on a given chromosome.

Once the 5'EST (or cDNA or genomic DNA obtainable therefrom) have been assigned to particular chromosomes using the techniques described in Examples 52-54 above, they may be utilized to construct a high resolution map of the chromosomes on which they are located or to identify the chromosomes in a sample.

### EXAMPLE 55

### Use of 5'EST to Construct or Expand Chromosome Maps

Chromosome mapping involves assigning a given unique sequence to a particular chromosome as described above. Once the unique sequence has been mapped to a given chromosome, it is ordered relative to other unique sequences located on the same chromosome. One approach to chromosome mapping utilizes a series of yeast artificial chromosomes (YACs) bearing several thousand long inserts derived from the chromosomes of the organism from which the extended cDNAs (or genomic DNAs obtainable therefrom) are obtained. This approach is described in Nagaraja *et al., Genome Research* **7**:210-222, 1997, the disclosure of which is incorporated herein by reference. Briefly, in this approach each chromosome is broken into overlapping pieces which are inserted into the YAC vector. The YAC inserts are screened using PCR or other methods to determine whether they include the 5'EST (or cDNA or genomic DNA obtainable therefrom) whose position is to be determined. Once an insert has been found which includes the 5'EST (or cDNA or genomic DNA obtainable therefrom), the insert can be analyzed by PCR or other methods to determine whether the insert also contains other sequences known to be on the chromosome or in the region from which the 5'EST (or cDNA or genomic DNA obtainable therefrom) was derived. This process can be repeated for each insert in the YAC library to determine the location of each of the extended cDNAs (or genomic DNAs obtainable therefrom) relative to one another and to other known chromosomal markers. In this way, a high resolution map of the distribution of numerous unique markers along each of the organisms chromosomes may be obtained.

As described in Example 56 below extended cDNAs (or genomic DNAs obtainable therefrom) may also be used to identify genes associated with a particular phenotype, such as hereditary disease or drug response.

### 3. Use of 5'ESTs or Sequences Obtained Therefrom or Fragments Thereof in Gene Identification

### EXAMPLE 56

### Identification of genes associated with hereditary diseases or drug response

This example illustrates an approach useful for the association of 5'ESTs (or cDNA or genomic DNA obtainable therefrom) with particular phenotypic characteristics. In this example, a particular 5'EST (or cDNA or genomic DNA obtainable therefrom) is used as a test probe to associate that 5'EST (or cDNA or genomic DNA obtainable therefrom) with a particular phenotypic characteristic.

5'ESTs (or cDNA or genomic DNA obtainable therefrom) are mapped to a particular location on a human chromosome using techniques such as those described in Examples 52 and 53 or other techniques known in the art. A search of Mendelian Inheritance in Man (McKusick in *Mendelian Inheritance in Man* (available on line through Johns Hopkins University Welch Medical Library) reveals the region of the human chromosome which contains the 5'EST (or cDNA or genomic DNA obtainable therefrom) to be a very gene rich region containing several known genes and several diseases or phenotypes for which genes have not been identified. The gene corresponding to this 5'EST (or cDNA or genomic DNA obtainable therefrom) thus becomes an immediate candidate for each of these genetic diseases.

Cells from patients with these diseases or phenotypes are isolated and expanded in culture. PCR primers from the 5'EST (or cDNA or genomic DNA obtainable therefrom) are used to screen genomic DNA, mRNA or cDNA obtained from the patients. 5'ESTs (or cDNA or genomic DNA obtainable therefrom) that are not amplified in the patients can be positively associated with a particular disease by further analysis. Alternatively, the PCR analysis may yield fragments of different lengths when the samples are derived from an individual having the phenotype associated with the disease than when the sample is derived from a healthy individual, indicating that the gene containing the 5'EST may be responsible for the genetic disease.

### VI. Use of 5'EST (or cDNA or Genomic DNA Obtainable Therefrom) to Construct Vectors

The present 5'ESTs (or cDNA or genomic DNA obtainable therefrom) may also be used to construct secretion vectors capable of directing the secretion of the proteins encoded by genes therein. Such secretion vectors may facilitate the purification or enrichment of the proteins encoded by genes inserted therein by reducing the number of background proteins from which the desired protein must be purified or enriched. Exemplary secretion vectors are described in Example 57 below.

### 1. Construction of Secretion Vectors

### EXAMPLE 57

### Construction of Secretion Vectors

The secretion vectors include a promoter capable of directing gene expression in the host cell, tissue, or organism of interest. Such promoters include the Rous Sarcoma Virus promoter, the SV40 promoter, the human cytomegalovirus promoter, and other promoters familiar to those skilled in the art.

A signal sequence from a 5' EST (or cDNAs or genomic DNAs obtainable therefrom) is operably linked to the promoter such that the mRNA transcribed from the promoter will direct the translation of the signal peptide. The host cell, tissue, or organism may be any cell, tissue, or organism which recognizes the signal peptide encoded by the signal sequence in the 5' EST (or cDNA or genomic DNA obtainable therefrom). Suitable hosts include mammalian cells, tissues or organisms, avian cells, tissues, or organisms, insect cells, tissues or organisms, or yeast.

In addition, the secretion vector contains cloning sites for inserting genes encoding the proteins which are to be secreted. The cloning sites facilitate the cloning of the insert gene in frame with the signal sequence such that a fusion protein in which the signal peptide is fused to the protein encoded by the inserted gene is expressed from the mRNA transcribed from the promoter. The signal peptide directs the extracellular secretion of the fusion protein.

The secretion vector may be DNA or RNA and may integrate into the chromosome of the host, be stably maintained as an extrachromosomal replicon in the host, be an artificial chromosome, or be transiently present in the host. Many nucleic acid backbones suitable for use as secretion vectors are known to those skilled in the art, including retroviral vectors, SV40 vectors, Bovine Papilloma Virus vectors, yeast integrating plasmids, yeast episomal plasmids, yeast artificial chromosomes, human artificial chromosomes, P element vectors, baculovirus vectors, or bacterial plasmids capable of being transiently introduced into the host.

The secretion vector may also contain a polyA signal such that the polyA signal is located downstream of the gene inserted into the secretion vector.

After the gene encoding the protein for which secretion is desired is inserted into the secretion vector, the secretion vector is introduced into the host cell, tissue, or organism using calcium phosphate precipitation, DEAE-Dextran, electroporation, liposome-mediated transfection, viral particles or as naked DNA. The protein encoded by the inserted gene is then purified or enriched from the supernatant using conventional techniques such as ammonium sulfate precipitation, immunoprecipitation, immunochromatography, size exclusion chromatography, ion exchange chromatography, and HPLC. Alternatively, the secreted protein may be in a sufficiently enriched or pure state in the supernatant or growth media of the host to permit it to be used for its intended purpose without further enrichment.

The signal sequences may also be inserted into vectors designed for gene therapy. In such vectors, the signal sequence is operably linked to a promoter such that mRNA transcribed from the promoter encodes the signal peptide. A cloning site is located downstream of the signal sequence such that a gene encoding a protein whose secretion is desired may readily be inserted into the vector and fused to the signal sequence. The vector is introduced into an appropriate host cell. The protein expressed from the promoter is secreted extracellularly, thereby producing a therapeutic effect.

The 5' ESTs may also be used to clone sequences located upstream of the 5' ESTs which are capable of regulating gene expression, including promoter sequences, enhancer sequences, and other upstream sequences which influence transcription or translation levels. Once identified and cloned, these upstream regulatory sequences may be used in expression vectors designed to direct the expression of an inserted gene in a desired spatial, temporal, developmental, or quantitative fashion. Example 58 describes a method for cloning sequences upstream of the extended cDNAs or 5' ESTs.

### 2. Identification of Upstream Sequences With Promoting or Regulatory Activities

### EXAMPLE 58

### Use of Extended cDNAs or 5' ESTs to Clone Upstream Sequences from Genomic DNA

Sequences derived from extended cDNAs or 5' ESTs may be used to isolate the promoters of the corresponding genes using chromosome walking techniques. In one chromosome walking technique, which utilizes the GenomeWalker™ kit available from Clontech, five complete genomic DNA samples are each digested with a different restriction enzyme which has a 6 base recognition site and leaves a blunt end. Following digestion, oligonucleotide adapters are ligated to each end of the resulting genomic DNA fragments.

For each of the five genomic DNA libraries, a first PCR reaction is performed according to the manufacturer's instructions (which are incorporated herein by reference) using an outer adaptor primer provided in the kit and an outer gene specific primer. The gene specific primer should be selected to be specific for the extended cDNA or 5' EST of interest and should have a melting temperature, length, and location in the extended cDNA or 5'EST which is consistent with its use in PCR reactions. Each first PCR reaction contains 5 ng of genomic DNA, 5 µl of 10X Tth reaction buffer, 0.2 mM of each dNTP, 0.2 µM each of outer adaptor primer and outer gene specific primer, 1.1 mM of Mg(OAc)₂, and 1 µl of the Tth polymerase 50X mix in a total volume of 50 µl. The reaction cycle for the first PCR reaction is as follows: 1 min - 94°C / 2 sec - 94°C, 3 min - 72°C (7 cycles) / 2 sec - 94°C, 3 min-67°C (32 cycles) / 5 min - 67°C.

The product of the first PCR reaction is diluted and used as a template for a second PCR reaction according to the manufacturer's instructions using a pair of nested primers which are located internally on the amplicon resulting from the first PCR reaction. For example, 5 µl of the reaction product of the first PCR reaction mixture may be diluted 180 times. Reactions are made in a 50 µl volume having a composition identical to that of the first PCR reaction except the nested primers are used. The first nested primer is specific for the adaptor, and is provided with the GenomeWalker^{TM} kit. The second nested primer is specific for the particular extended cDNA or 5' EST for which the promoter is to be cloned and should have a melting temperature, length, and location in the extended cDNA or 5' EST which is consistent with its use in PCR reactions. The reaction parameters of the second PCR reaction are as follows: 1 min - 94°C / 2 sec - 94°C, 3 min - 72°C (6 cycles) / 2 sec - 94°C, 3 min - 67°C (25 cycles) / 5 min - 67°C. The product of the second PCR reaction is purified, cloned, and sequenced using standard techniques.

Alternatively, two or more human genomic DNA libraries can be constructed by using two or more restriction enzymes. The digested genomic DNA is cloned into vectors which can be converted into single stranded, circular, or linear DNA. A biotinylated oligonucleotide comprising at least 15 nucleotides from the extended cDNA or 5' EST sequence is hybridized to the single stranded DNA. Hybrids between the biotinylated oligonucleotide and the single stranded DNA containing the extended cDNA or EST sequence are isolated as described in Example 29 above. Thereafter, the single stranded DNA containing the extended cDNA or EST sequence is released from the beads and converted into double stranded DNA using a primer specific for the extended cDNA or 5' EST sequence or a primer corresponding to a sequence included in the cloning vector. The resulting double stranded DNA is transformed into bacteria. DNAs containing the 5' EST or extended cDNA sequences are identified by colony PCR or colony hybridization.

Once the upstream genomic sequences have been cloned and sequenced as described above, prospective promoters and transcription start sites within the upstream sequences may be identified by comparing the sequences upstream of the extended cDNAs or 5' ESTs with databases containing known transcription start sites, transcription factor binding sites, or promoter sequences.

In addition, promoters in the upstream sequences may be identified using promoter reporter vectors as described in Example.

### EXAMPLE 59

### Identification of Promoters in Cloned Upstream Sequences

The genomic sequences upstream of the extended cDNAs or 5' ESTs are cloned into a suitable promoter reporter vector, such as the pSEAP-Basic, pSEAP-Enhancer, pβgal-Basic, pβgal-Enhancer, or pEGFP-1 Promoter Reporter vectors available from Clontech. Briefly, each of these promoter reporter vectors include multiple cloning sites positioned upstream of a reporter gene encoding a readily assayable protein such as secreted alkaline phosphatase, β galactosidase, or green fluorescent protein. The sequences upstream of the extended cDNAs or 5' ESTs are inserted into the cloning sites upstream of the reporter gene in both orientations and introduced into an appropriate host cell. The level of reporter protein is assayed and compared to the level obtained from a vector which lacks an insert in the cloning site. The presence of an elevated expression level in the vector containing the insert with respect to the control vector indicates the presence of a promoter in the insert. If necessary, the upstream sequences can be cloned into vectors which contain an enhancer for augmenting transcription levels from weak promoter sequences. A significant level of expression above that observed with the vector lacking an insert indicates that a promoter sequence is present in the inserted upstream sequence.

Appropriate host cells for the promoter reporter vectors may be chosen based on the results of the above described determination of expression patterns of the extended cDNAs and ESTs. For example, if the expression pattern analysis indicates that the mRNA corresponding to a particular extended cDNA or 5' EST is expressed in fibroblasts, the promoter reporter vector may be introduced into a human fibroblast cell line.

Promoter sequences within the upstream genomic DNA may be further defined by constructing nested deletions in the upstream DNA using conventional techniques such as Exonuclease III digestion. The resulting deletion fragments can be inserted into the promoter reporter vector to determine whether the deletion has reduced or obliterated promoter activity. In this way, the boundaries of the promoters may be defined. If desired, potential individual regulatory sites within the promoter may be identified using site directed mutagenesis or linker scanning to obliterate potential transcription factor binding sites within the promoter individually or in combination. The effects of these mutations on transcription levels may be determined by inserting the mutations into the cloning sites in the promoter reporter vectors.

### EXAMPLE 60

### Cloning and Identification of Promoters

Using the method described in Example 58 above with 5' ESTs, sequences upstream of several genes were obtained. Using the primer pairs GGG AAG ATG GAG ATA GTA TTG CCT G (SEQ ID NO:29) and CTG CCA TGT ACA TGA TAG AGA GAT TC (SEQ ID NO:30), the promoter having the internal designation PI3H2 (SEQ ID NO:31) was obtained.

Using the primer pairs GTA CCA GGGG ACT GTG ACC ATT GC (SEQ ID NO:32) and CTG TGA CCA TTG CTC CCA AGA GAG (SEQ ID NO:33), the promoter having the internal designation P15B4 (SEQ ID NO.34) was obtained.

Using the primer pairs CTG GGA TGG AAG GCA CGG TA (SEQ ID NO:35) and GAG ACC ACA CAG CTA GAC AA (SEQ ID NO:36), the promoter having the internal designation P29B6 (SEQ ID NO:37) was obtained.

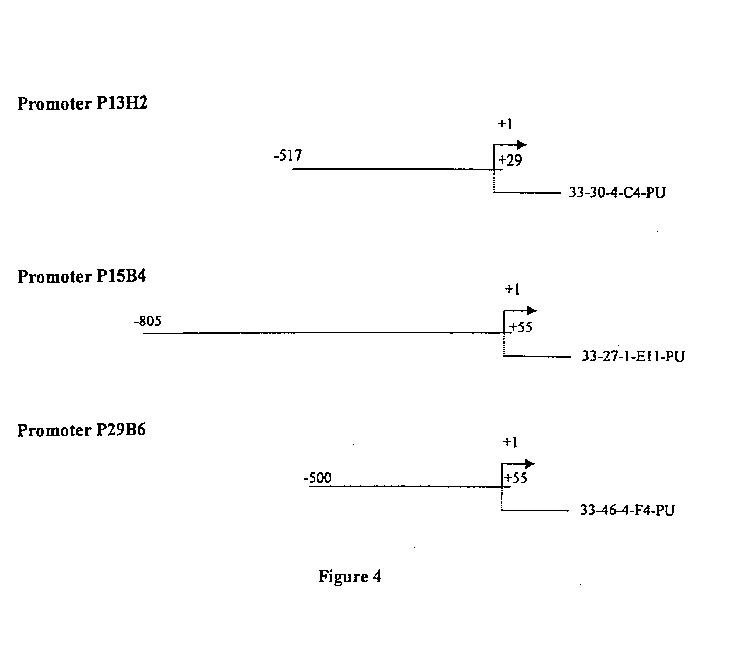

Figure 4 provides a schematic description of the promoters isolated and the way they are assembled with the corresponding 5' tags. The upstream sequences were screened for the presence of motifs resembling transcription factor binding sites or known transcription start sites using the computer program MatInspector release 2.0, August 1996.

Table VII describes the transcription factor binding sites present in each of these promoters. The columns labeled matrice provides the name of the MatInspector matrix used. The column labeled position provides the 5' position of the promoter site. Numeration of the sequence starts from the transcription site as determined by matching the genomic sequence with the 5' EST sequence. The column labeled "orientation" indicates the DNA strand on which the site is found, with the + strand being the coding strand as determined by matching the genomic sequence with the sequence of the 5' EST. The column labeled "score" provides the MatInspector score found for this site. The column labeled "length" provides the length of the site in nucleotides. The column labeled "sequence" provides the sequence of the site found.

Bacterial clones containing plasmids containing the promoter sequences described above described above are presently stored in the inventor's laboratories under the internal identification numbers provided above. The inserts may be recovered from the deposited materials by growing an aliquot of the appropriate bacterial clone in the appropriate medium. The plasmid DNA can then be isolated using plasmid isolation procedures familiar to those skilled in the art such as alkaline lysis minipreps or large scale alkaline lysis plasmid isolation procedures. If desired the plasmid DNA may be further enriched by centrifugation on a cesium chloride gradient, size exclusion chromatography, or anion exchange chromatography. The plasmid DNA obtained using these procedures may then be manipulated using standard cloning techniques familiar to those skilled in the art. Alternatively, a PCR can be done with primers designed at both ends of the EST insertion. The PCR product which corresponds to the 5' EST can then be manipulated using standard cloning techniques familiar to those skilled in the art.

The promoters and other regulatory sequences located upstream of the extended cDNAs or 5' ESTs may be used to design expression vectors capable of directing the expression of an inserted gene in a desired spatial, temporal, developmental, or quantitative manner. A promoter capable of directing the desired spatial, temporal, developmental, and quantitative patterns may be selected using the results of the expression analysis described in Example 26 above. For example, if a promoter which confers a high level of expression in muscle is desired, the promoter sequence upstream of an extended cDNA or 5' EST derived from an mRNA which is expressed at a high level in muscle, as determined by the method of Example 26, may be used in the expression vector.

Preferably, the desired promoter is placed near multiple restriction sites to facilitate the cloning of the desired insert downstream of the promoter, such that the promoter is able to drive expression of the inserted gene. The promoter may be inserted in conventional nucleic acid backbones designed for extrachromosomal replication, integration into the host chromosomes or transient expression. Suitable backbones for the present expression vectors include retroviral backbones, backbones from eukaryotic episomes such as SV40 or Bovine Papilloma Virus, backbones from bacterial episomes, or artificial chromosomes.

Preferably, the expression vectors also include a polyA signal downstream of the multiple restriction sites for directing the polyadenylation of mRNA transcribed from the gene inserted into the expression vector.

Following the identification of promoter sequences using the procedures of Examples 58-60, proteins which interact with the promoter may be identified as described in Example 61 below.

### EXAMPLE 61

### Identification of Proteins Which Interact with Promoter Sequences Upstream Regulatory Sequences, or mRNA

Sequences within the promoter region which are likely to bind transcription factors may be identified by homology to known transcription factor binding sites or through conventional mutagenesis or deletion analyses of reporter plasmids containing the promoter sequence. For example, deletions may be made in a reporter plasmid containing the promoter sequence of interest operably linked to an assayable reporter gene. The reporter plasmids carrying various deletions within the promoter region are transfected into an appropriate host cell and the effects of the deletions on expression levels is assessed. Transcription factor binding sites within the regions in which deletions reduce expression levels may be further localized using site directed mutagenesis, linker scanning analysis, or other techniques familiar to those skilled in the art.

Nucleic acids encoding proteins which interact with sequences in the promoter may be identified using one-hybrid systems such as those described in the manual accompanying the Matchmaker One-Hybrid System kit available from Clontech (Catalog No. K1603-1), the disclosure of which is incorporated herein by reference. Briefly, the Matchmaker One-hybrid system is used as follows. The target sequence for which it is desired to identify binding proteins is cloned upstream of a selectable reporter gene and integrated into the yeast genome. Preferably, multiple copies of the target sequences are inserted into the reporter plasmid in tandem. A library comprised of fusions between cDNAs to be evaluated for the ability to bind to the promoter and the activation domain of a yeast transcription factor, such as GAL4, is transformed into the yeast strain containing the integrated reporter sequence. The yeast are plated on selective media to select cells expressing the selectable marker linked to the promoter sequence. The colonies which grow on the selective media contain genes encoding proteins which bind the target sequence. The inserts in the genes encoding the fusion proteins are further characterized by sequencing. In addition, the inserts may be inserted into expression vectors or *in vitro* transcription vectors. Binding of the polypeptides encoded by the inserts to the promoter DNA may be confirmed by techniques familiar to those skilled in the art, such as gel shift analysis or DNAse protection analysis.

### VII. Use of 5' ESTs (or cDNAs or Genomic DNAs Obtainable Therefrom) in Gene Therapy

The present invention also comprises the use of 5'ESTs (or cDNA or genomic DNA obtainable therefrom) in gene therapy strategies, including antisense and triple helix strategies as described in Examples 62 and 63 below. In antisense approaches, nucleic acid sequences complementary to an mRNA are hybridized to the mRNA intracellularly, thereby blocking the expression of the protein encoded by the mRNA. The antisense sequences may prevent gene expression through a variety of mechanisms. For example, the antisense sequences may inhibit the ability of ribosomes to translate the mRNA. Alternatively, the antisense sequences may block transport of the mRNA from the nucleus to the cytoplasm, thereby limiting the amount of mRNA available for translation. Another mechanism through which antisense sequences may inhibit gene expression is by interfering with mRNA splicing. In yet another strategy, the antisense nucleic acid may be incorporated in a ribozyme capable of specifically cleaving the target mRNA.

### EXAMPLE 62

### Preparation and Use of Antisense Oligonucleotides

The antisense nucleic acid molecules to be used in gene therapy may be either DNA or RNA sequences. They may comprise a sequence complementary to the sequence of the 5'EST (or cDNA or genomic DNA obtainable therefrom). The antisense nucleic acids should have a length and melting temperature sufficient to permit formation of an intracellular duplex with sufficient stability to inhibit the expression of the mRNA in the duplex. Strategies for designing antisense nucleic acids suitable for use in gene therapy are disclosed in Green *et al., Arm. Rev. Biochem.* **55**:569-597, 1986; and Izant and Weintraub, *Cell* **36**:1007-1015, 1984, which are hereby incorporated by reference.

In some strategies, antisense molecules are obtained from a nucleotide sequence encoding a protein by reversing the orientation of the coding region with respect to a promoter so as to transcribe the opposite strand from that which is normally transcribed in the cell. The antisense molecules may be transcribed using *in vitro* transcription systems such as those which employ T7 or SP6 polymerase to generate the transcript. Another approach involves transcription of the antisense nucleic acids *in vivo* by operably linking DNA containing the antisense sequence to a promoter in an expression vector.

Alternatively, oligonucleotides which are complementary to the strand normally transcribed in the cell may be synthesized *in vitro.* Thus, the antisense nucleic acids are complementary to the corresponding mRNA and are capable of hybridizing to the mRNA to create a duplex. In some embodiments, the antisense sequences may contain modified sugar phosphate backbones to increase stability and make them less sensitive to RNase activity. Examples of modifications suitable for use in antisense strategies are described by Rossi *et al., Pharmacol. Ther.* **50(2)**:245-254, 1991, which is hereby incorporated by reference.

Various types of antisense oligonucleotides complementary to the sequence of the 5'EST (or cDNA or genomic DNA obtainable therefrom) may be used. In one preferred embodiment, stable and semi-stable antisense oligonucleotides described in International Application No. PCT WO94/23026, hereby incorporated by reference, are used. In these molecules, the 3' end or both the 3' and 5' ends are engaged in intramolecular hydrogen bonding between complementary base pairs. These molecules are better able to withstand exonuclease attacks and exhibit increased stability compared to conventional antisense oligonucleotides.

In another preferred embodiment, the antisense oligodeoxynucleotides against herpes simplex virus types 1 and 2 described in International Application No. WO 95/04141, hereby incorporated by reference, are used.

In yet another preferred embodiment, the covalently cross-linked antisense oligonucleotides described in International Application No. WO 96/31523, hereby incorporated by reference, are used. These double- or single-stranded oligonucleotides comprise one or more, respectively, inter- or intra-oligonucleotide covalent cross-linkages, wherein the linkage consists of an amide bond between a primary amine group of one strand and a carboxyl group of the other strand or of the same strand, respectively, the primary amine group being directly substituted in the 2' position of the strand nucleotide monosaccharide ring, and the carboxyl group being carried by an aliphatic spacer group substituted on a nucleotide or nucleotide analog of the other strand or the same strand, respectively.

The antisense oligodeoxynucleotides and oligonucleotides disclosed in International Application No. WO 92/18522, incorporated by reference, may also be used. These molecules are stable to degradation and contain at least one transcription control recognition sequence which binds to control proteins and are effective as decoys therefore. These molecules may contain "hairpin" structures, "dumbbell" structures, "modified dumbbell" structures, "cross-linked" decoy structures and "loop" structures.

In another preferred embodiment, the cyclic double-stranded oligonucleotides described in European Patent Application No. 0 572 287 A2, hereby incorporated by reference are used. These ligated oligonucleotide "dumbbells" contain the binding site for a transcription factor and inhibit expression of the gene under control of the transcription factor by sequestering the factor.

Use of the closed antisense oligonucleotides disclosed in International Application No. WO 92/19732, hereby incorporated by reference, is also contemplated. Because these molecules have no free ends, they are more resistant to degradation by exonucleases than are conventional oligonucleotides. These oligonucleotides may be multifunctional, interacting with several regions which are not adjacent to the target mRNA.

The appropriate level of antisense nucleic acids required to inhibit gene expression may be determined using *in vitro* expression analysis. The antisense molecule may be introduced into the cells by diffusion, injection, infection, transfection or h-region-mediated import using procedures known in the art. For example, the antisense nucleic acids can be introduced into the body as a bare or naked oligonucleotide, oligonucleotide encapsulated in lipid, oligonucleotide sequence encapsidated by viral protein, or as an oligonucleotide operably linked to a promoter contained in an expression vector. The expression vector may be any of a variety of expression vectors known in the art, including retroviral or viral vectors, vectors capable of extrachromosomal replication, or integrating vectors. The vectors may be DNA or RNA.

The antisense molecules are introduced onto cell samples at a number of different concentrations preferably between 1x10⁻¹⁰M to 1x10⁻¹M. Once the minimum concentration that can adequately control gene expression is identified, the optimized dose is translated into a dosage suitable for use *in vivo.* For example, an inhibiting concentration in culture of 1x10⁻⁷ translates into a dose of approximately 0.6 mg/kg bodyweight. Levels of oligonucleotide approaching 100 mg/kg bodyweight or higher may be possible after testing the toxicity of the oligonucleotide in laboratory animals. It is additionally contemplated that cells from the vertebrate are removed, treated with the antisense oligonucleotide, and reintroduced into the vertebrate.

It is further contemplated that the antisense oligonucleotide sequence is incorporated into a ribozyme sequence to enable the antisense to specifically bind and cleave its target mRNA. For technical applications of ribozyme and antisense oligonucleotides see Rossi *et al., supra.*

In a preferred application of this invention, the polypeptide encoded by the gene is first identified, so that the effectiveness of antisense inhibition on translation can be monitored using techniques that include but are not limited to antibody-mediated tests such as RIAs and ELISA, functional assays, or radiolabeling.

The 5' ESTs of the present invention (or cDNAs or genomic DNAs obtainable therefrom) may also be used in gene therapy approaches based on intracellular triple helix formation. Triple helix oligonucleotides are used to inhibit transcription from a genome. They are particularly useful for studying alterations in cell activity as it is associated with a particular gene. The 5' EST sequences (or cDNAs or genomic DNAs obtainable therefrom) of the present invention or, more preferably, a portion of those sequences, can be used to inhibit gene expression in individuals having diseases associated with expression of a particular gene. Similarly, a portion of 5' EST sequences (or cDNAs or genomic DNAs obtainable therefrom) can be used to study the effect of inhibiting transcription of a particular gene within a cell. Traditionally, homopurine sequences were considered the most useful for triple helix strategies. However, homopyrimidine sequences can also inhibit gene expression. Such homopyrimidine oligonucleotides bind to the major groove at homopurine:homopyrimidine sequences. Thus, both types of sequences from the 5'EST or from the gene corresponding to the 5'EST are contemplated within the scope of this invention.

### EXAMPLE 63

### Preparation and Use of Triple Helix Probes

The sequences of the 5' ESTs (or cDNAs or genomic DNAs obtainable therefrom) are scanned to identify 10-mer to 20-mer homopyrimidine or homopurine stretches which could be used in triple-helix based strategies for inhibiting gene expression. Following identification of candidate homopyrimidine or homopurine stretches, their efficiency in inhibiting gene expression is assessed by introducing varying amounts of oligonucleotides containing the candidate sequences into tissue culture cells which normally express the target gene. The oligonucleotides may be prepared on an oligonucleotide synthesizer or they may be purchased commercially from a company specializing in custom oligonucleotide synthesis, such as GENSET, Paris, France.

The oligonucleotides may be introduced into the cells using a variety of methods known to those skilled in the art, including but not limited to calcium phosphate precipitation, DEAE-Dextran, electroporation, liposome-mediated transfection or native uptake.

Treated cells are monitored for altered cell function or reduced gene expression using techniques such as Northern blotting, RNase protection assays, or PCR based strategies to monitor the transcription levels of the target gene in cells which have been treated with the oligonucleotide. The cell functions to be monitored are predicted based upon the homologies of the target gene corresponding to the extended cDNA from which the oligonucleotide was derived with known gene sequences that have been associated with a particular function. The cell functions can also be predicted based on the presence of abnormal physiologies within cells derived from individuals with a particular inherited disease, particularly when the extended cDNA is associated with the disease using techniques described in Example 56.

The oligonucleotides which are effective in inhibiting gene expression in tissue culture cells may then be introduced *in vivo* using the techniques described above and in Example 62 at a dosage calculated based on the *in vitro* results, as described in Example 62.

In some embodiments, the natural (beta) anomers of the oligonucleotide units can be replaced with alpha anomers to render the oligonucleotide more resistant to nucleases. Further, an intercalating agent such as ethidium bromide, or the like, can be attached to the 3' end of the alpha oligonucleotide to stabilize the triple helix. For information on the generation of oligonucleotides suitable for triple helix formation see Griffin *et al., Science* **245**:967-971, 1989, which is hereby incorporated by this reference.

### EXAMPLE 64

### Use of cDNAs Obtained Using the 5' ESTs to Express an Encoded Protein in a Host Organism

The cDNAs obtained as described above using the 5' ESTs of the present invention may also be used to express an encoded protein in a host organism to produce a beneficial effect. In such procedures, the encoded protein may be transiently expressed in the host organism or stably expressed in the host organism. The encoded protein may have any of the activities described above. The encoded protein may be a protein which the host organism lacks or, alternatively, the encoded protein may augment the existing levels of the protein in the host organism.

A full length extended cDNA encoding the signal peptide and the mature protein, or an extended cDNA encoding only the mature protein is introduced into the host organism. The extended cDNA may be introduced into the host organism using a variety of techniques known to those of skill in the art. For example, the extended cDNA may be injected into the host organism as naked DNA such that the encoded protein is expressed in the host organism, thereby producing a beneficial effect.

Alternatively, the extended cDNA may be cloned into an expression vector downstream of a promoter which is active in the host organism. The expression vector may be any of the expression vectors designed for use in gene therapy, including viral or retroviral vectors. The expression vector may be directly introduced into the host organism such that the encoded protein is expressed in the host organism to produce a beneficial effect. In another approach, the expression vector may be introduced into cells *in vitro.* Cells containing the expression vector are thereafter selected and introduced into the host organism, where they express the encoded protein to produce a beneficial effect.

### EXAMPLE 65

### Use of Signal Peptides Encoded by 5' ESTs or Sequences obtained Therefrom to Import Proteins Into Cells

The short core hydrophobic region (h) of signal peptides encoded by the 5'ESTS or extended cDNAs derived from SEQ ID NOs: 38-305 may also be used as a carrier to import a peptide or a protein of interest, so-called cargo, into tissue culture cells (Lin *et al., J. Biol. Chem.,* **270**: 14225-14258, 1995; Du *et al., J. Peptide Res.,* **51**: 235-243, 1998; Rojas *et al., Nature Biotech.,* **16**: 370-375, 1998).

When cell permeable peptides of limited size (approximately up to 25 amino acids) are to be translocated across cell membrane, chemical synthesis may be used in order to add the h region to either the C-terminus or the N-terminus to the cargo peptide of interest. Alternatively, when longer peptides or proteins are to be imported into cells, nucleic acids can be genetically engineered, using techniques familiar to those skilled in the art, in order to link the extended cDNA sequence encoding the h region to the 5' or the 3' end of a DNA sequence coding for a cargo polypeptide. Such genetically engineered nucleic acids are then translated either *in vitro* or *in vivo* after transfection into appropriate cells, using conventional techniques to produce the resulting cell permeable polypeptide. Suitable hosts cells are then simply incubated with the cell permeable polypeptide which is then translocated across the membrane.

This method may be applied to study diverse intracellular functions and cellular processes. For instance, it has been used to probe functionally relevant domains of intracellular proteins and to examine protein-protein interactions involved in signal transduction pathways (Lin *et al., supra;* Lin *et al., J. Biol. Chem.,* **271**: 5305-5308, 1996; Rojas *et al., J. Biol. Chem.,* **271**: 27456-27461, 1996; Liu *et al., Proc. Natl. Acad. Sci. USA,* **93**:11819-11824, 1996; Rojas *et al., Bioch. Biophys*. *Res. Commun.,* **234**: 675-680, 1997).

Such techniques may be used in cellular therapy to import proteins producing therapeutic effects. For instance, cells isolated from a patient may be treated with imported therapeutic proteins and then re-introduced into the host organism.

Alternatively, the h region of signal peptides of the present invention could be used in combination with a nuclear localization signal to deliver nucleic acids into cell nucleus. Such oligonucleotides may be antisense oligonucleotides or oligonucleotides designed to form triple helixes, as described in examples 62 and 63 respectively, in order to inhibit processing and/or maturation of a target cellular RNA.

As discussed above, the cDNAs or portions thereof obtained using the 5' ESTs of the present invention can be used for various purposes. The polynucleotides can be used to express recombinant protein for analysis, characterization or therapeutic use; as markers for tissues in which the corresponding protein is preferentially expressed (either constitutively or at a particular stage of tissue differentiation or development or in disease states); as molecular weight markers on Southern gels; as chromosome markers or tags (when labeled) to identify chromosomes or to map related gene positions; to compare with endogenous DNA sequences in patients to identify potential genetic disorders; as probes to hybridize and thus discover novel, related DNA sequences; as a source of information to derive PCR primers for genetic fingerprinting; for selecting and making oligomers for attachment to a "gene chip" or other support, including for examination for expression patterns; to raise anti-protein antibodies using DNA immunization techniques; and as an antigen to raise anti-DNA antibodies or elicit another immune response. Where the polynucleotide encodes a protein which binds or potentially binds to another protein (such as, for example, in a receptor-ligand interaction), the polynucleotide can also be used in interaction trap assays (such as, for example, that described in Gyuris *et al., Cell* **75**:791-803, 1993, the disclosure of which is hereby incorporated by reference) to identify polynucleotides encoding the other protein with which binding occurs or to identify inhibitors of the binding interaction.

The proteins or polypeptides provided by the present invention can similarly be used in assays to determine biological activity, including in a panel of multiple proteins for high-throughput screening; to raise antibodies or to elicit another immune response; as a reagent (including the labeled reagent) in assays designed to quantitatively determine levels of the protein (or its receptor) in biological fluids; as markers for tissues in which the corresponding protein is preferentially expressed (either constitutively or at a particular stage of tissue differentiation or development or in a disease state); and, of course, to isolate correlative receptors or ligands. Where the protein binds or potentially binds to another protein (such as, for example, in a receptor-ligand interaction), the protein can be used to identify the other protein with which binding occurs or to identify inhibitors of the binding interaction. Proteins involved in these binding interactions can also be used to screen for peptide or small molecule inhibitors or agonists of the binding interaction.

Any or all of these research utilities are capable of being developed into reagent grade or kit format for commercialization as research products.

Methods for performing the uses listed above are well known to those skilled in the art. References disclosing such methods include without limitation *Molecular Cloning*; *A Laboratory Manual,* 2d ed., Cold Spring Harbor Laboratory Press, Sambrook, Fritsch and Maniatis eds., 1989, and *Methods in Enzymology; Guide to Molecular Cloning Technigues,* Academic Press, Berger and Kimmel eds., 1987.

Polynucleotides and proteins of the present invention can also be used as nutritional sources or supplements. Such uses include without limitation use as a protein or amino acid supplement, use as a carbon source, use as a nitrogen source and use as a source of carbohydrate. In such cases the protein or polynucleotide of the invention can be added to the feed of a particular organism or can be administered as a separate solid or liquid preparation, such as in the form of powder, pills, solutions, suspensions or capsules. In the case of microorganisms, the protein or polynucleotide of the invention can be added to the medium in or on which the microorganism is cultured.

Although this invention has been described in terms of certain preferred embodiments, other embodiments which will be apparent to those of ordinary skill in the art in view of the disclosure herein are also within the scope of this invention. Accordingly, the scope of the invention is intended to be defined only by reference to the appended claims. All documents cited herein are incorporated herein by reference in their entirety.

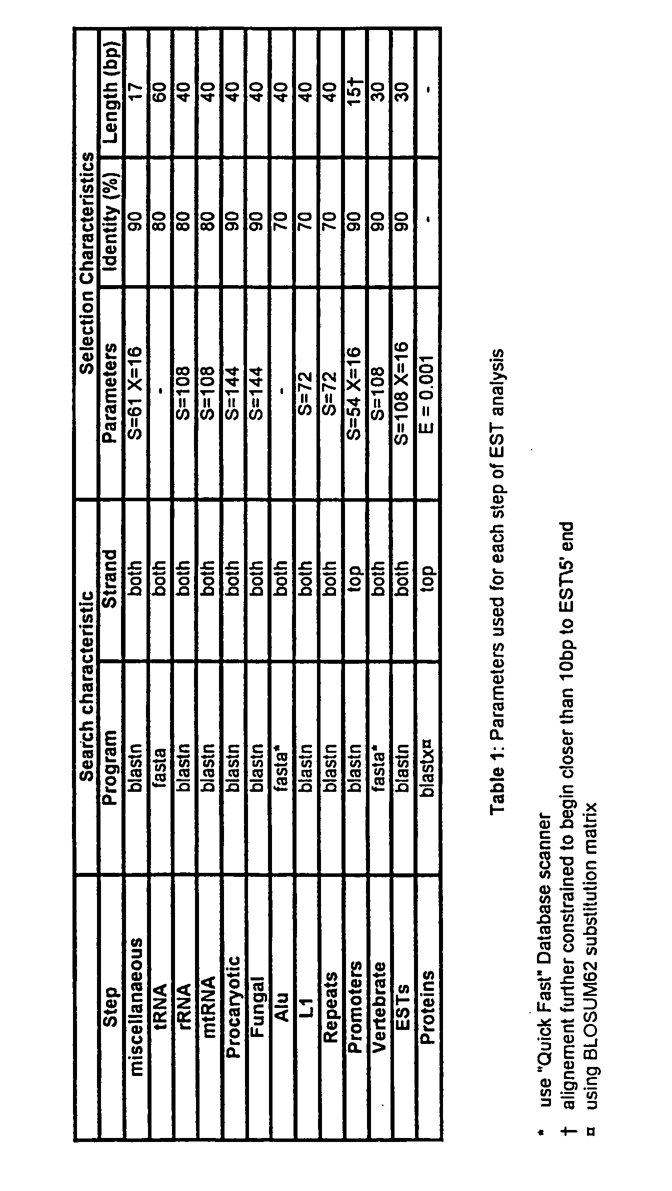

**TABLE ll**

| SEQ. ID NO. | CATEGORY | VON HEIJNE SCORE | TISSUE SOURCE | INTERNAL DESIGNATION |
|---|---|---|---|---|
| ID38 | new | 15.8 | Heart | 25-13-1-H10-PU |
| ID39 | new | 14 | Fetal kidney | 58-47-2-BI11-PU |
| ID40 | new | 12.3 | Dystrophic muscle | 29-3-3-H8-PU |
| ID41 | new | 12.2 | Fetal kidney | 58-4-2-A3-PU |
| ID42 | new | 11.9 | Kidney | 21-10-4-G1-PU |
| ID43 | new | 11.3 | Fetal kidney | 58-27-3-B10-PU |
| ID44 | new | 10.7 | Fetal kidney | 58-35-2-F10-PU |
| ID45 | new | 10.7 | Fetal kidney | 58-37-2-G10-PU |
| ID46 | new | 10.6 | Dystrophic muscle | 29-11-1-C11-PU |
| ID47 | new | 10 | Fetal kidney | 58-20-4-G7-PU |
| ID48 | new | 10 | Fetal kidney | 58-2-4-E9-PU |
| ID49 | new | 9.6 | Fetal kidney | 58-37-3-D8-PU |
| ID50 | new | 9.5 | Fetal kidney | 58-46-1-F1-PU |
| ID51 | new | 9.2 | Dystrophic muscle | 29-9-4-D8-PU |
| ID52 | new | 9.2 | Muscle | 27-10-4-C6-PU |
| ID53 | new | 8.3 | Heart | 67-5-4-H9-PU |
| ID54 | new | 8.1 | Fetal kidney | 58-4-3-H4-PU |
| ID55 | new | 8 | Muscle | 27-16-3-D12-PU |
| ID56 | new | 7.9 | Fetal kidney | 58-54-2-C2-PU |
| ID57 | new | 7.9 | Heart | 25-9-3-A3-PU |
| ID58 | new | 7.9 | Dystrophic muscle | 29-11-3-F1-PU |
| ID59 | new | 7.9 | Fetal kidney | 58-32-3-G6-PU |
| ID60 | new | 7.8 | Fetal kidney | 58-22-2-H8-PU |
| ID61 | new | 7.8 | Fetal kidney | 58-2-4-H4-PU |
| ID62 | new | 7.8 | Heart | 67-4-3-G3-PU |
| ID63 | new | 7.8 | Fetal kidney | 58-24-1-G11-PU |
| ID64 | new | 7.7 | Fetal kidney | 58-19-3-H1-PU |
| ID65 | new | 7.5 | Fetal kidney | 58-45-4-B11-PU |
| ID66 | new | 7.3 | Fetal kidney | 58-44-2-D3-PU |
| ID67 | new | 7.2 | Dystrophic muscle | 29-3-3-E7-PU |
| ID68 | new | 7.1 | Dystrophic muscle | 29-12-3-A3-PU |
| ID69 | new | 7.1 | Fetal kidney | 58-14-2-B3-PU |
| ID70 | new | 7.1 | Fetal kidney | 58-10-3-D12-PU |
| ID71 | new | 7 | Fetal kidney | 58-6-2-E5-PU |
| ID72 | new | 7 | Dystrophic muscle | 29-7-1-C1-PU |
| ID73 | new | 6.9 | Fetal kidney | 58-26-4-A12-PU |
| ID74 | new | 6.9 | Fetal kidney | 58-7-2-H9-PU |
| ID75 | new | 6.9 | Fetal kidney | 58-14-2-D5-PU |
| ID76 | new | 6.7 | Fetal kidney | 58-3-4-E1-PU |
| ID77 | new | 6.7 | Fetal kidney | 58-43-4-G3-PU |
| ID78 | new | 6.7 | Fetal kidney | 58-11-1-G10-PU |
| ID79 | new | 6.6 | Fetal kidney | 58-4-4-G2-PU |
| ID80 | new | 6.6 | Fetal kidney | 58-41-3-D6-PU |
| ID81 | new | 6.6 | Heart | 25-8-2-H10-PU |
| ID82 | new | 6.5 | Muscle | 27-18-4-E5-PU |
| ID83 | new | 6.4 | Dystrophic muscle | 29-4-1-G6-PU |
| ID84 | new | 6.4 | Muscle | 27-10-2-B1-PU |
| ID85 | new | 6.4 | Fetal kidney | 58-38-1-E5-PU |
| ID86 | new | 6.3 | Muscle | 27-4-3-D9-PU |
| ID87 | new | 6.3 | Fetal kidney | 58-53-1-G1-PU |
| ID88 | new | 6.3 | Fetal kidney | 58-7-3-F6-PU |
| ID89 | new | 6.3 | Heart | 25-7-2-B12-PU |
| ID90 | new | 6.1 | Fetal kidney | 58-16-3-E11-PU |
| ID91 | new | 6 | Fetal kidney | 58-15-4-C2-PU |
| ID92 | new | 6 | Fetal kidney | 58-34-3-A9-PU |
| ID93 | new | 5.9 | Fetal kidney | 58-16-1-E1-PU |
| ID94 | new | 5.9 | Fetal kidney | 58-4-3-E6-PU |
| ID95 | new | 5.9 | Fetal kidney | 58-37-3-B11-PU |
| ID96 | new | 5.9 | Fetal kidney | 58-35-3-C6-PU |
| ID97 | new | 5.8 | Fetal kidney | 58-35-1-D9-PU |
| ID98 | new | 5.8 | Fetal kidney | 58-26-3-B2-PU |
| ID99 | new | 5.7 | Fetal kidney | 58-48-1-F8-PU |
| ID100 | new | 5.7 | Fetal kidney | 58-27-4-A6-PU |
| ID101 | new | 5.7 | Fetal kidney | 58-26-3-D1-PU |
| ID102 | new | 5.7 | Muscle | 27-19-4-B4-PU |
| ID103 | new | 5.6 | Fetal kidney | 58-23-3-B2-PU |
| ID104 | new | 5.5 | Heart | 25-1-2-C1-PU |
| ID105 | new | 5.5 | Fetal kidney | 58-14-3-F10-PU |
| ID106 | new | 5.5 | Fetal kidney | 58-25-1-E11-PU |
| ID107 | new | 5.5 | Muscle | 27-9-4-A10-PU |
| ID108 | new | 5.5 | Heart | 25-4-2-D8-PU |
| ID109 | new | 5.4 | Fetal kidney | 58-29-3-G8-PU |
| ID110 | new | 5.4 | Fetal kidney | 58-4-4-E5-PU |
| ID111 | new | 5.4 | Fetal kidney | 58-24-2-H2-PU |
| ID112 | new | 5.4 | Muscle | 27-11-2-C8-PU |
| ID113 | new | 5.4 | Fetal kidney | 58-41-2-E3-PU |
| ID114 | new | 5.3 | Muscle | 27-22-1-G8-PU |
| ID115 | new | 5.3 | Dystrophic muscle | 29-1-1-C9-PU |
| ID116 | new | 5.3 | Fetal kidney | 58-22-2-A3-PU |
| ID117 | new | 5.2 | Fetal kidney | 58-42-2-G1-PU |
| ID118 | new | 5.2 | Fetal kidney | 58-52-2-E5-PU |
| ID119 | new | 5.2 | Fetal kidney | 58-24-2-G2-PU |
| ID120 | new | 5.2 | Fetal kidney | 58-29-1-A3-PU |
| ID121 | new | 5.1 | Fetal kidney | 58-26-1-G8-PU |
| ID122 | new | 5.1 | Fetal kidney | 58-29-4-G12-PU |
| ID123 | new | 5.1 | Dystrophic muscle | 29-8-3-E8-PU |
| ID124 | new | 5.1 | Dystrophic muscle | 29-3-4-C1-PU |
| ID125 | new | 5 | Fetal kidney | 58-17-2-H1-PU |
| ID126 | new | 5 | Fetal kidney | 58-9-3-E3-PU |
| ID127 | new | 5 | Muscle | 27-19-3-G7-PU |
| ID128 | new | 5 | Fetal kidney | 58-41-3-B4-PU |
| ID129 | new | 5 | Dystrophic muscle | 29-74-G7-PU |
| ID130 | new | 5 | Muscle | 27-9-3-D4-PU |
| ID131 | new | 4.9 | Kidney | 21-3-4-C5-PU |
| ID132 | new | 4.9 | Heart | 25-11-2-D6-PU |
| ID133 | new | 4.9 | Heart | 67-7-2-F3-PU |
| ID134 | new | 4.8 | Fetal kidney | 58-4-3-D3-PU |
| ID135 | new | 4.8 | Fetal kidney | 58-49-3-B5-PU |
| ID136 | new | 4.8 | Fetal kidney | 58-28-3-G12-PU |
| ID137 | new | 4.7 | Fetal kidney | 58-53-1-A5-PU |
| ID138 | new | 4.7 | Fetal kidney | 58-3-3-E10-PU |
| ID139 | new | 4.7 | Fetal kidney | 58-8-1-G7-PU |
| ID140 | new | 4.6 | Fetal kidney | 58-23-1-G9-PU |
| ID141 | new | 4.6 | Fetal kidney | 58-21-1-H8-PU |
| ID142 | new | 4.6 | Fetal kidney | 58-54-2-E10-PU |
| ID143 | new | 4.6 | Fetal kidney | 58-46-3-E4-PU |
| ID144 | new | 4.6 | Fetal kidney | 58-6-3-G3-PU |
| ID145 | new | 4.6 | Fetal kidney | 58-41-2-B5-PU |
| ID146 | new | 4.6 | Dystrophic muscle | 29-7-3-F2-PU |
| ID147 | new | 4.5 | Fetal kidney | 58-2-4-G12-PU |
| ID148 | new | 4.5 | Fetal kidney | 58-11-2-G8-PU |
| ID149 | new | 4.4 | Fetal kidney | 58-17-1-C4-PU |
| ID150 | new | 4.4 | Fetal kidney | 58-46-1-G7-PU |
| ID151 | new | 4.4 | Heart | 67-3-2-F4-PU |
| ID152 | new | 4.4 | Fetal kidney | 58-8-4-E12-PU |
| ID153 | new | 4.4 | Fetal kidney | 58-4-2-D9-PU |
| ID154 | new | 4.4 | Fetal kidney | 58-25-1-B5-PU |
| ID155 | new | 4.4 | Fetal kidney | 58-15-1-C10-PU |
| ID156 | new | 4.3 | Dystrophic muscle | 29-4-4-A10-PU |
| ID157 | new | 4.3 | Fetal kidney | 58-32-3-H7-PU |
| ID158 | new | 4.3 | Kidney | 21-4-4-D12-PU |
| ID159 | new | 4.3 | Fetal kidney | 58-45-4-G9-PU |
| ID160 | new | 4.3 | Fetal kidney | 58-1-2-E2-PU |
| ID161 | new | 4.2 | Fetal kidney | 58-25-4-E6-PU |
| ID162 | new | 4.2 | Fetal kidney | 58-36-1-C6-PU |
| ID163 | new | 4.2 | Drstrophic muscle | 29-9-3-D5-PU |
| ID164 | new | 4.2 | Fetal kidney | 58-3-3-B8-PU |
| ID165 | new | 4.2 | Heart | 25-4-4-B4-PU |
| ID166 | new | 4.2 | Kidney | 21-10-3-A3-PU |
| ID167 | new | 4.2 | Muscle | 27-19-4-B5-PU |
| ID168 | new | 4.2 | Fetal kidney | 58-23-3-D10-PU |
| ID169 | new | 4.1 | Fetal kidney | 58-41-1-F8-PU |
| ID170 | new | 4.1 | Heart | 25-7-2-B1-PU |
| ID 171 | new | 4.1 | Fetal kidney | 58-53-3-G4-PU |
| ID172 | new | 4.1 | Fetal kidney | 58-52-2-C2-PU |
| ID173 | new | 4 | Muscle | 27-21-4-E12-PU |
| ID174 | new | 4 | Fetal kidney | 58-22-2-B8-PU |
| ID175 | new | 4 | Fetal kidney | 58-9-3-A8-PU |
| ID176 | new | 4 | Muscle | 27-5-4-C10-PU |
| ID177 | new | 4 | Fetal kidney | 58-38-1-G5-PU |
| ID178 | new | 4 | Fetal kidney | 58-34-4-F6-PU |
| ID179 | new | 4 | Heart | 25-1-4-D2-PU |
| ID180 | new | 4 | Fetal kidney | 58-48-2-D6-PU |
| ID181 | new | 3.9 | Fetal kidney | 58-9-3-C10-PU |
| ID182 | new | 3.9 | Fetal kidney | 58-9-4-F2-PU |
| ID183 | new | 3.9 | Fetal kidney | 58-32-3-G3-PU |
| ID184 | new | 3.9 | Fetal kidney | 58-52-1-F6-PU |
| ID185 | new | 3.9 | Fetal kidney | 58-29-1-E1-PU |
| ID186 | new | 3.9 | Muscle | 27-3-4-A3-PU |
| ID187 | new | 3.9 | Muscle | 27-16-3-H2-PU |
| ID188 | new | 3.9 | Fetal kidney | 58-1-3-E1-PU |
| ID189 | new | 3.8 | Kidney | 21-5-4-F10-PU |
| ID190 | new | 3.8 | Kidney | 21-1-3-C9-PU |
| ID191 | new | 3.8 | Fetal kidney | 58-1-2-C7-PU |
| ID192 | new | 3.8 | Fetal kidney | 58-10-3-B6-PU |
| ID193 | new | 3.8 | Fetal kidney | 58-11-4-C |
| ID194 | new | 3.8 | Heart | 67-6-4-B12-PU |
| ID195 | new | 3.8 | Fetal kidney | 58-7-3-B5-PU |
| ID196 | new | 3.8 | Fetal kidney | 58-46-3-C6-PU |
| ID197 | new | 3.7 | Dystrophic muscle | 29-2-4-D8-PU |
| ID198 | new | 3.7 | Fetal kidney | 58-7-1-D10-PU |
| ID199 | new | 3.7 | Kidney | 21-2-4-A11-PU |
| ID200 | new | 3.6 | Fetal kidney | 58-45-3-B7-PU |
| ID201 | new | 3.6 | Fetal kidney | 58-29-1-D7-PU |
| ID202 | new | 3.6 | Fetal kidney | 58-16-3-B3-PU |
| ID203 | new | 3.6 | Dystrophic muscle | 29-7-3-C3-PU |
| ID204 | new | 3.6 | Fetal kidney | 58-42-3-C2-PU |
| ID205 | new | 3.5 | Fetal kidney | 58-38-3-G8-PU |
| ID206 | new | 3.5 | Dystrophic muscle | 29-6-2-B12-PU |
| ID207 | new | 3.5 | Fetal kidney | 58-8-1-D1-PU |
| ID208 | new | 3.5 | Fetal kidney | 58-24-1-H2-PU |
| ID209 | new | 3.5 | Fetal kidney | 58-41-4-G9-PU |
| ID210 | ext-est-not-vrt | 12.7 | Muscle | 27-22-3-H1-PU |
| ID211 | ext-est-not-vrt | 10.5 | Fetal kidney | 58-29-1-F11-PU |
| ID212 | ext-est-not-vrt | 8 | Fetal kidney | 58-14-2-B12-PU |
| ID213 | ext-est-not-vrt | 7.7 | Fetal kidney | 58-5-1-C4-PU |
| ID214 | ext-est-not-vrt | 7.1 | Fetal kidney | 58-37-4-C7-PU |
| ID215 | ext-est-not-vrt | 6.7 | Muscle | 27-21-2-C8-PU |
| ID216 | ext-est-not-vrt | 6.7 | Heart | 67-1-1-C8-PU |
| ID217 | ext-est-not-vrt | 6.3 | Fetal kidney | 58-26-3-G6-PU |
| ID218 | ext-est-not-vrt | 6.2 | Fetal kidney | 58-15-3-B12-PU |
| ID219 | ext-est-not-vrt | 6 | Muscle | 27-5-2-G11-PU |
| ID220 | ext-est-not-vrt | 6 | Fetal kidney | 58-8-1-H10-PU |
| ID221 | ext-est-not-vrt | 5.8 | Fetal kidney | 58-38-4-D2-PU |
| ID222 | ext-est-not-vrt | 5.6 | Fetal kidney | 58-53-2-E6-PU |
| ID223 | ext-est-not-vrt | 5.6 | Fetal kidney | 58-52-2-C7-PU |
| ID224 | ext-est-not-vrt | 5.5 | Fetal kidney | 58-34-2-E7-PU |
| ID225 | ext-est-not-vrt | 5.4 | Fetal kidney | 58-4-1-A2-PU |
| ID226 | ext-est-not-vrt | 5.2 | Fetal kidney | 58-11-1-D3-PU |
| ID227 | ext-est-not-vrt | 5.2 | Fetal kidney | 58-34-3-C9-PU |
| ID228 | ext-est-not-vrt | 5.2 | Fetal kidney | 58-35-4-H11-PU |
| ID229 | ext-est-not-vrt | 4.6 | Fetal kidney | 58-3-4-H7-PU |
| ID230 | ext-est-not-vrt | 4.5 | Fetal kidney | 58-25-1-F3-PU |
| ID231 | ext-est-not-vrt | 4.5 | Fetal kidney | 58-4-4-A8-PU |
| ID232 | ext-est-not-vrt | 4.4 | Fetal kidney | 58-11-1-C1-PU |
| ID233 | ext-est-not-vrt | 3.9 | Muscle | 27-19-2-F5-PU |
| ID234 | ext-est-not-vrt | 3.5 | Dystrophic muscle | 29-2-2-A2-PU |
| ID235 | est-not-ext | 14.1 | Fetal kidney | 58-29-2-B9-PU |
| ID236 | est-not-ext | 11.4 | Dystrophic muscle | 29-11-2-E4-PU |
| ID237 | est-not-ext | 11.2 | Fetal kidney | 58-7-2-A7-PU |
| ID238 | est-not-ext | 10.8 | Muscle | 27-22-3-G4-PU |
| ID239 | est-not-ext | 9.9 | Fetal kidney | 58-9-1-G1-PU |
| ID240 | est-not-ext | 9.7 | Dystrophic muscle | 29-8-1-H5-PU |
| ID241 | est-not-ext | 9.6 | Fetal kidney | 58-40-1-F5-PU |
| ID242 | est-not-ext | 9.5 | Fetal kidney | 58-6-4-G2-PU |
| ID243 | est-not-ext | 9.2 | Fetal kidney | 58-25-2-E7-PU |
| ID244 | est-not-ext | 8.9 | Fetal kidney | 58-48-1-A11-PU |
| ID245 | est-not-ext | 8.8 | Fetal kidney | 58-35-2-B6-PU |
| ID246 | est-not-ext | 8.5 | Kidney | 21-7-4-C7-PU |
| ID247 | est-not-ext | 8.4 | Fetal kidney | 58-45-1-E6-PU |
| ID248 | est-not-ext | 8.1 | Fetal kidney | 58-39-1-A12-PU |
| ID249 | est-not-ext | 8 | Fetal kidney | 58-46-1-C7-PU |
| ID250 | est-not-ext | 7.9 | Dystrophic muscle | 29-12-3-E10-PU |
| ID251 | est-not-ext | 7.9 | Fetal kidney | 58-17-2-D9-PU |
| ID252 | est-not-ext | 7.9 | Fetal kidney | 58-52-3-B7-PU |
| ID253 | est-not-ext | 7.6 | Fetal kidney | 58-24-3-E7-PU |
| ID254 | est-not-ext | 7.6 | Heart | 25-8-4-B12-PU |
| ID255 | est-not-ext | 7.6 | Dystrophic muscle | 29-4-4-D12-PU |
| ID256 | est-not-ext | 7.4 | Muscle | 27-1-2-B3-PU |
| ID257 | est-not-ext | 7.3 | Fetal kidney | 58-48-1-G3-PU |
| ID258 | est-not-ext | 7.3 | Dystrophic muscle | 29-2-3-F8-PU |
| ID259 | est-not-ext | 7.2 | Fetal kidney | 58-19-3-B3-PU |
| ID260 | est-not-ext | 7 | Fetal kidney | 58-14-2-C4-PU |
| ID261 | est-not-ext | 6.7 | Fetal kidney | 58-16-3-B6-PU |
| ID262 | est-not-ext | 6.6 | Fetal kidney | 58-9-4-F6-PU |
| ID263 | est-not-ext | 6.4 | Fetal kidney | 58-1-1-E3-PU |
| ID264 | est-not-ext | 6.4 | Fetal kidney | 58-33-3-B4-PU |
| ID265 | est-not-ext | 6.3 | Dystrophic muscle | 29-12-1-H1-PU |
| ID266 | est-not-ext | 6.3 | Muscle | 27-9-3-A5-PU |
| ID267 | est-not-ext | 6.2 | Muscle | 27-17-4-C12-PU |
| ID268 | est-not-ext | 6.2 | Fetal kidney | 58-33-1-F1-PU |
| ID269 | est-not-ext | 5.9 | Fetal kidney | 58-48-4-H2-PU |
| ID270 | est-not-ext | 5.9 | Fetal kidney | 58-42-1-A6-PU |
| ID271 | est-not-ext | 5.7 | Fetal kidney | 58-33-4-E1-PU |
| ID272 | est-not-ext | 5.7 | Fetal kidney | 58-26-2-E12-PU |
| ID273 | est-not-ext | 5.6 | Fetal kidney | 58-26-1-E12-PU |
| ID274 | est-not-ext | 5.5 | Fetal kidney | 58-54-1-D11-PU |
| ID275 | est-not-ext | 5.5 | Muscle | 27-9-2-F9-PU |
| ID276 | est-not-ext | 5.4 | Fetal kidney | 58-30-2-H10-PU |
| ID277 | est-not-ext | 5.3 | Fetal kidney | 58-29-1-H1-PU |
| ID278 | est-not-ext | 5.3 | Kidney | 21-1-4-F2-PU |
| ID279 | est-not-ext | 5.1 | Fetal kidney | 58-42-4-H7-PU |
| ID280 | est-not-ext | 5 | Fetal kidney | 58-34-3-H10-PU |
| ID281 | est-not-ext | 5 | Kidney | 21-7-3-B4-PU |
| ID282 | est-not-ext | 4.9 | Fetal kidney | 58-4-2-D12-PU |
| ID283 | est-not-ext | 4.8 | Fetal kidney | 58-31-2-C10-PU |
| ID284 | est-not-ext | 4.7 | Fetal kidney | 58-37-3-C10-PU |
| ID285 | est-not-ext | 4.7 | Fetal kidney | 58-1-1-D11-PU |
| ID286 | est-not-ext | 4.6 | Fetal kidney | 58-52-1-A11-PU |
| ID287 | est-not-ext | 4.3 | Fetal kidney | 58-4-3-E10-PU |
| ID288 | est-not-ext | 4.3 | Heart | 67-6-4-F2-PU |
| ID289 | est-not-ext | 4.2 | Fetal kidney | 58-49-3-G10-PU |
| ID290 | est-not-ext | 4.1 | Dystrophic muscle | 29-10-3-B11-PU |
| ID291 | est-not-ext | 4.1 | Heart | 25-5-4-A7-PU |
| ID292 | est-not-ext | 4.1 | Fetal kidney | 58-33-2-C6-PU |
| ID293 | est-not-ext | 4 | Heart | 25-7-3-D4-PU |
| ID294 | est-not-ext | 3.9 | Heart | 67-1-3-B11-PU |
| ID295 | est-not-ext | 3.9 | Fetal kidney | 58-23-1-G5-PU |
| ID296 | est-not-ext | 3.7 | Fetal kidney | 58-6-1-B6-PU |
| ID297 | est-not-ext | 3.7 | Dystrophic muscle | 29-6-2-H8-PU |
| ID298 | est-not-ext | 3.7 | Fetal kidney | 58-43-4-B8-PU |
| ID299 | est-not-ext | 3.6 | Muscle | 27-3-4-G9-PU |
| ID300 | est-not-ext | 3.6 | Fetal kidney | 58-38-1-F10-PU |
| ID301 | est-not-ext | 3.5 | Hean | 67-6-4-E7-PU |
| ID302 | est-not-ext | 3.5 | Fetal kidney | 58-54-1-E6-PU |
| ID303 | est-not-ext | 3.5 | Heart | 67-4-4-G7-PU |
| ID304 | est-not-ext | 3.5 | Fetal kidney | 58-23-4-F4-PU |
| ID305 | ext-vrt-not-genomic | 10.5 | Fetal kidney | 58-42-3-A12-PU |

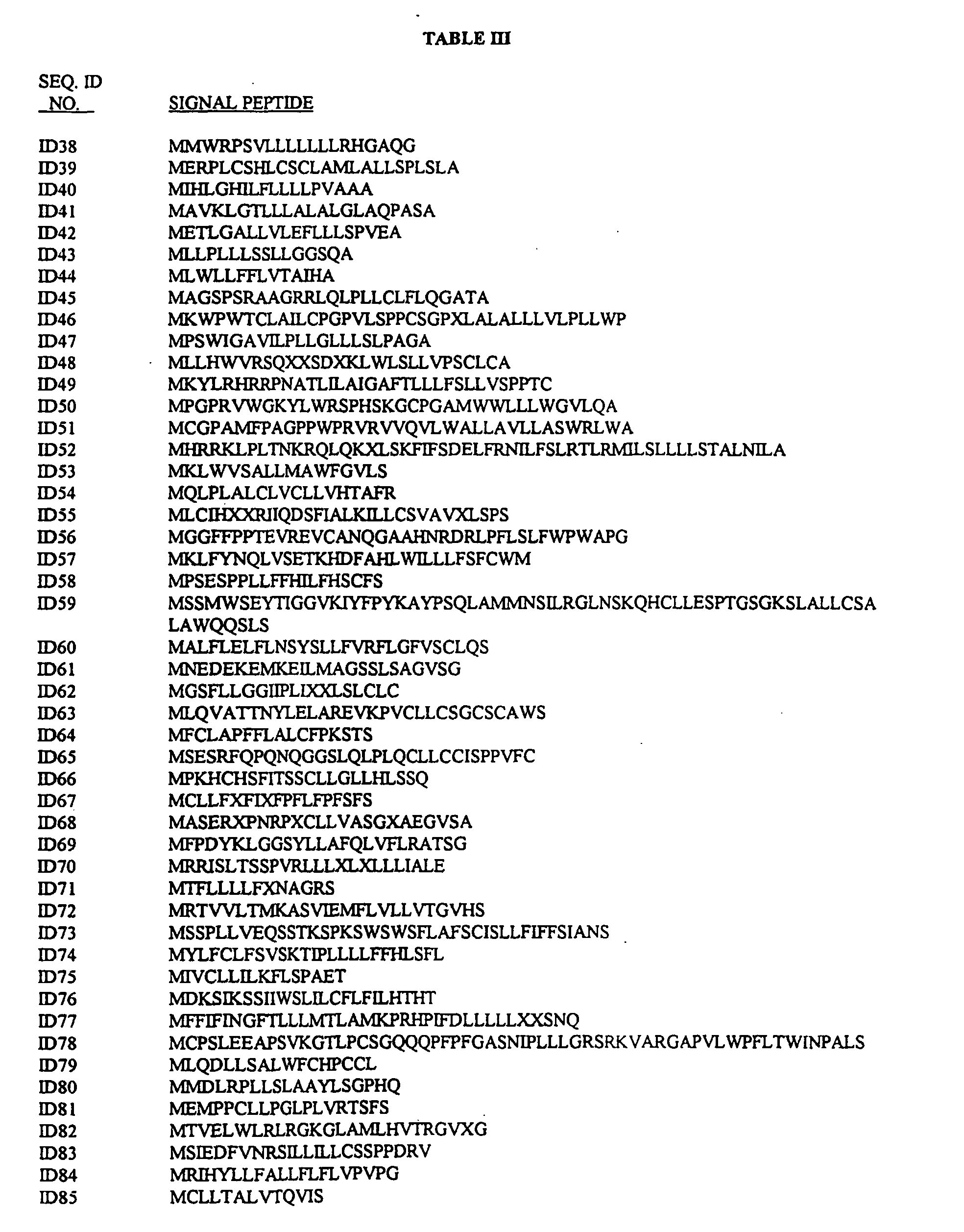

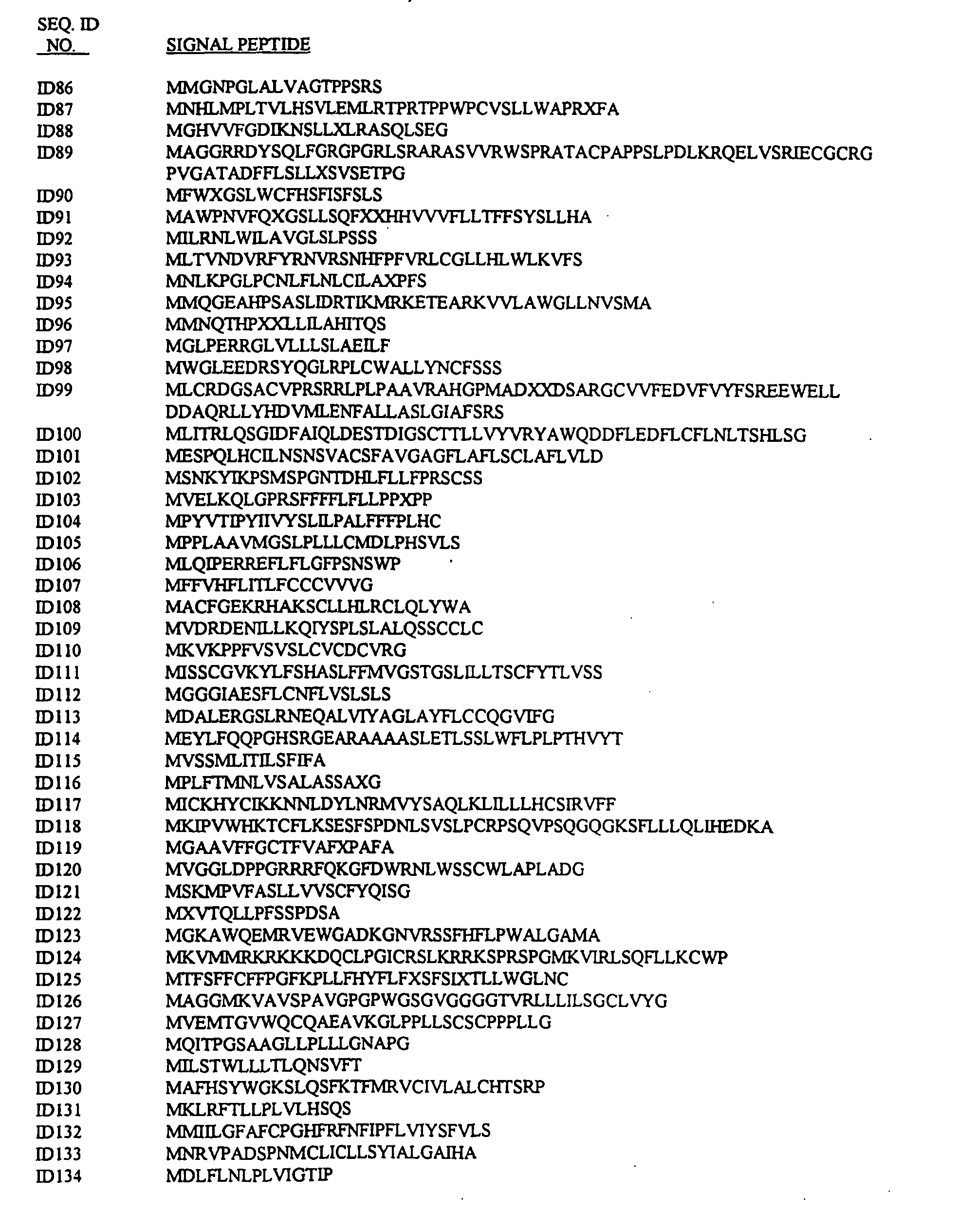

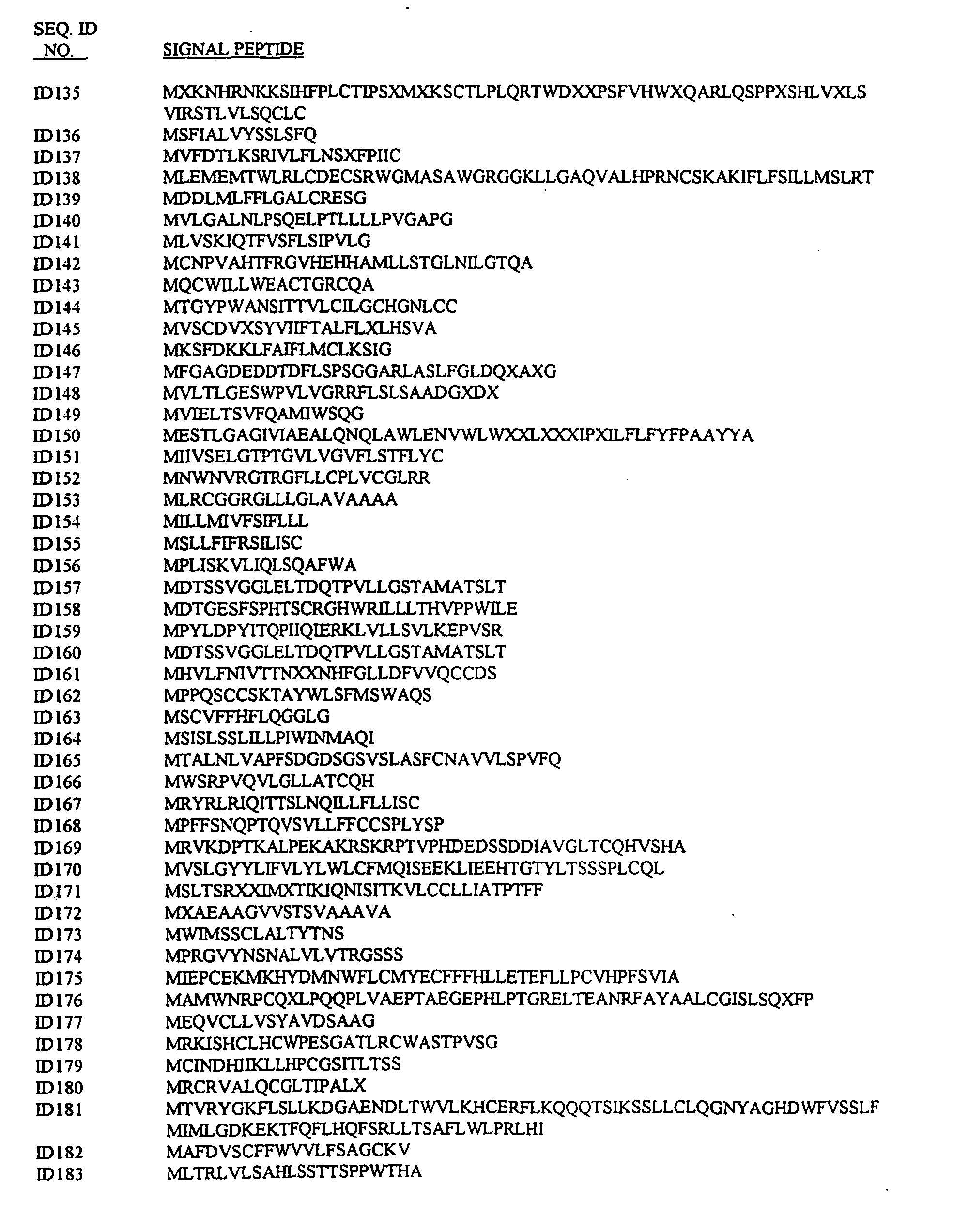

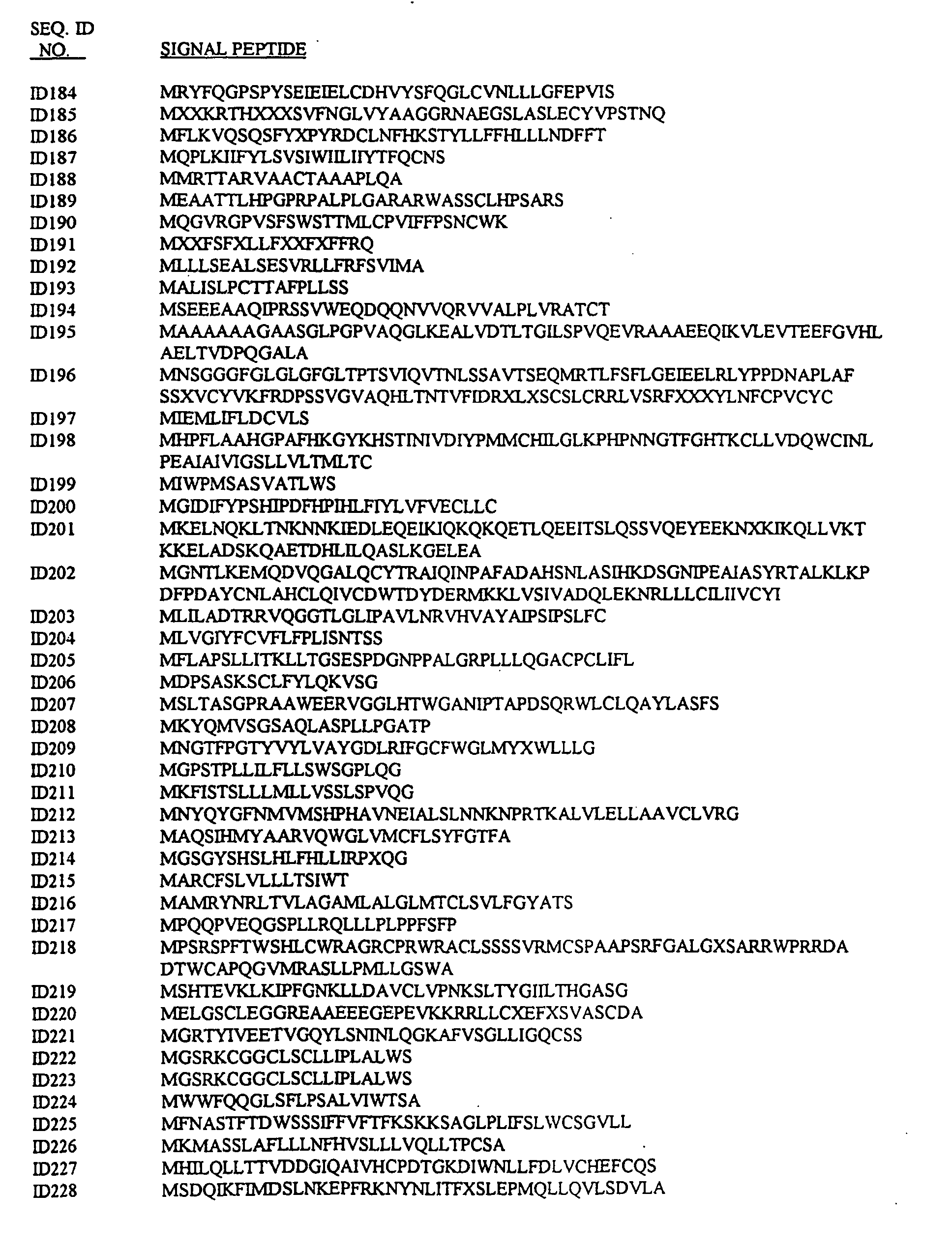

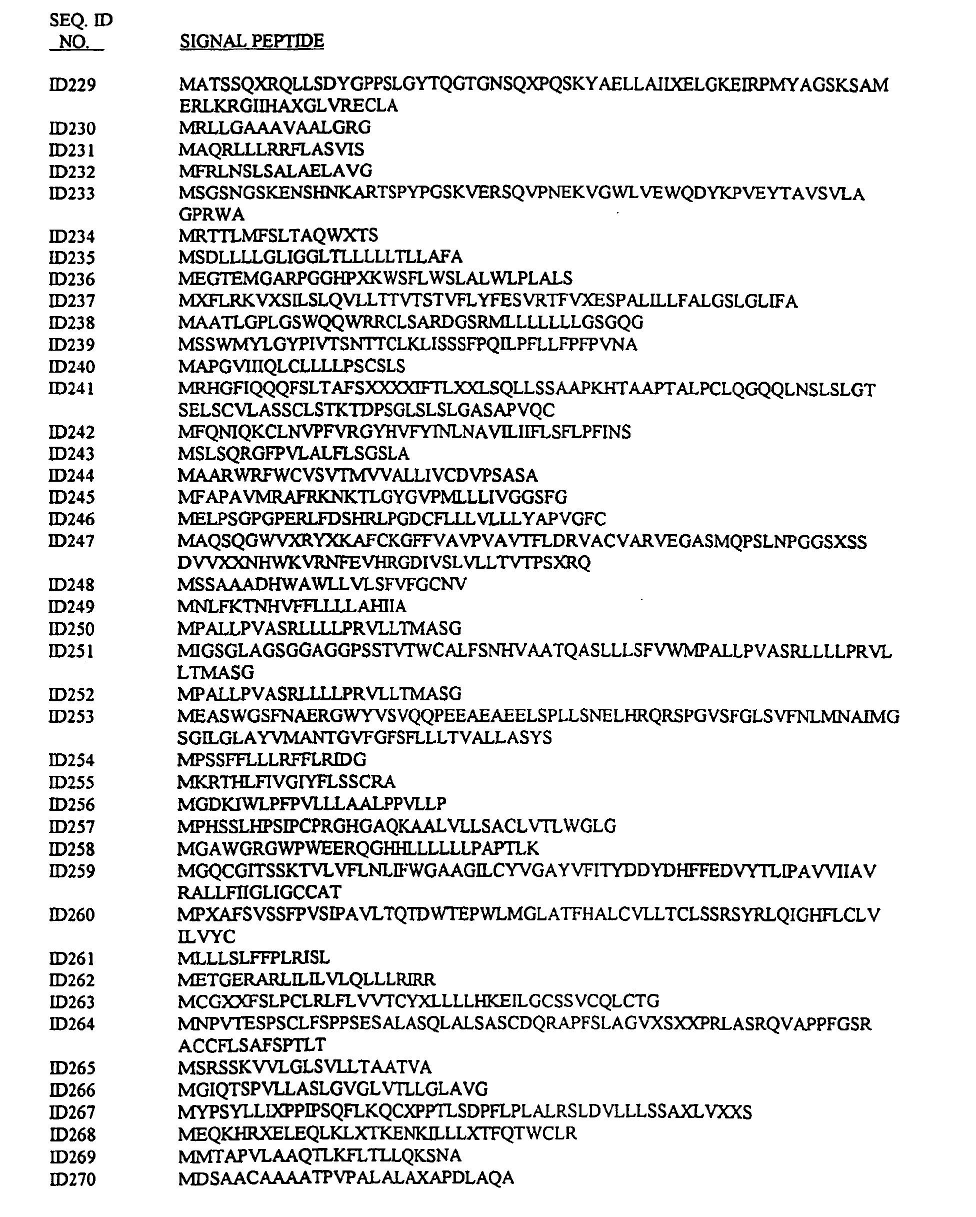

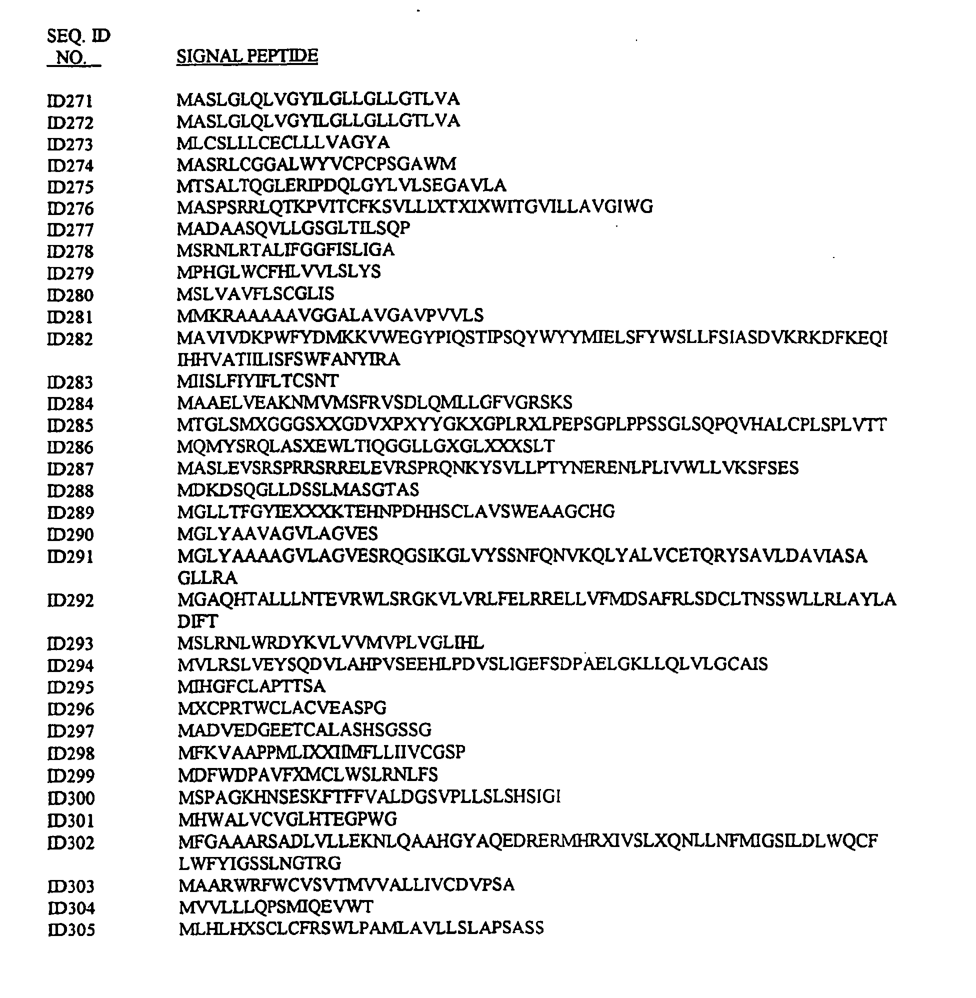

**TABLE IV**

| Minimum signal peptide score | false positive rate | false negative rate | proba(01) | proba(0.2) |
|---|---|---|---|---|
| 3.5 | 0.121 | 0.036 | 0.467 | 0.664 |
| 4 | 0.096 | 0.06 | 0.519 | 0.708 |
| 4.5 | 0.078 | 0.079 | 0.565 | 0.745 |
| 5 | 0.062 | 0.098 | 0.615 | 0.782 |
| 5.5 | 0.05 | 0.127 | 0.659 | 0.813 |
| 6 | 0.04 | 0.163 | 0.694 | 0.836 |
| 6.5 | 0.033 | 0.202 | 0.725 | 0.855 |
| 7 | 0.025 | 0.248 | 0.763 | 0.878 |
| 7.5 | 0.021 | 0.304 | 0.78 | 0.889 |
| 8 | 0.015 | 0.368 | 0.816 | 0.909 |
| 8.5 | 0.012 | 0.418 | 0.836 | 0.92 |
| 9 | 0.009 | 0.512 | 0.856 | 0.93 |
| 9.5 | 0.007 | 0.581 | 0.863 | 0.934 |
| 10 | 0.006 | 0.679 | 0.835 | 0.919 |

**TABLE V**

| Minimum signal peptide score | All ESTs | New ESTS | ESTs matching public EST closer than 40 bp from beginning | ESTs extending known mRNA more than 40 bp | ESTs extending public E8T more than 40 bp |
|---|---|---|---|---|---|
| 3.5 | 2674 | 947 | 599 | 23 | 150 |
| 4 | 2278 | 784 | 499 | 23 | 126 |
| 4.5 | 1943 | 647 | 425 | 22 | 112 |
| 5 | 1657 | 523 | 353 | 21 | 96 |
| 5.5 | 1417 | 419 | 307 | 19 | 80 |
| 6 | 1190 | 340 | 238 | 18 | 68 |
| 6.5 | 1035 | 280 | 186 | 18 | 60 |
| 7 | 893 | 219 | 161 | 15 | 48 |
| 7.5 | 753 | 173 | 132 | 12 | 36 |
| 8 | 636 | 133 | 101 | 11 | 29 |
| 8.5 | 543 | 104 | 83 | 8 | 26 |
| 9 | 456 | 81 | 63 | 6 | 24 |
| 9.5 | 364 | 57 | 48 | 6 | 18 |
| 10 | 303 | 47 | 35 | 6 | 15 |

**TABLE VI**

| Tissue | All ESTs | New ESTs | ESTs matching public EST closer from 40 bp from beginning | ESTS extending known mRNA more than 40 bp | ESTs extending public EST more than 40 bp bp |
|---|---|---|---|---|---|
| Brain | 329 | 131 | 75 | 3 | 24 |
| Cancerous prostate | 134 | 40 | 37 | 1 | 6 |
| Cerebellum | 17 | 9 | 1 | 0 | 6 |
| Colon | 21 | 11 | 4 | 0 | 0 |
| Dystrophic muscle | 41 | 18 | 8 | 0 | 1 |
| Fetal brain | 70 | 37 | 16 | 0 | 1 |
| Fetal kidney | 227 | 116 | 46 | 1 | 19 |
| Fetal liver | 13 | 7 | 2 | 0 | 0 |
| Heart | 30 | 15 | 7 | 0 | 1 |
| Hypertrophic prostate | 86 | 23 | 22 | 2 | 2 |
| Kidney | 10 | 7 | 3 | 0 | 0 |
| Large intestine | 21 | 8 | 4 | 0 | 1 |
| Liver | 23 | 9 | 6 | 0 | 0 |
| Lung | 24 | 12 | 4 | 0 | 1 |
| Lung (cells) | 57 | 38 | 6 | 0 | 4 |
| Lymph ganglia | 163 | 60 | 23 | 2 | 12 |
| Lymphocytes | 23 | 6 | 4 | 0 | 2 |
| Muscle | 33 | 16 | 6 | 0 | 4 |
| Normal prostate | 181 | 61 | 45 | 7 | 11 |
| Ovary | 90 | 57 | 12 | 1 | 2 |
| Pancreas | 48 | 11 | 6 | 0 | 1 |
| Placenta | 24 | 5 | 1 | 0 | 0 |
| Prostate | 34 | 16 | 4 | 0 | 2 |
| Spleen | 56 | 28 | 10 | 0 | 1 |
| Substantia nigra | 108 | 47 | 27 | 1 | 6 |
| Surrenals | 15 | 3 | 3 | 1 | 0 |
| Testis | 131 | 68 | 25 | 1 | 8 |
| Thyroid | 17 | 8 | 2 | 0 | 2 |
| Umbilical cord | 55 | 17 | 12 | 1 | 3 |
| Uterus | 28 | 15 | 3 | 0 | 2 |
| Non tissue-specific | 568 | 48 | 177 | 2 | 28 |
| Total | 2677 | 947 | 601 | 23 | 150 |

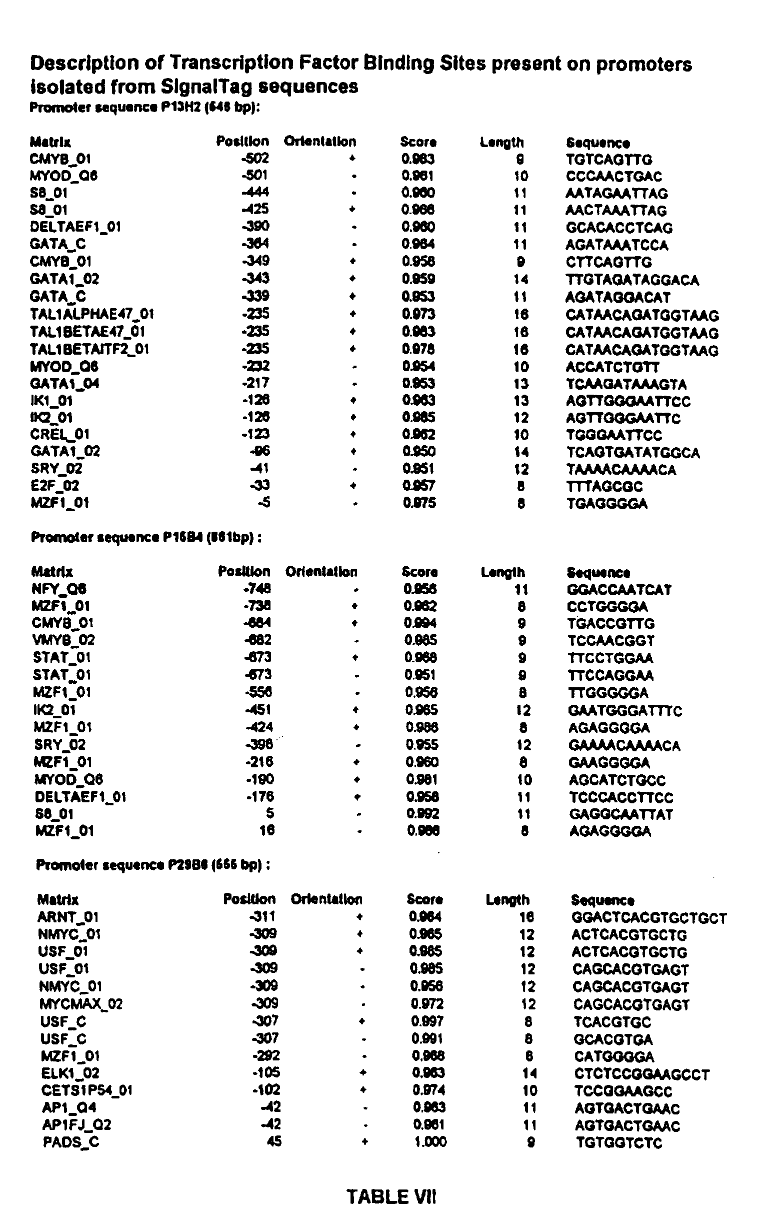

### EMBODIMENTS

The following are some preferred embodiments (wherein "Emb." = "Embodiment"):
1. A purified or isolated nucleic acid comprising the sequence of one of SEQ ID NOs: 38-305 or comprising a sequence complementary thereto.
2. The nucleic acid of Emb 1, wherein said nucleic acid is recombinant.
3. A purified or isolated nucleic acid comprising at least 10 consecutive bases of the sequence of one of SEQ ID NOs: 38-305 or one of the sequences complementary thereto.
4. A purified or isolated nucleic acid comprising at least 15 consecutive bases of one of the sequences of SEQ ID NOs: 38-305 or one of the sequences complementary thereto.
5. The nucleic acid of Emb. 4, wherein said nucleic acid is recombinant.
6. A purified or isolated nucleic acid of at least 15 bases capable of hybridizing under stringent conditions to the sequence of one of SEQ ID NOs: 38-305 or one of the sequences complementary to the sequences of SEQ ID NOs: 38-305.
7. The nucleic acid of Emb. 6, wherein said nucleic acid is recombinant.
8. A purified or isolated nucleic acid encoding a human gene product, said human gene product having a sequence partially encoded by one of the sequences of SEQ ID NO: 38-305.
9. A purified or isolated nucleic acid having the sequence of one of SEQ ID NOs: 38-305 or having a sequence complementary thereto.
10. A purified or isolated nucleic acid comprising the nucleotides of one of SEQ ID NOs: 38-305 which encode a signal peptide.
11. A purified or isolated polypeptides comprising a signal peptide encoded by one of the sequences of SEQ ID NOs: 38-305.
12. A vector encoding a fusion protein comprising a polypeptide and a signal peptide, said vector comprising a first nucleic acid encoding a signal peptide encoded by one of the sequences of SEQ ID NOs: 38-305 operably linked to a second nucleic acid encoding a polypeptide.
13. A method of directing the extracellular secretion of a polypeptide or the insertion of a polypetide into the membrane comprising the steps of:
   obtaining a vector according to Emb. 12; and
   introducing said vector into a host cell such that said fusion protein is secreted into the extracellular environment of said host cell or inserted into the membrane of said host cell.
14. A method of importing a polypeptide into a cell comprising contacting said cell with a fusion protein comprising a signal peptide encoded by one of the sequences of SEQ ID NOs: 38-305 operably linked to said polypeptide.
15. A method of making a cDNA encoding a human secretory protein that is partially encoded by one of SEQ ID NOs 38-305, comprising the steps of:
   obtaining a cDNA comprising one of the sequences of SEQ ID NOs: 38-305;
   contacting said cDNA with a detectable probe comprising at least 15 consecutive nucleotides of said sequence of SEQ ID NO: 38-305 or a sequence complementary thereto under conditions which permit said probe to hybridize to said cDNA;
   identifying a cDNA which hybridizes to said detectable probe; and
   isolating said cDNA which hybridizes to said probe.
16. An isolated or purified cDNA encoding a human secretory protein, said human secretory protein comprising the protein encoded by one of SEQ ID NOs 38-305 or a fragment thereof of at least 10 amino acids, said cDNA being obtainable by the method of Emb. 15.
17. The cDNA of Emb.16 wherein said cDNA comprises the full protein coding sequence partially included in one of the sequences of SEQ ID NOs: 38-305.
18. A method of making a cDNA comprising one of the sequences of SEQ ID NOs: 38-305, comprising the steps of:
   contacting a collection of mRNA molecules from human cells with a first primer capable of hybridizing to the polyA tail of said mRNA;
   hybridizing said first primer to said polyA tail;
   reverse transcribing said mRNA to make a first cDNA strand;
   making a second cDNA strand complementary to said first cDNA strand using at least one primer comprising at least 15 nucleotides of one of the sequences of SEQ ID NOs 38-305; and
   isolating the resulting cDNA comprising said first cDNA strand and said second cDNA strand.
19. An isolated or purified cDNA encoding a human secretory protein, said human secretory protein comprising the protein encoded by one of SEQ ID NOs 38-305 or a fragment thereof of at least 10 amino acids, said cDNA being obtainable by the method of Emb. 18.
20. The cDNA of Emb. 19 wherein said cDNA comprises the full protein coding sequence partially included in one of the sequences of SEQ ID NOs: 38-305.
21. The method of Emb. 18, wherein the second cDNA strand is made by:
   contacting said first cDNA strand with a first pair of primers, said first pair of primers comprising a second primer comprising at least 15 consecutive nucleotides of one of the sequences of SEQ ID NOs 38-305 and a third primer having a sequence therein which is included within the sequence of said first primer;
   performing a first polymerase chain reaction with said first pair of nested primers to generate a first PCR product;
   contacting said first PCR product with a second pair of primers, said second pair of primers comprising a fourth primer, said fourth primer comprising at least 15 consecutive nucleotides of said sequence of one of SEQ ID NO:s 38-305 , and a fifth primer, said fourth and fifth primers being capable of hybridizing to sequences within said first PCR product; and
   performing a second polymerase chain reaction, thereby generating a second PCR product.
22. An isolated or purified cDNA encoding a human secretory protein, said human secretory protein comprising the protein encoded by one of SEQ ID NOs 38-305, or a fragment thereof of at least 10 amino acids, said cDNA being obtainable by the method of Emb. 21.
23. The cDNA of Emb. 22 wherein said cDNA comprises the full protein coding sequence partially included in one of the sequences of SEQ ID NOs: 38-305.
24. The method of Emb. 18 wherein the second cDNA strand is made by:
   contacting said first cDNA strand with a second primer comprising at least 15 consecutive nucleotides of the sequences of SEQ ID NOs: 38-305;
   hybridizing said second primer to said first strand cDNA; and
   extending said hybridized second primer to generate said second cDNA strand.
25. An isolated or purified cDNA encoding a human secretory protein, said human secretory protein comprising the protein partially encoded by one of SEQ ID NOs 38-305 or comprising a fragment thereof of at least 10 amino acids, said cDNA being obtainable by the method of Emb. 24.
26. The cDNA of Emb. 25, wherein said cDNA comprises the full protein coding sequence partially included in of one of the sequences of SEQ ID NOs: 38-305.
27. A method of making a protein comprising one of the sequences of SEQ ID NO: 306-573, comprising the steps of:
   obtaining a cDNA encoding the full protein sequence partially included in one of the sequences of sequence of SEQ ID NO: 38-305;
   inserting said cDNA in an expression vector such that said cDNA is operably linked to a promoter,
   introducing said expression vector into a host cell whereby said host cell produces the protein encoded by said cDNA; and
   isolating said protein.
28. An isolated protein obtainable by the method of Emb. 27.
29. A method of obtaining a promoter DNA comprising the steps of:
   obtaining DNAs located upstream of the nucleic acids of SEQ ID NO: 38-305 or the sequences complementary thereto;
   screening said upstream DNAs to identify a promoter capable of directing transcription initiation; and
   isolating said DNA comprising said identified promoter.
30. The method of Emb. 29, wherein said obtaining step comprises chromosome walking from said nucleic adds of SEQ ID NO: 38-305 or sequences complementary thereto.
31. The method of Emb. 30, wherein said screening step comprises inserting said upstream sequences into a promoter reporter vector.
32. The method of Emb. 30, wherein said screening step comprises identifying motifs in said upstream DNAs which are transcription factor binding sites or transcription start sites.
33. An isolated promoter obtainable by the method of Emb. 32.
34. An isolated or purified protein comprising one of the sequences of SEQ ID NO: 306-573.
35. In an array of discrete ESTs or fragments thereof of at least 15 nucleotides in length, the improvement comprising inclusion in said array of at least one of the sequences of SEQ ID NOs: 38-305, or one of the sequences complementary to the sequences of SEQ ID NOs: 38-305, or a fragment thereof of at least 15 consecutive nucleotides.
36. The array of'Emb. 35 including therein at least two of the sequences of SEQ ID NOs: 38-305, the sequences complementary to the sequences of SEQ ID NOs: 38-305, or fragments thereof of at least 15 consecutive nucleotides.
37. The array of Emb. 35 including therein at least five of the sequences of SEQ ID NOs: 38-305, the sequences complementary to the sequences of SEQ ID NOs: 38-305, or fragments thereof of at least 15 consecutive nucleotides.

SEQUENCE LISTING

(1) GENERAL INFORMATION:

(i) APPLICANT:
(A) NAME : GENSET SA
(B) STREET :24, RUE ROYALE
(C) CITY: PARIS
(E) COUNTRY : FRANCE
(F) POSTAL CODE (ZIP) : 75008

(ii) TITLE OF INVENTION: 5' ESTs FOR SECRETED PROTEINS
EXPRESSED IN MUSCLE AND OTHER MESODERMAL TISSUES

(iii) NUMBER OF SEQUENCES: 573

(v) COMPUTER READABLE FORM:
(A) MEDIUM TYPE: Floppy Disk
(B) COMPUTER: IBM PC compatible
(C) OPERATING SYSTEM: Win95
(D) SOFTWARE: Word

(2) INFORMATION FOR SEQ ID NO: 1:

(i) SEQUENCE CHARACTERISTICS:
(A) LENGTH: 47 base pairs
(B) TYPE: NUCLEIC ACID
(C) STRANDEDNESS: SINGLE
(D) TOPOLOGY: LINEAR

(ii) MOLECULE TYPE: Other nucleic acid

(ix) FEATURE:
(A) NAME/KEY: Cap
(B) LOCATION: 1
(D) OTHER INFORMATION: m7Gppp added to 1

(xi) SEQUENCE DESCRIPTION: SEQ ID NO: 1:

GGCAUCCUAC UCCCAUCCAA UUCCACCCUA ACUCCUCCCA UCUCCAC 47

(2) INFORMATION FOR SEQ ID NO: 2:

(i) SEQUENCE CHARACTERISTICS:
(A) LENGTH: 46 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 2:

GCAUCCUACU CCCAUCCAAU UCCACCCUAA CUCCUCCCAU CUCCAC 46

(2) INFORMATION FOR SEQ ID NO: 3:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 3:

ATCAAGAATT CGCACGAGAC CATTA 25

(2) INFORMATION FOR SEQ ID NO: 4:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 4:

TAATGGTCTC GTGCGAATTC TTGAT 25

(2) INFORMATION FOR SEQ ID NO: 5:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 5:

CCGACAAGAC CAACGTCAAG GCCGC 25

(2) INFORMATION FOR SEQ ID NO: 6:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 6:

TCACCAGCAG GCAGTGGCTT AGGAG 25

(2) INFORMATION FOR SEQ ID NO: 7:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 7:

AGTGATTCCT GCTACTTTGG ATGGC 25

(2) INFORMATION FOR SEQ ID NO: 8:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 8:

GCTTGGTCTT GTTCTGGAGT TTAGA 25

(2) INFORMATION FOR SEQ ID NO: 9:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 9:

TCCAGAATGG GAGACAAGCC AATTT 25

(2) INFORMATION FOR SEQ ID NO: 10:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 10:

AGGGAGGAGG AAACAGCGTG AGTCC 25

(2) INFORMATION FOR SEQ ID NO: 11:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 11:

ATGGGAAAGG AAAAGACTCA TATCA 25

(2) INFORMATION FOR SEQ ID NO: 12:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 12:

AGCAGCAACA ATCAGGACAG CACAG 25

(2) INFORMATION FOR SEQ ID NO: 13:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 13:

ATCAAGAATT CGCACGAGAC CATTA 25

(2) INFORMATION FOR SEQ ID NO: 14:

(i) SEQUENCE CHARACTERISTICS:
(A) LENGTH: 67 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 14:

ATCGTTGAGA CTCGTACCAG CAGAGTCACG AGAGAGACTA CACGGTACTG GTTTTTTTTT 60

TTTTTVN 67

(2) INFORMATION FOR SEQ ID NO: 15:

(i) SEQUENCE CHARACTERISTICS:
(A) LENGTH: 29 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 15:

CCAGCAGAGT CACGAGAGAG ACTACACGG 29

(2) INFORMATION FOR SEQ ID NO: 16:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 16:

CACGAGAGAG ACTACACGGT ACTGG 25

(2) INFORMATION FOR SEQ ID NO: 17:

(i) SEQUENCE CHARACTERISTICS:
(A) LENGTH: 526 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Lymph ganglia

(ix) FEATURE:
(A) NAME/KEY: other
(B) LOCATION: complement(261..376)
(C) IDENTIFICATION METHOD: blastn
(D) OTHER INFORMATION: identity 96
region 166..281
id N70479
est

(ix) FEATURE:
(A) NAME/KEY: other
(B) LOCATION: complement(380..486)
(C) IDENTIFICATION METHOD: blastn
(D) OTHER INFORMATION: identity 97
region 54..160
id N70479
est

(ix) FEATURE:
(A) NAME/KEY: other
(B) LOCATION: complement(110..145)
(C) IDENTIFICATION METHOD: blastn
(D) OTHER INFORMATION: identity 94
region 403..438
id N70479
est

(ix) FEATURE:
(A) NAME/KEY: other
(B) LOCATION: complement(196..229)
(C) IDENTIFICATION METHOD: blastn
(D) OTHER INFORMATION: identity 94
region 315..348
id N70479
est

(ix) FEATURE:
(A) NAME/KEY: sig_peptide
(B) LOCATION: 90..140
(C) IDENTIFICATION METHOD: Von Heijne matrix
(D) OTHER INFORMATION: score 8.2
seq LLLITAILAVAVG/FP

(xi) SEQUENCE DESCRIPTION: SEQ ID NO: 17:

AATATRARAC AGCTACAATA TTCCAGGGCC ARTCACTTGC CATTTCTCAT AACAGCGTCA 60

GAGAGAAAGA ACTGACTGAR ACGTTTGAG ATG AAG AAA GTT CTC CTC CTG ATC 113

Met Lys Lys Val Leu Leu Leu Ile
-15 -10

ACA GCC ATC TTG GCA GTG GCT GTW GGT TTC CCA GTC TCT CAA GAC CAG 161
Thr Ala Ile Leu Ala Val Ala Val Gly Phe Pro Val Ser Gln Asp Gln
-5 1 5

GAA CGA GAA AAA AGA AGT ATC AGT GAC AGC GAT GAA TTA GCT TCA GGR 209
Glu Arg Glu Lys Arg Ser Ile Ser Asp Ser Asp Glu Leu Ala Ser Gly
10 15 20

WTT TTT GTG TTC CCT TAC CCA TAT CCA TTT CGC CCA CTT CCA CCA ATT 257
Xaa Phe Val Phe Pro Tyr Pro Tyr Pro Phe Arg Pro Leu Pro Pro Ile
25 30 35

CCA TTT CCA AGA TTT CCA TGG TTT AGA CGT AAN TTT CCT ATT CCA ATA 305
Pro Phe Pro Arg Phe Pro Trp Phe Arg Arg Xaa Phe Pro Ile Pro Ile
40 45 50 55

CCT GAA TCT GCC CCT ACA ACT CCC CTT CCT AGC GAA AAG TAAACAARAA 354
Pro Glu Ser Ala Pro Thr Thr Pro Leu Pro Ser Glu Lys
60 65

GGAAAAGTCA CRATAAACCT GGTCACCTGA AATTGAAATT GAGCCACTTC CTTGAARAAT 414

CAAAATTCCT GTTAATAAAA RAAAAACAAA TGTAATTGAA ATAGCACACA GCATTCTCTA 474

GTCAATATCT TTAGTGATCT TCTTTAATAA ACATGAAAGC AAAAAAAAAA AA 526

(2) INFORMATION FOR SEQ ID NO: 18:

(i) SEQUENCE CHARACTERISTICS:
(A) LENGTH: 17 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(ix) FEATURE:
(A) NAME/KEY: sig_peptide
(B) LOCATION: 1..17
(C) IDENTIFICATION METHOD: Von Heijne matrix
(D) OTHER INFORMATION: score 8.2
seq LLLITAILAVAVG/FP

(xi) SEQUENCE DESCRIPTION: SEQ ID NO: 18:

Met Lys Lys Val Leu Leu Leu Ile Thr Ala Ile Leu Ala Val Ala Val
1               5                   10                  15

Gly

(2) INFORMATION FOR SEQ ID NO: 19:

(i) SEQUENCE CHARACTERISTICS:
(A) LENGTH: 822 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 260..464
(C) IDENTIFICATION METHOD: blastn
(D) OTHER INFORMATION: identity 96
region 153..357
id H57434
est

(ix) FEATURE:
(A) NAME/KEY: other
(B) LOCATION: 118..184
(C) IDENTIFICATION METHOD: blastn
(D) OTHER INFORMATION: identity 98
region 98..164
id H57434
est

(ix) FEATURE:
(A) NAME/KEY: other
(B) LOCATION: 56..113
(C) IDENTIFICATION METHOD: blastn
(D) OTHER INFORMATION: identity 98
region 35..92
id H57434
est

(ix) FEATURE:
(A) NAME/KEY: other
(B) LOCATION: 454..485
(C) IDENTIFICATION METHOD: blastn
(D) OTHER INFORMATION: identity 100
region 348..379
id H57434
est

(ix) FEATURE:
(A) NAME/KEY: other
(B) LOCATION: 118..545
(C) IDENTIFICATION METHOD: blastn
(D) OTHER INFORMATION: identity 98
region 1..428
id N27248
est

(ix) FEATURE:
(A) NAME/KEY: other

(B) LOCATION: 65..369
(C) IDENTIFICATION METHOD: blastn
(D) OTHER INFORMATION: identity 98
region 41..345
id H94779
est

(ix) FEATURE:
(A) NAME/KEY: other
(B) LOCATION: 61..399
(C) IDENTIFICATION METHOD: blastn
(D) OTHER INFORMATION: identity 99
region 6..344
id H09880
est

(ix) FEATURE:
(A) NAME/KEY: other
(B) LOCATION: 408..458
(C) IDENTIFICATION METHOD: blastn
(D) OTHER INFORMATION: identity 92
region 355..405
id H09880
est

(ix) FEATURE:
(A) NAME/KEY: other
(B) LOCATION: 60..399
(C) IDENTIFICATION METHOD: blastn
(D) OTHER INFORMATION: identity 97
region 56..395
id H29351
est

(ix) FEATURE:
(A) NAME/KEY: other
(B) LOCATION: 393..432
(C) IDENTIFICATION METHOD: blastn
(D) OTHER INFORMATION: identity 90
region 391..430
id H29351
est

(ix) FEATURE:
(A) NAME/KEY: sig_peptide
(B) LOCATION: 346..408
(C) IDENTIFICATION METHOD: Von Heijne matrix
(D) OTHER INFORMATION: score 5.5
seq SFLPSALVIWTSA/AF

(xi) SEQUENCE DESCRIPTION: SEQ ID NO: 19:

ACTCCTTTTA GCATAGGGGC TTCGGCGCCA GCGGCCAGCG CTAGTCGGTC TGGTAAGTGC 60

CTGATGCCGA GTTCCGTCTC TCGCGTCTTT TCCTGGTCCC AGGCAAAGCG GASGNAGATC 120

CTCAAACGGC CTAGTGCTTC GCGCTTCCGG AGAAAATCAG CGGTCTAATT AATTCCTCTG 180

GTTTGTTGAA GCAGTTACCA AGAATCTTCA ACCCTTTCCC ACAAAAGCTA ATTGAGTACA 240

CGTTCCTGTT GAGTACACGT TCCTGTTGAT TTACAAAAGG TGCAGGTATG AGCAGGTCTG 300

AAGACTAACA TTTTGTGAAG TTGTAAAACA GAAAACCTGT TAGAA ATG TGG TGG TTT 357
Met Trp Trp Phe
-20

CAG CAA GGC CTC AGT TTC CTT CCT TCA GCC CTT GTA ATT TGG ACA TCT 405
Gln Gln Gly Leu Ser Phe Leu Pro Ser Ala Leu Val Ile Trp Thr Ser
-15 -10 -5

GCT GCT TTC ATA TTT TCA TAC ATT ACT GCA GTA ACA CTC CAC CAT ATA 453
Ala Ala Phe Ile Phe Ser Tyr Ile Thr Ala Val Thr Leu His His Ile
1               5                   10                  15

GAC CCG GCT TTA CCT TAT ATC AGT GAC ACT GGT ACA GTA GCT CCA RAA 501
Asp Pro Ala Leu Pro Tyr Ile Ser Asp Thr Gly Thr Val Ala Pro Xaa
            20                  25                  30

AAA TGC TTA TTT GGG GCA ATG CTA AAT ATT GCG GCA GTT TTA TGT CAA 549
Lys Cys Leu Phe Gly Ala Met Leu Asn Ile Ala Ala Val Leu Cys Gln
        35                  40                  45

AAA TAGAAATCAG GAARATAATT CAACTTAAAG AAKTTCATTT CATGACCAAA 602
Lys

CTCTTCARAA ACATGTCTTT ACAAGCATAT CTCTTGTATT GCTTTCTACA CTGTTGAATT 662

GTCTGGCAAT ATTTCTGCAG TGGAAAATTT GATTTARMTA GTTCTTGACT GATAAATATG 722

GTAAGGTGGG CTTTTCCCCC TGTGTAATTG GCTACTATGT CTTACTGAGC CAAGTTGTAW 782

TTTGAAATAA AATGATATGA GAGTGACACA AAAAAAAAAA 822

(2) INFORMATION FOR SEQ ID NO: 20:

(i) SEQUENCE CHARACTERISTICS:
(A) LENGTH: 21 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(ix) FEATURE:
(A) NAME/KEY: sig_peptide
(B) LOCATION: 1..21
(C) IDENTIFICATION METHOD: Von Heijne matrix
(D) OTHER INFORMATION: score 5.5
seq SFLPSALVIWTSA/AF

(xi) SEQUENCE DESCRIPTION: SEQ ID NO: 20:

Met Trp Trp Phe Gln Gln Gly Leu Ser Phe Leu Pro Ser Ala Leu Val
1               5                   10                  15

Ile Trp Thr Ser Ala
            20

(2) INFORMATION FOR SEQ ID NO: 21:

(i) SEQUENCE CHARACTERISTICS:
(A) LENGTH: 405 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Testis

(ix) FEATURE:
(A) NAME/KEY: other
(B) LOCATION: complement(103..398)
(C) IDENTIFICATION METHOD: blastn
(D) OTHER INFORMATION: identity 96
region 1..296
id AA442893
est

(ix) FEATURE:
(A) NAME/KEY: sig_peptide
(B) LOCATION: 185..295
(C) IDENTIFICATION METHOD: Von Heijne matrix
(D) OTHER INFORMATION: score 5.9
seq LSYASSALSPCLT/AP

(xi) SEQUENCE DESCRIPTION: SEQ ID NO: 21:

ATCACCTTCT TCTCCATCCT TSTCTGGGCC AGTCCCCARC CCAGTCCCTC TCCTGACCTG 60

CCCAGCCCAA GTCAGCCTTC AGCACGCGCT TTTCTGCACA CAGATATTCC AGGCCTACCT 120

GGCATTCCAG GACCTCCGMA ATGATGCTCC AGTCCCTTAC AAGCGCTTCC TGGATGAGGG 180

TGGC ATG GTG CTG ACC ACC CTC CCC TTG CCC TCT GCC AAC AGC CCT GTG 229
Met Val Leu Thr Thr Leu Pro Leu Pro Ser Ala Asn Ser Pro Val
-35 -30 -25

AAC ATG CCC ACC ACT GGC CCC AAC AGC CTG AGT TAT GCT AGC TCT GCC 277
Asn Met Pro Thr Thr Gly Pro Asn Ser Leu Ser Tyr Ala Ser Ser Ala
-20 -15 -10

CTG TCC CCC TGT CTG ACC GCT CCA AAK TCC CCC CGG CTT GCT ATG ATG 325
Leu Ser Pro Cys Leu Thr Ala Pro Xaa Ser Pro Arg Leu Ala Met Met
-5 1 5 10

CCT GAC AAC TAAATATCCT TATCCAAATC AATAAARWRA RAATCCTCCC TCCARAAGGG 384
Pro Asp Asn

TTTCTAAAAA CAAAAAAAAA A 405

(2) INFORMATION FOR SEQ ID NO: 22:

(i) SEQUENCE CHARACTERISTICS:
(A) LENGTH: 37 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(ix) FEATURE:
(A) NAME/KEY: sig_peptide
(B) LOCATION: 1..37
(C) IDENTIFICATION METHOD: Von Heijne matrix
(D) OTHER INFORMATION: score 5.9
seq LSYASSALSPCLT/AP

(xi) SEQUENCE DESCRIPTION: SEQ ID NO: 22:

Met Val Leu Thr Thr Leu Pro Leu Pro Ser Ala Asn Ser Pro Val Asn
1               5                   10                  15

Met Pro Thr Thr Gly Pro Asn Ser Leu Ser Tyr Ala Ser Ser Ala Leu
            20                  25                  30

Ser Pro Cys Leu Thr
        35

(2) INFORMATION FOR SEQ ID NO: 23:

(i) SEQUENCE CHARACTERISTICS:
(A) LENGTH: 496 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Cancerous prostate

(ix) FEATURE:
(A) NAME/KEY: other
(B) LOCATION: 149..331
(C) IDENTIFICATION METHOD: blastn
(D) OTHER INFORMATION: identity 98
region 1..183
id AA397994
est

(ix) FEATURE:
(A) NAME/KEY: other

(B) LOCATION: 328..485
(C) IDENTIFICATION METHOD: blastn
(D) OTHER INFORMATION: identity 96
region 179..336
id AA397994
est

(ix) FEATURE:
(A) NAME/KEY: other
(B) LOCATION: complement(182..496)
(C) IDENTIFICATION METHOD: blastn
(D) OTHER INFORMATION: identity 97
region 14..328
id AA399680
est

(ix) FEATURE:
(A) NAME/KEY: sig_peptide
(B) LOCATION: 196..240
(C) IDENTIFICATION METHOD: Von Heijne matrix
(D) OTHER INFORMATION: score 5.5
seq ILSTVTALTFAXA/LD

(xi) SEQUENCE DESCRIPTION: SEQ ID NO: 23:

AAAAAATTGG TCCCAGTTTT CACCCTGCCG CAGGGCTGGC TGGGGAGGGC AGCGGTTTAG 60

ATTAGCCGTG GCCTAGGCCG TTTAACGGGG TGACACGAGC NTGCAGGGCC GAGTCCAAGG 120

CCCGGAGATA GGACCAACCG TCAGGAATGC GAGGAATGTT TTTCTTCGGA CTCTATCGAG 180

GCACACAGAC AGACC ATG GGG ATT CTG TCT ACA GTG ACA GCC TTA ACA TTT 231
Met Gly Ile Leu Ser Thr Val Thr Ala Leu Thr Phe
-15 -10 -5

GCC ARA GCC CTG GAC GGC TGC AGA AAT GGC ATT GCC CAC CCT GCA AGT 279
Ala Xaa Ala Leu Asp Gly Cys Arg Asn Gly Ile Ala His Pro Ala Ser
1 5 10

GAG AAG CAC AGA CTC GAG AAA TGT AGG GAA CTC GAG ASC ASC CAC TCG 327
Glu Lys His Arg Leu Glu Lys Cys Arg Glu Leu Glu Xaa Xaa His Ser
15 20 25

GCC CCA GGA TCA ACC CAS CAC CGA AGA AAA ACA ACC AGA AGA AAT TAT 375
Ala Pro Gly Ser Thr Xaa His Arg Arg Lys Thr Thr Arg Arg Asn Tyr
30 35 40 45

TCT TCA GCC TGAAATGAAK CCGGGATCAA ATGGTTGCTG ATCARAGCCC ATATTTAAAT 434
Ser Ser Ala

TGGAAAAGTC AAATTGASCA TTATTAAATA AAGCTTGTTT AATATGTCTC AAACAAAAAA 494

AA 496

(2) INFORMATION FOR SEQ ID NO: 24:

(i) SEQUENCE CHARACTERISTICS:

(A) LENGTH: 15 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(ix) FEATURE:
(A) NAME/KEY: sig_peptide
(B) LOCATION: 1..15
(C) IDENTIFICATION METHOD: Von Heijne matrix
(D) OTHER INFORMATION: score 5.5
seq ILSTVTALTFAXA/LD

(xi) SEQUENCE DESCRIPTION: SEQ ID NO: 24:

Met Gly Ile Leu Ser Thr Val Thr Ala Leu Thr Phe Ala Xaa Ala
1               5                   10                  15

(2) INFORMATION FOR SEQ ID NO: 25:

(i) SEQUENCE CHARACTERISTICS:
(A) LENGTH: 623 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Testis

(ix) FEATURE:
(A) NAME/KEY: sig_peptide
(B) LOCATION: 49..96
(C) IDENTIFICATION METHOD: Von Heijne matrix
(D) OTHER INFORMATION: score 10.1
seq LVLTLCTLPLAVA/SA

(xi) SEQUENCE DESCRIPTION: SEQ ID NO: 25:

AAAGATCCCT GCAGCCCGGC AGGAGAGAAG GCTGAGCCTT CTGGCGTC ATG GAG AGG 57
Met Glu Arg
-15

CTC GTC CTA ACC CTG TGC ACC CTC CCG CTG GCT GTG GCG TCT GCT GGC 105
Leu Val Leu Thr Leu Cys Thr Leu Pro Leu Ala Val Ala Ser Ala Gly
-10 -5 1

TGC GCC ACG ACG CCA GCT CGC AAC CTG AGC TGC TAC CAG TGC TTC AAG 153
Cys Ala Thr Thr Pro Ala Arg Asn Leu Ser Cys Tyr Gln Cys Phe Lys
                5                   10                  15

GTC AGC AGC TGG ACG GAG TGC CCG CCC ACC TGG TGC AGC CCG CTG GAC 201

Val Ser Ser Trp Thr Glu Cys Pro Pro Thr Trp Cys Ser Pro Leu Asp
20                  25                  30                  35

CAA GTC TGC ATC TCC AAC GAG GTG GTC GTC TCT TTT AAA TGG AGT GTA     249
Gln Val Cys Ile Ser Asn Glu Val Val Val Ser Phe Lys Trp Ser Val
            40                  45                  50

CGC GTC CTG CTC AGC AAA CGC TGT GCT CCC AGA TGT CCC AAC GAC AAC     297
Arg Val Leu Leu Ser Lys Arg Cys Ala Pro Arg Cys Pro Asn Asp Asn
        55                  60                  65

ATG AAK TTC GAA TGG TCG CCG GCC CCC ATG GTG CAA GGC GTG ATC ACC     345
Met Xaa Phe Glu Trp Ser Pro Ala Pro Met Val Gln Gly Val Ile Thr
    70                  75                  80

AGG CGC TGC TGT TCC TGG GCT CTC TGC AAC AGG GCA CTG ACC CCA CAG     393
Arg Arg Cys Cys Ser Trp Ala Leu Cys Asn Arg Ala Leu Thr Pro Gln
85                  90                  95

GAG GGG CGC TGG GCC CTG CRA GGG GGG CTC CTG CTC CAG GAC CCT TCG     441
Glu Gly Arg Trp Ala Leu Xaa Gly Gly Leu Leu Leu Gln Asp Pro Ser
100                 105                 110                 115

AGG GGC ARA AAA ACC TGG GTG CGG CCA CAG CTG GGG CTC CCA CTC TGC     489
Arg Gly Xaa Lys Thr Trp Val Arg Pro Gln Leu Gly Leu Pro Leu Cys
            120                 125                 130

CTT CCC AWT TCC AAC CCC CTC TGC CCA RGG GAA ACC CAG GAA GGA         534
Leu Pro Xaa Ser Asn Pro Leu Cys Pro Xaa Glu Thr Gln Glu Gly
        135                 140                 145

TAACACTGTG GGTGCCCCCA CCTGTGCATT GGGACCACRA CTTCACCCTC TTGGARACAA   594

TAAACTCTCA TGCCCCCAAA AAAAAAAAA                                     623

(2) INFORMATION FOR SEQ ID NO: 26:

(i) SEQUENCE CHARACTERISTICS:
(A) LENGTH: 16 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(ix) FEATURE:
(A) NAME/KEY: sig_peptide
(B) LOCATION: 1..16
(C) IDENTIFICATION METHOD: Von Heijne matrix
(D) OTHER INFORMATION:  score 10.1
seq LVLTLCTLPLAVA/SA

(xi) SEQUENCE DESCRIPTION: SEQ ID NO: 26:

Met Glu Arg Leu Val Leu Thr Leu Cys Thr Leu Pro Leu Ala Val Ala
1               5                   10                  15

(2) INFORMATION FOR SEQ ID NO: 27:

(i) SEQUENCE CHARACTERISTICS:
(A) LENGTH: 848 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 32..73
(C) IDENTIFICATION METHOD: Von Heijne matrix
(D) OTHER INFORMATION: score 10.7
seq LWLLFFLVTAIHA/EL

(xi) SEQUENCE DESCRIPTION: SEQ ID NO: 27:

AACTTTGCCT TGTGTTTTCC ACCCTGAAAG A ATG TTG TGG CTG CTC TTT TTT CTG 55
Met Leu Trp Leu Leu Phe Phe Leu
-10

GTG ACT GCC ATT CAT GCT GAA CTC TGT CAA CCA GGT GCA GAA AAT GCT 103
Val Thr Ala Ile His Ala Glu Leu Cys Gln Pro Gly Ala Glu Asn Ala
-5 1 5 10

TTT AAA GTG AGA CTT AGT ATC AGA ACA GCT CTG GGA GAT AAA GCA TAT 151
Phe Lys Val Arg Leu Ser Ile Arg Thr Ala Leu Gly Asp Lys Ala Tyr
15 20 25

GCC TGG GAT ACC AAT GAA GAA TAC CTC TTC AAA GCG ATG GTA GCT TTC 199
Ala Trp Asp Thr Asn Glu Glu Tyr Leu Phe Lys Ala Met Val Ala Phe
30 35 40

TCC ATG AGA AAA GTT CCC AAC AGA GAA GCA ACA GAA ATT TCC CAT GTC 247
Ser Met Arg Lys Val Pro Asn Arg Glu Ala Thr Glu Ile Ser His Val
45 50 55

CTA CTT TGC AAT GTA ACC CAG AGG GTA TCA TTC TGG TTT GTG GTT ACA 295
Leu Leu Cys Asn Val Thr Gln Arg Val Ser Phe Trp Phe Val Val Thr
60 65 70

GAC CCT TCA AAA AAT CAC ACC CTT CCT GCT GTT GAG GTG CAA TCA GCC 343
Asp Pro Ser Lys Asn His Thr Leu Pro Ala Val Glu Val Gln Ser Ala
75 80 85 90

ATA AGA ATG AAC AAG AAC CGG ATC AAC AAT GCC TTC TTT CTA AAT GAC 391
Ile Arg Met Asn Lys Asn Arg Ile Asn Asn Ala Phe Phe Leu Asn Asp
95 100 105

CAA ACT CTG GAA TTT TTA AAA ATC CCT TCC ACA CTT GCA CCA CCC ATG 439

Gln Thr Leu Glu Phe Leu Lys Ile Pro Ser Thr Leu Ala Pro Pro Met
110 115 120

GAC CCA TCT GTG CCC ATC TGG ATT ATT ATA TTT GGT GTG ATA TTT TGC 487
Asp Pro Ser Val Pro Ile Trp Ile Ile Ile Phe Gly Val Ile Phe Cys
125 130 135

ATC ATC ATA GTT GCA ATT GCA CTA CTG ATT TTA TCA GGG ATC TGG CAA 535
Ile Ile Ile Val Ala Ile Ala Leu Leu Ile Leu Ser Gly Ile Trp Gln
140 145 150

CGT ADA ARA AAG AAC AAA GAA CCA TCT GAA GTG GAT GAC GCT GAA RAT 583
Arg Xaa Xaa Lys Asn Lys Glu Pro Ser Glu Val Asp Asp Ala Glu Xaa
155 160 165 170

AAK TGT GAA AAC ATG ATC ACA ATT GAA AAT GGC ATC CCC TCT GAT CCC 631
Xaa Cys Glu Asn Met Ile Thr Ile Glu Asn Gly Ile Pro Ser Asp Pro
175 180 185

CTG GAC ATG AAG GGA GGG CAT ATT AAT GAT GCC TTC ATG ACA GAG GAT 679
Leu Asp Met Lys Gly Gly His Ile Asn Asp Ala Phe Met Thr Glu Asp
190 195 200

GAG AGG CTC ACC CCT CTC TGAAGGGCTG TTGTTCTGCT TCCTCAARAA 727
Glu Arg Leu Thr Pro Leu
205

ATTAAACATT TGTTTCTGTG TGACTGCTGA GCATCCTGAA ATACCAAGAG CAGATCATAT 787

WTTTTGTTTC ACCATTCTTC TTTTGTAATA AATTTTGAAT GTGCTTGAAA AAAAAAAAAA 847

C 848

(2) INFORMATION FOR SEQ ID NO: 28:

(i) SEQUENCE CHARACTERISTICS:
(A) LENGTH: 14 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(ix) FEATURE:
(A) NAME/KEY: sig_peptide
(B) LOCATION: 1..14
(C) IDENTIFICATION METHOD: Von Heijne matrix
(D) OTHER INFORMATION: score 10.7
seq LWLLFFLVTAIHA/EL

(xi) SEQUENCE DESCRIPTION: SEQ ID NO: 28:

Met Leu Trp Leu Leu Phe Phe Leu Val Thr Ala Ile His Ala
1               5                   10

(2) INFORMATION FOR SEQ ID NO: 29:

(i) SEQUENCE CHARACTERISTICS:
(A) LENGTH: 25 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 29:

GGGAAGATGG AGATAGTATT GCCTG 25

(2) INFORMATION FOR SEQ ID NO: 30:

(i) SEQUENCE CHARACTERISTICS:
(A) LENGTH: 26 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 30:

CTGCCATGTA CATGATAGAG AGATTC 26

(2) INFORMATION FOR SEQ ID NO: 31:

(i) SEQUENCE CHARACTERISTICS:
(A) LENGTH: 546 base pairs
(B) TYPE: NUCLEIC ACID
(C) STRANDEDNESS: DOUBLE
(D) TOPOLOGY: LINEAR

(ii) MOLECULE TYPE: Genomic DNA

(ix) FEATURE:
(A) NAME/KEY: promoter
(B) LOCATION: 1..517

(ix) FEATURE:
(A) NAME/KEY: transcription start site
(B) LOCATION: 518

(ix) FEATURE:
(A) NAME/KEY: TF binding-site
(B) LOCATION: 17..25
(C) IDENTIFICATION METHOD: matinspector prediction
(D) OTHER INFORMATION: name CMYB_01
score 0.983
sequence TGTCAGTTG

(ix) FEATURE:
(A) NAME/KEY: TF binding-site
(B) LOCATION: complement(18..27)
(C) IDENTIFICATION METHOD: matinspector prediction
(D) OTHER INFORMATION: name MYOD_Q6
score 0.961
sequence CCCAACTGAC

(ix) FEATURE:
(A) NAME/KEY: TF binding-site
(B) LOCATION: complement(75..85)
(C) IDENTIFICATION METHOD: matinspector prediction
(D) OTHER INFORMATION: name S8_01
score 0.960
sequence AATAGAATTAG

(ix) FEATURE:
(A) NAME/KEY: TF binding-site
(B) LOCATION: 94..104
(C) IDENTIFICATION METHOD: matinspector prediction
(D) OTHER INFORMATION: name S8_01
score 0.966
sequence AACTAAATTAG

(ix) FEATURE:
(A) NAME/KEY: TF binding-site
(B) LOCATION: complement(129..139)
(C) IDENTIFICATION METHOD: matinspector prediction
(D) OTHER INFORMATION: name DELTAEF1_01
score 0.960
sequence GCACACCTCAG

(ix) FEATURE:
(A) NAME/KEY: TF binding-site
(B) LOCATION: complement(155..165)
(C) IDENTIFICATION METHOD: matinspector prediction
(D) OTHER INFORMATION: name GATA_C
score 0.964
sequence AGATAAATCCA

(ix) FEATURE:
(A) NAME/KEY: TF binding-site
(B) LOCATION: 170..178
(C) IDENTIFICATION METHOD: matinspector prediction
(D) OTHER INFORMATION: name CMYB_01
score 0.958
sequence CTTCAGTTG

(ix) FEATURE:
(A) NAME/KEY: TF binding-site
(B) LOCATION: 176..189
(C) IDENTIFICATION METHOD: matinspector prediction
(D) OTHER INFORMATION: name GATA1_02
score 0.959
sequence TTGTAGATAGGACA

(ix) FEATURE:
(A) NAME/KEY: TF binding-site
(B) LOCATION: 180..190
(C) IDENTIFICATION METHOD: matinspector prediction
(D) OTHER INFORMATION: name GATA_C

score 0.953
sequence AGATAGGACAT

(ix) FEATURE:
(A) NAME/KEY: TF binding-site
(B) LOCATION: 284..299
(C) IDENTIFICATION METHOD: matinspector prediction
(D) OTHER INFORMATION: name TAL1ALPHAE47_01
score 0.973
sequence CATAACAGATGGTAAG

(ix) FEATURE:
(A) NAME/KEY: TF binding-site
(B) LOCATION: 284..299
(C) IDENTIFICATION METHOD: matinspector prediction
(D) OTHER INFORMATION: name TAL1BETAE47_01
score 0.983
sequence CATAACAGATGGTAAG

(ix) FEATURE:
(A) NAME/KEY: TF binding-site
(B) LOCATION: 284..299
(C) IDENTIFICATION METHOD: matinspector prediction
(D) OTHER INFORMATION: name TAL1BETAITF2_01
score 0.978
sequence CATAACAGATGGTAAG

(ix) FEATURE:
(A) NAME/KEY: TF binding-site
(B) LOCATION: complement(287..296)
(C) IDENTIFICATION METHOD: matinspector prediction
(D) OTHER INFORMATION: name MYOD_Q6
score 0.954
sequence ACCATCTGTT

(ix) FEATURE:
(A) NAME/KEY: TF binding-site
(B) LOCATION: complement(302..314)
(C) IDENTIFICATION METHOD: matinspector prediction
(D) OTHER INFORMATION: name GATA1_04
score 0.953
sequence TCAAGATAAAGTA

(ix) FEATURE:
(A) NAME/KEY: TF binding-site
(B) LOCATION: 393..405
(C) IDENTIFICATION METHOD: matinspector prediction
(D) OTHER INFORMATION: name IK1_01
score 0.963
sequence AGTTGGGAATTCC

(ix) FEATURE:
(A) NAME/KEY: TF binding-site
(B) LOCATION: 393..404
(C) IDENTIFICATION METHOD: matinspector prediction
(D) OTHER INFORMATION: name IK2_01
score 0.985
sequence AGTTGGGAATTC

(ix) FEATURE:
(A) NAME/KEY: TF binding-site

(B) LOCATION: 396..405
(C) IDENTIFICATION METHOD: matinspector prediction
(D) OTHER INFORMATION: name CREL_01
score 0.962
sequence TGGGAATTCC

(ix) FEATURE:
(A) NAME/KEY: TF binding-site
(B) LOCATION: 423..436
(C) IDENTIFICATION METHOD: matinspector prediction
(D) OTHER INFORMATION: name GATA1_02
score 0.950
sequence TCAGTGATATGGCA

(ix) FEATURE:
(A) NAME/KEY: TF binding-site
(B) LOCATION: complement(478..489)
(C) IDENTIFICATION METHOD: matinspector prediction
(D) OTHER INFORMATION: name SRY_02
score 0.951
sequence TAAAACAAAACA

(ix) FEATURE:
(A) NAME/KEY: TF binding-site
(B) LOCATION: 486..493
(C) IDENTIFICATION METHOD: matinspector prediction
(D) OTHER INFORMATION: name E2F_02
score 0.957
sequence TTTAGCGC

(ix) FEATURE:
(A) NAME/KEY: TF binding-site
(B) LOCATION: complement(514..521)
(C) IDENTIFICATION METHOD: matinspector prediction
(D) OTHER INFORMATION: name MZF1_01
score 0.975
sequence TGAGGGGA

(xi) SEQUENCE DESCRIPTION: SEQ ID NO: 31:

TGAGTGCAGT GTTACATGTC AGTTGGGTTA AGTTTGTTAA TGTCATTCAA ATCTTCTATG 60

TCTTGATTTG CCTGCTAATT CTATTATTTC TGGAACTAAA TTAGTTTGAT GGTTCTATTA 120

GTTATTGACT GAGGTGTGCT AATCTCCCAT TATGTGGATT TATCTATTTC TTCAGTTGTA 180

GATAGGACAT TGATAGATAC ATAAGTACCA GGACAAAAGC AGGGAGATCT TTTTTCCAAA 240

ATCAGGAGAA AAAAATGACA TCTGGAAAAC CTATAGGGAA AGGCATAACA GATGGTAAGG 300

ATACTTTATC TTGAGTAGGA GAGCCTTCCT GTGGCAACGT GGAGAAGGGA AGAGGTCGTA 360

GAATTGAGGA GTCAGCTCAG TTAGAAGCAG GGAGTTGGGA ATTCCGTTCA TGTGATTTAG 420

CATCAGTGAT ATGGCAAATG TGGGACTAAG GGTAGTGATC AGAGGGTTAA AATTGTGTGT 480

TTTGTTTTAG CGCTGCTGGG GCATCGCCTT GGGTCCCCTC AAACAGATTC CCATGAATCT 540

CTTCAT 546

(2) INFORMATION FOR SEQ ID NO: 32:

(i) SEQUENCE CHARACTERISTICS:
(A) LENGTH: 23 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 32:

GTACCAGGGA CTGTGACCAT TGC 23

(2) INFORMATION FOR SEQ ID NO: 33:

(i) SEQUENCE CHARACTERISTICS:
(A) LENGTH: 24 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 33:

CTGTGACCAT TGCTCCCAAG AGAG 24

(2) INFORMATION FOR SEQ ID NO: 34:

(i) SEQUENCE CHARACTERISTICS:
(A) LENGTH: 861 base pairs
(B) TYPE: NUCLEIC ACID
(C) STRANDEDNESS: DOUBLE
(D) TOPOLOGY: LINEAR

(ii) MOLECULE TYPE: Genomic DNA

(ix) FEATURE:
(A) NAME/KEY: promoter
(B) LOCATION: 1..806

(ix) FEATURE:
(A) NAME/KEY: transcription start site
(B) LOCATION: 807

(ix) FEATURE:
(A) NAME/KEY: TF binding-site
(B) LOCATION: complement(60..70)
(C) IDENTIFICATION METHOD: matinspector prediction
(D) OTHER INFORMATION: name NFY_Q6
score 0.956
sequence GGACCAATCAT

(ix) FEATURE:
(A) NAME/KEY: TF binding-site
(B) LOCATION: 70..77
(C) IDENTIFICATION METHOD: matinspector prediction
(D) OTHER INFORMATION: name MZF1_01
score 0.962
sequence CCTGGGGA

(ix) FEATURE:
(A) NAME/KEY: TF binding-site
(B) LOCATION: 124..132
(C) IDENTIFICATION METHOD: matinspector prediction
(D) OTHER INFORMATION: name CMYB_01
score 0.994
sequence TGACCGTTG

(ix) FEATURE:
(A) NAME/KEY: TF binding-site
(B) LOCATION: complement(126..134)
(C) IDENTIFICATION METHOD: matinspector prediction
(D) OTHER INFORMATION: name VMYB_02
score 0.985
sequence TCCAACGGT

(ix) FEATURE:
(A) NAME/KEY: TF binding-site
(B) LOCATION: 135..143
(C) IDENTIFICATION METHOD: matinspector prediction
(D) OTHER INFORMATION: name STAT_01
score 0.968
sequence TTCCTGGAA

(ix) FEATURE:
(A) NAME/KEY: TF binding-site
(B) LOCATION: complement(135..143)
(C) IDENTIFICATION METHOD: matinspector prediction
(D) OTHER INFORMATION: name STAT_01
score 0.951
sequence TTCCAGGAA

(ix) FEATURE:
(A) NAME/KEY: TF binding-site
(B) LOCATION: complement(252..259)
(C) IDENTIFICATION METHOD: matinspector prediction
(D) OTHER INFORMATION: name MZF1_01
score 0.956
sequence TTGGGGGA

(ix) FEATURE:
(A) NAME/KEY: TF binding-site
(B) LOCATION: 357..368
(C) IDENTIFICATION METHOD: matinspector prediction
(D) OTHER INFORMATION: name IK2_01
score 0.965
sequence GAATGGGATTTC

(ix) FEATURE:
(A) NAME/KEY: TF binding-site
(B) LOCATION: 384..391
(C) IDENTIFICATION METHOD: matinspector prediction

(D) OTHER INFORMATION: name MZF1_01
score 0.986
sequence AGAGGGGA

(ix) FEATURE:
(A) NAME/KEY: TF binding-site
(B) LOCATION: complement(410..421)
(C) IDENTIFICATION METHOD: matinspector prediction
(D) OTHER INFORMATION: name SRY_02
score 0.955
sequence GAAAACAAAACA

(ix) FEATURE:
(A) NAME/KEY: TF binding-site
(B) LOCATION: 592..599
(C) IDENTIFICATION METHOD: matinspector prediction
(D) OTHER INFORMATION: name MZF1_01
score 0.960
sequence GAAGGGGA

(ix) FEATURE:
(A) NAME/KEY: TF binding-site
(B) LOCATION: 618..627
(C) IDENTIFICATION METHOD: matinspector prediction
(D) OTHER INFORMATION: name MYOD_Q6
score 0.981
sequence AGCATCTGCC

(ix) FEATURE:
(A) NAME/KEY: TF binding-site
(B) LOCATION: 632..642
(C) IDENTIFICATION METHOD: matinspector prediction
(D) OTHER INFORMATION: name DELTAEF1_01
score 0.958
sequence TCCCACCTTCC

(ix) FEATURE:
(A) NAME/KEY: TF binding-site
(B) LOCATION: complement(813..823)
(C) IDENTIFICATION METHOD: matinspector prediction
(D) OTHER INFORMATION: name S8_01
score 0.992
sequence GAGGCAATTAT

(ix) FEATURE:
(A) NAME/KEY: TF binding-site
(B) LOCATION: complement(824..831)
(C) IDENTIFICATION METHOD: matinspector prediction
(D) OTHER INFORMATION: name MZF1_01
score 0.986
sequence AGAGGGGA

(xi) SEQUENCE DESCRIPTION: SEQ ID NO: 34:
   TACTATAGGG CACGCGTGGT CGACGGCCGG GCTGTTCTGG AGCAGAGGGC ATGTCAGTAA   60
   TGATTGGTCC CTGGGGAAGG TCTGGCTGGC TCCAGCACAG TGAGGCATTT AGGTATCTCT 120
   CGGTGACCGT TGGATTCCTG GAAGCAGTAG CTGTTCTGTT TGGATCTGGT AGGGACAGGG 180
   CTCAGAGGGC TAGGCACGAG GGAAGGTCAG AGGAGAAGGS AGGSARGGCC CAGTGAGARG 240
   GGAGCATGCC TTCCCCCAAC CCTGGCTTSC YCTTGGYMAM AGGGCGKTTY TGGGMACTTR 300
   AAYTCAGGGC CCAASCAGAA SCACAGGCCC AKTCNTGGCT SMAAGCACAA TAGCCTGAAT 360
   GGGATTTCAG GTTAGNCAGG GTGAGAGGGG AGGCTCTCTG GCTTAGTTTT GTTTTGTTTT 420
   CCAAATCAAG GTAACTTGCT CCCTTCTGCT ACGGGCCTTG GTCTTGGCTT GTCCTCACCC 480
   AGTCGGAACT CCCTACCACT TTCAGGAGAG TGGTTTTAGG CCCGTGGGGC TGTTCTGTTC 540
   CAAGCAGTGT GAGAACATGG CTGGTAGAGG CTCTAGCTGT GTGCGGGGCC TGAAGGGGAG 600
   TGGGTTCTCG CCCAAAGAGC ATCTGCCCAT TTCCCACCTT CCCTTCTCCC ACCAGAAGCT 660
   TGCCTGAGCT GTTTGGACAA AAATCCAAAC CCCACTTGGC TACTCTGGCC TGGCTTCAGC 720
   TTGGAACCCA ATACCTAGGC TTACAGGCCA TCCTGAGCCA GGGGCCTCTG GAAATTCTCT 780
   TCCTGATGGT CCTTTAGGTT TGGGCACAAA ATATAATTGC CTCTCCCCTC TCCCATTTTC 840
   TCTCTTGGGA GCAATGGTCA C 861

(2) INFORMATION FOR SEQ ID NO: 35:

(i) SEQUENCE CHARACTERISTICS:
(A) LENGTH: 20 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 35:

CTGGGATGGA AGGCACGGTA 20

(2) INFORMATION FOR SEQ ID NO: 36:

(i) SEQUENCE CHARACTERISTICS:
(A) LENGTH: 20 base pairs
(B) TYPE: NUCLEIC ACID
(C) STRANDEDNESS: SINGLE
(D) TOPOLOGY: LINEAR

(ii) MOLECULE TYPE: Other nucleic acid

(xi) SEQUENCE DESCRIPTION: SEQ ID NO: 36:

GAGACCACAC AGCTAGACAA 20

(2) INFORMATION FOR SEQ ID NO: 37:

(i) SEQUENCE CHARACTERISTICS:
(A) LENGTH: 555 base pairs
(B) TYPE: NUCLEIC ACID
(C) STRANDEDNESS: DOUBLE
(D) TOPOLOGY: LINEAR

(ii) MOLECULE TYPE: Genomic DNA

(ix) FEATURE:
(A) NAME/KEY: promoter
(B) LOCATION: 1..500

(ix) FEATURE:
(A) NAME/KEY: transcription start site
(B) LOCATION: 501

(ix) FEATURE:
(A) NAME/KEY: TF binding-site
(B) LOCATION: 191..206
(C) IDENTIFICATION METHOD: matinspector prediction
(D) OTHER INFORMATION: name ARNT_01
score 0.964
sequence GGACTCACGTGCTGCT

(ix) FEATURE:
(A) NAME/KEY: TF binding-site
(B) LOCATION: 193..204
(C) IDENTIFICATION METHOD: matinspector prediction
(D) OTHER INFORMATION: name NMYC_01
score 0.965
sequence ACTCACGTGCTG

(ix) FEATURE:
(A) NAME/KEY: TF binding-site
(B) LOCATION: 193..204
(C) IDENTIFICATION METHOD: matinspector prediction
(D) OTHER INFORMATION: name USF_01
score 0.985
sequence ACTCACGTGCTG

(ix) FEATURE:
(A) NAME/KEY: TF binding-site
(B) LOCATION: complement(193..204)
(C) IDENTIFICATION METHOD: matinspector prediction
(D) OTHER INFORMATION: name USF_01
score 0.985
sequence CAGCACGTGAGT

(ix) FEATURE:
(A) NAME/KEY: TF binding-site
(B) LOCATION: complement(193..204)
(C) IDENTIFICATION METHOD: matinspector prediction
(D) OTHER INFORMATION: name NMYC_01
score 0.956
sequence CAGCACGTGAGT

(ix) FEATURE:
(A) NAME/KEY: TF binding-site
(B) LOCATION: complement(193..204)
(C) IDENTIFICATION METHOD: matinspector prediction

(D) OTHER INFORMATION:  name MYCMAX_02
                           score 0.972
                           sequence CAGCACGTGAGT

(ix) FEATURE:
     (A) NAME/KEY: TF binding-site
     (B) LOCATION: 195..202
     (C) IDENTIFICATION METHOD: matinspector prediction
     (D) OTHER INFORMATION:  name USF_C
                             score 0.997
                             sequence TCACGTGC

(ix) FEATURE:
     (A) NAME/KEY: TF binding-site
     (B) LOCATION: complement(195..202)
     (C) IDENTIFICATION METHOD: matinspector prediction
     (D) OTHER INFORMATION:  name USF_C
                             score 0.991
                             sequence GCACGTGA

(ix) FEATURE:
     (A) NAME/KEY: TF binding-site
     (B) LOCATION: complement(210..217)
     (C) IDENTIFICATION METHOD: matinspector prediction
     (D) OTHER INFORMATION:  name MZF1_01
                             score 0.968
                             sequence CATGGGGA

(ix) FEATURE:
     (A) NAME/KEY: TF binding-site
     (B) LOCATION: 397..410
     (C) IDENTIFICATION METHOD: matinspector prediction
     (D) OTHER INFORMATION:  name ELK1_02
                             score 0.963
                             sequence CTCTCCGGAAGCCT

(ix) FEATURE:
     (A) NAME/KEY: TF binding-site
     (B) LOCATION: 400..409
     (C) IDENTIFICATION METHOD: matinspector prediction
     (D) OTHER INFORMATION:  name CETS1P54_01
                             score 0.974
                             sequence TCCGGAAGCC

(ix) FEATURE:
     (A) NAME/KEY: TF binding-site
     (B) LOCATION: complement(460..470)
     (C) IDENTIFICATION METHOD: matinspector prediction
     (D) OTHER INFORMATION:  name AP1_Q4
                             score 0.963
                             sequence AGTGACTGAAC

(ix) FEATURE:
     (A) NAME/KEY: TF binding-site
     (B) LOCATION: complement(460..470)
     (C) IDENTIFICATION METHOD: matinspector prediction
     (D) OTHER INFORMATION:  name AP1FJ_Q2
                             score 0.961
                             sequence AGTGACTGAAC

(ix) FEATURE:

(A) NAME/KEY: TF binding-site
(B) LOCATION: 547..555
(C) IDENTIFICATION METHOD: matinspector prediction
(D) OTHER INFORMATION: name PADS_C
score 1.000
sequence TGTGGTCTC

(xi) SEQUENCE DESCRIPTION: SEQ ID NO: 37:

CTATAGGGCA CGCKTGGTCG ACGGCCCGGG CTGGTCTGGT CTGTKGTGGA GTCGGGTTGA 60

AGGACAGCAT TTGTKACATC TGGTCTACTG CACCTTCCCT CTGCCGTGCA CTTGGCCTTT 120

KAWAAGCTCA GCACCGGTGC CCATCACAGG GCCGGCAGCA CACACATCCC ATTACTCAGA 180

AGGAACTGAC GGACTCACGT GCTGCTCCGT CCCCATGAGC TCAGTGGACC TGTCTATGTA 240

GAGCAGTCAG ACAGTGCCTG GGATAGAGTG AGAGTTCAGC CAGTAAATCC AAGTGATTGT 300

CATTCCTGTC TGCATTAGTA ACTCCCAACC TAGATGTGAA AACTTAGTTC TTTCTCATAG 360

GTTGCTCTGC CCATGGTCCC ACTGCAGACC CAGGCACTCT CCGGAAGCCT GGAAATCACC 420

CGTGTCTTCT GCCTGCTCCC GCTCACATCC CACACTTGTG TTCAGTCACT GAGTTACAGA 480

TTTTGCCTCC TCAATTTCTC TTGTCTTAGT CCCATCCTCT GTTCCCCTGG CCAGTTTGTC 540

TAGCTGTGTG GTCTC 555

(2) INFORMATION FOR SEQ ID NO: 38:

(i) SEQUENCE CHARACTERISTICS:
(A) LENGTH: 140 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 63..122
(C) IDENTIFICATION METHOD: Von Heijne matrix
(D) OTHER INFORMATION: score 15.8
seq LLLLLLLRHGAQG/KP

(xi) SEQUENCE DESCRIPTION: SEQ ID NO: 38:

AACATTTGCG GGAACRSAGA GCGGANSGNG NGACAGCGGA GGAVSTGGAT AACAGGGGAC 60

CG ATG ATG TGG CGA CCA TCA GTT CTG CTG CTT CTG TTG CTA CTG AGG 107
Met Met Trp Arg Pro Ser Val Leu Leu Leu Leu Leu Leu Leu Arg
-20 -15 -10

CAC GGG GCC CAG GGG AAG CCA TCC CCA GAC GCA                    140
His Gly Ala Gln Gly Lys Pro Ser Pro Asp Ala
 -5                  1              5

(2) INFORMATION FOR SEQ ID NO: 39:

(i) SEQUENCE CHARACTERISTICS:
(A) LENGTH: 404 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 285..359
(C) IDENTIFICATION METHOD: Von Heijne matrix
(D) OTHER INFORMATION:  score 14
                        seq LAMLALLSPLSLA/QY

(xi) SEQUENCE DESCRIPTION: SEQ ID NO: 39:

ACTAGTTAAA AGTAAGTGGG AAAAGAGTAA ACGCGCGACT CCAGCGCGCG GCTACCTACG   60

CTTGGTGCTT GCTTTCTCCA GCCATCGGAG ACCAGAGCCG CCCCCTCTGC TCGAGAAAGG  120

GGCTCAGCGG CGGCGGAAGC GGAGGGGGAC CACCGTGGAG AGCGCGGTCC CAGCCCGGCC  180

ACTGCGGATC CCTGNAACCA AAAAGCTCCT GCTGCTTCTG TACCCCGCCT GTCCCTCCCA  240

GCTGCGCAGG GCCCCTTCGT GGGATCATCA GCCCGAAGAC AGGG ATG GAG AGG CCT   296
                                                 Met Glu Arg Pro
                                                 -25

CTG TGC TCC CAC CTC TGC AGC TGC CTG GCT ATG CTG GCC CTC CTG TCC   344
Leu Cys Ser His Leu Cys Ser Cys Leu Ala Met Leu Ala Leu Leu Ser
    -20                 -15                 -10

CCC CTG AGC CTG GCA CAG TAT GAC AGC TGG CCC CAD KAM CCC GAG TAC   392
Pro Leu Ser Leu Ala Gln Tyr Asp Ser Trp Pro Xaa Xaa Pro Glu Tyr
 -5                  1               5                  10

TTC CAG CAA CCG                                                   404
Phe Gln Gln Pro
            15

(2) INFORMATION FOR SEQ ID NO: 40:

(i) SEQUENCE CHARACTERISTICS:

(A) LENGTH: 231 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 67..120
(C) IDENTIFICATION METHOD: Von Heijne matrix
(D) OTHER INFORMATION: score 12.3
seq HILFLLLLPVAAA/QT

(xi) SEQUENCE DESCRIPTION: SEQ ID NO: 40:

AACAGTTCCT CTGGACTTCT CTGGACCACA GTCCTCTGCC AGACCCCTGC CAGACCCCAG 60

TCCACC ATG ATC CAT CTG GGT CAC ATC CTC TTC CTG CTT TTG CTC CCA 108
Met Ile His Leu Gly His Ile Leu Phe Leu Leu Leu Leu Pro
-15 -10 -5

GTG GCT GCA GCT CAG ACG ACT CCA GGA GAG AGA TCA TCA CTC CCT GCC 156
Val Ala Ala Ala Gln Thr Thr Pro Gly Glu Arg Ser Ser Leu Pro Ala
1 5 10

TTT TAC CCT GGC ACT TCA GGC TCT TGT TCC GGA TGT GGG TCC CTC TCT 204
Phe Tyr Pro Gly Thr Ser Gly Ser Cys Ser Gly Cys Gly Ser Leu Ser
15 20 25

CTG CCG CTC CTG GCA GGC CTC GTG GCT 231
Leu Pro Leu Leu Ala Gly Leu Val Ala
30 35

(2) INFORMATION FOR SEQ ID NO: 41:

(i) SEQUENCE CHARACTERISTICS:
(A) LENGTH: 161 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 69..134
(C) IDENTIFICATION METHOD: Von Heijne matrix
(D) OTHER INFORMATION: score 12.2

seq LALALGLAQPASA/RR

(xi) SEQUENCE DESCRIPTION: SEQ ID NO: 41:

ATTTCTCCAT CCTCAGTCTT TGCAAGGCGA CAGCTGTGCC AGCCGGGCTC TGGCAGGCTC 60

CTGGCAGC ATG GCA GTG AAG CTT GGG ACC CTC CTG CTG GCC CTT GCC CTG 110
Met Ala Val Lys Leu Gly Thr Leu Leu Leu Ala Leu Ala Leu
-20 -15 -10

GGC CTG GCC CAG CCA GCC TCT GCC CGC CGG AAG CTG CTG GTG TTT CTG 158
Gly Leu Ala Gln Pro Ala Ser Ala Arg Arg Lys Leu Leu Val Phe Leu
-5 1 5

CTG 161
Leu

(2) INFORMATION FOR SEQ ID NO: 42:

(i) SEQUENCE CHARACTERISTICS:
(A) LENGTH: 284 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: 63..122
(C) IDENTIFICATION METHOD: Von Heijne matrix
(D) OTHER INFORMATION: score 11.9
seq LVLEFLLLSPVEA/QQ

(xi) SEQUENCE DESCRIPTION: SEQ ID NO: 42:

AAAAAACCTG TGGACGCCGA CCCGGGACCG CCGCTGGCTG GCTGCTGGCT CACTCGACCG 60

TC ATG GAG ACC CTG GGG GCC CTT CTG GTG CTG GAG TTT CTG CTC CTC 107
Met Glu Thr Leu Gly Ala Leu Leu Val Leu Glu Phe Leu Leu Leu
-20 -15 -10

TCC CCG GTG GAG GCC CAG CAG GCC ACG GAG CAT CGC CTG AAG CCG TGG 155
Ser Pro Val Glu Ala Gln Gln Ala Thr Glu His Arg Leu Lys Pro Trp
-5 1 5 10

CTG GTG GGC CTG GCT GCG GTA GTC GGC TTC CTG TTC ATC GTC TAT TTG 203
Leu Val Gly Leu Ala Ala Val Val Gly Phe Leu Phe Ile Val Tyr Leu
15 20 25

GTC TTG CTG GCC AAC CGC CTC TGG TGT TCC AAG GCC AGG GCT GAG GAC 251
Val Leu Leu Ala Asn Arg Leu Trp Cys Ser Lys Ala Arg Ala Glu Asp
30 35 40

GAG GAG GAG ACC ACG TTC AGA ATG GAG TCC GGG                    284
Glu Glu Glu Thr Thr Phe Arg Met Glu Ser Gly
     45                  50

(2) INFORMATION FOR SEQ ID NO: 43:

(i) SEQUENCE CHARACTERISTICS:
(A) LENGTH: 233 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 63..110
(C) IDENTIFICATION METHOD: Von Heijne matrix
(D) OTHER INFORMATION:  score 11.3
                        seq PLLLSSLLGGSQA/MD

(xi) SEQUENCE DESCRIPTION: SEQ ID NO: 43:

AACTCACAGC ACGACCAGAG AACAGGCCTG TCTCAGGCAG GCCCTGCGCC TCCTATGCGG   60

AG ATG CTA CTG CCA CTG CTG CTG TCM TCG CTG CTG GGC GGG TCC CAG     107
   Met Leu Leu Pro Leu Leu Leu Ser Ser Leu Leu Gly Gly Ser Gln
       -15                 -10                     -5

GCT ATG GAT GGG AGA TTC TGG ATA CGA GTG CAG GAG TCA GTG ATG GTG    155
Ala Met Asp Gly Arg Phe Trp Ile Arg Val Gln Glu Ser Val Met Val
     1               5                  10                  15

CCG GAG GGC CTG TGC ATC TCT GTN KCC CTG CTC TTT CTC CTA CCC CCG    203
Pro Glu Gly Leu Cys Ile Ser Val Xaa Leu Leu Phe Leu Leu Pro Pro
                20                  25                  30

ACA AGA CTG GAC AGG GTC TAC CCC AGC CGG                            233
Thr Arg Leu Asp Arg Val Tyr Pro Ser Arg
            35                  40

(2) INFORMATION FOR SEQ ID NO: 44:

(i) SEQUENCE CHARACTERISTICS:
(A) LENGTH: 439 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 32..73
(C) IDENTIFICATION METHOD: Von Heijne matrix
(D) OTHER INFORMATION: score 10.7
seq LWLLFFLVTAIHA/EL

(xi) SEQUENCE DESCRIPTION: SEQ ID NO: 44:

AACTTTGCCT TGTGTTTTCC ACCCTGAAAG A ATG TTG TGG CTG CTC TTT TTT 52
Met Leu Trp Leu Leu Phe Phe
-10

CTG GTG ACT GCC ATT CAT GCT GAA CTC TGT CAA CCA GGT GCA GAA AAT 100
Leu Val Thr Ala Ile His Ala Glu Leu Cys Gln Pro Gly Ala Glu Asn
-5 1 5

GCT TTT AAA GTG AGA CTT AGT ATC AGA ACA GCT CTG GGA GAT AAA GCA 148
Ala Phe Lys Val Arg Leu Ser Ile Arg Thr Ala Leu Gly Asp Lys Ala
10 15 20 25

TAT GCC TGG GAT ACC AAT GAA GAA TAC CTC TTC AAA GCG ATG GTA GCT 196
Tyr Ala Trp Asp Thr Asn Glu Glu Tyr Leu Phe Lys Ala Met Val Ala
30 35 40

TTC TCC ATG AGA AAA GTT CCC AAC AGA GAA GCA ACA GAA ATT TCC CAT 244
Phe Ser Met Arg Lys Val Pro Asn Arg Glu Ala Thr Glu Ile Ser His
45 50 55

GTC CTA CTT TGC AAT GTA ACC CAG AGG GTA TCA TTC TGG TTT GTG GTT 292
Val Leu Leu Cys Asn Val Thr Gln Arg Val Ser Phe Trp Phe Val Val
60 65 70

ACA GAC CCT TCA AAA AAT CAC ACC CTT CCT GCT GTT GAG GTG CAA TCA 340
Thr Asp Pro Ser Lys Asn His Thr Leu Pro Ala Val Glu Val Gln Ser
75 80 85

GCC ATA AGA ATG AAC AAG AAC CGG ATC AAC AAT GCC TTC TTT CTA AAT 388
Ala Ile Arg Met Asn Lys Asn Arg Ile Asn Asn Ala Phe Phe Leu Asn
90 95 100 105

GAC CAA ACT CTG GAA TTT TTA AAA ATC CCT TCC ACA CTT GCA CCA ACC 436
Asp Gln Thr Leu Glu Phe Leu Lys Ile Pro Ser Thr Leu Ala Pro Thr
110 115 120

CGG 439
Arg

(2) INFORMATION FOR SEQ ID NO: 45:

(i) SEQUENCE CHARACTERISTICS:
(A) LENGTH: 169 base pairs

(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 20..100
(C) IDENTIFICATION METHOD: Von Heijne matrix
(D) OTHER INFORMATION: score 10.7
seq LPLLCLFLQGATA/VL

(xi) SEQUENCE DESCRIPTION: SEQ ID NO: 45:

AGAGATCGCA GCCCAACCC ATG GCC GGG TCT CCT AGC CGC GCC GCG GGC CGG 52
Met Ala Gly Ser Pro Ser Arg Ala Ala Gly Arg
-25 -20

CGA CTG CAG CTT CCC CTG CTG TGC CTC TTC CTC CAG GGC GCC ACT GCC 100
Arg Leu Gln Leu Pro Leu Leu Cys Leu Phe Leu Gln Gly Ala Thr Ala
-15 -10 -5

GTC CTC TTT GCT GTC TTT GTC CGC TAC AAC CAC AAA ACC GAC GCT GCC 148
Val Leu Phe Ala Val Phe Val Arg Tyr Asn His Lys Thr Asp Ala Ala
1               5                   10                  15

CTC TGG CAM CGG AAG CTT GGG 169
Leu Trp Xaa Arg Lys Leu Gly
            20

(2) INFORMATION FOR SEQ ID NO: 46:

(i) SEQUENCE CHARACTERISTICS:
(A) LENGTH: 204 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 40..156
(C) IDENTIFICATION METHOD: Von Heijne matrix
(D) OTHER INFORMATION: score 10.6
seq ALALLLVLPLLWP/CS

(xi) SEQUENCE DESCRIPTION: SEQ ID NO: 46:

ACTGCCCTGC CCTGGCCTGA CCCCAGGCCT ACTGAGTCC ATG AAA TGG CCC TGG 54
Met Lys Trp Pro Trp
-35

ACC TGC CTT GCC ATC CTC TGT CCT GGC CCT GTA TTG TCC CCA CCA TGC 102
Thr Cys Leu Ala Ile Leu Cys Pro Gly Pro Val Leu Ser Pro Pro Cys
-30 -25 -20

TCT GGT CCA RCG CTT GCC CTA GCC CTG TTG CTA GTC CTG CCA CTG CTA 150
Ser Gly Pro Xaa Leu Ala Leu Ala Leu Leu Leu Val Leu Pro Leu Leu
-15 -10 -5

TGG CCC TGC TCT GTT TTT GGC CAT GCC CTG TGC TAM CCT AGC CCT GCC 198
Trp Pro Cys Ser Val Phe Gly His Ala Leu Cys Xaa Pro Ser Pro Ala
1 5 10

CGA AGG 204
Arg Arg
15

(2) INFORMATION FOR SEQ ID NO: 47:

(i) SEQUENCE CHARACTERISTICS:
(A) LENGTH: 351 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 28..96
(C) IDENTIFICATION METHOD: Von Heijne matrix
(D) OTHER INFORMATION: score 10
seq PLLGLLLSLPAGA/DV

(xi) SEQUENCE DESCRIPTION: SEQ ID NO: 47:

AACCGAGCTG GATTTGTATG TTGCACC ATG CCT TCT TGG ATC GGG GCT GTG ATT 54
Met Pro Ser Trp Ile Gly Ala Val Ile
-20 -15

CTT CCC CTC TTG GGG CTG CTG CTC TCC CTC CCC GCC GGG GCG GAT GTG 102
Leu Pro Leu Leu Gly Leu Leu Leu Ser Leu Pro Ala Gly Ala Asp Val
-10 -5 1

AAG GCT CGG AGC TGC GGA GAG GTC CGC CAG GCG TAC GGT GCC AAG GGA 150
Lys Ala Arg Ser Cys Gly Glu Val Arg Gln Ala Tyr Gly Ala Lys Gly
                5                   10                  15

TTC AGC CTG GCG GAC ATC CCC TAC CAG GAG ATC GCA KGG GAA CAC TTA     198
Phe Ser Leu Ala Asp Ile Pro Tyr Gln Glu Ile Ala Xaa Glu His Leu
     20                  25                  30

AGA ATC TGT CCT CAG GAA TAT ACA TGC TGC ACC ACA GAA ATG GAR GAC     246
Arg Ile Cys Pro Gln Glu Tyr Thr Cys Cys Thr Thr Glu Met Glu Asp
 35                  40                  45                  50

AAG TTA AGC CAA CAA AGC AAA CTC GAA TTT GAA AAC CTT GTG GAA GAG     294
Lys Leu Ser Gln Gln Ser Lys Leu Glu Phe Glu Asn Leu Val Glu Glu
                 55                  60                  65

ACA AGC CAT TTT GTG CGC ACC ACT TTT GTG TCC AGG CAT AAG AAA TTT     342
Thr Ser His Phe Val Arg Thr Thr Phe Val Ser Arg His Lys Lys Phe
             70                  75                  80

GAC GGT AGG                                                         351
Asp Gly Arg
        85

(2) INFORMATION FOR SEQ ID NO: 48:

     (i) SEQUENCE CHARACTERISTICS:
          (A) LENGTH: 242 base pairs
          (B) TYPE: NUCLEIC ACID
          (C) STRANDEDNESS: DOUBLE
          (D) TOPOLOGY: LINEAR

     (ii) MOLECULE TYPE: CDNA

     (vi) ORIGINAL SOURCE:
          (A) ORGANISM: Homo Sapiens
          (D) DEVELOPMENTAL STAGE: Fetal
          (F) TISSUE TYPE: kidney

     (ix) FEATURE:
          (A) NAME/KEY: sig_peptide
          (B) LOCATION: 99..182
          (C) IDENTIFICATION METHOD: Von Heijne matrix
          (D) OTHER INFORMATION:  score 10
                                  seq LWLSLLVPSCLCA/SP

     (xi) SEQUENCE DESCRIPTION: SEQ ID NO: 48:

ACCACTGTGC CCAGCCATTG TCTATACAGT TTGAATAACA CACTGAAAAA ACAGATCAGT   60

GCATATCTTC CACAATTAAC AATGCATTTG TTTAGAGC ATG TTG CTG CAT TGG GTG  116
                                          Met Leu Leu His Trp Val
                                                      -25

CGC TCT CAG GMT GDC AGC GAC KCN AAG CTT TGG TTG AGT TTG CTA GTG     164
Arg Ser Gln Xaa Xaa Ser Asp Xaa Lys Leu Trp Leu Ser Leu Leu Val
        -20                 -15                 -10

CCA AGT TGT TTA TGT GCC TCC CCT TGG CCC CTT CCT TCC CTG CCA CTC     212
Pro Ser Cys Leu Cys Ala Ser Pro Trp Pro Leu Pro Ser Leu Pro Leu
     -5                  1               5                  10

CTT CTT CCT CCC AGC TTG CTG AGC TTG CTG 242
Leu Leu Pro Pro Ser Leu Leu Ser Leu Leu
15 20

(2) INFORMATION FOR SEQ ID NO: 49:

(i) SEQUENCE CHARACTERISTICS:
(A) LENGTH: 289 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 122..223
(C) IDENTIFICATION METHOD: Von Heijne matrix
(D) OTHER INFORMATION: score 9.6
seq LLLFSLLVSPPTC/KV

(xi) SEQUENCE DESCRIPTION: SEQ ID NO: 49:

AAAAACTCTT TCTTCGGCTC GCGAGCTGAG AGGAGCAGGT AGAGGGGCAG AGGCGGGACT 60

GTCGTCTGGG GGAGCCGCCC AGGAGGCTCC TCAGGCCGAC CCCAGACCCT GGCTGGCCAG 120

G ATG AAG TAT CTC CGG CAC CGG CGG CCC AAT GCC ACC CTC ATT CTG GCC 169
Met Lys Tyr Leu Arg His Arg Arg Pro Asn Ala Thr Leu Ile Leu Ala
-30 -25 -20

ATC GGC GCT TTC ACC CTC CTC CTC TTC AGT CTG CTA GTG TCA CCA CCC 217
Ile Gly Ala Phe Thr Leu Leu Leu Phe Ser Leu Leu Val Ser Pro Pro
-15 -10 -5

ACC TGC AAG GTC CAG GAG CAG CCA CCG GCG ATC CCC GAG GCC CTG GCC 265
Thr Cys Lys Val Gln Glu Gln Pro Pro Ala Ile Pro Glu Ala Leu Ala
1 5 10

TGG CHC ACT CCA CCT ACC CGA TGG 289
Trp Xaa Thr Pro Pro Thr Arg Trp
15 20

(2) INFORMATION FOR SEQ ID NO: 50:

(i) SEQUENCE CHARACTERISTICS:
(A) LENGTH: 406 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 26..130
(C) IDENTIFICATION METHOD: Von Heijne matrix
(D) OTHER INFORMATION: score 9.5
seq AMWWLLLWGVLQA/WP

(xi) SEQUENCE DESCRIPTION: SEQ ID NO: 50:

GCAGGTCCCA GATGTCCAGT TCCAG ATG CCT GGA CCC AGA GTG TGG GGG AAA 52
Met Pro Gly Pro Arg Val Trp Gly Lys
-35 -30

TAT CTC TGG AGA AGC CCT CAC TCC AAA GGC TGT CCA GGC GCA ATG TGG 100
Tyr Leu Trp Arg Ser Pro His Ser Lys Gly Cys Pro Gly Ala Met Trp
-25 -20 -15

TGG CTG CTT CTC TGG GGA GTC CTC CAG GCT TGG CCA AMC CCG GGG CTC 148
Trp Leu Leu Leu Trp Gly Val Leu Gln Ala Trp Pro Xaa Pro Gly Leu
-10 -5 1 5

CGT CCT CTT GGC CCA AGA GCT ACC CCA GCA GCT GAC ATC CCC CGG GTA 196
Arg Pro Leu Gly Pro Arg Ala Thr Pro Ala Ala Asp Ile Pro Arg Val
10 15 20

CCC AGA GCC GTA TGG CAA AGG CCA AGA GAG CAG CAC GGA CAT CAA GGC 244
Pro Arg Ala Val Trp Gln Arg Pro Arg Glu Gln His Gly His Gln Gly
25 30 35

TCC AGA GGG CTT TGC TGT GAG GCT CGT CTT CCA GGA CTT CGA CCT GGA 292
Ser Arg Gly Leu Cys Cys Glu Ala Arg Leu Pro Gly Leu Arg Pro Gly
40 45 50

GCC GTC CCA GGA CTG TGC AGG GGA CTC TRW BAC AAT CTC ATT CGT CGG 340
Ala Val Pro Gly Leu Cys Arg Gly Leu Xaa Xaa Asn Leu Ile Arg Arg
55 60 65 70

TTC GGA TCC AAG CCA GTT CTG TGG TCA GCA AGG CTC CCC TCT GGG CAG 388
Phe Gly Ser Lys Pro Val Leu Trp Ser Ala Arg Leu Pro Ser Gly Gln
75 80 85

GCC CCC TGG TCA GAG GGA 406
Ala Pro Trp Ser Glu Gly
90

(2) INFORMATION FOR SEQ ID NO: 51:

(i) SEQUENCE CHARACTERISTICS:
(A) LENGTH: 274 base pairs
(B) TYPE: NUCLEIC ACID
(C) STRANDEDNESS: DOUBLE

(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 62..172
(C) IDENTIFICATION METHOD: Von Heijne matrix
(D) OTHER INFORMATION: score 9.2
seq LLAVLLASWRLWA/IK

(xi) SEQUENCE DESCRIPTION: SEQ ID NO: 51:

AACTGGTGCG GCCGAGTGAC AGTTGACCGG TTTTAACCAA GTGACTGGTT CTAGCCACGT 60

T ATG TGC GGC CCA GCC ATG TTC CCT GCC GGT CCT CCG TGG CCC AGA GTC 109
Met Cys Gly Pro Ala Met Phe Pro Ala Gly Pro Pro Trp Pro Arg Val
-35 -30 -25

CGA GTC GTG CAG GTG CTG TGG GCC CTG CTG GCA GTG CTC CTG GCG TCG 157
Arg Val Val Gln Val Leu Trp Ala Leu Leu Ala Val Leu Leu Ala Ser
-20 -15 -10

TGG AGG CTG TGG GCG ATC AAG GAT TTC CAG GAA TGC ACC TGG CAG GTT 205
Trp Arg Leu Trp Ala Ile Lys Asp Phe Gln Glu Cys Thr Trp Gln Val
-5 1 5 10

GTC CTG AAC GAG TTT AAG AGG GTA GGC GAG AGT GGT GTG AGC GAC AST 253
Val Leu Asn Glu Phe Lys Arg Val Gly Glu Ser Gly Val Ser Asp Xaa
15 20 25

TCT TTG AGC AAG AGC CCG GGG 274
Ser Leu Ser Lys Ser Pro Gly
30

(2) INFORMATION FOR SEQ ID NO: 52:

(i) SEQUENCE CHARACTERISTICS:
(A) LENGTH: 259 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 71..235
(C) IDENTIFICATION METHOD: Von Heijne matrix
(D) OTHER INFORMATION: score 9.2

seq SLLLLSTALNILA/CQ

(xi) SEQUENCE DESCRIPTION: SEQ ID NO: 52:

ACATATCTTT GCAATTGTGA ACATTCAATC ATTTTCAACA CACGTTCATG GTTAATATTT 60

CTAGGAAACT ATG CAT AGA AGA AAA CTT CCT TTA ACC AAT AAA AGG CAA 109
Met His Arg Arg Lys Leu Pro Leu Thr Asn Lys Arg Gln
-55 -50 -45

CTT CAA AAA MCA TTG AGT AAA TTC ATA TTC AGT GAT GAA TTG TTT AGA 157
Leu Gln Lys Xaa Leu Ser Lys Phe Ile Phe Ser Asp Glu Leu Phe Arg
-40 -35 -30

AAT ATT CTC TTT AGT TTA AGA ACA TTA AGG ATG ATA CTA TCA CTA CTT 205
Asn Ile Leu Phe Ser Leu Arg Thr Leu Arg Met Ile Leu Ser Leu Leu
-25 -20 -15

CTG TTG AGC ACT GCA TTG AAT ATC TTA GCC TGC CAA ATA AAT GAA GAA 253
Leu Leu Ser Thr Ala Leu Asn Ile Leu Ala Cys Gln Ile Asn Glu Glu
-10 -5 1 5

CTG GGG 259
Leu Gly

(2) INFORMATION FOR SEQ ID NO: 53:

(i) SEQUENCE CHARACTERISTICS:
(A) LENGTH: 250 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 182..232
(C) IDENTIFICATION METHOD: Von Heijne matrix
(D) OTHER INFORMATION: score 8.3
seq VSALLMAWFGVLS/CV

(xi) SEQUENCE DESCRIPTION: SEQ ID NO: 53:

AAAACGCCGG GAGCTGCGAG TGTCCAGCTG CGGAGACCCG TGATAATTCG TTAACTAATT 60

CAACAAACGG GACCCTTCTG TGTGCCAGAA ACCGCAAGCA GTTGCTAACC CAGTGGGACA 120

GGCGGATTGG AAGAGCGGGA AGGTCCTGGC CCAGAGCAGT GTGACACTTC CCTCTGTGAC 180

C ATG AAA CTC TGG GTG TCT GCA TTG CTG ATG GCC TGG TTT GGT GTC CTG 229
Met Lys Leu Trp Val Ser Ala Leu Leu Met Ala Trp Phe Gly Val Leu
-15 -10 -5

AGC TGT GTG CAG GCC GAD HYG                                    250
Ser Cys Val Gln Ala Xaa Xaa
  1               5

(2) INFORMATION FOR SEQ ID NO: 54:

(i) SEQUENCE CHARACTERISTICS:
(A) LENGTH: 198 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 49..105
(C) IDENTIFICATION METHOD: Von Heijne matrix
(D) OTHER INFORMATION:  score 8.1
seq LCLVCLLVHTAFR/VV

(xi) SEQUENCE DESCRIPTION: SEQ ID NO: 54:

AAGAGCCTGT GCTACTGGAA GGTGGCGTGC CCTCCTCTGG CTGGTACC ATG CAG CTC     57
                                                     Met Gln Leu

CCA CTG GCC CTG TGT CTC GTC TGC CTG CTG GTA CAC ACA GCC TTC CGT     105
Pro Leu Ala Leu Cys Leu Val Cys Leu Leu Val His Thr Ala Phe Arg
    -15                 -10                 -5

GTA GTG GAG GGC CAG GGG TGG CAG GCG TTC AAG AAT GAT GCC ACG GAA     153
Val Val Glu Gly Gln Gly Trp Gln Ala Phe Lys Asn Asp Ala Thr Glu
  1               5                  10                  15

ATC ATC CCC GAG CTC GGA GAG TAC CCC GAG CCT CCA CCG GAA CGG         198
Ile Ile Pro Glu Leu Gly Glu Tyr Pro Glu Pro Pro Pro Glu Arg
            20                  25                  30

(2) INFORMATION FOR SEQ ID NO: 55:

(i) SEQUENCE CHARACTERISTICS:
(A) LENGTH: 206 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens

(F) TISSUE TYPE: Muscle

(ix) FEATURE:
(A) NAME/KEY: sig_peptide
(B) LOCATION: 99..191
(C) IDENTIFICATION METHOD: Von Heijne matrix
(D) OTHER INFORMATION: score 8
seq ILLCSVAVXLSPS/EP

(xi) SEQUENCE DESCRIPTION: SEQ ID NO: 55:

CATAGGGTTT CGAAAATTAT CCACACTTTC TATGGTAATA GAATCTGATA TGGTTCACTC 60

TTGGTGTTGT ACATTCTGTG GGTCTGGGTA AATGTATA ATG TTA TGT ATC CAC CAN 116
Met Leu Cys Ile His Xaa
-30

KAT AGG ATC ATA CAG GAC AGT TTC ATT GCC CTA AAA ATT CTC TTA TGT 164
Xaa Arg Ile Ile Gln Asp Ser Phe Ile Ala Leu Lys Ile Leu Leu Cys
-25 -20 -15 -10

TCT GTC GCT GTA TSM CTG TCT CCC TCC GAG CCC CTG GCG CCG 206
Ser Val Ala Val Xaa Leu Ser Pro Ser Glu Pro Leu Ala Pro
-5 1 5

(2) INFORMATION FOR SEQ ID NO: 56:

(i) SEQUENCE CHARACTERISTICS:
(A) LENGTH: 220 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 8..121
(C) IDENTIFICATION METHOD: Von Heijne matrix
(D) OTHER INFORMATION: score 7.9
seq LPFLSLFWPWAPG/AV

(xi) SEQUENCE DESCRIPTION: SEQ ID NO: 56:

AAGGAGC ATG GGT GGT TTT TTT CCC CCT ACC GAG GTC CGT GAG GTG TGT 49
Met Gly Gly Phe Phe Pro Pro Thr Glu Val Arg Glu Val Cys
-35 -30 -25

GCT AAC CAA GGG GCG GCT CAC AAC CGT GAC AGA CTG CCA TTC CTG AGT 97
Ala Asn Gln Gly Ala Ala His Asn Arg Asp Arg Leu Pro Phe Leu Ser
-20 -15 -10

CTC TTC TGG CCA TGG GCC CCC GGA GCC GTG AGC GTC GGG CAG GCG CGG     145
Leu Phe Trp Pro Trp Ala Pro Gly Ala Val Ser Val Gly Gln Ala Arg
            -5                  1               5

TAC AGA ACA CCA ACG ACA KSA GCG CCC TCA GCA AGC GTT CCC TGG CCG     193
Tyr Arg Thr Pro Thr Thr Xaa Ala Pro Ser Ala Ser Val Pro Trp Pro
    10                  15                  20

CGC GCG GGT ACG TGC AGG ACC CCT ACG                                 220
Arg Ala Gly Thr Cys Arg Thr Pro Thr
25                  30

(2) INFORMATION FOR SEQ ID NO: 57:

(i) SEQUENCE CHARACTERISTICS:
(A) LENGTH: 131 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 21..110
(C) IDENTIFICATION METHOD: Von Heijne matrix
(D) OTHER INFORMATION:  score 7.9
                        seq HLWILLLFSFCWM/SR

(xi) SEQUENCE DESCRIPTION: SEQ ID NO: 57:

ACTTCCCTAT TATTCCTGAA ATG AAA TTA TTT TAC AAC CAG CTC GTT TCA GAA     53
                      Met Lys Leu Phe Tyr Asn Gln Leu Val Ser Glu
                      -30                 -25                 -20

ACA AAA CAT GAT TTT GCA CAT TTG TGG ATT TTG TTG TTA TTC TCA TTT     101
Thr Lys His Asp Phe Ala His Leu Trp Ile Leu Leu Leu Phe Ser Phe
                -15                 -10                 -5

TGT TGG ATG TCT AGA AGC TTT TTT TTT TTT                             131
Cys Trp Met Ser Arg Ser Phe Phe Phe Phe
            1                   5

(2) INFORMATION FOR SEQ ID NO: 58:

(i) SEQUENCE CHARACTERISTICS:
(A) LENGTH: 179 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 111..170
(C) IDENTIFICATION METHOD: Von Heijne matrix
(D) OTHER INFORMATION: score 7.9
seq LLFFHILFHSCFS/HL

(xi) SEQUENCE DESCRIPTION: SEQ ID NO: 58:
ACCTTTAAGA TTACCTGTAT AATAAATGTG TGCAGACACC ATCCAAAAAG GTGTAAAAAA 60
TTGCAAAGGA AAAATAAATA CTGGCCAACA CAGTGTTCTT AAAAGTACCC ATG CCT 116
AGT GAG TCC CCT CCC TTG CTG TTC TTT CAC ATT CTG TTC CAT AGC TGT 164
TTC TCC CAC CTC TTG 179

(2) INFORMATION FOR SEQ ID NO: 59:

(i) SEQUENCE CHARACTERISTICS:
(A) LENGTH: 362 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 18..221
(C) IDENTIFICATION METHOD: Von Heijne matrix
(D) OTHER INFORMATION: score 7.9
seq LLCSALAWQQSLS/GK

(xi) SEQUENCE DESCRIPTION: SEQ ID NO: 59:

ATAAACAGGA AAGCACT ATG TCT TCA ATG TGG TCT GAA TAT ACA ATT GGT 50
Met Ser Ser Met Trp Ser Glu Tyr Thr Ile Gly
-65 -60

GGG GTG AAG ATT TAC TTT CCT TAT AAA GCT TAC CCG TCA CAG CTT GCT 98

Gly Val Lys Ile Tyr Phe Pro Tyr Lys Ala Tyr Pro Ser Gln Leu Ala
      -55                 -50                 -45

ATG ATG AAT TCT ATT CTC AGA GGA TTA AAC AGC AAG CAA CAT TGT TTG     146
Met Met Asn Ser Ile Leu Arg Gly Leu Asn Ser Lys Gln His Cys Leu
    -40                 -35                 -30

TTG GAG AGT CCC ACA GGA AGT GGA AAA AGC TTA GCC TTA CTT TGT TCT     194
Leu Glu Ser Pro Thr Gly Ser Gly Lys Ser Leu Ala Leu Leu Cys Ser
-25                 -20                 -15                 -10

GCT TTA GCA TGG CAA CAA TCT CTT AGT GGG AAA CCA GCA GAT GAG GGC     242
Ala Leu Ala Trp Gln Gln Ser Leu Ser Gly Lys Pro Ala Asp Glu Gly
                 -5                   1               5

GTA AGT GAA AAA GCT GAA GTA CAA TTG TCA TGT TGT TGT GCA TGC CAT     290
Val Ser Glu Lys Ala Glu Val Gln Leu Ser Cys Cys Cys Ala Cys His
        10                  15                  20

TCA AAG GAT TTT ACA AAC AAT GAC ATG AAC CAA GGA ACT TCA CGT CAT     338
Ser Lys Asp Phe Thr Asn Asn Asp Met Asn Gln Gly Thr Ser Arg His
    25                  30                  35

TTC AAC TAT CCA AGC ACA CCA CGG                                     362
Phe Asn Tyr Pro Ser Thr Pro Arg
 40                  45

(2) INFORMATION FOR SEQ ID NO: 60:

      (i) SEQUENCE CHARACTERISTICS:
            (A) LENGTH: 129 base pairs
            (B) TYPE: NUCLEIC ACID
            (C) STRANDEDNESS: DOUBLE
            (D) TOPOLOGY: LINEAR

      (ii) MOLECULE TYPE: CDNA

      (vi) ORIGINAL SOURCE:
            (A) ORGANISM: Homo Sapiens
            (D) DEVELOPMENTAL STAGE: Fetal
            (F) TISSUE TYPE: kidney

      (ix) FEATURE:
            (A) NAME/KEY: sig_peptide
            (B) LOCATION: 19..102
            (C) IDENTIFICATION METHOD: Von Heijne matrix
            (D) OTHER INFORMATION:  score 7.8
                                    seq FVRFLGFVSCLQS/DP

      (xi) SEQUENCE DESCRIPTION: SEQ ID NO: 60:

TAGCTATTTT CAGCGCTT ATG GCT CTG TTC TTG GAG TTA TTT CTA AAT TCT      51
                    Met Ala Leu Phe Leu Glu Leu Phe Leu Asn Ser
                                -25                 -20

TAT TCT CTT TTG TTT GTA AGG TTT CTT GGC TTT GTT TCC TGT TTG CAG      99
Tyr Ser Leu Leu Phe Val Arg Phe Leu Gly Phe Val Ser Cys Leu Gln
    -15                 -10                  -5

TCT GAT CCC ATT TGC TCT TTT TTT TTT TTT 129
Ser Asp Pro Ile Cys Ser Phe Phe Phe Phe
1 5

(2) INFORMATION FOR SEQ ID NO: 61:

(i) SEQUENCE CHARACTERISTICS:
(A) LENGTH: 329 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 114..185
(C) IDENTIFICATION METHOD: Von Heijne matrix
(D) OTHER INFORMATION: score 7.8
seq LMAGSSLSAGVSG/ED

(xi) SEQUENCE DESCRIPTION: SEQ ID NO: 61:

ATACTTCAAA TCTTGAATTA AATGAAGAAA TTTATTTTAC TGATTCTCTT GAAATAAAGA 60

GAAATGAAAA TTTTCCAAAG GATTATGTGA AATTTTCAGA TGAAGAAGAA TTT ATG 116
Met

AAT GAA GAT GAG AAG GAA ATG AAG GAA ATT CTA ATG GCA GGA AGT AGT 164
Asn Glu Asp Glu Lys Glu Met Lys Glu Ile Leu Met Ala Gly Ser Ser
-20 -15 -10

TTA TCA GCT GGA GTT AGT GGG GAA GAT AAA ACC GAG ATA TTG AAT CCC 212
Leu Ser Ala Gly Val Ser Gly Glu Asp Lys Thr Glu Ile Leu Asn Pro
-5 1 5

ACT CCA SCG ATG GCC AAA TCT CTG ACC ATA GAC TGT CTG GAA TTG GCA 260
Thr Pro Xaa Met Ala Lys Ser Leu Thr Ile Asp Cys Leu Glu Leu Ala
10 15 20 25

TTA CCC CCT GAA CTG GCT TTT CAA CTT AAT GAA TTA TTT GGT CCT GTT 308
Leu Pro Pro Glu Leu Ala Phe Gln Leu Asn Glu Leu Phe Gly Pro Val
30 35 40

GGT ATT GAT TCA GGG TCT CTA 329
Gly Ile Asp Ser Gly Ser Leu
45

(2) INFORMATION FOR SEQ ID NO: 62:

(i) SEQUENCE CHARACTERISTICS:
(A) LENGTH: 247 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 167..229
(C) IDENTIFICATION METHOD: Von Heijne matrix
(D) OTHER INFORMATION: score 7.8
seq IIPLIXXLSLCLC/LW

(xi) SEQUENCE DESCRIPTION: SEQ ID NO: 62:

CTATACGTGA TAAGTGAATA AAATGTGTCA GAGTGTACTA CTTAGAATTT TCATAGATTG 60

TAAAGATTTT CTATATATTT ATTTGAATTG GTAATTGGTT ATGAGCAGTT TGGTGTAGCT 120

GTTTTTAATT GTACAACAAT TAAGATATCA CCTATATTCT CGAAGA ATG GGA TCA 175
Met Gly Ser
-20

TTC CTT CTA GGA GGG ATT ATC CCT TTA ATA NNT TTN CTT TCT CTT TGT 223
Phe Leu Leu Gly Gly Ile Ile Pro Leu Ile Xaa Xaa Leu Ser Leu Cys
-15 -10 -5

CTT TGT TTA TGG TGG AGA ATA ATT 247
Leu Cys Leu Trp Trp Arg Ile Ile
1               5

(2) INFORMATION FOR SEQ ID NO: 63:

(i) SEQUENCE CHARACTERISTICS:
(A) LENGTH: 399 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 277..369
(C) IDENTIFICATION METHOD: Von Heijne matrix
(D) OTHER INFORMATION: score 7.8
seq VCLLCSGCSCAWS/VG

(xi) SEQUENCE DESCRIPTION: SEQ ID NO: 63:

ACGAGTGTTA CAGAGGAGAT CTGGTTTCTG GAGGTCTCCA GGATGGGGCT GTAGCCTAAA 60

AGGAAGACTA TGTGAGGCAG CAGGCAAGCA GCAGCAAGTG GAAAGGCTTG GAGATGTGGA 120

GGACGTTATA TGGTACTCAG AGAGCAGCAG TACATGGATG GCAAGTGTGG CGTTGTGCTG 180

CCACCCACTT CCCCATGCCA AAAGCATATA ACTGCTAATC AGTTACCGCA TTTTTTGCTG 240

CCGAATTCGT AAGCAGCCCC AAGAGTTCTC AACAGG ATG CTT CAG GTG GCC ACT 294
Met Leu Gln Val Ala Thr
-30

ACT AAT TAT TTG GAG TTG GCA CGT GAG GTT AAA CCT GTT TGT CTT CTT 342
Thr Asn Tyr Leu Glu Leu Ala Arg Glu Val Lys Pro Val Cys Leu Leu
-25 -20 -15 -10

TGT AGT GGG TGT TCC TGT GCC TGG AGC GTA GGA TGT GTG TKG GAG TCG 390
Cys Ser Gly Cys Ser Cys Ala Trp Ser Val Gly Cys Val Xaa Glu Ser
-5 1 5

GAG TCA GAA 399
Glu Ser Glu
10

(2) INFORMATION FOR SEQ ID NO: 64:

(i) SEQUENCE CHARACTERISTICS:
(A) LENGTH: 240 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 175..228
(C) IDENTIFICATION METHOD: Von Heijne matrix
(D) OTHER INFORMATION: score 7.7
seq [?]FFLALCFPKSTS/QP

(xi) SEQUENCE DESCRIPTION: SEQ ID NO: 64:

ATTACTTTGT CTAGATCAGG AGATGCTAGT ATATTCTTAG CACTAAGACC CCTCTGAAAT 60

CTTGTCCAAC ATTTAGCCAC CCAGRAGTTG TKCTTTACTA CACCTTTGAG GGTTATGCCC 120

TGTACATGTG CAGCTTAGGG GTTCAAGGAC AATCTCTTTA CACATTTTTG GGTT ATG 177
Met

TTC TGT CTA GCT CCT TTC TTT TTA GCA CTC TGC TTC CCA AAA TCT ACC     225
Phe Cys Leu Ala Pro Phe Phe Leu Ala Leu Cys Phe Pro Lys Ser Thr
        -15                 -10                 -5

TCA CAG CCC CAA AGG                                                 240
Ser Gln Pro Gln Arg
    1

(2) INFORMATION FOR SEQ ID NO: 65:

(i) SEQUENCE CHARACTERISTICS:
(A) LENGTH: 451 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 240..335
(C) IDENTIFICATION METHOD: blastn
(D) OTHER INFORMATION:  identity 95
                        region 1..96
                        id AA270737
                        est

(ix) FEATURE:
(A) NAME/KEY: sig_peptide
(B) LOCATION: 236..331
(C) IDENTIFICATION METHOD: Von Heijne matrix
(D) OTHER INFORMATION:  score 7.5
                        seq QCLLCCISPPVFC/EG

(xi) SEQUENCE DESCRIPTION: SEQ ID NO: 65:
TCCTCTTTGC TGTTTTCATC AAGATAGTAG AGCACATCTT CTTCTCACAG ACTACAACTA   60
TGTGGTTCAG CACGAGGCAG TAGAGGAAAG TGCCTCGACT GTGGGAGGCT TGGSCAARAT  120
CCAAAGACTT TCTCTCCTTG TTGCTGGAGT CGCTAAAAGA ACAGTTTAAT AATGCCACAC  180
CCATCCCCAC CCACAGTTGT CCCCTATCTC CAGACCTCAT TCGCAATGAA GTAGA ATG   238
TCT GAA AGC AGA TTT CAA CCA CAG AAT CAA GGA GGT TCT CTT CAA CTC     286
CCT CTT CAG TGC CTA CTA TGT TGC ATT TCT CCC CCT GTG TTT TGT GAA     334
GGT AAC TGG TTA TCT TAC TTT TAT GTG CTT CCT GGA TTT GTG TGT GAA 382
TTA CAT AAA CTG GGT ATT TCT TGT TTA ATC CCC CTT TTC TCT GTC TCC 430
CCT TTG GCA GCC TGG ATG GTG 451

(2) INFORMATION FOR SEQ ID NO: 66:

(i) SEQUENCE CHARACTERISTICS:
(A) LENGTH: 263 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 114..182
(C) IDENTIFICATION METHOD: Von Heijne matrix
(D) OTHER INFORMATION: score 7.3
seq SSCLLGLLHLSSQ/FS

(xi) SEQUENCE DESCRIPTION: SEQ ID NO: 66:
ATGGAGCAGA GGTCCAGCTG TGGTGAGGAT TGGCACAGTC GTGCTTGTGG GACTCCTCCT 60
TGGTCCAACT CTAATGCTCA ACCTACACCA TCACCCCTGT GCTTGCTCCT CTA ATG 116
CCT AAG CAC TGT CAT TCC TTT ATC ACT AGT AGT TGC CTG TTG GGT TTG 164
CTC CAT TTG TCC TCA CAG TTT AGC TGC CCT GGA AGG AAA CTC CAC CCT 212
GCT CAG AGA CAC ACT GAG GCT GAG ACC CAA GGG AGG CCC CTC TCT GAC 260
AGG 263

(2) INFORMATION FOR SEQ ID NO: 67:

(i) SEQUENCE CHARACTERISTICS:
(A) LENGTH: 351 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 166..222
(C) IDENTIFICATION METHOD: Von Heijne matrix
(D) OTHER INFORMATION: score 7.2
seq FIXFPFLFPFSFS/QT

(xi) SEQUENCE DESCRIPTION: SEQ ID NO: 67:

ATCTCTCCTT TTTTCCTGTA ACTGTGCTGG TTTTGTTTTG GTCTTCCTCT CATACCCGTT 60

TCTGCATTTC ATCTTTTCTT TCTATTGTGA CTTCATTTCA TTTTTTTTTT AACCTTATCT 120

TTTGTTTCTC TTGTTTATCC CATCCTTTTT GATAAAATCC ATCGC ATG TGT CTT CTT 177
Met Cys Leu Leu

TTT TYC TTT ATT TYC TTT CCT TTC CTT TTY CCT TTT TCT TTC TCC CAA 225
Phe Xaa Phe Ile Xaa Phe Pro Phe Leu Phe Pro Phe Ser Phe Ser Gln
-15 -10 -5 1

ACT TTT TCC TTT TCA CAG CAT TGG AAC ACG GGA GGT AGT CAC CCA GAA 273
Thr Phe Ser Phe Ser Gln His Trp Asn Thr Gly Gly Ser His Pro Glu
                5                   10                  15

GAA CTT GAG CGG CCT GGT GCC CAT CCG AGA CTT AAG GCT AGA CCC CAG 321
Glu Leu Glu Arg Pro Gly Ala His Pro Arg Leu Lys Ala Arg Pro Gln
            20                  25                  30

CCT CCT CTG TTC CAT CCC TTT ATT AGC TCT 351
Pro Pro Leu Phe His Pro Phe Ile Ser Ser
        35                  40

(2) INFORMATION FOR SEQ ID NO: 68:

(i) SEQUENCE CHARACTERISTICS:
(A) LENGTH: 227 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:

(A) ORGANISM: Homo Sapiens
(F) TISSUE TYPE: Dystrophic muscle

(ix) FEATURE:
(A) NAME/KEY: sig_peptide
(B) LOCATION: 30..104
(C) IDENTIFICATION METHOD: Von Heijne matrix
(D) OTHER INFORMATION: score 7.1
seq LLVASGXAEGVSA/QS

(xi) SEQUENCE DESCRIPTION: SEQ ID NO: 68:

ACGCGCAGAC CCAGCGCCGA GCCCGAGCC ATG GCG TCC GAG CGG MTC CCT AAY 53
Met Ala Ser Glu Arg Xaa Pro Asn
-25 -20

AGG CCC GHC TGT CTG CTC GTR GCC AGC GGC GMC GCC GAR GGT GTG TCG 101
Arg Pro Xaa Cys Leu Leu Val Ala Ser Gly Xaa Ala Glu Gly Val Ser
-15 -10 -5

GCC CAG TCC TTC CTC CAS TGT TTC ACG ATG GCC AGC ACC GSC TTC AAC 149
Ala Gln Ser Phe Leu Xaa Cys Phe Thr Met Ala Ser Thr Xaa Phe Asn
1               5                   10                  15

CTG CAG GTG GCC AYC CCT GGK GGG AAA GCC ATG GAA TTT GTS GAT GTG 197
Leu Gln Val Ala Xaa Pro Gly Gly Lys Ala Met Glu Phe Val Asp Val
            20                  25                  30

ACT GAS AGC AAT GCA CGC TGG GTG CAA GAC 227
Thr Xaa Ser Asn Ala Arg Trp Val Gln Asp
        35                  40

(2) INFORMATION FOR SEQ ID NO: 69:

(i) SEQUENCE CHARACTERISTICS:
(A) LENGTH: 327 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 160..234
(C) IDENTIFICATION METHOD: Von Heijne matrix
(D) OTHER INFORMATION: score 7.1
seq LAFQLVFLRATSG/SC

(xi) SEQUENCE DESCRIPTION: SEQ ID NO: 69:
AATTTCAAGT TGTCATAAAA GTTCAGACAA CCATCACTGG ACCTACAGAT TGAGTGATTA 60
TTATAGTGGG GATGTCCTTG GGTTAGTAAG CCTAAAGGAA GTAATTTCTG TTAAAGGAGA 120
TGTTAGTGGC CATTTGCATC TTAATGTCAA TCTTATCAG ATG TTC CCA GAC TAC 174
AAA CTG GGT GGG TCA TAT CTC TTA GCA TTT CAA CTG GTA TTT CTC AGA 222
GCA ACT AGT GGC TCA TGT TCC AAA TAT AGA AGG CAT TTG CAT AAC ATC 270
AAT GTT AGA CCT GGG CTT GTT AGA CTC TTG GGC TCA TGT ATA CAA AAG 318
CAA CCT GGG 327

(2) INFORMATION FOR SEQ ID NO: 70:

(i) SEQUENCE CHARACTERISTICS:
(A) LENGTH: 370 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 44..118
(C) IDENTIFICATION METHOD: Von Heijne matrix
(D) OTHER INFORMATION: score 7.1
seq LLLXLXLLLIALE/IM

(xi) SEQUENCE DESCRIPTION: SEQ ID NO: 70:

AAATGTGTAC ACGCCCAGCT TCCTGCCTGT TACTCTCCAC AGT ATG CGA AGA ATA 55
Met Arg Arg Ile
-25

TCC CTG ACT TCT AGC CCT GTG CGC CTT CTT TTG TDT CTG CWG TTR CTA 103
Ser Leu Thr Ser Ser Pro Val Arg Leu Leu Leu Xaa Leu Xaa Leu Leu
-20 -15 -10

CTA ATA GCC TTG GAG ATC ATG GTT GGT GGT CAC TCT CTT TGC TTC AAC 151
Leu Ile Ala Leu Glu Ile Met Val Gly Gly His Ser Leu Cys Phe Asn
-5 1 5 10

TTC ACT ATA AAA TCA TTG TCC AGA CCT GGA CAG CCC TGG TGT GAA GCG 199
Phe Thr Ile Lys Ser Leu Ser Arg Pro Gly Gln Pro Trp Cys Glu Ala
15 20 25

CAT GTC TTC TTG AAT AAA AAT CTT TTC CTT CAG TAC AAC AGT GAC AAC 247
His Val Phe Leu Asn Lys Asn Leu Phe Leu Gln Tyr Asn Ser Asp Asn
30 35 40

AAC ATG GTC AAA CCT CTG GGC CTC CTG GGG AAG AAG GTA TAT GCC ACC 295
Asn Met Val Lys Pro Leu Gly Leu Leu Gly Lys Lys Val Tyr Ala Thr
45 50 55

AGC ACT TGG GGA GAA TTG ACC CAA ACG CTG GGA GAA GTG GGG CGA GAC 343
Ser Thr Trp Gly Glu Leu Thr Gln Thr Leu Gly Glu Val Gly Arg Asp
60 65 70 75

CTC AGG ATG CTC CTT TGT GAC ATC AAA 370
Leu Arg Met Leu Leu Cys Asp Ile Lys
80

(2) INFORMATION FOR SEQ ID NO: 71:

(i) SEQUENCE CHARACTERISTICS:
(A) LENGTH: 246 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 193..234
(C) IDENTIFICATION METHOD: Von Heijne matrix
(D) OTHER INFORMATION: score 7
seq TFLLLLFXNAGRS/LR

(xi) SEQUENCE DESCRIPTION: SEQ ID NO: 71:

AAAATATTTC ATATTAGGGA GAGCTCTGTG CTGCCCTTTC CCAAAGCTTT GGTTATTTGA 60

TGGGAGGGGA AGTCTTCTCG AACCTATGTC MGAATATKCC GCTTTGRAAG AGGAGGGTTT 120

TTCTTGAGGC TAGTTTTGTA CCTGCTGTWT CTTTTAGAAA TGATTGCTTT ATGGATTTAA 180

AAGGTGACCC AA ATG ACT TTT TTA TTA TTA TTA TTT KTT AAT GCT GGG AGG 231
Met Thr Phe Leu Leu Leu Leu Phe Xaa Asn Ala Gly Arg
-10 -5

AGT TTG CGT ATG TGT 246
Ser Leu Arg Met Cys
1

(2) INFORMATION FOR SEQ ID NO: 72:

(i) SEQUENCE CHARACTERISTICS:
(A) LENGTH: 328 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 215..292
(C) IDENTIFICATION METHOD: Von Heijne matrix
(D) OTHER INFORMATION: score 7
seq EMFLVLLVTGVHS/NK

(xi) SEQUENCE DESCRIPTION: SEQ ID NO: 72:

AAAAAGTACT GAGAGGTTGA TGGGACTGTT CGATTAGCTC CTCTGAGAAG AAGAGAAAAG 60

GTTCTTGGAC CTCTCCCTGT TTCTTCCTTA GAATAATTTG GATGGGATTT GTGATGCAGA 120

AAAGCCTAAG GGAAAAAGAA TATTCATTCT GTGTGGTGAA AATTTTTTGA AAAAAAAATT 180

GCCTTCTTCA AACAAGGGTG TCATTCTGAT ATTT ATG AGG ACT GTT GTT CTC ACT 235
Met Arg Thr Val Val Leu Thr
-25 -20

ATG AAG GCA TCT GTT ATT GAA ATG TTC CTT GTT TTG CTG GTG ACT GGA 283
Met Lys Ala Ser Val Ile Glu Met Phe Leu Val Leu Leu Val Thr Gly
-15 -10 -5

GTA CAT TCA AAC AAA GAA ACG GCA AAG AAG ATT AAA AGG CCC GGG 328
Val His Ser Asn Lys Glu Thr Ala Lys Lys Ile Lys Arg Pro Gly
1 5 10

(2) INFORMATION FOR SEQ ID NO: 73:

(i) SEQUENCE CHARACTERISTICS:
(A) LENGTH: 281 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
     (A) NAME/KEY: sig_peptide
     (B) LOCATION: 150..269
     (C) IDENTIFICATION METHOD: Von Heijne matrix
     (D) OTHER INFORMATION:  score 6.9
                             seq ISLLFIFFSIANS/SP

(xi) SEQUENCE DESCRIPTION: SEQ ID NO: 73:
ATTCTTTCCT TCTCATATCT ACAATTGCTC CTTTCTAGTT CAGTTCCCTA GTACAGCTGG   60
AGTGATTATT KKSKKTTAAA AAATGCAAGC ATAAAAAAGA AATAAACAAA TAGTTAAATC  120
ATGTTATTCT TTTGTTTACA CTGTAATGA ATG TCT TCC CCA TTG CTT GTA GAA   173
CAA AGT TCT ACA AAG TCT CCC AAA AGC TGG TCC TGG TCC TTT CTA GCT   221
TTC TCT TGC ATA AGT CTT CTT TTT ATT TTT TTC AGC ATT GCA AAT TCT   269
TCC CCC TGC GGG                                                   281

(2) INFORMATION FOR SEQ ID NO: 74:

(i) SEQUENCE CHARACTERISTICS:
     (A) LENGTH: 179 base pairs
     (B) TYPE: NUCLEIC ACID
     (C) STRANDEDNESS: DOUBLE
     (D) TOPOLOGY: LINEAR

(ii) MOLECULE TYPE: CDNA

(vi) ORIGINAL SOURCE:
     (A) ORGANISM: Homo Sapiens
     (D) DEVELOPMENTAL STAGE: Fetal
     (F) TISSUE TYPE: kidney

(ix) FEATURE:
     (A) NAME/KEY: sig_peptide
     (B) LOCATION: 96..170
     (C) IDENTIFICATION METHOD: Von Heijne matrix
     (D) OTHER INFORMATION:  score 6.9
                             seq IPLLLLFFHLSFL/NS

(xi) SEQUENCE DESCRIPTION: SEQ ID NO: 74:

AGAACAAAGT TTAGAATGAT ATGTTTATGC CTGTGAACAT TTATCTTGTT AGATTATGCT   60

CACTAAGCCA TTGGGGTGTT TGGGGAATTT GATCA ATG TAT CTT TTC TGT CTC    113

Met Tyr Leu Phe Cys Leu
-25 -20

TTT TCA GTT TCG AAA ACT ATC CCT CTG CTG CTG CTT TTC TTC CAC TTG 161
Phe Ser Val Ser Lys Thr Ile Pro Leu Leu Leu Leu Phe Phe His Leu
-15 -10 -5

TCT TTT CTC AAT AGC TTG 179
Ser Phe Leu Asn Ser Leu
1

(2) INFORMATION FOR SEQ ID NO: 75:

(i) SEQUENCE CHARACTERISTICS:
(A) LENGTH: 298 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 170..217
(C) IDENTIFICATION METHOD: Von Heijne matrix
(D) OTHER INFORMATION: score 6.9
seq CLLILKFLSPAET/SI

(xi) SEQUENCE DESCRIPTION: SEQ ID NO: 75:

ACAGAGTTCA CTTCTAGGAT ATTCCTTCCC AATCTTCACA GTCACCTCAT AGTCACTATG 60

AGGATTACAT GAGTKAATAT TTGTAAAAAG CGTTCAGGAG AGTGCTTGCT TCACATCAAA 120

TACTATATAT ACTTGTTAAA TAAATAGATC TCATTCACCC CACGAAACA ATG ATC GTT 178
Met Ile Val
-15

TGT CTC CTG ATT CTC AAG TTT TTG TCT CCA GCA GAG ACB TCT ATT CTG 226
Cys Leu Leu Ile Leu Lys Phe Leu Ser Pro Ala Glu Thr Ser Ile Leu
-10 -5 1

AGC TCC ATA GCT ACA TAT GGG GCT TTT TAT TTC ATA GTT CCA CTG GAG 274
Ser Ser Ile Ala Thr Tyr Gly Ala Phe Tyr Phe Ile Val Pro Leu Glu
                5                   10                  15

GTT TCA CAA ATC CTT CAA ACT CAG 298
Val Ser Gln Ile Leu Gln Thr Gln
20 25

(2) INFORMATION FOR SEQ ID NO: 76:

(i) SEQUENCE CHARACTERISTICS:
(A) LENGTH: 275 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 180..254
(C) IDENTIFICATION METHOD: Von Heijne matrix
(D) OTHER INFORMATION: score 6.7
seq LILCFLFILHTHT/HT

(xi) SEQUENCE DESCRIPTION: SEQ ID NO: 76:

ACAAACTGGT TACCCTGCCA CATGTATACC CCCTTCTCCC CATTCTCACT TCCTCGTTAG 60

ACGAAATGAT CATCCAGTGA AGCCATAGAT TATATTGGCC ATCTAATATC AAACCATATT 120

GGTCTCATTT GAAAATCTTT CATGATGCTT TGTGGTATTC ACAGTGAAGT TTAGATTCC 179

ATG GAT AAG AGC ATC AAG TCC TCT ATA ATC TGG TCT CTG ATT CTC TGT 227
Met Asp Lys Ser Ile Lys Ser Ser Ile Ile Trp Ser Leu Ile Leu Cys
-25 -20 -15 -10

TTT CTT TTT ATC CTG CAC ACA CAC ACA CAC ACA CAC ACA CAC ACA CAC 275
Phe Leu Phe Ile Leu His Thr His Thr His Thr His Thr His Thr His
-5 1 5

(2) INFORMATION FOR SEQ ID NO: 77:

(i) SEQUENCE CHARACTERISTICS:
(A) LENGTH: 405 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 283..390
(C) IDENTIFICATION METHOD: Von Heijne matrix
(D) OTHER INFORMATION: score 6.7
seq IFDLLLLLXXSNQ/LP

(xi) SEQUENCE DESCRIPTION: SEQ ID NO: 77:

ACAGACCTCT TTGAAAATCT AATGAGAGCC ATAGACTTCA CCCTAAAAAA ATATATATGC 60

ATAAAAAGTT TAAATATAGT TTGGAGAGTA ACGCACCTTC CCCTAAAGCA ATTCCTAAAC 120

CTCATTTAAA GGATCTATAT TCTATAGTTC AGTTCTGCAT TTTTAATGTC TTCTATATTG 180

TCTCATGCTA GAATAGTCAT TATATCTTCA TATGTAATAT TTAAAGTGTG AATTATCATC 240

TAACACTTCC TGTCTTCTGT CCCCCAAATC TATACTTCTC CC ATG TTC TTT ATT 294
Met Phe Phe Ile
-35

TTC ATT AAT GGC TTT ACW CTC CTT CTA ATG ACC CTA GCC ATG AAA CCC 342
Phe Ile Asn Gly Phe Thr Leu Leu Leu Met Thr Leu Ala Met Lys Pro
-30 -25 -20

AGG CAT CCT ATT TTT GAC CTC TTG CTA TTG CTK RAB HTA TCT AAT CAA 390
Arg His Pro Ile Phe Asp Leu Leu Leu Leu Leu Xaa Xaa Ser Asn Gln
-15 -10 -5

TTG CCA GTT ACG GGG 405
Leu Pro Val Thr Gly
1               5

(2) INFORMATION FOR SEQ ID NO: 78:

(i) SEQUENCE CHARACTERISTICS:
(A) LENGTH: 215 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 3..182
(C) IDENTIFICATION METHOD: Von Heijne matrix
(D) OTHER INFORMATION: score 6.7
seq LWPFLTWINPALS/IC

(xi) SEQUENCE DESCRIPTION: SEQ ID NO: 78:

AC ATG TGC CCT AGT CTG GAA GAG GCT CCC AGT GTC AAG GGG ACT CTG 47
Met Cys Pro Ser Leu Glu Glu Ala Pro Ser Val Lys Gly Thr Leu
-60 -55 -50

CCC TGC TCA GGA CAA CAG CAG CCT TTC CCG TTT GGA GCC TCA AAC ATC 95
Pro Cys Ser Gly Gln Gln Gln Pro Phe Pro Phe Gly Ala Ser Asn Ile

-45                -40                -35                -30

CCA CTA CTC CTG GGC AGG AGC AGA AAG GTG GCT CGA GGT GCA CCG GTC     143
Pro Leu Leu Leu Gly Arg Ser Arg Lys Val Ala Arg Gly Ala Pro Val
                -25                 -20                 -15

CTG TGG CCA TTT CTC ACT TGG ATA AAC CCT GCA CTG TCC ATC TGT GAC     191
Leu Trp Pro Phe Leu Thr Trp Ile Asn Pro Ala Leu Ser Ile Cys Asp
            -10                 -5                  1

CCC TTA GGA TCC TGC GGA TGG CAG                                     215
Pro Leu Gly Ser Cys Gly Trp Gln
    5                   10

(2) INFORMATION FOR SEQ ID NO: 79:

    (i) SEQUENCE CHARACTERISTICS:
        (A) LENGTH: 400 base pairs
        (B) TYPE: NUCLEIC ACID
        (C) STRANDEDNESS: DOUBLE
        (D) TOPOLOGY: LINEAR

    (ii) MOLECULE TYPE: CDNA

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: sig_peptide
        (B) LOCATION: 287..337
        (C) IDENTIFICATION METHOD: Von Heijne matrix
        (D) OTHER INFORMATION:  score 6.6
                                seq LLSALWFCHPCCL/CC

    (xi) SEQUENCE DESCRIPTION: SEQ ID NO: 79:

AAGCTCCAAG GCAGGAAGAG AATTGGGCAT CGGGTACGAA CCTGGCAGCT CAGGAGTCGG    60

GGCTCCACTC ACCCCACACA AAAAGATGAA AAAAGCGCAW AGAGCTCAAT GCATTGATTG   120

GTTTGGCTGG GGACAGCCGG AGAAAGAAGC CCAAGAAAGG CCCAAGCAGT CACCGCCTGC   180

TTCGCACTGA GCCTCCCGAC TCATACTCTG AGTCCAGCTC CGAAGAGGAA GAGGAATTCG   240

GTGTGGTTGG AAATCGCTCT CGCTTTGCCA AGGGAGACTA TTTACG ATG CTG CAA      295
                                                   Met Leu Gln
                                                           -15

GAT CTG TTA TCC GCT CTG TGG TTT TGT CAT CCT TGC TGC CTG TGT TGT     343
Asp Leu Leu Ser Ala Leu Trp Phe Cys His Pro Cys Cys Leu Cys Cys
                -10                 -5                  1

GGC CTG TGT TGG CTT GGT GTG GAT GCA GGT TGC TCT CAA GGA GGA TCT     391
Gly Leu Cys Trp Leu Gly Val Asp Ala Gly Cys Ser Gln Gly Gly Ser
        5                   10                  15

GGA TGC CCG                                                    400
Gly Cys Pro
     20

(2) INFORMATION FOR SEQ ID NO: 80:

      (i) SEQUENCE CHARACTERISTICS:
            (A) LENGTH: 340 base pairs
            (B) TYPE: NUCLEIC ACID
            (C) STRANDEDNESS: DOUBLE
            (D) TOPOLOGY: LINEAR

      (ii) MOLECULE TYPE: CDNA

      (vi) ORIGINAL SOURCE:
            (A) ORGANISM: Homo Sapiens
            (D) DEVELOPMENTAL STAGE: Fetal
            (F) TISSUE TYPE: kidney

      (ix) FEATURE:
            (A) NAME/KEY: sig_peptide
            (B) LOCATION: 167..223
            (C) IDENTIFICATION METHOD: Von Heijne matrix
            (D) OTHER INFORMATION:  score 6.6
                                    seq LLSLAAYLSGPHQ/EP

      (xi) SEQUENCE DESCRIPTION: SEQ ID NO: 80:
AAAATGTCCT CCACAGCTTT GCCCAGTGGG ACACATGGCT CCTGACATAC GTAACCCAGG   60
ATGGGATGCC TTGTTGGAGT CTCTCAGATA TGGAGCAAAA TGGGCCATGT GCAGTCAAGA  120
CGCCATCTAM CCTGGGCAGC TTGCCTAAGC CTCGAGGGAC CTGCCA ATG ATG GAT     175
CTG AGA CCT CTT CTG TCC CTG GCT GCC TAT CTG TCT GGT CCT CAT CAA    223
GAA CCC AGT GTT CCC ACC CGA GAT GGA GAC GTG AAT AAT CTT CCT AAG    271
CCT AAT CCT GCC AGA AGC GTG AAG CAA GGG GGA ATH TGG AAG GCG GAA    319
CAG GAA AGA GTG GAA GTG GAG                                        340

(2) INFORMATION FOR SEQ ID NO: 81:

      (i) SEQUENCE CHARACTERISTICS:
            (A) LENGTH: 245 base pairs
            (B) TYPE: NUCLEIC ACID

(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 147..203
(C) IDENTIFICATION METHOD: Von Heijne matrix
(D) OTHER INFORMATION: score 6.6
seq LLPGLPLVRTSFS/HF

(xi) SEQUENCE DESCRIPTION: SEQ ID NO: 81:
AGCGGTCAGA GGATGCCCTC TTCGCCCTGT GAGCAGCTCT GTGGTTTGCC TCCCCAGATG 60
GCGGGTCCCC GCTTGCACCC CGTGGACACC GGGCACTGGC CACTCCTACA TCCCCAGCTC 120
CACACGGCCT GCACACCTGT GTTTCC ATG GAA ATG CCA CCG TGT CTG CTC CCA 173
GGC CTC CCA CTA GTC AGG ACC AGC TTC AGC CAC TTC TTT TCT CTG AGT 221
GGT GGG ACA ACT ACA GCC AGA GGG 245

(2) INFORMATION FOR SEQ ID NO: 82:

(i) SEQUENCE CHARACTERISTICS:
(A) LENGTH: 192 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 19..93
(C) IDENTIFICATION METHOD: Von Heijne matrix
(D) OTHER INFORMATION: score 6.5
seq GLAMLHVTRGVXG/SR

(xi) SEQUENCE DESCRIPTION: SEQ ID NO: 82:

ACATGCGCAG GAGGCTCA ATG ACA GTC GAG CTT TGG CTA AGG CTC CGG GGA 51
Met Thr Val Glu Leu Trp Leu Arg Leu Arg Gly
-25 -20 -15

AAG GGT CTA GCC ATG CTG CAT GTG ACC CGG GGG GTC TRG GGG TCC AGG 99
Lys Gly Leu Ala Met Leu His Val Thr Arg Gly Val Xaa Gly Ser Arg
-10 -5 1

GTC CGA GTA TRG YCA MTG TTG CCC GCG CTC CTC GGG MCC CCC MGG GCC 147
Val Arg Val Xaa Xaa Xaa Leu Pro Ala Leu Leu Gly Xaa Pro Arg Ala
                5                   10                  15

CTC TCA TCG MTG GCA GCC AAA ATG GGG GAK TAT CGC AAS ATG TGG 192
Leu Ser Ser Xaa Ala Ala Lys Met Gly Xaa Tyr Arg Xaa Met Trp
            20                  25                  30

(2) INFORMATION FOR SEQ ID NO: 83:

(i) SEQUENCE CHARACTERISTICS:
(A) LENGTH: 126 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 7..78
(C) IDENTIFICATION METHOD: Von Heijne matrix
(D) OTHER INFORMATION: score 6.4
seq LLILLCSSPPDRV/SY

(xi) SEQUENCE DESCRIPTION: SEQ ID NO: 83:
ACAAAC ATG TCT ATA GAA GAT TTT GTG AAT AGA AGC ATA CTT CTG ATC 48
TTG CTC TGT TCT TCC CCA CCT GAT AGG GTC AGC TAC AGA GCC AAG GTT 96
TTA CAC TCA TTG CTT CAA TTG CCC GCC CAG 126

(2) INFORMATION FOR SEQ ID NO: 84:

(i) SEQUENCE CHARACTERISTICS:
(A) LENGTH: 184 base pairs
(B) TYPE: NUCLEIC ACID

(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 32..91
(C) IDENTIFICATION METHOD: Von Heijne matrix
(D) OTHER INFORMATION: score 6.4
seq FALLFLFLVPVPG/HG

(xi) SEQUENCE DESCRIPTION: SEQ ID NO: 84:

AAGTCTCAGC GTGGGGTGAA GCCTAGCAGC T ATG AGG ATC CAT TAT CTT CTG 52
Met Arg Ile His Tyr Leu Leu
-20 -15

TTT GCT TTG CTC TTC CTG TTT TTG GTG CCT GTT CCA GGT CAT GGA GGA 100
Phe Ala Leu Leu Phe Leu Phe Leu Val Pro Val Pro Gly His Gly Gly
-10 -5 1

ATC ATA AAC ACA TTA CAG AAA TAT TAW TTG CAG AGT CAG AGG CGG CCG 148
Ile Ile Asn Thr Leu Gln Lys Tyr Xaa Leu Gln Ser Gln Arg Arg Pro
                5                   10                  15

GTG TGC TGT GCT CAG CTG CCT TCC AAA GGA GAA AGG 184
Val Cys Cys Ala Gln Leu Pro Ser Lys Gly Glu Arg
20 25 30

(2) INFORMATION FOR SEQ ID NO: 85:

(i) SEQUENCE CHARACTERISTICS:
(A) LENGTH: 375 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 217..255
(C) IDENTIFICATION METHOD: Von Heijne matrix
(D) OTHER INFORMATION: score 6.4
seq MCLLTALVTQVIS/LR

(xi) SEQUENCE DESCRIPTION: SEQ ID NO: 85:

AATGCCAGTG TCAGCTTCTC TCCGAAAACT GGGTAATACG AAATGGTCTT TATTGGTTGT 60

GAACACTCGA GCTGAGAAAC ATTTTAGGAT CTTTGTGTCT TTTGTGATGA TTTTGTTTCT 120

GRAAGRWGGA AASCTGTCTA AAAATATTCA AGTGTGCAAC CAAGGATTTA GATGAAGCCA 180

GCAAACAAAG GAATCATGTA ATCAGGACCT GAGCGA ATG TGC TTA CTC ACG GCG 234
Met Cys Leu Leu Thr Ala
-10

TTA GTT ACA CAG GTG ATT TCC TTA AGA AAA AAT GCA GAG AGA ACT TGT 282
Leu Val Thr Gln Val Ile Ser Leu Arg Lys Asn Ala Glu Arg Thr Cys
-5 1 5

TTA TGC AAG AGG AGA TGG CCC TGG NGC CCC TCG CCC CGG ATC TAC TGC 330
Leu Cys Lys Arg Arg Trp Pro Trp Xaa Pro Ser Pro Arg Ile Tyr Cys
10 15 20 25

TCA TCC ACC CCA TGC GAT TCC AAA TTC CCC ACC GTC TAC TCC AGT 375
Ser Ser Thr Pro Cys Asp Ser Lys Phe Pro Thr Val Tyr Ser Ser
30 35 40

(2) INFORMATION FOR SEQ ID NO: 86:

(i) SEQUENCE CHARACTERISTICS:
(A) LENGTH: 156 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 76..129
(C) IDENTIFICATION METHOD: Von Heijne matrix
(D) OTHER INFORMATION: score 6.3
seq GLALVAGTPPSRS/CP

(xi) SEQUENCE DESCRIPTION: SEQ ID NO: 86:
ATCTGGCGCG TGGTCTTGCA TTTCCTACTT GGTCCTGTTC GTGGCGCCGC GCCTCCGGGT 60
GTTGGGGAGT CCGGG ATG ATG GGG AAT CCG GGG CTC GCC CTA GTC GCG GGG 111
ACA CCG CCT TCC AGG AGC TGT CCC CAG GCA AAC TCA CAG ACG CGG 156

(2) INFORMATION FOR SEQ ID NO: 87:

(i) SEQUENCE CHARACTERISTICS:
(A) LENGTH: 458 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 186..299
(C) IDENTIFICATION METHOD: Von Heijne matrix
(D) OTHER INFORMATION: score 6.3
seq PCVSLLWAPRXFA/SS

(xi) SEQUENCE DESCRIPTION: SEQ ID NO: 87:

ATAACCCATA TAGTAGTTAA GCCATTGTGG TGAGGGTGTT TGAAACCCAG CTATCCTATG 60

TAATGCTATT TCCAGGGGAA AAATATTCCC AATTCCAGGT AAAAGATCAG AAACAGATAT 120

CACCTGSAWT TTGTTCCACC TTCACCCCAG GCTTCAGCTA TACTTAGGTA TTACTCTCTG 180

GTCCC ATG AAC CAT CTC ATG CCT TTG ACT GTG CTG CAC TCA GTG CTT GAA 230
Met Asn His Leu Met Pro Leu Thr Val Leu His Ser Val Leu Glu
-35 -30 -25

ATG CTC CGC ACA CCC CGC ACA CCT CCC TGG CCC TGT GTA TCC CTT CTA 278
Met Leu Arg Thr Pro Arg Thr Pro Pro Trp Pro Cys Val Ser Leu Leu
-20 -15 -10

TGG GCG CCC AGA GSA TTT GCT TCC TCT TGC TCT CAA GCA TTT ACC ACT 326
Trp Ala Pro Arg Xaa Phe Ala Ser Ser Cys Ser Gln Ala Phe Thr Thr
-5 1 5

CTG CAN KGC AAT TGC TTG CTT ACT AAT CCA TCT CCC ACA CTA GAT TGT 374
Leu Xaa Xaa Asn Cys Leu Leu Thr Asn Pro Ser Pro Thr Leu Asp Cys
10 15 20 25

GAC CTC CCT GAG GGC TCA GAA ATA TTA AAT TCT TCT CTG TAT CCT CAT 422
Asp Leu Pro Glu Gly Ser Glu Ile Leu Asn Ser Ser Leu Tyr Pro His
30 35 40

TGC CTA CTC AGT GCT TGG AAC ACA CGA CAC TCA ACA 458
Cys Leu Leu Ser Ala Trp Asn Thr Arg His Ser Thr
45 50

(2) INFORMATION FOR SEQ ID NO: 88:

(i) SEQUENCE CHARACTERISTICS:
(A) LENGTH: 138 base pairs

(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 13..84
(C) IDENTIFICATION METHOD: Von Heijne matrix
(D) OTHER INFORMATION: score 6.3
seq SLLXLRASQLSEG/DT

(xi) SEQUENCE DESCRIPTION: SEQ ID NO: 88:

ATTATTATTT TT ATG GGA CAT GTT GTG TTT GGG GAT ATA AAA AAT AGT TTA 51
Met Gly His Val Val Phe Gly Asp Ile Lys Asn Ser Leu
-20 -15

TTA KGT TTA AGG GCT TCG CAG CTT AGT GAG GGA GAC ACA TGR VTG AAM 99
Leu Xaa Leu Arg Ala Ser Gln Leu Ser Glu Gly Asp Thr Xaa Xaa Xaa
-10 -5 1 5

TVA TGT CCA BRT ATG RTG AGA GGT AAA CAC ATA TCC TAT 138
Xaa Cys Pro Xaa Met Xaa Arg Gly Lys His Ile Ser Tyr
10 15

(2) INFORMATION FOR SEQ ID NO: 89:

(i) SEQUENCE CHARACTERISTICS:
(A) LENGTH: 341 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 48..290
(C) IDENTIFICATION METHOD: Von Heijne matrix
(D) OTHER INFORMATION: score 6.3
seq FLSLLXSVSETPG/SL

(xi) SEQUENCE DESCRIPTION: SEQ ID NO: 89:

ACTTCTTCCC CGGGTCTCCG AAGCCGCTAG GGAAGCGCAA GTGGGCC ATG GCT GGC 56
Met Ala Gly

-80

GGG AGG CGG GAT TAC AGC CAG CTC TTT GGC CGC GGC CCC GGT CGG CTC     104
Gly Arg Arg Asp Tyr Ser Gln Leu Phe Gly Arg Gly Pro Gly Arg Leu
            -75                 -70                 -65

TCG CGA GCG CGA GCC TCT GTT GTG CGT TGG TCT CCC CGG GCA ACT GCT     152
Ser Arg Ala Arg Ala Ser Val Val Arg Trp Ser Pro Arg Ala Thr Ala
        -60                 -55                 -50

TGC CCT GCG CCA CCG AGC CTC CCG GAT TTA AAG CGG CAG GAG CTG GTT     200
Cys Pro Ala Pro Pro Ser Leu Pro Asp Leu Lys Arg Gln Glu Leu Val
    -45                 -40                 -35

AGC CGG ATA GAA TGT GGG TGC CGA GGG CCG GTG GGG GCC ACC GCA GAC     248
Ser Arg Ile Glu Cys Gly Cys Arg Gly Pro Val Gly Ala Thr Ala Asp
-30                 -25                 -20                 -15

TTC TTT CTG TCC CTG CTC TDC AGC GTC TCT GAA ACC CCT GGC AGC CTG     296
Phe Phe Leu Ser Leu Leu Xaa Ser Val Ser Glu Thr Pro Gly Ser Leu
                -10                 -5                  1

CGG RGA AAC GAT CTT TTC TTC GTC TCT CAG CTT ATT TGG GGC CGG         341
Arg Xaa Asn Asp Leu Phe Phe Val Ser Gln Leu Ile Trp Gly Arg
        5                   10                  15

(2) INFORMATION FOR SEQ ID NO: 90:

(i) SEQUENCE CHARACTERISTICS:
(A) LENGTH: 272 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 207..263
(C) IDENTIFICATION METHOD: Von Heijne matrix
(D) OTHER INFORMATION: score 6.1
seq LWCFHSFISFSLS/SS

(xi) SEQUENCE DESCRIPTION: SEQ ID NO: 90:

ATCCTCCATA GCTATATCCA TTTCCTGGGA CATGGGTTGG CCCAAGAGGG AATGAGAAGG   60

ACCTGCGATT GCACAGGAAA TTCTGGGGCA CATTTAACGT TAAATCATTA AGCTTCTGCC  120

AATAAATCCA TTACTGTTAA TTACACTGAG ATGGCCAACG ATCTGCTGAC AATATTCCTT  180

CATTGATTTT CATTCTCAGT GAATCG ATG TTC TGG CNT GGC TCT CTT TGG TGT   233
                             Met Phe Trp Xaa Gly Ser Leu Trp Cys

-15

TTT CAT TCT TTC ATT TCT TTC TCC CTG TCC TCA TCA CGG 272
Phe His Ser Phe Ile Ser Phe Ser Leu Ser Ser Ser Arg
-10 -5 1

(2) INFORMATION FOR SEQ ID NO: 91:

(i) SEQUENCE CHARACTERISTICS:
(A) LENGTH: 351 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 118..225
(C) IDENTIFICATION METHOD: Von Heijne matrix
(D) OTHER INFORMATION: score 6
seq FLLTFFSYSLLHA/SR

(xi) SEQUENCE DESCRIPTION: SEQ ID NO: 91:

AGGCNNNCGG ASCSGGGCTG GAGAGCGGCS NCCACTGCGG ATCTCGGAAG GAAGAAATGA 60

TGTAAATCAC TCATSSAVAC TTTAAGGTCN NNNGTGAGAM GGAAGGTCAG GMAGAAC 117

ATG GCC TGG CCA AAT GTT TTT CAA ABA GGG TCT CTG CTG TCC CAG TTC 165
Met Ala Trp Pro Asn Val Phe Gln Xaa Gly Ser Leu Leu Ser Gln Phe
-35 -30 -25

AKN BAT CAT CAT GTT GTA GTG TTC CTG CTC ACT TTC TTC AGT TAT TCG 213
Xaa Xaa His His Val Val Val Phe Leu Leu Thr Phe Phe Ser Tyr Ser
-20 -15 -10 -5

TTG CTC CAT GCT TCA CGA AAA ACA TTT RGC AAT GTC AAA GTC AGT ATC 261
Leu Leu His Ala Ser Arg Lys Thr Phe Xaa Asn Val Lys Val Ser Ile
1 5 10

TCT GAG CAG TGG ACC CCA AGT GCT TTT AAC ACG TCA GTT GAG CTG CCT 309
Ser Glu Gln Trp Thr Pro Ser Ala Phe Asn Thr Ser Val Glu Leu Pro
15 20 25

GTG GAG ATC TGG AGC AGC RAC CAT TTG TTC CCC AGT GCA GAG 351
Val Glu Ile Trp Ser Ser Xaa His Leu Phe Pro Ser Ala Glu
30 35 40

(2) INFORMATION FOR SEQ ID NO: 92:

(i) SEQUENCE CHARACTERISTICS:
(A) LENGTH: 466 base pairs
(B) TYPE: NUCLEIC ACID.
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 380..436
(C) IDENTIFICATION METHOD: Von Heijne matrix
(D) OTHER INFORMATION: score 6
seq WILAVGLSLPSSS/XI

(xi) SEQUENCE DESCRIPTION: SEQ ID NO: 92:

ACTCTCTTCT ACTGGAATGG TACCCTTGTT GACTGACTCA TGTATAGCTG CTTGGCTTAA 60

TGGTAGACCA GATATTCAGG TCCTCTGAGA CAGGCCCCTG ATGACTTTTG CAACTACATC 120

TTTCAMCACA GCCTGCCTTG CATTTTGGAC TCTAGCAACA CTGAAATACA TGTCATTTCC 180

CAAGGCATGT TAAGCTGTTT CTATTCTCTA GGCTCTCCCT TTTTCCTAGA ATGCCCTTTT 240

CCTCTAGGCT AATGTCTTTC TCCTTTAAAT TAGTCATCTT CAACAAAGGC TACCTTGACC 300

TTCTCTTGAC TTTGCCACAT TCCTGCTGCT GCCTTCCTTC CATGGCCTTT GTCACGCTAT 360

ATGGTAATTG ACAGGTTCC ATG ATC TTG AGG AAC TTA TGG ATT TTA GCA GTG 412
Met Ile Leu Arg Asn Leu Trp Ile Leu Ala Val
-15 -10

GGT CTT AGC TTG CCA TCT TCT TCA MCC ATC AAG TTT CAT TTC TCT CTT 460
Gly Leu Ser Leu Pro Ser Ser Ser Xaa Ile Lys Phe His Phe Ser Leu
-5 1 5

TAC TCA 466
Tyr Ser
10

(2) INFORMATION FOR SEQ ID NO: 93:

(i) SEQUENCE CHARACTERISTICS:
(A) LENGTH: 389 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens

(D) DEVELOPMENTAL STAGE: Fetal
(F) TISSUE TYPE: kidney

(ix) FEATURE:
(A) NAME/KEY: sig_peptide
(B) LOCATION: 267..371
(C) IDENTIFICATION METHOD: Von Heijne matrix
(D) OTHER INFORMATION: score 5.9
seq LCGLLHLWLKVFS/LK

(xi) SEQUENCE DESCRIPTION: SEQ ID NO: 93:

ACAATCAGTT TGCCAATACC TCAGAAACAA ATACCTCGGA CAAATCTTTC TCTAAAGACC 60

TCAGTCAGAT ACTAGTCAAT ATCAAATCAT GTAGATGGCG GCATTTTAGG CCTCGGACAC 120

CATCCCTACA TGACAGTGAC AATGATGAAC TCTCCTGTAG AAAATTATAT AGGAGTATAA 180

ACCGAACAGG AACAGCACAA CCTGGGACCC AGACATGCAG TACCTCTACG CAAAGTAAAA 240

GTAGCAGTGG TTCAGCACAC TTTGGT ATG TTG ACT GTT AAT GAT GTA CGT TTC 293
Met Leu Thr Val Asn Asp Val Arg Phe
-35 -30

TAT AGA AAT GTC AGG TCC AAC CAT TTC CCA TTT GTT CGA CTA TGT GGT 341
Tyr Arg Asn Val Arg Ser Asn His Phe Pro Phe Val Arg Leu Cys Gly
-25 -20 -15

CTG TTA CAT TTA TGG CTT AAA GTC TTT TCT CTT AAA CAG TTA AAA AAA 389
Leu Leu His Leu Trp Leu Lys Val Phe Ser Leu Lys Gln Leu Lys Lys
-10 -5 1 5

(2) INFORMATION FOR SEQ ID NO: 94:

(i) SEQUENCE CHARACTERISTICS:
(A) LENGTH: 272 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 111..179
(C) IDENTIFICATION METHOD: Von Heijne matrix
(D) OTHER INFORMATION: score 5.9
seq LFLNLCILAXPFS/KQ

(xi) SEQUENCE DESCRIPTION: SEQ ID NO: 94:

ATTAATTTTA ATTTTCATTG TCAATATTTT GAGCTTAGAA CATTTATGGT ATAAAAATTT 60

AAACTAATCA AAGTTGTGTG ATGATTTCCG GGAATTATTA TTGAAAGCCT ATG AAT 116
Met Asn

TTA AAA CCA GGT TTA CCA TGT AAT TTG TTT TTA AAT TTA TGT ATA CTA 164
Leu Lys Pro Gly Leu Pro Cys Asn Leu Phe Leu Asn Leu Cys Ile Leu
-20 -15 -10

GCC TGV CCT TTC TCC AAG CAA ATT ATT GAA CTA TTA GAA TAT GTT AGT 212
Ala Xaa Pro Phe Ser Lys Gln Ile Ile Glu Leu Leu Glu Tyr Val Ser
-5 1 5 10

TAT CAT CCT TGT GTC TTA GTA TAT AGT GAA TAC AGM AAC ATC AGC ATT 260
Tyr His Pro Cys Val Leu Val Tyr Ser Glu Tyr Xaa Asn Ile Ser Ile
15 20 25

GTA TAC ACT CTT 272
Val Tyr Thr Leu
30

(2) INFORMATION FOR SEQ ID NO: 95:

(i) SEQUENCE CHARACTERISTICS:
(A) LENGTH: 345 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 43..162
(C) IDENTIFICATION METHOD: Von Heijne matrix
(D) OTHER INFORMATION: score 5.9
seq VVLAWGLLNVSMA/GM

(xi) SEQUENCE DESCRIPTION: SEQ ID NO: 95:

ACCAGAGAGA GTGGCGCGAG CTGCGTTTTC CGGCCAGAGG AC ATG ATG CAG GGG 54
Met Met Gln Gly
-40

GAG GCA CAC CCT AGT GCT TCC CTT ATT GAC AGA ACC ATC AAG ATG AGA 102
Glu Ala His Pro Ser Ala Ser Leu Ile Asp Arg Thr Ile Lys Met Arg
-35 -30 -25

AAA GAA ACA GAG GCT AGG AAA GTG GTC TTA GCC TGG GGA CTC CTA AAT 150
Lys Glu Thr Glu Ala Arg Lys Val Val Leu Ala Trp Gly Leu Leu Asn
-20 -15 -10 -5

GTA TCT ATG GCT GGA ATG ATA TAT ACT GAA ATG ACT GGA AAA TTG ATT 198
Val Ser Met Ala Gly Met Ile Tyr Thr Glu Met Thr Gly Lys Leu Ile

1                5                   10

AGT TCA TAC TAC AAT GTG ACA TAC TGG CCC CTC TGG TAT ADY GAG CTT     246
Ser Ser Tyr Tyr Asn Val Thr Tyr Trp Pro Leu Trp Tyr Xaa Glu Leu
        15                  20                  25

GCC CTT GCA TCT CTC TTC AGC CTT AAT GCC TTA TTT GAT TTT TGG AGA     294
Ala Leu Ala Ser Leu Phe Ser Leu Asn Ala Leu Phe Asp Phe Trp Arg
    30                  35                  40

TAT TTC AAA TAT ACT GTG GCA CCA ACA AGT CTG GTT GTT AGT CCT GGA     342
Tyr Phe Lys Tyr Thr Val Ala Pro Thr Ser Leu Val Val Ser Pro Gly
45                  50                  55                  60

CGG                                                                 345
Arg

(2) INFORMATION FOR SEQ ID NO: 96:

(i) SEQUENCE CHARACTERISTICS:
(A) LENGTH: 447 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 274..330
(C) IDENTIFICATION METHOD: Von Heijne matrix
(D) OTHER INFORMATION:  score 5.9
seq PXXLLILAHITQS/CP

(xi) SEQUENCE DESCRIPTION: SEQ ID NO: 96:

AGTATTTGTT AAATGCTACA AGAGTGACTG GGATCATAAG TGTTACGGGA GTTTGGCAAA    60

GAAGCAGGAG GTAGTTAGTG TAACTGTTAA TGTGATTATA AGACTAATAC ATTTTGTKGG   120

RAGATAACTT ACCAAGTTTG GTTTGTGGAA AATTTGGATT GAGAAGGAAA TTGTATGTTT   180

CCGTTAGAAG TAGAACAACA ACAACAAAAT ATCTCCCATC ATTTGTTTGG TACTATCTGG   240

CCTCCCCAGT GCTGCTTGGG AGAATCATGA AAC ATG ATG AAT CAA ACA CAT CCT    294
                                     Met Met Asn Gln Thr His Pro
                                                     -15

TRM RTG TTG CTC ATC CTG GCA CAT ATT ACA CAG AGT TGC CCA TGG GCC     342
Xaa Xaa Leu Leu Ile Leu Ala His Ile Thr Gln Ser Cys Pro Trp Ala
        -10                 -5                  1

CAT GTA GGA GCA GCT CCA TCT GCC CTT CTA ATA CAT AGG TGG GAR CTG     390

His Val Gly Ala Ala Pro Ser Ala Leu Leu Ile His Arg Trp Glu Leu
  5                   10                  15                  20

AGG GGG TGC TCG TAT TTG AAA CTG TTT TTG GTT ATG GTG CTC ATA TTT     438
Arg Gly Cys Ser Tyr Leu Lys Leu Phe Leu Val Met Val Leu Ile Phe
                25                  30                  35

GAA ATG CTT                                                         447
Glu Met Leu

(2) INFORMATION FOR SEQ ID NO: 97:

      (i) SEQUENCE CHARACTERISTICS:
           (A) LENGTH: 355 base pairs
           (B) TYPE: NUCLEIC ACID
           (C) STRANDEDNESS: DOUBLE
           (D) TOPOLOGY: LINEAR

      (ii) MOLECULE TYPE: CDNA

      (vi) ORIGINAL SOURCE:
           (A) ORGANISM: Homo Sapiens
           (D) DEVELOPMENTAL STAGE: Fetal
           (F) TISSUE TYPE: kidney

      (ix) FEATURE:
           (A) NAME/KEY: sig_peptide
           (B) LOCATION: 35..94
           (C) IDENTIFICATION METHOD: Von Heijne matrix
           (D) OTHER INFORMATION:  score 5.8
                                   seq GLVLLLSLAEILF/KI

      (xi) SEQUENCE DESCRIPTION: SEQ ID NO: 97:

AGTCCTAGTC AGAGTTTTCT GTGAAGGCAA GGGC ATG GGG TTG CCG GAG AGA AGA    55
                                      Met Gly Leu Pro Glu Arg Arg
                                      -20                 -15

GGA TTG GTC CTG CTT TTA AGC CTA GCT GAA ATT CTT TTC AAG ATC ATG     103
Gly Leu Val Leu Leu Leu Ser Leu Ala Glu Ile Leu Phe Lys Ile Met
        -10                  -5                   1

ATT CTG GAA GGA GGT GGT GTA ATG AAT CTC AAC CCC GGC AAC AAC CTC     151
Ile Leu Glu Gly Gly Gly Val Met Asn Leu Asn Pro Gly Asn Asn Leu
    5                   10                  15

CTT CAC CAG CCG CCA GCC TGG ACA GAC AGC TAC TCC ACG TGC AAT GTT     199
Leu His Gln Pro Pro Ala Trp Thr Asp Ser Tyr Ser Thr Cys Asn Val
 20                  25                  30                  35

TCC AGT GGG TTT TTT GGA GGC CAG TGG CAT GAA ATT CAT CCT CAG TAC     247
Ser Ser Gly Phe Phe Gly Gly Gln Trp His Glu Ile His Pro Gln Tyr
                40                  45                  50

TGG ACC AAG TAC CAG GTG TGG GAG TGG CTC CAG CAC CTC CTG GAC ACC     295
Trp Thr Lys Tyr Gln Val Trp Glu Trp Leu Gln His Leu Leu Asp Thr
            55                  60                  65

AAC CAG CTG GAT GCC AAT TGT ATC CCT TTC CAA GAG TTC GAC ATC AAC     343
Asn Gln Leu Asp Ala Asn Cys Ile Pro Phe Gln Glu Phe Asp Ile Asn
        70                  75                  80

GGC GAG CAM CGG                                                     355
Gly Glu Xaa Arg
    85

(2) INFORMATION FOR SEQ ID NO: 98:

(i) SEQUENCE CHARACTERISTICS:
(A) LENGTH: 409 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 305..388
(C) IDENTIFICATION METHOD: Von Heijne matrix
(D) OTHER INFORMATION:  score 5.8
                        seq LCWALLYNCFSSS/CV

(xi) SEQUENCE DESCRIPTION: SEQ ID NO: 98:

ATCAGTCTGT GGAGACAGGT GAGCACGAAC TTCTGAGACA GGTGTGGGTG CGAGGGTCGG   60

GAGGGTCATG GGATTGGGAC CGAGGTGTGA GGAGGGAATC TGCAATTCCT TGCTACACAG  120

AGCGCTGGCA ACTTCTGACA GGCTGTTTCT GGGGTATGGG CTGCCTCGGG TTGTTGCTGT  180

TACAAGGAAA GAAAAGAGTT CCCCTGCCCA CCGCCTCCCA GCCACTGGGC TACCTCCTGG  240

CAGGAAATTT GCAAACTGAG TTTAACAAGT TAGGATCAGC AGAGGGTAGA GGAGGGCCTG  300

GCAG ATG TGG GGT CTA GAA GAG GAC AGG AGT TAT CAG GGS CTC CGG CCA   349
     Met Trp Gly Leu Glu Glu Asp Arg Ser Tyr Gln Gly Leu Arg Pro
                 -25                 -20                 -15

TTG TGC TGG GCT TTG CTG TAC AAT TGT TTC TCA AGC AGT TGT GTY CCT    397
Leu Cys Trp Ala Leu Leu Tyr Asn Cys Phe Ser Ser Ser Cys Val Pro
            -10                 -5                  1

GTG GCT TTG GTG                                                    409
Val Ala Leu Val
    5

(2) INFORMATION FOR SEQ ID NO: 99:

(i) SEQUENCE CHARACTERISTICS:
(A) LENGTH: 401 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 129..383
(C) IDENTIFICATION METHOD: Von Heijne matrix
(D) OTHER INFORMATION: score 5.7
seq ALLASLGIAFSRS/RA

(xi) SEQUENCE DESCRIPTION: SEQ ID NO: 99:

AGTAGCGGAC ATTTTGTTTC TGTCAGGCTG TCCCTGGCCG GGGTTCTGTA ACGCTTGTGT 60

GGGCCGCAGG TGGAGGTGTT GGGAAAGCGC GGAGGAGATG TTGTCCCCAG TGTCCCGAGA 120

CGCGTCTG ATG CTC TGC AGG GAC GGA AGT GCC TGC GTC CCC CGA TCG AGA 170
Met Leu Cys Arg Asp Gly Ser Ala Cys Val Pro Arg Ser Arg
-85 -80 -75

CGC CTG CCG CTC CCG GCA GCT GTC CGC GCC CAC GGT CCT ATG GCG GAC 218
Arg Leu Pro Leu Pro Ala Ala Val Arg Ala His Gly Pro Met Ala Asp
-70 -65 -60

TGN NCG GAC TCC GCG CGG GGC TGT GTG GTC TTT GAG GAT GTG TTT GTA 266
Xaa Xaa Asp Ser Ala Arg Gly Cys Val Val Phe Glu Asp Val Phe Val
-55 -50 -45 -40

TAC TTC TCT CGG GAA GAA TGG GAG CTT CTT GAT GAT GCT CAG AGA CTT 314
Tyr Phe Ser Arg Glu Glu Trp Glu Leu Leu Asp Asp Ala Gln Arg Leu
-35 -30 -25

TTG TAC CAT GAT GTG ATG CTG GAG AAC TTT GCA CTT TTA GCC TCA CTG 362
Leu Tyr His Asp Val Met Leu Glu Asn Phe Ala Leu Leu Ala Ser Leu
-20 -15 -10

GGA ATT GCA TTT TCC AGA TCA CGT GCA GTC ATG AAA CTA 401
Gly Ile Ala Phe Ser Arg Ser Arg Ala Val Met Lys Leu
-5 1 5

(2) INFORMATION FOR SEQ ID NO: 100:

(i) SEQUENCE CHARACTERISTICS:
(A) LENGTH: 261 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 61..228
(C) IDENTIFICATION METHOD: Von Heijne matrix
(D) OTHER INFORMATION: score 5.7
seq FLCFLNLTSHLSG/LD

(xi) SEQUENCE DESCRIPTION: SEQ ID NO: 100:

ATACCTAATG ATAACACAGT ATCTCTTCGA ATTTGTACTA TTGCAGAACA TTTAGAAACA 60

ATG CTT ATT ACT CGK TTA CAG TCT GGT ATA GAT TTT GCA ATC CAG CTT 108
Met Leu Ile Thr Arg Leu Gln Ser Gly Ile Asp Phe Ala Ile Gln Leu
-55 -50 -45

GAT GAA AGC ACT GAT ATT GGA AGC TGC ACA ACA CTT TTA GTT TAT GTC 156
Asp Glu Ser Thr Asp Ile Gly Ser Cys Thr Thr Leu Leu Val Tyr Val
-40 -35 -30 -25

AGA TAT GCG TGG CAA GAT GAT TTT TTG GAG GAT TTT TTG TGT TTT TTA 204
Arg Tyr Ala Trp Gln Asp Asp Phe Leu Glu Asp Phe Leu Cys Phe Leu
-20 -15 -10

AAT TTA ACC TCA CAC CTA AGT GGA TTA GAT ATT TTT ACA GAA TTA GAA 252
Asn Leu Thr Ser His Leu Ser Gly Leu Asp Ile Phe Thr Glu Leu Glu
-5 1 5

AGG CGC GGG 261
Arg Arg Gly
10

(2) INFORMATION FOR SEQ ID NO: 101:

(i) SEQUENCE CHARACTERISTICS:
(A) LENGTH: 382 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 191..304
(C) IDENTIFICATION METHOD: Von Heijne matrix
(D) OTHER INFORMATION: score 5.7

seq LAFLSCLAFLVLD/TQ

(xi) SEQUENCE DESCRIPTION: SEQ ID NO: 101:

AACTCTGCAG GGCCTCCAAG GCCAGGCTTC AGGGCTGGGA CTCAGTCCTG AGGCACTGGG 60

GAGCCATGAG GGGCTGTGGC AGGGAGGGGC AGGGTGTGGA AAGACTCCCC TGGGGCCATG 120

GTGGAGATGT GCTGAGGTCT TCTCCCTGAT CGTCTTCTCC TCCCTGCTGA CCGACGGCTA 180

CCAGAACKAG ATG GAG TCT CCG CAG CTC CAC TGC ATT CTC AAC AGC AAC 229
Met Glu Ser Pro Gln Leu His Cys Ile Leu Asn Ser Asn
-35 -30

AGC GTG GCC TGC AGC TTT GCC GTG GGA GCC GGC TTC CTG GCC TTC CTC 277
Ser Val Ala Cys Ser Phe Ala Val Gly Ala Gly Phe Leu Ala Phe Leu
-25 -20 -15 -10

AGC TGC CTG GCC TTC CTC GTC CTG GAC ACA CAG GAG ACC CGC ATT GCC 325
Ser Cys Leu Ala Phe Leu Val Leu Asp Thr Gln Glu Thr Arg Ile Ala
-5 1 5

GGC ACC CGC TTC AAG ACA GCC TTC CAG CTC CTG GAC HKC ATC CTG GCT 373
Gly Thr Arg Phe Lys Thr Ala Phe Gln Leu Leu Asp Xaa Ile Leu Ala
10 15 20

GTT CTC TGG 382
Val Leu Trp
25

(2) INFORMATION FOR SEQ ID NO: 102:

(i) SEQUENCE CHARACTERISTICS:
(A) LENGTH: 279 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 190..273
(C) IDENTIFICATION METHOD: Von Heijne matrix
(D) OTHER INFORMATION: score 5.7
seq DHLFLLFPRSCSS/LV

(xi) SEQUENCE DESCRIPTION: SEQ ID NO: 102:
CTCTTGTTAA CCTGTCTTTT GCTATAGGAG TGTCAGACCC TTATGAGGGG AGAGGAGAGA 60
TATCATACTT TTTCTACCTC TACACTTTTA ATATCATTAA TTTTCTAACA ATGCCCAAAT 120
CTTCAGTACA CCTCTCTCTC CTGAACCCTA TACTTGTACA GCAACTTTCT ATGTGACATT 180
TCTTCTTAA ATG TCT AAT AAG TAT ATC AAA CCT AGC ATG TCC CCA GGA AAC 231
ACT GAT CAT CTT TTC CTA CTC TTC CCC CGA AGT TGT TCC TCC CTC GTC 279

(2) INFORMATION FOR SEQ ID NO: 103:

(i) SEQUENCE CHARACTERISTICS:
(A) LENGTH: 340 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 263..334
(C) IDENTIFICATION METHOD: Von Heijne matrix
(D) OTHER INFORMATION: score 5.6
seq FFFFLFLLPPXPP/TG

(xi) SEQUENCE DESCRIPTION: SEQ ID NO: 103:

ATATGTGTAA TGTCTTTATT CCTTAGACTA TGGTCTCCGT GGAAGATTAC TGATACTCCC 60

ACTAGTATTA ATAACAATGT TAGGTAACAT TACTGAATGT TTACTGAGTG CCAGGTAATG 120

TTCTAATTGC TTTACATGTA TTAGGCTATG TATTCCTCAC ATGAACCATA TGAAAGAGAT 180

ACTCTTATTG TTGTCATTTT AGAAGTGAAG AAACTGAGGC ACAGAAAACT TAAGTAATTA 240

GTCCAATTCA TACAGGTAGT AT ATG GTA GAA CTG AAG CAG TTG GGC CCC AGG 292
Met Val Glu Leu Lys Gln Leu Gly Pro Arg
-20 -15

TCT TTT TTT TTC TTT CTT TTT CTT CTG CCG CCG RCT CCT CCA ACC GGG 340
Ser Phe Phe Phe Phe Leu Phe Leu Leu Pro Pro Xaa Pro Pro Thr Gly
-10 -5 1

(2) INFORMATION FOR SEQ ID NO: 104:

(i) SEQUENCE CHARACTERISTICS:
(A) LENGTH: 151 base pairs
(B) TYPE: NUCLEIC ACID
(C) STRANDEDNESS: DOUBLE

(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 17..94
(C) IDENTIFICATION METHOD: Von Heijne matrix
(D) OTHER INFORMATION: score 5.5
seq LILPALFFFPLHC/TF

(xi) SEQUENCE DESCRIPTION: SEQ ID NO: 104:

AAACACCTTC TCAGTG ATG CCT TAC GTC ACC ATC CCA TAT ATA ATA GTG TAC 52
Met Pro Tyr Val Thr Ile Pro Tyr Ile Ile Val Tyr
-25 -20 -15

TCA CTC ATT CTA CCT GCC CTC TTT TTT TTC CCT CTC CAC TGT ACT TTT 100
Ser Leu Ile Leu Pro Ala Leu Phe Phe Phe Pro Leu His Cys Thr Phe
-10 -5 1

CAC GGT CTA ACA TAC TAT ATA TCA TGT GTT TGT TCA TTA TCT CTA CCC 148
His Gly Leu Thr Tyr Tyr Ile Ser Cys Val Cys Ser Leu Ser Leu Pro
                5                   10                  15

ACG 151
Thr

(2) INFORMATION FOR SEQ ID NO: 105:

(i) SEQUENCE CHARACTERISTICS:
(A) LENGTH: 327 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 247..321
(C) IDENTIFICATION METHOD: Von Heijne matrix
(D) OTHER INFORMATION: score 5.5
seq LLLCMDLPHSVLS/NW

(xi) SEQUENCE DESCRIPTION: SEQ ID NO: 105:
AATTATTTTA TAAAACTTCT GGCTGGATTT AAATACTAGG CAGTATTCCA AGGGATGATA 60
AAATGTTTTT ACAAACTTAA TTAGACCCAT TTTTGTAATT AAACTTTATT ATACATGTGC 120
TATGAGGATT AAACTTTGCC TCATAAAAGT ATTCTGACAG GTGCTTTGCA CAGAGTAAGT 180
CCGCCAAAGT GGACGTTCTC ATATGTAATT CTGAGCTTAC TCATACTGGC CAGGAAGGAC 240
GTGCAC ATG CCA CCT TTG GCA GCT GTG ATG GGG AGC CTG CCT CTG CTC 288
TTG TGC ATG GAC CTT CCA CAT TCT GTC CTG TCC AAC TGG 327

(2) INFORMATION FOR SEQ ID NO: 106:

(i) SEQUENCE CHARACTERISTICS:
(A) LENGTH: 254 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 186..248
(C) IDENTIFICATION METHOD: Von Heijne matrix
(D) OTHER INFORMATION: score 5.5
seq EFLFLGFPSNSWP/HR

(xi) SEQUENCE DESCRIPTION: SEQ ID NO: 106:

ACAGCTAGAA TATGTTGGAT TCAGGAGCTT GTCCATTATT TGTAGGTAAA AAAAGCTGCA 60

CGTAGATTTG ACTTCAACTC CGTAAAAAAG ACAGCTGTAT TTTCCGTCCA ACTGGAATTG 120

TTGAATCACA CTGCATAGCT GCCCAAAAGA GAGTGTTTGG TCTTGAACTT TCTATACTTT 180

TATAA ATG TTA CAA ATT CCC GAA AGA AGG GAA TTT CTT TTT CTG GGG TTT 230
Met Leu Gln Ile Pro Glu Arg Arg Glu Phe Leu Phe Leu Gly Phe
-20 -15 -10

CCT TCA AAC TCT TGG CCC CAC AGG 254
Pro Ser Asn Ser Trp Pro His Arg
-5 1

(2) INFORMATION FOR SEQ ID NO: 107:

(i) SEQUENCE CHARACTERISTICS:

(A) LENGTH: 165 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 49..102
(C) IDENTIFICATION METHOD: Von Heijne matrix
(D) OTHER INFORMATION: score 5.5
seq FLITLFCCCVVVG/FF

(xi) SEQUENCE DESCRIPTION: SEQ ID NO: 107:
ACATGTATCT GTTGGCTATT TGTATATCAT CTTTGCATCT TTGGATAA ATG TTC TTT 57
GTC CAT TTT TTA ATC ACT TTA TTT TGT TGT TGT GTT GTA GTG GGG TTT 105
TTT GGC CAT GAT CAT TCA TTT ATC TCA CAG TTC ATT CTT GTT ACT TGG 153
GCC AGG GCA GGG 165

(2) INFORMATION FOR SEQ ID NO: 108:

(i) SEQUENCE CHARACTERISTICS:
(A) LENGTH: 163 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 83..157
(C) IDENTIFICATION METHOD: Von Heijne matrix
(D) OTHER INFORMATION: score 5.5
seq CLLHLRCLQLYWA/AR

(xi) SEQUENCE DESCRIPTION: SEQ ID NO: 108:
ATCAGTGTAT TTTTTTTATA GATTTAAAAT ATACCTGAAA ACTTTTCTAG GAAGAATAAT 60
TATTCATGGA AAGAGCATTG TA ATG GCA TGT TTT GGG GAG AAA AGA CAT GCC 112
AAG TCT TGT TTA CTA CAT TTA AGA TGT TTA CAA CTA TAC TGG GCT GCT 160
CGG 163

(2) INFORMATION FOR SEQ ID NO: 109:

(i) SEQUENCE CHARACTERISTICS:
(A) LENGTH: 374 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 279..362
(C) IDENTIFICATION METHOD: Von Heijne matrix
(D) OTHER INFORMATION: score 5.4
seq PLSLALQSSCCLC/LT

(xi) SEQUENCE DESCRIPTION: SEQ ID NO: 109:

AATAAACCTT ACTTTAACAG AATTTAACAG ATATCTCTTT AAAAAACTGC TTTAATGTTT 60

TTACCTTCTA TCTTCTTTTT CTCCAGCTTT ATCCTGACAG RGAAGTTAGC ACTAATTAAT 120

CTATTTTCTC TTCCCCCTCT TTTTTCCCTT GTGTGTGTCT TTTCTGCCTT CATCTACCCC 180

AGTGAATTTG TTCAGCATTT TGGCTCACTC ATTTCTTCAG CTAACTACAG CTTACTACTA 240

CAGCCACCAC TACTAGAGCC ACTCCTGTCT CATCCTGG ATG GTT GAC AGA GAT GAA 296
Met Val Asp Arg Asp Glu
-25

AAC ATC TTG CTA AAG CAA ATA TAC AGY CCC CTT TCA CTG GCT CTC CAG 344
Asn Ile Leu Leu Lys Gln Ile Tyr Ser Pro Leu Ser Leu Ala Leu Gln
-20 -15 -10

TCC TCC TGC TGT CTT TGC TTG ACC TCC TGC 374
Ser Ser Cys Cys Leu Cys Leu Thr Ser Cys
-5 1

(2) INFORMATION FOR SEQ ID NO: 110:

(i) SEQUENCE CHARACTERISTICS:
(A) LENGTH: 213 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 115..174
(C) IDENTIFICATION METHOD: Von Heijne matrix
(D) OTHER INFORMATION: score 5.4
seq VSVSLCVCDCVRG/ST

(xi) SEQUENCE DESCRIPTION: SEQ ID NO: 110:

ATAAAATTTA CAGAAAAGTT GCAAAGAAGA TAGAATTTCT GCTTAGCTTT TGCCCCAATT 60

TCCCACTTGC CACCCTTCCC TCTTTGTGTT TGTATCTTTT TTTTTCTGAG CCAC ATG 117
Met
-20

AAA GTA AAG CCG CCT TTT GTG TCT GTG TCA CTC TGT GTG TGT GAC TGT 165
Lys Val Lys Pro Pro Phe Val Ser Val Ser Leu Cys Val Cys Asp Cys
-15 -10 -5

GTA AGG GGT AGC ACA CTT ACA TGG AAC AGG TTA CTG CGT GTG GGA GGG 213
Val Arg Gly Ser Thr Leu Thr Trp Asn Arg Leu Leu Arg Val Gly Gly
1 5 10

(2) INFORMATION FOR SEQ ID NO: 111:

(i) SEQUENCE CHARACTERISTICS:
(A) LENGTH: 367 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 68..184

(C) IDENTIFICATION METHOD: Von Heijne matrix
(D) OTHER INFORMATION: score 5.4
seq ILLTSCFYTLVSS/TF

(xi) SEQUENCE DESCRIPTION: SEQ ID NO: 111:

ATGGCTAACA TATTCTTTTT TTTTTCTCTG TAGTAGTTTT TTGAAAGAAG AAATAGGCTA 60

TTCTAGC ATG ATC TCA TCC TGT GGA GTT AAA TAC TTG TTT TCA CAT GCC 109
Met Ile Ser Ser Cys Gly Val Lys Tyr Leu Phe Ser His Ala
-35 -30

TCC TTA TTT TTT ATG GTA GGG AGT ACA GGA AGT TTA ATA CTC TTA ACT 157
Ser Leu Phe Phe Met Val Gly Ser Thr Gly Ser Leu Ile Leu Leu Thr
-25 -20 -15 -10

TCT TGT TTC TAT ACC CTT GTT TCA TCA ACC TTT CTT CAA AAA CTC TCT 205
Ser Cys Phe Tyr Thr Leu Val Ser Ser Thr Phe Leu Gln Lys Leu Ser
-5 1 5

TCT TTG CTC TTG ATA TTA TTT ACC GAA ACA AGT GTY CTT ATG TTA AAA 253
Ser Leu Leu Leu Ile Leu Phe Thr Glu Thr Ser Val Leu Met Leu Lys
10 15 20

ACA TTT GTA GCT AAT TCT TGC TGT WAA TTG TGG TCT CAC AAT TGT ATT 301
Thr Phe Val Ala Asn Ser Cys Cys Xaa Leu Trp Ser His Asn Cys Ile
25 30 35

AAT TTC TTC AAA AAG GTC CKG CCT TCT TAT TGC KGC AGC AGT CTA CTC 349
Asn Phe Phe Lys Lys Val Xaa Pro Ser Tyr Cys Xaa Ser Ser Leu Leu
40 45 50 55

TTC CTG GCC GTA CCT AGG 367
Phe Leu Ala Val Pro Arg
60

(2) INFORMATION FOR SEQ ID NO: 112:

(i) SEQUENCE CHARACTERISTICS:
(A) LENGTH: 248 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 174..233
(C) IDENTIFICATION METHOD: Von Heijne matrix
(D) OTHER INFORMATION: score 5.4
seq SFLCNFLVSLSLS/FL

(xi) SEQUENCE DESCRIPTION: SEQ ID NO: 112:
AGAAGGGGGT GAAAGGAGTA ACTGCTATAT TTAGAAGGAG GTTAAGGATA GCAATTGATT 60
TTAAGGGTGG GGCTAGGGAA CTTGTCTTTA AAATCCTGCA TTTGCACAGC AAGCACAGTT 120
CGTATTGAGA TTTTGCTATT TGGAACTGTA AGGGAGGTAT AGGATGCTGC CTA ATG 176
GGA GGT GGG ATH GCA GAG AGT TTT CTA TGT AAT TTC TTG GTA TCA CTT 224
TCC CTC TCT TTC CTC CAT GGC CGG 248

(2) INFORMATION FOR SEQ ID NO: 113:

(i) SEQUENCE CHARACTERISTICS:
(A) LENGTH: 408 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 265..363
(C) IDENTIFICATION METHOD: Von Heijne matrix
(D) OTHER INFORMATION: score 5.4
seq LAYFLCCQGVIFG/SL

(xi) SEQUENCE DESCRIPTION: SEQ ID NO: 113:

CTATTTCTCA TTGTCTGTCT GGTTTTCCAT CCCCCTCACA TGTGGTGACC AGCACCTGGC 60

CCGCCACGGC AGCCAGGAGG CATTTGTTAA GCGAATAATC GAGACAGGGA AGAGGAGTGG 120

AGTTGGCTGC TCCAGACTCT GCTTAGTTTT CCTTTCTCAA AGTTCTCCCT CCTGTGTCCT 180

AGCCGGGGAA TTAGCTAAAA TGGAATTTTC TTTGGTGATC AGGTATCCTT CTGATGAAGA 240

GAAGAAAGGC CTAAACTCCC AGGC ATG GAT GCA TTA GAA AGA GGT AGT CTT 291
Met Asp Ala Leu Glu Arg Gly Ser Leu
-30 -25

AGA AAT GAG CAG GCG TTG GTT ATT TAT GCA GGA CTG GCA TAC TTT CTG 339
Arg Asn Glu Gln Ala Leu Val Ile Tyr Ala Gly Leu Ala Tyr Phe Leu
-20 -15 -10

TGC TGC CAA GGG GTG ATT TTT GGA AGT CTC CCC TCT AAT GCT GGT GCT     387
Cys Cys Gln Gly Val Ile Phe Gly Ser Leu Pro Ser Asn Ala Gly Ala
            -5                  1               5

GGG CCT TTG GGA TGG TCT AGC                                         408
Gly Pro Leu Gly Trp Ser Ser
    10                  15

(2) INFORMATION FOR SEQ ID NO: 114:

(i) SEQUENCE CHARACTERISTICS:
(A) LENGTH: 209 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 78..194
(C) IDENTIFICATION METHOD: Von Heijne matrix
(D) OTHER INFORMATION:  score 5.3
                        seq SLWFLPLPTHVYT/HT

(xi) SEQUENCE DESCRIPTION: SEQ ID NO: 114:

TTGCTTGAAC CTAACTGTCT TGTTTTTGTC TTCCTGTGAG TTCAAGGACA GGAGCAGTGC   60

TTAACACACA GTAGGTA ATG GAA TAT TTG TTC CAG CAG CCT GGA CAC TCA     110
                   Met Glu Tyr Leu Phe Gln Gln Pro Gly His Ser
                                   -35                 -30

AGG GGA GAA GCC AGG GCT GCT GCT GCC TCT CTG GAA ACC CTG TCT TCC     158
Arg Gly Glu Ala Arg Ala Ala Ala Ala Ser Leu Glu Thr Leu Ser Ser
            -25                 -20                 -15

CTT TGG TTT CTG CCT CTC CCA ACC CAC GTG TAC ACA CAT ACA CAT GCC     206
Leu Trp Phe Leu Pro Leu Pro Thr His Val Tyr Thr His Thr His Ala
        -10                 -5                  1

AAC                                                                 209
Asn
  5

(2) INFORMATION FOR SEQ ID NO: 115:

(i) SEQUENCE CHARACTERISTICS:
(A) LENGTH: 387 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 283..327
(C) IDENTIFICATION METHOD: Von Heijne matrix
(D) OTHER INFORMATION: score 5.3
seq SSMLITILSFIFA/LG

(xi) SEQUENCE DESCRIPTION: SEQ ID NO: 115:

ACCACAGTCA CTGTCACATT ATTCTGTTTT GTATTTTATT TACAGCTCTT ATAATTATCC 60

GAACTTACAA ATTTATTTTC TTGTGTTTTC TCCGCCTGCT CCTCCACTTC ATTCTGTAAT 120

ACTATAGTTC ACTATAATAC TTCTAGTTCC TAGGACTGGA ATTATGTGTC TGGCACATAG 180

TAGACAGTAG ATGTTCATTG AATGAATGAA TGATTCAAAT GAGATTTAAA TAGCAACAGT 240

CCTGACAGAA TGGTAAATTT CCACACTTAA GATGGTCTGT TA ATG GTA TCA TCA 294
Met Val Ser Ser
-15

ATG TTG ATA ACT ATT CTA TCG TTT ATT TTT GCC TTA GGG TAC CAC ACA 342
Met Leu Ile Thr Ile Leu Ser Phe Ile Phe Ala Leu Gly Tyr His Thr
-10 -5 1 5

GCT TCT TAT CCA GTC TCC CTT CAT CCA CTC TCC TTT TTC CTA CAC 387
Ala Ser Tyr Pro Val Ser Leu His Pro Leu Ser Phe Phe Leu His
10 15 20

(2) INFORMATION FOR SEQ ID NO: 116:

(i) SEQUENCE CHARACTERISTICS:
(A) LENGTH: 405 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 316..369
(C) IDENTIFICATION METHOD: Von Heijne matrix
(D) OTHER INFORMATION: score 5.3
seq MNLVSALASSAXG/QR

(xi) SEQUENCE DESCRIPTION: SEQ ID NO: 116:

ACAGTACTTG GAGGTATTCT AAAGGCAGAC ATACTTTATC TGAGCAGGTG CTTTTGGCGT   60

GGTCCTGCCA AGAAAGAAAC AATGGCTTAG ATGACGTCTA TTCTAAGGCC TCAAGGCTTG  120

CACCCCTGCC ATGCTAAATA CAGATGCGCT CCTCCACCAA GAGAATCCCC TCTGCCCTCT  180

GCCATCTCAG CCCCGAGCCA GCTCAGCTGC CCATGACCTG TGTGCAAAGC AGGGGGCGGG  240

ACAAACAGCT ATCGCCTTTG GCCTTCCCTT TGCTCCTGAC AGCGGTCTCA AACCTGGAGG  300

AGTCAAAGGT CCAAG ATG CCT TTG TTC ACT ATG AAC CTG GTG TCA GCT CTA   351
                 Met Pro Leu Phe Thr Met Asn Leu Val Ser Ala Leu
                             -15                     -10

GCG TCC TCA GCA RCA GGG CAG CGT GGA GCA GGG CCA GCC CTC TGG CAC   399
Ala Ser Ser Ala Xaa Gly Gln Arg Gly Ala Gly Pro Ala Leu Trp His
     -5                  1               5                  10

TTG TGT                                                           405
Leu Cys

(2) INFORMATION FOR SEQ ID NO: 117:

(i) SEQUENCE CHARACTERISTICS:
(A) LENGTH: 232 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 110..226
(C) IDENTIFICATION METHOD: Von Heijne matrix
(D) OTHER INFORMATION: score 5.2
seq LILLLHCSIRVFF/FF

(xi) SEQUENCE DESCRIPTION: SEQ ID NO: 117:
CTTGCTTGTA AACATAAGCA TGTATTATTA CCTAGGCTTT GAATTTCAAA ATACGGTGTA   60
AACTACTCAT GGTAATATAG ATCTTGTTAG ACAAACGTTC ATGTAAAAA ATG ATC TGC  118
AAG CAT TAC TGT ATA AAG AAA AAT AAC CTG GAT TAC TTG AAT AGA ATG   166
GTT TAC AGT GCT CAG TTA AAG TTG ATA CTT CTT CTA CAT TGC AGT ATT   214
AGG GTT TTT TTT TTT TTT                                         232

(2) INFORMATION FOR SEQ ID NO: 118:

(i) SEQUENCE CHARACTERISTICS:
(A) LENGTH: 429 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 232..390
(C) IDENTIFICATION METHOD: Von Heijne matrix
(D) OTHER INFORMATION: score 5.2
seq SFLLLQLIHEDKA/IQ

(xi) SEQUENCE DESCRIPTION: SEQ ID NO: 118:

AATTTGAGAA GTGCCCTCCT ATACTTAGAG AAAAGGAATA TCCATATCTC TGAAGACACA   60

GGGACACAGA GAGAATCTGA ACACACAGCC TTGGTAGGAT TCCTTCCGTT TATCATCATT  120

AGATCATAAC CCCYTTTGTC MAGTCCTATT TCTCCARGAC TGCCTCCTTC TTCATTAAAC  180

CTTGCATAAA AACTCACAAA TTTAACCATT TATTTGGATT CTTATTTCCT T ATG AAA  237
                                                         Met Lys

ATT CCT GTG TGG CAT AAA ACG TGC TTT TTA AAA TCT GAA AGT TTT TCT  285
Ile Pro Val Trp His Lys Thr Cys Phe Leu Lys Ser Glu Ser Phe Ser
    -50                 -45                 -40

CCT GAT AAT TTA TCT GTT AGT TTG CCT TGT AGA CCT AGC CAG GTA CCC  333
Pro Asp Asn Leu Ser Val Ser Leu Pro Cys Arg Pro Ser Gln Val Pro
-35                 -30                 -25                 -20

TCA CAG GGG CAA GGA AAA TCT TTT CTC CTC CTA CAA CTT ATA CAT GAG  381
Ser Gln Gly Gln Gly Lys Ser Phe Leu Leu Leu Gln Leu Ile His Glu
                -15                 -10                 -5

GAT AAA GCC ATC CAG AAT GAA GCT ATT TTC CAG CCT TCT CTG CAG CTG  429
Asp Lys Ala Ile Gln Asn Glu Ala Ile Phe Gln Pro Ser Leu Gln Leu
            1               5                   10

(2) INFORMATION FOR SEQ ID NO: 119:

(i) SEQUENCE CHARACTERISTICS:
(A) LENGTH: 222 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 133..189
(C) IDENTIFICATION METHOD: Von Heijne matrix
(D) OTHER INFORMATION: score 5.2
seq FGCTFVAFXPAFA/LS

(xi) SEQUENCE DESCRIPTION: SEQ ID NO: 119:

AGTCTGGGGG TGACATTGCA CCGCGCCCCT CGTGGGGTCG CGTTGCCACC CCACGCGGAC 60

TCCCCAGCTG GCGCGCCCCT CCCATTTGCC TGTCCTGGTC AGGCCCCCAC CCCCCTTCCC 120

ACCTGACCAG CC ATG GGG GCT GCG GTG TTT TTC GGC TGC ACT TTC GTC GCG 171
Met Gly Ala Ala Val Phe Phe Gly Cys Thr Phe Val Ala
-15 -10

TTC DGC CCG GCC TTC GCG CTT TCH TTG ATC ACT GTG GCT GGG GAC CGT 219
Phe Xaa Pro Ala Phe Ala Leu Ser Leu Ile Thr Val Ala Gly Asp Arg
-5 1 5 10

GGG 222
Gly

(2) INFORMATION FOR SEQ ID NO: 120:

(i) SEQUENCE CHARACTERISTICS:
(A) LENGTH: 358 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 80..181
(C) IDENTIFICATION METHOD: Von Heijne matrix

(D) OTHER INFORMATION:  score 5.2
                           seq LWSSCWLAPLADG/ML

(xi) SEQUENCE DESCRIPTION: SEQ ID NO: 120:

AAGATGAAGA GGAGGCDGTG GCAGTGGTGG AAGAAGAGGC GCGGCGGCGG GGGTAGGGAG    60

CCTGGAAACG CGAGCGGGG ATG GTA GGT GGT TTG GAC CCG CCG GGC CGC CGT    112
                     Met Val Gly Gly Leu Asp Pro Pro Gly Arg Arg
                                     -30                 -25

CGT TTC CAG AAA GGG TTT GAC TGG AGG AAC CTC TGG AGC AGC TGT TGG    160
Arg Phe Gln Lys Gly Phe Asp Trp Arg Asn Leu Trp Ser Ser Cys Trp
            -20                 -15                 -10

CTG GCT CCT CTG GCT GAT GGC ATG TTG AGG TAC ATG GGC CAG CVG CAG    208
Leu Ala Pro Leu Ala Asp Gly Met Leu Arg Tyr Met Gly Gln Xaa Gln
        -5                  1               5

CGA NGG GCA TCC AAT CCA GAG GGG TCC ACT CTA GAG GCC AGG CCA CCA    256
Arg Xaa Ala Ser Asn Pro Glu Gly Ser Thr Leu Glu Ala Arg Pro Pro
10                  15                  20                  25

GCA CCA TRG GCC AGT GTG TCA CCA AGT GTA AKH MTC CCT CAT CGA CCC    304
Ala Pro Xaa Ala Ser Val Ser Pro Ser Val Xaa Xaa Pro His Arg Pro
                30                  35                  40

TGG GCA GCA AAA ATG GAG ACC GTG AGC CCA GCA ACA AGT CRC ATA GCA    352
Trp Ala Ala Lys Met Glu Thr Val Ser Pro Ala Thr Ser Xaa Ile Ala
            45                  50                  55

GGC GGG                                                            358
Gly Gly

(2) INFORMATION FOR SEQ ID NO: 121:

    (i) SEQUENCE CHARACTERISTICS:
        (A) LENGTH: 178 base pairs
        (B) TYPE: NUCLEIC ACID
        (C) STRANDEDNESS: DOUBLE
        (D) TOPOLOGY: LINEAR

    (ii) MOLECULE TYPE: CDNA

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: sig_peptide
        (B) LOCATION: 110..172
        (C) IDENTIFICATION METHOD: Von Heijne matrix
        (D) OTHER INFORMATION:  score 5.1
                                seq SLLVVSCFYQISG/RW

    (xi) SEQUENCE DESCRIPTION: SEQ ID NO: 121:
ATAGAACTAC TGCGGAACCT CAAAATCAGT AGATTTGGAA GTGATTCAAA GCTAAACTTT 60
TTCCTTGGCC CTCCKTGTGT TCTAATTGCT TTGCAAGTGT AAKACTAGG ATG TCC AAG 118
ATG CCA GTT TTT GCT TCT TTG TTA GTT GTC AGC TGC TTT TAT CAA ATT 166
TCA GGC CGC TGG 178

(2) INFORMATION FOR SEQ ID NO: 122:

(i) SEQUENCE CHARACTERISTICS:
(A) LENGTH: 204 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 136..180
(C) IDENTIFICATION METHOD: Von Heijne matrix
(D) OTHER INFORMATION: score 5.1
seq VTQLLPFSSPDSA/GP

(xi) SEQUENCE DESCRIPTION: SEQ ID NO: 122:

AACAAAGAGA CACAGACAGG GGACTGTCAG CYGGYACCGG AGGMGCGGAC AACGAGTTAT 60

CAGCAACTSA AAGCACCTGA BGGGCCGCAC ATTCCANCCC CAGCCCAGTC CTCGTCCTCC 120

ACGCCAGCNC CAAGC ATG TSA GTA ACC CAA CTT CTC CCT TTC TCC TCC CCA 171
Met Xaa Val Thr Gln Leu Leu Pro Phe Ser Ser Pro
-15 -10 -5

GAC TCT GCG GGT CCT TTT CTG TCC CCT TTC TCT 204
Asp Ser Ala Gly Pro Phe Leu Ser Pro Phe Ser
1 5

(2) INFORMATION FOR SEQ ID NO: 123:

(i) SEQUENCE CHARACTERISTICS:
(A) LENGTH: 216 base pairs
(B) TYPE: NUCLEIC ACID

(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 1..102
(C) IDENTIFICATION METHOD: Von Heijne matrix
(D) OTHER INFORMATION: score 5.1
seq SFHFLPWALGAMA/SS

(xi) SEQUENCE DESCRIPTION: SEQ ID NO: 123:

ATG GGG AAA GCA TGG CAA GAG ATG AGG GTG GAA TGG GGG GCA GAC AAG 48
Met Gly Lys Ala Trp Gln Glu Met Arg Val Glu Trp Gly Ala Asp Lys
-30 -25 -20

GGG AAT GTC AGA AGC AGC TTC CAC TTT CTC CCC TGG GCA CTG GGA GCC 96
Gly Asn Val Arg Ser Ser Phe His Phe Leu Pro Trp Ala Leu Gly Ala
-15 -10 -5

ATG GCA AGT TCA GAG CAG GGG AAG GAG AGG TCC AAC TTG TGC TTT AGG 144
Met Ala Ser Ser Glu Gln Gly Lys Glu Arg Ser Asn Leu Cys Phe Arg
1 5 10

AAG ACT CCT CTG GCT ATC ACG GGG AGA GGA ATT GCC AGG AGA CCA GGG 192
Lys Thr Pro Leu Ala Ile Thr Gly Arg Gly Ile Ala Arg Arg Pro Gly
15 20 25 30

GGA GGT TGG ATG GGA ATG TGG GTG 216
Gly Gly Trp Met Gly Met Trp Val
35

(2) INFORMATION FOR SEQ ID NO: 124:

(i) SEQUENCE CHARACTERISTICS:
(A) LENGTH: 166 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 2..142
(C) IDENTIFICATION METHOD: Von Heijne matrix
(D) OTHER INFORMATION: score 5.1
seq VIRLSQFLLKCWP/RT

(xi) SEQUENCE DESCRIPTION: SEQ ID NO: 124:

A ATG AAA GTG ATG ATG AGG AAG AGG AAG AAA AAG GAC CAG TGT CTC CCA 49
Met Lys Val Met Met Arg Lys Arg Lys Lys Lys Asp Gln Cys Leu Pro
-45 -40 -35

GGA ATC TGC AGG AGT CTG AAG AGG AGG AAG TCC CCC AGG AGT CCT GGG 97
Gly Ile Cys Arg Ser Leu Lys Arg Arg Lys Ser Pro Arg Ser Pro Gly
-30 -25 -20

ATG AAG GTT ATT CGA CTC TCT CAA TTC CTC CTG AAA TGT TGG CCT CGT 145
Met Lys Val Ile Arg Leu Ser Gln Phe Leu Leu Lys Cys Trp Pro Arg
-15 -10 -5 1

ACA AGT CTT ACA GCA GCT ACG 166
Thr Ser Leu Thr Ala Ala Thr
                5

(2) INFORMATION FOR SEQ ID NO: 125:

(i) SEQUENCE CHARACTERISTICS:
(A) LENGTH: 415 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 254..361
(C) IDENTIFICATION METHOD: Von Heijne matrix
(D) OTHER INFORMATION: score 5
seq SFSIXTLLWGLNC/KR

(xi) SEQUENCE DESCRIPTION: SEQ ID NO: 125:

ACTGTTTTAG TGTTTTGAAT ATCTTCTTCC AGAGTTTGAT GTATATGTAT CTTGGAGGTA 60

TATGTATTTC TAATTATATA AATATTTGAC CCTCTTTGCC TARTTTGTTT TATTCACTTC 120

AACTTTGACC CTTTATACTT CTTTTTAAAT TTCACTTTCT TATGGTTGTT TTTCTACTTT 180

TCCTCAATGC CCTTTGTAAA ATTTTCATTT GAATCTATTA TTCTCCCTTG GACGTCTTAA 240

TTCCTTCTCT ACT ATG ACT TTT TCT TTC TTT TGT TTC TTT CCT GGG TTC 289
Met Thr Phe Ser Phe Phe Cys Phe Phe Pro Gly Phe
-35 -30 -25

AAG CCA CTC CTG TTT CAT TAC TTT CTT TTT WNK TCC TTT TCT ATT TKD 337
Lys Pro Leu Leu Phe His Tyr Phe Leu Phe Xaa Ser Phe Ser Ile Xaa

-20                    -15                    -10

ACT CTK CTT TGG GGC TTG AAC TGT AAG AGG TCC TGG AAC ATA AAT TTG     385
Thr Leu Leu Trp Gly Leu Asn Cys Lys Arg Ser Trp Asn Ile Asn Leu
            -5                  1                   5

AGA ATT GTT GSA TCA TAC AGT AGT GGT TAC                             415
Arg Ile Val Xaa Ser Tyr Ser Ser Gly Tyr
    10                  15

(2) INFORMATION FOR SEQ ID NO: 126:

(i) SEQUENCE CHARACTERISTICS:
(A) LENGTH: 205 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 11..133
(C) IDENTIFICATION METHOD: Von Heijne matrix
(D) OTHER INFORMATION:  score 5
                        seq RLLLILSGCLVYG/TA

(xi) SEQUENCE DESCRIPTION: SEQ ID NO: 126:

AGAGGCAACC ATG GCG GGA GGA ATG AAA GTG GCG GTC TCG CCG GCA GTT      49
           Met Ala Gly Gly Met Lys Val Ala Val Ser Pro Ala Val
               -40                 -35                 -30

GGT CCC GGG CCC TGG GGC TCG GGA GTC GGG GGC GGT GGG ACA GTG CGG     97
Gly Pro Gly Pro Trp Gly Ser Gly Val Gly Gly Gly Gly Thr Val Arg
            -25                 -20                 -15

CTA CTC TTG ATC CTC TCC GGC TGC TTG GTC TAC GGC ACA GCT GAA ACT    145
Leu Leu Leu Ile Leu Ser Gly Cys Leu Val Tyr Gly Thr Ala Glu Thr
        -10                 -5                  1

GAT GTA AAT GTG GTC ATG CTT CAG GAA TCC CAA GTT TGT GAA AAG CGT    193
Asp Val Asn Val Val Met Leu Gln Glu Ser Gln Val Cys Glu Lys Arg
  5                  10                  15                  20

GCC AGC CTC GGG                                                    205
Ala Ser Leu Gly

(2) INFORMATION FOR SEQ ID NO: 127:

(i) SEQUENCE CHARACTERISTICS:

(A) LENGTH: 240 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 58..153
(C) IDENTIFICATION METHOD: Von Heijne matrix
(D) OTHER INFORMATION: score 5
seq PLLSCSCPPPLLG/EG

(xi) SEQUENCE DESCRIPTION: SEQ ID NO: 127:

ACTTCCACGG GACCCACCAG CTTAAATGCC GGCAGCCCTG GGACTTCTGG CCTCACA 57

ATG GTT GAG ATG ACT GGG GTG TGG CAG TGC CAA GCC GAG GCT GTG AAA 105
Met Val Glu Met Thr Gly Val Trp Gln Cys Gln Ala Glu Ala Val Lys
-30 -25 -20

GGC CTT CCA CCT TTA CTC TCG TGC TCG TGC CCT CCC CCA TTG TTA GGA 153
Gly Leu Pro Pro Leu Leu Ser Cys Ser Cys Pro Pro Pro Leu Leu Gly
-15 -10 -5

GAA GGG CAT GCT CAG GCC AGC CCA TTA GCC CAG GAG GAG GAC AAG AAA 201
Glu Gly His Ala Gln Ala Ser Pro Leu Ala Gln Glu Glu Asp Lys Lys
1               5                   10                  15

CAC ACG GAG CAG ACA CAA GCC ACC TCA CCA ACC CAG CCT 240
His Thr Glu Gln Thr Gln Ala Thr Ser Pro Thr Gln Pro
            20                  25

(2) INFORMATION FOR SEQ ID NO: 128:

(i) SEQUENCE CHARACTERISTICS:
(A) LENGTH: 157 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 59..121
(C) IDENTIFICATION METHOD: Von Heijne matrix
(D) OTHER INFORMATION: score 5

seq AGLLPLLLGNAPG/ES

(xi) SEQUENCE DESCRIPTION: SEQ ID NO: 128:

AATTTGCTCA CACCCAGCAG GCAGAGAAGG CAGCAGCAGG CAGGACCGCC ACCCTCCC 58

ATG CAA ATC ACC CCC GGG AGT GCA GCT GGG CTC CTC CCG CTC CTC CTA 106
Met Gln Ile Thr Pro Gly Ser Ala Ala Gly Leu Leu Pro Leu Leu Leu
-20 -15 -10

GGC AAT GCT CCT GGG GAG TCT GTT GGG GGA AGA TGC SAT CCA GGG TGC 154
Gly Asn Ala Pro Gly Glu Ser Val Gly Gly Arg Cys Xaa Pro Gly Cys
-5 1 5 10

TGG 157
Trp

(2) INFORMATION FOR SEQ ID NO: 129:

(i) SEQUENCE CHARACTERISTICS:
(A) LENGTH: 250 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 152..202
(C) IDENTIFICATION METHOD: Von Heijne matrix
(D) OTHER INFORMATION: score 5
seq TWLLLTLQNSVFT/SF

(xi) SEQUENCE DESCRIPTION: SEQ ID NO: 129:
AGAATTTTGC TGGGAATTAA TATTAAATAC TCACTGGAAT TTATCTTTAC CAACTTTAGT 60
GGAATTCAGC CTATCTACAG CTCTCCTTTC CACTTTGTTT CTCAGAAATT CTCAGCAATG 120
GTTTCATGAA CCACTGGGAG GTCATTTGCC T ATG ATT TTG TCC ACC TGG CTC 172
TTA CTT ACC CTT CAA AAC TCA GTA TTT ACA TCT TTC AGG ATA TCT CCC 220
AAC AGA ATA CAA AGT ATG CTA CCT CCC ATG 250

(2) INFORMATION FOR SEQ ID NO: 130:

(i) SEQUENCE CHARACTERISTICS:
(A) LENGTH: 206 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 33..128
(C) IDENTIFICATION METHOD: Von Heijne matrix
(D) OTHER INFORMATION: score 5
seq VCIVLALCHTSRP/MS

(xi) SEQUENCE DESCRIPTION: SEQ ID NO: 130:
AAATCTCTTC TAATCCTCCT TAATGCATTT TG ATG GCT TTT CAT AGC TAT TGG 53
GGA AAA AGT TTA CAA TCC TTT AAG ACG TTC ATG AGA GTC TGC ATT GTC 101
TTG GCC CTT TGC CAC ACA TCC AGA CCC ATG TCT TAC CAT GTT CCC CTG 149
GCT GCT GGC TCC CCA CTC ATG CAC TGG TCT CCT TGT AGT CCT GTG CCC 197
TTC ATT GGG 206

(2) INFORMATION FOR SEQ ID NO: 131:

(i) SEQUENCE CHARACTERISTICS:
(A) LENGTH: 184 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: 113..160
(C) IDENTIFICATION METHOD: Von Heijne matrix
(D) OTHER INFORMATION: score 4.9
seq RFTLLPLVLHSQS/SC

(xi) SEQUENCE DESCRIPTION: SEQ ID NO: 131:

ATTTCTCGTA AATGATGAGA TGGGGTTAAA TGGTTTTGCA GAAATATGTG AGAGGTAATG 60

TGAAATAAGT TACTTTAAGA AGGCCTGGCC CTGGTAATGT CGTTACCAGC TG ATG AAG 118
Met Lys
-15

TTG CGG TTT ACC TTG CTG CCC CTG GTG CTA CAT TCA CAA AGC AGC TGT 166
Leu Arg Phe Thr Leu Leu Pro Leu Val Leu His Ser Gln Ser Ser Cys
-10 -5 1

GTC TTT TGG AAA GCC GGG 184
Val Phe Trp Lys Ala Gly
                5

(2) INFORMATION FOR SEQ ID NO: 132:

(i) SEQUENCE CHARACTERISTICS:
(A) LENGTH: 156 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 4..93
(C) IDENTIFICATION METHOD: Von Heijne matrix
(D) OTHER INFORMATION: score 4.9
seq FIPFLVIYSFVLS/SP

(xi) SEQUENCE DESCRIPTION: SEQ ID NO: 132:
ACC ATG ATG ATC ATT CTG GGG TTT GCT TTT TGC CCT GGT CAC TTT AGG 48
TTT AAT TTT ATT CCA TTC CTG GTC ATT TAC AGT TTT GTT CTG TCA TCT 96
CCC CAT ACC CAT CGA GAA CCC TAT TCT CCT GTG GCA GAC TTT AAT GAA 144
TGT AAC CGC AGT                                                    156

(2) INFORMATION FOR SEQ ID NO: 133:

    (i) SEQUENCE CHARACTERISTICS:
        (A) LENGTH: 335 base pairs
        (B) TYPE: NUCLEIC ACID
        (C) STRANDEDNESS: DOUBLE
        (D) TOPOLOGY: LINEAR

    (ii) MOLECULE TYPE: CDNA

    (vi) ORIGINAL SOURCE:
        (A) ORGANISM: Homo Sapiens
        (F) TISSUE TYPE: Heart

    (ix) FEATURE:
        (A) NAME/KEY: sig_peptide
        (B) LOCATION: 198..278
        (C) IDENTIFICATION METHOD: Von Heijne matrix
        (D) OTHER INFORMATION:  score 4.9
                                seq CLLSYIALGAIHA/KI

    (xi) SEQUENCE DESCRIPTION: SEQ ID NO: 133:

AACTTTGCCT GGGTGTCTTG CGTTCTGCAC ATTCCGGAGG ACCAGCTTCC CCATCAGAAG   60

TCTGACTCCA TGGAAACCAG ATGGGGCAAC GGGGTGGTTC TAGTGCAGAC TGTAGCTGCA  120

GCTCCTCTCC ACCTCTAGCC TGCTCATTTC CAGCTCAGAA ATTCTACTAA TGGCGTTTTT  180

TCTTCCTGAA AAAGGAA ATG AAC AGG GTC CCT GCT GAT TCT CCA AAT ATG     230
                   Met Asn Arg Val Pro Ala Asp Ser Pro Asn Met
                           -25                 -20

TGT CTA ATC TGT TTA CTG AGT TAC ATA GCA CTT GGA GCC ATC CAT GCA    278
Cys Leu Ile Cys Leu Leu Ser Tyr Ile Ala Leu Gly Ala Ile His Ala
    -15                 -10                 -5

AAA ATC TGT AGA AGA GCA TTC CAG GAA GAG GGA AGA GCA RRT GCA AAG    326
Lys Ile Cys Arg Arg Ala Phe Gln Glu Glu Gly Arg Ala Xaa Ala Lys
  1               5                  10                  15

ACG GGC GTG                                                        335
Thr Gly Val

(2) INFORMATION FOR SEQ ID NO: 134:

    (i) SEQUENCE CHARACTERISTICS:
        (A) LENGTH: 323 base pairs
        (B) TYPE: NUCLEIC ACID
        (C) STRANDEDNESS: DOUBLE
        (D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 195..239
(C) IDENTIFICATION METHOD: Von Heijne matrix
(D) OTHER INFORMATION: score 4.8
seq LFLNLPLVIGTIP/LH

(xi) SEQUENCE DESCRIPTION: SEQ ID NO: 134:

AATATGTAAA TGTACTATAC AGAATTATAC ATAAAAGAGA AACTTTTCAT GTATGTAAGT 60

TTAAAAATGA AGTAAATGGG GGTTTCAAAT AACATTARAA TTGGTTATGA GTTTTTGAAA 120

AGGAAATCAT ACTTGGCATT CTAAACTTAA TATTTCTTTG CAATGTTTAG GTATATGTGG 180

ATATTCCTGG AGCT ATG GAT TTA TTT CTT AAT TTG CCA CTT GTC ATC GGT 230
Met Asp Leu Phe Leu Asn Leu Pro Leu Val Ile Gly
-15 -10 -5

ACC ATT CCT CTA CAT CCA TTT GGT AGC AGA ACC TCA AGT GTA AGC AGT 278
Thr Ile Pro Leu His Pro Phe Gly Ser Arg Thr Ser Ser Val Ser Ser
1 5 10

CAG TGT AGC ATG AAT ATG AAC TGG CTC AGT TTA TCA CTT CCT GAA 323
Gln Cys Ser Met Asn Met Asn Trp Leu Ser Leu Ser Leu Pro Glu
15 20 25

(2) INFORMATION FOR SEQ ID NO: 135:

(i) SEQUENCE CHARACTERISTICS:
(A) LENGTH: 352 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 11..229
(C) IDENTIFICATION METHOD: Von Heijne matrix
(D) OTHER INFORMATION: score 4.8
seq VIRSTLVLSQCLC/SR

(xi) SEQUENCE DESCRIPTION: SEQ ID NO: 135:

AAAATATTAA ATG GMA AAA AAT CAC AGA AAT AAA AAA TCC ATA CAT TTT 49
Met Xaa Lys Asn His Arg Asn Lys Lys Ser Ile His Phe
-70 -65

CCA CTG TGC ACC ATT CCA AGT AGM ATG MTG AAA TCT TGT ACT CTC CCA 97
Pro Leu Cys Thr Ile Pro Ser Xaa Met Xaa Lys Ser Cys Thr Leu Pro
-60 -55 -50 -45

CTT CAG CGC ACC TGG GAC ATS MAT CCT TCC TTT GTC CAT TGG AWC CAA 145
Leu Gln Arg Thr Trp Asp Xaa Xaa Pro Ser Phe Val His Trp Xaa Gln
-40 -35 -30

GCC CGY CTA CAA TCC CCA CCG YCT AGT CAC TTA GTA SCC CTC TCG GTG 193
Ala Arg Leu Gln Ser Pro Pro Xaa Ser His Leu Val Xaa Leu Ser Val
-25 -20 -15

ATC AGA TCG ACT CTC GTG CTA TCC CAG TGC TTG TGT TCA AGG MAC CCT 241
Ile Arg Ser Thr Leu Val Leu Ser Gln Cys Leu Cys Ser Arg Xaa Pro
-10 -5 1

TAT TTT AGT GCA ATG ATG ACC CCA AAG TGC AAG AGT ATT GMT GCT GGC 289
Tyr Phe Ser Ala Met Met Thr Pro Lys Cys Lys Ser Ile Xaa Ala Gly
5 10 15 20

AAT TCA GGT ATG CCA AAG AGA AAC TGT AAA GTG CTT CCT TCA AGT GAA 337
Asn Ser Gly Met Pro Lys Arg Asn Cys Lys Val Leu Pro Ser Ser Glu
25 30 35

AAG ATG MAA GTT CAC 352
Lys Met Xaa Val His
40

(2) INFORMATION FOR SEQ ID NO: 136:

(i) SEQUENCE CHARACTERISTICS:
(A) LENGTH: 370 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 317..358
(C) IDENTIFICATION METHOD: Von Heijne matrix
(D) OTHER INFORMATION: score 4.8
seq SFIALVYSSLSFQ/KV

(xi) SEQUENCE DESCRIPTION: SEQ ID NO: 136:

AGAGCAAAGC AGACAGAAAT TCCTCTGGTT CTGTAGAGCT GACAATTCAT TAATGTGAGG 60

TAGTCAATAA CAAATATATT TTATGTCAAG TGGTGRATGG DTYCDATTGA AGAAAAATGA 120

CTCAATAAGA GGAGAGAAAA TGATGGTATG TGTATGGTGG GTAGGTGTGC GTGATGCTGT 180

TTTGGATAGC GAGGCCTCCG ATTAGATGCT ACGTGAGCAG GGACCCAAAA GAGCCATGTG 240

TTTCATCTAC CTGGGGGAGA AGCCTGCTGG CAGATCCTGT TGAACACTCG TTACCTAAAT 300

CTCTTGCATT GGCTCC ATG TCA TTT ATT GCT CTA GTG TAT TCT TCA CTA TCT 352
Met Ser Phe Ile Ala Leu Val Tyr Ser Ser Leu Ser
-10 -5

TTT CAG AAA GTG CCA GGG 370
Phe Gln Lys Val Pro Gly
1

(2) INFORMATION FOR SEQ ID NO: 137:

(i) SEQUENCE CHARACTERISTICS:
(A) LENGTH: 164 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 93..158
(C) IDENTIFICATION METHOD: Von Heijne matrix
(D) OTHER INFORMATION: score 4.7
seq IVLFLNSXFPIIC/SR

(xi) SEQUENCE DESCRIPTION: SEQ ID NO: 137:

ATAATATAGA TCTTTAATTT CTCTCAGCAA TGATTATAGT TCACAATGTG GAGGATTTAC 60

ATGTCTTTCA TTAAATTTAT CCAAAGTACT TT ATG GTT TTT GAT ACT TTA AAA 113
Met Val Phe Asp Thr Leu Lys
-20

AGT AGA ATT GTT CTT TTT TTA AAT TCG RWT TTC CCA ATC ATT TGC AGC 161
Ser Arg Ile Val Leu Phe Leu Asn Ser Xaa Phe Pro Ile Ile Cys Ser
-15 -10 -5 1

CGG 164
Arg

(2) INFORMATION FOR SEQ ID NO: 138:

(i) SEQUENCE CHARACTERISTICS:
(A) LENGTH: 274 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 68..244
(C) IDENTIFICATION METHOD: Von Heijne matrix
(D) OTHER INFORMATION: score 4.7
seq IFLFSILLMSLRT/FH

(xi) SEQUENCE DESCRIPTION: SEQ ID NO: 138:
AAAGCACAGA TGGCAGTCCA TTCATTGAAG ATGGTTTTTT TCAAGGTGAG TGTTGGTCTT 60
TTGCACA ATG CTT GAG ATG GAA ATG ACT TGG CTG AGA CTA TGT GAT GAG 109
TGC TCC AGA TGG GGC ATG GCA TCG GCA TGG GGT AGG GGT GGA AAG CTT 157
CTT GGA GCT CAA GTA GCC CTT CAT CCT AGA AAC TGC AGC AAA GCT AAG 205
ATC TTC CTG TTC AGT ATT TTA TTA ATG TCT TTA AGA ACT TTT CAC TGT 253
AAT TAT TTC AGA GGC AAT GGG 274

(2) INFORMATION FOR SEQ ID NO: 139:

(i) SEQUENCE CHARACTERISTICS:
(A) LENGTH: 400 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal

(F) TISSUE TYPE: kidney

(ix) FEATURE:
(A) NAME/KEY: sig_peptide
(B) LOCATION: 104..154
(C) IDENTIFICATION METHOD: Von Heijne matrix
(D) OTHER INFORMATION: score 4.7
seq MLFFLGALCRESG/VP

(xi) SEQUENCE DESCRIPTION: SEQ ID NO: 139:

AACAAAGGAG GGAAGGGTTA GAGTGAGGTA CTCACCCAGA GAAGAGCTGT CCCGGCCTGG 60

GGGTCCCATT CGTCCCTTCT CTTTCTTGCC AAAGAGACGG CCT ATG GAT GAC TTG 115
Met Asp Asp Leu
-15

ATG CTC TTC TTC TTG GGG GCT TTG TGC AGA GAA TCT GGG GTG CCC TCA 163
Met Leu Phe Phe Leu Gly Ala Leu Cys Arg Glu Ser Gly Val Pro Ser
-10 -5 1

CTG GGA AAG CAG GAG AGA ATG AGA GCA TAT GCT GCT GAG ATG CCC CCT 211
Leu Gly Lys Gln Glu Arg Met Arg Ala Tyr Ala Ala Glu Met Pro Pro
                5                   10                  15

CTC CTC CCA AGT CCT TGT CCA CCC CCT TCT CAT CTT CCC AAG CCA GCT 259
Leu Leu Pro Ser Pro Cys Pro Pro Pro Ser His Leu Pro Lys Pro Ala
20 25 30 35

TCT CCC TGT CCC TAT CCC TTG NNC CTG CTG ACC TTC CCC GTG GGG GTC 307
Ser Pro Cys Pro Tyr Pro Leu Xaa Leu Leu Thr Phe Pro Val Gly Val
40 45 50

CCC CAT CTT CCA GGG ACC CGC CTG CAG TGC CAA GGC CTG GGT CAT TCT 355
Pro His Leu Pro Gly Thr Arg Leu Gln Cys Gln Gly Leu Gly His Ser
55 60 65

CTC ARA CGG GCA GAG CGG GGA GTG GGT GGT GGG GTG TCT CCT GGG 400
Leu Xaa Arg Ala Glu Arg Gly Val Gly Gly Gly Val Ser Pro Gly
70 75 80

(2) INFORMATION FOR SEQ ID NO: 140:

(i) SEQUENCE CHARACTERISTICS:
(A) LENGTH: 225 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide

(B) LOCATION: 13..87
(C) IDENTIFICATION METHOD: Von Heijne matrix
(D) OTHER INFORMATION: . score 4.6
seq LPTLLLLPVGAPG/KK

(xi) SEQUENCE DESCRIPTION: SEQ ID NO: 140:

ATCGAATGCA GA ATG GTT TTG GGA GCC CTG AAC CTT CCC TCC CAG GAA CTC 51
Met Val Leu Gly Ala Leu Asn Leu Pro Ser Gln Glu Leu
-25 -20 -15

CCC ACT CTC CTG CTC CTC CCA GTG GGG GCA CCT GGR AAG AAA AAA GGC 99
Pro Thr Leu Leu Leu Leu Pro Val Gly Ala Pro Gly Lys Lys Lys Gly
-10 -5 1

ATG GAA GGC AAA ACT CCC TTG GAC CTG TTT GCT CAT TTT GGC CCT GAG 147
Met Glu Gly Lys Thr Pro Leu Asp Leu Phe Ala His Phe Gly Pro Glu
5 10 15 20

CCA GGG GAC CAC TCA GAT CCG CTG CCT CCC TCT GCA CCC TCT CCC ACT 195
Pro Gly Asp His Ser Asp Pro Leu Pro Pro Ser Ala Pro Ser Pro Thr
25 30 35

CGG GAG GGG GCT CTG ACC CCG CCC CCA GGG 225
Arg Glu Gly Ala Leu Thr Pro Pro Pro Gly
40 45

(2) INFORMATION FOR SEQ ID NO: 141:

(i) SEQUENCE CHARACTERISTICS:
(A) LENGTH: 308 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 207..263
(C) IDENTIFICATION METHOD: Von Heijne matrix
(D) OTHER INFORMATION: score 4.6
seq QTFVSFLSIPVLG/LV

(xi) SEQUENCE DESCRIPTION: SEQ ID NO: 141:

ATACACCTCC ATTTTAAATG TGCTGCAATA TGAATGAAGT GACCTGTGTT TCATCACTTG 60

TTCAAATGAT TCTTATCCAT GTTTTTGTAC TTAGTAAGGG CCATACGTAG TGGGATTAAA 120

TATTTGTGCC CTTGCTTTGA AAACAAAACT GAAAGTGAAT GACACATAAG GGCAGGGATT 180

TCAGAACAGA TTTTTCTTGA ATAAAA ATG CTT GTG TCA AAA ATT CAA ACA TTT 233
Met Leu Val Ser Lys Ile Gln Thr Phe
-15

GTC TCT TTC CTT TCC ATT CCA GTT CTA GGT CTC GTT CCA GAT CAT ATT 281
Val Ser Phe Leu Ser Ile Pro Val Leu Gly Leu Val Pro Asp His Ile
-10 -5 1 5

CTC CAG CTC ATA ACA GAG AAA GAA ACC 308
Leu Gln Leu Ile Thr Glu Lys Glu Thr
10 15

(2) INFORMATION FOR SEQ ID NO: 142:

(i) SEQUENCE CHARACTERISTICS:
(A) LENGTH: 304 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 188..280
(C) IDENTIFICATION METHOD: Von Heijne matrix
(D) OTHER INFORMATION: score 4.6
seq LLSTGLNILGTQA/FR

(xi) SEQUENCE DESCRIPTION: SEQ ID NO: 142:
ATCATAGTCA CTTTCCAAGT TTATGACCCA GAGCAATCTG ACCTTGGTAG CTTGTCTCCC 60
TCATTAAATT CTCTGACTTC ATAATCAGCT CACATTCCCT TCCTCTCTTT CCCTCTCTTT 120
TTAAATATCT GTAAAACATT CAAATTGATC CACGTAGATT TATCTTGCTT TTAGGCCACA 180
CTCTGAG ATG TGT AAT CCG GTT GCT CAC ACA TTT AGA GGA GTC CAT GAG 229
CAT CAC GCC ATG CTA CTC TCC ACT GGT TTG AAC ATC TTA GGC ACT CAG 277
GCA TTC CGT TAC GAA GAT GGG CAG CTG 304

(2) INFORMATION FOR SEQ ID NO: 143:

(i) SEQUENCE CHARACTERISTICS:
(A) LENGTH: 410 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 126..176
(C) IDENTIFICATION METHOD: Von Heijne matrix
(D) OTHER INFORMATION: score 4.6
seq ILLWEACTGRCQA/SL

(xi) SEQUENCE DESCRIPTION: SEQ ID NO: 143:
TATTCAGTTG GGGGCAAGCC AGCCATGATG TGGACCTTTC ATTGGGTAGG GCAAGTCCCC 60
AAAGTTGGAA AAATGGAAAG TGGGAGCTGT GAGGCACGTG TTACACCCAC ACTTTCCTCC 120
TACAG ATG CAG TGT TGG ATT TTG TTG TGG GAG GCA TGC ACA GGT AGG TGC 170
CAG GCC TCC CTA CTC TCT CCC TGG CCC AGA GGT GGC AGG GGC AAG TTA 218
GTG GCA GTG GTG GCT GCA AAA TGG TTG GCA GCA ATC TGT GGG ATT TGG 266
GCT ATC AAA GAA ATG CCA AGC CAT GGC CAC AGT CTT CAA GCA GGG GCA 314
GGG GAA GGT GCA CTG GTG ACC TGG AGC CTG CAA ACC TCA TTT GGT GTG 362
AAG CAG TAT AAG TGG GGA GTT GTG TGG CAT GAA GCA AAC CTG TTG CTT 410

(2) INFORMATION FOR SEQ ID NO: 144:

(i) SEQUENCE CHARACTERISTICS:
(A) LENGTH: 247 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 149..223
(C) IDENTIFICATION METHOD: Von Heijne matrix
(D) OTHER INFORMATION: score 4.6
seq VLCILGCHGNLCC/EP

(xi) SEQUENCE DESCRIPTION: SEQ ID NO: 144:
ATTTTAGAAA GTAAGGAAAT AAAACTTTAA TTGAACTTGG AATAAACTCA GTTCTGAGCA 60
TTCCATTCTA CTCTGCAGTT GTCATTTATA GACAGCTGTG GATCATAATA CCTATAGACT 120
AGATATCGTT ATCTACTTAT TTATATTA ATG ACA GGA TAT CCC TGG GCA AAC 172
AGC ATC ACC ACT GTA CTG TGT ATT CTT GGT TGT CAT GGG AAC CTT TGC 220
TGT GAA CCA GCA GTG AGA GCA CTC GGG 247

(2) INFORMATION FOR SEQ ID NO: 145:

(i) SEQUENCE CHARACTERISTICS:
(A) LENGTH: 561 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 475..546
(C) IDENTIFICATION METHOD: Von Heijne matrix
(D) OTHER INFORMATION: score 4.6
seq IFTALFLXLHSVA/IN

(xi) SEQUENCE DESCRIPTION: SEQ ID NO: 145:

AATTTATGGA TGCCTACCAT CTACCAGGTA CTGTTCTAGC TACAAGGAAT AACTAAAAAT 60

AGGTAAACAA AACAGATGAA AAACTTAGAA ATTTATACTG ATGTTATCAG AGTAATGTTT 120

AATTTTTCAG ATAATTGTTA TGTCTAAATT AGCATTTGAT TTTTCAATTA AGAATTTTTA 180

AATTATCCAA TATTGCAAGC ATATATAGAA ACATGGAAAA CAACAAAATT CTCATGCATA 240

TACTTCAAAC ACAGAGCTAA CAGATGTTAT TATTTTTTAT TTCTTTCACA ACCCAACTTT 300

CGGGAAACAA AATAGGCACA GCAAAACTGG GATCTCCTCA TCCCCTTCTC CTTTCTTATA 360

TAAAAGTAAT CCTGCTCTTG GTACAGCTAT GTATCATACT CATCCAGGTT TTAATTTTTC 420

TTATATAACG GAACATATAT GGTGTTATTT TACGGATTTT AAAGCTTTAC ATAA ATG 477
Met

GTG TCA TGT GAT GTW CVN TCT TAT GTG ATC ATT TTT ACT GCA CTC TTT 525
Val Ser Cys Asp Val Xaa Ser Tyr Val Ile Ile Phe Thr Ala Leu Phe
-20 -15 -10

TTA WTG CTG CAT AGT GTG GCA ATA AAT GAA GAG TTT 561
Leu Xaa Leu His Ser Val Ala Ile Asn Glu Glu Phe
-5 1 5

(2) INFORMATION FOR SEQ ID NO: 146:

(i) SEQUENCE CHARACTERISTICS:
(A) LENGTH: 160 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 80..139
(C) IDENTIFICATION METHOD: Von Heijne matrix
(D) OTHER INFORMATION: score 4.6
seq LFAIFLMCLKSIG/SV

(xi) SEQUENCE DESCRIPTION: SEQ ID NO: 146:
ATGATAAGGG CTTATTCACA TTATTCATTC TTGAATGAAT TTTGATAGTG TCTGTCTTTC 60
AGGAACTTTG TCCTAAGTA ATG AAA TCC TTT GAT AAA AAG TTG TTT GCA ATA 112
TTT CTT ATG TGT TTA AAG TCT ATA GGT TCT GTG GTG ATG CCC CAG CCG 160

(2) INFORMATION FOR SEQ ID NO: 147:

(i) SEQUENCE CHARACTERISTICS:
(A) LENGTH: 338 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 36..134
(C) IDENTIFICATION METHOD: Von Heijne matrix
(D) OTHER INFORMATION: score 4.5
seq LASLFGLDQXAXG/HG

(xi) SEQUENCE DESCRIPTION: SEQ ID NO: 147:

ATTTTCCTCC CCGCAACCTG GTGAAAGCCA AYKCA ATG TTC GGT GCG GGG GAC 53
Met Phe Gly Ala Gly Asp
-30

GAG GAC GAC ACC GAT TTC CTC TCG CCG AGC GGC GGT GCC AGA TTG GCC 101
Glu Asp Asp Thr Asp Phe Leu Ser Pro Ser Gly Gly Ala Arg Leu Ala
-25 -20 -15

TCA CTT TTT GGA CTG GAT CAG GYA GCY SST GGC CAT GGA AAT GAA TTT 149
Ser Leu Phe Gly Leu Asp Gln Xaa Ala Xaa Gly His Gly Asn Glu Phe
-10 -5 1 5

TTC CAG TAC ACA GCC CCA AAA CAG CCT AAG AAA GGC CAG GGA ACG GCA 197
Phe Gln Tyr Thr Ala Pro Lys Gln Pro Lys Lys Gly Gln Gly Thr Ala
10 15 20

GCA ACA GGA AAT CAG GCA RCA CCA AAA ACA GCA CCA GCC RSC ATG AGC 245
Ala Thr Gly Asn Gln Ala Xaa Pro Lys Thr Ala Pro Ala Xaa Met Ser
25 30 35

ACT CCC ACA ATA CTG GTC GCA ACA GCA GTC CAT GCA TAT CGA TAC ACA 293
Thr Pro Thr Ile Leu Val Ala Thr Ala Val His Ala Tyr Arg Tyr Thr
40 45 50

RAT GGT CRA TAT GTA AAG CAG GSR AAT TTG GTG CTG CAG TTC TGG 338
Xaa Gly Xaa Tyr Val Lys Gln Xaa Asn Leu Val Leu Gln Phe Trp
55 60 65

(2) INFORMATION FOR SEQ ID NO: 148:

(i) SEQUENCE CHARACTERISTICS:
(A) LENGTH: 292 base pairs
(B) TYPE: NUCLEIC ACID

(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 107..190
(C) IDENTIFICATION METHOD: Von Heijne matrix
(D) OTHER INFORMATION: score 4.5
seq RFLSLSAADGXDX/SX

(xi) SEQUENCE DESCRIPTION: SEQ ID NO: 148:

AAAGTCAGCG CTGGAGTCGG CTAGGCGGCT GGAAACGGCG GCTGCCGCCG GTGACTCAGG 60

GAGGCGGGAG GCCGMSGGMG GAGCTCTTCC TGCAGGCGTG GARACC ATG GTG CTC 115
Met Val Leu

ACG CTC GGA GAA AGT TGG CCG GTA TTG GTG GGG AGG AGG TTT CTC AGT 163
Thr Leu Gly Glu Ser Trp Pro Val Leu Val Gly Arg Arg Phe Leu Ser
-25 -20 -15 -10

CTG TCC GCA GCC GAC GGC ASC GAT GSC AGC CAM GAC AGC TGG GAC GTG 211
Leu Ser Ala Ala Asp Gly Xaa Asp Xaa Ser Xaa Asp Ser Trp Asp Val
-5 1 5

GAG CGC GTC GCC GAG TGG CCC TGG CTC TCC GGG ACC ATT CGA GCT GTT 259
Glu Arg Val Ala Glu Trp Pro Trp Leu Ser Gly Thr Ile Arg Ala Val
10 15 20

TCC CAC ACC GAC GTT ACC AAG AAG GAT CTG AAG 292
Ser His Thr Asp Val Thr Lys Lys Asp Leu Lys
25 30

(2) INFORMATION FOR SEQ ID NO: 149:

(i) SEQUENCE CHARACTERISTICS:
(A) LENGTH: 429 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 361..411

(C) IDENTIFICATION METHOD: Von Heijne matrix
(D) OTHER INFORMATION: score 4.4
seq LTSVFQAMIWSQG/VS

(xi) SEQUENCE DESCRIPTION: SEQ ID NO: 149:

ATGAAAACAG TTTTCTTTGT GATTTGTCAA TTGATGTTTA AACAGTGTTT ATCCTTCCAG 60

GTAGTATGAT GATGTATTTG TTGGAGACAA ARTATTTGCC CTAGCCTTTT TACTAATATT 120

TCAGATGAGA TTCTGTGGAG GAGAAGCATC TCCCCAAATG TCCTTGTTTT ATAGTAAATA 180

ATTCTACCAC GAGGATCCTT ATCCATAAAT CTATATTCAT GTTTATTTTG TGCTAGATAC 240

AGATCTTGCA ATATTCATGA AGCTTTAAGA AGAGCACTTT GAATCTTAAA AGAGATTCTC 300

TGAGCAGGGG TTGGCAGTGG TGAGGTCCAG GTAGTTATAA TAGCCATAAG AGCAGGGATT 360

ATG GTT ATT GAG CTC ACC AGT GTG TTT CAA GCC ATG ATC TGG AGT CAA 408
Met Val Ile Glu Leu Thr Ser Val Phe Gln Ala Met Ile Trp Ser Gln
-15 -10 -5

GGT GTT AGT GAT TCC TCT AAG 429
Gly Val Ser Asp Ser Ser Lys
1               5

(2) INFORMATION FOR SEQ ID NO: 150:

(i) SEQUENCE CHARACTERISTICS:
(A) LENGTH: 250 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 47..196
(C) IDENTIFICATION METHOD: Von Heijne matrix
(D) OTHER INFORMATION: score 4.4
seq ILFLFYFPAAYYA/SR

(xi) SEQUENCE DESCRIPTION: SEQ ID NO: 150:

ATDCCGCCCT GGAGCAAGCC GGGGCCTGGT CGGCARCTGG GCCGCC ATG GAG TCC 55
Met Glu Ser
-50

ACG CTG GGC GCG GGC ATC GTG ATA GCC GAG GCG CTA CAG AAC CAG CTA 103
Thr Leu Gly Ala Gly Ile Val Ile Ala Glu Ala Leu Gln Asn Gln Leu
-45 -40 -35

GCC TGG CTG GAG AAC GTG TGG CTC TGG RRT SAC CTT TKC TNG SCG ATC 151
Ala Trp Leu Glu Asn Val Trp Leu Trp Xaa Xaa Leu Xaa Xaa Xaa Ile
-30 -25 -20

CCA AGK ATC CTC TTT CTG TTC TAC TTC CCC GCG GCN TAC TAC GCC TCC 199
Pro Xaa Ile Leu Phe Leu Phe Tyr Phe Pro Ala Ala Tyr Tyr Ala Ser
-15 -10 -5 1

CGC CGT GTR GGC ATC GCG GTG CTC TGG ATC AGC CTS ATC ACC GAG TGG 247
Arg Arg Val Gly Ile Ala Val Leu Trp Ile Ser Leu Ile Thr Glu Trp
                5                   10                  15

CTC 250
Leu

(2) INFORMATION FOR SEQ ID NO: 151:

(i) SEQUENCE CHARACTERISTICS:
(A) LENGTH: 288 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 196..270
(C) IDENTIFICATION METHOD: Von Heijne matrix
(D) OTHER INFORMATION: score 4.4
seq VLVGVFLSTFLYC/EC

(xi) SEQUENCE DESCRIPTION: SEQ ID NO: 151:
ATNCTGTGTT ACTCATTTCC TGTCTCAGAT ACTTTGGATC CCTTGGTTCT GATCTTTCAG 60
GGGGAGAGGG CATGTTAAGA GGAGTAAGTA GATGGATGAT CTTACACAAT TGAACTCTTC 120
TTACCTCTGG CCTTGTATGC TCTTACATAG GCTGTCCCCT CTCTACATTT TCTTATTTAA 180
GGAAAAACAC AGAAC ATG ATT ATT GTC TCA GAA TTA GGA ACC CCT ACT GGT 231
GTG CTC GTA GGT GTC TTT TTG TCT ACT TTT CTC TAT TGT GAA TGT GTA 279
AAG GGG CCG 288

(2) INFORMATION FOR SEQ ID NO: 152:

(i) SEQUENCE CHARACTERISTICS:
(A) LENGTH: 190 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 80..145
(C) IDENTIFICATION METHOD: Von Heijne matrix
(D) OTHER INFORMATION: score 4.4
seq GFLLCPLVCGLRR/WT

(xi) SEQUENCE DESCRIPTION: SEQ ID NO: 152:
AGCGTTTATG GCCGCGTTAA GTCTGAGTGC CGCTTTGAGT TGTTGAATGA AGTGAACTTC 60
ATTTGTCAGC GTTCGGTTC ATG AAC TGG AAT GTA AGA GGC ACC AGA GGA TTC 112
CTG CTC TGT CCC CTG GTT TGC GGC TTG CGA CGT TGG ACA TCC CCG GAT 160
TGT TGT TTA ATA GAG AAA ACT CAC CGC GGG 190

(2) INFORMATION FOR SEQ ID NO: 153:

(i) SEQUENCE CHARACTERISTICS:
(A) LENGTH: 111 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 49..105
(C) IDENTIFICATION METHOD: Von Heijne matrix

(D) OTHER INFORMATION:  score 4.4
                           seq RGLLLGLAVAAAA/VR

(xi) SEQUENCE DESCRIPTION: SEQ ID NO: 153:

AAGATAGAGG CGGCAACCTC GGAAGTGCGG ACGGGTGGGC CTATATAG ATG TTG AGG    57
                                                     Met Leu Arg

TGC GGA GGC CGT GGG CTT TTG TTG GGC CTG GCT GTA GCC GCA GCA GCG    105
Cys Gly Gly Arg Gly Leu Leu Leu Gly Leu Ala Val Ala Ala Ala Ala
    -15                 -10                 -5

GTA AGG                                                            111
Val Arg
  1

(2) INFORMATION FOR SEQ ID NO: 154:

(i) SEQUENCE CHARACTERISTICS:
     (A) LENGTH: 175 base pairs
     (B) TYPE: NUCLEIC ACID
     (C) STRANDEDNESS: DOUBLE
     (D) TOPOLOGY: LINEAR

(ii) MOLECULE TYPE: CDNA

(vi) ORIGINAL SOURCE:
     (A) ORGANISM: Homo Sapiens
     (D) DEVELOPMENTAL STAGE: Fetal
     (F) TISSUE TYPE: kidney

(ix) FEATURE:
     (A) NAME/KEY: sig_peptide
     (B) LOCATION: 95..136
     (C) IDENTIFICATION METHOD: Von Heijne matrix
     (D) OTHER INFORMATION:  score 4.4
                             seq ILLMIVFSIFLLL/CN

(xi) SEQUENCE DESCRIPTION: SEQ ID NO: 154:
ACCCAGAGGC AGAAAGTAAT ATTGCTTACT ATGAGTCTAT ATATCCTGGG GAATTTAAGA   60
TGCCAAAGCA GCTCATTCAC ATACAGCGTA AGTA ATG ATT CTC TTA ATG ATT GTA  115
TTT TCT ATA TTT CTC TTA TTA TGT AAC TTG ACA GAT TTT TAT CTC TTC    163
AGG AGC GAT GGG                                                    175

(2) INFORMATION FOR SEQ ID NO: 155:

(i) SEQUENCE CHARACTERISTICS:
(A) LENGTH: 214 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 149..190
(C) IDENTIFICATION METHOD: Von Heijne matrix
(D) OTHER INFORMATION: score 4.4
seq SLLFIFRSILISC/FS

(xi) SEQUENCE DESCRIPTION: SEQ ID NO: 155:

ACAATTTGTT TTATAAGCCT ATATTAATTG GGTTTTGACT GAATTAATTA TATAACCATT 60

TATCTCAAAA TGAAATGTTC CATAAAATTT ATTTAAWAGT ATATACTGYA TAAGTGTTAA 120

ATTATGAAAT TTAGTGGTCT TATAGAGA ATG TCT TTA TTG TTT ATT TTT AGG 172
Met Ser Leu Leu Phe Ile Phe Arg
-10

TCA ATT TTG ATC TCC TGC TTT TCA GGA GAC TTT TTT TTT TTT 214
Ser Ile Leu Ile Ser Cys Phe Ser Gly Asp Phe Phe Phe Phe
-5 1 5

(2) INFORMATION FOR SEQ ID NO: 156:

(i) SEQUENCE CHARACTERISTICS:
(A) LENGTH: 164 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 27..77
(C) IDENTIFICATION METHOD: Von Heijne matrix
(D) OTHER INFORMATION: score 4.3
seq SKVLIQLSQAFWA/SP

(xi) SEQUENCE DESCRIPTION: SEQ ID NO: 156:
ACCTGGTATG AATTACAAAA CTGTAA ATG CCT TTG ATT AGT AAA GTT TTG ATA 53
CAG CTA AGC CAA GCA TTT TGG GCC TCA CCT GAG GGT AGG AAC AGT TCT 101
GGG AGT AAG AGG AAG CAG TTG GTA GCT GCA GTG GAG ATG CGA TAC TGT 149
AAA AGG CAG CAG GGG 164

(2) INFORMATION FOR SEQ ID NO: 157:

(i) SEQUENCE CHARACTERISTICS:
(A) LENGTH: 465 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 142..228
(C) IDENTIFICATION METHOD: Von Heijne matrix
(D) OTHER INFORMATION: score 4.3
seq VLLGSTAMATSLT/NV

(xi) SEQUENCE DESCRIPTION: SEQ ID NO: 157:

AAGTTGTAAT CCCACTAAGA ACCGCCAGGG CGAGACGAAA GCGACATCGC TTCCATCTTT 60

ACGACCAAGA ATCGCCTTCA GCCCTGTCTG GTGCATCCTT GGCAGAAAGT GAGGAGGRAA 120

ACACCCCCAT TGTTCTTTGG C ATG GAC ACA AGT TCA GTG GGA GGA TTA GAA 171
Met Asp Thr Ser Ser Val Gly Gly Leu Glu
-25 -20

TTG ACT GAT CAG ACT CCT GTT TTA TTA GGG AGT ACG GCC ATG GCA ACT 219
Leu Thr Asp Gln Thr Pro Val Leu Leu Gly Ser Thr Ala Met Ala Thr
-15 -10 -5

AGT CTC ACG AAT GTA GGA AAC TCA TTT AGT GGT CCA GCT AAT CCT TTA 267
Ser Leu Thr Asn Val Gly Asn Ser Phe Ser Gly Pro Ala Asn Pro Leu
1 5 10

GTG TCT AGA TCT AAT AAG TTT CAG AAC TCG TCA GTG GAA GAT GAT GAT 315

Val Ser Arg Ser Asn Lys Phe Gln Asn Ser Ser Val Glu Asp Asp Asp
     15                  20                  25

GAT GTT GTT TTT ATC GAA CCT GTA CAA CCT CCC CCA CCT TCT GTA CCA     363
Asp Val Val Phe Ile Glu Pro Val Gln Pro Pro Pro Pro Ser Val Pro
 30                  35                  40                  45

GTG GTA GCT GAT CAA AGA ACC ATA ACA TTT ACA TCA TCA AAA AAT GRA     411
Val Val Ala Asp Gln Arg Thr Ile Thr Phe Thr Ser Ser Lys Asn Xaa
                 50                  55                  60

GAA CTA CAA GGA AAT GAT TCC AAA ATT ACT CCT TCC TCA AAA GAG TTG     459
Glu Leu Gln Gly Asn Asp Ser Lys Ile Thr Pro Ser Ser Lys Glu Leu
             65                  70                  75

GCA TCT                                                             465
Ala Ser

(2) INFORMATION FOR SEQ ID NO: 158:

     (i) SEQUENCE CHARACTERISTICS:
          (A) LENGTH: 244 base pairs
          (B) TYPE: NUCLEIC ACID
          (C) STRANDEDNESS: DOUBLE
          (D) TOPOLOGY: LINEAR

     (ii) MOLECULE TYPE: CDNA

     (vi) ORIGINAL SOURCE:
          (A) ORGANISM: Homo Sapiens
          (F) TISSUE TYPE: Kidney

     (ix) FEATURE:
          (A) NAME/KEY: sig_peptide
          (B) LOCATION: 92..184
          (C) IDENTIFICATION METHOD: Von Heijne matrix
          (D) OTHER INFORMATION:  score 4.3
                                  seq ILLLTHVPPWILE/NP

     (xi) SEQUENCE DESCRIPTION: SEQ ID NO: 158:

ACACACGTCC CGCMGTGGAT ACTGGAGAAT CCTTGCCACA CACGTCCTGC CGTGGACACT    60

GGAGAATCCT TCTCGCCACA CACTTCCCAC C ATG GAC ACT GGA GAA TCC TTC      112
                                   Met Asp Thr Gly Glu Ser Phe
                                       -30                 -25

TCG CCA CAC ACG TCC TGC CGT GGA CAC TGG AGA ATC CTT CTA CTC ACA     160
Ser Pro His Thr Ser Cys Arg Gly His Trp Arg Ile Leu Leu Leu Thr
                -20                 -15                 -10

CAC GTC CCA CCG TGG ATA CTG GAG AAT CCT TCT TGC CAC ACA CGT CCC     208
His Val Pro Pro Trp Ile Leu Glu Asn Pro Ser Cys His Thr Arg Pro
             -5                   1               5

GCC GTG GAC ACT GGA GAA TCC TTC TCG CCA CAA CGG                     244
Ala Val Asp Thr Gly Glu Ser Phe Ser Pro Gln Arg
     10                  15                  20

(2) INFORMATION FOR SEQ ID NO: 159:

(i) SEQUENCE CHARACTERISTICS:
(A) LENGTH: 453 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 154..246
(C) IDENTIFICATION METHOD: Von Heijne matrix
(D) OTHER INFORMATION: score 4.3
seq LVLLSVLKEPVSR/SI

(xi) SEQUENCE DESCRIPTION: SEQ ID NO: 159:

ATAGGACTGC TACAAAAACC CCATGTTTAC GAATTTGCCA GTGATATTGC CCCCTTCCTG 60

TGTCATCCCA ATTTATGGAT ACGTTATGGT GCCGTGGGAT TTATCACAGT GGTAGCTCGT 120

CAAATAAGTA CAGCTGATGT CTACTGTAAA CTG ATG CCT TAT CTT GAC CCA TAT 174
Met Pro Tyr Leu Asp Pro Tyr
-30 -25

ATT ACC CAA CCA ATA ATA CAG ATT GAA AGA AAA CTT GTT CTG CTC AGT 222
Ile Thr Gln Pro Ile Ile Gln Ile Glu Arg Lys Leu Val Leu Leu Ser
-20 -15 -10

GTT TTA AAG GAA CCA GTA AGT CGT TCT ATA TTT GAT TAT GCT TTG AGG 270
Val Leu Lys Glu Pro Val Ser Arg Ser Ile Phe Asp Tyr Ala Leu Arg
-5 1 5

TCT AAA GAT ATT ACT AGC TTG TTC AGA CAT CTT CAC ATG CGT CAG AAG 318
Ser Lys Asp Ile Thr Ser Leu Phe Arg His Leu His Met Arg Gln Lys
10 15 20

AAA CGA AAT GGT TCT CTT CCC GAC TGC CCT CCG CCA GAG GAT CCT GCC 366
Lys Arg Asn Gly Ser Leu Pro Asp Cys Pro Pro Pro Glu Asp Pro Ala
25 30 35 40

ATA GCA CAG CTT CTG AAG AAG TTG CTC TCA CAG GGA ATG ACA GAG GAA 414
Ile Ala Gln Leu Leu Lys Lys Leu Leu Ser Gln Gly Met Thr Glu Glu
45 50 55

GAG GAA GAC AAA CTT CTG GCA CTG AAA GAC TTC ATG ATG 453
Glu Glu Asp Lys Leu Leu Ala Leu Lys Asp Phe Met Met
60 65

(2) INFORMATION FOR SEQ ID NO: 160:

(i) SEQUENCE CHARACTERISTICS:
(A) LENGTH: 312 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 181..267
(C) IDENTIFICATION METHOD: Von Heijne matrix
(D) OTHER INFORMATION: score 4.3
seq VLLGSTAMATSLT/NV

(xi) SEQUENCE DESCRIPTION: SEQ ID NO: 160:

ARRAAAGCCG GGACTGGACC GAGCGGAGTK KTGCGTGTCG CCGAAGGGGG GTKGGCCGGG 60

GGAGGKGAGG TTCGTTCCGC GGAKCCGCAG YCAGAASCGK GRACCAAGAA TCGCCTTCAG 120

CCCTGTCTKG TGCATCCTTG GCAGAAAGTG RKGAKGAAAA CACCCCCATT GTTCTTTGGC 180

ATG GAC ACA AGT TCA GTG GGA GGA TTA GAA TTG ACT GAT CAG ACT CCT 228
Met Asp Thr Ser Ser Val Gly Gly Leu Glu Leu Thr Asp Gln Thr Pro
-25 -20 -15

GTT TTA TTA GGG AGT ACG GCC ATG GCA ACT AGT CTC ACG AAT GTA GGA 276
Val Leu Leu Gly Ser Thr Ala Met Ala Thr Ser Leu Thr Asn Val Gly
-10 -5 1

AAC TCA TTT AGT GGT CCA GCT AAT CCT TTA GTG TCT 312
Asn Ser Phe Ser Gly Pro Ala Asn Pro Leu Val Ser
5 10 15

(2) INFORMATION FOR SEQ ID NO: 161:

(i) SEQUENCE CHARACTERISTICS:
(A) LENGTH: 182 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 33..116
(C) IDENTIFICATION METHOD: Von Heijne matrix
(D) OTHER INFORMATION: score 4.2
seq FGLLDFVVQCCDS/LR

(xi) SEQUENCE DESCRIPTION: SEQ ID NO: 161:

ATTTTTTATG ACATCTAWTT ATATTGAGTT GC ATG CAT GTT TTG TTC AAC ATA 53
Met His Val Leu Phe Asn Ile
-25

GTC ACA ACA AAT WRR RAT AAC CAT TTT GGG TTG TTA GAT TTT GTT GTG 101
Val Thr Thr Asn Xaa Xaa Asn His Phe Gly Leu Leu Asp Phe Val Val
-20 -15 -10

CAG TGT TGT GAT TCA TTA AGA AAC CAT ARG WGG TCA TTT CAG TCA TCT 149
Gln Cys Cys Asp Ser Leu Arg Asn His Xaa Xaa Ser Phe Gln Ser Ser
-5 1 5 10

TAC TTG AGG CTA AAT CAT TCA TGR CAT ACA TGT 182
Tyr Leu Arg Leu Asn His Ser Xaa His Thr Cys
15 20

(2) INFORMATION FOR SEQ ID NO: 162:

(i) SEQUENCE CHARACTERISTICS:
(A) LENGTH: 347 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 150..215
(C) IDENTIFICATION METHOD: Von Heijne matrix
(D) OTHER INFORMATION: score 4.2
seq TAYWLSFMSWAQS/SS

(xi) SEQUENCE DESCRIPTION: SEQ ID NO: 162:

ATGTATACTG AGGTTCAGGA ACTGCTGGAG AGATGACTGG GCACCAAGAG GATGACAGTG 60

ACTCAGCTGG CATCCCTTAG CTGGTTCATG GCAGAGCTGA GTGGCCACTC CTGTCTCTGA 120

CCCCAGCTTC AGTGCTCTTT ATCTCCTCC ATG CCT CCT CAG TCG TGC TGC TCT 173
Met Pro Pro Gln Ser Cys Cys Ser
-20 -15

AAG ACT GCT TAC TGG CTT TCC TTC ATG TCC TGG GCA CAG AGC AGT TCT     221
Lys Thr Ala Tyr Trp Leu Ser Phe Met Ser Trp Ala Gln Ser Ser Ser
                -10                 -5                   1

TTT GGT AGC AGA HTT GAG TCC ACT TCC CCC TGC ACA GAT CAC TGC TCA     269
Phe Gly Ser Arg Xaa Glu Ser Thr Ser Pro Cys Thr Asp His Cys Ser
         5                  10                  15

GGA CCC AGA GAG GAG CAG CTC TGC TCC AGC AGG GTT TTC CAT TGC ATC     317
Gly Pro Arg Glu Glu Gln Leu Cys Ser Ser Arg Val Phe His Cys Ile
     20                  25                  30

ACA CAC CCA AAC GGT AGG ATC CAC CGG TGG                             347
Thr His Pro Asn Gly Arg Ile His Arg Trp
 35                  40

(2) INFORMATION FOR SEQ ID NO: 163:

(i) SEQUENCE CHARACTERISTICS:
(A) LENGTH: 127 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 53..94
(C) IDENTIFICATION METHOD: Von Heijne matrix
(D) OTHER INFORMATION:  score 4.2
                        seq SCVFFHFLQGGLG/FG

(xi) SEQUENCE DESCRIPTION: SEQ ID NO: 163:

AACTTTCTTC AAGGCGGTTT GGGATTTGGC TCCGCTGGCC GCTGTGCTGG TG ATG TCC    58
                                                          Met Ser

TGT GTT TTC TTT CAC TTT CTT CAA GGC GGT TTG GGA TTT GGC TCC GCT     106
Cys Val Phe Phe His Phe Leu Gln Gly Gly Leu Gly Phe Gly Ser Ala
        -10                 -5                   1

GGC CGC TGT GCT GGT GAC AGG                                         127
Gly Arg Cys Ala Gly Asp Arg
  5                  10

(2) INFORMATION FOR SEQ ID NO: 164:

(i) SEQUENCE CHARACTERISTICS:
(A) LENGTH: 317 base pairs
(B) TYPE: NUCLEIC ACID
(C) STRANDEDNESS: DOUBLE

(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 156..215
(C) IDENTIFICATION METHOD: Von Heijne matrix
(D) OTHER INFORMATION: score 4.2
seq LILLPIWINMAQI/QQ

(xi) SEQUENCE DESCRIPTION: SEQ ID NO: 164:

AAACTCGAAC TTGGTCGGGG CGCGGATCCC GAGAGGGAAA GTCATAACAA CCGCACGAGG 60

GAGTTCGACT GGCGAACTGG AAGGCCACGC CTCCTCCCGC CTGCCCCCTC AGCCCTGTGG 120

CTGGGGGCAG AGCTCAGACT GTCTTCTGAA GATTG ATG TCT ATT TCC TTG AGC 173
Met Ser Ile Ser Leu Ser
-20 -15

TCT TTA ATT TTG TTG CCA ATT TGG ATA AAC ATG GCA CAA ATC CAG CAG 221
Ser Leu Ile Leu Leu Pro Ile Trp Ile Asn Met Ala Gln Ile Gln Gln
-10 -5 1

GGA GGT CCA GAT GAA AAA GAA AAG ACT ACC GCA CTG AAA GAT TTA TTA 269
Gly Gly Pro Asp Glu Lys Glu Lys Thr Thr Ala Leu Lys Asp Leu Leu
                5                   10                  15

TCT AGG ATA GAT TTG GAT GAA CTA ATG AAA AAA GAT GAA CCG CCA GGG 317
Ser Arg Ile Asp Leu Asp Glu Leu Met Lys Lys Asp Glu Pro Pro Gly
            20                  25                  30

(2) INFORMATION FOR SEQ ID NO: 165:

(i) SEQUENCE CHARACTERISTICS:
(A) LENGTH: 205 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 50..151
(C) IDENTIFICATION METHOD: Von Heijne matrix
(D) OTHER INFORMATION: score 4.2
seq SFCNAVVLSPVFQ/EE

(xi) SEQUENCE DESCRIPTION: SEQ ID NO: 165:
AAGTTATACA GAAGACTTGT AGGAAGGATG GACAAACGTT CTTAAGCCC ATG ACG GCC 58
CTT AAC CTG GTC GCT CCC TTT TCT GAT GGA GAC TCA GGC AGC GTC TCT 106
CTA GCT TCT TTC TGC AAT GCT GTA GTA CTC TCT CCA GTA TTT CAG GAG 154
GAG GAG CAT TTG CTA TTT CAA AAA CGA AAA ACA AAA ACC TGG CCA CCC 202
AGG 205

(2) INFORMATION FOR SEQ ID NO: 166:

(i) SEQUENCE CHARACTERISTICS:
(A) LENGTH: 270 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: 154..204
(C) IDENTIFICATION METHOD: Von Heijne matrix
(D) OTHER INFORMATION: score 4.2
seq PVQVLGLLATCQH/AP

(xi) SEQUENCE DESCRIPTION: SEQ ID NO: 166:
AATATGTAAC CAAAAATAAA GTGTTTCAAT AGTTTATTCC TCTTTCATAT AATGGTCTAG 60
AGAGAGTGTC ATTGGGGCAA AGGGCAAAGA TACAGAGGAT CTGTTTCCCT TCTATCTTGT 120
TTTTCTGTAA TCACCTAGAG CAGTGCTACT CAA ATG TGG TCC AGA CCA GTG CAG 174
GTC TTG GGA CTT CTT GCC ACT TGT CAG CAT GCT CCC TCT CCC TCC TTT 222
AAA GGT GAG ACA TGT ACA GAA ATT GAG AGT GTT TAT CTG GCC CCC ATG 270

(2) INFORMATION FOR SEQ ID NO: 167:

(i) SEQUENCE CHARACTERISTICS:
(A) LENGTH: 208 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 125..196
(C) IDENTIFICATION METHOD: Von Heijne matrix
(D) OTHER INFORMATION: score 4.2
seq SLNQILLFLLISC/RT

(xi) SEQUENCE DESCRIPTION: SEQ ID NO: 167:

TACTGTGGTA AGCACTTAGT AATGCAAAGT ATTGTTATTC TAATTATTTC CAATAAGAAT 60

AGTGCCTTTT ATTGGGGAAA GAGTCTACTT GGCTGATCAC AACAAGAGGT TTATTTCTTC 120

CTCC ATG AGG TAC CGG TTA AGG ATT CAA ATC ACA ACA TCC CTC AAT CAG 169
Met Arg Tyr Arg Leu Arg Ile Gln Ile Thr Thr Ser Leu Asn Gln
-20 -15 -10

ATC CTG CTA TTC TTA CTG ATA AGT TGT AGG ACC TTG AGC 208
Ile Leu Leu Phe Leu Leu Ile Ser Cys Arg Thr Leu Ser
-5 1

(2) INFORMATION FOR SEQ ID NO: 168:

(i) SEQUENCE CHARACTERISTICS:
(A) LENGTH: 375 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 271..345

(C) IDENTIFICATION METHOD: Von Heijne matrix
(D) OTHER INFORMATION: score 4.2
seq VLLFFCCSPLYSP/LF

(xi) SEQUENCE DESCRIPTION: SEQ ID NO: 168:

ATGTAATGGA AGCAATCATT TTGAAAAGAG TTAAAGTTTT TTGGTAAGTC AAATAAGGAT 60

CAATGCTGCT GAAAGCTGGG ACAACACACG GGCCCTGACC AAATTGGGGT TTCTTTGTCT 120

ACCTCATACC TTCCAAATCA AAAAATAATT TCCCTAGTAT TTTAATTACT CCCCCAAATC 180

AGGAATAACT TCCTCACTGT GCTGATTTTG GTTCTTTTAA AATAAGGTGG TAATTTGAAG 240

GTAATAGTTA AACCAGTCAT AGATTATTCT ATG CCA TTC TTT TCA AAT CAG CCC 294
Met Pro Phe Phe Ser Asn Gln Pro
-25 -20

ACT CAG GTG TCA GTC CTA CTT TTC TTT TGT TGT AGT CCT CTT TAT TCT 342
Thr Gln Val Ser Val Leu Leu Phe Phe Cys Cys Ser Pro Leu Tyr Ser
-15 -10 -5

CCT TTG TTT CTG CTC CAV CTC ATC CCC CAC CAG 375
Pro Leu Phe Leu Leu Xaa Leu Ile Pro His Gln
1               5                   10

(2) INFORMATION FOR SEQ ID NO: 169:

(i) SEQUENCE CHARACTERISTICS:
(A) LENGTH: 376 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 32..163
(C) IDENTIFICATION METHOD: Von Heijne matrix
(D) OTHER INFORMATION: score 4.1
seq IAVGLTCQHVSHA/IS

(xi) SEQUENCE DESCRIPTION: SEQ ID NO: 169:

GCTGCGGCCC GGCCCGGCGG GTAAATAACA G ATG CGG GTG AAA GAT CCA ACT 52
Met Arg Val Lys Asp Pro Thr
-40

AAA GCT TTA CCT GAG AAA GCC AAA AGA AGT AAA AGG CCT ACT GTA CCT 100
Lys Ala Leu Pro Glu Lys Ala Lys Arg Ser Lys Arg Pro Thr Val Pro
-35 -30 -25

CAT GAT GAA GAC TCT TCA GAT GAT ATT GCT GTA GGT TTA ACT TGC CAA     148
His Asp Glu Asp Ser Ser Asp Asp Ile Ala Val Gly Leu Thr Cys Gln
    -20                 -15                 -10

CAT GTA AGT CAT GCT ATC AGC GTG AAT CAT GTA AAG AGA GCA ATA GCT     196
His Val Ser His Ala Ile Ser Val Asn His Val Lys Arg Ala Ile Ala
 -5                     1               5                   10

GAG AAT CTG TGG TCA GTT TGC TCA GAA TGT TTA AAA GAA AGA AGA TTC     244
Glu Asn Leu Trp Ser Val Cys Ser Glu Cys Leu Lys Glu Arg Arg Phe
            15                  20                  25

TAT GAT GGG CAG CTA GTA CTT ACT TCT GAT ATT TGG TTG TGC CTC AAG     292
Tyr Asp Gly Gln Leu Val Leu Thr Ser Asp Ile Trp Leu Cys Leu Lys
        30                  35                  40

TGT GGC TTC CAG GGA TGT GGT AAA AAC TCA GAA AGC CAA CAT TCA TTG     340
Cys Gly Phe Gln Gly Cys Gly Lys Asn Ser Glu Ser Gln His Ser Leu
    45                  50                  55

AAG CAC TTT AAG AGT TCC AGA ACA GAG CCC CTC AGG                     376
Lys His Phe Lys Ser Ser Arg Thr Glu Pro Leu Arg
 60                 65                  70

(2) INFORMATION FOR SEQ ID NO: 170:

    (i) SEQUENCE CHARACTERISTICS:
        (A) LENGTH: 152 base pairs
        (B) TYPE: NUCLEIC ACID
        (C) STRANDEDNESS: DOUBLE
        (D) TOPOLOGY: LINEAR

    (ii) MOLECULE TYPE: CDNA

    (vi) ORIGINAL SOURCE:
        (A) ORGANISM: Homo Sapiens
        (F) TISSUE TYPE: Heart

    (ix) FEATURE:
        (A) NAME/KEY: sig_peptide
        (B) LOCATION: 9..140
        (C) IDENTIFICATION METHOD: Von Heijne matrix
        (D) OTHER INFORMATION:  score 4.1
                                seq GTYLTSSSPLCQL/QP

    (xi) SEQUENCE DESCRIPTION: SEQ ID NO: 170:
ACTTTAAT ATG GTG TCC TTG GGT TAT TAT TTA ATA TTT GTC CTA TAT CTT     50
TGG CTT TGT TTC ATG CAA ATT AGT GAA GAG AAG TTA ATA GAG GAA CAC     98
ACA GGT ACA TAT TTA ACC TCC AGT TCA CCC CTC TGC CAG CTC CAG CCC     146
CCA GGG 152

(2) INFORMATION FOR SEQ ID NO: 171:

(i) SEQUENCE CHARACTERISTICS:
(A) LENGTH: 259 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 128..232
(C) IDENTIFICATION METHOD: Von Heijne matrix
(D) OTHER INFORMATION: score 4.1
seq VLCCLLIATPTFF/LL

(xi) SEQUENCE DESCRIPTION: SEQ ID NO: 171:

ATATTATTAA ACTTTTTATT TTGAGGTTAG TGTGGATTGA AATACACTTC CAACAATTAA 60

CACAAAGGTC CCCTGTGTCC TTTACCCAGT TTTCCACAAT GGTAACATCT TACAAAACTG 120

GAGTACA ATG TCA CTC ACA TCC AGG RTA MYA ATW ATG GWT ACA ATC AAG 169
Met Ser Leu Thr Ser Arg Xaa Xaa Ile Met Xaa Thr Ile Lys
-35 -30 -25

ATA CAG AAT ATT TCT ATT ACA AAG GTC TTG TGT TGC CTT CTT ATA GCA 217
Ile Gln Asn Ile Ser Ile Thr Lys Val Leu Cys Cys Leu Leu Ile Ala
-20 -15 -10

ACA CCT ACT TTC TTC CTA CTC CTT CCC TCA TCC ATT CCA CGG 259
Thr Pro Thr Phe Phe Leu Leu Leu Pro Ser Ser Ile Pro Arg
-5 1 5

(2) INFORMATION FOR SEQ ID NO: 172:

(i) SEQUENCE CHARACTERISTICS:
(A) LENGTH: 217 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:

(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 137..190
(C) IDENTIFICATION METHOD: Von Heijne matrix
(D) OTHER INFORMATION: score 4.1
seq AGVVSTSVAAAVA/AV

(xi) SEQUENCE DESCRIPTION: SEQ ID NO: 172:

AAGCGCAACC GGAACTAGCC TTCTGGGGGC CGGCTTCCTT TATCTCTGGC GGCCTTGTAG 60

TCGTCTCCGA GACTCCCCAC CCCTCCTTCC CTCTTGACCC CCTAGGTTTG ATTGCCCTTT 120

CCCCGAAACA ACTATC ATG ARC GCC GAG GCT GCC GGT GTT GTC TCC ACC TCG 172
Met Xaa Ala Glu Ala Ala Gly Val Val Ser Thr Ser
-15 -10

GTG GCC GCG GCT GTT GCT GCT GTC GCT GCT CCT GCT GGG GCC GGG 217
Val Ala Ala Ala Val Ala Ala Val Ala Ala Pro Ala Gly Ala Gly
-5 1 5

(2) INFORMATION FOR SEQ ID NO: 173:

(i) SEQUENCE CHARACTERISTICS:
(A) LENGTH: 196 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 101..145
(C) IDENTIFICATION METHOD: Von Heijne matrix
(D) OTHER INFORMATION: score 4
seq IMSSCLALTYTNS/IS

(xi) SEQUENCE DESCRIPTION: SEQ ID NO: 173:

TTGGTATCTG GAGTGTTGTA GTGTGTTTGT ATTTGCTTAT AAATAAGTAT TATAGATAAA 60

GATAAACTTC ATAAAGAGTG GATATTTTGG GGAAAATTTC ATG TGG ATA ATG TCA 115
Met Trp Ile Met Ser
-15

TCC TGT CTG GCA TTG ACA TAC ACA AAT TCA ATC TCA CAT AGT CTT TGC 163
Ser Cys Leu Ala Leu Thr Tyr Thr Asn Ser Ile Ser His Ser Leu Cys
-10 -5 1 5

CTT GAG AGA GCG TAC AGT CTA TTC AAA GTT GAC                    196
Leu Glu Arg Ala Tyr Ser Leu Phe Lys Val Asp
            10                  15

(2) INFORMATION FOR SEQ ID NO: 174:

      (i) SEQUENCE CHARACTERISTICS:
            (A) LENGTH: 214 base pairs
            (B) TYPE: NUCLEIC ACID
            (C) STRANDEDNESS: DOUBLE
            (D) TOPOLOGY: LINEAR

      (ii) MOLECULE TYPE: CDNA

      (vi) ORIGINAL SOURCE:
            (A) ORGANISM: Homo Sapiens
            (D) DEVELOPMENTAL STAGE: Fetal
            (F) TISSUE TYPE: kidney

      (ix) FEATURE:
            (A) NAME/KEY: sig_peptide
            (B) LOCATION: 65..124
            (C) IDENTIFICATION METHOD: Von Heijne matrix
            (D) OTHER INFORMATION:  score 4
                                    seq SNALVLVTRGSSS/LP

      (xi) SEQUENCE DESCRIPTION: SEQ ID NO: 174:
ACAGTGTGGC TCGGTTGAAT AGGAGAGCTT TAACTGCATT CTCTTGTGAG AATGCAGTBG   60
AAGA ATG CCA AGA GGA GTG TAC AAT TCA AAT GCG TTA GTG CTT GTA ACA   109
CGT GGT TCC AGT TCT CTC CCT CTT GGC TTG TAT GGT ATA AAT TGT GTA   157
CAG GTA ATT AAG TTA TTT TAT AGA GGC CAT CTC CAC TGG GAA ACT TTG   205
CTG CCA TCG                                                        214

(2) INFORMATION FOR SEQ ID NO: 175:

      (i) SEQUENCE CHARACTERISTICS:
            (A) LENGTH: 353 base pairs
            (B) TYPE: NUCLEIC ACID
            (C) STRANDEDNESS: DOUBLE
            (D) TOPOLOGY: LINEAR

      (ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 210..341
(C) IDENTIFICATION METHOD: Von Heijne matrix
(D) OTHER INFORMATION: score 4
seq FLLPCVHPFSVIA/VY

(xi) SEQUENCE DESCRIPTION: SEQ ID NO: 175:

AATTTATGAT AGGAAATGAT TGATCAAGTG TCACACAGCT GATTATCAGG TCTCAGTCTA 60

ATATTTATTC CTTATTGGTC TCTGCTTAAC TTCAAGTAGG TTATAGATTC CTTAATGGAC 120

TGATAGTTTA TGTCTTATAG CTTTACCTTT CAGGCGCTTA GTTTCATATT GGGAACATGA 180

CAAGTGAATA ATAAATACAT GATAGCTCT ATG ATT GAA CCC TGT GAG AAA ATG 233
Met Ile Glu Pro Cys Glu Lys Met
-40

AAG CAT TAT GAT ATG AAT TGG TTT CTG TGT ATG TAT GAG TGT TTT TTT 281
Lys His Tyr Asp Met Asn Trp Phe Leu Cys Met Tyr Glu Cys Phe Phe
-35 -30 -25

TTY CAT CTT TTG GAA ACA GAA TTT CTG CTC CCC TGT GTA CAC CCT TTC 329
Phe His Leu Leu Glu Thr Glu Phe Leu Leu Pro Cys Val His Pro Phe
-20 -15 -10 -5

TCT GTA ATT GCA GTG TAT GTT TTT 353
Ser Val Ile Ala Val Tyr Val Phe
1

(2) INFORMATION FOR SEQ ID NO: 176:

(i) SEQUENCE CHARACTERISTICS:
(A) LENGTH: 307 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 134..298
(C) IDENTIFICATION METHOD: Von Heijne matrix
(D) OTHER INFORMATION: score 4
seq AALCGISLSQXFP/EP

(xi) SEQUENCE DESCRIPTION: SEQ ID NO: 176:

AGCCTCCGCC TTTGCCTTCG CAGCCGCCTC CAGGGCAATT TGCATATTTC TCCAAAGAAC 60

CATCCAGAAC CTGAGCAGCC TGTCTTCAGA CAGAGATAGG CCCACGGCTG TTTCTTGAAA 120

TCTGGCGCTG GGA ATG GCC ATG TGG AAC AGG CCA TGC CAG ARG CTG CCT 169
Met Ala Met Trp Asn Arg Pro Cys Gln Xaa Leu Pro
-55 -50 -45

CAG CAG CCT CTG GTA GCT GAG CCC ACT GCA GAG GGG GAG CCA CAC CTG 217
Gln Gln Pro Leu Val Ala Glu Pro Thr Ala Glu Gly Glu Pro His Leu
-40 -35 -30

CCC ACG GGC CGG GAG CTG ACT GAG GCC AAC CGC TTC GCC TAT GCT GCC 265
Pro Thr Gly Arg Glu Leu Thr Glu Ala Asn Arg Phe Ala Tyr Ala Ala
-25 -20 -15

CTC TGT GGC ATC TCC CTG TCC CAG TKA TTT CCT GAA CCG GGG 307
Leu Cys Gly Ile Ser Leu Ser Gln Xaa Phe Pro Glu Pro Gly
-10 -5 1

(2) INFORMATION FOR SEQ ID NO: 177:

(i) SEQUENCE CHARACTERISTICS:
(A) LENGTH: 189 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 130..180
(C) IDENTIFICATION METHOD: Von Heijne matrix
(D) OTHER INFORMATION: score 4
seq CLLVSYAVDSAAG/RF

(xi) SEQUENCE DESCRIPTION: SEQ ID NO: 177:

ATTGTCAAAA AGACATCAAA CTCAACTTCT GGGAAGACAG ATTTTTAATA CACATACTTG 60

GCTAATACTC ACAAACATAT CTAAAGTTTT GGCAAAATTA TGAGGGTGAT GGGTKGGTAC 120

TAACCTGGC ATG GAG CAG GTG TGT CTT TTG GTT TCT TAT GCA GTT GAC TCT 171
Met Glu Gln Val Cys Leu Leu Val Ser Tyr Ala Val Asp Ser
-15 -10 -5

GCT GCA GGG AGA TTC GGG 189
Ala Ala Gly Arg Phe Gly
1

(2) INFORMATION FOR SEQ ID NO: 178:

(i) SEQUENCE CHARACTERISTICS:
(A) LENGTH: 364 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 20..103
(C) IDENTIFICATION METHOD: Von Heijne matrix
(D) OTHER INFORMATION: score 4
seq ATLRCWASTPVSG/RL

(xi) SEQUENCE DESCRIPTION: SEQ ID NO: 178:

ACAAAGAGGC AGCTCCGGA ATG AGA AAG ATA AGC CAC TGC CTC CAC TGC TGG 52
Met Arg Lys Ile Ser His Cys Leu His Cys Trp
-25 -20

CCC GAG TCG GGG GCA ACA TTG AGG TGC TGG GCT TCA ACA CCC GTC AGC 100
Pro Glu Ser Gly Ala Thr Leu Arg Cys Trp Ala Ser Thr Pro Val Ser
-15 -10 -5

GGA AGG CTT TCC TCA ATG GCT GTK RWG SSG CKG GGG GAA AKG CCA CCA 148
Gly Arg Leu Ser Ser Met Ala Val Xaa Xaa Xaa Gly Glu Xaa Pro Pro
1               5                   10                  15

CAG GAT GCC TTC ACC ACA CAG TGG CTG GTG CGG GAC CTG AGG GGC AAG 196
Gln Asp Ala Phe Thr Thr Gln Trp Leu Val Arg Asp Leu Arg Gly Lys
            20                  25                  30

ACT GAG AAG GAG TTT AAG GCC TAT GTG TCT TTG TTC ATG CGC CAT CTG 244
Thr Glu Lys Glu Phe Lys Ala Tyr Val Ser Leu Phe Met Arg His Leu
        35                  40                  45

TGT GAG CCT GGG GCA GAC GGC TCT GAA ACC TTT GCC GAT GGG GTC CCT 292
Cys Glu Pro Gly Ala Asp Gly Ser Glu Thr Phe Ala Asp Gly Val Pro
    50                  55                  60

CGG GAG GGA CTG AGT CGC CAG CAG GTG TTG ACC CGC ATT GGA GTC ATG 340
Arg Glu Gly Leu Ser Arg Gln Gln Val Leu Thr Arg Ile Gly Val Met
65 70 75

TCT CTC GTC AAA AAG AAG GGG CAG 364
Ser Leu Val Lys Lys Lys Gly Gln
80 85

(2) INFORMATION FOR SEQ ID NO: 179:

(i) SEQUENCE CHARACTERISTICS:
(A) LENGTH: 249 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 172..237
(C) IDENTIFICATION METHOD: Von Heijne matrix
(D) OTHER INFORMATION: score 4
seq LLHPCGSITLTSS/ST

(xi) SEQUENCE DESCRIPTION: SEQ ID NO: 179:
AAAATTTTTT TAGCCTCTAA CATGAAAGGG TCTCTTCATT GTTCTCATTT GTCTTACCCG 60
CCATCCAGTG TTAAGCAGTA TGTTAAAGAG CTTCTTCTTT ACAACTTTTC CCCTCACATT 120
ATTTTYCTAC ATGCAGCAAC TTCTTTAACC AAGTTGTTTG ATTAGGAGTA A ATG TGC 177
ATA AAC GAT CAT ATT ATT AAG CTT CTG CAC CCA TGT GGC AGC ATC ACT 225
TTA ACT TCT TCC TCA ACC ACA CGG 249

(2) INFORMATION FOR SEQ ID NO: 180:

(i) SEQUENCE CHARACTERISTICS:
(A) LENGTH: 269 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 135..185
(C) IDENTIFICATION METHOD: Von Heijne matrix

(D) OTHER INFORMATION:  score 4
                           seq VALQCGLTIPALX/LP

(xi) SEQUENCE DESCRIPTION: SEQ ID NO: 180:

AGAAGGGGTG TCAAACTCCA ATGGAAAAGG TTTAGGAAAA GACCTTTTAC AAATCCAAAG   60

ATGTTTCACA GTGGGCGAGG CTGGTGTGGC GACAGTAGTG GCCCACATGG CTGGGTTGGG  120

AGCCAGCTCT GCCC ATG AGG TGC CGT GTG GCT TTG CAG TGT GGC CTC ACA   170
                Met Arg Cys Arg Val Ala Leu Gln Cys Gly Leu Thr
                        -15                 -10

ATC CCA GCT TTG TNT CTT CCC CAG GGA GAT GAG GCT GGT GAT GCT CAA   218
Ile Pro Ala Leu Xaa Leu Pro Gln Gly Asp Glu Ala Gly Asp Ala Gln
 -5                  1               5                  10

GAT CTC AGA GGC CCT GCC CAG GCT GAG TAT CTG TAT ATA ATA TCC CCC   266
Asp Leu Arg Gly Pro Ala Gln Ala Glu Tyr Leu Tyr Ile Ile Ser Pro
            15                  20                  25

TCG                                                               269
Ser

(2) INFORMATION FOR SEQ ID NO: 181:

(i) SEQUENCE CHARACTERISTICS:
     (A) LENGTH: 441 base pairs
     (B) TYPE: NUCLEIC ACID
     (C) STRANDEDNESS: DOUBLE
     (D) TOPOLOGY: LINEAR

(ii) MOLECULE TYPE: CDNA

(vi) ORIGINAL SOURCE:
     (A) ORGANISM: Homo Sapiens
     (D) DEVELOPMENTAL STAGE: Fetal
     (F) TISSUE TYPE: kidney

(ix) FEATURE:
     (A) NAME/KEY: sig_peptide
     (B) LOCATION: 88..366
     (C) IDENTIFICATION METHOD: Von Heijne matrix
     (D) OTHER INFORMATION:  score 3.9
                             seq LTSAFLWLPRLHI/SV

(xi) SEQUENCE DESCRIPTION: SEQ ID NO: 181:

ATATAACTCA GTTTTCTGTT GTCTTTAGCT ACTGATGCAA ATGTGAAGAA TGAAAGTCTT   60

TCATCTGTGC AGCAGCTTGG CATTAAA ATG ACT GTC AGG TAT GGC AAA TTC CTC  114
                              Met Thr Val Arg Tyr Gly Lys Phe Leu
                                          -90                 -85

AGT CTC TTA AAA GAT GGT GCA GAA AAT GAT CTT ACC TGG GTT TTA AAG   162
Ser Leu Leu Lys Asp Gly Ala Glu Asn Asp Leu Thr Trp Val Leu Lys
            -80                 -75                 -70

CAT TGT GAG AGA TTC CTG AAA CAG CAG CAA ACT TCC ATA AAA TCT TCT 210
His Cys Glu Arg Phe Leu Lys Gln Gln Gln Thr Ser Ile Lys Ser Ser
-65 -60 -55

CTT CTC TGC CTG CAA GGG AAT TAT GCT GGC CAT GAC TGG TTT GTA TCT 258
Leu Leu Cys Leu Gln Gly Asn Tyr Ala Gly His Asp Trp Phe Val Ser
-50 -45 -40

TCT CTG TTC ATG ATA ATG TTG GGA GAC AAA GAA AAA ACA TTC CAA TTT 306
Ser Leu Phe Met Ile Met Leu Gly Asp Lys Glu Lys Thr Phe Gln Phe
-35 -30 -25

CTT CAT CAA TTC TCC AGG CTT CTG ACT TCT GCT TTT CTT TGG TTG CCA 354
Leu His Gln Phe Ser Arg Leu Leu Thr Ser Ala Phe Leu Trp Leu Pro
-20 -15 -10 -5

AGG CTA CAT ATT TCT GTA AGA CTT CAA TCT GTT TTT AAA GGA GGG TTT 402
Arg Leu His Ile Ser Val Arg Leu Gln Ser Val Phe Lys Gly Gly Phe
1 5 10

GAM ATT TTA AGA ACA TTA TAC TTA CAT TCA MCG GGA CGG 441
Xaa Ile Leu Arg Thr Leu Tyr Leu His Ser Xaa Gly Arg
15 20 25

(2) INFORMATION FOR SEQ ID NO: 182:

(i) SEQUENCE CHARACTERISTICS:
(A) LENGTH: 261 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 160..219
(C) IDENTIFICATION METHOD: Von Heijne matrix
(D) OTHER INFORMATION: score 3.9
seq FFWVVLFSAGCKV/IT

(xi) SEQUENCE DESCRIPTION: SEQ ID NO: 182:

AACAGAGCCA CAGAATGCTG AGCAGTCAAC AGCATTTCTT GTTCCAAGAT CACCCTTCTG 60

AGTACCTCTC TGGCTGCCAA ATTGCCAGGG CCTTCACAGT TTGATTCCAT TTCTCAGCTC 120

CAAGCATTAG GTAAACCCAC CAAGCAATCC TAGCCTGTG ATG GCG TTT GAC GTC 174
Met Ala Phe Asp Val
-20

AGC TGC TTC TTT TGG GTG GTG CTG TTT TCT GCC GGC TGT AAA GTC ATC 222

Ser Cys Phe Phe Trp Val Val Leu Phe Ser Ala Gly Cys Lys Val Ile
-15                 -10                 -5                  1

ACC TCC TGG GAT CAG ATG TGC ATT GAG AAA GAA GCC ACA                261
Thr Ser Trp Asp Gln Met Cys Ile Glu Lys Glu Ala Thr
            5                   10

(2) INFORMATION FOR SEQ ID NO: 183:

(i) SEQUENCE CHARACTERISTICS:
(A) LENGTH: 289 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 167..232
(C) IDENTIFICATION METHOD: Von Heijne matrix
(D) OTHER INFORMATION: score 3.9
seq HLSSTTSPPWTHA/AI

(xi) SEQUENCE DESCRIPTION: SEQ ID NO: 183:

AAAAACGCCT TGAGGATAAG GAAGGAGAAT CAGCAAGTCC CGAGTTCCTA CGGTGTGTCA   60

GCATCGTGCT CCCACTCCCG GGAGAGAGGC ATTATCTTCA GTTTACAAAA GGGGAAAACA  120

GGTCTGGGGT TTCCAGAGTC CGCGGTTTTG CTAAGAAGCC GCAGTG ATG TTG ACG     175
                                                   Met Leu Thr
                                                           -20

CGG CTG GTC CTC AGT GCA CAC CTG AGT AGC ACG ACC TCT CCG CCC TGG    223
Arg Leu Val Leu Ser Ala His Leu Ser Ser Thr Thr Ser Pro Pro Trp
                -15                 -10                 -5

ACG CAC GCT GCC ATC AGC TGG GAG CTG GAC AAC GTG CTG ATG CCT AGT    271
Thr His Ala Ala Ile Ser Trp Glu Leu Asp Asn Val Leu Met Pro Ser
            1               5                   10

CCC AGA ATC TGG CCC CTG                                            289
Pro Arg Ile Trp Pro Leu
    15

(2) INFORMATION FOR SEQ ID NO: 184:

(i) SEQUENCE CHARACTERISTICS:
(A) LENGTH: 478 base pairs
(B) TYPE: NUCLEIC ACID

(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 326..445
(C) IDENTIFICATION METHOD: Von Heijne matrix
(D) OTHER INFORMATION: score 3.9
seq CVNLLLGFEPVIS/RS

(xi) SEQUENCE DESCRIPTION: SEQ ID NO: 184:

ATAAAACTTA GGGGGAAGAT TTGCCTCTCA CTTTTTTTCT TGGAAAATGT GGGCAGCAAT 60

TTTAAAGAGA ACATGAAAAT GGAGTAGGTT GAAACCAACA TTCAGAACTT CCTTTCATGG 120

ATTGAAACTT AAAGCTGAGG GAGGKTTTRA GGGTGGARKT RAGGAAGGGC TAGAAGATAG 180

CAAATTTCAG AGTCATATCA GAGAATATGA ACTGTCAGTG TTTCCAATGT TTCTCTTGGC 240

TCTGCACAGC ACTTCCAAGC CCTTTTGCTC ACTGTTTTGC TTCTGCCACA CCTAGGAGAA 300

GATTCAGAGC TTGCTGAGGC AAAAC ATG CGA TAT TTC CAA GGG CCT TCC CCC 352
Met Arg Tyr Phe Gln Gly Pro Ser Pro
-40 -35

TAT TCT GAA ATA GAA ATT GAG CTT TGT GAT CAT GTG TAT TCA TTC CAA 400
Tyr Ser Glu Ile Glu Ile Glu Leu Cys Asp His Val Tyr Ser Phe Gln
-30 -25 -20

GGT CTA TGT GTT AAC CTT TTG CTA GGA TTT GAA CCT GTT ATT AGT AGG 448
Gly Leu Cys Val Asn Leu Leu Leu Gly Phe Glu Pro Val Ile Ser Arg
-15 -10 -5 1

AGC CGR MGC AGT TCA CTT GCT GTT GAG TCT 478
Ser Arg Xaa Ser Ser Leu Ala Val Glu Ser
                5                   10

(2) INFORMATION FOR SEQ ID NO: 185:

(i) SEQUENCE CHARACTERISTICS:
(A) LENGTH: 257 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal

(F) TISSUE TYPE: kidney

(ix) FEATURE:
(A) NAME/KEY: sig_peptide
(B) LOCATION: 48..170
(C) IDENTIFICATION METHOD: Von Heijne matrix
(D) OTHER INFORMATION: score 3.9
seq LASLECYVPSTNQ/WQ

(xi) SEQUENCE DESCRIPTION: SEQ ID NO: 185:

ACTGCAGATA CGATCCCCGC TTCAACACCT GGATACACCT GGCCAGS ATG AAN HAG 56
Met Xaa Xaa
-40

AAG CGC ACG CAC TKV VNS STG AGC GTG TTC AAC GGG CTC GTG TAC GCC 104
Lys Arg Thr His Xaa Xaa Xaa Ser Val Phe Asn Gly Leu Val Tyr Ala
-35 -30 -25

GCG GGC GGC CGC AAC GCA GAA GGA AGC CTG GCC TCG CTG GAG TGC TAC 152
Ala Gly Gly Arg Asn Ala Glu Gly Ser Leu Ala Ser Leu Glu Cys Tyr
-20 -15 -10

GTG CCC TCC ACC AAT CAG TGG CAG CCG AAG HHN SCC CTG GAG GTG GCG 200
Val Pro Ser Thr Asn Gln Trp Gln Pro Lys Xaa Xaa Leu Glu Val Ala
-5 1 5 10

CGC TGC TGC CAC GCT AGC GCG GTC GCC GAC GGC CGC GTG CTG GTG ACC 248
Arg Cys Cys His Ala Ser Ala Val Ala Asp Gly Arg Val Leu Val Thr
15 20 25

GGA GGC TTG 257
Gly Gly Leu

(2) INFORMATION FOR SEQ ID NO: 186:

(i) SEQUENCE CHARACTERISTICS:
(A) LENGTH: 377 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 249..362
(C) IDENTIFICATION METHOD: Von Heijne matrix
(D) OTHER INFORMATION: score 3.9
seq LLFFHLLLNDFFT/FY

(xi) SEQUENCE DESCRIPTION: SEQ ID NO: 186:

ACATCCAGCT CTGGTAGTTT AGGCTCAATC TTACGGTGTA ATTATACAGA ATAATTAGAG 60

GCAGCTGTAT CCTTGTTTCT GATTTTAAAA TCTGRATGTT TCTYCAATTC TTTGTGTACT 120

CTCCCTTCAT TTGGTACATA TAGAAGTCTT CTTATGTGTT ATTAAAGTCT TCTAAGATAG 180

TATTCTGGTC ATTGGAGACA CCAAAAATCT ATGGGCACAG TCCTGTTCCT GTTTCTTTTG 240

CCAATAGA ATG TTC CTT AAG GTT CAG TCA CAG TCC TTT TAC DTC CCT TAC 290
Met Phe Leu Lys Val Gln Ser Gln Ser Phe Tyr Xaa Pro Tyr
-35 -30 -25

AGA GAT TGT TTA AAT TTC CAC AAA AGC ACG TAT TTA CTC TTC TTT CAC 338
Arg Asp Cys Leu Asn Phe His Lys Ser Thr Tyr Leu Leu Phe Phe His
-20 -15 -10

TTG TTA CTA AAT GAC TTC TTC ACA TTT TAC NTT GCT AAA 377
Leu Leu Leu Asn Asp Phe Phe Thr Phe Tyr Xaa Ala Lys
-5 1 5

(2) INFORMATION FOR SEQ ID NO: 187:

(i) SEQUENCE CHARACTERISTICS:
(A) LENGTH: 226 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: 119..199
(C) IDENTIFICATION METHOD: Von Heijne matrix
(D) OTHER INFORMATION: score 3.9
seq WIILIIYTFQCNS/SL

(xi) SEQUENCE DESCRIPTION: SEQ ID NO: 187:

CAGAATGTTC TTTGCTGCCT CGCTTACATG GCAAAACTCA CAAACCACCT ATACAATCCA 60

AAAGAGGGGA AACAGCTCAT CTCATATTAA TTATGGTCCA TTTCBATGAT AGGATATT 118

ATG CAA CCA TTA AAA ATC ATA TTT TAT CTG AGT GTT AGT ATA TGG ATT 166
Met Gln Pro Leu Lys Ile Ile Phe Tyr Leu Ser Val Ser Ile Trp Ile
-25 -20 -15

ATT TTA ATT ATT TAT ACT TTT CAG TGT AAT TCT TCT CTG AGC ATA CTA 214
Ile Leu Ile Ile Tyr Thr Phe Gln Cys Asn Ser Ser Leu Ser Ile Leu
-10 -5 1 5

CTT TTG GAG TTA 226
Leu Leu Glu Leu

(2) INFORMATION FOR SEQ ID NO: 188:

(i) SEQUENCE CHARACTERISTICS:
(A) LENGTH: 192 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 10..66
(C) IDENTIFICATION METHOD: Von Heijne matrix
(D) OTHER INFORMATION: score 3.9
seq RVAACTAAAPLQA/HG

(xi) SEQUENCE DESCRIPTION: SEQ ID NO: 188:

AAGTGATGG ATG ATG AGA ACG ACA GCG AGA GTC GCT GCG TGT ACT GCT GCA 51
Met Met Arg Thr Thr Ala Arg Val Ala Ala Cys Thr Ala Ala
-15 -10

GCC CCA TTG CAA GCC CAC GGT GCA GRC ATT CAG CAG GRT CCA GAC AGS 99
Ala Pro Leu Gln Ala His Gly Ala Xaa Ile Gln Gln Xaa Pro Asp Xaa
-5 1 5 10

CTC TGS TCT RGA AGG CTC AGC AGA GRR GGR CTT TCT GCA GGG CGR CTG 147
Leu Xaa Ser Xaa Arg Leu Ser Arg Xaa Gly Leu Ser Ala Gly Arg Leu
15 20 25

CAC CAR AGC GAA ACA GAA GCT GAA CTG GAR GCC CCG GGT CGC GCG 192
His Gln Ser Glu Thr Glu Ala Glu Leu Glu Ala Pro Gly Arg Ala
30 35 40

(2) INFORMATION FOR SEQ ID NO: 189:

(i) SEQUENCE CHARACTERISTICS:
(A) LENGTH: 274 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Kidney

(ix) FEATURE:
(A) NAME/KEY: sig_peptide

(B) LOCATION: 140..241
(C) IDENTIFICATION METHOD: Von Heijne matrix
(D) OTHER INFORMATION: score 3.8
seq RWASSCLHPSARS/SN

(xi) SEQUENCE DESCRIPTION: SEQ ID NO: 189:

AASCCCAASG TGCTGCCGTT GCCCGTACAA CTCGGACTTG CTGTTGCTCG AGCCGCGTCT 60

GCACGGGTCT CGGACCGAGC GGAGTCCMAG CCTCGGTCCC GGAGCCCACC TTCGCCTCGC 120

CCTTGCCCAG CCTGCGGTG ATG GAG GCG GCC ACC ACA CTG CAC CCA GGC CCG 172
Met Glu Ala Ala Thr Thr Leu His Pro Gly Pro
-30 -25

CGC CCG GCG CTG CCC CTC GGG GCC CGG GCC CGC TGG GCG AGT TCC TGC 220
Arg Pro Ala Leu Pro Leu Gly Ala Arg Ala Arg Trp Ala Ser Ser Cys
-20 -15 -10

CTC CAC CCG AGT GCC CGG TCT TCG AAC CCA GCT GGG AAG AGT TCG CGG 268
Leu His Pro Ser Ala Arg Ser Ser Asn Pro Ala Gly Lys Ser Ser Arg
-5 1 5

ACC CCT 274
Thr Pro
10

(2) INFORMATION FOR SEQ ID NO: 190:

(i) SEQUENCE CHARACTERISTICS:
(A) LENGTH: 196 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: 92..178
(C) IDENTIFICATION METHOD: Von Heijne matrix
(D) OTHER INFORMATION: score 3.8
seq LCPVIFFPSNCWK/EY

(xi) SEQUENCE DESCRIPTION: SEQ ID NO: 190:
AAGAAAGGAC ATTTTTTTTT TCTTGTACTA ACTAGGCTGG ATTYYCCAAA TTGTTTGAGT 60
GGCCCCTGCC CCTCTTAATG CTTCTGTAAG A ATG CAA GGT GTC AGG GGA CCT 112
GTG TCC TTT TCC TGG AGC ACA ACC ATG TTG TGT CCT GTT ATA TTC TTT 160
CCA TCC AAC TGT TGG AAA GAA TAT AAC AGG ACA CAG                196

(2) INFORMATION FOR SEQ ID NO: 191:

(i) SEQUENCE CHARACTERISTICS:
(A) LENGTH: 236 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 177..230
(C) IDENTIFICATION METHOD: Von Heijne matrix
(D) OTHER INFORMATION:  score 3.8
                        seq FXLLFXXFXFFRQ/XG

(xi) SEQUENCE DESCRIPTION: SEQ ID NO: 191:

ACAAGTCTGT CCTCCCTAGG CTGGCAGCTC TGTCAGCACC CAGGTTGTTA GAATAGTTGT   60

TAAAACAGGT CATTCTGTTG CCAAGTAATT ACGGGGCCTT GSACTCAGTA ACCTTCCCCA  120

CGAAGCAGGC CGTAGTGTGC TTACTGCTCT CCCTTGSCTT TCCATCCCCT ACTTTG ATG  179
                                                              Met

TKG GRR TTT TCT TTC YTT TTA CTT TTC YTT TAW TTT CYT TTT TTC CGC    227
Xaa Xaa Phe Ser Phe Xaa Leu Leu Phe Xaa Xaa Phe Xaa Phe Phe Arg
        -15                 -10                 -5

CAG KCT GGG                                                        236
Gln Xaa Gly
    1

(2) INFORMATION FOR SEQ ID NO: 192:

(i) SEQUENCE CHARACTERISTICS:
(A) LENGTH: 451 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 359..427
(C) IDENTIFICATION METHOD: Von Heijne matrix
(D) OTHER INFORMATION: score 3.8
seq SVRLLFRFSVIMA/SE

(xi) SEQUENCE DESCRIPTION: SEQ ID NO: 192:

ACACTGTGAA ATGCAATTGT GCCTTGAATA AGAAGGTACC TAGAAGCCAA ATTAAAGTAA 60

TAATGACTTC TTATTGGCTT TGATTTTTCA TTGCAGTATA TGGGAATTGT ACAGCAGGAA 120

ATGCTTATCA TTAATTTCTG ATGTTTTTTA AAGCACAACT CGAAACATTT CGATCATACA 180

TACATAGCAG TAGAGATCTG TGCCCTTCAG GTACATTGWA TCTGACCATC AGTTTATATA 240

TGTCATTGAA TTTTAAGAAT ACTCATGTTA ATAATAGTCA TCTATCCTTG CATTTTGAAA 300

CTGTTCTAAT CTTAGTGAAC TTGAATTGGA TTTCTGGGTA AAAGAATGTG TTTCTTTT 358

ATG TTG CTT CTG TCC GAA GCC TTG TCA GAA TCT GTC AGA CTC TTG TTT 406
Met Leu Leu Leu Ser Glu Ala Leu Ser Glu Ser Val Arg Leu Leu Phe
-20 -15 -10

AGG TTT AGT GTG ATC ATG GCG TCA GAG AAG CAA AGC TTT CAA ATA 451
Arg Phe Ser Val Ile Met Ala Ser Glu Lys Gln Ser Phe Gln Ile
-5 1 5

(2) INFORMATION FOR SEQ ID NO: 193:

(i) SEQUENCE CHARACTERISTICS:
(A) LENGTH: 399 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 319..369
(C) IDENTIFICATION METHOD: Von Heijne matrix
(D) OTHER INFORMATION: score 3.8
seq SLPCTTAFPLLSS/KV

(xi) SEQUENCE DESCRIPTION: SEQ ID NO: 193:

ATTCTTCTCT GGTTACCTCT ATCTACCCCC GAGTCAACAA GCCCTGCCTG ATTACGCAGC 60

AGCAGTTTCT CCTGGAGAGT ATATGCCCTT CCCTACCAGA GTGGCTGTGC TCTGTGGACC 120

AACGGCATTT GTGCCGTGGC TGGTGTTTCC ACCATTCCAG TGGGTTGGCT GCAGAGTTAT 180

CCTTTGTGGG TGGGAGAGAG CACCAGGCCT CAGGAATCTC CCTGCTGGTC CCAGCCTCCA 240

TCTCCTCCTC CCCAACCCTG AACCTCTCCC GCAACCTGCA CCTCCCCCGA GAAGCCAGCC 300

ACAGAGGCAG AGAGCATC ATG GCT CTT ATC AGC CTG CCA TGC ACG ACA GCT 351
Met Ala Leu Ile Ser Leu Pro Cys Thr Thr Ala
-15 -10

TTC CCT TTA CTG TCC AGC AAG GTT TCC CAG CTT CTC TTG CCC CTC AGC 399
Phe Pro Leu Leu Ser Ser Lys Val Ser Gln Leu Leu Leu Pro Leu Ser
-5 1 5 10

(2) INFORMATION FOR SEQ ID NO: 194:

(i) SEQUENCE CHARACTERISTICS:
(A) LENGTH: 253 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: 83..193
(C) IDENTIFICATION METHOD: Von Heijne matrix
(D) OTHER INFORMATION: score 3.8
seq RVVALPLVRATCT/AV

(xi) SEQUENCE DESCRIPTION: SEQ ID NO: 194:

AGTGGAGAGT CGAGCCTGGG GTCGGCGGAG ACWGCTGGTG TCTGAAGCCG CTCGCGCCCA 60

GGGTGACCCT GTTTGCAGCA CG ATG TCT GAA GAA GAG GCG GCT CAG ATC CCC 112
Met Ser Glu Glu Glu Ala Ala Gln Ile Pro
-35 -30

AGA TCC AGT GTG TGG GAG CAG GAC CAG CAG AAC GTG GTG CAG CGT GTG 160
Arg Ser Ser Val Trp Glu Gln Asp Gln Gln Asn Val Val Gln Arg Val
-25 -20 -15

GTG GCT CTG CCC CTG GTC AGG GCC ACG TGC ACC GCG GTC TGC GAT GTT 208
Val Ala Leu Pro Leu Val Arg Ala Thr Cys Thr Ala Val Cys Asp Val
-10 -5 1 5

TAC AGT GCA GCC AAG GAC AGG CAC CCG CTG CTG GGC TCC GCC TGG 253
Tyr Ser Ala Ala Lys Asp Arg His Pro Leu Leu Gly Ser Ala Trp

10 15 20

(2) INFORMATION FOR SEQ ID NO: 195:

(i) SEQUENCE CHARACTERISTICS:
(A) LENGTH: 298 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 8..223
(C) IDENTIFICATION METHOD: Von Heijne matrix
(D) OTHER INFORMATION: score 3.8
seq LAELTVDPQGALA/IR

(xi) SEQUENCE DESCRIPTION: SEQ ID NO: 195:

AAAAAAG ATG GCG GCG GCG GCG GCA GCT GGT GCG GCC TCC GGG CTG CCG 49
Met Ala Ala Ala Ala Ala Ala Gly Ala Ala Ser Gly Leu Pro
-70 -65 -60

GGT CCA GTG GCA CAA GGA TTA AAG GAA GCG TTA GTG GAT ACG CTC ACC 97
Gly Pro Val Ala Gln Gly Leu Lys Glu Ala Leu Val Asp Thr Leu Thr
-55 -50 -45

GGG ATC CTA TCC CCA GTA CAG GAG GTG CGG GCG GCT GCT GAA GAA CAG 145
Gly Ile Leu Ser Pro Val Gln Glu Val Arg Ala Ala Ala Glu Glu Gln
-40 -35 -30

ATT AAG GTG CTG GAG GTG ACG GAG GAA TTT GGT GTT CAC TTG GCA GAA 193
Ile Lys Val Leu Glu Val Thr Glu Glu Phe Gly Val His Leu Ala Glu
-25 -20 -15

CTG ACT GTA GAT CCC CAG GGG GCA CTG GCA ATC CGT CAG CTG GCA TCA 241
Leu Thr Val Asp Pro Gln Gly Ala Leu Ala Ile Arg Gln Leu Ala Ser
-10 -5 1 5

GTC ATC TTG AAA CAA TAT GTG GAG ACT CAC TGG TGT GCC CAA TCA GAG 289
Val Ile Leu Lys Gln Tyr Val Glu Thr His Trp Cys Ala Gln Ser Glu
10 15 20

AAA TTT AGG 298
Lys Phe Arg
25

(2) INFORMATION FOR SEQ ID NO: 196:

(i) SEQUENCE CHARACTERISTICS:
(A) LENGTH: 503 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 114..464
(C) IDENTIFICATION METHOD: Von Heijne matrix
(D) OTHER INFORMATION: score 3.8
seq XXXYLNFCPVCYC/FS

(xi) SEQUENCE DESCRIPTION: SEQ ID NO: 196:

GTGAATTCGC CAGCGGGAGC GCGCTCGCGG WCCGCGCGTT CTCCGCTTTC CCGGCTCCGT 60

CGCTGACGCG TCGTAGASTT GGSVWGCGGG AAGGCAACGG CAGCGGGATC GGG ATG 116
Met

AAC AGC GGC GGC GGC TTC GGT TTG GGC TTA GGC TTC GGC CTC ACC CCC 164
Asn Ser Gly Gly Gly Phe Gly Leu Gly Leu Gly Phe Gly Leu Thr Pro
-115 -110 -105

ACG TCG GTG ATT CAG GTG ACG AAT CTG TCG TCG GCG GTG ACC AGC GAG 212
Thr Ser Val Ile Gln Val Thr Asn Leu Ser Ser Ala Val Thr Ser Glu
-100 -95 -90 -85

CAG ATG CGG ACG CTT TTT TCC TTC CTA GGA GAA ATC GAG GAG CTG CGG 260
Gln Met Arg Thr Leu Phe Ser Phe Leu Gly Glu Ile Glu Glu Leu Arg
-80 -75 -70

CTC TAC CCC CCG GAC AAC GCA CCT CTT GCT TTT TCC TCB DRA GTA TGT 308
Leu Tyr Pro Pro Asp Asn Ala Pro Leu Ala Phe Ser Ser Xaa Val Cys
-65 -60 -55

TAT GTT AAG TTT CGT GAT CCA TCA AGT GTT GGA GTG GCC CAG CAT CTA 356
Tyr Val Lys Phe Arg Asp Pro Ser Ser Val Gly Val Ala Gln His Leu
-50 -45 -40

ACT AAC ACG GTT TTT ATT GAC AGA GST CTG RAT AGT TGT TCC TTG TGC 404
Thr Asn Thr Val Phe Ile Asp Arg Xaa Leu Xaa Ser Cys Ser Leu Cys
-35 -30 -25

AGA AGG TTG GTA TCT CGC TTT KTT TGN HBT TAT TTG AAT TTC TGT CCT 452
Arg Arg Leu Val Ser Arg Phe Xaa Xaa Xaa Tyr Leu Asn Phe Cys Pro
-20 -15 -10 -5

GTC TGT TAT TGC TTT AGC TTT CCT AGA GAT TGG CAA GTA GAC AGT ACT 500
Val Cys Tyr Cys Phe Ser Phe Pro Arg Asp Trp Gln Val Asp Ser Thr
1 5 10

CTC 503

Leu

(2) INFORMATION FOR SEQ ID NO: 197:

(i) SEQUENCE CHARACTERISTICS:
(A) LENGTH: 175 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 47..85
(C) IDENTIFICATION METHOD: Von Heijne matrix
(D) OTHER INFORMATION: score 3.7
seq MIEMLIFLDCVLS/SK

(xi) SEQUENCE DESCRIPTION: SEQ ID NO: 197:

ATTAACAAAG AGCAAGTTTA ACCTGAGTGG TCAACTTTTG CAGCAG ATG ATT GAR 55
Met Ile Glu

ATG CTA ATA TTT CTA GAC TGT GTC CTG TCT TCC AAA GAT ACA ATA ACC 103
Met Leu Ile Phe Leu Asp Cys Val Leu Ser Ser Lys Asp Thr Ile Thr
-10 -5 1 5

ATG TTT GTG AAG TTC ATA CCT ATT TTT CCT TTT CCT TTG CAG TTT TAT 151
Met Phe Val Lys Phe Ile Pro Ile Phe Pro Phe Pro Leu Gln Phe Tyr
10 15 20

TTG CCC TCT TTC CTT CTT TTG GAG 175
Leu Pro Ser Phe Leu Leu Leu Glu
25 30

(2) INFORMATION FOR SEQ ID NO: 198:

(i) SEQUENCE CHARACTERISTICS:
(A) LENGTH: 291 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:

(A) NAME/KEY: sig_peptide
(B) LOCATION: 49..285
(C) IDENTIFICATION METHOD: Von Heijne matrix
(D) OTHER INFORMATION: score 3.7
seq VIGSLLVLTMLTC/RR

(xi) SEQUENCE DESCRIPTION: SEQ ID NO: 198:

ACATCACAAA AATTAGGTGA CCATGGTTAT GATAATTCTT TGCCTAGT ATG CAT CCA 57
Met His Pro

TTT CTA GCT GCC CAC GGA CCT GCA TTT CAC AAA GGC TAC AAG CAT AGC 105
Phe Leu Ala Ala His Gly Pro Ala Phe His Lys Gly Tyr Lys His Ser
-75 -70 -65

ACA ATT AAC ATT GTG GAT ATT TAT CCA ATG ATG TGC CAC ATC CTG GGA 153
Thr Ile Asn Ile Val Asp Ile Tyr Pro Met Met Cys His Ile Leu Gly
-60 -55 -50 -45

TTA AAA CCA CAT CCC AAT AAT GGG ACC TTT GGT CAT ACT AAG TGC TTG 201
Leu Lys Pro His Pro Asn Asn Gly Thr Phe Gly His Thr Lys Cys Leu
-40 -35 -30

TTA GTT GAC CAG TGG TGC ATT AAT CTC CCA GAA GCC ATC GCG ATT GTT 249
Leu Val Asp Gln Trp Cys Ile Asn Leu Pro Glu Ala Ile Ala Ile Val
-25 -20 -15

ATC GGT TCA CTC TTG GTG TTA ACC ATG CTA ACA TGC CGC CGG 291
Ile Gly Ser Leu Leu Val Leu Thr Met Leu Thr Cys Arg Arg
-10 -5 1

(2) INFORMATION FOR SEQ ID NO: 199:

(i) SEQUENCE CHARACTERISTICS:
(A) LENGTH: 122 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: 33..74
(C) IDENTIFICATION METHOD: Von Heijne matrix
(D) OTHER INFORMATION: score 3.7
seq IWPMSASVATLWS/FT

(xi) SEQUENCE DESCRIPTION: SEQ ID NO: 199:
ATCTTAGTGT GACACATGAA CCCCTCCCCT TC ATG ATC TGG CCT ATG TCT GCC 53
TCT GTA GCT ACT CTC TGG TCC TTT ACC TCT TAC ATA AGC TAC CCA AGC     101
AGG TTT TAC TAT GAT GCT TGG                                         122

(2) INFORMATION FOR SEQ ID NO: 200:

(i) SEQUENCE CHARACTERISTICS:
(A) LENGTH: 266 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 12..104
(C) IDENTIFICATION METHOD: Von Heijne matrix
(D) OTHER INFORMATION:  score 3.6
                        seq LFIYLVFVECLLC/TR

(xi) SEQUENCE DESCRIPTION: SEQ ID NO: 200:

AAGGGTAATG G ATG GGA ATT GAT ATT TTC TAT CCT TCA CAC ATC CCA GAC     50
             Met Gly Ile Asp Ile Phe Tyr Pro Ser His Ile Pro Asp
                 -30                 -25                 -20

TTT CAT CCT ATT CAT TTA TTC ATT TAT CTA GTG TTT GTA GAG TGC CTT      98
Phe His Pro Ile His Leu Phe Ile Tyr Leu Val Phe Val Glu Cys Leu
            -15                 -10                 -5

CTG TGT ACC AGG AAC TGR GAW AGK TTG TCC KGA TTC AAC TGT GAT AAC     146
Leu Cys Thr Arg Asn Xaa Xaa Xaa Leu Ser Xaa Phe Asn Cys Asp Asn
        1               5                   10

GCT CAA ATA ATC TTC ACA ACA GGC TCA TCC TCT AGT GGA GGA AAT AAA     194
Ala Gln Ile Ile Phe Thr Thr Gly Ser Ser Ser Ser Gly Gly Asn Lys
 15                 20                  25                  30

CCA TTT AAA AGT AGT TTA TGT ACA GTA CAT AGA GGC CAA GAA AGG GAA     242
Pro Phe Lys Ser Ser Leu Cys Thr Val His Arg Gly Gln Glu Arg Glu
                35                  40                  45

AGA ATA GAG TGC CAA GGG AAT GGG                                     266
Arg Ile Glu Cys Gln Gly Asn Gly
            50

(2) INFORMATION FOR SEQ ID NO: 201:

(i) SEQUENCE CHARACTERISTICS:
(A) LENGTH: 371 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 24..284
(C) IDENTIFICATION METHOD: Von Heijne matrix
(D) OTHER INFORMATION: score 3.6
seq LILQASLKGELEA/SQ

(xi) SEQUENCE DESCRIPTION: SEQ ID NO: 201:

AAATAGCTGA TTATGAACGT TTG ATG AAA GAA CTA AAT CAA AAG TTA ACT AAT 53
Met Lys Glu Leu Asn Gln Lys Leu Thr Asn
-85 -80

AAA AAC AAC AAG ATA GAA GAT TTG GAG CAA GAA ATA AAA ATT CAA AAA 101
Lys Asn Asn Lys Ile Glu Asp Leu Glu Gln Glu Ile Lys Ile Gln Lys
-75 -70 -65

CAG AAA CAA GAA ACC CTA CAA GAA GAA ATA ACT TCA TTA CAG TCT TCA 149
Gln Lys Gln Glu Thr Leu Gln Glu Glu Ile Thr Ser Leu Gln Ser Ser
-60 -55 -50

GTA CAA GAA TAT GAA GAA AAA AAC WCC AAA ATC AAG CAA TTG CTT GTG 197
Val Gln Glu Tyr Glu Glu Lys Asn Xaa Lys Ile Lys Gln Leu Leu Val
-45 -40 -35 -30

AAA ACC AAA AAG GAA CTG GCA GAT TCA AAG CAA GCA GAA ACT GAT CAC 245
Lys Thr Lys Lys Glu Leu Ala Asp Ser Lys Gln Ala Glu Thr Asp His
-25 -20 -15

TTA ATA CTT CAA GCA TCT TTA AAA GGT GAG CTG GAG GCA AGC CAG CAG 293
Leu Ile Leu Gln Ala Ser Leu Lys Gly Glu Leu Glu Ala Ser Gln Gln
-10 -5 1

CAA GTA GAA GTC TAT AAA GTA AGG GTT TTA CTT TTT AAG ATT AAA AAA 341
Gln Val Glu Val Tyr Lys Val Arg Val Leu Leu Phe Lys Ile Lys Lys
                5                   10                  15

ATG TTT TTT CAT GTA GAA GTG AGG AAC GGG 371
Met Phe Phe His Val Glu Val Arg Asn Gly
            20                  25

(2) INFORMATION FOR SEQ ID NO: 202:

(i) SEQUENCE CHARACTERISTICS:
(A) LENGTH: 383 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 33..371
(C) IDENTIFICATION METHOD: Von Heijne matrix
(D) OTHER INFORMATION: score 3.6
seq RLLLCILIIVCYI/LF

(xi) SEQUENCE DESCRIPTION: SEQ ID NO: 202:

ACAGTCCTAC CTTTGCTGAT GCCTACTCTA AT ATG GGA AAC ACT CTA AAG GAG 53
Met Gly Asn Thr Leu Lys Glu
-110

ATG CAG GAT GTT CAG GGA GCC TTG CAG TGT TAT ACG CGT GCC ATC CAA 101
Met Gln Asp Val Gln Gly Ala Leu Gln Cys Tyr Thr Arg Ala Ile Gln
-105 -100 -95

ATT AAT CCT GCA TTT GCA GAT GCA CAT AGC AAT CTG GCT TCC ATT CAT 149
Ile Asn Pro Ala Phe Ala Asp Ala His Ser Asn Leu Ala Ser Ile His
-90 -85 -80 -75

AAG GAT TCA GGG AAT ATT CCA GAA GCC ATA GCT TCT TAC CGC ACG GCT 197
Lys Asp Ser Gly Asn Ile Pro Glu Ala Ile Ala Ser Tyr Arg Thr Ala
-70 -65 -60

CTG AAA CTT AAG CCT GAT TTT CCT GAT GCT TAT TGT AAC TTG GCT CAT 245
Leu Lys Leu Lys Pro Asp Phe Pro Asp Ala Tyr Cys Asn Leu Ala His
-55 -50 -45

TGC CTG CAG ATT GTC TGT GAT TGG ACA GAC TAT GAT GAG CGA ATG AAG 293
Cys Leu Gln Ile Val Cys Asp Trp Thr Asp Tyr Asp Glu Arg Met Lys
-40 -35 -30

AAG TTG GTC AGT ATT GTG GCT GAC CAG TTA GAG AAG AAT AGG TTG CTT 341
Lys Leu Val Ser Ile Val Ala Asp Gln Leu Glu Lys Asn Arg Leu Leu
-25 -20 -15

CTG TGC ATC CTC ATC ATA GTA TGC TAT ATC CTC TTT CTC ATG 383
Leu Cys Ile Leu Ile Ile Val Cys Tyr Ile Leu Phe Leu Met
-10 -5 1

(2) INFORMATION FOR SEQ ID NO: 203:

(i) SEQUENCE CHARACTERISTICS:

(A) LENGTH: 217 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 92..208
(C) IDENTIFICATION METHOD: Von Heijne matrix
(D) OTHER INFORMATION: score 3.6
seq VAYAIPSIPSLFC/QR

(xi) SEQUENCE DESCRIPTION: SEQ ID NO: 203:
ACATGTTGAG TACTTTTTCC TCACCTGTTT TTCCATTCCT GTTAGCCGGA GCAAAAGGGC 60
CTCCAACTCC TCTTTTAGAG AGAAATGACT A ATG CTC ATA CTA GCA GAT ACC 112
AGA CGT GTC CAA GGA GGT ACC TTG GGC TTA ATT CCA GCA GTT CTC AAC 160
AGA GTC CAC GTG GCA TAT GCT ATA CCC AGC ATA CCT AGC CTC TTC TGC 208
CAG CGC TGG 217

(2) INFORMATION FOR SEQ ID NO: 204:

(i) SEQUENCE CHARACTERISTICS:
(A) LENGTH: 450 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 343..402
(C) IDENTIFICATION METHOD: Von Heijne matrix
(D) OTHER INFORMATION: score 3.6

seq CVFLFPLISNTSS/YK

(xi) SEQUENCE DESCRIPTION: SEQ ID NO: 204:
CACACAATTA ATATTAATGG ATAACTAATT GGAGTAATGA TTATTAGCTA CTGAATGCTG 60
ATAATAGAAG TCATATTTAA ATGCTTACTT AGTTACTTAA GTTAGTCAAG GACTCTGAAA 120
AAAATAAGGT TTAAAGTTAA CAGTGTCATC AGTCATTCCC AGTTATCTTC TTATTTAAGA 180
ACAAGATGGT AATGCAGTTG CCTTTGTTTA TTTAAATAGA AAAAATTAAA TCAGGATAAA 240
ATGACCCAAC TACAGTGATG TATTTGGACA CACTACTTCT TATCTTTCAA TATAGACTTT 300
TATTTCTGGA TTACCATAGA TGGAAATAGT ATTACTGGAC AT ATG TTG GTA GGT 354
ATT TAC TTC TGT GTT TTT CTT TTT CCC TTA ATT TCG AAT ACT TCT AGC 402
TAC AAA AAT TGT CAT AAA ACT TTG CAA CAC ACT ATA CCT CCC CAC GGG 450

(2) INFORMATION FOR SEQ ID NO: 205:

(i) SEQUENCE CHARACTERISTICS:
(A) LENGTH: 201 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 1..126
(C) IDENTIFICATION METHOD: Von Heijne matrix
(D) OTHER INFORMATION: score 3.5
seq LLLQGACPCLIFL/RP

(xi) SEQUENCE DESCRIPTION: SEQ ID NO: 205:

ATG TTT CTC GCT CCC TCT CTG CTG ATC ACA AAG CTG CTG ACC GGG TCA 48
Met Phe Leu Ala Pro Ser Leu Leu Ile Thr Lys Leu Leu Thr Gly Ser
-40 -35 -30

GAA AGT CCT GAT GGA AAT CCA CCA GCG CTG GGC AGG CCC CTC CTC CTC 96
Glu Ser Pro Asp Gly Asn Pro Pro Ala Leu Gly Arg Pro Leu Leu Leu
-25 -20 -15

CAG GGA GCT TGT CCT TGC CTA ATT TTT CTT CGT CCT GAT GAG AAC AAA     144
Gln Gly Ala Cys Pro Cys Leu Ile Phe Leu Arg Pro Asp Glu Asn Lys
-10                 -5                  1               5

AAA GAG GGG GRG GAG GAA AAG AAA AAC CAC AAA CTT CCT TTG AAA ACC     192
Lys Glu Gly Xaa Glu Glu Lys Lys Asn His Lys Leu Pro Leu Lys Thr
            10                  15                  20

AGC TTA GGG                                                         201
Ser Leu Gly
        25

(2) INFORMATION FOR SEQ ID NO: 206:

(i) SEQUENCE CHARACTERISTICS:
(A) LENGTH: 306 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: 235..288
(C) IDENTIFICATION METHOD: Von Heijne matrix
(D) OTHER INFORMATION:  score 3.5
seq SKSCLFYLQKVSG/IP

(xi) SEQUENCE DESCRIPTION: SEQ ID NO: 206:

AAAGGTGGCT TCAGGACCAC CTCCTGAGAG CTTCGTTGTA TTTCATGTAT ATTTCCCCAA    60

ATATATCAGC ATCTGACCCT TGGCTTCTGG GAGAAAGACA GAGGCGGAAC CCTGGCCGCC   120

CCAGAGAGAG GCAGCTGTGG GGGCAGAGAT GTAACAACCC TTTGAACCTT GACCTTGGAC   180

GCCAGGCTGT CCGGGAGCTT CTCCCACAAT GGCTGTTTTG GGGATGTGAC CTGG ATG     237
                                                            Met

GAC CCA TCT GCT AGC AAA TCC TGT CTG TTT TAC CTC CAA AAA GTA TCT     285
Asp Pro Ser Ala Ser Lys Ser Cys Leu Phe Tyr Leu Gln Lys Val Ser
        -15                 -10                 -5

GGA ATT CCA GGG CTT CTC ACC                                         306
Gly Ile Pro Gly Leu Leu Thr
    1               5

(2) INFORMATION FOR SEQ ID NO: 207:

(i) SEQUENCE CHARACTERISTICS:

(A) LENGTH: 251 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 54..191
(C) IDENTIFICATION METHOD: Von Heijne matrix
(D) OTHER INFORMATION: score 3.5
seq RWLCLQAYLASFS/LE

(xi) SEQUENCE DESCRIPTION: SEQ ID NO: 207:
ACGTGTCCTC AGGATTTTCC TCTTGGGCTG GACAGTTTGC TCCCCTGGAG GGT ATG 56
AGC CTG ACT GCT AGT GGG CCA AGA GCT GCC TGG GAG GAA AGG GTG GGG 104
GGT CTC CAC ACT TGG GGT GCC AAC ATT CCT ACC GCC CCT GAT TCC CAG 152
CGG TGG CTC TGT CTT CAG GCG TAC CTG GCA TCC TTC AGT CTT GAG AGC 200
CCC CAC AGA ATC TAC CTK GAA TCT CCT CCC ACG CTC CTT TTC CCC CCG 248
CCG 251

(2) INFORMATION FOR SEQ ID NO: 208:

(i) SEQUENCE CHARACTERISTICS:
(A) LENGTH: 242 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 117..182
(C) IDENTIFICATION METHOD: Von Heijne matrix
(D) OTHER INFORMATION: score 3.5
seq AQLASPLLPGATP/VA

(xi) SEQUENCE DESCRIPTION: SEQ ID NO: 208:

ACCGCAGAAA ATGCTAGGTG CAAAGTTTGT CGAAAGAAAG GTGAGGATGA CAAATTGATC 60

TTGTGTGATG AGTGTAATAA AGCCTTYCCA CCTGTTTTGT CTGAGGCCGG CCCTCT ATG 119
Met

AAG TAC CAG ATG GTG AGT GGC AGT GCC CAG CTT GCC AGC CCG CTA CTG 167
Lys Tyr Gln Met Val Ser Gly Ser Ala Gln Leu Ala Ser Pro Leu Leu
-20 -15 -10

CCA GGC GCA ACT CCC GTG GCA GGA ACT ATA CTG AAG AGT CTG CTT CTG 215
Pro Gly Ala Thr Pro Val Ala Gly Thr Ile Leu Lys Ser Leu Leu Leu
-5 1 5 10

AGG ACA GTG AAG ATG ATG AGA GTG ATG 242
Arg Thr Val Lys Met Met Arg Val Met
15 20

(2) INFORMATION FOR SEQ ID NO: 209:

(i) SEQUENCE CHARACTERISTICS:
(A) LENGTH: 342 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: 229..333
(C) IDENTIFICATION METHOD: Von Heijne matrix
(D) OTHER INFORMATION: score 3.5
seq CFWGLMYXWLLLG/SX

(xi) SEQUENCE DESCRIPTION: SEQ ID NO: 209:

ACATCTGATC GATAATTATG TCACCTGTAC CTGTCGCCAG CTTGTCTTGT TATGACGTTA 60

GTTTTACTGC TAGAAATATC TAGTAGATGG CTGGAAATCT GCAGGCAAAG TGCAGAGGGA 120

GTGAGCCTGC GAGGAGAGGG SCTGGGCAAA GTGAMBGCCC TGGGCCGCAG AGTTCTTATC 180

TAAAAAATGG GAACAGTAGT GTCTTCCTAA AGGCACCATG GACTTAAA ATG AAT GGC 237
Met Asn Gly
-35

ACG TTT CCT GGG ACT TAT GTA TAT TTG GTT GCT TAT GGG GAC TTA CGT 285
Thr Phe Pro Gly Thr Tyr Val Tyr Leu Val Ala Tyr Gly Asp Leu Arg
-30 -25 -20

ATA TTT GGT TGC TTT TGG GGA CTT ATG TAT ATK TGG TTG CTT TTG GGG 333
Ile Phe Gly Cys Phe Trp Gly Leu Met Tyr Xaa Trp Leu Leu Leu Gly
-15 -10 -5

TCT NAA GGG 342
Ser Xaa Gly
1

(2) INFORMATION FOR SEQ ID NO: 210:

(i) SEQUENCE CHARACTERISTICS:
(A) LENGTH: 340 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: other
(B) LOCATION: 131..222
(C) IDENTIFICATION METHOD: blastn
(D) OTHER INFORMATION: identity 98
region 66..157
id AA134726
est

(ix) FEATURE:
(A) NAME/KEY: other
(B) LOCATION: 216..282
(C) IDENTIFICATION METHOD: blastn
(D) OTHER INFORMATION: identity 97
region 152..218
id AA134726
est

(ix) FEATURE:
(A) NAME/KEY: other
(B) LOCATION: 283..342
(C) IDENTIFICATION METHOD: blastn
(D) OTHER INFORMATION: identity 90
region 220..279
id AA134726
est

(ix) FEATURE:
(A) NAME/KEY: other

(B) LOCATION: 64..103
(C) IDENTIFICATION METHOD: blastn
(D) OTHER INFORMATION:. identity 100
region 1..40
id AA134726
est

(ix) FEATURE:
(A) NAME/KEY: other
(B) LOCATION: 98..130
(C) IDENTIFICATION METHOD: blastn
(D) OTHER INFORMATION: identity 93
region 34..66
id AA134726
est

(ix) FEATURE:
(A) NAME/KEY: other
(B) LOCATION: 81..285
(C) IDENTIFICATION METHOD: blastn
(D) OTHER INFORMATION: identity 97
region 1..205
id R17226
est

(ix) FEATURE:
(A) NAME/KEY: sig_peptide
(B) LOCATION: 50..112
(C) IDENTIFICATION METHOD: Von Heijne matrix
(D) OTHER INFORMATION: score 12.7
seq ILFLLSWSGPLQG/QQ

(xi) SEQUENCE DESCRIPTION: SEQ ID NO: 210:

GAGGCTGACT GTACGTTCCT TCTACTCTGG CACCACTCTC CAGGCTGCC ATG GGG CCC 58
Met Gly Pro
-20

AGC ACC CCT CTC CTC ATC TTG TTC CTT TTG TCA TGG TCG GGA CCC CTC 106
Ser Thr Pro Leu Leu Ile Leu Phe Leu Leu Ser Trp Ser Gly Pro Leu
-15 -10 -5

CAA GGA CAG CAG CAC CAC CTT GTG GAG TAC ATG GAA CGC CGA CTA GCT 154
Gln Gly Gln Gln His His Leu Val Glu Tyr Met Glu Arg Arg Leu Ala
1 5 10

GCT TTA GAG GAA CGG CTG GCC CAG TGC CAG GAC CAG AGT AGT CGG CAT 202
Ala Leu Glu Glu Arg Leu Ala Gln Cys Gln Asp Gln Ser Ser Arg His
15 20 25 30

GCT GCT GAG CTG CGG AAC TTC AAG AAC AAG ATG CTG CCA CTG CTG GAG 250
Ala Ala Glu Leu Arg Asn Phe Lys Asn Lys Met Leu Pro Leu Leu Glu
        35                  40                  45

GTG GCA GAG AAG GAG CGG GAG GCA CTC AGA ACT GAG GCC GRC ACC ATC 298
Val Ala Glu Lys Glu Arg Glu Ala Leu Arg Thr Glu Ala Xaa Thr Ile
    50                  55                  60

TCN NVN GGA GTG GAT CGT CTG GAG CGG GAG GTA GAC TAT CTG 340
Ser Xaa Gly Val Asp Arg Leu Glu Arg Glu Val Asp Tyr Leu

65                  70                  75

(2) INFORMATION FOR SEQ ID NO: 211:

(i) SEQUENCE CHARACTERISTICS:
(A) LENGTH: 321 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 124..310
(C) IDENTIFICATION METHOD: blastn
(D) OTHER INFORMATION: identity 100
region 46..232
id T39765
est

(ix) FEATURE:
(A) NAME/KEY: other
(B) LOCATION: 78..123
(C) IDENTIFICATION METHOD: blastn
(D) OTHER INFORMATION: identity 95
region 1..46
id T39765
est

(ix) FEATURE:
(A) NAME/KEY: sig_peptide
(B) LOCATION: 76..141
(C) IDENTIFICATION METHOD: Von Heijne matrix
(D) OTHER INFORMATION: score 10.5
seq LMLLVSSLSPVQG/VL

(xi) SEQUENCE DESCRIPTION: SEQ ID NO: 211:

AAAATAGGAG TCTCTGGTAC TGCAAACCCA CAGCCTGGAC TCAGAGCTCA AGTCTGAACT 60

CTACCTCCAG ACAGA ATG AAG TTC ATC TCG ACA TCT CTG CTT CTC ATG CTG 111
Met Lys Phe Ile Ser Thr Ser Leu Leu Leu Met Leu
-20 -15

CTG GTC AGC AGC CTC TCT CCA GTC CAA GGT GTT CTG GAG GTC TAT TAC 159
Leu Val Ser Ser Leu Ser Pro Val Gln Gly Val Leu Glu Val Tyr Tyr
-10 -5 1 5

ACA AGC TTG AGG TGT AGA TGT GTC CAA GAG AGC TCA GTC TTT ATC CCT 207
Thr Ser Leu Arg Cys Arg Cys Val Gln Glu Ser Ser Val Phe Ile Pro
10 15 20

AGA CGC TTC ATT GAT CGA ATT CAA ATC TTG CCC CGT GGG AAT GGT TGT     255
Arg Arg Phe Ile Asp Arg Ile Gln Ile Leu Pro Arg Gly Asn Gly Cys
        25                  30                  35

CCA AGA AAA GAA ATC ATA GTC TGG AAG AAG AAC AAG TCA ATT GTG TGT     303
Pro Arg Lys Glu Ile Ile Val Trp Lys Lys Asn Lys Ser Ile Val Cys
    40                  45                  50

GTG GAC CTC AAG CAT AGG                                             321
Val Asp Leu Lys His Arg
55                  60

(2) INFORMATION FOR SEQ ID NO: 212:

    (i) SEQUENCE CHARACTERISTICS:
        (A) LENGTH: 426 base pairs
        (B) TYPE: NUCLEIC ACID
        (C) STRANDEDNESS: DOUBLE
        (D) TOPOLOGY: LINEAR

    (ii) MOLECULE TYPE: CDNA

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: other
        (B) LOCATION: 241..426
        (C) IDENTIFICATION METHOD: blastn
        (D) OTHER INFORMATION:  identity 98
                                region 1..186
                                id T07474
                                est

    (ix) FEATURE:
        (A) NAME/KEY: sig_peptide
        (B) LOCATION: 16..156
        (C) IDENTIFICATION METHOD: Von Heijne matrix
        (D) OTHER INFORMATION:  score 8
                                seq VLELLAAVCLVRG/GH

    (xi) SEQUENCE DESCRIPTION: SEQ ID NO: 212:

AGTTTACGTG CCATC ATG AAT TAT CAG TAT GGT TTC AAC ATG GTC ATG TCT     51
                 Met Asn Tyr Gln Tyr Gly Phe Asn Met Val Met Ser
                         -45                 -40

CAT CCA CAC GCT GTC AAT GAG ATT GCA CTA AGC CTG AAC AAC AAG AAT     99
His Pro His Ala Val Asn Glu Ile Ala Leu Ser Leu Asn Asn Lys Asn
-35                 -30                 -25                 -20

CCC AGA ACA AAA GCC CTT GTC TTA GAA CTG TTG GCA GCC GTT TGT CTT     147
Pro Arg Thr Lys Ala Leu Val Leu Glu Leu Leu Ala Ala Val Cys Leu
                -15                 -10                 -5

GTC AGA GGC GGG CAT GAA ATC ATT TTA TCA GCA TTT GAT AAC TTT AAA     195

Val Arg Gly Gly His Glu Ile Ile Leu Ser Ala Phe Asp Asn Phe Lys
1 5 10

GAG GTT TGT GGA GAA AAA CAG CGC TTT GAG AAG TTG ATG GAA CAT TTC 243
Glu Val Cys Gly Glu Lys Gln Arg Phe Glu Lys Leu Met Glu His Phe
15 20 25

AGG AAT GAA GAC AAT AAC ATA GAT TTT ATG GTG GCT TCT ATG CAG TTT 291
Arg Asn Glu Asp Asn Asn Ile Asp Phe Met Val Ala Ser Met Gln Phe
30 35 40 45

ATT AAT ATT GTA GTC CAT TCA GTA GAA GAT ATG AAT TTC AGA GTT CAC 339
Ile Asn Ile Val Val His Ser Val Glu Asp Met Asn Phe Arg Val His
    50                  55                  60

CTG CAG TAT GAA TTT ACC AAA TTA GGC CTG GMC GAA TAC TTG GRC AAG 387
Leu Gln Tyr Glu Phe Thr Lys Leu Gly Leu Xaa Glu Tyr Leu Xaa Lys
65 70 75

CTG AAA CAC ACT GAG AGT GAC AAG CTT CAA GTC CAG ATC 426
Leu Lys His Thr Glu Ser Asp Lys Leu Gln Val Gln Ile
80 85 90

(2) INFORMATION FOR SEQ ID NO: 213:

(i) SEQUENCE CHARACTERISTICS:
(A) LENGTH: 387 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 246..387
(C) IDENTIFICATION METHOD: blastn
(D) OTHER INFORMATION: identity 100
region 1..142
id HUM75821
est

(ix) FEATURE:
(A) NAME/KEY: other
(B) LOCATION: 246..387
(C) IDENTIFICATION METHOD: blastn
(D) OTHER INFORMATION: identity 100
region 1..142
id T08488
est

(ix) FEATURE:
(A) NAME/KEY: other
(B) LOCATION: 261..387

(C) IDENTIFICATION METHOD: blastn
(D) OTHER INFORMATION: identity 100
region 1..127
id R54273
est

(ix) FEATURE:
(A) NAME/KEY: sig_peptide
(B) LOCATION: 205..288
(C) IDENTIFICATION METHOD: Von Heijne matrix
(D) OTHER INFORMATION: score 7.7
seq LVMCFLSYFGTFA/VE

(xi) SEQUENCE DESCRIPTION: SEQ ID NO: 213:

ATTGGTAATT TTCAGCTCAC AAATGATGAA GAAATCCATA ACGTCGGAAC TTCCTTGACC 60

TTTGGATTTG GCACATTGAC CTGCTGGATC CAGGCTGCGC TGACACTCAA GGTCAACATC 120

AASAATGAAG GACGGAGAGT TGGAATTCCA CGGGTTATTC TGTCGGCATC TATCACTCTC 180

TGTGTGGTCC TCTACTTCAT CCTC ATG GCC CAA AGC ATC CAC ATG TAT GCA 231
Met Ala Gln Ser Ile His Met Tyr Ala
-25 -20

GCC AGG GTC CAG TGG GGC CTG GTC ATG TGC TTC CTG TCT TAT TTT GGC 279
Ala Arg Val Gln Trp Gly Leu Val Met Cys Phe Leu Ser Tyr Phe Gly
-15 -10 -5

ACC TTT GCC GTG GAG TTC CGG CAT TAC CGC TAT GAG ATT GTT TGC TCT 327
Thr Phe Ala Val Glu Phe Arg His Tyr Arg Tyr Glu Ile Val Cys Ser
1 5 10

GAG TAC CAG GAG AAT TTC CTA AGC TTC TCA GAA AGC CTG TCA GAA GCT 375
Glu Tyr Gln Glu Asn Phe Leu Ser Phe Ser Glu Ser Leu Ser Glu Ala
15 20 25

TCT GAA TAT CAG 387
Ser Glu Tyr Gln
30

(2) INFORMATION FOR SEQ ID NO: 214:

(i) SEQUENCE CHARACTERISTICS:
(A) LENGTH: 339 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other

(B) LOCATION: 124..335
(C) IDENTIFICATION METHOD: blastn
(D) OTHER INFORMATION: identity 95
region 1..212
id AA081335
est

(ix) FEATURE:
(A) NAME/KEY: other
(B) LOCATION: 212..309
(C) IDENTIFICATION METHOD: blastn
(D) OTHER INFORMATION: identity 95
region 1..98
id H88204
est

(ix) FEATURE:
(A) NAME/KEY: other
(B) LOCATION: 296..335
(C) IDENTIFICATION METHOD: blastn
(D) OTHER INFORMATION: identity 100
region 82..121
id H88204
est

(ix) FEATURE:
(A) NAME/KEY: other
(B) LOCATION: 284..335
(C) IDENTIFICATION METHOD: blastn
(D) OTHER INFORMATION: identity 100
region 1..52
id W31695
est

(ix) FEATURE:
(A) NAME/KEY: sig_peptide
(B) LOCATION: 76..138
(C) IDENTIFICATION METHOD: Von Heijne matrix
(D) OTHER INFORMATION: score 7.1
seq LHLFHLLIRPXQG/WX

(xi) SEQUENCE DESCRIPTION: SEQ ID NO: 214:

ACTCTCTGCT GAACTCCCAA AGGGAGTGTG TGTATTTCCT CCCGTTCTTN ATCAGAGCCC 60

CCAAAATAAG TAGGA ATG GGC AGT GGC TAT TCA CAT TCA CTA CAC CTT TTC 111
Met Gly Ser Gly Tyr Ser His Ser Leu His Leu Phe
-20 -15 -10

CAT TTG CTA ATA AGG CCC TGS CAA GGT TGG RAG GRA ATT GTC CCT GCC 159
His Leu Leu Ile Arg Pro Xaa Gln Gly Trp Xaa Xaa Ile Val Pro Ala
-5 1 5

TGC TTC TGG AGA AAG AAG ATA TTG ACA CCA TCT ACG GGC ACC ATG GAA 207
Cys Phe Trp Arg Lys Lys Ile Leu Thr Pro Ser Thr Gly Thr Met Glu
10 15 20

CTG CTT CAA GTG ACC ATT CTT TTT CTT CTG CCC AGT ATT TGC AGC AGT 255
Leu Leu Gln Val Thr Ile Leu Phe Leu Leu Pro Ser Ile Cys Ser Ser
25 30 35

AAC AGC ACA GGT GTT TTA GAG GCA GCT AAT AAT TCA CTT GTT GTT ACT    303
Asn Ser Thr Gly Val Leu Glu Ala Ala Asn Asn Ser Leu Val Val Thr
 40                  45                  50                  55

ACA ACA AAA CCA TCT ATA ACA ACA CCA AAC ACG TGG                    339
Thr Thr Lys Pro Ser Ile Thr Thr Pro Asn Thr Trp
                60                  65

(2) INFORMATION FOR SEQ ID NO: 215:

(i) SEQUENCE CHARACTERISTICS:
(A) LENGTH: 363 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: other
(B) LOCATION: 209..324
(C) IDENTIFICATION METHOD: blastn
(D) OTHER INFORMATION:  identity 97
                        region 1..116
                        id AA081350
                        est

(ix) FEATURE:
(A) NAME/KEY: other
(B) LOCATION: 277..324
(C) IDENTIFICATION METHOD: blastn
(D) OTHER INFORMATION:  identity 97
                        region 3..50
                        id AA046671
                        est

(ix) FEATURE:
(A) NAME/KEY: sig_peptide
(B) LOCATION: 157..204
(C) IDENTIFICATION METHOD: Von Heijne matrix
(D) OTHER INFORMATION:  score 6.7
                        seq CFSLVLLLTSIWT/TR

(xi) SEQUENCE DESCRIPTION: SEQ ID NO: 215:

AGGGAAATCC GGATGTCTCG GTTATGAAGT GGAGCAGTGA GTGTGAGCCT CAACATAGTT   60

CCAGAACTCT CCATCCGGAC TAGTTATTGA GCATCTGCCT CTCATATCAC CAGTGGCCAT  120

CTGAGGTGTT TCCCTGGCTC TGAAGGGGTA GGCACG ATG GCC AGG TGC TTC AGC    174
                                        Met Ala Arg Cys Phe Ser
                                            -15

CTG GTG TTG CTT CTC ACT TCC ATC TGG ACC ACG AGG CTC CTG GTC CAA     222
Leu Val Leu Leu Leu Thr Ser Ile Trp Thr Thr Arg Leu Leu Val Gln
-10                 -5                  1                   5

GGC TCT TTG CGT GCA GAA GAG CTT TCC ATC CAG GTG TCA TGC AGA ATT     270
Gly Ser Leu Arg Ala Glu Glu Leu Ser Ile Gln Val Ser Cys Arg Ile
            10                  15                  20

ATG GNN RTC ACC CTT GTG AGC AAA AAG GCG AAC CAG CAG CTG AAT TTC     318
Met Xaa Xaa Thr Leu Val Ser Lys Lys Ala Asn Gln Gln Leu Asn Phe
        25                  30                  35

ACA GAA NNV NAA GGA GGC CWW WAR GCT GCT GGG ACT AAG TTT GGC         363
Thr Glu Xaa Xaa Gly Gly Xaa Xaa Ala Ala Gly Thr Lys Phe Gly
    40                  45                  50

(2) INFORMATION FOR SEQ ID NO: 216:

(i) SEQUENCE CHARACTERISTICS:
(A) LENGTH: 290 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: other
(B) LOCATION: 20..194
(C) IDENTIFICATION METHOD: blastn
(D) OTHER INFORMATION: identity 96
region 144..318
id AA045920
est

(ix) FEATURE:
(A) NAME/KEY: other
(B) LOCATION: 194..257
(C) IDENTIFICATION METHOD: blastn
(D) OTHER INFORMATION: identity 100
region 319..382
id AA045920
est

(ix) FEATURE:
(A) NAME/KEY: other
(B) LOCATION: 20..226
(C) IDENTIFICATION METHOD: blastn
(D) OTHER INFORMATION: identity 94
region 153..359
id N25870
est

(ix) FEATURE:
(A) NAME/KEY: other

(B) LOCATION: 220..262
(C) IDENTIFICATION METHOD: blastn
(D) OTHER INFORMATION: identity 95
region 355..397
id N25870
est

(ix) FEATURE:
(A) NAME/KEY: other
(B) LOCATION: 20..176
(C) IDENTIFICATION METHOD: blastn
(D) OTHER INFORMATION: identity 94
region 143..299
id H99323
est

(ix) FEATURE:
(A) NAME/KEY: other
(B) LOCATION: 212..267
(C) IDENTIFICATION METHOD: blastn
(D) OTHER INFORMATION: identity 92
region 335..390
id H99323
est

(ix) FEATURE:
(A) NAME/KEY: other
(B) LOCATION: 67..262
(C) IDENTIFICATION METHOD: blastn
(D) OTHER INFORMATION: identity 95
region 1..196
id AA150024
est

(ix) FEATURE:
(A) NAME/KEY: sig_peptide
(B) LOCATION: 171..269
(C) IDENTIFICATION METHOD: Von Heijne matrix
(D) OTHER INFORMATION: score 6.7
seq MTCLSVLFGYATS/HP

(xi) SEQUENCE DESCRIPTION: SEQ ID NO: 216:

AATCTTGTCA GAAGTCGTCG AAAATATTTA CACCAGCAGC TCCAGTTCAT ACCAATAAAG 60

AAGATCCTGC TACCCAAACT AATTTGGGRW TTATCCAWGC ATTTGKCGCT GCCATATCAG 120

TTATTAWTGK ATCYGAATTG GGTGATAAGA CATTTTTTAT AGCAGCCATC ATG GCA 176
Met Ala

ATG CGC TAT AAC CGC CTG ACC GTG CTG GCT GGT GCA ATG CTT GCC TTG 224
Met Arg Tyr Asn Arg Leu Thr Val Leu Ala Gly Ala Met Leu Ala Leu
-30 -25 -20

GGA CTA ATG ACA TGC TTG TCA GTT TTG TTT GGC TAT GCC ACC AGT CAT 272
Gly Leu Met Thr Cys Leu Ser Val Leu Phe Gly Tyr Ala Thr Ser His
-15 -10 -5 1

CCC CAG GGK CTA TAC ATA 290
Pro Gln Gly Leu Tyr Ile

                5

(2) INFORMATION FOR SEQ ID NO: 217:

(i) SEQUENCE CHARACTERISTICS:
(A) LENGTH: 369 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 319..370
(C) IDENTIFICATION METHOD: blastn
(D) OTHER INFORMATION: identity 92
region 31..82
id R51759
est

(ix) FEATURE:
(A) NAME/KEY: other
(B) LOCATION: 288..318
(C) IDENTIFICATION METHOD: blastn
(D) OTHER INFORMATION: identity 100
region 1..31
id R51759
est

(ix) FEATURE:
(A) NAME/KEY: sig_peptide
(B) LOCATION: 211..288
(C) IDENTIFICATION METHOD: Von Heijne matrix
(D) OTHER INFORMATION: score 6.3
seq RQLLLPLPPFSFP/AP

(xi) SEQUENCE DESCRIPTION: SEQ ID NO: 217:

AGTCAATTCT AGGAGCCATC AAGCATGAAA GTGGTTCTGT CTCCTGAGCG CAASCTCGCC 60

GGACCCCTGG GCGAAGGCCT GGACTTGCAG ATGTGTGTTC CCTGTGCGGG TGGACAGAGG 120

GGGCCCTTAT GACCCACATT GCAGCCCCAT TCCACCACCC CTTCCTCCCC AGAGCAGTCT 180

CTGCCGAGGG ACAGCACCTG TGTCCCTTCG ATG CCA CAA CAG CCA GTT GAA CAG 234
Met Pro Gln Gln Pro Val Glu Gln
-25 -20

GGG AGC CCT TTG CTC AGG CAG CTT CTC CTG CCT CTC CCT CCT TTC TCC 282
Gly Ser Pro Leu Leu Arg Gln Leu Leu Leu Pro Leu Pro Pro Phe Ser
-15 -10 -5

TTC CCT GCC CCA TCC CCG TGC CCT TCT TGG CCT GTG GCG CTG GGG AGC     330
Phe Pro Ala Pro Ser Pro Cys Pro Ser Trp Pro Val Ala Leu Gly Ser
        1               5       .           10

CAT GGT GTG GCA TAC TGG GGC TCC TGC TCC TTG GGS CAC                 369
His Gly Val Ala Tyr Trp Gly Ser Cys Ser Leu Gly His
15                  20                  25

(2) INFORMATION FOR SEQ ID NO: 218:

(i) SEQUENCE CHARACTERISTICS:
(A) LENGTH: 390 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 117..390
(C) IDENTIFICATION METHOD: blastn
(D) OTHER INFORMATION:  identity 99
                        region 1..274
                        id C16636
                        est

(ix) FEATURE:
(A) NAME/KEY: sig_peptide
(B) LOCATION: 121..360
(C) IDENTIFICATION METHOD: Von Heijne matrix
(D) OTHER INFORMATION:  score 6.2
                        seq RASLLPMLLGSWA/FL

(xi) SEQUENCE DESCRIPTION: SEQ ID NO: 218:

AAAAAAGAGC TGGTTCCCTG GCAGGCTGGA GGGCAGGAGC TGGGGCCACG CTGGTCTGGG   60

ATAGTTGGGC AGGGAGGCTG TCTACCTGGT CTTCCAGAAT GGACGGCCCT GTGGCAGAGC  120

ATG CCA AGC AGG AGC CCT TTC ACG TGG TCA CAC CTC TGT TGG AGA GCT     168
Met Pro Ser Arg Ser Pro Phe Thr Trp Ser His Leu Cys Trp Arg Ala
-80                 -75                 -70                 -65

GGG CGC TGT CCC AGG TGG CGG GCA TGC CTG TCT TCC TCA AGT GTG AGA     216
Gly Arg Cys Pro Arg Trp Arg Ala Cys Leu Ser Ser Ser Ser Val Arg
                -60                 -55                 -50

ATG TGC AGC CCA GCG GCT CCT TCA AGA TTC GGG GCA TTG GGC ATN TCT     264
Met Cys Ser Pro Ala Ala Pro Ser Arg Phe Gly Ala Leu Gly Xaa Ser
            -45                 -40                 -35

GCC AGG AGA TGG CCA AGA AGG GAT GCA GAC ACC TGG TGT GCT CCT CAG     312

Ala Arg Arg Trp Pro Arg Arg Asp Ala Asp Thr Trp Cys Ala Pro Gln
     -30                -25                -20

GGG GTA ATG CGG GCA TCG CTG CTG CCT ATG CTG CTA GGA AGC TGG GCA     360
Gly Val Met Arg Ala Ser Leu Leu Pro Met Leu Leu Gly Ser Trp Ala
    -15                 -10                 -5

TTC CTG CCA CCA TCG TGC TCC CCG AGA GCA                             390
Phe Leu Pro Pro Ser Cys Ser Pro Arg Ala
  1               5                  10

(2) INFORMATION FOR SEQ ID NO: 219:

     (i) SEQUENCE CHARACTERISTICS:
          (A) LENGTH: 449 base pairs
          (B) TYPE: NUCLEIC ACID
          (C) STRANDEDNESS: DOUBLE
          (D) TOPOLOGY: LINEAR

     (ii) MOLECULE TYPE: CDNA

     (vi) ORIGINAL SOURCE:
          (A) ORGANISM: Homo Sapiens
          (F) TISSUE TYPE: Muscle

     (ix) FEATURE:
          (A) NAME/KEY: other
          (B) LOCATION: 86..409
          (C) IDENTIFICATION METHOD: blastn
          (D) OTHER INFORMATION:  identity 97
                                  region 50..373
                                  id AA147010
                                  est

     (ix) FEATURE:
          (A) NAME/KEY: other
          (B) LOCATION: 132..450
          (C) IDENTIFICATION METHOD: blastn
          (D) OTHER INFORMATION:  identity 91
                                  region 156..474
                                  id AA142584
                                  est

     (ix) FEATURE:
          (A) NAME/KEY: other
          (B) LOCATION: 222..450
          (C) IDENTIFICATION METHOD: blastn
          (D) OTHER INFORMATION:  identity 99
                                  region 1..229
                                  id AA043641
                                  est

     (ix) FEATURE:
          (A) NAME/KEY: other
          (B) LOCATION: 101..304
          (C) IDENTIFICATION METHOD: blastn
          (D) OTHER INFORMATION:  identity 98
                                  region 72..275
                                  id T18932

est

(ix) FEATURE:
(A) NAME/KEY: other
(B) LOCATION: 132..243
(C) IDENTIFICATION METHOD: blastn
(D) OTHER INFORMATION: identity 90
region 146..257
id AA123074
est

(ix) FEATURE:
(A) NAME/KEY: sig_peptide
(B) LOCATION: 165..284
(C) IDENTIFICATION METHOD: Von Heijne matrix
(D) OTHER INFORMATION: score 6
seq LTYGIILTHGASG/DM

(xi) SEQUENCE DESCRIPTION: SEQ ID NO: 219:

AACGCTGTGG CGGGGCAGGC GAGGCGGTCG CTTCGAGCGC GCTAGTCAGC TCCCTGAAGG 60

GAGTGACGGC GGTTGGGTGC CCGCGGCCAC TTTTGCCTTC CCGGGGAGAT GTCCTTTGCT 120

TCTCAGATGT AAAKGCACTT TAAGTTTGKW ATTCAACAGT GAAA ATG AGT CAT ACA 176
Met Ser His Thr
-40

GAG GTT AAA TTA AAA ATA CCT TTT GGA AAT AAA TTA CTA GAT GCT GTT 224
Glu Val Lys Leu Lys Ile Pro Phe Gly Asn Lys Leu Leu Asp Ala Val
-35 -30 -25

TGT TTG GTA CCT AAC AAG AGC TTA ACA TAT GGA ATA ATT CTT ACA CAT 272
Cys Leu Val Pro Asn Lys Ser Leu Thr Tyr Gly Ile Ile Leu Thr His
-20 -15 -10 -5

GGA GCA TCA GGA GAT ATG AAT CTT CCT CAT TTG ATG TCA CTG GCA TCC 320
Gly Ala Ser Gly Asp Met Asn Leu Pro His Leu Met Ser Leu Ala Ser
1 5 10

CAT CTT GCA TCT CAT GGG TTT TTC TGC CTG AGA TTT ACC TGT AAA GGC 368
His Leu Ala Ser His Gly Phe Phe Cys Leu Arg Phe Thr Cys Lys Gly
15 20 25

CTT AAT ATT GTA CAT AGA ATT AAG GCG TAT AAA TCA GTT TTG AAT TAC 416
Leu Asn Ile Val His Arg Ile Lys Ala Tyr Lys Ser Val Leu Asn Tyr
30 35 40

CTG AAG ACA TCA GGM RAA TAC AAA CTT GCA GGT 449
Leu Lys Thr Ser Gly Xaa Tyr Lys Leu Ala Gly
45 50 55

(2) INFORMATION FOR SEQ ID NO: 220:

(i) SEQUENCE CHARACTERISTICS:
(A) LENGTH: 258 base pairs
(B) TYPE: NUCLEIC ACID
(C) STRANDEDNESS: DOUBLE

(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 75..254
(C) IDENTIFICATION METHOD: blastn
(D) OTHER INFORMATION: identity 97
region 1..180
id T31666
est

(ix) FEATURE:
(A) NAME/KEY: other
(B) LOCATION: 73..126
(C) IDENTIFICATION METHOD: blastn
(D) OTHER INFORMATION: identity 92
region 88..141
id R58665
est

(ix) FEATURE:
(A) NAME/KEY: other
(B) LOCATION: 23..77
(C) IDENTIFICATION METHOD: blastn
(D) OTHER INFORMATION: identity 90
region 39..93
id R58665
est

(ix) FEATURE:
(A) NAME/KEY: other
(B) LOCATION: 157..231
(C) IDENTIFICATION METHOD: blastn
(D) OTHER INFORMATION: identity 98
region 63..137
id R14990
est

(ix) FEATURE:
(A) NAME/KEY: other
(B) LOCATION: 95..144
(C) IDENTIFICATION METHOD: blastn
(D) OTHER INFORMATION: identity 94
region 1..50
id R14990
est

(ix) FEATURE:
(A) NAME/KEY: other
(B) LOCATION: 135..254
(C) IDENTIFICATION METHOD: blastn
(D) OTHER INFORMATION: identity 99
region 1..120
id T26956

est

(ix) FEATURE:
(A) NAME/KEY: sig_peptide
(B) LOCATION: 31..150
(C) IDENTIFICATION METHOD: Von Heijne matrix
(D) OTHER INFORMATION: score 6
seq LCXEFXSVASCDA/AV

(xi) SEQUENCE DESCRIPTION: SEQ ID NO: 220:

AAAAAGGGGC GGTGCAGAGG CGGCAGGAAG ATG GAG TTG GGG AGT TGC CTG GAG 54
Met Glu Leu Gly Ser Cys Leu Glu
-40 -35

GGC GGG AGG GAG GCG GCG GAG GAA GAG GGC GAG CCT GAG GTG AAA AAG 102
Gly Gly Arg Glu Ala Ala Glu Glu Glu Gly Glu Pro Glu Val Lys Lys
-30 -25 -20

CGG CGA CTT CTG TGT STR GAG TTT RCC TCG GTC GCA AGC TGC GAT GCC 150
Arg Arg Leu Leu Cys Xaa Glu Phe Xaa Ser Val Ala Ser Cys Asp Ala
-15 -10 -5

GCA GTG GCT CAG TGC TTC CTG GCC GAK AAC GAC TGG GAG ATG GAA AGG 198
Ala Val Ala Gln Cys Phe Leu Ala Xaa Asn Asp Trp Glu Met Glu Arg
1               5                   10                  15

GCT CTG AAC TCC TAC TTC GAG CCT CCG GTG GAG GAG AGC GCC TTG GAA 246
Ala Leu Asn Ser Tyr Phe Glu Pro Pro Val Glu Glu Ser Ala Leu Glu
            20                  25                  30

CGC CGA CCA DGG 258
Arg Arg Pro Xaa
        35

(2) INFORMATION FOR SEQ ID NO: 221:

(i) SEQUENCE CHARACTERISTICS:
(A) LENGTH: 318 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 138..317
(C) IDENTIFICATION METHOD: blastn
(D) OTHER INFORMATION: identity 98
region 52..231
id AA099777
est

(ix) FEATURE:
(A) NAME/KEY: other
(B) LOCATION: 85..135
(C) IDENTIFICATION METHOD: blastn
(D) OTHER INFORMATION: identity 94
region 1..51
id AA099777
est

(ix) FEATURE:
(A) NAME/KEY: other
(B) LOCATION: 138..222
(C) IDENTIFICATION METHOD: blastn
(D) OTHER INFORMATION: identity 95
region 83..167
id HSB16C031
est

(ix) FEATURE:
(A) NAME/KEY: other
(B) LOCATION: 80..135
(C) IDENTIFICATION METHOD: blastn
(D) OTHER INFORMATION: identity 94
region 27..82
id HSB16C031
est

(ix) FEATURE:
(A) NAME/KEY: other
(B) LOCATION: 145..314
(C) IDENTIFICATION METHOD: blastn
(D) OTHER INFORMATION: identity 91
region 43..212
id AA068028
est

(ix) FEATURE:
(A) NAME/KEY: sig_peptide
(B) LOCATION: 148..255
(C) IDENTIFICATION METHOD: Von Heijne matrix
(D) OTHER INFORMATION: score 5.8
seq AFVSGLLIGQCSS/QK

(xi) SEQUENCE DESCRIPTION: SEQ ID NO: 221:

AGGCAGTGAA TTGAGACCGG AGGGAATCTG GCCCCTAGAG GCTGGTACTT GGGCCCGAAA 60

CCCCCATCTC CGGCGGAGAG ACCGTCCGAG GTAATTGTCT GCCACGAGTG CACATTCTGA 120

AAACAGGRGR WTTTAAGKTT CCTAAAA ATG GGA AGA ACC TAC ATT GTA GAA GAG 174
Met Gly Arg Thr Tyr Ile Val Glu Glu
-35 -30

ACT GTT GGC CAG TAT CTT TCA AAC ATA AAT CTC CAA GGA AAG GCT TTT 222
Thr Val Gly Gln Tyr Leu Ser Asn Ile Asn Leu Gln Gly Lys Ala Phe
-25 -20 -15

GTC TCT GGC CTT TTA ATA GGA CAG TGT TCG TCA CAA AAG GAT TAT GTG 270
Val Ser Gly Leu Leu Ile Gly Gln Cys Ser Ser Gln Lys Asp Tyr Val

-10                    -5                    1                    5

ATT CTT GCC ACT AGA ACG CCA CCC AAA GAG GAG CAA AGT GAG AAC TTG    318
Ile Leu Ala Thr Arg Thr Pro Pro Lys Glu Glu Gln Ser Glu Asn Leu
                10                  15                  20

(2) INFORMATION FOR SEQ ID NO: 222:

(i) SEQUENCE CHARACTERISTICS:
(A) LENGTH: 474 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 227..433
(C) IDENTIFICATION METHOD: blastn
(D) OTHER INFORMATION:  identity 100
                        region 1..207
                        id R16604
                        est

(ix) FEATURE:
(A) NAME/KEY: other
(B) LOCATION: 432..474
(C) IDENTIFICATION METHOD: blastn
(D) OTHER INFORMATION:  identity 100
                        region 207..249
                        id R16604
                        est

(ix) FEATURE:
(A) NAME/KEY: other
(B) LOCATION: 227..440
(C) IDENTIFICATION METHOD: blastn
(D) OTHER INFORMATION:  identity 98
                        region 1..214
                        id N99558
                        est

(ix) FEATURE:
(A) NAME/KEY: sig_peptide
(B) LOCATION: 109..171
(C) IDENTIFICATION METHOD: Von Heijne matrix
(D) OTHER INFORMATION:  score 5.6
                        seq CLSCLLIPLALWS/II

(xi) SEQUENCE DESCRIPTION: SEQ ID NO: 222:

AGTATTTCAC ACTGAGATTG TCGGCTGCGG GTATATTCCA ATTCCCCGTC TCCTCATGAA    60

TATGAAGTGA AGGGCTCTGA CCCTGGAAGT GGTTCTAAGC AGGGCAAA ATG GGG TCT 117
Met Gly Ser
-20

CGG AAG TGT GGA GGC TGC CTA AGT TGT TTG CTG ATT CCG CTT GCA CTT 165
Arg Lys Cys Gly Gly Cys Leu Ser Cys Leu Leu Ile Pro Leu Ala Leu
-15 -10 -5

TGG AGT ATA ATC GTG AAC ATA TTA TTG TAT TTC CCG AAT GGG CAA ACT 213
Trp Ser Ile Ile Val Asn Ile Leu Leu Tyr Phe Pro Asn Gly Gln Thr
1 5 10

TCC TAT GCA TCC AGC AAT AAA CTC ACC AAC TAC GTG TGG TAT TTT GAA 261
Ser Tyr Ala Ser Ser Asn Lys Leu Thr Asn Tyr Val Trp Tyr Phe Glu
15 20 25 30

GGA ATC TGT TTC TCA GGC ATC ATG ATG CTT ATA GTA ACA ACA GTT CTT 309
Gly Ile Cys Phe Ser Gly Ile Met Met Leu Ile Val Thr Thr Val Leu
        35                  40                  45

CTG GTA CTG GAG AAT AAT AAC AAC TAT AAA TGT TGC CAG AGT GAA AAC 357
Leu Val Leu Glu Asn Asn Asn Asn Tyr Lys Cys Cys Gln Ser Glu Asn
    50                  55                  60

TGC AGC AAA AAA TAT GTG ACA CTG CTG TCA ATT ATC TTT TCT TCC CTC 405
Cys Ser Lys Lys Tyr Val Thr Leu Leu Ser Ile Ile Phe Ser Ser Leu
65 70 75

GGA ATT GCT TTT TCT GGA TAC TGC CTG GTC ATC TCT GCC TTG GGT CTT 453
Gly Ile Ala Phe Ser Gly Tyr Cys Leu Val Ile Ser Ala Leu Gly Leu
80 85 90

GTC CAA GGG CCA TAT TGC CGC 474
Val Gln Gly Pro Tyr Cys Arg
95 100

(2) INFORMATION FOR SEQ ID NO: 223:

(i) SEQUENCE CHARACTERISTICS:
(A) LENGTH: 459 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 128..341
(C) IDENTIFICATION METHOD: blastn
(D) OTHER INFORMATION: identity 98
region 1..214
id N99558

est

(ix) FEATURE:
(A) NAME/KEY: other
(B) LOCATION: 399..459
(C) IDENTIFICATION METHOD: blastn
(D) OTHER INFORMATION: identity 95
region 278..338
id N99558
est

(ix) FEATURE:
(A) NAME/KEY: other
(B) LOCATION: 359..407
(C) IDENTIFICATION METHOD: blastn
(D) OTHER INFORMATION: identity 93
region 237..285
id N99558
est

(ix) FEATURE:
(A) NAME/KEY: other
(B) LOCATION: 128..334
(C) IDENTIFICATION METHOD: blastn
(D) OTHER INFORMATION: identity 100
region 1..207
id R16604
est

(ix) FEATURE:
(A) NAME/KEY: other
(B) LOCATION: 333..386
(C) IDENTIFICATION METHOD: blastn
(D) OTHER INFORMATION: identity 100
region 207..260
id R16604
est

(ix) FEATURE:
(A) NAME/KEY: sig_peptide
(B) LOCATION: 10..72
(C) IDENTIFICATION METHOD: Von Heijne matrix
(D) OTHER INFORMATION: score 5.6
seq CLSCLLIPLALWS/II

(xi) SEQUENCE DESCRIPTION: SEQ ID NO: 223:

AAGGGCAAA ATG GGG TCT CGG AAG TGT GGA GGC TGC CTA AGT TGT TTG CTG 51
Met Gly Ser Arg Lys Cys Gly Gly Cys Leu Ser Cys Leu Leu
-20 -15 -10

ATT CCG CTT GCA CTT TGG AGT ATA ATC GTG AAC ATA TTA TTG TAT TTC 99
Ile Pro Leu Ala Leu Trp Ser Ile Ile Val Asn Ile Leu Leu Tyr Phe
-5 1 5

CCG AAT GGG CAA ACT TCC TAT GCA TCC AGC AAT AAA CTC ACC AAC TAC 147
Pro Asn Gly Gln Thr Ser Tyr Ala Ser Ser Asn Lys Leu Thr Asn Tyr
10 15 20 25

GTG TGG TAT TTT GAA GGA ATC TGT TTC TCA GGC ATC ATG ATG CTT ATA 195

Val Trp Tyr Phe Glu Gly Ile Cys Phe Ser Gly Ile Met Met Leu Ile
                30                  35                  40

GTA ACA ACA GTT CTT CTG GTA CTG GAG AAT AAT AAC AAC TAT AAA TGT     243
Val Thr Thr Val Leu Leu Val Leu Glu Asn Asn Asn Asn Tyr Lys Cys
            45                  50                  55

TGC CAG AGT GAA AAC TGC AGC AAA AAA TAT GTG ACA CTG CTG TCA ATT     291
Cys Gln Ser Glu Asn Cys Ser Lys Lys Tyr Val Thr Leu Leu Ser Ile
        60                  65                  70

ATC TTT TCT TCC CTC GGA ATT GCT TTT TCT GGA TAC TGC CTG GTC ATC     339
Ile Phe Ser Ser Leu Gly Ile Ala Phe Ser Gly Tyr Cys Leu Val Ile
    75                  80                  85

TCT GCC TTG GGT CTT GTC CAA GGG CCA TAT TGC CGC ACC CTT GAT GGC     387
Ser Ala Leu Gly Leu Val Gln Gly Pro Tyr Cys Arg Thr Leu Asp Gly
 90                  95                 100                 105

TGG GAG TAT GCT TTT GAA GGC ACT RCT GGA CGT TTC CTT ACA GAT TCT     435
Trp Glu Tyr Ala Phe Glu Gly Thr Xaa Gly Arg Phe Leu Thr Asp Ser
                110                 115                 120

AGC ATA TGG ATT CAG TGC CTG GAA                                     459
Ser Ile Trp Ile Gln Cys Leu Glu
            125

(2) INFORMATION FOR SEQ ID NO: 224:

    (i) SEQUENCE CHARACTERISTICS:
        (A) LENGTH: 453 base pairs
        (B) TYPE: NUCLEIC ACID
        (C) STRANDEDNESS: DOUBLE
        (D) TOPOLOGY: LINEAR

    (ii) MOLECULE TYPE: CDNA

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: other
        (B) LOCATION: 61..399
        (C) IDENTIFICATION METHOD: blastn
        (D) OTHER INFORMATION:  identity 98
                                region 6..344
                                id H09880
                                est

    (ix) FEATURE:
        (A) NAME/KEY: other
        (B) LOCATION: 408..454
        (C) IDENTIFICATION METHOD: blastn
        (D) OTHER INFORMATION:  identity 95
                                region 355..401
                                id H09880
                                est

(ix) FEATURE:
      (A) NAME/KEY: other
      (B) LOCATION: 60..399
      (C) IDENTIFICATION METHOD: blastn
      (D) OTHER INFORMATION:  identity 97
                              region 56..395
                              id H29351
                              est

(ix) FEATURE:
      (A) NAME/KEY: other
      (B) LOCATION: 393..432
      (C) IDENTIFICATION METHOD: blastn
      (D) OTHER INFORMATION:  identity 90
                              region 391..430
                              id H29351
                              est

(ix) FEATURE:
      (A) NAME/KEY: other
      (B) LOCATION: 65..369
      (C) IDENTIFICATION METHOD: blastn
      (D) OTHER INFORMATION:  identity 93
                              region 41..345
                              id H94779
                              est

(ix) FEATURE:
      (A) NAME/KEY: other
      (B) LOCATION: 118..455
      (C) IDENTIFICATION METHOD: blastn
      (D) OTHER INFORMATION:  identity 99
                              region 1..338
                              id N27248
                              est

(ix) FEATURE:
      (A) NAME/KEY: other
      (B) LOCATION: 122..399
      (C) IDENTIFICATION METHOD: blastn
      (D) OTHER INFORMATION:  identity 98
                              region 1..278
                              id T74091
                              est

(ix) FEATURE:
      (A) NAME/KEY: other
      (B) LOCATION: 393..434
      (C) IDENTIFICATION METHOD: blastn
      (D) OTHER INFORMATION:  identity 95
                              region 273..314
                              id T74091
                              est

(ix) FEATURE:
      (A) NAME/KEY: sig_peptide
      (B) LOCATION: 346..408
      (C) IDENTIFICATION METHOD: Von Heijne matrix
      (D) OTHER INFORMATION:  score 5.5
                              seq SFLPSALVIWTSA/AF

(xi) SEQUENCE DESCRIPTION: SEQ ID NO: 224:

ACTCCTTTTA GCATAGGGGC TTCGGCGCCA GCGGCCAGCG CTAGTCGGTC TGGTAAGTGC 60

CTGATGCCGA GTTCCGTCTC TCGCGTCTTT TCCTGGTCCC AGGCAAAGCG GASGNAGATC 120

CTCAAACGGC CTAGTGCTTC GCGCTTCCGG AGAAAATCAG CGGTCTAATT AATTCCTCTG 180

GTTTGTTGAA GCAGTTACCA AGAATCTTCA ACCCTTTCCC ACAAAAGCTA ATTGAGTACA 240

CGTTCCTGTT GAGTACACGT TCCTGTTGAT TTACAAAAGG TGCAGGTATG AGCAGGTCTG 300

AAGACTAACA TTTTGTGAAG TTGTAAAACA GAAAACCTGT TAGAA ATG TGG TGG TTT 357
Met Trp Trp Phe
-20

CAG CAA GGC CTC AGT TTC CTT CCT TCA GCC CTT GTA ATT TGG ACA TCT 405
Gln Gln Gly Leu Ser Phe Leu Pro Ser Ala Leu Val Ile Trp Thr Ser
-15 -10 -5

GCT GCT TTC ATA TTT TCA TAC ATT ACT GCA GTA ACA CTC CAC CAT ATA 453
Ala Ala Phe Ile Phe Ser Tyr Ile Thr Ala Val Thr Leu His His Ile
1               5                   10                  15

(2) INFORMATION FOR SEQ ID NO: 225:

(i) SEQUENCE CHARACTERISTICS:
(A) LENGTH: 282 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 11..277
(C) IDENTIFICATION METHOD: blastn
(D) OTHER INFORMATION: identity 92
region 29..295
id AA041777
est

(ix) FEATURE:
(A) NAME/KEY: other
(B) LOCATION: 56..277
(C) IDENTIFICATION METHOD: blastn
(D) OTHER INFORMATION: identity 99
region 1..222
id HSC1QB111
est

(ix) FEATURE:
(A) NAME/KEY: other
(B) LOCATION: 135..281
(C) IDENTIFICATION METHOD: blastn
(D) OTHER INFORMATION: identity 97
region 56..202
id H10738
est

(ix) FEATURE:
(A) NAME/KEY: other
(B) LOCATION: 81..133
(C) IDENTIFICATION METHOD: blastn
(D) OTHER INFORMATION: identity 100
region 1..53
id H10738
est

(ix) FEATURE:
(A) NAME/KEY: other
(B) LOCATION: 75..277
(C) IDENTIFICATION METHOD: blastn
(D) OTHER INFORMATION: identity 97
region 6..208
id HSC2KE111
est

(ix) FEATURE:
(A) NAME/KEY: other
(B) LOCATION: 89..263
(C) IDENTIFICATION METHOD: blastn
(D) OTHER INFORMATION: identity 98
region 2..176
id W24981
est

(ix) FEATURE:
(A) NAME/KEY: sig_peptide
(B) LOCATION: 106..228
(C) IDENTIFICATION METHOD: Von Heijne matrix
(D) OTHER INFORMATION: score 5.4
seq PLIFSLWCSGVLL/HI

(xi) SEQUENCE DESCRIPTION: SEQ ID NO: 225:
AAGAGTGCGC GGRSATTGGG GCTTTCCAGC TCTCACAGAA CCTTCAGCAT CCCCAGCTGC 60
CGGTCTTGGC ATCTTCGAAG TAAGGAGAGT TTTAGATGCT TCTGG ATG TTC AAT GCT 117
AGC ACC TTT ACA GAC TGG AGC AGC TCG ATT TTC TTC GTA TTT ACT TTC 165
AAG AGC AAG AAA AGT GCT GGG CTC CCA CTT ATT TTC TCC CTG TGG TGT 213
TCC GGA GTT CTG CTC CAT ATC CAC CAG AAA GCT GGC GGC CCA CGG CTT 261
TGG CGC ATC CAT GGC GAG CAG                                          282

(2) INFORMATION FOR SEQ ID NO: 226:

      (i) SEQUENCE CHARACTERISTICS:
            (A) LENGTH: 332 base pairs
            (B) TYPE: NUCLEIC ACID
            (C) STRANDEDNESS: DOUBLE
            (D) TOPOLOGY: LINEAR

      (ii) MOLECULE TYPE: CDNA

      (vi) ORIGINAL SOURCE:
            (A) ORGANISM: Homo Sapiens
            (D) DEVELOPMENTAL STAGE: Fetal
            (F) TISSUE TYPE: kidney

      (ix) FEATURE:
            (A) NAME/KEY: other
            (B) LOCATION: 155..334
            (C) IDENTIFICATION METHOD: fasta
            (D) OTHER INFORMATION:  identity 98.3
                                    region 1..181
                                    id HSU90144
                                    vrt

      (ix) FEATURE:
            (A) NAME/KEY: other
            (B) LOCATION: 218..328
            (C) IDENTIFICATION METHOD: blastn
            (D) OTHER INFORMATION:  identity 100
                                    region 90..200
                                    id T70246
                                    est

      (ix) FEATURE:
            (A) NAME/KEY: other
            (B) LOCATION: 128..216
            (C) IDENTIFICATION METHOD: blastn
            (D) OTHER INFORMATION:  identity 97
                                    region 1..89
                                    id T70246
                                    est

      (ix) FEATURE:
            (A) NAME/KEY: other
            (B) LOCATION: 170..328
            (C) IDENTIFICATION METHOD: blastn
            (D) OTHER INFORMATION:  identity 96
                                    region 50..208
                                    id T70127
                                    est

      (ix) FEATURE:

(A) NAME/KEY: other
(B) LOCATION: 219..328
(C) IDENTIFICATION METHOD: blastn
(D) OTHER INFORMATION: identity 100
region 62..171
id AA114263
est

(ix) FEATURE:
(A) NAME/KEY: other
(B) LOCATION: 159..218
(C) IDENTIFICATION METHOD: blastn
(D) OTHER INFORMATION: identity 96
region 1..60
id AA114263
est

(ix) FEATURE:
(A) NAME/KEY: sig_peptide
(B) LOCATION: 222..308
(C) IDENTIFICATION METHOD: Von Heijne matrix
(D) OTHER INFORMATION: score 13.4
seq SLLLVQLLTPCSA/QF

(xi) SEQUENCE DESCRIPTION: SEQ ID NO: 226:

GACTCTTACT GTTTCTCATG GTGAGAAGAC AATATTTGCT TTCTCTTTTT CCTTTCTTCC 60

GGATGAGAGG NTAAGCCATA ATAGAAAGAA TGGAGAATTA TTGATTGACC GTCTTTATTC 120

TGTGGGCTCT GATTCTCCAA TGGGAATACC AAGGGATGGT TTTCCATACT GGAACCCWWA 180

GGTAAAGACA CTCAAGGACA GACATTTTTG GCAGAGCATA G ATG AAA ATG GCA AGT 236
Met Lys Met Ala Ser
-25

TCC CTG GCT TTC CTT CTG CTC AAC TTT CAT GTC TCC CTC CTC TTG GTC 284
Ser Leu Ala Phe Leu Leu Leu Asn Phe His Val Ser Leu Leu Leu Val
-20 -15 -10

CAG CTG CTC ACT CCT TGC TCA GCT CAG TTT TCT GTG CTT GGA CCT CTG 332
Gln Leu Leu Thr Pro Cys Ser Ala Gln Phe Ser Val Leu Gly Pro Leu
-5 1 5

(2) INFORMATION FOR SEQ ID NO: 227:

(i) SEQUENCE CHARACTERISTICS:
(A) LENGTH: 414 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 182..411
(C) IDENTIFICATION METHOD: blastn
(D) OTHER INFORMATION: identity 99
region 1..230
id C15003
est

(ix) FEATURE:
(A) NAME/KEY: other
(B) LOCATION: 182..411
(C) IDENTIFICATION METHOD: blastn
(D) OTHER INFORMATION: identity 99
region 1..230
id HUM407E11B
est

(ix) FEATURE:
(A) NAME/KEY: other
(B) LOCATION: 182..369
(C) IDENTIFICATION METHOD: blastn
(D) OTHER INFORMATION: identity 100
region 1..188
id C15677
est

(ix) FEATURE:
(A) NAME/KEY: other
(B) LOCATION: 212..369
(C) IDENTIFICATION METHOD: blastn
(D) OTHER INFORMATION: identity 99
region 26..183
id HUM169E08B
est

(ix) FEATURE:
(A) NAME/KEY: sig_peptide
(B) LOCATION: 274..399
(C) IDENTIFICATION METHOD: Von Heijne matrix
(D) OTHER INFORMATION: score 5.2
seq LLFDLVCHEFCQS/DD

(xi) SEQUENCE DESCRIPTION: SEQ ID NO: 227:

ACCAGGAACA TCCAGCTATT TATGATAGCA TTTGCTTCAT TATGTCAAGT TCAACAAATG 60

TTGACTTGCT GGTGAAGGTG GGGGAGGTTG TGGACAAGCT CTTTGATTTG GATGAGAAAC 120

TAATGTTAAG AATGGGTCAG AAATGGGGCT GCTCAGCCTC TGGACCAACC CCAGGAAGAG 180

TCTGAAGAGC AGCCAGTGTT TCGGCTTGTG CCCTGTATAC TTGAAGCTGC CAAACAAGTA 240

CGTTCTGAAA ATCCAGAATG GCTTGATGTT TAC ATG CAC ATT TTA CAA CTG CTT 294
Met His Ile Leu Gln Leu Leu
-40

ACT ACA GTG GAT GAT GGA ATT CAA GCA ATT GTA CAT TGT CCT GAC ACT 342
Thr Thr Val Asp Asp Gly Ile Gln Ala Ile Val His Cys Pro Asp Thr

-35                    -30                    -25                    -20

GGA AAA GAC ATT TGG AAT TTA CTT TTT GAC CTG GTC TGC CAT GAA TTC     390
Gly Lys Asp Ile Trp Asn Leu Leu Phe Asp Leu Val Cys His Glu Phe
                -15                 -10                 -5

TGC CAG TCT GAT GAT CCA GCC CGG                                     414
Cys Gln Ser Asp Asp Pro Ala Arg
            1               5

(2) INFORMATION FOR SEQ ID NO: 228:

      (i) SEQUENCE CHARACTERISTICS:
           (A) LENGTH: 419 base pairs
           (B) TYPE: NUCLEIC ACID
           (C) STRANDEDNESS: DOUBLE
           (D) TOPOLOGY: LINEAR

      (ii) MOLECULE TYPE: CDNA

      (vi) ORIGINAL SOURCE:
           (A) ORGANISM: Homo Sapiens
           (D) DEVELOPMENTAL STAGE: Fetal
           (F) TISSUE TYPE: kidney

      (ix) FEATURE:
           (A) NAME/KEY: other
           (B) LOCATION: 66..96
           (C) IDENTIFICATION METHOD: blastn
           (D) OTHER INFORMATION:  identity 96
                                   region 1..31
                                   id AA017364
                                   est

      (ix) FEATURE:
           (A) NAME/KEY: sig_peptide
           (B) LOCATION: 114..242
           (C) IDENTIFICATION METHOD: Von Heijne matrix
           (D) OTHER INFORMATION:  score 5.2
                                   seq PMQLLQVLSDVLA/EI

      (xi) SEQUENCE DESCRIPTION: SEQ ID NO: 228:

AAACCGTTGC CAAGGAGCTC GACTCTGGGA GCGGTCTAGA GCCCGGGCGC CTCCTGGGGG    60

GTGGGGAAAC GGTTTCGTGA GGAGAATTTG AGTTAAAATT ATAAGACCTA ATT ATG      116
                                                           Met

AGT GAT CAA ATT AAA TTC ATT ATG GAC AGT CTC AAT AAG GAG CCC TTT     164
Ser Asp Gln Ile Lys Phe Ile Met Asp Ser Leu Asn Lys Glu Pro Phe
        -40                 -35                 -30

AGG AAG AAC TAT AAT TTA ATC ACG TTT GWT TCC TTG GAG CCA ATG CAA     212
Arg Lys Asn Tyr Asn Leu Ile Thr Phe Xaa Ser Leu Glu Pro Met Gln
    -25                 -20                 -15

CTA TTA CAA GTT CTC AGT GAT GTT CTG GCT GAG ATT GAC CCA AAG CAA     260
Leu Leu Gln Val Leu Ser Asp Val Leu Ala Glu Ile Asp Pro Lys Gln

-10                    -5                   1                5

CTT GTG GAT ATC AGA GAG GAG ATG CCA GAG CAG ACA GCC AAA CGA ATG     308
Leu Val Asp Ile Arg Glu Glu Met Pro Glu Gln Thr Ala Lys Arg Met
            10                  15                  20

TTG AGC CTT CTT GGT ATT CTT AAG TAC AAA CCT TCA GGA AAT GCC ACA     356
Leu Ser Leu Leu Gly Ile Leu Lys Tyr Lys Pro Ser Gly Asn Ala Thr
        25                  30                  35

GAT ATG AGT ACT TTT CGT CAG GGT TTG GTG ATT GGA AGT AAA CCT GTA     404
Asp Met Ser Thr Phe Arg Gln Gly Leu Val Ile Gly Ser Lys Pro Val
    40                  45                  50

ATT TAC CCA GTG CTC                                                 419
Ile Tyr Pro Val Leu
 55

(2) INFORMATION FOR SEQ ID NO: 229:

      (i) SEQUENCE CHARACTERISTICS:
           (A) LENGTH: 371 base pairs
           (B) TYPE: NUCLEIC ACID
           (C) STRANDEDNESS: DOUBLE
           (D) TOPOLOGY: LINEAR

      (ii) MOLECULE TYPE: CDNA

      (vi) ORIGINAL SOURCE:
           (A) ORGANISM: Homo Sapiens
           (D) DEVELOPMENTAL STAGE: Fetal
           (F) TISSUE TYPE: kidney

      (ix) FEATURE:
           (A) NAME/KEY: other
           (B) LOCATION: 53..203
           (C) IDENTIFICATION METHOD: blastn
           (D) OTHER INFORMATION:  identity 96
                                   region 1..151
                                   id T34361
                                   est

      (ix) FEATURE:
           (A) NAME/KEY: other
           (B) LOCATION: 205..358
           (C) IDENTIFICATION METHOD: blastn
           (D) OTHER INFORMATION:  identity 93
                                   region 152..305
                                   id T34361
                                   est

      (ix) FEATURE:
           (A) NAME/KEY: other
           (B) LOCATION: 205..342
           (C) IDENTIFICATION METHOD: blastn
           (D) OTHER INFORMATION:  identity 94
                                   region 131..268
                                   id HSC16A051
                                   est

(ix) FEATURE:
(A) NAME/KEY: other
(B) LOCATION: 74..203
(C) IDENTIFICATION METHOD: blastn
(D) OTHER INFORMATION: identity 94
region 1..130
id HSC16A051
est

(ix) FEATURE:
(A) NAME/KEY: other
(B) LOCATION: 340..373
(C) IDENTIFICATION METHOD: blastn
(D) OTHER INFORMATION: identity 94
region 267..300
id HSC16A051
est

(ix) FEATURE:
(A) NAME/KEY: other
(B) LOCATION: 61..256
(C) IDENTIFICATION METHOD: blastn
(D) OTHER INFORMATION: identity 95
region 41..236
id T35252
est

(ix) FEATURE:
(A) NAME/KEY: other
(B) LOCATION: 255..302
(C) IDENTIFICATION METHOD: blastn
(D) OTHER INFORMATION: identity 93
region 236..283
id T35252
est

(ix) FEATURE:
(A) NAME/KEY: other
(B) LOCATION: 60..146
(C) IDENTIFICATION METHOD: blastn
(D) OTHER INFORMATION: identity 94
region 57..143
id H92421
est

(ix) FEATURE:
(A) NAME/KEY: other
(B) LOCATION: 205..278
(C) IDENTIFICATION METHOD: blastn
(D) OTHER INFORMATION: identity 95
region 200..273
id H92421
est

(ix) FEATURE:
(A) NAME/KEY: other
(B) LOCATION: 61..203
(C) IDENTIFICATION METHOD: blastn
(D) OTHER INFORMATION: identity 95
region 85..227

id T19059
est

(ix) FEATURE:
(A) NAME/KEY: other
(B) LOCATION: 205..270
(C) IDENTIFICATION METHOD: blastn
(D) OTHER INFORMATION: identity 93
region 228..293
id T19059
est

(ix) FEATURE:
(A) NAME/KEY: sig_peptide
(B) LOCATION: 93..329
(C) IDENTIFICATION METHOD: Von Heijne matrix
(D) OTHER INFORMATION: score 4.6
seq IIHAXGLVRECLA/XT

(xi) SEQUENCE DESCRIPTION: SEQ ID NO: 229:

AAGACCTGGG CGTCTGGAAT GATCTACGTG CTTAAATACA CCACTCGCCA CCATTTTCTC 60

CAGCTGGGAG TGTCCACTCG CCTTCCACCA GC ATG GCA ACG TCK TCA CAG KAC 113
Met Ala Thr Ser Ser Gln Xaa
-75

CGC CAG CTG CTC AGT GAC TAC GGG CCA CCG TCC CTA GGC TAC ACC CAG 161
Arg Gln Leu Leu Ser Asp Tyr Gly Pro Pro Ser Leu Gly Tyr Thr Gln
-70 -65 -60

GGA ACT GGG AAC AGC CAR RTG CCC CAA AGC AAA TAC GCG GAG CTG CTG 209
Gly Thr Gly Asn Ser Gln Xaa Pro Gln Ser Lys Tyr Ala Glu Leu Leu
-55 -50 -45

GCC ATC ATT GRA GAG CTG GGG AAG GAG ATC AGA CCC ATG TAC GCA GGG 257
Ala Ile Ile Xaa Glu Leu Gly Lys Glu Ile Arg Pro Met Tyr Ala Gly
-40 -35 -30 -25

AGC AAG AGT GCC ATG GAG AGG CTG AAG CGC GGC ATC ATT CAC GCT MSA 305
Ser Lys Ser Ala Met Glu Arg Leu Lys Arg Gly Ile Ile His Ala Xaa
-20 -15 -10

GGM CTR GTT CGG GAG TGC TTG GCA GAM ACG GAA CGA ATG CCA GAT CCT 353
Gly Leu Val Arg Glu Cys Leu Ala Xaa Thr Glu Arg Met Pro Asp Pro
-5 1 5

AGC TGC CTT GTT GGT TTT 371
Ser Cys Leu Val Gly Phe
10

(2) INFORMATION FOR SEQ ID NO: 230:

(i) SEQUENCE CHARACTERISTICS:
(A) LENGTH: 235 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 107..234
(C) IDENTIFICATION METHOD: blastn
(D) OTHER INFORMATION: identity 100
region 1..128
id N88564
est

(ix) FEATURE:
(A) NAME/KEY: sig_peptide
(B) LOCATION: 59..103
(C) IDENTIFICATION METHOD: Von Heijne matrix
(D) OTHER INFORMATION: score 4.5
seq LLGAAAVAALGRG/RA

(xi) SEQUENCE DESCRIPTION: SEQ ID NO: 230:

AACCGGCAGC TGAACCCACC CGGCGCCACG GGACTTTGAC GCGTGCTCTG CGCTTGCC 58

ATG AGA CTC CTG GGA GCT GCA GCC GTC GCG GCT CTG GGG CGC GGA AGG 106
Met Arg Leu Leu Gly Ala Ala Ala Val Ala Ala Leu Gly Arg Gly Arg
-15 -10 -5 1

GCC CCC GCC TCC CTA GGC TGG CAG AGG AAG CAG GTT AAT TGG AAG GCC 154
Ala Pro Ala Ser Leu Gly Trp Gln Arg Lys Gln Val Asn Trp Lys Ala
                5                   10                  15

TGC CGA TGG TCT TCA TCA GGG GTG ATT CCT AAT GAA AAA ATA CGA AAT 202
Cys Arg Trp Ser Ser Ser Gly Val Ile Pro Asn Glu Lys Ile Arg Asn
            20                  25                  30

ATT GGA ATC TCA GCT CAC ATT GAT TCT GGG AAG 235
Ile Gly Ile Ser Ala His Ile Asp Ser Gly Lys
        35                  40

(2) INFORMATION FOR SEQ ID NO: 231:

(i) SEQUENCE CHARACTERISTICS:
(A) LENGTH: 165 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 13..162
(C) IDENTIFICATION METHOD: blastn
(D) OTHER INFORMATION: identity 95
region 20..169
id N41898
est

(ix) FEATURE:
(A) NAME/KEY: other
(B) LOCATION: 26..162
(C) IDENTIFICATION METHOD: blastn
(D) OTHER INFORMATION: identity 95
region 38..174
id H69272
est

(ix) FEATURE:
(A) NAME/KEY: other
(B) LOCATION: 45..162
(C) IDENTIFICATION METHOD: blastn
(D) OTHER INFORMATION: identity 97
region 1..118
id N20619
est

(ix) FEATURE:
(A) NAME/KEY: sig_peptide
(B) LOCATION: 13..60
(C) IDENTIFICATION METHOD: Von Heijne matrix
(D) OTHER INFORMATION: score 4.5
seq RLLLRRFLASVIS/RK

(xi) SEQUENCE DESCRIPTION: SEQ ID NO: 231:

AATTGCAGGG AG ATG GCT CAG CGA CTT CTT CTG AGG AGG TTC CTG GCC TCT 51
Met Ala Gln Arg Leu Leu Leu Arg Arg Phe Leu Ala Ser
-15 -10 -5

GTC ATC TCC AGG AAG CCC TCT CAG GGT CAG TGG CCA CCC CTC ACT TCC 99
Val Ile Ser Arg Lys Pro Ser Gln Gly Gln Trp Pro Pro Leu Thr Ser
1 5 10

AGA GCC CTG CAG ACC CCA CAA TGC AGT CCT GGT GGC CTG ACT GTA ACA 147
Arg Ala Leu Gln Thr Pro Gln Cys Ser Pro Gly Gly Leu Thr Val Thr
15 20 25

CCC AAC CCA GCG CCG GGG 165
Pro Asn Pro Ala Pro Gly
30 35

(2) INFORMATION FOR SEQ ID NO: 232:

(i) SEQUENCE CHARACTERISTICS:
(A) LENGTH: 217 base pairs
(B) TYPE: NUCLEIC ACID

(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 59..214
(C) IDENTIFICATION METHOD: blastn
(D) OTHER INFORMATION: identity 98
region 1..156
id AA069390
est

(ix) FEATURE:
(A) NAME/KEY: sig_peptide
(B) LOCATION: 122..169
(C) IDENTIFICATION METHOD: Von Heijne matrix
(D) OTHER INFORMATION: score 4.4
seq LNSLSALAELAVG/SR

(xi) SEQUENCE DESCRIPTION: SEQ ID NO: 232:

AAGGAGAGTC ACGTGAGAGT GGGCGGAGGG GGTGGAGGTT TGTCTCCGCT GTTTCATCTC 60

TATGGCTGTC AGAGGTGGGC GGCTTTGACC GAGAGGCTGC TGGAGCTCGT GTTTGGACGC 120

G ATG TTT CGT CTG AAC TCA CTT TCT GCT TTG GCA GAA CTG GCT GTG GGT 169
Met Phe Arg Leu Asn Ser Leu Ser Ala Leu Ala Glu Leu Ala Val Gly
-15 -10 -5

TCT CGA TGG TAC CAT GGA GGA TCA CAG CCC ATC CAG ATC CGG CTA GCG 217
Ser Arg Trp Tyr His Gly Gly Ser Gln Pro Ile Gln Ile Arg Leu Ala
1               5                   10                  15

(2) INFORMATION FOR SEQ ID NO: 233:

(i) SEQUENCE CHARACTERISTICS:
(A) LENGTH: 358 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: other
(B) LOCATION: 44..169
(C) IDENTIFICATION METHOD: blastn

(D) OTHER INFORMATION:  identity 100
                           region 1..126
                           id AA094226
                           est

(ix) FEATURE:
     (A) NAME/KEY: other
     (B) LOCATION: 170..231
     (C) IDENTIFICATION METHOD: blastn
     (D) OTHER INFORMATION:  identity 100
                             region 126..187
                             id AA094226
                             est

(ix) FEATURE:
     (A) NAME/KEY: other
     (B) LOCATION: 230..261
     (C) IDENTIFICATION METHOD: blastn
     (D) OTHER INFORMATION:  identity 96
                             region 185..216
                             id AA094226
                             est

(ix) FEATURE:
     (A) NAME/KEY: other
     (B) LOCATION: 44..195
     (C) IDENTIFICATION METHOD: blastn
     (D) OTHER INFORMATION:  identity 100
                             region 129..280
                             id R13710
                             est

(ix) FEATURE:
     (A) NAME/KEY: other
     (B) LOCATION: 193..254
     (C) IDENTIFICATION METHOD: blastn
     (D) OTHER INFORMATION:  identity 98
                             region 279..340
                             id R13710
                             est

(ix) FEATURE:
     (A) NAME/KEY: other
     (B) LOCATION: 44..282
     (C) IDENTIFICATION METHOD: blastn
     (D) OTHER INFORMATION:  identity 97
                             region 172..410
                             id R54574
                             est

(ix) FEATURE:
     (A) NAME/KEY: other
     (B) LOCATION: 44..184
     (C) IDENTIFICATION METHOD: blastn
     (D) OTHER INFORMATION:  identity 100
                             region 159..299
                             id T78111
                             est

(ix) FEATURE:
     (A) NAME/KEY: other

(B) LOCATION: 182..222
(C) IDENTIFICATION METHOD: blastn
(D) OTHER INFORMATION: identity 92
region 298..338
id T78111
est

(ix) FEATURE:
(A) NAME/KEY: other
(B) LOCATION: 220..254
(C) IDENTIFICATION METHOD: blastn
(D) OTHER INFORMATION: identity 97
region 337..371
id T78111
est

(ix) FEATURE:
(A) NAME/KEY: sig_peptide
(B) LOCATION: 89..271
(C) IDENTIFICATION METHOD: Von Heijne matrix
(D) OTHER INFORMATION: score 3.9
seq YTAVSVLAGPRWA/DP

(xi) SEQUENCE DESCRIPTION: SEQ ID NO: 233:

GCGGCGCGGC CGTAAAGCGC CATTACGCAG AGAGAAAGTT ACGAGAAACT CGTTTTCATC 60

TTCTTGGTTT CATCTTAATA CCAACGTC ATG TCT GGT TCT AAT GGT TCC AAA 112
Met Ser Gly Ser Asn Gly Ser Lys
-60 -55

GAA AAT TCT CAC AAT AAG GCT CGG ACG TCT CCT TAC CCA GGT TCA AAA 160
Glu Asn Ser His Asn Lys Ala Arg Thr Ser Pro Tyr Pro Gly Ser Lys
-50 -45 -40

GTT GAA CGA AGC CAG GTT CCT AAT GAG AAA GTG GGC TGG CTT GTT GAG 208
Val Glu Arg Ser Gln Val Pro Asn Glu Lys Val Gly Trp Leu Val Glu
-35 -30 -25

TGG CAA GAC TAT AAG CCT GTG GAA TAC ACT GCA GTC TCT GTC TTG GCT 256
Trp Gln Asp Tyr Lys Pro Val Glu Tyr Thr Ala Val Ser Val Leu Ala
-20 -15 -10

GGA CCC AGG TGG GCA GAT CCT CAG ATC AGT GAA AGT AAT TTT TCT CCC 304
Gly Pro Arg Trp Ala Asp Pro Gln Ile Ser Glu Ser Asn Phe Ser Pro
-5 1 5 10

AAG TTT AAC GAA AAG GAT GGG CAT GTT GAG AGA AAG AGC AAG AAT GGC 352
Lys Phe Asn Glu Lys Asp Gly His Val Glu Arg Lys Ser Lys Asn Gly
15 20 25

CTG TAT 358
Leu Tyr

(2) INFORMATION FOR SEQ ID NO: 234:

(i) SEQUENCE CHARACTERISTICS:
(A) LENGTH: 346 base pairs

(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: other
(B) LOCATION: 294..347
(C) IDENTIFICATION METHOD: blastn
(D) OTHER INFORMATION: identity 94
region 297..350
id AA038489
est

(ix) FEATURE:
(A) NAME/KEY: other
(B) LOCATION: 134..347
(C) IDENTIFICATION METHOD: blastn
(D) OTHER INFORMATION: identity 99
region 1..214
id AA111922
est

(ix) FEATURE:
(A) NAME/KEY: sig_peptide
(B) LOCATION: 284..331
(C) IDENTIFICATION METHOD: Von Heijne matrix
(D) OTHER INFORMATION: score 3.5
seq TLMFSLTAQWXTS/RS

(xi) SEQUENCE DESCRIPTION: SEQ ID NO: 234:

AAAAAAAAGC TGCTGGACCC CAGGGAGAGC TGACCACTGC CCGAGCAGCC GGCTGAATCC 60

ACCTCCACAA TGCSGCTCTC AGGAACCCCG GYCCCTAATA AGAAGAGGAA ATCCAGCAAG 120

CTGATCATGG AACTCACTGG AGGTGGACAG GAGAGCTCAG GCTTGAACCT GGGCAAAAAG 180

ATCAGTGTCC CAAGGGATGT GATGTTGGAG GAACTGTCGC TGCTTACCAA CCGGGGCTCC 240

AAGATGTTCA AACTGSGGCA GATGAGGGTG GAGAAGTTTA TTT ATG AGA ACC ACC 295
Met Arg Thr Thr
-15

CTG ATG TTT TCT CTG ACA GCT CAA TGG WTC ACT TCC AGA AGT TCC TTC 343
Leu Met Phe Ser Leu Thr Ala Gln Trp Xaa Thr Ser Arg Ser Ser Phe
-10 -5 1

CAA 346
Gln
5

(2) INFORMATION FOR SEQ ID NO: 235:

(i) SEQUENCE CHARACTERISTICS:
(A) LENGTH: 384 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 35..384
(C) IDENTIFICATION METHOD: blastn
(D) OTHER INFORMATION: identity 98
region 8..357
id H11129
est

(ix) FEATURE:
(A) NAME/KEY: other
(B) LOCATION: 43..346
(C) IDENTIFICATION METHOD: blastn
(D) OTHER INFORMATION: identity 99
region 16..319
id R11829
est

(ix) FEATURE:
(A) NAME/KEY: other
(B) LOCATION: 50..302
(C) IDENTIFICATION METHOD: blastn
(D) OTHER INFORMATION: identity 99
region 1..253
id R18811
est

(ix) FEATURE:
(A) NAME/KEY: other
(B) LOCATION: 302..366
(C) IDENTIFICATION METHOD: blastn
(D) OTHER INFORMATION: identity 96
region 254..318
id R18811
est

(ix) FEATURE:
(A) NAME/KEY: other
(B) LOCATION: 183..371
(C) IDENTIFICATION METHOD: blastn
(D) OTHER INFORMATION: identity 96
region 6..194
id R10511
est

(ix) FEATURE:
(A) NAME/KEY: sig_peptide

(B) LOCATION: 73..147
(C) IDENTIFICATION METHOD: Von Heijne matrix
(D) OTHER INFORMATION: score 14.1
seq LTLLLLLTLLAFA/GY

(xi) SEQUENCE DESCRIPTION: SEQ ID NO: 235:

ACTGCGCGGA TCGGCGTCCG CAGCGGGCGG CTGCTGAGCT GCCTTGAGGT GCAGTGTTGG 60

GGATCCAGAG CC ATG TCG GAC CTG CTA CTA CTG GGC CTG ATT GGG GGC CTG 111
Met Ser Asp Leu Leu Leu Leu Gly Leu Ile Gly Gly Leu
-25 -20 -15

ACT CTC TTA CTG CTG CTG ACG CTG CTG GCC TTT GCC GGG TAC TCA GGG 159
Thr Leu Leu Leu Leu Leu Thr Leu Leu Ala Phe Ala Gly Tyr Ser Gly
-10 -5 1

CTA CTG GCT GGG GTG GAA GTG AGT GCT GGG TCA CCC CCC ATC CGC AAC 207
Leu Leu Ala Gly Val Glu Val Ser Ala Gly Ser Pro Pro Ile Arg Asn
5 10 15 20

GTC ACT GTG GCC TAC AAG TTC CAC ATG GGG CTC TAT GGT GAG ACT GGG 255
Val Thr Val Ala Tyr Lys Phe His Met Gly Leu Tyr Gly Glu Thr Gly
25 30 35

CGG CTT TTC ACT GAG AGC TGC AGC ATC TCT CCC AAG CTC CGC TCC ATC 303
Arg Leu Phe Thr Glu Ser Cys Ser Ile Ser Pro Lys Leu Arg Ser Ile
40 45 50

GCT GTC TAC TAT GAC AAC CCC CAC ATG GTG CCC CCT GAT AAG TGC CGA 351
Ala Val Tyr Tyr Asp Asn Pro His Met Val Pro Pro Asp Lys Cys Arg
55 60 65

TGT GCC GTG GGC AGC ATC CTG AGT GAA GGT GAG 384
Cys Ala Val Gly Ser Ile Leu Ser Glu Gly Glu
70 75

(2) INFORMATION FOR SEQ ID NO: 236:

(i) SEQUENCE CHARACTERISTICS:
(A) LENGTH: 269 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: other
(B) LOCATION: 75..213
(C) IDENTIFICATION METHOD: blastn
(D) OTHER INFORMATION: identity 97
region 29..172
id T64530

est

(ix) FEATURE:
(A) NAME/KEY: sig_peptide
(B) LOCATION: 36..131
(C) IDENTIFICATION METHOD: Von Heijne matrix
(D) OTHER INFORMATION: score 11.4
seq LWSLALWLPLALS/VS

(xi) SEQUENCE DESCRIPTION: SEQ ID NO: 236:

AATCCGGACT GATAACCAGC CGGCCAGACT GAGGG ATG GAA GGC ACT GAG ATG 53
Met Glu Gly Thr Glu Met
-30

GGG GCC CGT CCA GGC GGA CAC CCG CRG AAA TGG AGC TTT CTG TGG TCT 101
Gly Ala Arg Pro Gly Gly His Pro Xaa Lys Trp Ser Phe Leu Trp Ser
-25 -20 -15

CTT GCA CTC TGG CTG CCT CTT GCC CTC TCT GTG TCT CTC TTT CTT GGT 149
Leu Ala Leu Trp Leu Pro Leu Ala Leu Ser Val Ser Leu Phe Leu Gly
-10 -5 1 5

CTC TCC CTC TCT CCT CCT CAG CCT GGT CTT TCT CTT TGG TGC ACA CTT 197
Leu Ser Leu Ser Pro Pro Gln Pro Gly Leu Ser Leu Trp Cys Thr Leu
10 15 20

AGT TAT TGT TGT GAG CAA TGG AAG TTC AAA GGA ACT CCC TCT CCA GCT 245
Ser Tyr Cys Cys Glu Gln Trp Lys Phe Lys Gly Thr Pro Ser Pro Ala
25 30 35

CTT CTG AAT CTK GGG ACA CGC GGG 269
Leu Leu Asn Leu Gly Thr Arg Gly
40 45

(2) INFORMATION FOR SEQ ID NO: 237:

(i) SEQUENCE CHARACTERISTICS:
(A) LENGTH: 395 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 220..396
(C) IDENTIFICATION METHOD: blastn
(D) OTHER INFORMATION: identity 98
region 207..383
id N28787
est

(ix) FEATURE:
(A) NAME/KEY: other
(B) LOCATION: 108..207
(C) IDENTIFICATION METHOD: blastn
(D) OTHER INFORMATION: identity 93
region 95..194
id N28787
est

(ix) FEATURE:
(A) NAME/KEY: other
(B) LOCATION: 220..316
(C) IDENTIFICATION METHOD: blastn
(D) OTHER INFORMATION: identity 97
region 209..305
id AA019783
est

(ix) FEATURE:
(A) NAME/KEY: other
(B) LOCATION: 108..207
(C) IDENTIFICATION METHOD: blastn
(D) OTHER INFORMATION: identity 92
region 97..196
id AA019783
est

(ix) FEATURE:
(A) NAME/KEY: other
(B) LOCATION: 307..392
(C) IDENTIFICATION METHOD: blastn
(D) OTHER INFORMATION: identity 91
region 297..382
id AA019783
est

(ix) FEATURE:
(A) NAME/KEY: other
(B) LOCATION: 108..207
(C) IDENTIFICATION METHOD: blastn
(D) OTHER INFORMATION: identity 92
region 99..198
id H86396
est

(ix) FEATURE:
(A) NAME/KEY: other
(B) LOCATION: 307..374
(C) IDENTIFICATION METHOD: blastn
(D) OTHER INFORMATION: identity 95
region 300..367
id H86396
est

(ix) FEATURE:
(A) NAME/KEY: other
(B) LOCATION: 255..313
(C) IDENTIFICATION METHOD: blastn
(D) OTHER INFORMATION: identity 96
region 247..305

id H86396
est

(ix) FEATURE:
(A) NAME/KEY: other
(B) LOCATION: 220..336
(C) IDENTIFICATION METHOD: blastn
(D) OTHER INFORMATION: identity 95
region 210..326
id H86516
est

(ix) FEATURE:
(A) NAME/KEY: other
(B) LOCATION: 108..207
(C) IDENTIFICATION METHOD: blastn
(D) OTHER INFORMATION: identity 93
region 98..197
id H86516
est

(ix) FEATURE:
(A) NAME/KEY: other
(B) LOCATION: 327..368
(C) IDENTIFICATION METHOD: blastn
(D) OTHER INFORMATION: identity 92
region 318..359
id H86516
est

(ix) FEATURE:
(A) NAME/KEY: other
(B) LOCATION: 108..207
(C) IDENTIFICATION METHOD: blastn
(D) OTHER INFORMATION: identity 92
region 111..210
id AA059290
est

(ix) FEATURE:
(A) NAME/KEY: other
(B) LOCATION: 272..354
(C) IDENTIFICATION METHOD: blastn
(D) OTHER INFORMATION: identity 92
region 285..367
id AA059290
est

(ix) FEATURE:
(A) NAME/KEY: other
(B) LOCATION: 220..286
(C) IDENTIFICATION METHOD: blastn
(D) OTHER INFORMATION: identity 91
region 223..289
id AA059290
est

(ix) FEATURE:
(A) NAME/KEY: sig_peptide
(B) LOCATION: 139..302
(C) IDENTIFICATION METHOD: Von Heijne matrix

(D) OTHER INFORMATION:  score 11.2
                           seq LLFALGSLGLIFA/LI

(xi) SEQUENCE DESCRIPTION: SEQ ID NO: 237:

ARCGGTTAGT GGACCGGGAC CGGTAVGGGT GCTGTWGCCA TCATGGCTGA CCCCGMMCCC   60

CGGBACMCTC GCTCCTCGAT CGAGGACGAC TTCANMTMNG GCAGGCAAGC GTGGCCTCCG  120

CCACCGTGYM BNTCCGA ATG VCC TTT CTG AGA AAA GTC TMN AGC ATT CTT     170
                   Met Xaa Phe Leu Arg Lys Val Xaa Ser Ile Leu
                   -55                 -50                 -45

TCT CTG CAG GTT CTC TTA ACT ACA GTG ACT TCA ACA GTT TTT TTA TAC    218
Ser Leu Gln Val Leu Leu Thr Thr Val Thr Ser Thr Val Phe Leu Tyr
            -40                 -35                 -30

TTT GAG TCT GTA CGG ACA TTT GTA CMT GAG AGT CCT GCC TTA ATT TTG    266
Phe Glu Ser Val Arg Thr Phe Val Xaa Glu Ser Pro Ala Leu Ile Leu
            -25                 -20                 -15

CTG TTT GCC CTC GGA TCT CTG GGT TTG ATT TTT GCG TTG ATT TTA AAC    314
Leu Phe Ala Leu Gly Ser Leu Gly Leu Ile Phe Ala Leu Ile Leu Asn
        -10                  -5                   1

AGV CAT AAG TAT CCC CTT AAC CTG TAC CTA CTT TTT GGA TTT ACG CTG    362
Xaa His Lys Tyr Pro Leu Asn Leu Tyr Leu Leu Phe Gly Phe Thr Leu
  5                  10                  15                  20

TTG GMA GCT CTG ACT GTG GCA GTT GTT GTT ACT                        395
Leu Xaa Ala Leu Thr Val Ala Val Val Val Thr
                25                  30

(2) INFORMATION FOR SEQ ID NO: 238:

(i) SEQUENCE CHARACTERISTICS:
    (A) LENGTH: 156 base pairs
    (B) TYPE: NUCLEIC ACID
    (C) STRANDEDNESS: DOUBLE
    (D) TOPOLOGY: LINEAR

(ii) MOLECULE TYPE: CDNA

(vi) ORIGINAL SOURCE:
    (A) ORGANISM: Homo Sapiens
    (F) TISSUE TYPE: Muscle

(ix) FEATURE:
    (A) NAME/KEY: other
    (B) LOCATION: 53..155
    (C) IDENTIFICATION METHOD: blastn
    (D) OTHER INFORMATION:  identity 100
                            region 24..126
                            id AA075942
                            est

(ix) FEATURE:
    (A) NAME/KEY: other

(B) LOCATION: 66..136
(C) IDENTIFICATION METHOD: blastn
(D) OTHER INFORMATION: identity 100
region 37..107
id AA262924
est

(ix) FEATURE:
(A) NAME/KEY: sig_peptide
(B) LOCATION: 22..135
(C) IDENTIFICATION METHOD: Von Heijne matrix
(D) OTHER INFORMATION: score 10.8
seq MLLLLLLLGSGQG/PQ

(xi) SEQUENCE DESCRIPTION: SEQ ID NO: 238:

AAAGGGTCGT TGGTGGGAAA G ATG GCG GCG ACT CTG GGA CCC CTT GGG TCG 51
Met Ala Ala Thr Leu Gly Pro Leu Gly Ser
-35 -30

TGG CAG CAG TGG CGG CGA TGT TTG TCG GCT CGG GAT GGG TCC AGG ATG 99
Trp Gln Gln Trp Arg Arg Cys Leu Ser Ala Arg Asp Gly Ser Arg Met
-25 -20 -15

TTA CTC CTT CTT CTT TTG TTG GGG TCT GGG CAG GGG CCA CAG CAA GTC 147
Leu Leu Leu Leu Leu Leu Leu Gly Ser Gly Gln Gly Pro Gln Gln Val
-10 -5 1

GGG GCG GGG 156
Gly Ala Gly
5

(2) INFORMATION FOR SEQ ID NO: 239:

(i) SEQUENCE CHARACTERISTICS:
(A) LENGTH: 353 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: complement(64..95)
(C) IDENTIFICATION METHOD: blastn
(D) OTHER INFORMATION: identity 90
region 79..110
id N98118
est

(ix) FEATURE:
(A) NAME/KEY: sig_peptide

(B) LOCATION: 195..317
(C) IDENTIFICATION METHOD: Von Heijne matrix
(D) OTHER INFORMATION: score 9.9
seq ILPFLLFPFPVNA/RS

(xi) SEQUENCE DESCRIPTION: SEQ ID NO: 239:

ATAGTGATCC TTTTCCTTCT CCCACTCCGT AAGTTTCTAT CCTTGGCCTC CTATTCTTTT 60

TACTACATAT ATACTTTATA TATACATATA TACTTGGAAC AGGCTTAATG AGTTCCAAGG 120

TTTCAAGTAT AATAGAAGGA TAGTTTCCCT AATATTTCTT CAAAACAGAT TTCTCTTCTG 180

AAATCCAGAG TCAT ATG TCC AGT TGG ATG TAT CTT GGA TAC CCC ATT GTC 230
Met Ser Ser Trp Met Tyr Leu Gly Tyr Pro Ile Val
-40 -35 -30

ACC TCA AAC ACT ACT TGT CTA AAA CTG ATC TCA TCA TCT TTT CCC CAA 278
Thr Ser Asn Thr Thr Cys Leu Lys Leu Ile Ser Ser Ser Phe Pro Gln
-25 -20 -15

ATC CTT CCT TTT CTT CTA TTT CCC TTC CCA GTG AAT GCC AGA TCT CAC 326
Ile Leu Pro Phe Leu Leu Phe Pro Phe Pro Val Asn Ala Arg Ser His
-10 -5 1

TYA GTT GCT CAA ACT AAA AGC CCG AGG 353
Xaa Val Ala Gln Thr Lys Ser Pro Arg
5 10

(2) INFORMATION FOR SEQ ID NO: 240:

(i) SEQUENCE CHARACTERISTICS:
(A) LENGTH: 159 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: other
(B) LOCATION: 88..132
(C) IDENTIFICATION METHOD: blastn
(D) OTHER INFORMATION: identity 100
region 352..396
id AA021024
est

(ix) FEATURE:
(A) NAME/KEY: sig_peptide
(B) LOCATION: 46..108
(C) IDENTIFICATION METHOD: Von Heijne matrix
(D) OTHER INFORMATION: score 9.7
seq QLCLLLLPSCSLS/VS

(xi) SEQUENCE DESCRIPTION: SEQ ID NO: 240:
ACCTCTTGGG GCCTACTTTG GGATGAAGTR GCCTCCCTCA GCAGC ATG GCC CCT GGG   57
GTC ATC ATC ATC CAG CTC TGC CTC TTG CTC CTG CCT TCC TGC TCC CTT   105
TCT GTT TCC GGA TGT TCC TGC CCT AGT GCC TGC TTC AGC ACC ACC AGC   153
CGC GAG                                                           159

(2) INFORMATION FOR SEQ ID NO: 241:

(i) SEQUENCE CHARACTERISTICS:
(A) LENGTH: 428 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 283..322
(C) IDENTIFICATION METHOD: blastn
(D) OTHER INFORMATION: identity 90
region 179..218
id N78639
est

(ix) FEATURE:
(A) NAME/KEY: other
(B) LOCATION: 283..322
(C) IDENTIFICATION METHOD: blastn
(D) OTHER INFORMATION: identity 90
region 193..232
id AA150442
est

(ix) FEATURE:
(A) NAME/KEY: sig_peptide
(B) LOCATION: 99..377
(C) IDENTIFICATION METHOD: Von Heijne matrix
(D) OTHER INFORMATION: score 9.6
seq LSLSLGASAP[?][?][?]/QQ

(xi) SEQUENCE DESCRIPTION: SEQ ID NO: 241:

ACATGCTCAG GGTCAGGTTC CAGCCCCAGC TGAGGGCTGA GGGGAGTGGG TGGACATGGG 60

GCAGGGAGCT GGAAGAACAC TCGAGAGACA GCAGGTAG ATG AGA CAT GGC TTT ATT 116
Met Arg His Gly Phe Ile
-90

CAG CAG CAG TTT TCA TTA ACA GCT TTC TCA MAC STT WRG SCW ATC TTC 164
Gln Gln Gln Phe Ser Leu Thr Ala Phe Ser Xaa Xaa Xaa Xaa Ile Phe
-85 -80 -75

ACA CTG KST GSC CTG TCT CAG TTG CTT AGT TCA GCA GCT CCC AAA CAC 212
Thr Leu Xaa Xaa Leu Ser Gln Leu Leu Ser Ser Ala Ala Pro Lys His
-70 -65 -60

ACA GCT GCA CCG ACG GCC CTC CCT TGC CTT CAG GGT CAG CAG CTT AAC 260
Thr Ala Ala Pro Thr Ala Leu Pro Cys Leu Gln Gly Gln Gln Leu Asn
-55 -50 -45 -40

TCT CTC TCT CTG GGC ACA AGT GAG CTG AGC TGT GTC CTG GCT TCC TCC 308
Ser Leu Ser Leu Gly Thr Ser Glu Leu Ser Cys Val Leu Ala Ser Ser
-35 -30 -25

TGT CTA TCT ACA AAG ACA GAC CCC TCT GGT CTC TCT CTC TCT TTG GGT 356
Cys Leu Ser Thr Lys Thr Asp Pro Ser Gly Leu Ser Leu Ser Leu Gly
-20 -15 -10

GCC AGC GCA CCT GTA CAG TGT CAG CAG GAC AAT TAT ACC TTT TGC KNN 404
Ala Ser Ala Pro Val Gln Cys Gln Gln Asp Asn Tyr Thr Phe Cys Xaa
-5 1 5

CAA TAC TGG CTT AGA GCA AGG CAT 428
Gln Tyr Trp Leu Arg Ala Arg His
10 15

(2) INFORMATION FOR SEQ ID NO: 242:

(i) SEQUENCE CHARACTERISTICS:
(A) LENGTH: 370 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 325..371
(C) IDENTIFICATION METHOD: blastn
(D) OTHER INFORMATION: identity 95
region 277..323
id AA015589

est

(ix) FEATURE:
(A) NAME/KEY: other
(B) LOCATION: 325..371
(C) IDENTIFICATION METHOD: blastn
(D) OTHER INFORMATION: identity 95
region 277..323
id AA019963
est

(ix) FEATURE:
(A) NAME/KEY: sig_peptide
(B) LOCATION: 140..262
(C) IDENTIFICATION METHOD: Von Heijne matrix
(D) OTHER INFORMATION: score 9.5
seq LIIFLSFLPFINS/SF

(xi) SEQUENCE DESCRIPTION: SEQ ID NO: 242:

ACAAGTGGGA TAGGTCCTGT GACAGAATTG TGTGATACAG GTCAAACAGG AGTTGGGTTA 60

TGGGGAAAAT GCCAGTTGAA ATATGTTTTG ATCTTTGGAG AAACCTATTT TTTCATTTAA 120

CCTGTTCTTT AAATCCAGT ATG TTC CAG AAC ATA CAA AAA TGT TTA AAT GTT 172
Met Phe Gln Asn Ile Gln Lys Cys Leu Asn Val
-40 -35

CCA TTT GTA AGA GGA TAT CAT GTA TTT TAT ATC AAT TTA AAT GCA GTT 220
Pro Phe Val Arg Gly Tyr His Val Phe Tyr Ile Asn Leu Asn Ala Val
-30 -25 -20 -15

ATC CTA ATC ATT TTT CTT TCA TTT TTA CCC TTT ATT AAC TCT TCA TTT 268
Ile Leu Ile Ile Phe Leu Ser Phe Leu Pro Phe Ile Asn Ser Ser Phe
-10 -5 1

GTT TAC AAA ACA AAT CCA CTC TAT GAC GCA ATC TCT AAT TAT GTG TTT 316
Val Tyr Lys Thr Asn Pro Leu Tyr Asp Ala Ile Ser Asn Tyr Val Phe
                5                   10                  15

TCT TTC AGG TAT CCA AAC CTT GRA ASC TTT GCT CTA GAT GTC AGG CTT 364
Ser Phe Arg Tyr Pro Asn Leu Xaa Xaa Phe Ala Leu Asp Val Arg Leu
            20                  25                  30

GTT TTT 370
Val Phe
35

(2) INFORMATION FOR SEQ ID NO: 243:

(i) SEQUENCE CHARACTERISTICS:
(A) LENGTH: 361 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: complement(215..358)
(C) IDENTIFICATION METHOD: blastn
(D) OTHER INFORMATION: identity 97
region 165..308
id R98055
est

(ix) FEATURE:
(A) NAME/KEY: other
(B) LOCATION: 185..289
(C) IDENTIFICATION METHOD: blastn
(D) OTHER INFORMATION: identity 97
region 252..356
id W23510
est

(ix) FEATURE:
(A) NAME/KEY: other
(B) LOCATION: 136..186
(C) IDENTIFICATION METHOD: blastn
(D) OTHER INFORMATION: identity 98
region 202..252
id W23510
est

(ix) FEATURE:
(A) NAME/KEY: other
(B) LOCATION: 73..109
(C) IDENTIFICATION METHOD: blastn
(D) OTHER INFORMATION: identity 97
region 139..175
id W23510
est

(ix) FEATURE:
(A) NAME/KEY: other
(B) LOCATION: 315..352
(C) IDENTIFICATION METHOD: blastn
(D) OTHER INFORMATION: identity 92
region 385..422
id W23510
est

(ix) FEATURE:
(A) NAME/KEY: other
(B) LOCATION: complement(215..358)
(C) IDENTIFICATION METHOD: blastn
(D) OTHER INFORMATION: identity 97
region 144..287
id T46976
est

(ix) FEATURE:
(A) NAME/KEY: other

(B) LOCATION: complement(227..358)
(C) IDENTIFICATION METHOD: blastn
(D) OTHER INFORMATION: identity 99
region 167..298
id AA084768
est

(ix) FEATURE:
(A) NAME/KEY: other
(B) LOCATION: complement(248..358)
(C) IDENTIFICATION METHOD: blastn
(D) OTHER INFORMATION: identity 97
region 169..279
id R50108
est

(ix) FEATURE:
(A) NAME/KEY: other
(B) LOCATION: complement(215..250)
(C) IDENTIFICATION METHOD: blastn
(D) OTHER INFORMATION: identity 94
region 278..313
id R50108
est

(ix) FEATURE:
(A) NAME/KEY: sig_peptide
(B) LOCATION: 281..340
(C) IDENTIFICATION METHOD: Von Heijne matrix
(D) OTHER INFORMATION: score 9.2
seq FPVLALFLSGSLA/LF

(xi) SEQUENCE DESCRIPTION: SEQ ID NO: 243:

AGAGTGAGAC GGGCAGATGG AGGAGGGATT GTAATGGCGG YAGCGGCAGC TCCCSTGCTC 60

TGACCCACGG CAGGCATACA GCATCCGATT TAATCTGGAT CCATTCCGGC GCCTTCCTCT 120

CCCAGTCACC CAGAGGGCCC CAACCCCGGC GGCCCTTTCT TCCTCAAATG TCCTCGGCTC 180

TATACCGTGC CTGGGTCTTT TCTCTTTCTC TCTGCCTGGA AGATTCCTTC TTTCCCCTTT 240

TGTCTTGCCC ACTCCTGTTT ACCCTTCAAG TTTCAAGTTC ATG TCA CTG TCT CAG 295
Met Ser Leu Ser Gln
-20

AGA GGT TTT CCT GTG CTC GCC CTG TTT CTC TCA GGA AGC CTT GCT CTT 343
Arg Gly Phe Pro Val Leu Ala Leu Phe Leu Ser Gly Ser Leu Ala Leu
-15 -10 -5 1

TTC CAT CAT ACC TCT GGG 361
Phe His His Thr Ser Gly
                5

(2) INFORMATION FOR SEQ ID NO: 244:

(i) SEQUENCE CHARACTERISTICS:
(A) LENGTH: 268 base pairs

(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 19..132
(C) IDENTIFICATION METHOD: blastn
(D) OTHER INFORMATION: identity 99
region 1..114
id N87112
est

(ix) FEATURE:
(A) NAME/KEY: other
(B) LOCATION: 194..267
(C) IDENTIFICATION METHOD: blastn
(D) OTHER INFORMATION: identity 94
region 174..247
id N87112
est

(ix) FEATURE:
(A) NAME/KEY: other
(B) LOCATION: 130..195
(C) IDENTIFICATION METHOD: blastn
(D) OTHER INFORMATION: identity 98
region 111..176
id N87112
est

(ix) FEATURE:
(A) NAME/KEY: other
(B) LOCATION: 68..267
(C) IDENTIFICATION METHOD: blastn
(D) OTHER INFORMATION: identity 99
region 1..200
id T68050
est

(ix) FEATURE:
(A) NAME/KEY: other
(B) LOCATION: 63..209
(C) IDENTIFICATION METHOD: blastn
(D) OTHER INFORMATION: identity 95
region 1..147
id AA157180
est

(ix) FEATURE:
(A) NAME/KEY: other
(B) LOCATION: 66..195
(C) IDENTIFICATION METHOD: blastn
(D) OTHER INFORMATION: identity 98

region 1..130
id AA094982
est

(ix) FEATURE:
(A) NAME/KEY: other
(B) LOCATION: 190..264
(C) IDENTIFICATION METHOD: blastn
(D) OTHER INFORMATION: identity 97
region 5..79
id W00395
est

(ix) FEATURE:
(A) NAME/KEY: sig_peptide
(B) LOCATION: 59..145
(C) IDENTIFICATION METHOD: Von Heijne matrix
(D) OTHER INFORMATION: score 8.9
seq ALLIVCDVPSASA/QR

(xi) SEQUENCE DESCRIPTION: SEQ ID NO: 244:

ACCCACCCTC AGACCTAGCC GGAGCAAAGT TTCACTTATA GAAGGGAGAG AAGCGAAC 58

ATG GCA GCG CGT TGG CGG TTT TGG TGT GTC TCT GTG ACC ATG GTG GTG 106
Met Ala Ala Arg Trp Arg Phe Trp Cys Val Ser Val Thr Met Val Val
-25 -20 -15

GCG CTG CTC ATC GTT TGC GAC GTT CCC TCA GCC TCT GCC CAA AGA AAG 154
Ala Leu Leu Ile Val Cys Asp Val Pro Ser Ala Ser Ala Gln Arg Lys
-10 -5 1

AAG GAG ATG GTG TTA TCT GAA AAG GTT AGT CAG CTG ATG GAA TGG ACT 202
Lys Glu Met Val Leu Ser Glu Lys Val Ser Gln Leu Met Glu Trp Thr
                5                   10                  15

AAC AAA AGA CCT GTA ATA AGA ATG AAT GGA GAC AAG TTC CGT CGC CTT 250
Asn Lys Arg Pro Val Ile Arg Met Asn Gly Asp Lys Phe Arg Arg Leu
20 25 30 35

GTG AAA GMN CCA CCG AGG 268
Val Lys Xaa Pro Pro Arg
40

(2) INFORMATION FOR SEQ ID NO: 245:

(i) SEQUENCE CHARACTERISTICS:
(A) LENGTH: 328 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 131..327
(C) IDENTIFICATION METHOD: blastn
(D) OTHER INFORMATION: identity 98
region 45..241
id H81225
est

(ix) FEATURE:
(A) NAME/KEY: other
(B) LOCATION: 86..123
(C) IDENTIFICATION METHOD: blastn
(D) OTHER INFORMATION: identity 100
region 1..38
id H81225
est

(ix) FEATURE:
(A) NAME/KEY: other
(B) LOCATION: 121..327
(C) IDENTIFICATION METHOD: blastn
(D) OTHER INFORMATION: identity 98
region 2..208
id W01412
est

(ix) FEATURE:
(A) NAME/KEY: other
(B) LOCATION: 129..327
(C) IDENTIFICATION METHOD: blastn
(D) OTHER INFORMATION: identity 98
region 1..199
id AA044118
est

(ix) FEATURE:
(A) NAME/KEY: other
(B) LOCATION: 131..327
(C) IDENTIFICATION METHOD: blastn
(D) OTHER INFORMATION: identity 98
region 13..209
id W42797
est

(ix) FEATURE:
(A) NAME/KEY: other
(B) LOCATION: 209..327
(C) IDENTIFICATION METHOD: blastn
(D) OTHER INFORMATION: identity 97
region 95..213
id R39635
est

(ix) FEATURE:
(A) NAME/KEY: other
(B) LOCATION: 130..209
(C) IDENTIFICATION METHOD: blastn
(D) OTHER INFORMATION: identity 98
region 15..94

id R39635
est

(ix) FEATURE:
(A) NAME/KEY: sig_peptide
(B) LOCATION: 191..286
(C) IDENTIFICATION METHOD: Von Heijne matrix
(D) OTHER INFORMATION: score 8.8
seq VPMLLLIVGGSFG/LR

(xi) SEQUENCE DESCRIPTION: SEQ ID NO: 245:

AACAAGTATG TTACGATGGC TCGATTGCTT TTGCCTAGCG GAAACCATTC ACTAAGGACC 60

GAGCACCAAA TAACCAAGGA AAAGGAAGTG AGTTAAGGAC GTACTCGTCT TGGTGAGAGC 120

GTGAGCTGCT GAGATTTGGG AGTCTGCGCT AGGCCCGCTT GGAGTTCTGA GCCGATGGAA 180

GAGTTCACTC ATG TTT GCA CCC GCG GTG ATG CGT GCT TTT CGC AAG AAC 229
Met Phe Ala Pro Ala Val Met Arg Ala Phe Arg Lys Asn
-30 -25 -20

AAG ACT CTC GGC TAT GGA GTC CCC ATG TTG TTG CTG ATT GTT GGA GGT 277
Lys Thr Leu Gly Tyr Gly Val Pro Met Leu Leu Leu Ile Val Gly Gly
-15 -10 -5

TCT TTT GGT CTT CGT GAG TTT TCT CNA ATC CGA TAT GAT GCT GTG AAG 325
Ser Phe Gly Leu Arg Glu Phe Ser Xaa Ile Arg Tyr Asp Ala Val Lys
1 5 10

GGG 328
Gly

(2) INFORMATION FOR SEQ ID NO: 246:

(i) SEQUENCE CHARACTERISTICS:
(A) LENGTH: 378 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Kidney

(ix) FEATURE:
(A) NAME/KEY: other
(B) LOCATION: 106..210
(C) IDENTIFICATION METHOD: blastn
(D) OTHER INFORMATION: identity 97
region 104..208
id AA131932
est

(ix) FEATURE:
(A) NAME/KEY: other

(B) LOCATION: 298..342
(C) IDENTIFICATION METHOD: blastn
(D) OTHER INFORMATION: identity 95
region 293..337
id AA131932
est

(ix) FEATURE:
(A) NAME/KEY: other
(B) LOCATION: 86..291
(C) IDENTIFICATION METHOD: blastn
(D) OTHER INFORMATION: identity 95
region 67..272
id AA001989
est

(ix) FEATURE:
(A) NAME/KEY: other
(B) LOCATION: 29..102
(C) IDENTIFICATION METHOD: blastn
(D) OTHER INFORMATION: identity 93
region 11..84
id AA001989
est

(ix) FEATURE:
(A) NAME/KEY: other
(B) LOCATION: 102..331
(C) IDENTIFICATION METHOD: blastn
(D) OTHER INFORMATION: identity 96
region 76..305
id W32996
est

(ix) FEATURE:
(A) NAME/KEY: other
(B) LOCATION: 55..96
(C) IDENTIFICATION METHOD: blastn
(D) OTHER INFORMATION: identity 97
region 31..72
id W32996
est

(ix) FEATURE:
(A) NAME/KEY: other
(B) LOCATION: 236..377
(C) IDENTIFICATION METHOD: blastn
(D) OTHER INFORMATION: identity 98
region 165..306
id AA121218
est

(ix) FEATURE:
(A) NAME/KEY: other
(B) LOCATION: 106..235
(C) IDENTIFICATION METHOD: blastn
(D) OTHER INFORMATION: identity 99
region 34..163
id AA121218
est

(ix) FEATURE:
(A) NAME/KEY: sig_peptide
(B) LOCATION: 70..180
(C) IDENTIFICATION METHOD: Von Heijne matrix
(D) OTHER INFORMATION: score 8.5
seq LLVLLLYAPVGFC/LL

(xi) SEQUENCE DESCRIPTION: SEQ ID NO: 246:

AAGAGCSSCT GCGGCCGGGC GCGAAAATGG CGGCGGCGGC GACGGCCNGG CGCTCCTGAA 60

GCAGCAGTT ATG GAG CTT CCC TCA GGG CCG GGG CCG GAG CGG CTC TTT GAC 111
Met Glu Leu Pro Ser Gly Pro Gly Pro Glu Arg Leu Phe Asp
-35 -30 -25

TCG CAC CGG CTT CCG GGT GAC TGC TTC CTA CTG CTC GTG CTG CTG CTC 159
Ser His Arg Leu Pro Gly Asp Cys Phe Leu Leu Leu Val Leu Leu Leu
-20 -15 -10

TAC GCG CCA GTC GGG TTC TGC CTC CTC GTC CTG SGC CTC TTT CTC GGG 207
Tyr Ala Pro Val Gly Phe Cys Leu Leu Val Leu Xaa Leu Phe Leu Gly
-5 1 5

ATC CAC GTC TTC CTG GTC AGC TGC GCG CTG CCA GAC AGC GTC CTT CGC 255
Ile His Val Phe Leu Val Ser Cys Ala Leu Pro Asp Ser Val Leu Arg
10 15 20 25

AGA TTC GTA GTG CGG ACC ATG TGT GCG GTG CTA GGG CTC GTG GCC CGG 303
Arg Phe Val Val Arg Thr Met Cys Ala Val Leu Gly Leu Val Ala Arg
30 35 40

CAG GAG GAC TCC GGA CTC CGG GAT CAC AGT GTC AGG GTC CTC ATT TCC 351
Gln Glu Asp Ser Gly Leu Arg Asp His Ser Val Arg Val Leu Ile Ser
45 50 55

AAC CAT GTG ACA CCT TTC GAC CAC CAG 378
Asn His Val Thr Pro Phe Asp His Gln
60 65

(2) INFORMATION FOR SEQ ID NO: 247:

(i) SEQUENCE CHARACTERISTICS:
(A) LENGTH: 381 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 38..181
(C) IDENTIFICATION METHOD: blastn

(D) OTHER INFORMATION:  identity 97
                           region 1..144
                           id W60505
                           est

(ix) FEATURE:
     (A) NAME/KEY: other
     (B) LOCATION: 186..312
     (C) IDENTIFICATION METHOD: blastn
     (D) OTHER INFORMATION:  identity 93
                             region 150..276
                             id W60505
                             est

(ix) FEATURE:
     (A) NAME/KEY: other
     (B) LOCATION: 305..346
     (C) IDENTIFICATION METHOD: blastn
     (D) OTHER INFORMATION:  identity 100
                             region 270..311
                             id W60505
                             est

(ix) FEATURE:
     (A) NAME/KEY: other
     (B) LOCATION: 38..312
     (C) IDENTIFICATION METHOD: blastn
     (D) OTHER INFORMATION:  identity 94
                             region 1..275
                             id W60589
                             est

(ix) FEATURE:
     (A) NAME/KEY: other
     (B) LOCATION: 305..346
     (C) IDENTIFICATION METHOD: blastn
     (D) OTHER INFORMATION:  identity 100
                             region 269..310
                             id W60589
                             est

(ix) FEATURE:
     (A) NAME/KEY: other
     (B) LOCATION: 32..175
     (C) IDENTIFICATION METHOD: blastn
     (D) OTHER INFORMATION:  identity 97
                             region 1..144
                             id R33763
                             est

(ix) FEATURE:
     (A) NAME/KEY: other
     (B) LOCATION: 176..261
     (C) IDENTIFICATION METHOD: blastn
     (D) OTHER INFORMATION:  identity 96
                             region 144..229
                             id R33763
                             est

(ix) FEATURE:
     (A) NAME/KEY: other

(B) LOCATION: 268..312
(C) IDENTIFICATION METHOD: blastn
(D) OTHER INFORMATION: identity 91
region 238..282
id R33763
est

(ix) FEATURE:
(A) NAME/KEY: other
(B) LOCATION: 305..337
(C) IDENTIFICATION METHOD: blastn
(D) OTHER INFORMATION: identity 93
region 276..308
id R33763
est

(ix) FEATURE:
(A) NAME/KEY: other
(B) LOCATION: 33..176
(C) IDENTIFICATION METHOD: blastn
(D) OTHER INFORMATION: identity 90
region 3..146
id AA123856
est

(ix) FEATURE:
(A) NAME/KEY: other
(B) LOCATION: 181..346
(C) IDENTIFICATION METHOD: blastn
(D) OTHER INFORMATION: identity 90
region 88..253
id HSB31E112
est

(ix) FEATURE:
(A) NAME/KEY: other
(B) LOCATION: 93..181
(C) IDENTIFICATION METHOD: blastn
(D) OTHER INFORMATION: identity 95
region 1..89
id HSB31E112
est

(ix) FEATURE:
(A) NAME/KEY: sig_peptide
(B) LOCATION: 106..375
(C) IDENTIFICATION METHOD: Von Heijne matrix
(D) OTHER INFORMATION: score 8.4
seq SLVLLTVTPSXRQ/QE

(xi) SEQUENCE DESCRIPTION: SEQ ID NO: 247:

AGGACTTCCC CCGGGCTGAG CTGCGCASGG GGTTTTGGCC AAATTGGGCG AGGGCACAAA 60

ATAACCACTT ACCCCTTCTC ACCGAGGAAG AGCGGGAGAA AGGGT ATG GCA CAG TCA 117
Met Ala Gln Ser
-90

CAA GGG TGG GTG RAA AGR TAC KTC AAG GCC TTT TGT AAA GGC TTC TTT 165
Gln Gly Trp Val Xaa Arg Tyr Xaa Lys Ala Phe Cys Lys Gly Phe Phe

-85                    -80                    -75

GTG GCG GTG CCT GTG GCA GTG ACT TTC TTG GAT CGG GTC GCC TGT GTG     213
Val Ala Val Pro Val Ala Val Thr Phe Leu Asp Arg Val Ala Cys Val
-70                 -65                 -60                 -55

GCA AGA GTA GAA GGA GCA TCG ATG CAG CCT TCT TTG AAT CCT GGG GGG     261
Ala Arg Val Glu Gly Ala Ser Met Gln Pro Ser Leu Asn Pro Gly Gly
                -50                 -45                 -40

AGC NAG TCA TCT GAT GTG GTG SDD DTG AAC CAC TGG AAA GTG AGG AAT     309
Ser Xaa Ser Ser Asp Val Val Xaa Xaa Asn His Trp Lys Val Arg Asn
            -35                 -30                 -25

TTT GAA GTA CAC CGT GGT GAC ATT GTA TCA TTG GTG TTG CTC ACT GTG     357
Phe Glu Val His Arg Gly Asp Ile Val Ser Leu Val Leu Leu Thr Val
        -20                 -15                 -10

ACG CCC TCC ASC CGA CAA CAG GAG                                     381
Thr Pro Ser Xaa Arg Gln Gln Glu
    -5                  1

(2) INFORMATION FOR SEQ ID NO: 248:

    (i) SEQUENCE CHARACTERISTICS:
        (A) LENGTH: 321 base pairs
        (B) TYPE: NUCLEIC ACID
        (C) STRANDEDNESS: DOUBLE
        (D) TOPOLOGY: LINEAR

    (ii) MOLECULE TYPE: CDNA

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: other
        (B) LOCATION: 11..158
        (C) IDENTIFICATION METHOD: blastn
        (D) OTHER INFORMATION:  identity 93
                                region 11..158
                                id H56585
                                est

    (ix) FEATURE:
        (A) NAME/KEY: other
        (B) LOCATION: 201..322
        (C) IDENTIFICATION METHOD: blastn
        (D) OTHER INFORMATION:  identity 97
                                region 201..322
                                id H56585
                                est

    (ix) FEATURE:
        (A) NAME/KEY: other
        (B) LOCATION: 151..322
        (C) IDENTIFICATION METHOD: blastn

(D) OTHER INFORMATION: identity 93
region 119..290
id AA147898
est

(ix) FEATURE:
(A) NAME/KEY: other
(B) LOCATION: 39..159
(C) IDENTIFICATION METHOD: blastn
(D) OTHER INFORMATION: identity 95
region 8..128
id AA147898
est

(ix) FEATURE:
(A) NAME/KEY: other
(B) LOCATION: 201..322
(C) IDENTIFICATION METHOD: blastn
(D) OTHER INFORMATION: identity 98
region 83..204
id R52248
est

(ix) FEATURE:
(A) NAME/KEY: other
(B) LOCATION: 170..202
(C) IDENTIFICATION METHOD: blastn
(D) OTHER INFORMATION: identity 93
region 51..83
id R52248
est

(ix) FEATURE:
(A) NAME/KEY: other
(B) LOCATION: 177..264
(C) IDENTIFICATION METHOD: blastn
(D) OTHER INFORMATION: identity 94
region 87..174
id H54950
est

(ix) FEATURE:
(A) NAME/KEY: other
(B) LOCATION: 284..315
(C) IDENTIFICATION METHOD: blastn
(D) OTHER INFORMATION: identity 96
region 192..223
id H54950
est

(ix) FEATURE:
(A) NAME/KEY: other
(B) LOCATION: complement(199..320)
(C) IDENTIFICATION METHOD: blastn
(D) OTHER INFORMATION: identity 95
region 40..161
id W22146
est

(ix) FEATURE:
(A) NAME/KEY: sig_peptide

(B) LOCATION: 67..135
(C) IDENTIFICATION METHOD: Von Heijne matrix
(D) OTHER INFORMATION: score 8.1
seq WLLVLSFVFGCNV/LR

(xi) SEQUENCE DESCRIPTION: SEQ ID NO: 248:

AGCCGCTGTT GTTGTGGTCC CCATGGAGCT GCCGTAGCGG ACCCAGCACA GCCAGGAGCG 60

TCCGGG ATG AGC TCA GCC GCG GCC GAC CAC TGG GCG TGG TTG CTG GTG 108
Met Ser Ser Ala Ala Ala Asp His Trp Ala Trp Leu Leu Val
-20 -15 -10

CTC AGC TTC GTG TTT GGA TGC AAT GTT CTT AGG ATC CTC CKC CCG GBC 156
Leu Ser Phe Val Phe Gly Cys Asn Val Leu Arg Ile Leu Xaa Pro Xaa
-5 1 5

YTC STM ATC STG CAK GTC CAG GGT GCT GCA GAA GGA CGC GGA SAG GAG 204
Xaa Xaa Ile Xaa Xaa Val Gln Gly Ala Ala Glu Gly Arg Gly Xaa Glu
10 15 20

TCA CAG ATG AGA GCG GAG ATC CAG GAC ATG AAG CAG GAG CTC TCC ACA 252
Ser Gln Met Arg Ala Glu Ile Gln Asp Met Lys Gln Glu Leu Ser Thr
25 30 35

GTC AAC ATG ATG GAC GAG TTT GCC AGA TAT GCC AGG CTG GAN AGA AAG 300
Val Asn Met Met Asp Glu Phe Ala Arg Tyr Ala Arg Leu Xaa Arg Lys
40 45 50 55

ATC AAC AAG ATG ACG GAT AAG 321
Ile Asn Lys Met Thr Asp Lys
60

(2) INFORMATION FOR SEQ ID NO: 249:

(i) SEQUENCE CHARACTERISTICS:
(A) LENGTH: 382 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 196..382
(C) IDENTIFICATION METHOD: blastn
(D) OTHER INFORMATION: identity 98
region 10..196
id HSC2EA121
est

(ix) FEATURE:

(A) NAME/KEY: other
(B) LOCATION: 121..205
(C) IDENTIFICATION METHOD: blastn
(D) OTHER INFORMATION: identity 100
region 134..218
id AA095017
est

(ix) FEATURE:
(A) NAME/KEY: other
(B) LOCATION: 197..252
(C) IDENTIFICATION METHOD: blastn
(D) OTHER INFORMATION: identity 92
region 209..264
id AA095017
est

(ix) FEATURE:
(A) NAME/KEY: sig_peptide
(B) LOCATION: 281..340
(C) IDENTIFICATION METHOD: Von Heijne matrix
(D) OTHER INFORMATION: score 8
seq HVFFLLLLAHIIA/LE

(xi) SEQUENCE DESCRIPTION: SEQ ID NO: 249:

GTTTTTGTTT GTGTGTGCGT GTTGTTGGCC TCCATCCCCA CTCCCCAGAC TCCACTTCTC 60

CAGGCCTCTC TCCCGCCTTT TCATCCCGCA TCCGCAGGAC ACCCAATCAC CGGGGCAACA 120

GGATGCCTTC CGCGCCTTCC ACCCTGACCT GGAATTCGTG GGCAAGTTCT TGAAACCCCT 180

GCTGATTGGT GAACTGGCCC CGGAGGAGCC CAGCCAGGAC CACGGCAAGA ACTCAAAGAT 240

CACTGAGGAC TTCCGGGCCC TGAGGAAGAC GGCTGAGGAC ATG AAC CTG TTC AAG 295
Met Asn Leu Phe Lys
-20

ACC AAC CAC GTG TTC TTC CTC CTC CTC CTG GCC CAC ATC ATC GCC CTG 343
Thr Asn His Val Phe Phe Leu Leu Leu Leu Ala His Ile Ile Ala Leu
-15 -10 -5 1

GAG AGC ATT GCA TGG TTC ACT GTC TTT TAC TTT GGC AAT 382
Glu Ser Ile Ala Trp Phe Thr Val Phe Tyr Phe Gly Asn
                5                   10

(2) INFORMATION FOR SEQ ID NO: 250:

(i) SEQUENCE CHARACTERISTICS:
(A) LENGTH: 298 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens

(F) TISSUE TYPE: Dystrophic muscle

(ix) FEATURE:
(A) NAME/KEY: other
(B) LOCATION: 80..300
(C) IDENTIFICATION METHOD: blastn
(D) OTHER INFORMATION: identity 100
region 101..321
id H21228
est

(ix) FEATURE:
(A) NAME/KEY: other
(B) LOCATION: 60..300
(C) IDENTIFICATION METHOD: blastn
(D) OTHER INFORMATION: identity 100
region 117..357
id R72127
est

(ix) FEATURE:
(A) NAME/KEY: other
(B) LOCATION: 19..59
(C) IDENTIFICATION METHOD: blastn
(D) OTHER INFORMATION: identity 100
region 77..117
id R72127
est

(ix) FEATURE:
(A) NAME/KEY: other
(B) LOCATION: 60..204
(C) IDENTIFICATION METHOD: blastn
(D) OTHER INFORMATION: identity 97
region 63..207
id H18908
est

(ix) FEATURE:
(A) NAME/KEY: other
(B) LOCATION: 195..269
(C) IDENTIFICATION METHOD: blastn
(D) OTHER INFORMATION: identity 100
region 199..273
id H18908
est

(ix) FEATURE:
(A) NAME/KEY: other
(B) LOCATION: 19..59
(C) IDENTIFICATION METHOD: blastn
(D) OTHER INFORMATION: identity 100
region 23..63
id H18908
est

(ix) FEATURE:
(A) NAME/KEY: other
(B) LOCATION: 65..203
(C) IDENTIFICATION METHOD: blastn
(D) OTHER INFORMATION: identity 95

region 144..282
id W93461
est

(ix) FEATURE:
(A) NAME/KEY: other
(B) LOCATION: 19..59
(C) IDENTIFICATION METHOD: blastn
(D) OTHER INFORMATION: identity 100
region 98..138
id W93461
est

(ix) FEATURE:
(A) NAME/KEY: other
(B) LOCATION: 252..288
(C) IDENTIFICATION METHOD: blastn
(D) OTHER INFORMATION: identity 97
region 333..369
id W93461
est

(ix) FEATURE:
(A) NAME/KEY: other
(B) LOCATION: 228..259
(C) IDENTIFICATION METHOD: blastn
(D) OTHER INFORMATION: identity 90
region 308..339
id W93461
est

(ix) FEATURE:
(A) NAME/KEY: other
(B) LOCATION: 136..300
(C) IDENTIFICATION METHOD: blastn
(D) OTHER INFORMATION: identity 98
region 93..257
id HUM085F04B
est

(ix) FEATURE:
(A) NAME/KEY: sig_peptide
(B) LOCATION: 170..241
(C) IDENTIFICATION METHOD: Von Heijne matrix
(D) OTHER INFORMATION: score 7.9
seq LLLPRVLLTMASG/SP

(xi) SEQUENCE DESCRIPTION: SEQ ID NO: 250:

AATCACGTGG CTGCCACCCA GGGGCATTCT TCGGGGGTGC ATCAGAGGGA GGGCAGAGCC 60

TGAGGATCTA AGCGAAGGCT TCCCCGGGTG TAATTTCCTG GGCTGTTTGT GAGGAGAGAT 120

CGAATTCGCC TCCTGCTCTC AGGCCTCTCT GCTCCTGTCT TTTGTTTGG ATG CCG GCG 178
Met Pro Ala

CTG CTG CCT GTG GCC TCC CGC CTT TTG TTG CTA CCC CGA GTC TTG CTG 226
Leu Leu Pro Val Ala Ser Arg Leu Leu Leu Leu Pro Arg Val Leu Leu
-20 -15 -10

ACC ATG GCC TCT GGA AGC CCT CCG ACC CAG CCC TCG CCG GCC TCG GAT     274
Thr Met Ala Ser Gly Ser Pro Pro Thr Gln Pro Ser Pro Ala Ser Asp
 -5                 1               5                   10

TCC GGC TCT GGC TAC GTT CCG GGC                                     298
Ser Gly Ser Gly Tyr Val Pro Gly
            15

(2) INFORMATION FOR SEQ ID NO: 251:

     (i) SEQUENCE CHARACTERISTICS:
          (A) LENGTH: 288 base pairs
          (B) TYPE: NUCLEIC ACID
          (C) STRANDEDNESS: DOUBLE
          (D) TOPOLOGY: LINEAR

     (ii) MOLECULE TYPE: CDNA

     (vi) ORIGINAL SOURCE:
          (A) ORGANISM: Homo Sapiens
          (D) DEVELOPMENTAL STAGE: Fetal
          (F) TISSUE TYPE: kidney

     (ix) FEATURE:
          (A) NAME/KEY: other
          (B) LOCATION: 1..286
          (C) IDENTIFICATION METHOD: blastn
          (D) OTHER INFORMATION:  identity 99
                                  region 1..286
                                  id HUM085F04B
                                  est

     (ix) FEATURE:
          (A) NAME/KEY: other
          (B) LOCATION: 147..245
          (C) IDENTIFICATION METHOD: blastn
          (D) OTHER INFORMATION:  identity 97
                                  region 167..265
                                  id R64509
                                  est

     (ix) FEATURE:
          (A) NAME/KEY: other
          (B) LOCATION: 99..161
          (C) IDENTIFICATION METHOD: blastn
          (D) OTHER INFORMATION:  identity 93
                                  region 118..180
                                  id R64509
                                  est

     (ix) FEATURE:
          (A) NAME/KEY: other
          (B) LOCATION: 245..286
          (C) IDENTIFICATION METHOD: blastn
          (D) OTHER INFORMATION:  identity 100
                                  region 266..307
                                  id R64509
                                  est

(ix) FEATURE:
(A) NAME/KEY: other
(B) LOCATION: 147..262
(C) IDENTIFICATION METHOD: blastn
(D) OTHER INFORMATION: identity 98
region 182..297
id H85714
est

(ix) FEATURE:
(A) NAME/KEY: other
(B) LOCATION: 99..161
(C) IDENTIFICATION METHOD: blastn
(D) OTHER INFORMATION: identity 93
region 133..195
id H85714
est

(ix) FEATURE:
(A) NAME/KEY: other
(B) LOCATION: 95..286
(C) IDENTIFICATION METHOD: blastn
(D) OTHER INFORMATION: identity 99
region 159..350
id H21228
est

(ix) FEATURE:
(A) NAME/KEY: other
(B) LOCATION: 201..286
(C) IDENTIFICATION METHOD: blastn
(D) OTHER INFORMATION: identity 96
region 151..236
id AA009893
est

(ix) FEATURE:
(A) NAME/KEY: other
(B) LOCATION: 148..206
(C) IDENTIFICATION METHOD: blastn
(D) OTHER INFORMATION: identity 98
region 97..155
id AA009893
est

(ix) FEATURE:
(A) NAME/KEY: other
(B) LOCATION: 99..160
(C) IDENTIFICATION METHOD: blastn
(D) OTHER INFORMATION: identity 91
region 49..110
id AA009893
est

(ix) FEATURE:
(A) NAME/KEY: sig_peptide
(B) LOCATION: 1..198
(C) IDENTIFICATION METHOD: Von Heijne matrix
(D) OTHER INFORMATION: score 7.9
seq LLLPRVLLTMASG/SP

(xi) SEQUENCE DESCRIPTION: SEQ ID NO: 251:

ATG ATA GGG TCG GGA TTG GCT GGC TCT GGA GGC GCA GGT GGT CCT TCT     48
Met Ile Gly Ser Gly Leu Ala Gly Ser Gly Gly Ala Gly Gly Pro Ser
    -65                 -60                 -55

TCT ACT GTC ACA TGG TGC GCG CTG TTT TCT AAT CAC GTG GCT GCM ACC     96
Ser Thr Val Thr Trp Cys Ala Leu Phe Ser Asn His Val Ala Ala Thr
-50                 -45                 -40                 -35

CAG GCC TCT CTG CTC CTG TCT TTT GTT TGG ATG CCG GCG CTG CTG CCT    144
Gln Ala Ser Leu Leu Leu Ser Phe Val Trp Met Pro Ala Leu Leu Pro
                -30                 -25                 -20

GTG GCC TCC CGC CTT TTG TTG CTA CCC CGA GTC TTG CTG ACC ATG GCC    192
Val Ala Ser Arg Leu Leu Leu Leu Pro Arg Val Leu Leu Thr Met Ala
            -15                 -10                 -5

TCT GGA AGC CCT CCG ACC CAG CCC TCG CCG GCC TCG GAT TCC GGC TCT    240
Ser Gly Ser Pro Pro Thr Gln Pro Ser Pro Ala Ser Asp Ser Gly Ser
        1               5                   10

GGC TAC GTT CCG GGC TCG GTC TCT GCA GCC TTT GTT ACT TGC CCC AGG    288
Gly Tyr Val Pro Gly Ser Val Ser Ala Ala Phe Val Thr Cys Pro Arg
15                  20                  25                  30

(2) INFORMATION FOR SEQ ID NO: 252:

(i) SEQUENCE CHARACTERISTICS:
(A) LENGTH: 322 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 32..319
(C) IDENTIFICATION METHOD: blastn
(D) OTHER INFORMATION:  identity 99
                        region 53..340
                        id AA056366
                        est

(ix) FEATURE:
(A) NAME/KEY: other
(B) LOCATION: 32..319
(C) IDENTIFICATION METHOD: blastn
(D) OTHER INFORMATION:  identity 99
                        region 80..367
                        id R77008
                        est

(ix) FEATURE:
(A) NAME/KEY: other
(B) LOCATION: 32..223
(C) IDENTIFICATION METHOD: blastn
(D) OTHER INFORMATION: identity 98
region 77..268
id W75983
est

(ix) FEATURE:
(A) NAME/KEY: other
(B) LOCATION: 223..319
(C) IDENTIFICATION METHOD: blastn
(D) OTHER INFORMATION: identity 98
region 269..365
id W75983
est

(ix) FEATURE:
(A) NAME/KEY: other
(B) LOCATION: 32..223
(C) IDENTIFICATION METHOD: blastn
(D) OTHER INFORMATION: identity 98
region 129..320
id W39055
est

(ix) FEATURE:
(A) NAME/KEY: other
(B) LOCATION: 223..319
(C) IDENTIFICATION METHOD: blastn
(D) OTHER INFORMATION: identity 98
region 321..417
id W39055
est

(ix) FEATURE:
(A) NAME/KEY: other
(B) LOCATION: 32..236
(C) IDENTIFICATION METHOD: blastn
(D) OTHER INFORMATION: identity 98
region 84..288
id N48534
est

(ix) FEATURE:
(A) NAME/KEY: other
(B) LOCATION: 264..319
(C) IDENTIFICATION METHOD: blastn
(D) OTHER INFORMATION: identity 92
region 318..373
id N48534
est

(ix) FEATURE:
(A) NAME/KEY: sig_peptide
(B) LOCATION: 11..82
(C) IDENTIFICATION METHOD: Von Heijne matrix
(D) OTHER INFORMATION: score 7.9
seq LLLPRVLLTMASG/SP

(xi) SEQUENCE DESCRIPTION: SEQ ID NO: 252:

ATTTGTTTGG ATG CCG GCG CTG CTG CCT GTG GCC TCC CGC CTT TTG TTG 49
Met Pro Ala Leu Leu Pro Val Ala Ser Arg Leu Leu Leu
-20 -15

CTA CCC CGA GTC TTG CTG ACC ATG GCC TCT GGA AGC CCT CCG ACC CAG 97
Leu Pro Arg Val Leu Leu Thr Met Ala Ser Gly Ser Pro Pro Thr Gln
-10 -5 1 5

CCC TCG CCG GCC TCG GAT TCC GGC TCT GGC TAC GTT CCG GGC TCG GTC 145
Pro Ser Pro Ala Ser Asp Ser Gly Ser Gly Tyr Val Pro Gly Ser Val
10 15 20

TCT GCA GCC TTT GTT ACT TGC CCC AAC GAG AAG GTC GCC AAG GAG ATC 193
Ser Ala Ala Phe Val Thr Cys Pro Asn Glu Lys Val Ala Lys Glu Ile
25 30 35

GCC AGG GCC GTG GTG GAG AAG CGC CTA GCA GCC TGC GTC AAC CTC ATC 241
Ala Arg Ala Val Val Glu Lys Arg Leu Ala Ala Cys Val Asn Leu Ile
40 45 50

CCT CAG ATT ACA TCC ATC TAT GAG TGG AAA GGG AHG ATC GAG GAA GAC 289
Pro Gln Ile Thr Ser Ile Tyr Glu Trp Lys Gly Xaa Ile Glu Glu Asp
55 60 65

AGT GAG GTG CTG ATG ATG ATT AAA ACC CAA GCG 322
Ser Glu Val Leu Met Met Ile Lys Thr Gln Ala
70 75 80

(2) INFORMATION FOR SEQ ID NO: 253:

(i) SEQUENCE CHARACTERISTICS:
(A) LENGTH: 395 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 138..193
(C) IDENTIFICATION METHOD: blastn
(D) OTHER INFORMATION: identity 100
region 247..302
id T80036
est

(ix) FEATURE:
(A) NAME/KEY: sig_peptide
(B) LOCATION: 33..308

(C) IDENTIFICATION METHOD: Von Heijne matrix
(D) OTHER INFORMATION: score 7.6
seq FLLLTVALLASYS/VH

(xi) SEQUENCE DESCRIPTION: SEQ ID NO: 253:

AAGATGGAAC TGGTAGTCAG CTGGAGAGCA GC ATG GAG GCG TCC TGG GGG AGC 53
Met Glu Ala Ser Trp Gly Ser
-90

TTC AAC GCT GAG CGG GGC TGG TAT GTC TCT GTG CAG CAG CCT GAA GAA 101
Phe Asn Ala Glu Arg Gly Trp Tyr Val Ser Val Gln Gln Pro Glu Glu
-85 -80 -75 -70

GCG GAG GCC GAA GAG TTG AGT CCG TTG CTA AGC AAC GAA CTT CAC AGA 149
Ala Glu Ala Glu Glu Leu Ser Pro Leu Leu Ser Asn Glu Leu His Arg
-65 -60 -55

CAG CGA TCC CCA GGT GTT TCA TTT GGT TTA TCA GTG TTT AAT TTG ATG 197
Gln Arg Ser Pro Gly Val Ser Phe Gly Leu Ser Val Phe Asn Leu Met
-50 -45 -40

AAT GCC ATC ATG GGA AGT GGC ATC CTT GGC TTA GCT TAT GTT ATG GCT 245
Asn Ala Ile Met Gly Ser Gly Ile Leu Gly Leu Ala Tyr Val Met Ala
-35 -30 -25

AAT ACC GGT GTC TTT GGA TTT AGC TTC TTG CTG CTG ACA GTT GCT CTC 293
Asn Thr Gly Val Phe Gly Phe Ser Phe Leu Leu Leu Thr Val Ala Leu
-20 -15 -10

CTG GCT TCT TAC TCA GTC CAT CTT CTG CTT AGT ATG TGT ATT CAG ACA 341
Leu Ala Ser Tyr Ser Val His Leu Leu Leu Ser Met Cys Ile Gln Thr
-5 1 5 10

GCT GTA ACA TCT TAT GAA GAT CTT GGA CTC TTT GCA TTT GGA TTA CCT 389
Ala Val Thr Ser Tyr Glu Asp Leu Gly Leu Phe Ala Phe Gly Leu Pro
15 20 25

GGA CTG 395
Gly Leu

(2) INFORMATION FOR SEQ ID NO: 254:

(i) SEQUENCE CHARACTERISTICS:
(A) LENGTH: 134 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: other
(B) LOCATION: 18..132

(C) IDENTIFICATION METHOD: blastn
(D) OTHER INFORMATION:  identity 95
                        region 1..115
                        id T10447
                        est

(ix) FEATURE:
     (A) NAME/KEY: sig_peptide
     (B) LOCATION: 78..128
     (C) IDENTIFICATION METHOD: Von Heijne matrix
     (D) OTHER INFORMATION:  score 7.6
                             seq FFLLLRFFLRIDG/VP

(xi) SEQUENCE DESCRIPTION: SEQ ID NO: 254:

ATTTTGAAGA AGTTCTCCTT TTTGAGGATG AACTTCATGA TCATGGAGTT TCAAGCCTGA   60

GTGTGAAGAT TAGAGTA ATG CCT TCT AGC TTT TTC CTG CTG TTG CGG TTT     110
                   Met Pro Ser Ser Phe Phe Leu Leu Leu Arg Phe
                           -15                 -10

TTC TTG AGA ATT GAC GGG GTG CCG                                    134
Phe Leu Arg Ile Asp Gly Val Pro
    -5                  1

(2) INFORMATION FOR SEQ ID NO: 255:

(i) SEQUENCE CHARACTERISTICS:
     (A) LENGTH: 337 base pairs
     (B) TYPE: NUCLEIC ACID
     (C) STRANDEDNESS: DOUBLE
     (D) TOPOLOGY: LINEAR

(ii) MOLECULE TYPE: CDNA

(vi) ORIGINAL SOURCE:
     (A) ORGANISM: Homo Sapiens
     (F) TISSUE TYPE: Dystrophic muscle

(ix) FEATURE:
     (A) NAME/KEY: other
     (B) LOCATION: 44..276
     (C) IDENTIFICATION METHOD: blastn
     (D) OTHER INFORMATION:  identity 98
                             region 1..233
                             id N83601
                             est

(ix) FEATURE:
     (A) NAME/KEY: other
     (B) LOCATION: 51..276
     (C) IDENTIFICATION METHOD: blastn
     (D) OTHER INFORMATION:  identity 92
                             region 15..240
                             id N56180
                             est

(ix) FEATURE:

(A) NAME/KEY: other
(B) LOCATION: 69..216
(C) IDENTIFICATION METHOD: blastn
(D) OTHER INFORMATION: identity 95
region 23..170
id R57553
est

(ix) FEATURE:
(A) NAME/KEY: other
(B) LOCATION: 46..75
(C) IDENTIFICATION METHOD: blastn
(D) OTHER INFORMATION: identity 96
region 1..30
id R57553
est

(ix) FEATURE:
(A) NAME/KEY: other
(B) LOCATION: 58..142
(C) IDENTIFICATION METHOD: blastn
(D) OTHER INFORMATION: identity 98
region 42..126
id R57171
est

(ix) FEATURE:
(A) NAME/KEY: other
(B) LOCATION: 18..56
(C) IDENTIFICATION METHOD: blastn
(D) OTHER INFORMATION: identity 94
region 1..39
id R57171
est

(ix) FEATURE:
(A) NAME/KEY: other
(B) LOCATION: 142..182
(C) IDENTIFICATION METHOD: blastn
(D) OTHER INFORMATION: identity 95
region 97..137
id N88966
est

(ix) FEATURE:
(A) NAME/KEY: other
(B) LOCATION: 49..83
(C) IDENTIFICATION METHOD: blastn
(D) OTHER INFORMATION: identity 100
region 1..35
id N88966
est

(ix) FEATURE:
(A) NAME/KEY: sig_peptide
(B) LOCATION: 200..256
(C) IDENTIFICATION METHOD: Von Heijne matrix
(D) OTHER INFORMATION: score 7.6
seq FIVGIYFLSSCRA/EE

(xi) SEQUENCE DESCRIPTION: SEQ ID NO: 255:

AGTCTTTGTC CTGAGCCCAC GATTCCAGAG CTGGCTGGAC CCAAGGAGGT GAAGAGTCAC 60

TTTTCAGCCC CAGGAAGGGC AAAGAAGAGA GARAATCAGC CTGTCTGCTC TCTCCTTGGC 120

TCAACAAGGC CTCTAACAGT CTTCTGTCCT CTATTCTGCA CACGGCATAT TTGGGAACGA 180

GAAACAAAAG TTTTCCCAA ATG AAG AGA ACT CAC TTG TTT ATT GTG GGG ATT 232
Met Lys Arg Thr His Leu Phe Ile Val Gly Ile
-15 -10

TAT TTT CTG TCC TCT TGC AGG GCA GAA GAG GGG CTT AAT TTC CCC ACA 280
Tyr Phe Leu Ser Ser Cys Arg Ala Glu Glu Gly Leu Asn Phe Pro Thr
-5 1 5

TAT GAT GGG AAG GAC CGA GTG GTA AGT CTT TCC GAG AAG AAC TTC AAG 328
Tyr Asp Gly Lys Asp Arg Val Val Ser Leu Ser Glu Lys Asn Phe Lys
10 15 20

CAG GTT TTA 337
Gln Val Leu
25

(2) INFORMATION FOR SEQ ID NO: 256:

(i) SEQUENCE CHARACTERISTICS:
(A) LENGTH: 327 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: other
(B) LOCATION: 98..223
(C) IDENTIFICATION METHOD: blastn
(D) OTHER INFORMATION: identity 97
region 57..182
id AA019348
est

(ix) FEATURE:
(A) NAME/KEY: other
(B) LOCATION: 215..329
(C) IDENTIFICATION METHOD: blastn
(D) OTHER INFORMATION: identity 98
region 173..287
id AA019348
est

(ix) FEATURE:
(A) NAME/KEY: other
(B) LOCATION: 43..98

(C) IDENTIFICATION METHOD: blastn
(D) OTHER INFORMATION: identity 96
region 1..56
id AA019348
est

(ix) FEATURE:
(A) NAME/KEY: other
(B) LOCATION: 98..217
(C) IDENTIFICATION METHOD: blastn
(D) OTHER INFORMATION: identity 99
region 57..176
id AA013099
est

(ix) FEATURE:
(A) NAME/KEY: other
(B) LOCATION: 211..329
(C) IDENTIFICATION METHOD: blastn
(D) OTHER INFORMATION: identity 97
region 171..289
id AA013099
est

(ix) FEATURE:
(A) NAME/KEY: other
(B) LOCATION: 43..98
(C) IDENTIFICATION METHOD: blastn
(D) OTHER INFORMATION: identity 96
region 1..56
id AA013099
est

(ix) FEATURE:
(A) NAME/KEY: other
(B) LOCATION: 215..319
(C) IDENTIFICATION METHOD: blastn
(D) OTHER INFORMATION: identity 98
region 130..234
id R54717
est

(ix) FEATURE:
(A) NAME/KEY: other
(B) LOCATION: 142..223
(C) IDENTIFICATION METHOD: blastn
(D) OTHER INFORMATION: identity 95
region 58..139
id R54717
est

(ix) FEATURE:
(A) NAME/KEY: other
(B) LOCATION: 95..149
(C) IDENTIFICATION METHOD: blastn
(D) OTHER INFORMATION: identity 92
region 10..64
id R54717
est

(ix) FEATURE:

(A) NAME/KEY: other
(B) LOCATION: 105..173
(C) IDENTIFICATION METHOD: blastn
(D) OTHER INFORMATION: identity 98
region 1..69
id AA112675
est

(ix) FEATURE:
(A) NAME/KEY: other
(B) LOCATION: 215..267
(C) IDENTIFICATION METHOD: blastn
(D) OTHER INFORMATION: identity 92
region 108..160
id AA112675
est

(ix) FEATURE:
(A) NAME/KEY: other
(B) LOCATION: 296..329
(C) IDENTIFICATION METHOD: blastn
(D) OTHER INFORMATION: identity 94
region 185..218
id AA112675
est

(ix) FEATURE:
(A) NAME/KEY: other
(B) LOCATION: 167..196
(C) IDENTIFICATION METHOD: blastn
(D) OTHER INFORMATION: identity 93
region 62..91
id AA112675
est

(ix) FEATURE:
(A) NAME/KEY: other
(B) LOCATION: 88..223
(C) IDENTIFICATION METHOD: blastn
(D) OTHER INFORMATION: identity 91
region 3..138
id H27167
est

(ix) FEATURE:
(A) NAME/KEY: other
(B) LOCATION: 215..319
(C) IDENTIFICATION METHOD: blastn
(D) OTHER INFORMATION: identity 98
region 129..233
id H27167
est

(ix) FEATURE:
(A) NAME/KEY: sig_peptide
(B) LOCATION: 145..213
(C) IDENTIFICATION METHOD: Von Heijne matrix
(D) OTHER INFORMATION: score 7.4
seq VLLLAALPPVLLP/GA

(xi) SEQUENCE DESCRIPTION: SEQ ID NO: 256:

AGAGTGTTCG CCGCCGCCGC GGCCGCCACC TGGAGTTTCT TCAGACTCCA GATTTCCCTG 60

TCAACCACGA GGAGTCCAGA GAGGAAACGC GGAGGAGACA ACAGTACCTG ACGCCTCTTT 120

CAGCCCGGGA TCGCCCCAGC AGGG ATG GGC GAC AAG ATC TGG CTG CCC TTC 171
Met Gly Asp Lys Ile Trp Leu Pro Phe
-20 -15

CCC GTG CTC CTT CTG GCC GCT CTG CCT CCG GTG CTG CTG CCT GGG GCG 219
Pro Val Leu Leu Leu Ala Ala Leu Pro Pro Val Leu Leu Pro Gly Ala
-10 -5 1

GCC GGC TTC ACA CCT TCC CTC GAT AGC GAC TTC ACC TTT ACC CTT CCC 267
Ala Gly Phe Thr Pro Ser Leu Asp Ser Asp Phe Thr Phe Thr Leu Pro
                5                   10                  15

GCC GGC CAG AAG GAG TGC TTC TAC CAG CCC ATG CCC CTG RAG GCC TCG 315
Ala Gly Gln Lys Glu Cys Phe Tyr Gln Pro Met Pro Leu Xaa Ala Ser
            20                  25                  30

CTG GAG ATC GAG 327
Leu Glu Ile Glu
        35

(2) INFORMATION FOR SEQ ID NO: 257:

(i) SEQUENCE CHARACTERISTICS:
(A) LENGTH: 476 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 166..415
(C) IDENTIFICATION METHOD: blastn
(D) OTHER INFORMATION: identity 96
region 1..250
id HSU52870
est

(ix) FEATURE:
(A) NAME/KEY: other
(B) LOCATION: 182..337
(C) IDENTIFICATION METHOD: blastn
(D) OTHER INFORMATION: identity 94
region 156..311
id T35951
est

(ix) FEATURE:
(A) NAME/KEY: other
(B) LOCATION: 32..132
(C) IDENTIFICATION METHOD: blastn
(D) OTHER INFORMATION: identity 96
region 7..107
id T35951
est

(ix) FEATURE:
(A) NAME/KEY: other
(B) LOCATION: 136..193
(C) IDENTIFICATION METHOD: blastn
(D) OTHER INFORMATION: identity 96
region 109..166
id T35951
est

(ix) FEATURE:
(A) NAME/KEY: other
(B) LOCATION: 182..328
(C) IDENTIFICATION METHOD: blastn
(D) OTHER INFORMATION: identity 92
region 156..302
id T35949
est

(ix) FEATURE:
(A) NAME/KEY: other
(B) LOCATION: 32..132
(C) IDENTIFICATION METHOD: blastn
(D) OTHER INFORMATION: identity 96
region 7..107
id T35949
est

(ix) FEATURE:
(A) NAME/KEY: other
(B) LOCATION: 136..193
(C) IDENTIFICATION METHOD: blastn
(D) OTHER INFORMATION: identity 94
region 109..166
id T35949
est

(ix) FEATURE:
(A) NAME/KEY: other
(B) LOCATION: 233..409
(C) IDENTIFICATION METHOD: blastn
(D) OTHER INFORMATION: identity 96
region 53..229
id W17267
est

(ix) FEATURE:
(A) NAME/KEY: other
(B) LOCATION: 401..476
(C) IDENTIFICATION METHOD: blastn
(D) OTHER INFORMATION: identity 96
region 220..295
id W17267

est

(ix) FEATURE:
(A) NAME/KEY: other
(B) LOCATION: 182..399
(C) IDENTIFICATION METHOD: blastn
(D) OTHER INFORMATION: identity 96
region 54..271
id HSC34G011
est

(ix) FEATURE:
(A) NAME/KEY: other
(B) LOCATION: 136..192
(C) IDENTIFICATION METHOD: blastn
(D) OTHER INFORMATION: identity 96
region 7..63
id HSC34G011
est

(ix) FEATURE:
(A) NAME/KEY: sig_peptide
(B) LOCATION: 306..416
(C) IDENTIFICATION METHOD: Von Heijne matrix
(D) OTHER INFORMATION: score 7.3
seq LLSACLVTLWGLG/EP

(xi) SEQUENCE DESCRIPTION: SEQ ID NO: 257:

AATTCATTTT TCACTCCTCC CTCCTAGGTC ACACTTTTCA GAAAAAGAAT CTGCATCCTG 60

GAAACCAGAA GAAAAATATG AGACGGGGAA TCATCGTGTG ATGTGTGTGC TGCCTTTGGC 120

TKWGTGTGTK GAAGTYCCKG CTCAGGTGTT AGGTACAGTG TGTTTGATCG TGGTGGCTTG 180

AGGGGAACCC GCTGTTCAGA GCTGTGACTG CGGCTGCACT CAGAGAAGCT GCCCTTGGCT 240

GCTCGTAGCG CCGGGCCTTC TCTCCTCGTC ATCATCCAGA GCAGCCAGTG TCCGGGAGGC 300

ADVNG ATG CCC CAC TCC AGC CTG CAT CCA TCC ATC CCG TGT CCC AGG GGT 350
Met Pro His Ser Ser Leu His Pro Ser Ile Pro Cys Pro Arg Gly
-35 -30 -25

CAC GGG GCC CAG AAG GCA GCC TTG GTT CTG CTG AGT GCC TGC CTG GTG 398
His Gly Ala Gln Lys Ala Ala Leu Val Leu Leu Ser Ala Cys Leu Val
-20 -15 -10

ACC CTT TGG GGG CTA GGA GAG CCA CCA GAG CAC ACT CTC CGG TAC CTG 446
Thr Leu Trp Gly Leu Gly Glu Pro Pro Glu His Thr Leu Arg Tyr Leu
-5 1 5 10

GTG CTC CAM CTA GCC TCC CTG CAG CTG GGA 476
Val Leu Xaa Leu Ala Ser Leu Gln Leu Gly
15 20

(2) INFORMATION FOR SEQ ID NO: 258:

(i) SEQUENCE CHARACTERISTICS:

(A) LENGTH: 220 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: other
(B) LOCATION: complement(28..221)
(C) IDENTIFICATION METHOD: blastn
(D) OTHER INFORMATION: identity 98
region 32..225
id AA025879
est

(ix) FEATURE:
(A) NAME/KEY: other
(B) LOCATION: complement(1..154)
(C) IDENTIFICATION METHOD: blastn
(D) OTHER INFORMATION: identity 98
region 97..250
id N33067
est

(ix) FEATURE:
(A) NAME/KEY: other
(B) LOCATION: complement(144..221)
(C) IDENTIFICATION METHOD: blastn
(D) OTHER INFORMATION: identity 94
region 31..108
id N33067
est

(ix) FEATURE:
(A) NAME/KEY: other
(B) LOCATION: complement(1..221)
(C) IDENTIFICATION METHOD: blastn
(D) OTHER INFORMATION: identity 98
region 31..251
id AA132495
est

(ix) FEATURE:
(A) NAME/KEY: other
(B) LOCATION: complement(1..221)
(C) IDENTIFICATION METHOD: blastn
(D) OTHER INFORMATION: identity 98
region 31..251
id AA063545
est

(ix) FEATURE:
(A) NAME/KEY: other
(B) LOCATION: complement(28..221)
(C) IDENTIFICATION METHOD: blastn
(D) OTHER INFORMATION: identity 98

region 47..240
id N99132
est

(ix) FEATURE:
(A) NAME/KEY: sig_peptide
(B) LOCATION: 59..145
(C) IDENTIFICATION METHOD: Von Heijne matrix
(D) OTHER INFORMATION: score 7.3
seq HLLLLLLPAPTLK/GL

(xi) SEQUENCE DESCRIPTION: SEQ ID NO: 258:

ACACTCGGGC CCCACTCAAG GATGTAGGGC CTTTTCTGGC CCCTGACCCC TCCCTGGC 58

ATG GGA GCG TGG GGA CGG GGC TGG CCT TGG GAG GAG CGG CAG GGG CAT 106
Met Gly Ala Trp Gly Arg Gly Trp Pro Trp Glu Glu Arg Gln Gly His
-25 -20 -15

CAC CTC CTT CTG CTG CTT CTC CCT GCT CCT ACC CTC AAG GGC CTG GGG 154
His Leu Leu Leu Leu Leu Leu Pro Ala Pro Thr Leu Lys Gly Leu Gly
-10 -5 1

GCT GCC CAG CTG CCT CTA TGC CCT TCT GGG GGT CTC AGC CCA CTG CTG 202
Ala Ala Gln Leu Pro Leu Cys Pro Ser Gly Gly Leu Ser Pro Leu Leu
                5                   10                  15

ACA CTT CTG CAA TCC GGG 220
Thr Leu Leu Gln Ser Gly
20 25

(2) INFORMATION FOR SEQ ID NO: 259:

(i) SEQUENCE CHARACTERISTICS:
(A) LENGTH: 428 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 56..429
(C) IDENTIFICATION METHOD: blastn
(D) OTHER INFORMATION: identity 98
region 65..438
id W27019
est

(ix) FEATURE:
(A) NAME/KEY: other
(B) LOCATION: complement(79..429)

(C) IDENTIFICATION METHOD: blastn
(D) OTHER INFORMATION: identity 99
region 91..441
id W26783
est

(ix) FEATURE:
(A) NAME/KEY: other
(B) LOCATION: 284..390
(C) IDENTIFICATION METHOD: blastn
(D) OTHER INFORMATION: identity 92
region 343..449
id W85233
est

(ix) FEATURE:
(A) NAME/KEY: sig_peptide
(B) LOCATION: 57..281
(C) IDENTIFICATION METHOD: Von Heijne matrix
(D) OTHER INFORMATION: score 7.2
seq LLFIIGLIGCCAT/IR

(xi) SEQUENCE DESCRIPTION: SEQ ID NO: 259:

ACTCTCGGTG AGCGCRSCCC GCTCTCCGGG CCGGGTCTTC GCGGGCCACC GGCGCC ATG 59
Met
-75

GGC CAG TGC GGC ATC ACC TCC TCC AAG ACC GTG CTG GTC TTT CTC AAC 107
Gly Gln Cys Gly Ile Thr Ser Ser Lys Thr Val Leu Val Phe Leu Asn
-70 -65 -60

CTC ATC TTC TGG GGG GCA GCT GGC ATT TTA TGC TAT GTG GGA GCC TAT 155
Leu Ile Phe Trp Gly Ala Ala Gly Ile Leu Cys Tyr Val Gly Ala Tyr
-55 -50 -45

GTC TTC ATC ACT TAT GAT GAC TAT GAC CAC TTC TTT GAA GAT GTG TAC 203
Val Phe Ile Thr Tyr Asp Asp Tyr Asp His Phe Phe Glu Asp Val Tyr
-40 -35 -30

ACG CTC ATC CCT GCT GTA GTG ATC ATA GCT GTA AGA GCC CTG CTT TTC 251
Thr Leu Ile Pro Ala Val Val Ile Ile Ala Val Arg Ala Leu Leu Phe
-25 -20 -15

ATC ATT GGG CTA ATT GGC TGC TGT GCC ACA ATC CGG GAA AGT CGC TGT 299
Ile Ile Gly Leu Ile Gly Cys Cys Ala Thr Ile Arg Glu Ser Arg Cys
-10 -5 1 5

GGA CTT GCC ACG TTT GTC ATC ATC CTG CTC TTG GTT TTT GTC ACA GAA 347
Gly Leu Ala Thr Phe Val Ile Ile Leu Leu Leu Val Phe Val Thr Glu
10 15 20

GTT GTT GTA GTG GTT TTG GGA TAT GTT TAC AGA GCA AAG GTG GAA AAT 395
Val Val Val Val Val Leu Gly Tyr Val Tyr Arg Ala Lys Val Glu Asn
25 30 35

GAG GTT GAT CGC AGC ATT CAG AAA GTG TAT AAG 428
Glu Val Asp Arg Ser Ile Gln Lys Val Tyr Lys
40 45

(2) INFORMATION FOR SEQ ID NO: 260:

(i) SEQUENCE CHARACTERISTICS:
(A) LENGTH: 425 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 167..425
(C) IDENTIFICATION METHOD: blastn
(D) OTHER INFORMATION: identity 97
region 106..364
id N39913
est

(ix) FEATURE:
(A) NAME/KEY: other
(B) LOCATION: 63..170
(C) IDENTIFICATION METHOD: blastn
(D) OTHER INFORMATION: identity 98
region 1..108
id N39913
est

(ix) FEATURE:
(A) NAME/KEY: other
(B) LOCATION: 61..188
(C) IDENTIFICATION METHOD: blastn
(D) OTHER INFORMATION: identity 96
region 39..166
id HUM527C01B
est

(ix) FEATURE:
(A) NAME/KEY: other
(B) LOCATION: 188..303
(C) IDENTIFICATION METHOD: blastn
(D) OTHER INFORMATION: identity 94
region 165..280
id HUM527C01B
est

(ix) FEATURE:
(A) NAME/KEY: other
(B) LOCATION: 24..61
(C) IDENTIFICATION METHOD: blastn
(D) OTHER INFORMATION: identity 100
region 1..38
id HUM527C01B
est

(ix) FEATURE:
(A) NAME/KEY: sig_peptide
(B) LOCATION: 81..275
(C) IDENTIFICATION METHOD: Von Heijne matrix
(D) OTHER INFORMATION: score 7
seq IGHFLCLVILVYC/AE

(xi) SEQUENCE DESCRIPTION: SEQ ID NO: 260:

AAGAGGATTT GGCGCCCTCC TCTGTGGATT CTGGCCAGGC CGGGTTCGGC GGTTGCTGTG 60

AGAGCGGGCT TCCCAACACC ATG CCG KCC GCC TTC TCT GTC AGC TCT TTC CCC 113
Met Pro Xaa Ala Phe Ser Val Ser Ser Phe Pro
-65 -60 -55

GTC AGC ATC CCA GCC GTG CTC ACG CAG ACG GAC TGG ACT GAG CCC TGG 161
Val Ser Ile Pro Ala Val Leu Thr Gln Thr Asp Trp Thr Glu Pro Trp
-50 -45 -40

CTC ATG GGG CTG GCC ACC TTC CAC GCG CTC TGC GTG CTC CTC ACC TGC 209
Leu Met Gly Leu Ala Thr Phe His Ala Leu Cys Val Leu Leu Thr Cys
-35 -30 -25

TTG TCC TCC CGA AGC TAC AGA CTA CAG ATC GGG CAC TTT CTG TGT CTA 257
Leu Ser Ser Arg Ser Tyr Arg Leu Gln Ile Gly His Phe Leu Cys Leu
-20 -15 -10

GTC ATC TTA GTC TAC TGT GCT GAA TAC ATC AAT GAG GCG GCT GCG ATG 305
Val Ile Leu Val Tyr Cys Ala Glu Tyr Ile Asn Glu Ala Ala Ala Met
-5 1 5 10

AAC TGG AGA TTA TTT TCG MAA TAC CAG TAT TTC GAC TCC AGG GGG ATG 353
Asn Trp Arg Leu Phe Ser Xaa Tyr Gln Tyr Phe Asp Ser Arg Gly Met
15 20 25

TTC ATT TCT ATA GTA TTT TCA GCC CCA CTG CTG GTG AAT GCC ATG ATC 401
Phe Ile Ser Ile Val Phe Ser Ala Pro Leu Leu Val Asn Ala Met Ile
30 35 40

ATT GTG GTT ATG TGG GTA TGG AAG 425
Ile Val Val Met Trp Val Trp Lys
45 50

(2) INFORMATION FOR SEQ ID NO: 261:

(i) SEQUENCE CHARACTERISTICS:
(A) LENGTH: 213 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
     (A) NAME/KEY: other
     (B) LOCATION: 133..165
     (C) IDENTIFICATION METHOD: blastn
     (D) OTHER INFORMATION:  identity 90
                             region 11..43
                             id HUM153A05B
                             est

(ix) FEATURE:
     (A) NAME/KEY: sig_peptide
     (B) LOCATION: 136..177
     (C) IDENTIFICATION METHOD: Von Heijne matrix
     (D) OTHER INFORMATION:  score 6.7
                             seq LLLSLFFPLRISL/SP

(xi) SEQUENCE DESCRIPTION: SEQ ID NO: 261:

ATTTTTCTCC GGTACAGCCT GGGAACGTAG GTCCCGCGCC TGTGATAAGT AAGGTTGGAT   60

TTTCTCTTCC CTGAGGTGAA GGATGCCCGG RAGSCCTCGG CAGGACCGCG CGGAAACGGG  120

CCTTCTGCCC AAAAG ATG CTG CTT CTC TCC TTA TTC TTT CCC CTC AGA ATC   171
                 Met Leu Leu Leu Ser Leu Phe Phe Pro Leu Arg Ile
                                 -10                 -5

TCG CTG TCT CCT TCC AAC CAC CTG TGG TCG GCA TCC TCC GGG            213
Ser Leu Ser Pro Ser Asn His Leu Trp Ser Ala Ser Ser Gly
        1               5                   10


(2) INFORMATION FOR SEQ ID NO: 262:

(i) SEQUENCE CHARACTERISTICS:
     (A) LENGTH: 321 base pairs
     (B) TYPE: NUCLEIC ACID
     (C) STRANDEDNESS: DOUBLE
     (D) TOPOLOGY: LINEAR

(ii) MOLECULE TYPE: CDNA

(vi) ORIGINAL SOURCE:
     (A) ORGANISM: Homo Sapiens
     (D) DEVELOPMENTAL STAGE: Fetal
     (F) TISSUE TYPE: kidney

(ix) FEATURE:
     (A) NAME/KEY: other
     (B) LOCATION: 16..319
     (C) IDENTIFICATION METHOD: blastn
     (D) OTHER INFORMATION:  identity 98
                             region 1..304
                             id HSC26A021
                             est

(ix) FEATURE:
     (A) NAME/KEY: other
     (B) LOCATION: 17..174

(C) IDENTIFICATION METHOD: blastn
(D) OTHER INFORMATION: identity 96
region 1..158
id W07871
est

(ix) FEATURE:
(A) NAME/KEY: other
(B) LOCATION: 205..319
(C) IDENTIFICATION METHOD: blastn
(D) OTHER INFORMATION: identity 97
region 192..306
id W07871
est

(ix) FEATURE:
(A) NAME/KEY: other
(B) LOCATION: 174..203
(C) IDENTIFICATION METHOD: blastn
(D) OTHER INFORMATION: identity 96
region 159..188
id W07871
est

(ix) FEATURE:
(A) NAME/KEY: other
(B) LOCATION: 169..305
(C) IDENTIFICATION METHOD: blastn
(D) OTHER INFORMATION: identity 98
region 144..280
id T75539
est

(ix) FEATURE:
(A) NAME/KEY: other
(B) LOCATION: 64..172
(C) IDENTIFICATION METHOD: blastn
(D) OTHER INFORMATION: identity 94
region 41..149
id T75539
est

(ix) FEATURE:
(A) NAME/KEY: other
(B) LOCATION: 175..319
(C) IDENTIFICATION METHOD: blastn
(D) OTHER INFORMATION: identity 100
region 161..305
id H94774
est

(ix) FEATURE:
(A) NAME/KEY: other
(B) LOCATION: 24..165
(C) IDENTIFICATION METHOD: blastn
(D) OTHER INFORMATION: identity 94
region 10..151
id H94774
est

(ix) FEATURE:

(A) NAME/KEY: other
(B) LOCATION: 228..319
(C) IDENTIFICATION METHOD: blastn
(D) OTHER INFORMATION: identity 93
region 203..294
id W89738
est

(ix) FEATURE:
(A) NAME/KEY: other
(B) LOCATION: 43..102
(C) IDENTIFICATION METHOD: blastn
(D) OTHER INFORMATION: identity 91
region 22..81
id W89738
est

(ix) FEATURE:
(A) NAME/KEY: sig_peptide
(B) LOCATION: 82..150
(C) IDENTIFICATION METHOD: Von Heijne matrix
(D) OTHER INFORMATION: score 6.6
seq LILVLQLLLRIRR/NR

(xi) SEQUENCE DESCRIPTION: SEQ ID NO: 262:

ACTCGCACCC GGAACAACAA AGCAAGGAAG ACGGAGTCCG AGCCTCGGGG GCTCCTAGCA 60

ACGGGCCGGG GCGGGAGTTC C ATG GAG ACT GGG GAG CGC GCC CGT CTC ATC 111
Met Glu Thr Gly Glu Arg Ala Arg Leu Ile
-20 -15

CTC ATC CTT GTC CTC CAG CTT CTC CTT CGC ATC CGA CGC AAC CGG CAG 159
Leu Ile Leu Val Leu Gln Leu Leu Leu Arg Ile Arg Arg Asn Arg Gln
-10 -5 1

CAG CGC TGC SCC GCG TCC TCA GCC ACC GCT CCC TCT TCC CAC GGA TGT 207
Gln Arg Cys Xaa Ala Ser Ser Ala Thr Ala Pro Ser Ser His Gly Cys
                5                   10                  15

GAT CTT CGT GGT GGA AAG CTA AAT TTT AAA ACC ACC CCA ATG GAT GCA 255
Asp Leu Arg Gly Gly Lys Leu Asn Phe Lys Thr Thr Pro Met Asp Ala
20 25 30 35

GAC AGT GAT GTT GCA TTG GAC ATT CTA ATT ACA AAT GTA GTC TGT GTT 303
Asp Ser Asp Val Ala Leu Asp Ile Leu Ile Thr Asn Val Val Cys Val
40 45 50

TTT AGA ACA AGA TGT CGG 321
Phe Arg Thr Arg Cys Arg
55

(2) INFORMATION FOR SEQ ID NO: 263:

(i) SEQUENCE CHARACTERISTICS:
(A) LENGTH: 325 base pairs
(B) TYPE: NUCLEIC ACID
(C) STRANDEDNESS: DOUBLE

(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 2..88
(C) IDENTIFICATION METHOD: blastn
(D) OTHER INFORMATION: identity 96
region 18..104
id R56970
est

(ix) FEATURE:
(A) NAME/KEY: sig_peptide
(B) LOCATION: 128..250
(C) IDENTIFICATION METHOD: Von Heijne matrix
(D) OTHER INFORMATION: score 6.4
seq ILGCSSVCQLCTG/RQ

(xi) SEQUENCE DESCRIPTION: SEQ ID NO: 263:

AGGAGTTAAG AAATGTCGTT CTTCAGATTT AAAAAGAAAA CCTTTACTGA ATCAGCTGAG 60

TGTTAATAAT ACGAATTTCC TTKTCNTGCC AATKCDRMYC TGRDDCAGRA RATCSNWGAA 120

CAGGGWT ATG TGT GGA TTW YAG TTT TCT CTG CCT TGC CTA CGA CTG TTT 169
Met Cys Gly Xaa Xaa Phe Ser Leu Pro Cys Leu Arg Leu Phe
-40 -35 -30

CTG GTT GTT ACC TGT TAT CKT TTA TTA TTA CTC CAC AAA GAA ATA CTT 217
Leu Val Val Thr Cys Tyr Xaa Leu Leu Leu Leu His Lys Glu Ile Leu
-25 -20 -15

GGA TGT TCG TCT GTT TGT CAG CTC TGC ACT GGG AGA CAA ATT AAC TGC 265
Gly Cys Ser Ser Val Cys Gln Leu Cys Thr Gly Arg Gln Ile Asn Cys
-10 -5 1 5

CGT AAC TTA GGC CTT TCG AGT ATT CTA AGA ATT TTC CTG AAA GTA CAG 313
Arg Asn Leu Gly Leu Ser Ser Ile Leu Arg Ile Phe Leu Lys Val Gln
10 15 20

TTT TTC TGT ATC 325
Phe Phe Cys Ile
25

(2) INFORMATION FOR SEQ ID NO: 264:

(i) SEQUENCE CHARACTERISTICS:
(A) LENGTH: 366 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 140..316
(C) IDENTIFICATION METHOD: blastn
(D) OTHER INFORMATION: identity 98
region 176..352
id W42809
est

(ix) FEATURE:
(A) NAME/KEY: other
(B) LOCATION: 14..129
(C) IDENTIFICATION METHOD: blastn
(D) OTHER INFORMATION: identity 97
region 50..165
id W42809
est

(ix) FEATURE:
(A) NAME/KEY: other
(B) LOCATION: 140..242
(C) IDENTIFICATION METHOD: blastn
(D) OTHER INFORMATION: identity 97
region 116..218
id N99674
est

(ix) FEATURE:
(A) NAME/KEY: other
(B) LOCATION: 58..129
(C) IDENTIFICATION METHOD: blastn
(D) OTHER INFORMATION: identity 100
region 34..105
id N99674
est

(ix) FEATURE:
(A) NAME/KEY: other
(B) LOCATION: 243..285
(C) IDENTIFICATION METHOD: blastn
(D) OTHER INFORMATION: identity 100
region 218..260
id N99674
est

(ix) FEATURE:
(A) NAME/KEY: other
(B) LOCATION: 27..57
(C) IDENTIFICATION METHOD: blastn
(D) OTHER INFORMATION: identity 100
region 2..32
id N99674
est

(ix) FEATURE:
(A) NAME/KEY: other
(B) LOCATION: 140..272
(C) IDENTIFICATION METHOD: blastn
(D) OTHER INFORMATION: identity 96
region 78..210
id R20073
est

(ix) FEATURE:
(A) NAME/KEY: other
(B) LOCATION: 267..364
(C) IDENTIFICATION METHOD: blastn
(D) OTHER INFORMATION: identity 96
region 206..303
id R20073
est

(ix) FEATURE:
(A) NAME/KEY: other
(B) LOCATION: 63..129
(C) IDENTIFICATION METHOD: blastn
(D) OTHER INFORMATION: identity 100
region 1..67
id R20073
est

(ix) FEATURE:
(A) NAME/KEY: other
(B) LOCATION: 35..139
(C) IDENTIFICATION METHOD: blastn
(D) OTHER INFORMATION: identity 98
region 1..105
id N99685
est

(ix) FEATURE:
(A) NAME/KEY: other
(B) LOCATION: 140..242
(C) IDENTIFICATION METHOD: blastn
(D) OTHER INFORMATION: identity 97
region 105..207
id N99685
est

(ix) FEATURE:
(A) NAME/KEY: other
(B) LOCATION: 286..316
(C) IDENTIFICATION METHOD: blastn
(D) OTHER INFORMATION: identity 100
region 251..281
id N99685
est

(ix) FEATURE:
(A) NAME/KEY: other
(B) LOCATION: 6..139
(C) IDENTIFICATION METHOD: blastn
(D) OTHER INFORMATION: identity 98
region 1..134

id AA154228
est

(ix) FEATURE:
(A) NAME/KEY: other
(B) LOCATION: 140..206
(C) IDENTIFICATION METHOD: blastn
(D) OTHER INFORMATION: identity 95
region 134..200
id AA154228
est

(ix) FEATURE:
(A) NAME/KEY: sig_peptide
(B) LOCATION: 10..228
(C) IDENTIFICATION METHOD: Von Heijne matrix
(D) OTHER INFORMATION: score 6.4
seq ACCFLSAFSPTLT/KS

(xi) SEQUENCE DESCRIPTION: SEQ ID NO: 264:

ATAATAAAA ATG AAC CCC GTT ACA GAG TCA CCA TCA TGT CTC TTC TCA CCA 51
Met Asn Pro Val Thr Glu Ser Pro Ser Cys Leu Phe Ser Pro
-70 -65 -60

CCC TCT GAA TCT GCA TTA GCC AGT CAA CTA GCC CTT TCA GCG TCA TGT 99
Pro Ser Glu Ser Ala Leu Ala Ser Gln Leu Ala Leu Ser Ala Ser Cys
-55 -50 -45

GAC CAG CGC GCC CCA TTC AGC TTG GCT GGT GTC GKT TCA MMA KRA CCC 147
Asp Gln Arg Ala Pro Phe Ser Leu Ala Gly Val Xaa Ser Xaa Xaa Pro
-40 -35 -30

AGG CTG GCC AGT CGT CAG GTT GCA CCG CCC TTT GGT TCC CGA GCA TGC 195
Arg Leu Ala Ser Arg Gln Val Ala Pro Pro Phe Gly Ser Arg Ala Cys
-25 -20 -15

TGT TTT CTC TCA GCC TTC TCT CCA ACC TTA ACC AAA TCG GCA GCA GCC 243
Cys Phe Leu Ser Ala Phe Ser Pro Thr Leu Thr Lys Ser Ala Ala Ala
-10 -5 1 5

ACC TCG ACC GCC CAC ACA TTC CTG GCC AAT CAG CTC AGC TGT TTA TTT 291
Thr Ser Thr Ala His Thr Phe Leu Ala Asn Gln Leu Ser Cys Leu Phe
10 15 20

ACC AAA TGT CTT CAC AAC AAC TAC AGC AGC AGC CTT CGG CTA ACA AAA 339
Thr Lys Cys Leu His Asn Asn Tyr Ser Ser Ser Leu Arg Leu Thr Lys
25 30 35

AAG CAG GAA AAA TCC ACA ACA CCC CAG 366
Lys Gln Glu Lys Ser Thr Thr Pro Gln
40 45

(2) INFORMATION FOR SEQ ID NO: 265:

(i) SEQUENCE CHARACTERISTICS:
(A) LENGTH: 114 base pairs
(B) TYPE: NUCLEIC ACID

(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: other
(B) LOCATION: 2..86
(C) IDENTIFICATION METHOD: blastn
(D) OTHER INFORMATION: identity 96
region 8..92
id AA070287
est

(ix) FEATURE:
(A) NAME/KEY: other
(B) LOCATION: 15..80
(C) IDENTIFICATION METHOD: blastn
(D) OTHER INFORMATION: identity 100
region 1..66
id T10748
est

(ix) FEATURE:
(A) NAME/KEY: other
(B) LOCATION: 22..88
(C) IDENTIFICATION METHOD: blastn
(D) OTHER INFORMATION: identity 100
region 17..83
id N67981
est

(ix) FEATURE:
(A) NAME/KEY: other
(B) LOCATION: 21..85
(C) IDENTIFICATION METHOD: blastn
(D) OTHER INFORMATION: identity 100
region 17..81
id AA069568
est

(ix) FEATURE:
(A) NAME/KEY: sig_peptide
(B) LOCATION: 25..87
(C) IDENTIFICATION METHOD: Von Heijne matrix
(D) OTHER INFORMATION: score 6.3
seq LGLSVLLTAATVA/GV

(xi) SEQUENCE DESCRIPTION: SEQ ID NO: 265:

AAGGCCGCGG CCGCCAGCGT GGGG ATG TCT AGG AGC TCG AAG GTG GTG CTG 51
Met Ser Arg Ser Ser Lys Val Val Leu
-20 -15

GGC CTC TCG GTG CTG CTG ACG GCG GCC ACA GTG GCC GGC GTA CAT GTG 99
Gly Leu Ser Val Leu Leu Thr Ala Ala Thr Val Ala Gly Val His Val

-10                    -5                    1

AAG CAG CAG TGG GAC                                                    114
Lys Gln Gln Trp Asp
  5

(2) INFORMATION FOR SEQ ID NO: 266:

(i) SEQUENCE CHARACTERISTICS:
(A) LENGTH: 204 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: other
(B) LOCATION: 1..197
(C) IDENTIFICATION METHOD: blastn
(D) OTHER INFORMATION:  identity 99
                        region 8..204
                        id H10448
                        est

(ix) FEATURE:
(A) NAME/KEY: other
(B) LOCATION: 5..197
(C) IDENTIFICATION METHOD: blastn
(D) OTHER INFORMATION:  identity 98
                        region 1..193
                        id AA127134
                        est

(ix) FEATURE:
(A) NAME/KEY: other
(B) LOCATION: 5..197
(C) IDENTIFICATION METHOD: blastn
(D) OTHER INFORMATION:  identity 98
                        region 1..193
                        id HUML13653
                        est

(ix) FEATURE:
(A) NAME/KEY: other
(B) LOCATION: 1..197
(C) IDENTIFICATION METHOD: blastn
(D) OTHER INFORMATION:  identity 95
                        region 9..205
                        id HSC18H071
                        est

(ix) FEATURE:
(A) NAME/KEY: other
(B) LOCATION: 34..197

(C) IDENTIFICATION METHOD: blastn
(D) OTHER INFORMATION:  identity 96
                        region 13..176
                        id AA194682
                        est

(ix) FEATURE:
     (A) NAME/KEY: sig_peptide
     (B) LOCATION: 31..108
     (C) IDENTIFICATION METHOD: Von Heijne matrix
     (D) OTHER INFORMATION:  score 6.3
                             seq GVGLVTLLGLAVG/SY

(xi) SEQUENCE DESCRIPTION: SEQ ID NO: 266:

GTCAGGTGGT GGAGGAAAAG GCGCTCCGTC ATG GGG ATC CAG ACG AGC CCC GTC     54
                                 Met Gly Ile Gln Thr Ser Pro Val
                                     -25                 -20

CTG CTG GCC TCC CTG GGG GTG GGG CTG GTC ACT CTG CTC GGC CTG GCT     102
Leu Leu Ala Ser Leu Gly Val Gly Leu Val Thr Leu Leu Gly Leu Ala
            -15                 -10                 -5

GTG GGC TCC TAC TTG GTT CGG AGG TCC CGC CGG CCT CAG GTC ACT CTC     150
Val Gly Ser Tyr Leu Val Arg Arg Ser Arg Arg Pro Gln Val Thr Leu
        1               5                   10

CTG GAC CCC AGT GAA AAG TAC CTG CTA CGA CTG CTA GAC AAG ACG ACC     198
Leu Asp Pro Ser Glu Lys Tyr Leu Leu Arg Leu Leu Asp Lys Thr Thr
 15                 20                  25                  30

CCC GGG                                                             204
Pro Gly

(2) INFORMATION FOR SEQ ID NO: 267:

(i) SEQUENCE CHARACTERISTICS:
     (A) LENGTH: 340 base pairs
     (B) TYPE: NUCLEIC ACID
     (C) STRANDEDNESS: DOUBLE
     (D) TOPOLOGY: LINEAR

(ii) MOLECULE TYPE: CDNA

(vi) ORIGINAL SOURCE:
     (A) ORGANISM: Homo Sapiens
     (F) TISSUE TYPE: Muscle

(ix) FEATURE:
     (A) NAME/KEY: other
     (B) LOCATION: 33..227
     (C) IDENTIFICATION METHOD: blastn
     (D) OTHER INFORMATION:  identity 95
                             region 1..195
                             id W00881
                             est

(ix) FEATURE:

(A) NAME/KEY: sig_peptide
(B) LOCATION: 167..319
(C) IDENTIFICATION METHOD: Von Heijne matrix
(D) OTHER INFORMATION:  score 6.2
                        seq VLLLSSAXLVXXS/SP

(xi) SEQUENCE DESCRIPTION: SEQ ID NO: 267:

CATTTGCTCT TCTCTTAACT CCTACCTGAA AACCCCATTC CTAAATTATT CACTATATTT   60

CAGACTTCTT CACTCTTCTC CMAAAACCTG AATCAGCTTG TGCTGATTTT TTCCTATCTG  120

CTATCCCTAA AAGGACTAGA CCTTCTTTCT ATCCTTACTC CCCTCA ATG TAT CCA     175
                                                   Met Tyr Pro
                                                       -50

TCT TAC CTC TTG ATT KKS CCT CCC ATT CCC TCA CAG TTC CTG AAA CAG     223
Ser Tyr Leu Leu Ile Xaa Pro Pro Ile Pro Ser Gln Phe Leu Lys Gln
            -45                 -40                 -35

TGC SCC CCC CCG ACC CTA AGC GAC CCC TTT CTG CCC CTG GCC TTG AGG     271
Cys Xaa Pro Pro Thr Leu Ser Asp Pro Phe Leu Pro Leu Ala Leu Arg
        -30                 -25                 -20

TCC CTT GAC GTG CTG CTC CTG TCT TCT GCT CNB YTA GTB VVC NAT TCC     319
Ser Leu Asp Val Leu Leu Leu Ser Ser Ala Xaa Leu Val Xaa Xaa Ser
    -15                 -10                 -5

TCT CCC TTG GAA TTC ATC AGA                                         340
Ser Pro Leu Glu Phe Ile Arg
  1               5

(2) INFORMATION FOR SEQ ID NO: 268:

(i) SEQUENCE CHARACTERISTICS:
    (A) LENGTH: 368 base pairs
    (B) TYPE: NUCLEIC ACID
    (C) STRANDEDNESS: DOUBLE
    (D) TOPOLOGY: LINEAR

(ii) MOLECULE TYPE: CDNA

(vi) ORIGINAL SOURCE:
    (A) ORGANISM: Homo Sapiens
    (D) DEVELOPMENTAL STAGE: Fetal
    (F) TISSUE TYPE: kidney

(ix) FEATURE:
    (A) NAME/KEY: other
    (B) LOCATION: 253..332
    (C) IDENTIFICATION METHOD: blastn
    (D) OTHER INFORMATION:  identity 90
                            region 159..238
                            id AA114672
                            est

(ix) FEATURE:
    (A) NAME/KEY: sig_peptide

(B) LOCATION: 195..293
(C) IDENTIFICATION METHOD: Von Heijne matrix
(D) OTHER INFORMATION: score 6.2
seq ILLLXTFQTWCLR/IS

(xi) SEQUENCE DESCRIPTION: SEQ ID NO: 268:

AGGGGTACCT GGTCGTCATG GCAGGCGGTA TTGACCGAAG AGCTTGRTGA GGAAGAGCAG 60

CTGCTGAGAA GGCATCGCAA AKAGAAGAAG GAGTTGCAAS CCAAAATTCA GGGCATGAAG 120

AATGCTGTTC CCAAGAATGA CAATGAAGAG GDAGGARGCA GCTCACCGRA GATGTGGCCA 180

AGTTGGAAAA AGAW ATG GAA CAG AAA CAY AGA GAS GAA CTG GAG CAA TTG 230
Met Glu Gln Lys His Arg Xaa Glu Leu Glu Gln Leu
-30 -25

AAG CTG RCT ACT AAG GAG AAT AAG ATT CTG TTG CTG YWA ACA TTT CAA 278
Lys Leu Xaa Thr Lys Glu Asn Lys Ile Leu Leu Leu Xaa Thr Phe Gln
-20 -15 -10

ACT TGG TGC TTG AGA ATC AGC CAC CTC GGA TAT CAR AAG CAC AWA AGA 326
Thr Trp Cys Leu Arg Ile Ser His Leu Gly Tyr Gln Lys His Xaa Arg
-5 1 5 10

GRC GGG TGC CTG GAT MSA AGG AGC TCT CTG TGT TGT CCT TGG 368
Xaa Gly Cys Leu Asp Xaa Arg Ser Ser Leu Cys Cys Pro Trp
15 20 25

(2) INFORMATION FOR SEQ ID NO: 269:

(i) SEQUENCE CHARACTERISTICS:
(A) LENGTH: 398 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: complement(1..43)
(C) IDENTIFICATION METHOD: blastn
(D) OTHER INFORMATION: identity 90
region 209..251
id AA013573
est

(ix) FEATURE:
(A) NAME/KEY: other
(B) LOCATION: complement(1..43)
(C) IDENTIFICATION METHOD: blastn
(D) OTHER INFORMATION: identity 90

region 153..195
id AA014924
est

(ix) FEATURE:
(A) NAME/KEY: sig_peptide
(B) LOCATION: 54..122
(C) IDENTIFICATION METHOD: Von Heijne matrix
(D) OTHER INFORMATION: score 5.9
seq TLKFLTLLQKSNA/KR

(xi) SEQUENCE DESCRIPTION: SEQ ID NO: 269:

AGACGAAGCT CGATGAAGAT TTAGAGAGTT CCAGTGAATC CGATGTGAGT CTG ATG 56
Met

ATG ACA GCA CCT GTT CTA GCA GCT CAG ACT CTG AAG TTT TTG ACG TTA 104
Met Thr Ala Pro Val Leu Ala Ala Gln Thr Leu Lys Phe Leu Thr Leu
-20 -15 -10

TTG CAG AAA TCA AAC GCA AAA AGG SCC AAC CTT GAC CGA CTT CAT GAT 152
Leu Gln Lys Ser Asn Ala Lys Arg Xaa Asn Leu Asp Arg Leu His Asp
-5 1 5 10

GAA CTT TGG TAC AAC GAT CCA GGC CAG ATG AAT GAT GGA CCA CTC TGC 200
Glu Leu Trp Tyr Asn Asp Pro Gly Gln Met Asn Asp Gly Pro Leu Cys
15 20 25

AAA TGC AGC GCA AAG GCA AGA CGC ACA GGA ATT AGG CAC AGC ATT TAT 248
Lys Cys Ser Ala Lys Ala Arg Arg Thr Gly Ile Arg His Ser Ile Tyr
30 35 40

CCT GGA GAA GAG GCC ATC AAG CCC TGT CGT CCT ATG ACC AAC AAT GCT 296
Pro Gly Glu Glu Ala Ile Lys Pro Cys Arg Pro Met Thr Asn Asn Ala
45 50 55

GGC AGA CTT TTC CAC TAC CGG ATC ACA GTM TCC CCG CCT ACG AAC TTT 344
Gly Arg Leu Phe His Tyr Arg Ile Thr Val Ser Pro Pro Thr Asn Phe
60 65 70

TTA ACT GAC AGG CCA ACT GTT ATA GAA TAC GAT GAT CAC GAG TAT ATC 392
Leu Thr Asp Arg Pro Thr Val Ile Glu Tyr Asp Asp His Glu Tyr Ile
75 80 85 90

TTT GAA 398
Phe Glu

(2) INFORMATION FOR SEQ ID NO: 270:

(i) SEQUENCE CHARACTERISTICS:
(A) LENGTH: 359 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:

(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 105..208
(C) IDENTIFICATION METHOD: blastn
(D) OTHER INFORMATION: identity 97
region 81..184
id N51797
est

(ix) FEATURE:
(A) NAME/KEY: other
(B) LOCATION: 30..110
(C) IDENTIFICATION METHOD: blastn
(D) OTHER INFORMATION: identity 97
region 7..87
id N51797
est

(ix) FEATURE:
(A) NAME/KEY: sig_peptide
(B) LOCATION: 54..134
(C) IDENTIFICATION METHOD: Von Heijne matrix
(D) OTHER INFORMATION: score 5.9
seq ALALAXAPDLAQA/PL

(xi) SEQUENCE DESCRIPTION: SEQ ID NO: 270:

AGTGCAGAAG GTTCTGGGAA GTAGGAGACC CCACTGGCTT TGGTCCCCTA AGA ATG 56
Met

GAC TCT GCT GCC TGT GCT GCT GCT GCC ACC CCT GTT CCA GCC CTG GCT 104
Asp Ser Ala Ala Cys Ala Ala Ala Ala Thr Pro Val Pro Ala Leu Ala
-25 -20 -15

TTG GCC HTA GCT CCA GAC CTA GCA CAA GCC CCA CTG GCA CTC CCT GGC 152
Leu Ala Xaa Ala Pro Asp Leu Ala Gln Ala Pro Leu Ala Leu Pro Gly
-10 -5 1 5

CTG TTA AGC CCA TCT TGC CTT CTC TCC TCT GGA CAA GAA GTA AAT GGG 200
Leu Leu Ser Pro Ser Cys Leu Leu Ser Ser Gly Gln Glu Val Asn Gly
10 15 20

AGT GAA AGA GGA ACT TGT CTC TGG AGG CCC TGG CTG TCT TCC ACA AAT 248
Ser Glu Arg Gly Thr Cys Leu Trp Arg Pro Trp Leu Ser Ser Thr Asn
25 30 35

GAC TCC CCA AGG CAG ATG AGG AAG CTG GTG GAT TTG GCT GCT GGT GGG 296
Asp Ser Pro Arg Gln Met Arg Lys Leu Val Asp Leu Ala Ala Gly Gly
40 45 50

GCA ACG GCT GCT GAG GTC ACC AAG GCT GAA TCC ATR NTC CAT CAC CCT 344
Ala Thr Ala Ala Glu Val Thr Lys Ala Glu Ser Xaa Xaa His His Pro
55 60 65 70

GTC AGG CTC TTC TGG 359
Val Arg Leu Phe Trp

75

(2) INFORMATION FOR SEQ ID NO: 271:

(i) SEQUENCE CHARACTERISTICS:
(A) LENGTH: 405 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 2..304
(C) IDENTIFICATION METHOD: blastn
(D) OTHER INFORMATION: identity 96
region 15..317
id T86266
est

(ix) FEATURE:
(A) NAME/KEY: sig_peptide
(B) LOCATION: 64..135
(C) IDENTIFICATION METHOD: Von Heijne matrix
(D) OTHER INFORMATION: score 5.7
seq ILGLLGLLGTLVA/ML

(xi) SEQUENCE DESCRIPTION: SEQ ID NO: 271:

AAAGAGCTTC AGCCTGAAGA CAAGGGAGCA GTCCCTGAAG ACGCTTCTAC TGAGAGGTCT 60

GCC ATG GCC TCT CTT GGC CTC CAA CTT GTG GGC TAC ATC CTA GGC CTT 108
Met Ala Ser Leu Gly Leu Gln Leu Val Gly Tyr Ile Leu Gly Leu
-20 -15 -10

CTG GGG CTT TTG GGS ACA CTG GTT GCC ATG CTG CTC CCC AGC TGG AAA 156
Leu Gly Leu Leu Gly Thr Leu Val Ala Met Leu Leu Pro Ser Trp Lys
-5 1 5

ACA AGT TCT TAT GTC GGT GCC AGC ATT GTG ACA GCA GTT GGC TTC TCC 204
Thr Ser Ser Tyr Val Gly Ala Ser Ile Val Thr Ala Val Gly Phe Ser
10 15 20

AAG GGC CTC TGG ATG GAA TGT GCC ACA YAC AGC ACA GGC ATC ACC CAG 252
Lys Gly Leu Trp Met Glu Cys Ala Thr Xaa Ser Thr Gly Ile Thr Gln
25 30 35

TGT GAC ATC TAT AGC ACC CTT CTG GGC CTG CCC GCT GAC ATC CAG GCT 300
Cys Asp Ile Tyr Ser Thr Leu Leu Gly Leu Pro Ala Asp Ile Gln Ala
40 45 50 55

GCC CAG GCC ATG ATG GTG ACA TCC AGT GCA ATC TCC TCC CTG GCC TGC 348

Ala Gln Ala Met Met Val Thr Ser Ser Ala Ile Ser Ser Leu Ala Cys
                60                  65                  70

ATT ATC TCT GTG GTG GGC ATG AGA TGC ACA GTC TTC TGC CAG GAA TCC     396
Ile Ile Ser Val Val Gly Met Arg Cys Thr Val Phe Cys Gln Glu Ser
            75                  80                  85

CGA GCC AGG                                                         405
Arg Ala Arg
        90

(2) INFORMATION FOR SEQ ID NO: 272:

(i) SEQUENCE CHARACTERISTICS:
(A) LENGTH: 324 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 98..326
(C) IDENTIFICATION METHOD: blastn
(D) OTHER INFORMATION: identity 100
region 15..243
id T86266
est

(ix) FEATURE:
(A) NAME/KEY: sig_peptide
(B) LOCATION: 160..231
(C) IDENTIFICATION METHOD: Von Heijne matrix
(D) OTHER INFORMATION: score 5.7
seq ILGLLGLLGTLVA/ML

(xi) SEQUENCE DESCRIPTION: SEQ ID NO: 272:

AGCTGCTTGT GGCCACCCAC AGACACTTGT AAGGAGGAGA GAAGTCAGCC TGGCAGAGAG    60

ACTCTGAAAT GASSGATTAG AGGTGTTCAA GGRAGCAAAG AGCTTCAGCC TGAAGACAAG   120

GGAGCAGTCC CTGAAGACGC TTCTACTGAG AGGTCTGCC ATG GCC TCT CTT GGC      174
                                           Met Ala Ser Leu Gly
                                                           -20

CTC CAA CTT GTG GGC TAC ATC CTA GGC CTT CTG GGG CTT TTG GGC ACA     222
Leu Gln Leu Val Gly Tyr Ile Leu Gly Leu Leu Gly Leu Leu Gly Thr
            -15                 -10                     -5

CTG GTT GCC ATG CTG CTC CCC AGC TGG AAA ACA AGT TCT TAT GTC GGT     270
Leu Val Ala Met Leu Leu Pro Ser Trp Lys Thr Ser Ser Tyr Val Gly

1                 5                 10

GCC AGC ATT GTG ACA GCA GTT GGC TTC TCC AAG GGC CTC TGG ATG GAA    318
Ala Ser Ile Val Thr Ala Val Gly Phe Ser Lys Gly Leu Trp Met Glu
     15                  20                  25

TGT GCC                                                            324
Cys Ala
 30

(2) INFORMATION FOR SEQ ID NO: 273:

(i) SEQUENCE CHARACTERISTICS:
(A) LENGTH: 397 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 95..260
(C) IDENTIFICATION METHOD: blastn
(D) OTHER INFORMATION:  identity 98
                        region 19..184
                        id AA132585
                        est

(ix) FEATURE:
(A) NAME/KEY: other
(B) LOCATION: 347..399
(C) IDENTIFICATION METHOD: blastn
(D) OTHER INFORMATION:  identity 100
                        region 2..54
                        id N57441
                        est

(ix) FEATURE:
(A) NAME/KEY: sig_peptide
(B) LOCATION: 272..325
(C) IDENTIFICATION METHOD: Von Heijne matrix
(D) OTHER INFORMATION:  score 5.6
                        seq LLCECLLLVAGYA/HD

(xi) SEQUENCE DESCRIPTION: SEQ ID NO: 273:

ACGCAGCCGT CAGCCGAACA ATTCGATGAC GAGGCCCAGG AAGCACGCTG AAACCCTGGG    6[?]

CGGCGGCAAG CTGTGCGACC TCTTCTGCGG CCGGCCTGGA CTAGCTTTAT CGTCATCTGG    12[?]

GAAATTGTTA AAAATGCAAA TTCGCAAGTT TGAGAGCCAT GGTTCCAAGA AACTGCATAA    1[?][?]

GCATACGAAA TAAGTTGCAG CCTCCCGWCT TATACCCTGG TACTTCTAGT CTAAAACAGG 240

ATTTGACTCT ACTAATCCAG CCTTATACAG G.ATG CTG TGT TCT TTG CTC CTT 292
Met Leu Cys Ser Leu Leu Leu
-15

TGT GAA TGT CTG TTG CTG GTA GCT GGT TAT GCT CAT GAT GAT GAC TGG 340
Cys Glu Cys Leu Leu Leu Val Ala Gly Tyr Ala His Asp Asp Asp Trp
-10 -5 1 5

ATT GAC CCC ACA GAC ATG CTT AAC TAT GAT GCT GCT TCA GGA ACA ATG 388
Ile Asp Pro Thr Asp Met Leu Asn Tyr Asp Ala Ala Ser Gly Thr Met
10 15 20

AGA AAA TCT 397
Arg Lys Ser

(2) INFORMATION FOR SEQ ID NO: 274:

(i) SEQUENCE CHARACTERISTICS:
(A) LENGTH: 96 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 1..42
(C) IDENTIFICATION METHOD: blastn
(D) OTHER INFORMATION: identity 90
region 14..55
id H32593
est

(ix) FEATURE:
(A) NAME/KEY: sig_peptide
(B) LOCATION: 22..87
(C) IDENTIFICATION METHOD: Von Heijne matrix
(D) OTHER INFORMATION: score 5.5
seq LWYVCPCPSGAWM/VP

(xi) SEQUENCE DESCRIPTION: SEQ ID NO: 274:

AGACGCTGCC CTTCCGCAGC G ATG GCA TCC CGG CTC TGT GGA GGG GCC CTC 51
Met Ala Ser Arg Leu Cys Gly Gly Ala Leu
-20 -15

TGG TAT GTG TGT CCC TGT CCT TCT GGG GCG TGG ATG GTK CCT GGG 96
Trp Tyr Val Cys Pro Cys Pro Ser Gly Ala Trp Met Val Pro Gly
-10 -5 1

(2) INFORMATION FOR SEQ ID NO: 275:

(i) SEQUENCE CHARACTERISTICS:
(A) LENGTH: 257 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: other
(B) LOCATION: 19..254
(C) IDENTIFICATION METHOD: blastn
(D) OTHER INFORMATION: identity 98
region 15..250
id H23844
est

(ix) FEATURE:
(A) NAME/KEY: other
(B) LOCATION: 25..254
(C) IDENTIFICATION METHOD: blastn
(D) OTHER INFORMATION: identity 99
region 8..237
id AA036876
est

(ix) FEATURE:
(A) NAME/KEY: other
(B) LOCATION: 24..254
(C) IDENTIFICATION METHOD: blastn
(D) OTHER INFORMATION: identity 97
region 21..251
id H22656
est

(ix) FEATURE:
(A) NAME/KEY: other
(B) LOCATION: 35..217
(C) IDENTIFICATION METHOD: blastn
(D) OTHER INFORMATION: identity 98
region 1..183
id W05714
est

(ix) FEATURE:
(A) NAME/KEY: other
(B) LOCATION: 218..254
(C) IDENTIFICATION METHOD: blastn
(D) OTHER INFORMATION: identity 100
region 183..219
id W05714
est

(ix) FEATURE:
(A) NAME/KEY: other
(B) LOCATION: 34..244
(C) IDENTIFICATION METHOD: blastn
(D) OTHER INFORMATION: identity 99
region 1..211
id AA100765
est

(ix) FEATURE:
(A) NAME/KEY: sig_peptide
(B) LOCATION: 69..152
(C) IDENTIFICATION METHOD: Von Heijne matrix
(D) OTHER INFORMATION: score 5.5
seq LGYLVLSEGAVLA/SS

(xi) SEQUENCE DESCRIPTION: SEQ ID NO: 275:

ACGTGACCGG GGCCTGAAGC CGGAAGCTAC CTATCTGGTA GGGAGCTCCC CCAGCACCGA 60

AGACTGCG ATG ACT TCT GCA CTG ACC CAG GGG CTG GAG CGA ATC CCA GAC 110
Met Thr Ser Ala Leu Thr Gln Gly Leu Glu Arg Ile Pro Asp
-25 -20 -15

CAG CTC GGC TAC CTG GTA CTG AGT GAA GGT GCA GTG CTG GCG TCA TCT 158
Gln Leu Gly Tyr Leu Val Leu Ser Glu Gly Ala Val Leu Ala Ser Ser
-10 -5 1

GGG GAC CTG GAG AAT GAT GAG CAG GCA DCC AGT GCC ATC TCT GAG CTG 206
Gly Asp Leu Glu Asn Asp Glu Gln Ala Xaa Ser Ala Ile Ser Glu Leu
                5                   10                  15

GTC AGC ACA GCC TGC GGT TTC CGG CTG CAC CGC GGC ATG AAT GTG CCC 254
Val Ser Thr Ala Cys Gly Phe Arg Leu His Arg Gly Met Asn Val Pro
            20                  25                  30

AGG 257
Arg
35

(2) INFORMATION FOR SEQ ID NO: 276:

(i) SEQUENCE CHARACTERISTICS:
(A) LENGTH: 254 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 9..243

(C) IDENTIFICATION METHOD: blastn
(D) OTHER INFORMATION: identity 96
region 6..245
id H64050
est

(ix) FEATURE:
(A) NAME/KEY: other
(B) LOCATION: 15..248
(C) IDENTIFICATION METHOD: blastn
(D) OTHER INFORMATION: identity 96
region 1..234
id R17172
est

(ix) FEATURE:
(A) NAME/KEY: other
(B) LOCATION: 14..248
(C) IDENTIFICATION METHOD: blastn
(D) OTHER INFORMATION: identity 94
region 1..235
id HSC15C081
est

(ix) FEATURE:
(A) NAME/KEY: other
(B) LOCATION: 22..248
(C) IDENTIFICATION METHOD: blastn
(D) OTHER INFORMATION: identity 96
region 1..227
id AA149663
est

(ix) FEATURE:
(A) NAME/KEY: other
(B) LOCATION: 43..248
(C) IDENTIFICATION METHOD: blastn
(D) OTHER INFORMATION: identity 94
region 29..234
id HSU46380
est

(ix) FEATURE:
(A) NAME/KEY: sig_peptide
(B) LOCATION: 24..149
(C) IDENTIFICATION METHOD: Von Heijne matrix
(D) OTHER INFORMATION: score 5.4
seq ITGVILLAVGIWG/KV

(xi) SEQUENCE DESCRIPTION: SEQ ID NO: 276:

AGGTGCAGGG TCTCGGGCTA GTC ATG GCG TCC CCG TCT CGG AGA CTG CAG ACT 53
Met Ala Ser Pro Ser Arg Arg Leu Gln Thr
-40 -35

AAA CCA GTC ATT ACT TGT TTC AAG AGC GTT CTG CTA ATC KAC ACT NTK 101
Lys Pro Val Ile Thr Cys Phe Lys Ser Val Leu Leu Ile Xaa Thr Xaa
-30 -25 -20

ATT TKC TGG ATC ACT GGC GTK ATC CTT CTT GCA GTT GGC ATT TGG GGC 149

Ile Xaa Trp Ile Thr Gly Val Ile Leu Leu Ala Val Gly Ile Trp Gly
    -15                 -10                 -5

AAG GTG AGC CTG GAG AAT TAC TTT KCK CTT TTA AAT GAG AAG GCC ACC     197
Lys Val Ser Leu Glu Asn Tyr Phe Xaa Leu Leu Asn Glu Lys Ala Thr
  1               5                  10                  15

AAT GTC CCC TTC GKG CTC ATT GCT ACT GGT ACC GTC ATK ATT CTT TTG     245
Asn Val Pro Phe Xaa Leu Ile Ala Thr Gly Thr Val Xaa Ile Leu Leu
             20                  25                  30

GGC TAC CGG                                                         254
Gly Tyr Arg
         35

(2) INFORMATION FOR SEQ ID NO: 277:

      (i) SEQUENCE CHARACTERISTICS:
           (A) LENGTH: 231 base pairs
           (B) TYPE: NUCLEIC ACID
           (C) STRANDEDNESS: DOUBLE
           (D) TOPOLOGY: LINEAR

      (ii) MOLECULE TYPE: CDNA

      (vi) ORIGINAL SOURCE:
           (A) ORGANISM: Homo Sapiens
           (D) DEVELOPMENTAL STAGE: Fetal
           (F) TISSUE TYPE: kidney

      (ix) FEATURE:
           (A) NAME/KEY: other
           (B) LOCATION: 1..228
           (C) IDENTIFICATION METHOD: blastn
           (D) OTHER INFORMATION:  identity 97
                                   region 19..246
                                   id HUMHG1206
                                   est

      (ix) FEATURE:
           (A) NAME/KEY: other
           (B) LOCATION: 1..222
           (C) IDENTIFICATION METHOD: blastn
           (D) OTHER INFORMATION:  identity 98
                                   region 1..222
                                   id C15962
                                   est

      (ix) FEATURE:
           (A) NAME/KEY: other
           (B) LOCATION: 37..222
           (C) IDENTIFICATION METHOD: blastn
           (D) OTHER INFORMATION:  identity 95
                                   region 35..220
                                   id HUM417F07B
                                   est

      (ix) FEATURE:
           (A) NAME/KEY: other

(B) LOCATION: 2[?].33
(C) IDENTIFICATION METHOD: blastn
(D) OTHER INFORMATION: identity 100
region 1..32
id HUM417F07B
est

(ix) FEATURE:
(A) NAME/KEY: other
(B) LOCATION: 59..228
(C) IDENTIFICATION METHOD: blastn
(D) OTHER INFORMATION: identity 95
region 18..187
id AA139623
est

(ix) FEATURE:
(A) NAME/KEY: other
(B) LOCATION: 94..178
(C) IDENTIFICATION METHOD: blastn
(D) OTHER INFORMATION: identity 96
region 1..85
id N88476
est

(ix) FEATURE:
(A) NAME/KEY: other
(B) LOCATION: 177..228
(C) IDENTIFICATION METHOD: blastn
(D) OTHER INFORMATION: identity 94
region 82..133
id N88476
est

(ix) FEATURE:
(A) NAME/KEY: sig_peptide
(B) LOCATION: 49..108
(C) IDENTIFICATION METHOD: Von Heijne matrix
(D) OTHER INFORMATION: score 5.3
seq VLLGSGLTILSQP/LM

(xi) SEQUENCE DESCRIPTION: SEQ ID NO: 277:

GTCGCTTGGT GGCTCCGTCT GTCTGTCCGT CCGCCCGCGG GTGCCATC ATG GCG GAC 57
Met Ala Asp
-20

GCG GCC AGT CAG GTG CTC CTG GGC TCC GGT CTC ACC ATC CTG TCC CAG 105
Ala Ala Ser Gln Val Leu Leu Gly Ser Gly Leu Thr Ile Leu Ser Gln
-15 -10 -5

CCG CTC ATG TAC GTG AAA GTG CTC ATC CAG GTG GGA TAT GAG CCT CTT 153
Pro Leu Met Tyr Val Lys Val Leu Ile Gln Val Gly Tyr Glu Pro Leu
1               5                   10                  15

CCT CCA ACA ATA GGA CGA AAT ATT TTT GGG CGG CAA GTG TGN YAG CTT 201
Pro Pro Thr Ile Gly Arg Asn Ile Phe Gly Arg Gln Val Xaa Xaa Leu
            20                  25                  30

CCT NGT CTC TTT AGT TAT GCT CAG CAC GGG 231

Pro Xaa Leu Phe Ser Tyr Ala Gln His Gly
            35                  40

(2) INFORMATION FOR SEQ ID NO: 278:

      (i) SEQUENCE CHARACTERISTICS:
            (A) LENGTH: 190 base pairs
            (B) TYPE: NUCLEIC ACID
            (C) STRANDEDNESS: DOUBLE
            (D) TOPOLOGY: LINEAR

      (ii) MOLECULE TYPE: CDNA

      (vi) ORIGINAL SOURCE:
            (A) ORGANISM: Homo Sapiens
            (F) TISSUE TYPE: Kidney

      (ix) FEATURE:
            (A) NAME/KEY: other
            (B) LOCATION: complement(2..185)
            (C) IDENTIFICATION METHOD: blastn
            (D) OTHER INFORMATION:  identity 93
                                    region 93..276
                                    id AA136898
                                    est

      (ix) FEATURE:
            (A) NAME/KEY: other
            (B) LOCATION: 43..89
            (C) IDENTIFICATION METHOD: blastn
            (D) OTHER INFORMATION:  identity 93
                                    region 30..76
                                    id W96077
                                    est

      (ix) FEATURE:
            (A) NAME/KEY: other
            (B) LOCATION: 125..161
            (C) IDENTIFICATION METHOD: blastn
            (D) OTHER INFORMATION:  identity 97
                                    region 110..146
                                    id W96077
                                    est

      (ix) FEATURE:
            (A) NAME/KEY: other
            (B) LOCATION: 83..119
            (C) IDENTIFICATION METHOD: blastn
            (D) OTHER INFORMATION:  identity 94
                                    region 69..105
                                    id W96077
                                    est

      (ix) FEATURE:
            (A) NAME/KEY: other
            (B) LOCATION: 15..49
            (C) IDENTIFICATION METHOD: blastn
            (D) OTHER INFORMATION:  identity 91
                                    region 1..35

id W96077
est

(ix) FEATURE:
(A) NAME/KEY: other
(B) LOCATION: 126..161
(C) IDENTIFICATION METHOD: blastn
(D) OTHER INFORMATION: identity 97
region 129..164
id N41630
est

(ix) FEATURE:
(A) NAME/KEY: other
(B) LOCATION: 58..89
(C) IDENTIFICATION METHOD: blastn
(D) OTHER INFORMATION: identity 93
region 63..94
id N41630
est

(ix) FEATURE:
(A) NAME/KEY: other
(B) LOCATION: 2..31
(C) IDENTIFICATION METHOD: blastn
(D) OTHER INFORMATION: identity 96
region 7..36
id N41630
est

(ix) FEATURE:
(A) NAME/KEY: other
(B) LOCATION: 38..161
(C) IDENTIFICATION METHOD: blastn
(D) OTHER INFORMATION: identity 94
region 19..142
id AA043148
est

(ix) FEATURE:
(A) NAME/KEY: other
(B) LOCATION: 121..185
(C) IDENTIFICATION METHOD: blastn
(D) OTHER INFORMATION: identity 98
region 61..125
id HUM430A04B
est

(ix) FEATURE:
(A) NAME/KEY: other
(B) LOCATION: 60..119
(C) IDENTIFICATION METHOD: blastn
(D) OTHER INFORMATION: identity 98
region 1..60
id HUM430A04B
est

(ix) FEATURE:
(A) NAME/KEY: sig_peptide
(B) LOCATION: 98..157
(C) IDENTIFICATION METHOD: Von Heijne matrix

(D) OTHER INFORMATION: score 5.3
seq ALIFGGFISLIGA/AF

(xi) SEQUENCE DESCRIPTION: SEQ ID NO: 278:

AACCTCTTCC GAGCGGGGTC ACGGCCCGGC CGTCGGTAAC CTGGTTTCCG AGAGTGCCGG 60

GCGGTCGGCG GGTCAGGGCA GCCCGGGGCC TGACGCC ATG TCC CGG AAC CTG CGC 115
Met Ser Arg Asn Leu Arg
-20 -15

ACC GCG CTC ATT TTC GGC GGC TTC ATC TCC CTG ATC GGC GCC GCC TTC 163
Thr Ala Leu Ile Phe Gly Gly Phe Ile Ser Leu Ile Gly Ala Ala Phe
-10 -5 1

TAT CCC ATC TAC TTC CGA CCC CAT GGG 190
Tyr Pro Ile Tyr Phe Arg Pro His Gly
5 10

(2) INFORMATION FOR SEQ ID NO: 279:

(i) SEQUENCE CHARACTERISTICS:
(A) LENGTH: 274 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: complement(97..229)
(C) IDENTIFICATION METHOD: blastn
(D) OTHER INFORMATION: identity 95
region 10..142
id H62783
est

(ix) FEATURE:
(A) NAME/KEY: other
(B) LOCATION: 80..218
(C) IDENTIFICATION METHOD: blastn
(D) OTHER INFORMATION: identity 94
region 54..192
id T71240
est

(ix) FEATURE:
(A) NAME/KEY: other
(B) LOCATION: 148..221
(C) IDENTIFICATION METHOD: blastn
(D) OTHER INFORMATION: identity 98
region 356..429

id AA075451
est

(ix) FEATURE:
(A) NAME/KEY: other
(B) LOCATION: 80..140
(C) IDENTIFICATION METHOD: blastn
(D) OTHER INFORMATION: identity 95
region 288..348
id AA075451
est

(ix) FEATURE:
(A) NAME/KEY: other
(B) LOCATION: 135..222
(C) IDENTIFICATION METHOD: blastn
(D) OTHER INFORMATION: identity 92
region 350..437
id AA009954
est

(ix) FEATURE:
(A) NAME/KEY: other
(B) LOCATION: 105..140
(C) IDENTIFICATION METHOD: blastn
(D) OTHER INFORMATION: identity 94
region 319..354
id AA009954
est

(ix) FEATURE:
(A) NAME/KEY: other
(B) LOCATION: 148..216
(C) IDENTIFICATION METHOD: blastn
(D) OTHER INFORMATION: identity 98
region 384..452
id W15396
est

(ix) FEATURE:
(A) NAME/KEY: other
(B) LOCATION: 80..117
(C) IDENTIFICATION METHOD: blastn
(D) OTHER INFORMATION: identity 97
region 315..352
id W15396
est

(ix) FEATURE:
(A) NAME/KEY: sig_peptide
(B) LOCATION: 206..256
(C) IDENTIFICATION METHOD: Von Heijne matrix
(D) OTHER INFORMATION: score 5.1
seq LWCFHLVVLSLYS/SV

(xi) SEQUENCE DESCRIPTION: SEQ ID NO: 279:
ATGAGTGTTG ATGTTTTTCT GCACTAGAAG GCACTATGTT GAACTATTAA ACTTACCAGC 60
ACTTTCTTTT TCCACTCCAT AGTTTCATTG TACTGACAAC CTCAGCTGGC ATCATGGACC 120
ATGAAGAAGC AAGACGAAAA CACACAGGRA GGGAAAATCC TGGGATTCTT TTTCTAGGGA 180
TGTAATACAT ATATTTACAA ATAAA ATG CCT CAT GGA CTC TGG TGC TTC CAC 232
TTG GTC GTT TTG AGC CTT TAC AGC AGT GTA GCC ACA GCC CGG 274

(2) INFORMATION FOR SEQ ID NO: 280:

(i) SEQUENCE CHARACTERISTICS:
(A) LENGTH: 125 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: complement(2..124)
(C) IDENTIFICATION METHOD: blastn
(D) OTHER INFORMATION: identity 100
region 104..226
id W94087
est

(ix) FEATURE:
(A) NAME/KEY: other
(B) LOCATION: 2..124
(C) IDENTIFICATION METHOD: blastn
(D) OTHER INFORMATION: identity 100
region 12..134
id R37206
est

(ix) FEATURE:
(A) NAME/KEY: other
(B) LOCATION: 2..124
(C) IDENTIFICATION METHOD: blastn
(D) OTHER INFORMATION: identity 100
region 19..141
id N42384
est

(ix) FEATURE:
(A) NAME/KEY: other
(B) LOCATION: complement(2..92)
(C) IDENTIFICATION METHOD: blastn
(D) OTHER INFORMATION: identity 96
region 177..267

id H84930
est

(ix) FEATURE:
(A) NAME/KEY: other
(B) LOCATION: complement(81..124)
(C) IDENTIFICATION METHOD: blastn
(D) OTHER INFORMATION: identity 93
region 144..187
id H84930
est

(ix) FEATURE:
(A) NAME/KEY: other
(B) LOCATION: complement(2..124)
(C) IDENTIFICATION METHOD: blastn
(D) OTHER INFORMATION: identity 99
region 148..270
id H82795
est

(ix) FEATURE:
(A) NAME/KEY: sig_peptide
(B) LOCATION: 21..62
(C) IDENTIFICATION METHOD: Von Heijne matrix
(D) OTHER INFORMATION: score 5
seq SLVAVFLSCGLIS/KN

(xi) SEQUENCE DESCRIPTION: SEQ ID NO: 280:

ATAAATTAGC AGTATTAGTT ATG AGT TTG GTT GCA GTG TTC TTA TCT TGT GGG 53
Met Ser Leu Val Ala Val Phe Leu Ser Cys Gly
-10 -5

CTG ATT TCC AAA AAC CAC ATG CTG CTG AAT TTA CCA GGG ATC CTC ATA 101
Leu Ile Ser Lys Asn His Met Leu Leu Asn Leu Pro Gly Ile Leu Ile
1 5 10

CCT CAC AAT GCA AAC CAC TTA CTG 125
Pro His Asn Ala Asn His Leu Leu
15 20

(2) INFORMATION FOR SEQ ID NO: 281:

(i) SEQUENCE CHARACTERISTICS:
(A) LENGTH: 152 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Kidney

(ix) FEATURE:
(A) NAME/KEY: other

(B) LOCATION: 2..85
(C) IDENTIFICATION METHOD: blastn
(D) OTHER INFORMATION: identity 91
region 4..87
id HUML1521
est

(ix) FEATURE:
(A) NAME/KEY: other
(B) LOCATION: 85..120
(C) IDENTIFICATION METHOD: blastn
(D) OTHER INFORMATION: identity 94
region 86..121
id HUML1521
est

(ix) FEATURE:
(A) NAME/KEY: other
(B) LOCATION: 89..148
(C) IDENTIFICATION METHOD: blastn
(D) OTHER INFORMATION: identity 96
region 123..182
id W52706
est

(ix) FEATURE:
(A) NAME/KEY: other
(B) LOCATION: 34..84
(C) IDENTIFICATION METHOD: blastn
(D) OTHER INFORMATION: identity 92
region 69..119
id W52706
est

(ix) FEATURE:
(A) NAME/KEY: other
(B) LOCATION: complement(75..148)
(C) IDENTIFICATION METHOD: blastn
(D) OTHER INFORMATION: identity 91
region 324..397
id AA132959
est

(ix) FEATURE:
(A) NAME/KEY: sig_peptide
(B) LOCATION: 27..98
(C) IDENTIFICATION METHOD: Von Heijne matrix
(D) OTHER INFORMATION: score 5
seq GALAVGAVPVVLS/AM

(xi) SEQUENCE DESCRIPTION: SEQ ID NO: 281:

AAAGTTGNSA CCCGGACGGC CTCACC ATG ATG AAA CGG GCA GCT GCT GCT GCA 53
Met Met Lys Arg Ala Ala Ala Ala Ala
-20

GTG GGA GGA GCC CTG GCA GTG GGG GCT GTG CCC GTG GTG CTC AGT GCC 101
Val Gly Gly Ala Leu Ala Val Gly Ala Val Pro Val Val Leu Ser Ala
-15 -10 -5 1

ATG GGC TTC ACT GGG GCA GGA ATC GCC GCG TCC TCC ATA GCA GCC CAT     149
Met Gly Phe Thr Gly Ala Gly Ile Ala Ala Ser Ser Ile Ala Ala His
            5                   10                  15

GGG                                                                 152
Gly

(2) INFORMATION FOR SEQ ID NO: 282:

(i) SEQUENCE CHARACTERISTICS:
(A) LENGTH: 429 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 232..430
(C) IDENTIFICATION METHOD: blastn
(D) OTHER INFORMATION:  identity 97
                        region 14..212
                        id H14129
                        est

(ix) FEATURE:
(A) NAME/KEY: sig_peptide
(B) LOCATION: 19..261
(C) IDENTIFICATION METHOD: Von Heijne matrix
(D) OTHER INFORMATION:  score 4.9
                        seq LISFSWFANYIRA/GT

(xi) SEQUENCE DESCRIPTION: SEQ ID NO: 282:

ATTGCCTTCA TTGCCGGC ATG GCC GTC ATT GTG GAT AAA CCC TGG TTC TAT      51
                    Met Ala Val Ile Val Asp Lys Pro Trp Phe Tyr
                        -80                 -75

GAC ATG AAG AAA GTT TGG GAG GGA TAT CCC ATA CAG AGC ACT ATC CCT      99
Asp Met Lys Lys Val Trp Glu Gly Tyr Pro Ile Gln Ser Thr Ile Pro
-70                 -65                 -60                 -55

TCC CAG TAT TGG TAC TAC ATG ATT GAA CTT TCC TTC TAC TGG TCC CTG     147
Ser Gln Tyr Trp Tyr Tyr Met Ile Glu Leu Ser Phe Tyr Trp Ser Leu
                -50                 -45                 -40

CTC TTC AGC ATT GCC TCT GAT GTC AAG CGA AAG GAT TTC AAG GAA CAG     195
Leu Phe Ser Ile Ala Ser Asp Val Lys Arg Lys Asp Phe Lys Glu Gln
            -35                 -30                 -25

ATC ATC CAC CAT GTG GCC ACC ATC ATT CTC ATC AGC TTT TCC TGG TTT     243
Ile Ile His His Val Ala Thr Ile Ile Leu Ile Ser Phe Ser Trp Phe

-20                    -15                    -10

GCC AAT TAC ATC CGA GCT GGG ACT CTA ATC ATG GCT CTG CAT GAC TCT     291
Ala Asn Tyr Ile Arg Ala Gly Thr Leu Ile Met Ala Leu His Asp Ser
     -5                   1               5                  10

TCC GAT TAC CTG CTG GAG TCA GCC AAG ATG TTT AAC TAC GCG GGA TGG     339
Ser Asp Tyr Leu Leu Glu Ser Ala Lys Met Phe Asn Tyr Ala Gly Trp
                 15                  20                  25

AAG AAC ACC TGC AAC AAC ATC TTC ACC GTC TTC GCC ATT GTT TTT ATC     387
Lys Asn Thr Cys Asn Asn Ile Phe Thr Val Phe Ala Ile Val Phe Ile
             30                  35                  40

ATC ACC CGA CTG GTC ATC CTG CCC TTC TGG ATC CTG CAT TGC             429
Ile Thr Arg Leu Val Ile Leu Pro Phe Trp Ile Leu His Cys
         45                  50                  55


(2) INFORMATION FOR SEQ ID NO: 283:

      (i) SEQUENCE CHARACTERISTICS:
           (A) LENGTH: 268 base pairs
           (B) TYPE: NUCLEIC ACID
           (C) STRANDEDNESS: DOUBLE
           (D) TOPOLOGY: LINEAR

      (ii) MOLECULE TYPE: CDNA

      (vi) ORIGINAL SOURCE:
           (A) ORGANISM: Homo Sapiens
           (D) DEVELOPMENTAL STAGE: Fetal
           (F) TISSUE TYPE: kidney

      (ix) FEATURE:
           (A) NAME/KEY: other
           (B) LOCATION: 111..221
           (C) IDENTIFICATION METHOD: blastn
           (D) OTHER INFORMATION:  identity 97
                                   region 37..147
                                   id T82645
                                   est

      (ix) FEATURE:
           (A) NAME/KEY: sig_peptide
           (B) LOCATION: 35..82
           (C) IDENTIFICATION METHOD: Von Heijne matrix
           (D) OTHER INFORMATION:  score 4.8
                                   seq SLFIYIFLTCSNT/SP

      (xi) SEQUENCE DESCRIPTION: SEQ ID NO: 283:


ATAGTATCTA TTGAAAAGGA AGCAGTGTGT ATCT ATG ATT ATA TCT CTG TTC ATC    55
                                      Met Ile Ile Ser Leu Phe Ile
                                          -15                 -10

TAT ATA TTT TTG ACA TGT AGC AAC ACC TCT CCA TCT TAT CAA GGA ACT     103
Tyr Ile Phe Leu Thr Cys Ser Asn Thr Ser Pro Ser Tyr Gln Gly Thr
                 -5                   1               5

CAA CTC GGT CTG GGT CTC CCC AGT GCC CAG TGG TGG CCT TTG ACA GGT     151
Gln Leu Gly Leu Gly Leu Pro Ser Ala Gln Trp Trp Pro Leu Thr Gly
        10                  15                  20

AGG AGG ATG CAG TGC TGC AGG CTA TTT TGT TTT TTG TTA CAA AAC TGT     199
Arg Arg Met Gln Cys Cys Arg Leu Phe Cys Phe Leu Leu Gln Asn Cys
    25                  30                  35

CTT TTC CCT TTT CCC CTC CAC CTG ATT CAG CAT GAT CCC TGT GAG CTG     247
Leu Phe Pro Phe Pro Leu His Leu Ile Gln His Asp Pro Cys Glu Leu
40                  45                  50                  55

GTT CTC ACA ATC TCT GGG ACT                                         268
Val Leu Thr Ile Ser Gly Thr
                60

(2) INFORMATION FOR SEQ ID NO: 284:

(i) SEQUENCE CHARACTERISTICS:
(A) LENGTH: 266 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 9..250
(C) IDENTIFICATION METHOD: blastn
(D) OTHER INFORMATION: identity 98
region 7..248
id HSC2OD111
est

(ix) FEATURE:
(A) NAME/KEY: other
(B) LOCATION: 122..257
(C) IDENTIFICATION METHOD: blastn
(D) OTHER INFORMATION: identity 97
region 1..136
id T77096
est

(ix) FEATURE:
(A) NAME/KEY: other
(B) LOCATION: 18..146
(C) IDENTIFICATION METHOD: blastn
(D) OTHER INFORMATION: identity 95
region 19..147
id N32450
est

(ix) FEATURE:
(A) NAME/KEY: sig_peptide
(B) LOCATION: 9..104
(C) IDENTIFICATION METHOD: Von Heijne matrix
(D) OTHER INFORMATION: score 4.7
seq LQMLLGFVGRSKS/GL

(xi) SEQUENCE DESCRIPTION: SEQ ID NO: 284:

AGACCAAG ATG GCG GCG GAG CTG GTG GAG GCC AAA AAC ATG GTG ATG AGT 50
Met Ala Ala Glu Leu Val Glu Ala Lys Asn Met Val Met Ser
-30 -25 -20

TTT CGA GTC TCC GAC CTT CAG ATG CTC CTG GGT TTC GTG GGC CGG AGT 98
Phe Arg Val Ser Asp Leu Gln Met Leu Leu Gly Phe Val Gly Arg Ser
-15 -10 -5

AAG AGT GGA CTG AAG CAC GAG CTC GTC ACC AGG GCC CTC CAG CTG GTG 146
Lys Ser Gly Leu Lys His Glu Leu Val Thr Arg Ala Leu Gln Leu Val
1 5 10

CAG TTT GAC TGT AGC CCT GAG CTG TTC AAG AAG ATC AAG GAG CTG TAC 194
Gln Phe Asp Cys Ser Pro Glu Leu Phe Lys Lys Ile Lys Glu Leu Tyr
15 20 25 30

GAG ACC CGC TAC GCC AAG AAG AAC TCG GAG CCT GCC CCA CAG CCG CAC 242
Glu Thr Arg Tyr Ala Lys Lys Asn Ser Glu Pro Ala Pro Gln Pro His
        35                  40                  45

CGG CCC CTG GAC CCC CTG ACC GGG 266
Arg Pro Leu Asp Pro Leu Thr Gly
50

(2) INFORMATION FOR SEQ ID NO: 285:

(i) SEQUENCE CHARACTERISTICS:
(A) LENGTH: 264 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 10..105
(C) IDENTIFICATION METHOD: blastn
(D) OTHER INFORMATION: identity 93
region 1..96
id R05622
est

(ix) FEATURE:

(A) NAME/KEY: other
(B) LOCATION: 24..92
(C) IDENTIFICATION METHOD: blastn
(D) OTHER INFORMATION: identity 97
region 2..70
id H94933
est

(ix) FEATURE:
(A) NAME/KEY: sig_peptide
(B) LOCATION: 64..243
(C) IDENTIFICATION METHOD: Von Heijne matrix
(D) OTHER INFORMATION: score 4.7
seq VHALCPLSPLVTT/GC

(xi) SEQUENCE DESCRIPTION: SEQ ID NO: 285:

AACTCTCCAA AAAGCAGAGA CAGCAGGAAG AGGGGAGTGG AGGCAGCCCA TTCACCTGGG 60

GAA ATG ACT GGG TTG TCG ATG GMC GGT GGC GGB AGC CSA AMG GGG GAY 108
Met Thr Gly Leu Ser Met Xaa Gly Gly Gly Ser Xaa Xaa Gly Asp
-60 -55 -50

GTG GAS CCG TDC TAC TAT GGT AAR CVT GGG CCC CTG CGC RCC CTT CCT 156
Val Xaa Pro Xaa Tyr Tyr Gly Lys Xaa Gly Pro Leu Arg Xaa Leu Pro
-45 -40 -35 -30

GAG CCC TCA GGA CCC CTT CCA CCA AGC AGC GGC CTC TCC CAG CCC CAG 204
Glu Pro Ser Gly Pro Leu Pro Pro Ser Ser Gly Leu Ser Gln Pro Gln
-25 -20 -15

GTC CAT GCT CTG TGC CCC TTA TCT CCC CTG GTT ACC ACG GGC TGC TGC 252
Val His Ala Leu Cys Pro Leu Ser Pro Leu Val Thr Thr Gly Cys Cys
-10 -5 1

GGG CAG GCT GCG 264
Gly Gln Ala Ala
5

(2) INFORMATION FOR SEQ ID NO: 286:

(i) SEQUENCE CHARACTERISTICS:
(A) LENGTH: 465 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 157..269
(C) IDENTIFICATION METHOD: blastn

(D) OTHER INFORMATION: identity 97
region 95..207
id N41379
est

(ix) FEATURE:
(A) NAME/KEY: other
(B) LOCATION: 62..173
(C) IDENTIFICATION METHOD: blastn
(D) OTHER INFORMATION: identity 90
region 1..112
id N41379
est

(ix) FEATURE:
(A) NAME/KEY: other
(B) LOCATION: 275..319
(C) IDENTIFICATION METHOD: blastn
(D) OTHER INFORMATION: identity 95
region 213..257
id N41379
est

(ix) FEATURE:
(A) NAME/KEY: other
(B) LOCATION: 8..173
(C) IDENTIFICATION METHOD: blastn
(D) OTHER INFORMATION: identity 90
region 1..166
id AA044371
est

(ix) FEATURE:
(A) NAME/KEY: other
(B) LOCATION: 157..219
(C) IDENTIFICATION METHOD: blastn
(D) OTHER INFORMATION: identity 98
region 149..211
id AA044371
est

(ix) FEATURE:
(A) NAME/KEY: other
(B) LOCATION: complement(272..319)
(C) IDENTIFICATION METHOD: blastn
(D) OTHER INFORMATION: identity 93
region 423..470
id N30852
est

(ix) FEATURE:
(A) NAME/KEY: other
(B) LOCATION: complement(225..264)
(C) IDENTIFICATION METHOD: blastn
(D) OTHER INFORMATION: identity 92
region 478..517
id N30852
est

(ix) FEATURE:
(A) NAME/KEY: other

(B) LOCATION: complement(320..349)
(C) IDENTIFICATION METHOD: blastn
(D) OTHER INFORMATION: . identity 96
region 394..423
id N30852
est

(ix) FEATURE:
(A) NAME/KEY: other
(B) LOCATION: complement(238..271)
(C) IDENTIFICATION METHOD: blastn
(D) OTHER INFORMATION: identity 94
region 481..514
id AA044232
est

(ix) FEATURE:
(A) NAME/KEY: other
(B) LOCATION: 303..349
(C) IDENTIFICATION METHOD: blastn
(D) OTHER INFORMATION: identity 100
region 5..51
id R78468
est

(ix) FEATURE:
(A) NAME/KEY: sig_peptide
(B) LOCATION: 367..459
(C) IDENTIFICATION METHOD: Von Heijne matrix
(D) OTHER INFORMATION: score 4.6
seq GLLGXGLXXXSLT/AG

(xi) SEQUENCE DESCRIPTION: SEQ ID NO: 286:

AAAGTCCTAG AGGGGGTCGG GGTMTGGGTG GACAAGCTTT CCTCGTCCTC TCCCNACAGA 60

GCTGACGTGT CCTGGGTTCC ACCGGGAGCG GGCATTTCCA CCGGACGGGA GGGTTCGGGG 120

TGTCCGGGGC TGGGGAATAC GTARGGGKTG CSGCGCCGGT GTGGGAAGTT GGGGCGTGTG 180

GCTGCAGTCC CGGGAGTTCT TGGAGGGGGT CGGCCCACCG AGCTTCCGGA CCGGCTGATC 240

TGCCCGTAGC TTGCCGGAGG GAGGGCGGAG CTGACTCTCC GTCCCTTCTC CCATCCCCTC 300

SAGTGGTGGG TACGGGCACC TCGCTGGCGC TCTCCTCCCT CCTGTCCCTN GNNSNTCTTT 360

GCTGGG ATG CAG ATG TAC AGC CGT CAG CTG GCC TCC AMC GAG TGG CTC 408
Met Gln Met Tyr Ser Arg Gln Leu Ala Ser Xaa Glu Trp Leu
-30 -25 -20

ACC ATC CAG GGC GGC CTG CTT GGW KCG GGT CTC TTS KRG TYC TCG CTC 456
Thr Ile Gln Gly Gly Leu Leu Gly Xaa Gly Leu Xaa Xaa Xaa Ser Leu
-15 -10 -5

ACT GCG GGG 465
Thr Ala Gly
1

(2) INFORMATION FOR SEQ ID NO: 287:

(i) SEQUENCE CHARACTERISTICS:
(A) LENGTH: 384 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 63..344
(C) IDENTIFICATION METHOD: blastn
(D) OTHER INFORMATION: identity 97
region 56..337
id AA203498
est

(ix) FEATURE:
(A) NAME/KEY: other
(B) LOCATION: 7..65
(C) IDENTIFICATION METHOD: blastn
(D) OTHER INFORMATION: identity 100
region 1..59
id AA203498
est

(ix) FEATURE:
(A) NAME/KEY: other
(B) LOCATION: 344..385
(C) IDENTIFICATION METHOD: blastn
(D) OTHER INFORMATION: identity 97
region 338..379
id AA203498
est

(ix) FEATURE:
(A) NAME/KEY: other
(B) LOCATION: 63..292
(C) IDENTIFICATION METHOD: blastn
(D) OTHER INFORMATION: identity 99
region 44..273
id W87295
est

(ix) FEATURE:
(A) NAME/KEY: other
(B) LOCATION: 292..344
(C) IDENTIFICATION METHOD: blastn
(D) OTHER INFORMATION: identity 92
region 274..326
id W87295
est

(ix) FEATURE:

(A) NAME/KEY: other
(B) LOCATION: 20..65
(C) IDENTIFICATION METHOD: blastn
(D) OTHER INFORMATION:  identity 100
                        region 2..47
                        id W87295
                        est

(ix) FEATURE:
(A) NAME/KEY: other
(B) LOCATION: 344..385
(C) IDENTIFICATION METHOD: blastn
(D) OTHER INFORMATION:  identity 97
                        region 327..368
                        id W87295
                        est

(ix) FEATURE:
(A) NAME/KEY: other
(B) LOCATION: 33..344
(C) IDENTIFICATION METHOD: blastn
(D) OTHER INFORMATION:  identity 98
                        region 1..312
                        id AA248429
                        est

(ix) FEATURE:
(A) NAME/KEY: other
(B) LOCATION: 344..385
(C) IDENTIFICATION METHOD: blastn
(D) OTHER INFORMATION:  identity 97
                        region 313..354
                        id AA248429
                        est

(ix) FEATURE:
(A) NAME/KEY: other
(B) LOCATION: 76..344
(C) IDENTIFICATION METHOD: blastn
(D) OTHER INFORMATION:  identity 98
                        region 1..269
                        id W01758
                        est

(ix) FEATURE:
(A) NAME/KEY: other
(B) LOCATION: 344..385
(C) IDENTIFICATION METHOD: blastn
(D) OTHER INFORMATION:  identity 97
                        region 270..311
                        id W01758
                        est

(ix) FEATURE:
(A) NAME/KEY: other
(B) LOCATION: 63..234
(C) IDENTIFICATION METHOD: blastn
(D) OTHER INFORMATION:  identity 97
                        region 30..201
                        id AA249697
                        est

(ix) FEATURE:
(A) NAME/KEY: other
(B) LOCATION: 33..65
(C) IDENTIFICATION METHOD: blastn
(D) OTHER INFORMATION: identity 100
region 1..33
id AA249697
est

(ix) FEATURE:
(A) NAME/KEY: other
(B) LOCATION: 257..289
(C) IDENTIFICATION METHOD: blastn
(D) OTHER INFORMATION: identity 100
region 222..254
id AA249697
est

(ix) FEATURE:
(A) NAME/KEY: other
(B) LOCATION: 227..256
(C) IDENTIFICATION METHOD: blastn
(D) OTHER INFORMATION: identity 96
region 193..222
id AA249697
est

(ix) FEATURE:
(A) NAME/KEY: sig_peptide
(B) LOCATION: 19..180
(C) IDENTIFICATION METHOD: Von Heijne matrix
(D) OTHER INFORMATION: score 4.3
seq LIVWLLVKSFSES/GI

(xi) SEQUENCE DESCRIPTION: SEQ ID NO: 287:

ATCTGGCTCA GTTCCGCC ATG GCC TCC TTG GAA GTC AGT CGT AGT CCT CGC 51
Met Ala Ser Leu Glu Val Ser Arg Ser Pro Arg
-50 -45

AGG TCT CGG CGG GAG CTG GAA GTG CGC AGT CCA CGA CAG AAC AAA TAT 99
Arg Ser Arg Arg Glu Leu Glu Val Arg Ser Pro Arg Gln Asn Lys Tyr
-40 -35 -30

TCG GTG CTT TTA CCT ACC TAC AAC GAG CGC GAG AAC CTG CCG CTC ATC 147
Ser Val Leu Leu Pro Thr Tyr Asn Glu Arg Glu Asn Leu Pro Leu Ile
-25 -20 -15

GTG TGG CTG CTG GTG AAA AGC TTC TCC GAG AGT GGA ATC AAC TAT GAA 195
Val Trp Leu Leu Val Lys Ser Phe Ser Glu Ser Gly Ile Asn Tyr Glu
-10 -5 1 5

ATT ATA ATC ATA GAT GAT GGA AGC CCA GAT GGA ACA AGG GAT GTT GCT 243
Ile Ile Ile Ile Asp Asp Gly Ser Pro Asp Gly Thr Arg Asp Val Ala
10 15 20

GAA CAG TTG GAG AAG ATC TAT GGG TCA GAC AGA ATT CTT CTA AGA CCA 291
Glu Gln Leu Glu Lys Ile Tyr Gly Ser Asp Arg Ile Leu Leu Arg Pro
25 30 35

CGA GAG AAA AAG TTG GGA CTA GGA ACT GCA TAT ATT CDY SRA ATG AAA     339
Arg Glu Lys Lys Leu Gly Leu Gly Thr Ala Tyr Ile Xaa Xaa Met Lys
        40                  45                  50

CAT GCA CAG GAA ACT ACA TCA TTA TTA TGG ATS CTG ATC TCT CAC         384
His Ala Gln Glu Thr Thr Ser Leu Leu Trp Xaa Leu Ile Ser His
    55                  60                  65

(2) INFORMATION FOR SEQ ID NO: 288:

(i) SEQUENCE CHARACTERISTICS:
(A) LENGTH: 332 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: other
(B) LOCATION: 36..268
(C) IDENTIFICATION METHOD: blastn
(D) OTHER INFORMATION: identity 97
region 13..245
id AA134651
est

(ix) FEATURE:
(A) NAME/KEY: other
(B) LOCATION: 266..303
(C) IDENTIFICATION METHOD: blastn
(D) OTHER INFORMATION: identity 97
region 244..281
id AA134651
est

(ix) FEATURE:
(A) NAME/KEY: other
(B) LOCATION: 14..272
(C) IDENTIFICATION METHOD: blastn
(D) OTHER INFORMATION: identity 97
region 95..353
id W26888
est

(ix) FEATURE:
(A) NAME/KEY: other
(B) LOCATION: 61..262
(C) IDENTIFICATION METHOD: blastn
(D) OTHER INFORMATION: identity 99
region 7..208
id T66207
est

(ix) FEATURE:
(A) NAME/KEY: other
(B) LOCATION: 263..325
(C) IDENTIFICATION METHOD: blastn
(D) OTHER INFORMATION: identity 98
region 208..270
id T66207
est

(ix) FEATURE:
(A) NAME/KEY: other
(B) LOCATION: 39..267
(C) IDENTIFICATION METHOD: blastn
(D) OTHER INFORMATION: identity 96
region 1..229
id W00383
est

(ix) FEATURE:
(A) NAME/KEY: other
(B) LOCATION: 35..304
(C) IDENTIFICATION METHOD: blastn
(D) OTHER INFORMATION: identity 96
region 13..282
id HSC36A071
est

(ix) FEATURE:
(A) NAME/KEY: sig_peptide
(B) LOCATION: 207..266
(C) IDENTIFICATION METHOD: Von Heijne matrix
(D) OTHER INFORMATION: score 4.3
seq LLDSSLMASGTAS/RS

(xi) SEQUENCE DESCRIPTION: SEQ ID NO: 288:

AAACCGGGKG TAGACGTACC TCACGGAAGC CGGCTTTGGC CCTGCGGCTK YTACCGTCGC 60

CGCGGAGAAA TTGTTGGATC TGGCAGTCTA GGAATGAATC TCCTCTCAGC CTTTAAGCTC 120

ACCTGGTCAG AATCCTTGGA TGAGCCTGTG GGACCGTTCC TCCTAGCCCG GTGGTTTGGA 180

ACCAGTGGCT TTGGGACTGT AAGAGG ATG GAC AAA GAT TCT CAG GGG CTG CTA 233
Met Asp Lys Asp Ser Gln Gly Leu Leu
-20 -15

GAT TCA TCC CTG ATG GCA TCA GGC ACT GCC AGC CGC TCA GAG GAT GAG 281
Asp Ser Ser Leu Met Ala Ser Gly Thr Ala Ser Arg Ser Glu Asp Glu
-10 -5 1 5

GAG TCA CTG GCA GGG CAG AAG CGA GCC TCC TCC CAG GCC CTG GGC ACC 329
Glu Ser Leu Ala Gly Gln Lys Arg Ala Ser Ser Gln Ala Leu Gly Thr
10 15 20

GGG 332
Gly

(2) INFORMATION FOR SEQ ID NO: 289:

(i) SEQUENCE CHARACTERISTICS:
(A) LENGTH: 348 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 126..226
(C) IDENTIFICATION METHOD: blastn
(D) OTHER INFORMATION: identity 95
region 38..138
id AA009514
est

(ix) FEATURE:
(A) NAME/KEY: other
(B) LOCATION: 252..343
(C) IDENTIFICATION METHOD: blastn
(D) OTHER INFORMATION: identity 98
region 161..252
id AA009514
est

(ix) FEATURE:
(A) NAME/KEY: other
(B) LOCATION: 102..131
(C) IDENTIFICATION METHOD: blastn
(D) OTHER INFORMATION: identity 93
region 15..44
id AA009514
est

(ix) FEATURE:
(A) NAME/KEY: sig_peptide
(B) LOCATION: 100..207
(C) IDENTIFICATION METHOD: Von Heijne matrix
(D) OTHER INFORMATION: score 4.2
seq CLAVSWEAAGCHG/AG

(xi) SEQUENCE DESCRIPTION: SEQ ID NO: 289:

AAAGGAATAC TGACAGATAA GGCCGGAAAC AAAACTGATG GCTTGAAAAA CATTTTTATG 60

GAATGTATTT ACTATCATTT TGTTTTACTA TAGAGGTAG ATG GGA CTC TTA ACT 114
Met Gly Leu Leu Thr
-35

TTT GGG TAC ATT GAA AMC AKG CKG AAA ACT GAA CAC AAT CCT GAT CAT 162
Phe Gly Tyr Ile Glu Xaa Xaa Xaa Lys Thr Glu His Asn Pro Asp His
-30 -25 -20

CAC TCC TGC CTG GCT GTC TCC TGG GAG GCT GCC GGG TGC CAC GGA GCT     210
His Ser Cys Leu Ala Val Ser Trp Glu Ala Ala Gly Cys His Gly Ala
-15                 -10              -5                      1

GGG ACA CAG CAG AGC CCG CTA GGT GTT GCA GGG CCC TGG AGG CCA AGG     258
Gly Thr Gln Gln Ser Pro Leu Gly Val Ala Gly Pro Trp Arg Pro Arg
                5                   10                  15

CCA CCC TGT GTG GGG TCC CTG TTG GCA GCC AGG TCC CTA CAC AAA CAA     306
Pro Pro Cys Val Gly Ser Leu Leu Ala Ala Arg Ser Leu His Lys Gln
        20                  25                  30

GTA ATC CTG TTT GGC CTC CTA GGT TTT GCA TAT GAC CAC TGG             348
Val Ile Leu Phe Gly Leu Leu Gly Phe Ala Tyr Asp His Trp
    35                  40                  45

(2) INFORMATION FOR SEQ ID NO: 290:

(i) SEQUENCE CHARACTERISTICS:
(A) LENGTH: 206 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: other
(B) LOCATION: 73..208
(C) IDENTIFICATION METHOD: blastn
(D) OTHER INFORMATION: identity 97
region 53..188
id T06781
est

(ix) FEATURE:
(A) NAME/KEY: other
(B) LOCATION: 20..80
(C) IDENTIFICATION METHOD: blastn
(D) OTHER INFORMATION: identity 91
region 1..61
id T06781
est

(ix) FEATURE:
(A) NAME/KEY: other
(B) LOCATION: 16..105
(C) IDENTIFICATION METHOD: blastn
(D) OTHER INFORMATION: identity 95
region 1..90
id AA101354
est

(ix) FEATURE:
(A) NAME/KEY: sig_peptide

(B) LOCATION: 12..59
(C) IDENTIFICATION METHOD: Von Heijne matrix
(D) OTHER INFORMATION:- score 4.1
seq YAAVAGVLAGVES/RQ

(xi) SEQUENCE DESCRIPTION: SEQ ID NO: 290:

AGCGCGGGAA C ATG GGG CTG TAC GCT GCG GTG GCA GGC GTG CTG GCC GGC 50
Met Gly Leu Tyr Ala Ala Val Ala Gly Val Leu Ala Gly
-15 -10 -5

GTG GAG AGC CGC CAG GGC TCT AAT CAA GGG GCT GGT GTA CTC CAG CAA 98
Val Glu Ser Arg Gln Gly Ser Asn Gln Gly Ala Gly Val Leu Gln Gln
1 5 10

CTT CCA GAA CGT GAA RCA GCT GTA CGC GCT GGT GTG CGA AAS GCA GCG 146
Leu Pro Glu Arg Glu Xaa Ala Val Arg Ala Gly Val Arg Xaa Ala Ala
15 20 25

CTA CTC CGC CGT GCT GGA TRC CGT GAT CTC CAR CGC CGG CCT CCT CAG 194
Leu Leu Arg Arg Ala Gly Xaa Arg Asp Leu Gln Arg Arg Pro Pro Gln
30 35 40 45

TGC GAA GAA GCT 206
Cys Glu Glu Ala

(2) INFORMATION FOR SEQ ID NO: 291:

(i) SEQUENCE CHARACTERISTICS:
(A) LENGTH: 299 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: other
(B) LOCATION: 26..219
(C) IDENTIFICATION METHOD: blastn
(D) OTHER INFORMATION: identity 91
region 1..194
id T06781
est

(ix) FEATURE:
(A) NAME/KEY: other
(B) LOCATION: 204..234
(C) IDENTIFICATION METHOD: blastn
(D) OTHER INFORMATION: identity 93
region 176..206
id T06781
est

(ix) FEATURE:
(A) NAME/KEY: other
(B) LOCATION: 22..74
(C) IDENTIFICATION METHOD: blastn
(D) OTHER INFORMATION: identity 90
region 1..53
id AA101354
est

(ix) FEATURE:
(A) NAME/KEY: other
(B) LOCATION: 71..110
(C) IDENTIFICATION METHOD: blastn
(D) OTHER INFORMATION: identity 92
region 51..90
id AA101354
est

(ix) FEATURE:
(A) NAME/KEY: sig_peptide
(B) LOCATION: 18..203
(C) IDENTIFICATION METHOD: Von Heijne matrix
(D) OTHER INFORMATION: score 4.1
seq LDAVIASAGLLRA/EK

(xi) SEQUENCE DESCRIPTION: SEQ ID NO: 291:

AAAAGGCGCG CGGGAAC ATG GGG CTG TAT GCT GCA GCT GCA GGC GTG TTG 50
Met Gly Leu Tyr Ala Ala Ala Ala Gly Val Leu
-60 -55

GCC GGC GTG GAG AGC CGC CAG GGC TCT ATC AAG GGG TTG GTG TAC TCC 98
Ala Gly Val Glu Ser Arg Gln Gly Ser Ile Lys Gly Leu Val Tyr Ser
-50 -45 -40

AGC AAC TTC CAG AAC GTG AAG CAG CTG TAC GCG CTG GTG TGC GAA ACG 146
Ser Asn Phe Gln Asn Val Lys Gln Leu Tyr Ala Leu Val Cys Glu Thr
-35 -30 -25 -20

CAG CGC TAC TCC GCC GTG CTG GAT GCT GTG ATC GCC AGC GCC GGC CTC 194
Gln Arg Tyr Ser Ala Val Leu Asp Ala Val Ile Ala Ser Ala Gly Leu
-15 -10 -5

CTC CGT GCG GAG AAG AAG CTG CGG CCG CAC CTG GCC AAG GTG CTA GTG 242
Leu Arg Ala Glu Lys Lys Leu Arg Pro His Leu Ala Lys Val Leu Val
1 5 10

TAT GAG TTG TTG TTG GGA AAG GGC TTT CGA GGG GGT GGG GGC CGA TGG 290
Tyr Glu Leu Leu Leu Gly Lys Gly Phe Arg Gly Gly Gly Gly Arg Trp
15 20 25

AAG GCC CGG 299
Lys Ala Arg
30

(2) INFORMATION FOR SEQ ID NO: 292:

(i) SEQUENCE CHARACTERISTICS:

(A) LENGTH: 457 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 296..458
(C) IDENTIFICATION METHOD: blastn
(D) OTHER INFORMATION: identity 100
region 1..163
id R50658
est

(ix) FEATURE:
(A) NAME/KEY: other
(B) LOCATION: complement(413..458)
(C) IDENTIFICATION METHOD: blastn
(D) OTHER INFORMATION: identity 100
region 442..487
id AA016001
est

(ix) FEATURE:
(A) NAME/KEY: sig_peptide
(B) LOCATION: 5..196
(C) IDENTIFICATION METHOD: Von Heijne matrix
(D) OTHER INFORMATION: score 4.1
seq WLLRLAYLADIFT/KL

(xi) SEQUENCE DESCRIPTION: SEQ ID NO: 292:

AGAA ATG GGT GCT CAG CAC ACA GCA CTT CTT CTA AAT ACA GAG GTG AGG 49
Met Gly Ala Gln His Thr Ala Leu Leu Leu Asn Thr Glu Val Arg
-60 -55 -50

TGG CTT TCT CGA GGT AAA GTT CTT GTA AGA CTT TTT GAA CTT CGT CGT 97
Trp Leu Ser Arg Gly Lys Val Leu Val Arg Leu Phe Glu Leu Arg Arg
-45 -40 -35

GAA CTT TTG GTT TTC ATG GAT TCT GCT TTT CGA CTA TCT GAT TGT TTA 145
Glu Leu Leu Val Phe Met Asp Ser Ala Phe Arg Leu Ser Asp Cys Leu
-30 -25 -20

ACA AAT TCA TCT TGG CTG CTA AGA CTT GCA TAT CTT GCA GAT ATT TTT 193
Thr Asn Ser Ser Trp Leu Leu Arg Leu Ala Tyr Leu Ala Asp Ile Phe
-15 -10 -5

ACT AAA TTA AAT GAA GTT AAT TTG TCA ATG CAA GGA AAA AAT GTG ACC 241
Thr Lys Leu Asn Glu Val Asn Leu Ser Met Gln Gly Lys Asn Val Thr
1               5                   10                  15

GTT TTT ACA GTA TTT GAT AAA ATG TCG TCA TTG TTA AGA AAA TTG GAA 239

Val Phe Thr Val Phe Asp Lys Met Ser Ser Leu Leu Arg Lys Leu Glu
            20                  25                  30

TTT TGG GCC TCA TCT GTA GAA GAA GAA AAC TTT GAT TGT TTT CCT ACA 337
Phe Trp Ala Ser Ser Val Glu Glu Glu Asn Phe Asp Cys Phe Pro Thr
        35                  40                  45

CTC AGT GAT TTT TTG ACT GAA ATT AAT TCT ACA GTT GAT AAA GAT ATT 385
Leu Ser Asp Phe Leu Thr Glu Ile Asn Ser Thr Val Asp Lys Asp Ile
    50                  55                  60

TGC AGT GCC ATT GTG CAG CAC CTA AGG GGT TTG CGC GCT ACT CTG TTA 433
Cys Ser Ala Ile Val Gln His Leu Arg Gly Leu Arg Ala Thr Leu Leu
65 70 75

AAA TAC TTT CCT GTA ACA AAT GAC 457
Lys Tyr Phe Pro Val Thr Asn Asp
80 85

(2) INFORMATION FOR SEQ ID NO: 293:

(i) SEQUENCE CHARACTERISTICS:
(A) LENGTH: 248 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: other
(B) LOCATION: 60..247
(C) IDENTIFICATION METHOD: blastn
(D) OTHER INFORMATION: identity 95
region 19..206
id AA044042
est

(ix) FEATURE:
(A) NAME/KEY: other
(B) LOCATION: 70..247
(C) IDENTIFICATION METHOD: blastn
(D) OTHER INFORMATION: identity 97
region 6..183
id AA127902
est

(ix) FEATURE:
(A) NAME/KEY: other
(B) LOCATION: 88..247
(C) IDENTIFICATION METHOD: blastn
(D) OTHER INFORMATION: identity 99
region 1..160
id AA056679
est

(ix) FEATURE:
(A) NAME/KEY: other
(B) LOCATION: complement(99..247)
(C) IDENTIFICATION METHOD: blastn
(D) OTHER INFORMATION: identity 99
region 302..450
id W93399
est

(ix) FEATURE:
(A) NAME/KEY: other
(B) LOCATION: 90..237
(C) IDENTIFICATION METHOD: blastn
(D) OTHER INFORMATION: identity 97
region 11..158
id R29154
est

(ix) FEATURE:
(A) NAME/KEY: sig_peptide
(B) LOCATION: 117..191
(C) IDENTIFICATION METHOD: Von Heijne matrix
(D) OTHER INFORMATION: score 4
seq LVVMVPLVGLIHL/GW

(xi) SEQUENCE DESCRIPTION: SEQ ID NO: 293:

AATCCGCGGC AGAGCGGCTG CTTGAGATCT GTTTCTGGGG CCTCTGGCGG TGGCGGCCTG 60

TGGCGGCCTG GGGCGGCGCG ACGGCTGGTG CGCAGGTACA CTGATGCTGA AGTACT ATG 119
Met
-25

AGC CTT CGG AAC TTG TGG AGA GAC TAC AAA GTT TTG GTT GTT ATG GTC 167
Ser Leu Arg Asn Leu Trp Arg Asp Tyr Lys Val Leu Val Val Met Val
-20 -15 -10

CCT TTA GTT GGG CTC ATA CAT TTG GGG TGG TAC AGA ATC AAA AGC AGC 215
Pro Leu Val Gly Leu Ile His Leu Gly Trp Tyr Arg Ile Lys Ser Ser
-5 1 5

CCT GTT TTC CAA ATA CCT AAA AAC GAC AAC ATG 248
Pro Val Phe Gln Ile Pro Lys Asn Asp Asn Met
10 15

(2) INFORMATION FOR SEQ ID NO: 294:

(i) SEQUENCE CHARACTERISTICS:
(A) LENGTH: 389 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens

(F) TISSUE TYPE: Heart

(ix) FEATURE:
(A) NAME/KEY: other
(B) LOCATION: 245..374
(C) IDENTIFICATION METHOD: blastn
(D) OTHER INFORMATION: identity 97
region 20..149
id T41381
est

(ix) FEATURE:
(A) NAME/KEY: sig_peptide
(B) LOCATION: 75..227
(C) IDENTIFICATION METHOD: Von Heijne matrix
(D) OTHER INFORMATION: score 3.9
seq GKLLQLVLGCAIS/CE

(xi) SEQUENCE DESCRIPTION: SEQ ID NO: 294:

AAAAATAAAA TGTAGGCAGC AAAAGTGGAA GAGGAGAGGC AGCTGGTGCA CTAATCCAGG 60

TCAGCAATCT GAAG ATG GTC TTA CGG AGC CTA GTA GAG TAC TCC CAG GAT 110
Met Val Leu Arg Ser Leu Val Glu Tyr Ser Gln Asp
-50 -45 -40

GTC CTG GCG CAT CCT GTG TCA GAA GAG CAT CTC CCA GAT GTG AGC CTC 158
Val Leu Ala His Pro Val Ser Glu Glu His Leu Pro Asp Val Ser Leu
-35 -30 -25

ATT GGA GAG TTC TCA GAC CCG GCA GAG CTC GGC AAG CTG CTT CAG CTG 206
Ile Gly Glu Phe Ser Asp Pro Ala Glu Leu Gly Lys Leu Leu Gln Leu
-20 -15 -10

GTG CTG GGC TGT GCC ATC AGT TGC GAG AAA AAG CAG GAC CAC ATC CAG 254
Val Leu Gly Cys Ala Ile Ser Cys Glu Lys Lys Gln Asp His Ile Gln
-5 1 5

AGA ATC ATG ACG CTG GAA GAA TCG GTT CAG CAT GTG GTG ATG GAA GCC 302
Arg Ile Met Thr Leu Glu Glu Ser Val Gln His Val Val Met Glu Ala
10 15 20 25

ATC CAA GAG CTC ATG ACC AAA GAC ACT CCT GAC TCC CTG TCA CCA GAG 350
Ile Gln Glu Leu Met Thr Lys Asp Thr Pro Asp Ser Leu Ser Pro Glu
30 35 40

ACG TAT GGC AAC TTT GAC AGC CAG TCC CGC AGT ACT GGG 389
Thr Tyr Gly Asn Phe Asp Ser Gln Ser Arg Ser Thr Gly
45 50

(2) INFORMATION FOR SEQ ID NO: 295:

(i) SEQUENCE CHARACTERISTICS:
(A) LENGTH: 405 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 241..406
(C) IDENTIFICATION METHOD: blastn
(D) OTHER INFORMATION: identity 99
region 105..270
id AA084830
est

(ix) FEATURE:
(A) NAME/KEY: other
(B) LOCATION: 200..229
(C) IDENTIFICATION METHOD: blastn
(D) OTHER INFORMATION: identity 100
region 64..93
id AA084830
est

(ix) FEATURE:
(A) NAME/KEY: other
(B) LOCATION: 241..406
(C) IDENTIFICATION METHOD: blastn
(D) OTHER INFORMATION: identity 99
region 66..231
id W01570
est

(ix) FEATURE:
(A) NAME/KEY: other
(B) LOCATION: 200..229
(C) IDENTIFICATION METHOD: blastn
(D) OTHER INFORMATION: identity 96
region 26..55
id W01570
est

(ix) FEATURE:
(A) NAME/KEY: other
(B) LOCATION: 296..406
(C) IDENTIFICATION METHOD: blastn
(D) OTHER INFORMATION: identity 99
region 1..111
id H82170
est

(ix) FEATURE:
(A) NAME/KEY: other
(B) LOCATION: 298..406
(C) IDENTIFICATION METHOD: blastn
(D) OTHER INFORMATION: identity 100
region 7..115
id N71014
est

(ix) FEATURE:
(A) NAME/KEY: other
(B) LOCATION: complement(147..201)
(C) IDENTIFICATION METHOD: blastn
(D) OTHER INFORMATION: identity 96
region 238..292
id N35296
est

(ix) FEATURE:
(A) NAME/KEY: sig_peptide
(B) LOCATION: 358..396
(C) IDENTIFICATION METHOD: Von Heijne matrix
(D) OTHER INFORMATION: score 3.9
seq MIHGFCLAPTTSA/KN

(xi) SEQUENCE DESCRIPTION: SEQ ID NO: 295:

ATGGGGGCGG ASTAGCCGGA GCCGCGAGTC CATTTTGGGG CTGTGCTTGG CGCGTACCGT 60

GCGGTCCCTG TAGTTGGAGG ACGGGCGGTC GCGCSGGCCT TTCCCACTAG CCGGAGGTCG 120

GAGATAAGTA CCCGCCGCCC GGCTTCTCTC GGGAAAGCGG GGTGGTCCTC GAACCTTCAG 180

CGAGGGTGGG GAGTTGCCCA GTAGCCTCTA GTTCGTTAGT CAAAACGTGA AAAAAAAAGA 240

CCTGCTTTGC CVTGGGAAAT AGTAACCCTG CCAAATACAT CAGCTTGTAG GAGACAGAGG 300

ATGTGATGGA GCTGCTTGAA GAAGATCTCA CATGCCCTAT TTGTTGTAGT CTGTTTG 357

ATG ATC CAC GGG TTT TGC CTT GCT CCC ACA ACT TCT GCA AAA AAT GCT 405
Met Ile His Gly Phe Cys Leu Ala Pro Thr Thr Ser Ala Lys Asn Ala
-10 -5 1

(2) INFORMATION FOR SEQ ID NO: 296:

(i) SEQUENCE CHARACTERISTICS:
(A) LENGTH: 167 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 24..86
(C) IDENTIFICATION METHOD: blastn
(D) OTHER INFORMATION: identity 95
region 1..63
id C16698
est

(ix) FEATURE:
(A) NAME/KEY: other
(B) LOCATION: 25..86
(C) IDENTIFICATION METHOD: blastn
(D) OTHER INFORMATION: identity 95
region 9..70
id H48377
est

(ix) FEATURE:
(A) NAME/KEY: other
(B) LOCATION: 38..86
(C) IDENTIFICATION METHOD: blastn
(D) OTHER INFORMATION: identity 95
region 36..84
id R17245
est

(ix) FEATURE:
(A) NAME/KEY: other
(B) LOCATION: 38..86
(C) IDENTIFICATION METHOD: blastn
(D) OTHER INFORMATION: identity 95
region 7..55
id H19182
est

(ix) FEATURE:
(A) NAME/KEY: other
(B) LOCATION: complement(19..54)
(C) IDENTIFICATION METHOD: blastn
(D) OTHER INFORMATION: identity 91
region 181..216
id T12463
est

(ix) FEATURE:
(A) NAME/KEY: sig_peptide
(B) LOCATION: 90..140
(C) IDENTIFICATION METHOD: Von Heijne matrix
(D) OTHER INFORMATION: score 3.7
seq RTWCLACVEASPG/QP

(xi) SEQUENCE DESCRIPTION: SEQ ID NO: 296:

AAGCCTGGGA CACCGCCGGC GGGGAGAGAA GCGGATCCCG TCCGAGCCCC GGCCCCAAGT 60

AACGCCGCCG CCCCGGAGCC GCCGTGAGT ATG CYT TGT CCC AGG ACC TGG TGT 113
Met Xaa Cys Pro Arg Thr Trp Cys
-15 -10

CTC GCC TGC GTT GAA GCA TCT CCA GGG CAG CCC TTC CTC CCG CCC CGC 161
Leu Ala Cys Val Glu Ala Ser Pro Gly Gln Pro Phe Leu Pro Pro Arg
-5 1 5

CCC GGG 167
Pro Gly

(2) INFORMATION FOR SEQ ID NO: 297:

(i) SEQUENCE CHARACTERISTICS:
(A) LENGTH: 224 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Dystrophic muscle

(ix) FEATURE:
(A) NAME/KEY: other
(B) LOCATION: 89..222
(C) IDENTIFICATION METHOD: blastn
(D) OTHER INFORMATION: identity 97
region 93..226
id W81645
est

(ix) FEATURE:
(A) NAME/KEY: other
(B) LOCATION: 26..90
(C) IDENTIFICATION METHOD: blastn
(D) OTHER INFORMATION: identity 95
region 31..95
id W81645
est

(ix) FEATURE:
(A) NAME/KEY: other
(B) LOCATION: 89..222
(C) IDENTIFICATION METHOD: blastn
(D) OTHER INFORMATION: identity 97
region 62..195
id W06951
est

(ix) FEATURE:
(A) NAME/KEY: other
(B) LOCATION: 33..90
(C) IDENTIFICATION METHOD: blastn
(D) OTHER INFORMATION: identity 96
region 7..64
id W06951
est

(ix) FEATURE:
(A) NAME/KEY: other
(B) LOCATION: 45..222
(C) IDENTIFICATION METHOD: blastn
(D) OTHER INFORMATION: identity 97
region 2..179
id W38711
est

(ix) FEATURE:
(A) NAME/KEY: sig_peptide

(B) LOCATION: 24..86
(C) IDENTIFICATION METHOD: Von Heijne matrix
(D) OTHER INFORMATION: score 3.7
seq ETCALASHSGSSG/SK

(xi) SEQUENCE DESCRIPTION: SEQ ID NO: 297:

GCNGTCGGCT CCGCGGCGCC GCC ATG GCC GAC GTG GAA GAC GGA GAG GAA ACC 53
Met Ala Asp Val Glu Asp Gly Glu Glu Thr
-20 -15

TGC GCC CTG GCC TCT CAC TCC GGG AGC TCA GGC TCC AAG TCG GGA GGC 101
Cys Ala Leu Ala Ser His Ser Gly Ser Ser Gly Ser Lys Ser Gly Gly
-10 -5 1 5

GAC AAG ATG TTC TCC CTC AAG AAG TGG AAC GCG GTG GCC ATG TGG AGC 149
Asp Lys Met Phe Ser Leu Lys Lys Trp Asn Ala Val Ala Met Trp Ser
10 15 20

TGG GAC GTG GAG TGC GAT ACG TGC GCC ATC TGC AGG GTC CAG GTG ATG 197
Trp Asp Val Glu Cys Asp Thr Cys Ala Ile Cys Arg Val Gln Val Met
25 30 35

GAT GCC TGT MTT AGA TGT CAA GCG GGG 224
Asp Ala Cys Xaa Arg Cys Gln Ala Gly
40 45

(2) INFORMATION FOR SEQ ID NO: 298:

(i) SEQUENCE CHARACTERISTICS:
(A) LENGTH: 356 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 122..188
(C) IDENTIFICATION METHOD: blastn
(D) OTHER INFORMATION: identity 100
region 198..264
id R58050
est

(ix) FEATURE:
(A) NAME/KEY: other
(B) LOCATION: complement(122..188)
(C) IDENTIFICATION METHOD: blastn
(D) OTHER INFORMATION: identity 100
region 193..259
id H98670

est

(ix) FEATURE:
(A) NAME/KEY: other
(B) LOCATION: complement(122..188)
(C) IDENTIFICATION METHOD: blastn
(D) OTHER INFORMATION: identity 100
region 194..260
id N66980
est

(ix) FEATURE:
(A) NAME/KEY: other
(B) LOCATION: complement(122..188)
(C) IDENTIFICATION METHOD: blastn
(D) OTHER INFORMATION: identity 100
region 198..264
id AA159781
est

(ix) FEATURE:
(A) NAME/KEY: other
(B) LOCATION: complement(122..188)
(C) IDENTIFICATION METHOD: blastn
(D) OTHER INFORMATION: identity 100
region 161..227
id H45410
est

(ix) FEATURE:
(A) NAME/KEY: sig_peptide
(B) LOCATION: 273..350
(C) IDENTIFICATION METHOD: Von Heijne matrix
(D) OTHER INFORMATION: score 3.7
seq IIMFLLIIVCGSP/RP

(xi) SEQUENCE DESCRIPTION: SEQ ID NO: 298:

TAGAAGTAGC AGGATCGCCT TAATAATAAT AATAGTTTTG TAGCATGAAG CCTGAGCATT 60

GTCCAAAGTT TGGAAATGTG AACGCTGATA GTCACATCTG TCCATCTTTC CACATTTCTA 120

GGATGCTGAC AGACAGCACC AAGAAGTAAT TGCAATTTAT CGGACACACC TTCTTAGTGC 180

TGCACAGGTA AAGAACTACT TCTCCTTTGG AAAGAATATT GCTTTAGAGA TAATAATTTT 240

TATTTTCAAA TAAATTTATG TGAAAGTAAT TG ATG TTT AAA GTA GCT GCA CCC 293
Met Phe Lys Val Ala Ala Pro
-25 -20

CCT ATG CTT ATT TAW KAA ATA ATT ATG TTT CTT TTA ATC ATT GTT TGT 341
Pro Met Leu Ile Xaa Xaa Ile Ile Met Phe Leu Leu Ile Ile Val Cys
-15 -10 -5

GGA TCT CCC AGG CCG 356
Gly Ser Pro Arg Pro
1

(2) INFORMATION FOR SEQ ID NO: 299:

(i) SEQUENCE CHARACTERISTICS:
(A) LENGTH: 216 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Muscle

(ix) FEATURE:
(A) NAME/KEY: other
(B) LOCATION: complement(87..181)
(C) IDENTIFICATION METHOD: blastn
(D) OTHER INFORMATION: identity 98
region 228..322
id N29854
est

(ix) FEATURE:
(A) NAME/KEY: other
(B) LOCATION: complement(1..46)
(C) IDENTIFICATION METHOD: blastn
(D) OTHER INFORMATION: identity 100
region 363..408
id N29854
est

(ix) FEATURE:
(A) NAME/KEY: other
(B) LOCATION: complement(44..93)
(C) IDENTIFICATION METHOD: blastn
(D) OTHER INFORMATION: identity 94
region 315..364
id N29854
est

(ix) FEATURE:
(A) NAME/KEY: other
(B) LOCATION: complement(87..181)
(C) IDENTIFICATION METHOD: blastn
(D) OTHER INFORMATION: identity 98
region 67..161
id T32629
est

(ix) FEATURE:
(A) NAME/KEY: other
(B) LOCATION: complement(1..93)
(C) IDENTIFICATION METHOD: blastn
(D) OTHER INFORMATION: identity 97
region 154..246
id T32629
est

(ix) FEATURE:
(A) NAME/KEY: other

(B) LOCATION: complement(87..181)
(C) IDENTIFICATION METHOD: blastn
(D) OTHER INFORMATION: identity 98
region 230..324
id W61289
est

(ix) FEATURE:
(A) NAME/KEY: other
(B) LOCATION: complement(6..93)
(C) IDENTIFICATION METHOD: blastn
(D) OTHER INFORMATION: identity 97
region 317..404
id W61289
est

(ix) FEATURE:
(A) NAME/KEY: other
(B) LOCATION: complement(87..181)
(C) IDENTIFICATION METHOD: blastn
(D) OTHER INFORMATION: identity 97
region 232..326
id N53422
est

(ix) FEATURE:
(A) NAME/KEY: other
(B) LOCATION: complement(3..93)
(C) IDENTIFICATION METHOD: blastn
(D) OTHER INFORMATION: identity 97
region 319..409
id N53422
est

(ix) FEATURE:
(A) NAME/KEY: other
(B) LOCATION: 87..181
(C) IDENTIFICATION METHOD: blastn
(D) OTHER INFORMATION: identity 98
region 78..172
id N50275
est

(ix) FEATURE:
(A) NAME/KEY: other
(B) LOCATION: 9..93
(C) IDENTIFICATION METHOD: blastn
(D) OTHER INFORMATION: identity 94
region 1..85
id N50275
est

(ix) FEATURE:
(A) NAME/KEY: sig_peptide
(B) LOCATION: 64..126
(C) IDENTIFICATION METHOD: Von Heijne matrix
(D) OTHER INFORMATION: score 3.6
seq FXMCLWSLRNLFS/RC

(xi) SEQUENCE DESCRIPTION: SEQ ID NO: 299:

AGCTATTTGG ATAGTGTAGC TTTAATGTGC TGCACATGAT ACTGGCAGCC CTAGAGTTCA 60

TAG ATG GAC TTT TGG GAC CCA GCA GTT TTT RAA ATG TGT TTA TGG AGT 108
Met Asp Phe Trp Asp Pro Ala Val Phe Xaa Met Cys Leu Trp Ser
-20 -15 -10

TTA AGA AAT TTA TTT TCC AGG TGC AGC CCC TGT CTA ACT GAA ATT TCT 156
Leu Arg Asn Leu Phe Ser Arg Cys Ser Pro Cys Leu Thr Glu Ile Ser
-5 1 5 10

CTT CAC CTT GTA CAC TTG ACA GCT GAA AAA AAA CAA CAT GGG AGT AAT 204
Leu His Leu Val His Leu Thr Ala Glu Lys Lys Gln His Gly Ser Asn
15 20 25

AAT GGG TCG GCG 216
Asn Gly Ser Ala
30

(2) INFORMATION FOR SEQ ID NO: 300:

(i) SEQUENCE CHARACTERISTICS:
(A) LENGTH: 273 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 9..122
(C) IDENTIFICATION METHOD: blastn
(D) OTHER INFORMATION: identity 97
region 1..114
id R56502
est

(ix) FEATURE:
(A) NAME/KEY: other
(B) LOCATION: 173..269
(C) IDENTIFICATION METHOD: blastn
(D) OTHER INFORMATION: identity 100
region 162..258
id R56502
est

(ix) FEATURE:
(A) NAME/KEY: other
(B) LOCATION: 129..172
(C) IDENTIFICATION METHOD: blastn
(D) OTHER INFORMATION: identity 95
region 119..162
id R56502

est

(ix) FEATURE:
(A) NAME/KEY: sig_peptide
(B) LOCATION: 160..261
(C) IDENTIFICATION METHOD: Von Heijne matrix
(D) OTHER INFORMATION: score 3.6
seq SVPLLSLSHSIGI/SP

(xi) SEQUENCE DESCRIPTION: SEQ ID NO: 300:
AGTGACCAAA TGACTTAACC ACAGATGGAG TGAAGACAGG GGTAACTGCT TGGTCTGGTC 60
CCCAGTAGAG CATTGCTCAC TATAAACCAC AAGCTGCTTC TAATTTATTT GAGRTGKTAW 120
TAAYCGTGGS CCTTKATATT CTGGTCTCTC TTGCTGCAA ATG AGT CCG GCA GGC 174
AAG CAC AAC TCA GAA AGC AAA TTC ACC TTC TTT GTA GCC CTT GAT GGG 222
TCG GTC CCC CTG TTG TCT CTT TCT CAT TCC ATA GGC ATT TCC CCC ACA 270
AGG 273

(2) INFORMATION FOR SEQ ID NO: 301:

(i) SEQUENCE CHARACTERISTICS:
(A) LENGTH: 163 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(F) TISSUE TYPE: Heart

(ix) FEATURE:
(A) NAME/KEY: other
(B) LOCATION: complement(78..160)
(C) IDENTIFICATION METHOD: blastn
(D) OTHER INFORMATION: identity 97
region 149..231
id H15081
est

(ix) FEATURE:
(A) NAME/KEY: other
(B) LOCATION: complement(1..71)
(C) IDENTIFICATION METHOD: blastn
(D) OTHER INFORMATION: identity 98

region 238..308
id H15081
est

(ix) FEATURE:
(A) NAME/KEY: other
(B) LOCATION: complement(2..71)
(C) IDENTIFICATION METHOD: blastn
(D) OTHER INFORMATION: identity 98
region 234..303
id H16744
est

(ix) FEATURE:
(A) NAME/KEY: other
(B) LOCATION: complement(78..160)
(C) IDENTIFICATION METHOD: blastn
(D) OTHER INFORMATION: identity 96
region 148..230
id R61691
est

(ix) FEATURE:
(A) NAME/KEY: other
(B) LOCATION: complement(2..72)
(C) IDENTIFICATION METHOD: blastn
(D) OTHER INFORMATION: identity 98
region 236..306
id R61691
est

(ix) FEATURE:
(A) NAME/KEY: other
(B) LOCATION: complement(2..85)
(C) IDENTIFICATION METHOD: blastn
(D) OTHER INFORMATION: identity 90
region 223..306
id H17833
est

(ix) FEATURE:
(A) NAME/KEY: other
(B) LOCATION: complement(109..160)
(C) IDENTIFICATION METHOD: blastn
(D) OTHER INFORMATION: identity 96
region 148..199
id H17833
est

(ix) FEATURE:
(A) NAME/KEY: sig_peptide
(B) LOCATION: 23..73
(C) IDENTIFICATION METHOD: Von Heijne matrix
(D) OTHER INFORMATION: score 3.5
seq LVCVGLHTEGPWG/RP

(xi) SEQUENCE DESCRIPTION: SEQ ID NO: 301:
ATGTGGTGBT TGTGTCTTAA CT ATG CAC TGG GCC CTT GTC TGC GTC GGC TTG 52
CAT ACA GAG GGC CCC TGG GGT CGG CCC TCC GGC CTG GCC TCA GCC AGT     100
GGG ATG GAC AGG GCC AGG CAG GCC TCT GAA CTT CCA CCT CCT GGG GCC     148
TCC CAG ACC CCC CAG                                                 163

(2) INFORMATION FOR SEQ ID NO: 302:

(i) SEQUENCE CHARACTERISTICS:
(A) LENGTH: 256 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 86..256
(C) IDENTIFICATION METHOD: blastn
(D) OTHER INFORMATION: identity 98
region 155..325
id H16532
est

(ix) FEATURE:
(A) NAME/KEY: other
(B) LOCATION: 5..62
(C) IDENTIFICATION METHOD: blastn
(D) OTHER INFORMATION: identity 98
region 1..58
id H16532
est

(ix) FEATURE:
(A) NAME/KEY: other
(B) LOCATION: 86..256
(C) IDENTIFICATION METHOD: blastn
(D) OTHER INFORMATION: identity 98
region 170..340
id H17763
est

(ix) FEATURE:
(A) NAME/KEY: other
(B) LOCATION: 8..62

(C) IDENTIFICATION METHOD: blastn
(D) OTHER INFORMATION:  identity 100
                        region 1..55
                        id H17763
                        est

(ix) FEATURE:
     (A) NAME/KEY: other
     (B) LOCATION: 86..165
     (C) IDENTIFICATION METHOD: blastn
     (D) OTHER INFORMATION:  identity 96
                             region 168..247
                             id R21494
                             est

(ix) FEATURE:
     (A) NAME/KEY: other
     (B) LOCATION: 11..62
     (C) IDENTIFICATION METHOD: blastn
     (D) OTHER INFORMATION:  identity 100
                             region 1..52
                             id R21494
                             est

(ix) FEATURE:
     (A) NAME/KEY: other
     (B) LOCATION: 183..222
     (C) IDENTIFICATION METHOD: blastn
     (D) OTHER INFORMATION:  identity 97
                             region 268..307
                             id R21494
                             est

(ix) FEATURE:
     (A) NAME/KEY: other
     (B) LOCATION: 86..238
     (C) IDENTIFICATION METHOD: blastn
     (D) OTHER INFORMATION:  identity 98
                             region 111..263
                             id AA084554
                             est

(ix) FEATURE:
     (A) NAME/KEY: other
     (B) LOCATION: 86..256
     (C) IDENTIFICATION METHOD: blastn
     (D) OTHER INFORMATION:  identity 97
                             region 136..306
                             id R52491
                             est

(ix) FEATURE:
     (A) NAME/KEY: sig_peptide
     (B) LOCATION: 20..235
     (C) IDENTIFICATION METHOD: Von Heijne matrix
     (D) OTHER INFORMATION:  score 3.5
                             seq WFYIGSSLNGTRG/KR

(xi) SEQUENCE DESCRIPTION: SEQ ID NO: 302:

AGAGCTCGCT GTGGCCCGG ATG TTC GGT GCA GCT GCC AGA TCC GCT GAT CTA     52
                    Met Phe Gly Ala Ala Ala Arg Ser Ala Asp Leu
                            -70                 -65

GTG CTT CTC GAA AAA AAC CTT CAG GCG GCC CAT GGG TAT GCC CAA GAG     100
Val Leu Leu Glu Lys Asn Leu Gln Ala Ala His Gly Tyr Ala Gln Glu
    -60                 -55                 -50

GAC AGA GAA CGA ATG CAC AGA DRT ATT GTC AGC CTT GSA CAG AAT CTC     148
Asp Arg Glu Arg Met His Arg Xaa Ile Val Ser Leu Xaa Gln Asn Leu
-45                 -40                 -35                 -30

CTG AAC TTT ATG ATT GGC TCT ATC TTG GAT TTA TGG CAA TGC TTC CTC     196
Leu Asn Phe Met Ile Gly Ser Ile Leu Asp Leu Trp Gln Cys Phe Leu
                -25                 -20                 -15

TGG TTT TAC ATT GGT TCT TCA TTG AAT GGT ACT CGG GGA AAA AGA GTT     244
Trp Phe Tyr Ile Gly Ser Ser Leu Asn Gly Thr Arg Gly Lys Arg Val
            -10                 -5                  1

CCA GCG CAC TTT                                                     256
Pro Ala His Phe
    5

(2) INFORMATION FOR SEQ ID NO: 303:

    (i) SEQUENCE CHARACTERISTICS:
        (A) LENGTH: 132 base pairs
        (B) TYPE: NUCLEIC ACID
        (C) STRANDEDNESS: DOUBLE
        (D) TOPOLOGY: LINEAR

    (ii) MOLECULE TYPE: CDNA

    (vi) ORIGINAL SOURCE:
        (A) ORGANISM: Homo Sapiens
        (F) TISSUE TYPE: Heart

    (ix) FEATURE:
        (A) NAME/KEY: other
        (B) LOCATION: 3..116
        (C) IDENTIFICATION METHOD: blastn
        (D) OTHER INFORMATION:  identity 100
                                region 1..114
                                id N87112
                                est

    (ix) FEATURE:
        (A) NAME/KEY: other
        (B) LOCATION: 50..130
        (C) IDENTIFICATION METHOD: blastn
        (D) OTHER INFORMATION:  identity 98
                                region 1..81
                                id AA094982
                                est

    (ix) FEATURE:
        (A) NAME/KEY: other
        (B) LOCATION: 52..130

(C) IDENTIFICATION METHOD: blastn
(D) OTHER INFORMATION: identity 100
region 1..79
id T68050
est

(ix) FEATURE:
(A) NAME/KEY: other
(B) LOCATION: 47..130
(C) IDENTIFICATION METHOD: blastn
(D) OTHER INFORMATION: identity 96
region 1..84
id AA157180
est

(ix) FEATURE:
(A) NAME/KEY: other
(B) LOCATION: 50..130
(C) IDENTIFICATION METHOD: blastn
(D) OTHER INFORMATION: identity 97
region 1..81
id AA186993
est

(ix) FEATURE:
(A) NAME/KEY: sig_peptide
(B) LOCATION: 43..123
(C) IDENTIFICATION METHOD: Von Heijne matrix
(D) OTHER INFORMATION: score 3.5
seq VVALLIVCDVPSA/SA

(xi) SEQUENCE DESCRIPTION: SEQ ID NO: 303:

AGCCGGAGCA AAGTTTCACT TATAGAAGGG AGAGGAGCGA AC ATG GCA GCG CGT 54
Met Ala Ala Arg
-25

TGG CGG TTT TGG TGT GTC TCT GTG ACC ATG GTG GTG GCG CTG CTC ATC 102
Trp Arg Phe Trp Cys Val Ser Val Thr Met Val Val Ala Leu Leu Ile
-20 -15 -10

GTT TGC GAC GTT CCC TCA GCC TCT GCC CGG 132
Val Cys Asp Val Pro Ser Ala Ser Ala Arg
-5 1

(2) INFORMATION FOR SEQ ID NO: 304:

(i) SEQUENCE CHARACTERISTICS:
(A) LENGTH: 436 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal

(F) TISSUE TYPE: kidney

(ix) FEATURE:
(A) NAME/KEY: other
(B) LOCATION: 73..238
(C) IDENTIFICATION METHOD: blastn
(D) OTHER INFORMATION: identity 100
region 16..181
id W32979
est

(ix) FEATURE:
(A) NAME/KEY: other
(B) LOCATION: 316..394
(C) IDENTIFICATION METHOD: blastn
(D) OTHER INFORMATION: identity 96
region 260..338
id W32979
est

(ix) FEATURE:
(A) NAME/KEY: other
(B) LOCATION: 251..322
(C) IDENTIFICATION METHOD: blastn
(D) OTHER INFORMATION: identity 98
region 194..265
id W32979
est

(ix) FEATURE:
(A) NAME/KEY: other
(B) LOCATION: 251..437
(C) IDENTIFICATION METHOD: blastn
(D) OTHER INFORMATION: identity 99
region 107..293
id AA128556
est

(ix) FEATURE:
(A) NAME/KEY: other
(B) LOCATION: 145..238
(C) IDENTIFICATION METHOD: blastn
(D) OTHER INFORMATION: identity 100
region 1..94
id AA128556
est

(ix) FEATURE:
(A) NAME/KEY: other
(B) LOCATION: 251..381
(C) IDENTIFICATION METHOD: blastn
(D) OTHER INFORMATION: identity 98
region 104..234
id T20234
est

(ix) FEATURE:
(A) NAME/KEY: other
(B) LOCATION: 153..238
(C) IDENTIFICATION METHOD: blastn
(D) OTHER INFORMATION: identity 98

region 6..91
id T20234
est

(ix) FEATURE:
(A) NAME/KEY: other
(B) LOCATION: 383..437
(C) IDENTIFICATION METHOD: blastn
(D) OTHER INFORMATION: identity 98
region 235..289
id T20234
est

(ix) FEATURE:
(A) NAME/KEY: other
(B) LOCATION: 115..238
(C) IDENTIFICATION METHOD: blastn
(D) OTHER INFORMATION: identity 100
region 65..188
id T32594
est

(ix) FEATURE:
(A) NAME/KEY: other
(B) LOCATION: 251..318
(C) IDENTIFICATION METHOD: blastn
(D) OTHER INFORMATION: identity 95
region 201..268
id T32594
est

(ix) FEATURE:
(A) NAME/KEY: other
(B) LOCATION: 52..115
(C) IDENTIFICATION METHOD: blastn
(D) OTHER INFORMATION: identity 98
region 1..64
id T32594
est

(ix) FEATURE:
(A) NAME/KEY: sig_peptide
(B) LOCATION: 245..292
(C) IDENTIFICATION METHOD: Von Heijne matrix
(D) OTHER INFORMATION: score 3.5
seq LLLQPSMIQEVWT/XY

(xi) SEQUENCE DESCRIPTION: SEQ ID NO: 304:

ATCAGACGCC AGTATAAGCC TTTGAGTCTC AATAGACTGC AGTATCTTAT TGATTTGGGT 60

CGTGTTGATC CTAGTCAACC TATTGACTTA ACCCAGCTTG TCAATGGGAG AGGTGTGACC 120

ATCCAGCCAC TTAAAAGGGA TTATGGTGTC CAGCTGGTTG AGGAGGGTGC TGACACCTTT 180

ACGGCAAAAG TTAATATTGA AGTACAGTTG GCTTCAGAAC TAGCTATTGC TGCCATTGAA 240

AAAA ATG GTG GTG TTG TTA CTA CAG CCT TCT ATG ATC CAA GAA GTC TGG 289
Met Val Val Leu Leu Leu Gln Pro Ser Met Ile Gln Glu Val Trp
-15 -10 -5

ACA THG TAT GCA AAC CTG TTC CAT TCT TTC TTC GTG GAC AAC CCA TTC     337
Thr Xaa Tyr Ala Asn Leu Phe His Ser Phe Phe Val Asp Asn Pro Phe
  1               5                  10                  15

CAA AAA GAA TGC TTC CAC CAG AAG AAC TGG TAC CAT ATT ACA CTG ATG     385
Gln Lys Glu Cys Phe His Gln Lys Asn Trp Tyr His Ile Thr Leu Met
                 20                  25                  30

CAA AGA ACC GTG GGT ACC TGG CGG ATC CTG CCA AAT TTC CTG AAG CAC     433
Gln Arg Thr Val Gly Thr Trp Arg Ile Leu Pro Asn Phe Leu Lys His
             35                  40                  45

GAC                                                                 436
Asp

(2) INFORMATION FOR SEQ ID NO: 305:

(i) SEQUENCE CHARACTERISTICS:
(A) LENGTH: 406 base pairs
(B) TYPE: NUCLEIC ACID
(C) STRANDEDNESS: DOUBLE
(D) TOPOLOGY: LINEAR

(ii) MOLECULE TYPE: CDNA

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: other
(B) LOCATION: 82..407
(C) IDENTIFICATION METHOD: fasta
(D) OTHER INFORMATION:  identity 98.5
                        region 1..326
                        id HSARSE
                        vrt

(ix) FEATURE:
(A) NAME/KEY: other
(B) LOCATION: 88..171
(C) IDENTIFICATION METHOD: blastn
(D) OTHER INFORMATION:  identity 96
                        region 1..84
                        id AA160312
                        est

(ix) FEATURE:
(A) NAME/KEY: sig_peptide
(B) LOCATION: 149..241
(C) IDENTIFICATION METHOD: Von Heijne matrix
(D) OTHER INFORMATION:  score 10.5
                        seq LAVLLSLAPSASS/DI

(xi) SEQUENCE DESCRIPTION: SEQ ID NO: 305:

AAAACTCGAA GTTAATCATT CCCAGCTCAA AGCCTTGTGC AAGTGCTCTC TGCCTTCACG 60

CTTGCTTCCT TTGGGAGAGA ACCTTCCTCT TCTTGATCGG GGATTCAGGA AGGAGCCCAG 120

GRGCAGAGGA AGTAGAGAGA GAGRCAAC ATG TTA CAT CTG CAC CMT TCT TGT 172
Met Leu His Leu His Xaa Ser Cys
-30 -25

TTG TGT TTC AGG AGC TGG CTG CCA GCG ATG CTC GCT GTA CTG CTA AGT 220
Leu Cys Phe Arg Ser Trp Leu Pro Ala Met Leu Ala Val Leu Leu Ser
-20 -15 -10

TTG GCA CCA TCA GCT TCC AGC GAC ATT TCC GCC TCC CGA CCG AAC ATC 268
Leu Ala Pro Ser Ala Ser Ser Asp Ile Ser Ala Ser Arg Pro Asn Ile
-5 1 5

CTT CTT CTG ATG GCG GAC GAC CTT GGC ATT GGG GAC ATT GGC TGC TAT 316
Leu Leu Leu Met Ala Asp Asp Leu Gly Ile Gly Asp Ile Gly Cys Tyr
10 15 20 25

GGC AAC AAC ACC ATG AGG ACT CCG ARN ATT GAC CGC CTT GCA GAG GAC 364
Gly Asn Asn Thr Met Arg Thr Pro Xaa Ile Asp Arg Leu Ala Glu Asp
30 35 40

GGC GTG AAG CTG ACC CAA CAC ATC TCT GCC GCA TCT TTG TGC 406
Gly Val Lys Leu Thr Gln His Ile Ser Ala Ala Ser Leu Cys
45 50 55

(2) INFORMATION FOR SEQ ID NO: 306:

(i) SEQUENCE CHARACTERISTICS:
(A) LENGTH: 26 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -20..-1
(C) IDENTIFICATION METHOD: Von Heijne matrix
(D) OTHER INFORMATION: score 15.8
seq LLLLLLLRHGAQG/KP

(xi) SEQUENCE DESCRIPTION: SEQ ID NO: 306:

Met Met Trp Arg Pro Ser Val Leu Leu Leu Leu Leu Leu Leu Arg His
-20 -15 -10 -5

Gly Ala Gln Gly Lys Pro Ser Pro Asp Ala
1 5

(2) INFORMATION FOR SEQ ID NO: 307:

(i) SEQUENCE CHARACTERISTICS:
(A) LENGTH: 40 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -25..-1
(C) IDENTIFICATION METHOD: Von Heijne matrix
(D) OTHER INFORMATION: score 14
seq LAMLALLSPLSLA/QY

(xi) SEQUENCE DESCRIPTION: SEQ ID NO: 307:

Met Glu Arg Pro Leu Cys Ser His Leu Cys Ser Cys Leu Ala Met Leu
-25 -20 -15 -10

Ala Leu Leu Ser Pro Leu Ser Leu Ala Gln Tyr Asp Ser Trp Pro Xaa
-5 1 5

Xaa Pro Glu Tyr Phe Gln Gln Pro
10 15

(2) INFORMATION FOR SEQ ID NO: 308:

(i) SEQUENCE CHARACTERISTICS:
(A) LENGTH: 55 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -18..-1
(C) IDENTIFICATION METHOD: Von Heijne matrix
(D) OTHER INFORMATION: score 12.3
seq HILFLLLLPVAAA/QT

(xi) SEQUENCE DESCRIPTION: SEQ ID NO: 308:

Met Ile His Leu Gly His Ile Leu Phe Leu Leu Leu Leu Pro Val Ala
-15 -10 -5

Ala Ala Gln Thr Thr Pro Gly Glu Arg Ser Ser Leu Pro Ala Phe Tyr
        1               5                   10

Pro Gly Thr Ser Gly Ser Cys Ser Gly Cys Gly Ser Leu Ser Leu Pro
 15                 20                  25                  30

Leu Leu Ala Gly Leu Val Ala
                35

(2) INFORMATION FOR SEQ ID NO: 309:

(i) SEQUENCE CHARACTERISTICS:
(A) LENGTH: 31 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -22..-1
(C) IDENTIFICATION METHOD: Von Heijne matrix
(D) OTHER INFORMATION: score 12.2
seq LALALGLAQPASA/RR

(xi) SEQUENCE DESCRIPTION: SEQ ID NO: 309:

Met Ala Val Lys Leu Gly Thr Leu Leu Leu Ala Leu Ala Leu Gly Leu
        -20                 -15                 -10

Ala Gln Pro Ala Ser Ala Arg Arg Lys Leu Leu Val Phe Leu Leu
    -5                  1               5

(2) INFORMATION FOR SEQ ID NO: 310:

(i) SEQUENCE CHARACTERISTICS:
(A) LENGTH: 74 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 11.9

seq LVLEFLLLSPVEA/QQ

(xi) SEQUENCE DESCRIPTION: SEQ ID NO: 310:

Met Glu Thr Leu Gly Ala Leu Leu Val Leu Glu Phe Leu Leu Leu Ser
-20                 -15                 -10                 -5

Pro Val Glu Ala Gln Gln Ala Thr Glu His Arg Leu Lys Pro Trp Leu
                1               5                   10

Val Gly Leu Ala Ala Val Val Gly Phe Leu Phe Ile Val Tyr Leu Val
    15                  20                  25

Leu Leu Ala Asn Arg Leu Trp Cys Ser Lys Ala Arg Ala Glu Asp Glu
30                  35                  40

Glu Glu Thr Thr Phe Arg Met Glu Ser Gly
45                  50

(2) INFORMATION FOR SEQ ID NO: 311:

(i) SEQUENCE CHARACTERISTICS:
(A) LENGTH: 57 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -16..-1
(C) IDENTIFICATION METHOD: Von Heijne matrix
(D) OTHER INFORMATION: score 11.3
seq PLLLSSLLGGSQA/MD

(xi) SEQUENCE DESCRIPTION: SEQ ID NO: 311:

Met Leu Leu Pro Leu Leu Leu Ser Ser Leu Leu Gly Gly Ser Gln Ala
    -15                 -10                 -5

Met Asp Gly Arg Phe Trp Ile Arg Val Gln Glu Ser Val Met Val Pro
1               5                   10                  15

Glu Gly Leu Cys Ile Ser Val Xaa Leu Leu Phe Leu Leu Pro Pro Thr
            20                  25                  30

Arg Leu Asp Arg Val Tyr Pro Ser Arg
        35                  40

(2) INFORMATION FOR SEQ ID NO: 312:

(i) SEQUENCE CHARACTERISTICS:
(A) LENGTH: 136 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -14..-1
(C) IDENTIFICATION METHOD: Von Heijne matrix
(D) OTHER INFORMATION: score 10.7
seq LWLLFFLVTAIHA/EL

(xi) SEQUENCE DESCRIPTION: SEQ ID NO: 312:

Met Leu Trp Leu Leu Phe Phe Leu Val Thr Ala Ile His Ala Glu Leu
-10 -5 1

Cys Gln Pro Gly Ala Glu Asn Ala Phe Lys Val Arg Leu Ser Ile Arg
                5                   10                  15

Thr Ala Leu Gly Asp Lys Ala Tyr Ala Trp Asp Thr Asn Glu Glu Tyr
            20                  25                  30

Leu Phe Lys Ala Met Val Ala Phe Ser Met Arg Lys Val Pro Asn Arg
35 40 45 50

Glu Ala Thr Glu Ile Ser His Val Leu Leu Cys Asn Val Thr Gln Arg
55 60 65

Val Ser Phe Trp Phe Val Val Thr Asp Pro Ser Lys Asn His Thr Leu
70 75 80

Pro Ala Val Glu Val Gln Ser Ala Ile Arg Met Asn Lys Asn Arg Ile
                85                  90                  95

Asn Asn Ala Phe Phe Leu Asn Asp Gln Thr Leu Glu Phe Leu Lys Ile
            100                 105                 110

Pro Ser Thr Leu Ala Pro Thr Arg
        115                 120

(2) INFORMATION FOR SEQ ID NO: 313:

(i) SEQUENCE CHARACTERISTICS:
(A) LENGTH: 50 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -27..-1
(C) IDENTIFICATION METHOD: Von Heijne matrix
(D) OTHER INFORMATION: score 10.7
seq LPLLCLFLQGATA/VL

(xi) SEQUENCE DESCRIPTION: SEQ ID NO: 313:

Met Ala Gly Ser Pro Ser Arg Ala Ala Gly Arg Arg Leu Gln Leu Pro
-25 -20 -15

Leu Leu Cys Leu Phe Leu Gln Gly Ala Thr Ala Val Leu Phe Ala Val
-10 -5 1 5

Phe Val Arg Tyr Asn His Lys Thr Asp Ala Ala Leu Trp Xaa Arg Lys
10 15 20

Leu Gly

(2) INFORMATION FOR SEQ ID NO: 314:

(i) SEQUENCE CHARACTERISTICS:
(A) LENGTH: 55 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -39..-1
(C) IDENTIFICATION METHOD: Von Heijne matrix
(D) OTHER INFORMATION: score 10.6
seq ALALLLVLPLLWP/CS

(xi) SEQUENCE DESCRIPTION: SEQ ID NO: 314:

Met Lys Trp Pro Trp Thr Cys Leu Ala Ile Leu Cys Pro Gly Pro Val
-35 -30 -25

Leu Ser Pro Pro Cys Ser Gly Pro Xaa Leu Ala Leu Ala Leu Leu Leu
-20 -15 -10

Val Leu Pro Leu Leu Trp Pro Cys Ser Val Phe Gly His Ala Leu Cys
-5 1 5

Xaa Pro Ser Pro Ala Arg Arg

10                    15

(2) INFORMATION FOR SEQ ID NO: 315:

(i) SEQUENCE CHARACTERISTICS:
(A) LENGTH: 108 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -23..-1
(C) IDENTIFICATION METHOD: Von Heijne matrix
(D) OTHER INFORMATION:  score 10
seq PLLGLLLSLPAGA/DV

(xi) SEQUENCE DESCRIPTION: SEQ ID NO: 315:

Met Pro Ser Trp Ile Gly Ala Val Ile Leu Pro Leu Leu Gly Leu Leu
-20                 -15                 -10

Leu Ser Leu Pro Ala Gly Ala Asp Val Lys Ala Arg Ser Cys Gly Glu
-5                  1               5

Val Arg Gln Ala Tyr Gly Ala Lys Gly Phe Ser Leu Ala Asp Ile Pro
10                  15                  20                  25

Tyr Gln Glu Ile Ala Xaa Glu His Leu Arg Ile Cys Pro Gln Glu Tyr
30                  35                  40

Thr Cys Cys Thr Thr Glu Met Glu Asp Lys Leu Ser Gln Gln Ser Lys
45                  50                  55

Leu Glu Phe Glu Asn Leu Val Glu Glu Thr Ser His Phe Val Arg Thr
60                  65                  70

Thr Phe Val Ser Arg His Lys Lys Phe Asp Gly Arg
75                  80                  85

(2) INFORMATION FOR SEQ ID NO: 316:

(i) SEQUENCE CHARACTERISTICS:
(A) LENGTH: 48 amino acids
(B) TYPE: AMINO ACID
(D) TOPOLOGY: LINEAR

(ii) MOLECULE TYPE: PROTEIN

(vi) ORIGINAL SOURCE:

(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -28..-1
(C) IDENTIFICATION METHOD: Von Heijne matrix
(D) OTHER INFORMATION: score 10
seq LWLSLLVPSCLCA/SP

(xi) SEQUENCE DESCRIPTION: SEQ ID NO: 316:

Met Leu Leu His Trp Val Arg Ser Gln Xaa Xaa Ser Asp Xaa Lys Leu
-25 -20 -15

Trp Leu Ser Leu Leu Val Pro Ser Cys Leu Cys Ala Ser Pro Trp Pro
-10 -5 1

Leu Pro Ser Leu Pro Leu Leu Leu Pro Pro Ser Leu Leu Ser Leu Leu
5 10 15 20

(2) INFORMATION FOR SEQ ID NO: 317:

(i) SEQUENCE CHARACTERISTICS:
(A) LENGTH: 56 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -34..-1
(C) IDENTIFICATION METHOD: Von Heijne matrix
(D) OTHER INFORMATION: score 9.6
seq LLLFSLLVSPPTC/KV

(xi) SEQUENCE DESCRIPTION: SEQ ID NO: 317:

Met Lys Tyr Leu Arg His Arg Arg Pro Asn Ala Thr Leu Ile Leu Ala
-30 -25 -20

Ile Gly Ala Phe Thr Leu Leu Leu Phe Ser Leu Leu Val Ser Pro Pro
-15 -10 -5

Thr Cys Lys Val Gln Glu Gln Pro Pro Ala Ile Pro Glu Ala Leu Ala
1 5 10

Trp Xaa Thr Pro Pro Thr Arg Trp
15 20

(2) INFORMATION FOR SEQ ID NO: 318:

(i) SEQUENCE CHARACTERISTICS:
(A) LENGTH: 127 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -35..-1
(C) IDENTIFICATION METHOD: Von Heijne matrix
(D) OTHER INFORMATION: score 9.5
seq AMWWLLLWGVLQA/WP

(xi) SEQUENCE DESCRIPTION: SEQ ID NO: 318:

Met Pro Gly Pro Arg Val Trp Gly Lys Tyr Leu Trp Arg Ser Pro His
-35 -30 -25 -20

Ser Lys Gly Cys Pro Gly Ala Met Trp Trp Leu Leu Leu Trp Gly Val
-15 -10 -5

Leu Gln Ala Trp Pro Xaa Pro Gly Leu Arg Pro Leu Gly Pro Arg Ala
1 5 10

Thr Pro Ala Ala Asp Ile Pro Arg Val Pro Arg Ala Val Trp Gln Arg
15 20 25

Pro Arg Glu Gln His Gly His Gln Gly Ser Arg Gly Leu Cys Cys Glu
30 35 40 45

Ala Arg Leu Pro Gly Leu Arg Pro Gly Ala Val Pro Gly Leu Cys Arg
    50                  55                  60

Gly Leu Xaa Xaa Asn Leu Ile Arg Arg Phe Gly Ser Lys Pro Val Leu
65 70 75

Trp Ser Ala Arg Leu Pro Ser Gly Gln Ala Pro Trp Ser Glu Gly
80 85 90

(2) INFORMATION FOR SEQ ID NO: 319:

(i) SEQUENCE CHARACTERISTICS:
(A) LENGTH: 71 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -37..-1
(C) IDENTIFICATION METHOD: Von Heijne matrix
(D) OTHER INFORMATION: score 9.2
seq LLAVLLASWRLWA/IK

(xi) SEQUENCE DESCRIPTION: SEQ ID NO: 319:

Met Cys Gly Pro Ala Met Phe Pro Ala Gly Pro Pro Trp Pro Arg Val
-35 -30 -25

Arg Val Val Gln Val Leu Trp Ala Leu Leu Ala Val Leu Leu Ala Ser
-20 -15 -10

Trp Arg Leu Trp Ala Ile Lys Asp Phe Gln Glu Cys Thr Trp Gln Val
-5 1 5 10

Val Leu Asn Glu Phe Lys Arg Val Gly Glu Ser Gly Val Ser Asp Xaa
15 20 25

Ser Leu Ser Lys Ser Pro Gly
30

(2) INFORMATION FOR SEQ ID NO: 320:

(i) SEQUENCE CHARACTERISTICS:
(A) LENGTH: 63 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -55..-1
(C) IDENTIFICATION METHOD: Von Heijne matrix
(D) OTHER INFORMATION: score 9.2
seq SLLLLSTALNILA/CQ

(xi) SEQUENCE DESCRIPTION: SEQ ID NO: 320:

Met His Arg Arg Lys Leu Pro Leu Thr Asn Lys Arg Gln Leu Gln Lys
-55 -50 -45 -40

Xaa Leu Ser Lys Phe Ile Phe Ser Asp Glu Leu Phe Arg Asn Ile Leu
-35 -30 -25

Phe Ser Leu Arg Thr Leu Arg Met Ile Leu Ser Leu Leu Leu Leu Ser

-20                    -15                    -10

Thr Ala Leu Asn Ile Leu Ala Cys Gln Ile Asn Glu Glu Leu Gly
        -5                  1                   5

(2) INFORMATION FOR SEQ ID NO: 321:

(i) SEQUENCE CHARACTERISTICS:
(A) LENGTH: 23 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -17..-1
(C) IDENTIFICATION METHOD: Von Heijne matrix
(D) OTHER INFORMATION:  score 8.3
                        seq VSALLMAWFGVLS/CV

(xi) SEQUENCE DESCRIPTION: SEQ ID NO: 321:

Met Lys Leu Trp Val Ser Ala Leu Leu Met Ala Trp Phe Gly Val Leu
        -15                 -10                 -5

Ser Cys Val Gln Ala Xaa Xaa
    1               5

(2) INFORMATION FOR SEQ ID NO: 322:

(i) SEQUENCE CHARACTERISTICS:
(A) LENGTH: 50 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION:  score 8.1
                        seq LCLVCLLVHTAFR/VV

(xi) SEQUENCE DESCRIPTION: SEQ ID NO: 322:

Met Gln Leu Pro Leu Ala Leu Cys Leu Val Cys Leu Leu Val His Thr
                -15                 -10                 -5

Ala Phe Arg Val Val Glu Gly Gln Gly Trp Gln Ala Phe Lys Asn Asp
            1               5                   10

Ala Thr Glu Ile Ile Pro Glu Leu Gly Glu Tyr Pro Glu Pro Pro Pro
    15                  20                  25

Glu Arg
 30

(2) INFORMATION FOR SEQ ID NO: 323:

    (i) SEQUENCE CHARACTERISTICS:
        (A) LENGTH: 36 amino acids
        (B) TYPE: AMINO ACID
        (D) TOPOLOGY: LINEAR

    (ii) MOLECULE TYPE: PROTEIN

    (vi) ORIGINAL SOURCE:
        (A) ORGANISM: Homo Sapiens
        (F) TISSUE TYPE: Muscle

    (ix) FEATURE:
        (A) NAME/KEY: sig_peptide
        (B) LOCATION: -31..-1
        (C) IDENTIFICATION METHOD: Von Heijne matrix
        (D) OTHER INFORMATION: score 8
                               seq ILLCSVAVXLSPS/EP

    (xi) SEQUENCE DESCRIPTION: SEQ ID NO: 323:

Met Leu Cys Ile His Xaa Xaa Arg Ile Ile Gln Asp Ser Phe Ile Ala
    -30                 -25                 -20

Leu Lys Ile Leu Leu Cys Ser Val Ala Val Xaa Leu Ser Pro Ser Glu
-15                 -10                 -5                   1

Pro Leu Ala Pro
            5

(2) INFORMATION FOR SEQ ID NO: 324:

    (i) SEQUENCE CHARACTERISTICS:
        (A) LENGTH: 71 amino acids
        (B) TYPE: AMINO ACID
        (D) TOPOLOGY: LINEAR

    (ii) MOLECULE TYPE: PROTEIN

    (vi) ORIGINAL SOURCE:
        (A) ORGANISM: Homo Sapiens
        (D) DEVELOPMENTAL STAGE: Fetal

(F) TISSUE TYPE: kidney

(ix) FEATURE:
(A) NAME/KEY: sig_peptide
(B) LOCATION: -38..-1
(C) IDENTIFICATION METHOD: Von Heijne matrix
(D) OTHER INFORMATION: score 7.9
seq LPFLSLFWPWAPG/AV

(xi) SEQUENCE DESCRIPTION: SEQ ID NO: 324:

Met Gly Gly Phe Phe Pro Pro Thr Glu Val Arg Glu Val Cys Ala Asn
-35 -30 -25

Gln Gly Ala Ala His Asn Arg Asp Arg Leu Pro Phe Leu Ser Leu Phe
-20 -15 -10

Trp Pro Trp Ala Pro Gly Ala Val Ser Val Gly Gln Ala Arg Tyr Arg
-5 1 5 10

Thr Pro Thr Thr Xaa Ala Pro Ser Ala Ser Val Pro Trp Pro Arg Ala
15 20 25

Gly Thr Cys Arg Thr Pro Thr
30

(2) INFORMATION FOR SEQ ID NO: 325:

(i) SEQUENCE CHARACTERISTICS:
(A) LENGTH: 37 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -30..-1
(C) IDENTIFICATION METHOD: Von Heijne matrix
(D) OTHER INFORMATION: score 7.9
seq HLWILLLFSFCWM/SR

(xi) SEQUENCE DESCRIPTION: SEQ ID NO: 325:

Met Lys Leu Phe Tyr Asn Gln Leu Val Ser Glu Thr Lys His Asp Phe
-30 -25 -20 -15

Ala His Leu Trp Ile Leu Leu Leu Phe Ser Phe Cys Trp Met Ser Arg
-10 -5 1

Ser Phe Phe Phe Phe
                5

(2) INFORMATION FOR SEQ ID NO: 326:

(i) SEQUENCE CHARACTERISTICS:
(A) LENGTH: 23 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -20..-1
(C) IDENTIFICATION METHOD: Von Heijne matrix
(D) OTHER INFORMATION: score 7.9
seq LLFFHILFHSCFS/HL

(xi) SEQUENCE DESCRIPTION: SEQ ID NO: 326:

(2) INFORMATION FOR SEQ ID NO: 327:

(i) SEQUENCE CHARACTERISTICS:
(A) LENGTH: 115 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -68..-1
(C) IDENTIFICATION METHOD: Von Heijne matrix
(D) OTHER INFORMATION: score 7.9
seq LLCSALAWQQSLS/GK

(xi) SEQUENCE DESCRIPTION: SEQ ID NO: 327:

Met Ser Ser Met Trp Ser Glu Tyr Thr Ile Gly Gly Val Lys Ile Tyr
-65 -60 -55

Phe Pro Tyr Lys Ala Tyr Pro Ser Gln Leu Ala Met Met Asn Ser Ile

-50                    -45                    -40

Leu Arg Gly Leu Asn Ser Lys Gln His Cys Leu Leu Glu Ser Pro Thr
    -35                     -30                     -25

Gly Ser Gly Lys Ser Leu Ala Leu Leu Cys Ser Ala Leu Ala Trp Gln
-20                     -15                     -10                     -5

Gln Ser Leu Ser Gly Lys Pro Ala Asp Glu Gly Val Ser Glu Lys Ala
                    1                   5                       10

Glu Val Gln Leu Ser Cys Cys Cys Ala Cys His Ser Lys Asp Phe Thr
        15                      20                      25

Asn Asn Asp Met Asn Gln Gly Thr Ser Arg His Phe Asn Tyr Pro Ser
    30                      35                      40

Thr Pro Arg
45

(2) INFORMATION FOR SEQ ID NO: 328:

(i) SEQUENCE CHARACTERISTICS:
(A) LENGTH: 37 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -28..-1
(C) IDENTIFICATION METHOD: Von Heijne matrix
(D) OTHER INFORMATION:   score 7.8
                         seq FVRFLGFVSCLQS/DP

(xi) SEQUENCE DESCRIPTION: SEQ ID NO: 328:

Met Ala Leu Phe Leu Glu Leu Phe Leu Asn Ser Tyr Ser Leu Leu Phe
            -25                     -20                     -15

Val Arg Phe Leu Gly Phe Val Ser Cys Leu Gln Ser Asp Pro Ile Cys
        -10                     -5                      1

Ser Phe Phe Phe Phe
                5

(2) INFORMATION FOR SEQ ID NO: 329:

(i) SEQUENCE CHARACTERISTICS:
(A) LENGTH: 72 amino acids

(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -24..-1
(C) IDENTIFICATION METHOD: Von Heijne matrix
(D) OTHER INFORMATION: score 7.8
seq LMAGSSLSAGVSG/ED

(xi) SEQUENCE DESCRIPTION: SEQ ID NO: 329:

Met Asn Glu Asp Glu Lys Glu Met Lys Glu Ile Leu Met Ala Gly Ser
-20 -15 -10

Ser Leu Ser Ala Gly Val Ser Gly Glu Asp Lys Thr Glu Ile Leu Asn
-5 1 5

Pro Thr Pro Xaa Met Ala Lys Ser Leu Thr Ile Asp Cys Leu Glu Leu
10 15 20

Ala Leu Pro Pro Glu Leu Ala Phe Gln Leu Asn Glu Leu Phe Gly Pro
25 30 35 40

Val Gly Ile Asp Ser Gly Ser Leu
45

(2) INFORMATION FOR SEQ ID NO: 330:

(i) SEQUENCE CHARACTERISTICS:
(A) LENGTH: 27 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -21..-1
(C) IDENTIFICATION METHOD: Von Heijne matrix
(D) OTHER INFORMATION: score 7.8
seq IIPLIXXLSLCLC/LW

(xi) SEQUENCE DESCRIPTION: SEQ ID NO: 330:

Met Gly Ser Phe Leu Leu Gly Gly Ile Ile Pro Leu Ile Xaa Xaa Leu

-20                    -15                    -10

Ser Leu Cys Leu Cys Leu Trp Trp Arg Ile Ile
 -5                  1                   5

(2) INFORMATION FOR SEQ ID NO: 331:

(i) SEQUENCE CHARACTERISTICS:
(A) LENGTH: 41 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -31..-1
(C) IDENTIFICATION METHOD: Von Heijne matrix
(D) OTHER INFORMATION:  score 7.8
                        seq VCLLCSGCSCAWS/VG

(xi) SEQUENCE DESCRIPTION: SEQ ID NO: 331:

Met Leu Gln Val Ala Thr Thr Asn Tyr Leu Glu Leu Ala Arg Glu Val
    -30                 -25                 -20

Lys Pro Val Cys Leu Leu Cys Ser Gly Cys Ser Cys Ala Trp Ser Val
-15                 -10                 -5                   1

Gly Cys Val Xaa Glu Ser Glu Ser Glu
            5                   10

(2) INFORMATION FOR SEQ ID NO: 332:

(i) SEQUENCE CHARACTERISTICS:
(A) LENGTH: 22 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -18..-1
(C) IDENTIFICATION METHOD: Von Heijne matrix
(D) OTHER INFORMATION:  score 7.7

seq PFFLALCFPKSTS/QP

(xi) SEQUENCE DESCRIPTION: SEQ ID NO: 332:

Met Phe Cys Leu Ala Pro Phe Phe Leu Ala Leu Cys Phe Pro Lys Ser
-15 -10 -5

Thr Ser Gln Pro Gln Arg
1

(2) INFORMATION FOR SEQ ID NO: 333:

(i) SEQUENCE CHARACTERISTICS:
(A) LENGTH: 72 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -32..-1
(C) IDENTIFICATION METHOD: Von Heijne matrix
(D) OTHER INFORMATION: score 7.5
seq QCLLCCISPPVFC/EG

(xi) SEQUENCE DESCRIPTION: SEQ ID NO: 333:

(2) INFORMATION FOR SEQ ID NO: 334:

(i) SEQUENCE CHARACTERISTICS:
(A) LENGTH: 50 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -23..-1
(C) IDENTIFICATION METHOD: Von Heijne matrix
(D) OTHER INFORMATION: score 7.3
seq SSCLLGLLHLSSQ/FS

(xi) SEQUENCE DESCRIPTION: SEQ ID NO: 334:

(2) INFORMATION FOR SEQ ID NO: 335:

(i) SEQUENCE CHARACTERISTICS:
(A) LENGTH: 62 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -19..-1
(C) IDENTIFICATION METHOD: Von Heijne matrix
(D) OTHER INFORMATION: score 7.2
seq FIXFPFLFPFSFS/QT

(xi) SEQUENCE DESCRIPTION: SEQ ID NO: 335:

Met Cys Leu Leu Phe Xaa Phe Ile Xaa Phe Pro Phe Leu Phe Pro Phe
-15 -10 -5

Ser Phe Ser Gln Thr Phe Ser Phe Ser Gln His Trp Asn Thr Gly Gly
1 5 10

Ser His Pro Glu Glu Leu Glu Arg Pro Gly Ala His Pro Arg Leu Lys
15 20 25

Ala Arg Pro Gln Pro Pro Leu Phe His Pro Phe Ile Ser Ser
30                  35                  40

(2) INFORMATION FOR SEQ ID NO: 336:

(i) SEQUENCE CHARACTERISTICS:
(A) LENGTH: 66 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -25..-1
(C) IDENTIFICATION METHOD: Von Heijne matrix
(D) OTHER INFORMATION:  score 7.1
seq LLVASGXAEGVSA/QS

(xi) SEQUENCE DESCRIPTION: SEQ ID NO: 336:

Met Ala Ser Glu Arg Xaa Pro Asn Arg Pro Xaa Cys Leu Leu Val Ala
-25                 -20                 -15                 -10

Ser Gly Xaa Ala Glu Gly Val Ser Ala Gln Ser Phe Leu Xaa Cys Phe
                -5                  1               5

Thr Met Ala Ser Thr Xaa Phe Asn Leu Gln Val Ala Xaa Pro Gly Gly
        10                  15                  20

Lys Ala Met Glu Phe Val Asp Val Thr Xaa Ser Asn Ala Arg Trp Val
    25                  30                  35

Gln Asp
40

(2) INFORMATION FOR SEQ ID NO: 337:

(i) SEQUENCE CHARACTERISTICS:
(A) LENGTH: 56 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:

(A) NAME/KEY: sig_peptide
(B) LOCATION: -25..-1
(C) IDENTIFICATION METHOD: Von Heijne matrix
(D) OTHER INFORMATION: score 7.1
seq LAFQLVFLRATSG/SC

(xi) SEQUENCE DESCRIPTION: SEQ ID NO: 337:

(2) INFORMATION FOR SEQ ID NO: 338:

(i) SEQUENCE CHARACTERISTICS:
(A) LENGTH: 109 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -25..-1
(C) IDENTIFICATION METHOD: Von Heijne matrix
(D) OTHER INFORMATION: score 7.1
seq LLLXLXLLLIALE/IM

(xi) SEQUENCE DESCRIPTION: SEQ ID NO: 338:

Met Arg Arg Ile Ser Leu Thr Ser Ser Pro Val Arg Leu Leu Leu Xaa
-25 -20 -15 -10

Leu Xaa Leu Leu Leu Ile Ala Leu Glu Ile Met Val Gly Gly His Ser
-5 1 5

Leu Cys Phe Asn Phe Thr Ile Lys Ser Leu Ser Arg Pro Gly Gln Pro
10 15 20

Trp Cys Glu Ala His Val Phe Leu Asn Lys Asn Leu Phe Leu Gln Tyr
25 30 35

Asn Ser Asp Asn Asn Met Val Lys Pro Leu Gly Leu Leu Gly Lys Lys
40 45 50 55

Val Tyr Ala Thr Ser Thr Trp Gly Glu Leu Thr Gln Thr Leu Gly Glu
                60                  65                  70

Val Gly Arg Asp Leu Arg Met Leu Leu Cys Asp Ile Lys
            75                  80

(2) INFORMATION FOR SEQ ID NO: 339:

(i) SEQUENCE CHARACTERISTICS:
(A) LENGTH: 18 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -14..-1
(C) IDENTIFICATION METHOD: Von Heijne matrix
(D) OTHER INFORMATION: score 7
seq TFLLLLFXNAGRS/LR

(xi) SEQUENCE DESCRIPTION: SEQ ID NO: 339:

Met Thr Phe Leu Leu Leu Leu Phe Xaa Asn Ala Gly Arg Ser Leu Arg
                -10                 -5                  1

Met Cys

(2) INFORMATION FOR SEQ ID NO: 340:

(i) SEQUENCE CHARACTERISTICS:
(A) LENGTH: 38 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -26..-1
(C) IDENTIFICATION METHOD: Von Heijne matrix
(D) OTHER INFORMATION: score 7
seq EMFLVLLVTGVHS/NK

(xi) SEQUENCE DESCRIPTION: SEQ ID NO: 340:

Met Arg Thr Val Val Leu Thr Met Lys Ala Ser Val Ile Glu Met Phe
    -25                 -20                 -15

Leu Val Leu Leu Val Thr Gly Val His Ser Asn Lys Glu Thr Ala Lys
-10                 -5                  1               5

Lys Ile Lys Arg Pro Gly
            10

(2) INFORMATION FOR SEQ ID NO: 341:

(i) SEQUENCE CHARACTERISTICS:
(A) LENGTH: 44 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -40..-1
(C) IDENTIFICATION METHOD: Von Heijne matrix
(D) OTHER INFORMATION: score 6.9
seq ISLLFIFFSIANS/SP

(xi) SEQUENCE DESCRIPTION: SEQ ID NO: 341:

(2) INFORMATION FOR SEQ ID NO: 342:

(i) SEQUENCE CHARACTERISTICS:
(A) LENGTH: 28 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -25..-1
(C) IDENTIFICATION METHOD: Von Heijne matrix
(D) OTHER INFORMATION: score 6.9
seq IPLLLLFFHLSFL/NS

(xi) SEQUENCE DESCRIPTION: SEQ ID NO: 342:

Met Tyr Leu Phe Cys Leu Phe Ser Val Ser Lys Thr Ile Pro Leu Leu
-25 -20 -15 -10

Leu Leu Phe Phe His Leu Ser Phe Leu Asn Ser Leu
-5 1

(2) INFORMATION FOR SEQ ID NO: 343:

(i) SEQUENCE CHARACTERISTICS:
(A) LENGTH: 43 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -16..-1
(C) IDENTIFICATION METHOD: Von Heijne matrix
(D) OTHER INFORMATION: score 6.9
seq CLLILKFLSPAET/SI

(xi) SEQUENCE DESCRIPTION: SEQ ID NO: 343:

Met Ile Val Cys Leu Leu Ile Leu Lys Phe Leu Ser Pro Ala Glu Thr
-15 -10 -5

Ser Ile Leu Ser Ser Ile Ala Thr Tyr Gly Ala Phe Tyr Phe Ile Val
1               5                   10                  15

Pro Leu Glu Val Ser Gln Ile Leu Gln Thr Gln
            20                  25

(2) INFORMATION FOR SEQ ID NO: 344:

(i) SEQUENCE CHARACTERISTICS:
(A) LENGTH: 32 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -25..-1
(C) IDENTIFICATION METHOD: Von Heijne matrix
(D) OTHER INFORMATION: score 6.7
seq LILCFLFILHTHT/HT

(xi) SEQUENCE DESCRIPTION: SEQ ID NO: 344:

Met Asp Lys Ser Ile Lys Ser Ser Ile Ile Trp Ser Leu Ile Leu Cys
-25 -20 -15 -10

Phe Leu Phe Ile Leu His Thr His Thr His Thr His Thr His Thr His
-5 1 5

(2) INFORMATION FOR SEQ ID NO: 345:

(i) SEQUENCE CHARACTERISTICS:
(A) LENGTH: 41 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -36..-1
(C) IDENTIFICATION METHOD: Von Heijne matrix
(D) OTHER INFORMATION: score 6.7
seq IFDLLLLLXXSNQ/LP

(xi) SEQUENCE DESCRIPTION: SEQ ID NO: 345:

Met Phe Phe Ile Phe Ile Asn Gly Phe Thr Leu Leu Leu Met Thr Leu
-35 -30 -25

Ala Met Lys Pro Arg His Pro Ile Phe Asp Leu Leu Leu Leu Leu Xaa
-20 -15 -10 -5

Xaa Ser Asn Gln Leu Pro Val Thr Gly
1               5

(2) INFORMATION FOR SEQ ID NO: 346:

(i) SEQUENCE CHARACTERISTICS:
(A) LENGTH: 71 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -60..-1
(C) IDENTIFICATION METHOD: Von Heijne matrix
(D) OTHER INFORMATION: score 6.7
seq LWPFLTWINPALS/IC

(xi) SEQUENCE DESCRIPTION: SEQ ID NO: 346:

Met Cys Pro Ser Leu Glu Glu Ala Pro Ser Val Lys Gly Thr Leu Pro
-60 -55 -50 -45

Cys Ser Gly Gln Gln Gln Pro Phe Pro Phe Gly Ala Ser Asn Ile Pro
-40 -35 -30

Leu Leu Leu Gly Arg Ser Arg Lys Val Ala Arg Gly Ala Pro Val Leu
-25 -20 -15

Trp Pro Phe Leu Thr Trp Ile Asn Pro Ala Leu Ser Ile Cys Asp Pro
-10 -5 1

Leu Gly Ser Cys Gly Trp Gln
5 10

(2) INFORMATION FOR SEQ ID NO: 347:

(i) SEQUENCE CHARACTERISTICS:
(A) LENGTH: 38 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -17..-1
(C) IDENTIFICATION METHOD: Von Heijne matrix
(D) OTHER INFORMATION: score 6.6
seq LLSALWFCHPCCL/CC

(xi) SEQUENCE DESCRIPTION: SEQ ID NO: 347:

Met Leu Gln Asp Leu Leu Ser Ala Leu Trp Phe Cys His Pro Cys Cys
        -15                 -10                 -5

Leu Cys Cys Gly Leu Cys Trp Leu Gly Val Asp Ala Gly Cys Ser Gln
    1               5                   10                  15

Gly Gly Ser Gly Cys Pro
                20

(2) INFORMATION FOR SEQ ID NO: 348:

(i) SEQUENCE CHARACTERISTICS:
(A) LENGTH: 58 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION: score 6.6
seq LLSLAAYLSGPHQ/EP

(xi) SEQUENCE DESCRIPTION: SEQ ID NO: 348:

(2) INFORMATION FOR SEQ ID NO: 349:

(i) SEQUENCE CHARACTERISTICS:
(A) LENGTH: 33 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -19..-1
(C) IDENTIFICATION METHOD: Von Heijne matrix
(D) OTHER INFORMATION: score 6.6
seq LLPGLPLVRTSFS/HF

(xi) SEQUENCE DESCRIPTION: SEQ ID NO: 349:

(2) INFORMATION FOR SEQ ID NO: 350:

(i) SEQUENCE CHARACTERISTICS:
(A) LENGTH: 58 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -25..-1
(C) IDENTIFICATION METHOD: Von Heijne matrix
(D) OTHER INFORMATION: score 6.5
seq GLAMLHVTRGVXG/SR

(xi) SEQUENCE DESCRIPTION: SEQ ID NO: 350:

Met Thr Val Glu Leu Trp Leu Arg Leu Arg Gly Lys Gly Leu Ala Met
-25 -20 -15 -10

Leu His Val Thr Arg Gly Val Xaa Gly Ser Arg Val Arg Val Xaa Xaa
-5 1 5

Xaa Leu Pro Ala Leu Leu Gly Xaa Pro Arg Ala Leu Ser Ser Xaa Ala
10 15 20

Ala Lys Met Gly Xaa Tyr Arg Xaa Met Trp
25 30

(2) INFORMATION FOR SEQ ID NO: 351:

(i) SEQUENCE CHARACTERISTICS:
(A) LENGTH: 40 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -24..-1
(C) IDENTIFICATION METHOD: Von Heijne matrix
(D) OTHER INFORMATION: score 6.4
seq LLILLCSSPPDRV/SY

(xi) SEQUENCE DESCRIPTION: SEQ ID NO: 351:

(2) INFORMATION FOR SEQ ID NO: 352:

(i) SEQUENCE CHARACTERISTICS:
(A) LENGTH: 51 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -20..-1
(C) IDENTIFICATION METHOD: Von Heijne matrix
(D) OTHER INFORMATION: score 6.4
seq FALLFLFLVPVPG/HG

(xi) SEQUENCE DESCRIPTION: SEQ ID NO: 352:

Met Arg Ile His Tyr Leu Leu Phe Ala Leu Leu Phe Leu Phe Leu Val
-20 -15 -10 -5

Pro Val Pro Gly His Gly Gly Ile Ile Asn Thr Leu Gln Lys Tyr Xaa

1                   5                   10

Leu Gln Ser Gln Arg Arg Pro Val Cys Cys Ala Gln Leu Pro Ser Lys
        15                  20                  25

Gly Glu Arg
    30

(2) INFORMATION FOR SEQ ID NO: 353:

(i) SEQUENCE CHARACTERISTICS:
(A) LENGTH: 53 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -13..-1
(C) IDENTIFICATION METHOD: Von Heijne matrix
(D) OTHER INFORMATION: score 6.4
seq MCLLTALVTQVIS/LR

(xi) SEQUENCE DESCRIPTION: SEQ ID NO: 353:

Met Cys Leu Leu Thr Ala Leu Val Thr Gln Val Ile Ser Leu Arg Lys
            -10                 -5                  1

Asn Ala Glu Arg Thr Cys Leu Cys Lys Arg Arg Trp Pro Trp Xaa Pro
    5                   10                  15

Ser Pro Arg Ile Tyr Cys Ser Ser Thr Pro Cys Asp Ser Lys Phe Pro
20                  25                  30                  35

Thr Val Tyr Ser Ser
                40

(2) INFORMATION FOR SEQ ID NO: 354:

(i) SEQUENCE CHARACTERISTICS:
(A) LENGTH: 27 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -18..-1 .
(C) IDENTIFICATION METHOD: Von Heijne matrix
(D) OTHER INFORMATION: score 6.3
seq GLALVAGTPPSRS/CP

(xi) SEQUENCE DESCRIPTION: SEQ ID NO: 354:

(2) INFORMATION FOR SEQ ID NO: 355:

(i) SEQUENCE CHARACTERISTICS:
(A) LENGTH: 91 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -38..-1
(C) IDENTIFICATION METHOD: Von Heijne matrix
(D) OTHER INFORMATION: score 6.3
seq PCVSLLWAPRXFA/SS

(xi) SEQUENCE DESCRIPTION: SEQ ID NO: 355:

Met Asn His Leu Met Pro Leu Thr Val Leu His Ser Val Leu Glu Met
-35 -30 -25

Leu Arg Thr Pro Arg Thr Pro Pro Trp Pro Cys Val Ser Leu Leu Trp
-20 -15 -10

Ala Pro Arg Xaa Phe Ala Ser Ser Cys Ser Gln Ala Phe Thr Thr Leu
-5 1 5 10

Xaa Xaa Asn Cys Leu Leu Thr Asn Pro Ser Pro Thr Leu Asp Cys Asp
15 20 25

Leu Pro Glu Gly Ser Glu Ile Leu Asn Ser Ser Leu Tyr Pro His Cys
30 35 40

Leu Leu Ser Ala Trp Asn Thr Arg His Ser Thr
45 50

(2) INFORMATION FOR SEQ ID NO: 356:

(i) SEQUENCE CHARACTERISTICS:
(A) LENGTH: 42 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -24..-1
(C) IDENTIFICATION METHOD: Von Heijne matrix
(D) OTHER INFORMATION: score 6.3
seq SLLXLRASQLSEG/DT

(xi) SEQUENCE DESCRIPTION: SEQ ID NO: 356:

Met Gly His Val Val Phe Gly Asp Ile Lys Asn Ser Leu Leu Xaa Leu
-20 -15 -10

Arg Ala Ser Gln Leu Ser Glu Gly Asp Thr Xaa Xaa Xaa Xaa Cys Pro
-5 1 5

Xaa Met Xaa Arg Gly Lys His Ile Ser Tyr
10 15

(2) INFORMATION FOR SEQ ID NO: 357:

(i) SEQUENCE CHARACTERISTICS:
(A) LENGTH: 98 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -81..-1
(C) IDENTIFICATION METHOD: Von Heijne matrix
(D) OTHER INFORMATION: score 6.3
seq FLSLLXSVSETPG/SL

(xi) SEQUENCE DESCRIPTION: SEQ ID NO: 357:

Met Ala Gly Gly Arg Arg Asp Tyr Ser Gln Leu Phe Gly Arg Gly Pro
-80 -75 -70

Gly Arg Leu Ser Arg Ala Arg Ala Ser Val Val Arg Trp Ser Pro Arg
-65                 -60            .    -55                 -50

Ala Thr Ala Cys Pro Ala Pro Pro Ser Leu Pro Asp Leu Lys Arg Gln
                -45                 -40                 -35

Glu Leu Val Ser Arg Ile Glu Cys Gly Cys Arg Gly Pro Val Gly Ala
            -30                 -25                 -20

Thr Ala Asp Phe Phe Leu Ser Leu Leu Xaa Ser Val Ser Glu Thr Pro
        -15                 -10                 -5

Gly Ser Leu Arg Xaa Asn Asp Leu Phe Phe Val Ser Gln Leu Ile Trp
    1               5                   10                  15

Gly Arg

(2) INFORMATION FOR SEQ ID NO: 358:

(i) SEQUENCE CHARACTERISTICS:
(A) LENGTH: 22 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION: score 6.1
seq LWCFHSFISFSLS/SS

(xi) SEQUENCE DESCRIPTION: SEQ ID NO: 358:

Met Phe Trp Xaa Gly Ser Leu Trp Cys Phe His Ser Phe Ile Ser Phe
                -15                 -10                 -5

Ser Leu Ser Ser Ser Arg
                1

(2) INFORMATION FOR SEQ ID NO: 359:

(i) SEQUENCE CHARACTERISTICS:
(A) LENGTH: 78 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -36..-1
(C) IDENTIFICATION METHOD: Von Heijne matrix
(D) OTHER INFORMATION: score 6
seq FLLTFFSYSLLHA/SR

(xi) SEQUENCE DESCRIPTION: SEQ ID NO: 359:

Met Ala Trp Pro Asn Val Phe Gln Xaa Gly Ser Leu Leu Ser Gln Phe
-35 -30 -25

Xaa Xaa His His Val Val Val Phe Leu Leu Thr Phe Phe Ser Tyr Ser
-20 -15 -10 -5

Leu Leu His Ala Ser Arg Lys Thr Phe Xaa Asn Val Lys Val Ser Ile
1 5 10

Ser Glu Gln Trp Thr Pro Ser Ala Phe Asn Thr Ser Val Glu Leu Pro
15 20 25

Val Glu Ile Trp Ser Ser Xaa His Leu Phe Pro Ser Ala Glu
30 35 40

(2) INFORMATION FOR SEQ ID NO: 360:

(i) SEQUENCE CHARACTERISTICS:
(A) LENGTH: 29 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION: score 6
seq WILAVGLSLPSSS/XI

(xi) SEQUENCE DESCRIPTION: SEQ ID NO: 360:

Met Ile Leu Arg Asn Leu Trp Ile Leu Ala Val Gly Leu Ser Leu Pro
-15 -10 -5

Ser Ser Ser Xaa Ile Lys Phe His Phe Ser Leu Tyr Ser
1               5                   10

(2) INFORMATION FOR SEQ ID NO: 361:

(i) SEQUENCE CHARACTERISTICS:
(A) LENGTH: 41 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -35..-1
(C) IDENTIFICATION METHOD: Von Heijne matrix
(D) OTHER INFORMATION: score 5.9
seq LCGLLHLWLKVFS/LK

(xi) SEQUENCE DESCRIPTION: SEQ ID NO: 361:

Met Leu Thr Val Asn Asp Val Arg Phe Tyr Arg Asn Val Arg Ser Asn
-35 -30 -25 -20

His Phe Pro Phe Val Arg Leu Cys Gly Leu Leu His Leu Trp Leu Lys
-15 -10 -5

Val Phe Ser Leu Lys Gln Leu Lys Lys
1               5

(2) INFORMATION FOR SEQ ID NO: 362:

(i) SEQUENCE CHARACTERISTICS:
(A) LENGTH: 54 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -23..-1
(C) IDENTIFICATION METHOD: Von Heijne matrix
(D) OTHER INFORMATION: score 5.9
seq LFLNLCILAXPFS/KQ

(xi) SEQUENCE DESCRIPTION: SEQ ID NO: 362:

Met Asn Leu Lys Pro Gly Leu Pro Cys Asn Leu Phe Leu Asn Leu Cys
-20 -15 -10

Ile Leu Ala Xaa Pro Phe Ser Lys Gln Ile Ile Glu Leu Leu Glu Tyr
-5 1 5

Val Ser Tyr His Pro Cys Val Leu Val Tyr Ser Glu Tyr Xaa Asn Ile
10 15 20 25

Ser Ile Val Tyr Thr Leu
30

(2) INFORMATION FOR SEQ ID NO: 363:

(i) SEQUENCE CHARACTERISTICS:
(A) LENGTH: 101 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -40..-1
(C) IDENTIFICATION METHOD: Von Heijne matrix
(D) OTHER INFORMATION: score 5.9
seq VVLAWGLLNVSMA/GM

(xi) SEQUENCE DESCRIPTION: SEQ ID NO: 363:

Met Met Gln Gly Glu Ala His Pro Ser Ala Ser Leu Ile Asp Arg Thr
-40 -35 -30 -25

Ile Lys Met Arg Lys Glu Thr Glu Ala Arg Lys Val Val Leu Ala Trp
-20 -15 -10

Gly Leu Leu Asn Val Ser Met Ala Gly Met Ile Tyr Thr Glu Met Thr
-5 1 5

Gly Lys Leu Ile Ser Ser Tyr Tyr Asn Val Thr Tyr Trp Pro Leu Trp
10 15 20

Tyr Xaa Glu Leu Ala Leu Ala Ser Leu Phe Ser Leu Asn Ala Leu Phe
25 30 35 40

Asp Phe Trp Arg Tyr Phe Lys Tyr Thr Val Ala Pro Thr Ser Leu Val
45 50 55

Val Ser Pro Gly Arg
60

(2) INFORMATION FOR SEQ ID NO: 364:

(i) SEQUENCE CHARACTERISTICS:
(A) LENGTH: 58 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION: score 5.9
seq PXXLLILAHITQS/CP

(xi) SEQUENCE DESCRIPTION: SEQ ID NO: 364:

Met Met Asn Gln Thr His Pro Xaa Xaa Leu Leu Ile Leu Ala His Ile
-15 -10 -5

Thr Gln Ser Cys Pro Trp Ala His Val Gly Ala Ala Pro Ser Ala Leu
1 5 10

Leu Ile His Arg Trp Glu Leu Arg Gly Cys Ser Tyr Leu Lys Leu Phe
15 20 25

Leu Val Met Val Leu Ile Phe Glu Met Leu
30 35

(2) INFORMATION FOR SEQ ID NO: 365:

(i) SEQUENCE CHARACTERISTICS:
(A) LENGTH: 107 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 5.8
seq GLVLLLSLAEILF/KI

(xi) SEQUENCE DESCRIPTION: SEQ ID NO: 365:

Met Gly Leu Pro Glu Arg Arg Gly Leu Val Leu Leu Leu Ser Leu Ala
-20                 -15                 -10                 -5

Glu Ile Leu Phe Lys Ile Met Ile Leu Glu Gly Gly Gly Val Met Asn
                1                   5                   10

Leu Asn Pro Gly Asn Asn Leu Leu His Gln Pro Pro Ala Trp Thr Asp
        15                  20                  25

Ser Tyr Ser Thr Cys Asn Val Ser Ser Gly Phe Phe Gly Gly Gln Trp
    30                  35                  40

His Glu Ile His Pro Gln Tyr Trp Thr Lys Tyr Gln Val Trp Glu Trp
45                  50                  55                  60

Leu Gln His Leu Leu Asp Thr Asn Gln Leu Asp Ala Asn Cys Ile Pro
                65                  70                  75

Phe Gln Glu Phe Asp Ile Asn Gly Glu Xaa Arg
            80                  85

(2) INFORMATION FOR SEQ ID NO: 366:

(i) SEQUENCE CHARACTERISTICS:
(A) LENGTH: 35 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -28..-1
(C) IDENTIFICATION METHOD: Von Heijne matrix
(D) OTHER INFORMATION: score 5.8
seq LCWALLYNCFSSS/CV

(xi) SEQUENCE DESCRIPTION: SEQ ID NO: 366:

Met Trp Gly Leu Glu Glu Asp Arg Ser Tyr Gln Gly Leu Arg Pro Leu
            -25                 -20                 -15

Cys Trp Ala Leu Leu Tyr Asn Cys Phe Ser Ser Ser Cys Val Pro Val
        -10                 -5                  1

Ala Leu Val
  5

(2) INFORMATION FOR SEQ ID NO: 367:

(i) SEQUENCE CHARACTERISTICS:
(A) LENGTH: 91 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -85..-1
(C) IDENTIFICATION METHOD: Von Heijne matrix
(D) OTHER INFORMATION: score 5.7
seq ALLASLGIAFSRS/RA

(xi) SEQUENCE DESCRIPTION: SEQ ID NO: 367:

Met Leu Cys Arg Asp Gly Ser Ala Cys Val Pro Arg Ser Arg Arg Leu
-85 -80 -75 -70

Pro Leu Pro Ala Ala Val Arg Ala His Gly Pro Met Ala Asp Xaa Xaa
-65 -60 -55

Asp Ser Ala Arg Gly Cys Val Val Phe Glu Asp Val Phe Val Tyr Phe
-50 -45 -40

Ser Arg Glu Glu Trp Glu Leu Leu Asp Asp Ala Gln Arg Leu Leu Tyr
-35 -30 -25

His Asp Val Met Leu Glu Asn Phe Ala Leu Leu Ala Ser Leu Gly Ile
-20 -15 -10

Ala Phe Ser Arg Ser Arg Ala Val Met Lys Leu
-5 1 5

(2) INFORMATION FOR SEQ ID NO: 368:

(i) SEQUENCE CHARACTERISTICS:
(A) LENGTH: 67 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -56..-1
(C) IDENTIFICATION METHOD: Von Heijne matrix

(D) OTHER INFORMATION: score 5.7
seq FLCFLNLTSHLSG/LD

(xi) SEQUENCE DESCRIPTION: SEQ ID NO: 368:

Met Leu Ile Thr Arg Leu Gln Ser Gly Ile Asp Phe Ala Ile Gln Leu
-55 -50 -45

Asp Glu Ser Thr Asp Ile Gly Ser Cys Thr Thr Leu Leu Val Tyr Val
-40 -35 -30 -25

Arg Tyr Ala Trp Gln Asp Asp Phe Leu Glu Asp Phe Leu Cys Phe Leu
-20 -15 -10

Asn Leu Thr Ser His Leu Ser Gly Leu Asp Ile Phe Thr Glu Leu Glu
-5 1 5

Arg Arg Gly
10

(2) INFORMATION FOR SEQ ID NO: 369:

(i) SEQUENCE CHARACTERISTICS:
(A) LENGTH: 64 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -38..-1
(C) IDENTIFICATION METHOD: Von Heijne matrix
(D) OTHER INFORMATION: score 5.7
seq LAFLSCLAFLVLD/TQ

(xi) SEQUENCE DESCRIPTION: SEQ ID NO: 369:

Met Glu Ser Pro Gln Leu His Cys Ile Leu Asn Ser Asn Ser Val Ala
-35 -30 -25

Cys Ser Phe Ala Val Gly Ala Gly Phe Leu Ala Phe Leu Ser Cys Leu
-20 -15 -10

Ala Phe Leu Val Leu Asp Thr Gln Glu Thr Arg Ile Ala Gly Thr Arg
-5 1 5 10

Phe Lys Thr Ala Phe Gln Leu Leu Asp Xaa Ile Leu Ala Val Leu Trp
15 20 25

(2) INFORMATION FOR SEQ ID NO: 370:

(i) SEQUENCE CHARACTERISTICS:
(A) LENGTH: 30 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -28..-1
(C) IDENTIFICATION METHOD: Von Heijne matrix
(D) OTHER INFORMATION: score 5.7
seq DHLFLLFPRSCSS/LV

(xi) SEQUENCE DESCRIPTION: SEQ ID NO: 370:

(2) INFORMATION FOR SEQ ID NO: 371:

(i) SEQUENCE CHARACTERISTICS:
(A) LENGTH: 26 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -24..-1
(C) IDENTIFICATION METHOD: Von Heijne matrix
(D) OTHER INFORMATION: score 5.6
seq FFFFLFLLPPXPP/TG

(xi) SEQUENCE DESCRIPTION: SEQ ID NO: 371:

Met Val Glu Leu Lys Gln Leu Gly Pro Arg Ser Phe Phe Phe Phe Leu
-20 -15 -10

Phe Leu Leu Pro Pro Xaa Pro Pro Thr Gly
-5 1

(2) INFORMATION FOR SEQ ID NO: 372:

(i) SEQUENCE CHARACTERISTICS:
(A) LENGTH: 45 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -26..-1
(C) IDENTIFICATION METHOD: Von Heijne matrix
(D) OTHER INFORMATION: score 5.5
seq LILPALFFFPLHC/TF

(xi) SEQUENCE DESCRIPTION: SEQ ID NO: 372:

Met Pro Tyr Val Thr Ile Pro Tyr Ile Ile Val Tyr Ser Leu Ile Leu
-25 -20 -15

Pro Ala Leu Phe Phe Phe Pro Leu His Cys Thr Phe His Gly Leu Thr
-10 -5 1 5

Tyr Tyr Ile Ser Cys Val Cys Ser Leu Ser Leu Pro Thr
10 15

(2) INFORMATION FOR SEQ ID NO: 373:

(i) SEQUENCE CHARACTERISTICS:
(A) LENGTH: 27 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -25..-1
(C) IDENTIFICATION METHOD: Von Heijne matrix
(D) OTHER INFORMATION: score 5.5
seq LLLCMDLPHSVLS/NW

(xi) SEQUENCE DESCRIPTION: SEQ ID NO: 373:

(2) INFORMATION FOR SEQ ID NO: 374:

      (i) SEQUENCE CHARACTERISTICS:
            (A) LENGTH: 23 amino acids
            (B) TYPE: AMINO ACID
            (D) TOPOLOGY: LINEAR

      (ii) MOLECULE TYPE: PROTEIN

      (vi) ORIGINAL SOURCE:
            (A) ORGANISM: Homo Sapiens
            (D) DEVELOPMENTAL STAGE: Fetal
            (F) TISSUE TYPE: kidney

      (ix) FEATURE:
            (A) NAME/KEY: sig_peptide
            (B) LOCATION: -21..-1
            (C) IDENTIFICATION METHOD: Von Heijne matrix
            (D) OTHER INFORMATION:  score 5.5
                                    seq EFLFLGFPSNSWP/HR

      (xi) SEQUENCE DESCRIPTION: SEQ ID NO: 374:

Met Leu Gln Ile Pro Glu Arg Arg Glu Phe Leu Phe Leu Gly Phe Pro
    -20                 -15                 -10

Ser Asn Ser Trp Pro His Arg
 -5                  1

(2) INFORMATION FOR SEQ ID NO: 375:

      (i) SEQUENCE CHARACTERISTICS:
            (A) LENGTH: 39 amino acids
            (B) TYPE: AMINO ACID
            (D) TOPOLOGY: LINEAR

      (ii) MOLECULE TYPE: PROTEIN

      (vi) ORIGINAL SOURCE:
            (A) ORGANISM: Homo Sapiens
            (F) TISSUE TYPE: Muscle

      (ix) FEATURE:
            (A) NAME/KEY: sig_peptide
            (B) LOCATION: -18..-1
            (C) IDENTIFICATION METHOD: Von Heijne matrix
            (D) OTHER INFORMATION:  score 5.5
                                    seq FLITLFCCCVVVG/FF

      (xi) SEQUENCE DESCRIPTION: SEQ ID NO: 375:

(2) INFORMATION FOR SEQ ID NO: 376:

(i) SEQUENCE CHARACTERISTICS:
(A) LENGTH: 27 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -25..-1
(C) IDENTIFICATION METHOD: Von Heijne matrix
(D) OTHER INFORMATION: score 5.5
seq CLLHLRCLQLYWA/AR

(xi) SEQUENCE DESCRIPTION: SEQ ID NO: 376:

(2) INFORMATION FOR SEQ ID NO: 377:

(i) SEQUENCE CHARACTERISTICS:
(A) LENGTH: 32 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -28..-1
(C) IDENTIFICATION METHOD: Von Heijne matrix

(D) OTHER INFORMATION:  score 5.4
                           seq PLSLALQSSCCLC/LT

(xi) SEQUENCE DESCRIPTION: SEQ ID NO: 377:

Met Val Asp Arg Asp Glu Asn Ile Leu Leu Lys Gln Ile Tyr Ser Pro
        -25                     -20                 -15

Leu Ser Leu Ala Leu Gln Ser Ser Cys Cys Leu Cys Leu Thr Ser Cys
    -10                     -5                  1

(2) INFORMATION FOR SEQ ID NO: 378:

(i) SEQUENCE CHARACTERISTICS:
(A) LENGTH: 33 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION:  score 5.4
                        seq VSVSLCVCDCVRG/ST

(xi) SEQUENCE DESCRIPTION: SEQ ID NO: 378:

Met Lys Val Lys Pro Pro Phe Val Ser Val Ser Leu Cys Val Cys Asp
-20                 -15                 -10                 -5

Cys Val Arg Gly Ser Thr Leu Thr Trp Asn Arg Leu Leu Arg Val Gly
                1               5                   10

Gly

(2) INFORMATION FOR SEQ ID NO: 379:

(i) SEQUENCE CHARACTERISTICS:
(A) LENGTH: 100 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -39..-1
(C) IDENTIFICATION METHOD: Von Heijne matrix
(D) OTHER INFORMATION: score 5.4
seq ILLTSCFYTLVSS/TF

(xi) SEQUENCE DESCRIPTION: SEQ ID NO: 379:

Met Ile Ser Ser Cys Gly Val Lys Tyr Leu Phe Ser His Ala Ser Leu
-35 -30 -25

Phe Phe Met Val Gly Ser Thr Gly Ser Leu Ile Leu Leu Thr Ser Cys
-20 -15 -10

Phe Tyr Thr Leu Val Ser Ser Thr Phe Leu Gln Lys Leu Ser Ser Leu
-5 1 5

Leu Leu Ile Leu Phe Thr Glu Thr Ser Val Leu Met Leu Lys Thr Phe
10 15 20 25

Val Ala Asn Ser Cys Cys Xaa Leu Trp Ser His Asn Cys Ile Asn Phe
30 35 40

Phe Lys Lys Val Xaa Pro Ser Tyr Cys Xaa Ser Ser Leu Leu Phe Leu
45 50 55

Ala Val Pro Arg
60

(2) INFORMATION FOR SEQ ID NO: 380:

(i) SEQUENCE CHARACTERISTICS:
(A) LENGTH: 25 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -20..-1
(C) IDENTIFICATION METHOD: Von Heijne matrix
(D) OTHER INFORMATION: score 5.4
seq SFLCNFLVSLSLS/FL

(xi) SEQUENCE DESCRIPTION: SEQ ID NO: 380:

(2) INFORMATION FOR SEQ ID NO: 381:

(i) SEQUENCE CHARACTERISTICS:
(A) LENGTH: 48 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -33..-1
(C) IDENTIFICATION METHOD: Von Heijne matrix
(D) OTHER INFORMATION:  score 5.4
seq LAYFLCCQGVIFG/SL

(xi) SEQUENCE DESCRIPTION: SEQ ID NO: 381:

Met Asp Ala Leu Glu Arg Gly Ser Leu Arg Asn Glu Gln Ala Leu Val
-30                 -25                 -20

Ile Tyr Ala Gly Leu Ala Tyr Phe Leu Cys Cys Gln Gly Val Ile Phe
-15                 -10                 -5

Gly Ser Leu Pro Ser Asn Ala Gly Ala Gly Pro Leu Gly Trp Ser Ser
1               5                   10                  15

(2) INFORMATION FOR SEQ ID NO: 382:

(i) SEQUENCE CHARACTERISTICS:
(A) LENGTH: 44 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -39..-1
(C) IDENTIFICATION METHOD: Von Heijne matrix
(D) OTHER INFORMATION:  score 5.3
seq SLWFLPLPTHVYT/HT

(xi) SEQUENCE DESCRIPTION: SEQ ID NO: 382:

Met Glu Tyr Leu Phe Gln Gln Pro Gly His Ser Arg Gly Glu Ala Arg
                -35                 -30                 -25

Ala Ala Ala Ala Ser Leu Glu Thr Leu Ser Ser Leu Trp Phe Leu Pro
            -20                 -15                 -10

Leu Pro Thr His Val Tyr Thr His Thr His Ala Asn
        -5                  1               5

(2) INFORMATION FOR SEQ ID NO: 383:

(i) SEQUENCE CHARACTERISTICS:
(A) LENGTH: 35 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -15..-1
(C) IDENTIFICATION METHOD: Von Heijne matrix
(D) OTHER INFORMATION: score 5.3
seq SSMLITILSFIFA/LG

(xi) SEQUENCE DESCRIPTION: SEQ ID NO: 383:

Met Val Ser Ser Met Leu Ile Thr Ile Leu Ser Phe Ile Phe Ala Leu
-15                 -10                 -5                  1

Gly Tyr His Thr Ala Ser Tyr Pro Val Ser Leu His Pro Leu Ser Phe
            5                   10                  15

Phe Leu His
        20

(2) INFORMATION FOR SEQ ID NO: 384:

(i) SEQUENCE CHARACTERISTICS:
(A) LENGTH: 30 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:

(A) NAME/KEY: sig_peptide
(B) LOCATION: -18..-1
(C) IDENTIFICATION METHOD: Von Heijne matrix
(D) OTHER INFORMATION: score 5.3
seq MNLVSALASSAXG/QR

(xi) SEQUENCE DESCRIPTION: SEQ ID NO: 384:

Met Pro Leu Phe Thr Met Asn Leu Val Ser Ala Leu Ala Ser Ser Ala
-15 -10 -5

Xaa Gly Gln Arg Gly Ala Gly Pro Ala Leu Trp His Leu Cys
1               5                   10

(2) INFORMATION FOR SEQ ID NO: 385:

(i) SEQUENCE CHARACTERISTICS:
(A) LENGTH: 41 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -39..-1
(C) IDENTIFICATION METHOD: Von Heijne matrix
(D) OTHER INFORMATION: score 5.2
seq LILLLHCSIRVFF/FF

(xi) SEQUENCE DESCRIPTION: SEQ ID NO: 385:

(2) INFORMATION FOR SEQ ID NO: 386:

(i) SEQUENCE CHARACTERISTICS:
(A) LENGTH: 66 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -53..-1
(C) IDENTIFICATION METHOD: Von Heijne matrix
(D) OTHER INFORMATION: score 5.2
seq SFLLLQLIHEDKA/IQ

(xi) SEQUENCE DESCRIPTION: SEQ ID NO: 386:

Met Lys Ile Pro Val Trp His Lys Thr Cys Phe Leu Lys Ser Glu Ser
-50 -45 -40

Phe Ser Pro Asp Asn Leu Ser Val Ser Leu Pro Cys Arg Pro Ser Gln
-35 -30 -25

Val Pro Ser Gln Gly Gln Gly Lys Ser Phe Leu Leu Leu Gln Leu Ile
-20 -15 -10

His Glu Asp Lys Ala Ile Gln Asn Glu Ala Ile Phe Gln Pro Ser Leu
-5 1 5 10

Gln Leu

(2) INFORMATION FOR SEQ ID NO: 387:

(i) SEQUENCE CHARACTERISTICS:
(A) LENGTH: 30 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION: score 5.2
seq FGCTFVAFXPAFA/LS

(xi) SEQUENCE DESCRIPTION: SEQ ID NO: 387:

Met Gly Ala Ala Val Phe Phe Gly Cys Thr Phe Val Ala Phe Xaa Pro
-15 -10 -5

Ala Phe Ala Leu Ser Leu Ile Thr Val Ala Gly Asp Arg Gly
1               5                   10

(2) INFORMATION FOR SEQ ID NO: 388:

(i) SEQUENCE CHARACTERISTICS:
(A) LENGTH: 93 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -34..-1
(C) IDENTIFICATION METHOD: Von Heijne matrix
(D) OTHER INFORMATION: score 5.2
seq LWSSCWLAPLADG/ML

(xi) SEQUENCE DESCRIPTION: SEQ ID NO: 388:

Met Val Gly Gly Leu Asp Pro Pro Gly Arg Arg Arg Phe Gln Lys Gly
-30 -25 -20

Phe Asp Trp Arg Asn Leu Trp Ser Ser Cys Trp Leu Ala Pro Leu Ala
-15 -10 -5

Asp Gly Met Leu Arg Tyr Met Gly Gln Xaa Gln Arg Xaa Ala Ser Asn
1 5 10

Pro Glu Gly Ser Thr Leu Glu Ala Arg Pro Pro Ala Pro Xaa Ala Ser
15 20 25 30

Val Ser Pro Ser Val Xaa Xaa Pro His Arg Pro Trp Ala Ala Lys Met
        35                  40                  45

Glu Thr Val Ser Pro Ala Thr Ser Xaa Ile Ala Gly Gly
50 55

(2) INFORMATION FOR SEQ ID NO: 389:

(i) SEQUENCE CHARACTERISTICS:
(A) LENGTH: 23 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:

(A) NAME/KEY: sig_peptide
(B) LOCATION: -21..-1
(C) IDENTIFICATION METHOD: Von Heijne matrix
(D) OTHER INFORMATION: score 5.1
seq SLLVVSCFYQISG/RW

(xi) SEQUENCE DESCRIPTION: SEQ ID NO: 389:

(2) INFORMATION FOR SEQ ID NO: 390:

(i) SEQUENCE CHARACTERISTICS:
(A) LENGTH: 23 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -15..-1
(C) IDENTIFICATION METHOD: Von Heijne matrix
(D) OTHER INFORMATION: score 5.1
seq VTQLLPFSSPDSA/GP

(xi) SEQUENCE DESCRIPTION: SEQ ID NO: 390:

Met Xaa Val Thr Gln Leu Leu Pro Phe Ser Ser Pro Asp Ser Ala Gly
-15 -10 -5 1

Pro Phe Leu Ser Pro Phe Ser
                5

(2) INFORMATION FOR SEQ ID NO: 391:

(i) SEQUENCE CHARACTERISTICS:
(A) LENGTH: 72 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -34..-1
(C) IDENTIFICATION METHOD: Von Heijne matrix
(D) OTHER INFORMATION: score 5.1
seq SFHFLPWALGAMA/SS

(xi) SEQUENCE DESCRIPTION: SEQ ID NO: 391:

Met Gly Lys Ala Trp Gln Glu Met Arg Val Glu Trp Gly Ala Asp Lys
-30 -25 -20

Gly Asn Val Arg Ser Ser Phe His Phe Leu Pro Trp Ala Leu Gly Ala
-15 -10 -5

Met Ala Ser Ser Glu Gln Gly Lys Glu Arg Ser Asn Leu Cys Phe Arg
1 5 10

Lys Thr Pro Leu Ala Ile Thr Gly Arg Gly Ile Ala Arg Arg Pro Gly
15 20 25 30

Gly Gly Trp Met Gly Met Trp Val
35

(2) INFORMATION FOR SEQ ID NO: 392:

(i) SEQUENCE CHARACTERISTICS:
(A) LENGTH: 55 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -47..-1
(C) IDENTIFICATION METHOD: Von Heijne matrix
(D) OTHER INFORMATION: score 5.1
seq VIRLSQFLLKCWP/RT

(xi) SEQUENCE DESCRIPTION: SEQ ID NO: 392:

Met Lys Val Met Met Arg Lys Arg Lys Lys Lys Asp Gln Cys Leu Pro
-45 -40 -35

Gly Ile Cys Arg Ser Leu Lys Arg Arg Lys Ser Pro Arg Ser Pro Gly
-30 -25 -20

Met Lys Val Ile Arg Leu Ser Gln Phe Leu Leu Lys Cys Trp Pro Arg
-15 -10 -5 1

Thr Ser Leu Thr Ala Ala Thr

                5

(2) INFORMATION FOR SEQ ID NO: 393:

(i) SEQUENCE CHARACTERISTICS:
(A) LENGTH: 54 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -36..-1
(C) IDENTIFICATION METHOD: Von Heijne matrix
(D) OTHER INFORMATION: score 5
seq SFSIXTLLWGLNC/KR

(xi) SEQUENCE DESCRIPTION: SEQ ID NO: 393:

Met Thr Phe Ser Phe Phe Cys Phe Phe Pro Gly Phe Lys Pro Leu Leu
-35 -30 -25

Phe His Tyr Phe Leu Phe Xaa Ser Phe Ser Ile Xaa Thr Leu Leu Trp
-20 -15 -10 -5

Gly Leu Asn Cys Lys Arg Ser Trp Asn Ile Asn Leu Arg Ile Val Xaa
1 5 10

Ser Tyr Ser Ser Gly Tyr
15

(2) INFORMATION FOR SEQ ID NO: 394:

(i) SEQUENCE CHARACTERISTICS:
(A) LENGTH: 65 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -41..-1
(C) IDENTIFICATION METHOD: Von Heijne matrix
(D) OTHER INFORMATION: score 5

seq RLLLILSGCLVYG/TA

(xi) SEQUENCE DESCRIPTION: SEQ ID NO: 394:

Met Ala Gly Gly Met Lys Val Ala Val Ser Pro Ala Val Gly Pro Gly
    -40                 -35                     -30

Pro Trp Gly Ser Gly Val Gly Gly Gly Gly Thr Val Arg Leu Leu Leu
-25                 -20                     -15                 -10

Ile Leu Ser Gly Cys Leu Val Tyr Gly Thr Ala Glu Thr Asp Val Asn
                -5                      1               5

Val Val Met Leu Gln Glu Ser Gln Val Cys Glu Lys Arg Ala Ser Leu
        10                  15                  20

Gly

(2) INFORMATION FOR SEQ ID NO: 395:

(i) SEQUENCE CHARACTERISTICS:
(A) LENGTH: 61 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -32..-1
(C) IDENTIFICATION METHOD: Von Heijne matrix
(D) OTHER INFORMATION: score 5
seq PLLSCSCPPPLLG/EG

(xi) SEQUENCE DESCRIPTION: SEQ ID NO: 395:

Met Val Glu Met Thr Gly Val Trp Gln Cys Gln Ala Glu Ala Val Lys
        -30                 -25                     -20

Gly Leu Pro Pro Leu Leu Ser Cys Ser Cys Pro Pro Pro Leu Leu Gly
    -15                     -10                 -5

Glu Gly His Ala Gln Ala Ser Pro Leu Ala Gln Glu Glu Asp Lys Lys
 1                  5                   10                  15

His Thr Glu Gln Thr Gln Ala Thr Ser Pro Thr Gln Pro
            20                  25

(2) INFORMATION FOR SEQ ID NO: 396:

(i) SEQUENCE CHARACTERISTICS:

(A) LENGTH: 33 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -21..-1
(C) IDENTIFICATION METHOD: Von Heijne matrix
(D) OTHER INFORMATION: score 5
seq AGLLPLLLGNAPG/ES

(xi) SEQUENCE DESCRIPTION: SEQ ID NO: 396:

Met Gln Ile Thr Pro Gly Ser Ala Ala Gly Leu Leu Pro Leu Leu Leu
-20 -15 -10

Gly Asn Ala Pro Gly Glu Ser Val Gly Gly Arg Cys Xaa Pro Gly Cys
-5 1 5 10

Trp

(2) INFORMATION FOR SEQ ID NO: 397:

(i) SEQUENCE CHARACTERISTICS:
(A) LENGTH: 33 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -17..-1
(C) IDENTIFICATION METHOD: Von Heijne matrix
(D) OTHER INFORMATION: score 5
seq TWLLLTLQNSVFT/SF

(xi) SEQUENCE DESCRIPTION: SEQ ID NO: 397:

(2) INFORMATION FOR SEQ ID NO: 398:

(i) SEQUENCE CHARACTERISTICS:
(A) LENGTH: 58 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -32..-1
(C) IDENTIFICATION METHOD: Von Heijne matrix
(D) OTHER INFORMATION: score 5
seq VCIVLALCHTSRP/MS

(xi) SEQUENCE DESCRIPTION: SEQ ID NO: 398:

(2) INFORMATION FOR SEQ ID NO: 399:

(i) SEQUENCE CHARACTERISTICS:
(A) LENGTH: 24 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: -16..-1
(C) IDENTIFICATION METHOD: Von Heijne matrix
(D) OTHER INFORMATION: score 4.9
seq R[?]TLLPLVLHSQS/SC

(xi) SEQUENCE DESCRIPTION: SEQ ID NO: 399:

Met Lys Leu Arg Phe Thr Leu Leu Pro Leu Val Leu His Ser Gln Ser
    -15                 -10                 -5

Ser Cys Val Phe Trp Lys Ala Gly
  1                 5

(2) INFORMATION FOR SEQ ID NO: 400:

      (i) SEQUENCE CHARACTERISTICS:
            (A) LENGTH: 51 amino acids
            (B) TYPE: AMINO ACID
            (D) TOPOLOGY: LINEAR

      (ii) MOLECULE TYPE: PROTEIN

      (vi) ORIGINAL SOURCE:
            (A) ORGANISM: Homo Sapiens
            (F) TISSUE TYPE: Heart

      (ix) FEATURE:
            (A) NAME/KEY: sig_peptide
            (B) LOCATION: -30..-1
            (C) IDENTIFICATION METHOD: Von Heijne matrix
            (D) OTHER INFORMATION:  score 4.9
                                    seq FIPFLVIYSFVLS/SP

      (xi) SEQUENCE DESCRIPTION: SEQ ID NO: 400:

(2) INFORMATION FOR SEQ ID NO: 401:

      (i) SEQUENCE CHARACTERISTICS:
            (A) LENGTH: 46 amino acids
            (B) TYPE: AMINO ACID
            (D) TOPOLOGY: LINEAR

      (ii) MOLECULE TYPE: PROTEIN

      (vi) ORIGINAL SOURCE:
            (A) ORGANISM: Homo Sapiens
            (F) TISSUE TYPE: Heart

      (ix) FEATURE:

(A) NAME/KEY: sig_peptide
(B) LOCATION: -27..-1
(C) IDENTIFICATION METHOD: Von Heijne matrix
(D) OTHER INFORMATION:  score 4.9
                        seq CLLSYIALGAIHA/KI

(xi) SEQUENCE DESCRIPTION: SEQ ID NO: 401:

Met Asn Arg Val Pro Ala Asp Ser Pro Asn Met Cys Leu Ile Cys Leu
    -25                 -20                 -15

Leu Ser Tyr Ile Ala Leu Gly Ala Ile His Ala Lys Ile Cys Arg Arg
    -10                 -5                  1               5

Ala Phe Gln Glu Glu Gly Arg Ala Xaa Ala Lys Thr Gly Val
                10                  15

(2) INFORMATION FOR SEQ ID NO: 402:

(i) SEQUENCE CHARACTERISTICS:
(A) LENGTH: 43 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -15..-1
(C) IDENTIFICATION METHOD: Von Heijne matrix
(D) OTHER INFORMATION:  score 4.8
                        seq LFLNLPLVIGTIP/LH

(xi) SEQUENCE DESCRIPTION: SEQ ID NO: 402:

Met Asp Leu Phe Leu Asn Leu Pro Leu Val Ile Gly Thr Ile Pro Leu
-15                 -10                 -5                  1

His Pro Phe Gly Ser Arg Thr Ser Ser Val Ser Ser Gln Cys Ser Met
            5                   10                  15

Asn Met Asn Trp Leu Ser Leu Ser Leu Pro Glu
    20                  25

(2) INFORMATION FOR SEQ ID NO: 403:

(i) SEQUENCE CHARACTERISTICS:
(A) LENGTH: 114 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -73..-1
(C) IDENTIFICATION METHOD: Von Heijne matrix
(D) OTHER INFORMATION: score 4.8
seq VIRSTLVLSQCLC/SR

(xi) SEQUENCE DESCRIPTION: SEQ ID NO: 403:

Met Xaa Lys Asn His Arg Asn Lys Lys Ser Ile His Phe Pro Leu Cys
-70 -65 -60

Thr Ile Pro Ser Xaa Met Xaa Lys Ser Cys Thr Leu Pro Leu Gln Arg
-55 -50 -45

Thr Trp Asp Xaa Xaa Pro Ser Phe Val His Trp Xaa Gln Ala Arg Leu
-40 -35 -30

Gln Ser Pro Pro Xaa Ser His Leu Val Xaa Leu Ser Val Ile Arg Ser
-25 -20 -15 -10

Thr Leu Val Leu Ser Gln Cys Leu Cys Ser Arg Xaa Pro Tyr Phe Ser
-5 1 5

Ala Met Met Thr Pro Lys Cys Lys Ser Ile Xaa Ala Gly Asn Ser Gly
10 15 20

Met Pro Lys Arg Asn Cys Lys Val Leu Pro Ser Ser Glu Lys Met Xaa
25 30 35

Val His
40

(2) INFORMATION FOR SEQ ID NO: 404:

(i) SEQUENCE CHARACTERISTICS:
(A) LENGTH: 18 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -14..-1

(C) IDENTIFICATION METHOD: Von Heijne matrix
(D) OTHER INFORMATION: score 4.8
. seq SFIALVYSSLSFQ/KV

(xi) SEQUENCE DESCRIPTION: SEQ ID NO: 404:

Met Ser Phe Ile Ala Leu Val Tyr Ser Ser Leu Ser Phe Gln Lys Val
-10 -5 1

Pro Gly

(2) INFORMATION FOR SEQ ID NO: 405:

(i) SEQUENCE CHARACTERISTICS:
(A) LENGTH: 24 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -22..-1
(C) IDENTIFICATION METHOD: Von Heijne matrix
(D) OTHER INFORMATION: score 4.7
seq IVLFLNSXFPIIC/SR

(xi) SEQUENCE DESCRIPTION: SEQ ID NO: 405:

Met Val Phe Asp Thr Leu Lys Ser Arg Ile Val Leu Phe Leu Asn Ser
-20 -15 -10

Xaa Phe Pro Ile Ile Cys Ser Arg
-5 1

(2) INFORMATION FOR SEQ ID NO: 406:

(i) SEQUENCE CHARACTERISTICS:
(A) LENGTH: 69 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:

(A) NAME/KEY: sig_peptide
(B) LOCATION: -59..-1
(C) IDENTIFICATION METHOD: Von Heijne matrix
(D) OTHER INFORMATION: score 4.7
seq IFLFSILLMSLRT/FH

(xi) SEQUENCE DESCRIPTION: SEQ ID NO: 406:

(2) INFORMATION FOR SEQ ID NO: 407:

(i) SEQUENCE CHARACTERISTICS:
(A) LENGTH: 99 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -17..-1
(C) IDENTIFICATION METHOD: Von Heijne matrix
(D) OTHER INFORMATION: score 4.7
seq MLFFLGALCRESG/VP

(xi) SEQUENCE DESCRIPTION: SEQ ID NO: 407:

Met Asp Asp Leu Met Leu Phe Phe Leu Gly Ala Leu Cys Arg Glu Ser
-15 -10 -5

Gly Val Pro Ser Leu Gly Lys Gln Glu Arg Met Arg Ala Tyr Ala Ala
1               5                   10                  15

Glu Met Pro Pro Leu Leu Pro Ser Pro Cys Pro Pro Pro Ser His Leu
            20                  25                  30

Pro Lys Pro Ala Ser Pro Cys Pro Tyr Pro Leu Xaa Leu Leu Thr Phe
        35                  40                  45

Pro Val Gly Val Pro His Leu Pro Gly Thr Arg Leu Gln Cys Gln Gly
    50                  55                  60

Leu Gly His Ser Leu Xaa Arg Ala Glu Arg Gly Val Gly Gly Gly Val
65 70 75

Ser Pro Gly
80

(2) INFORMATION FOR SEQ ID NO: 408:

(i) SEQUENCE CHARACTERISTICS:
(A) LENGTH: 71 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -25..-1
(C) IDENTIFICATION METHOD: Von Heijne matrix
(D) OTHER INFORMATION: score 4.6
seq LPTLLLLPVGAPG/KK

(xi) SEQUENCE DESCRIPTION: SEQ ID NO: 408:

Met Val Leu Gly Ala Leu Asn Leu Pro Ser Gln Glu Leu Pro Thr Leu
-25 -20 -15 -10

Leu Leu Leu Pro Val Gly Ala Pro Gly Lys Lys Lys Gly Met Glu Gly
-5 1 5

Lys Thr Pro Leu Asp Leu Phe Ala His Phe Gly Pro Glu Pro Gly Asp
10 15 20

His Ser Asp Pro Leu Pro Pro Ser Ala Pro Ser Pro Thr Arg Glu Gly
25 30 35

Ala Leu Thr Pro Pro Pro Gly
40 45

(2) INFORMATION FOR SEQ ID NO: 409:

(i) SEQUENCE CHARACTERISTICS:
(A) LENGTH: 34 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION: score 4.6
seq QTFVSFLSIPVLG/LV

(xi) SEQUENCE DESCRIPTION: SEQ ID NO: 409:

Met Leu Val Ser Lys Ile Gln Thr Phe Val Ser Phe Leu Ser Ile Pro
-15 -10 -5

Val Leu Gly Leu Val Pro Asp His Ile Leu Gln Leu Ile Thr Glu Lys
1 5 10

Glu Thr
15

(2) INFORMATION FOR SEQ ID NO: 410:

(i) SEQUENCE CHARACTERISTICS:
(A) LENGTH: 39 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -31..-1
(C) IDENTIFICATION METHOD: Von Heijne matrix
(D) OTHER INFORMATION: score 4.6
seq LLSTGLNILGTQA/FR

(xi) SEQUENCE DESCRIPTION: SEQ ID NO: 410:

(2) INFORMATION FOR SEQ ID NO: 411:

(i) SEQUENCE CHARACTERISTICS:
(A) LENGTH: 95 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -17..-1
(C) IDENTIFICATION METHOD: Von Heijne matrix
(D) OTHER INFORMATION: score 4.6
seq ILLWEACTGRCQA/SL

(xi) SEQUENCE DESCRIPTION: SEQ ID NO: 411:

(2) INFORMATION FOR SEQ ID NO: 412:

(i) SEQUENCE CHARACTERISTICS:
(A) LENGTH: 33 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide

(B) LOCATION: -25..-1
(C) IDENTIFICATION METHOD: Von Heijne matrix
(D) OTHER INFORMATION: score 4.6
seq VLCILGCHGNLCC/EP

(xi) SEQUENCE DESCRIPTION: SEQ ID NO: 412:

(2) INFORMATION FOR SEQ ID NO: 413:

(i) SEQUENCE CHARACTERISTICS:
(A) LENGTH: 29 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -24..-1
(C) IDENTIFICATION METHOD: Von Heijne matrix
(D) OTHER INFORMATION: score 4.6
seq IFTALFLXLHSVA/IN

(xi) SEQUENCE DESCRIPTION: SEQ ID NO: 413:

Met Val Ser Cys Asp Val Xaa Ser Tyr Val Ile Ile Phe Thr Ala Leu
                -20                 -15                 -10

Phe Leu Xaa Leu His Ser Val Ala Ile Asn Glu Glu Phe
            -5                  1               5

(2) INFORMATION FOR SEQ ID NO: 414:

(i) SEQUENCE CHARACTERISTICS:
(A) LENGTH: 27 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(F) TISSUE TYPE: Dystrophic muscle

(ix) FEATURE:
(A) NAME/KEY: sig_peptide
(B) LOCATION: -20..-1
(C) IDENTIFICATION METHOD: Von Heijne matrix
(D) OTHER INFORMATION: score 4.6
seq LFAIFLMCLKSIG/SV

(xi) SEQUENCE DESCRIPTION: SEQ ID NO: 414:

(2) INFORMATION FOR SEQ ID NO: 415:

(i) SEQUENCE CHARACTERISTICS:
(A) LENGTH: 101 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -33..-1
(C) IDENTIFICATION METHOD: Von Heijne matrix
(D) OTHER INFORMATION: score 4.5
seq LASLFGLDQXAXG/HG

(xi) SEQUENCE DESCRIPTION: SEQ ID NO: 415:

Met Phe Gly Ala Gly Asp Glu Asp Asp Thr Asp Phe Leu Ser Pro Ser
-30 -25 -20

Gly Gly Ala Arg Leu Ala Ser Leu Phe Gly Leu Asp Gln Xaa Ala Xaa
-15 -10 -5

Gly His Gly Asn Glu Phe Phe Gln Tyr Thr Ala Pro Lys Gln Pro Lys
1               5                   10                  15

Lys Gly Gln Gly Thr Ala Ala Thr Gly Asn Gln Ala Xaa Pro Lys Thr
            20                  25                  30

Ala Pro Ala Xaa Met Ser Thr Pro Thr Ile Leu Val Ala Thr Ala Val
        35                  40                  45

His Ala Tyr Arg Tyr Thr Xaa Gly Xaa Tyr Val Lys Gln Xaa Asn Leu
    50                  55                  60

Val Leu Gln Phe Trp
    65

(2) INFORMATION FOR SEQ ID NO: 416:

    (i) SEQUENCE CHARACTERISTICS:
        (A) LENGTH: 62 amino acids
        (B) TYPE: AMINO ACID
        (D) TOPOLOGY: LINEAR

    (ii) MOLECULE TYPE: PROTEIN

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: sig_peptide
        (B) LOCATION: -28..-1
        (C) IDENTIFICATION METHOD: Von Heijne matrix
        (D) OTHER INFORMATION:  score 4.5
                                seq RFLSLSAADGXDX/SX

    (xi) SEQUENCE DESCRIPTION: SEQ ID NO: 416:

Met Val Leu Thr Leu Gly Glu Ser Trp Pro Val Leu Val Gly Arg Arg
            -25                 -20                 -15

Phe Leu Ser Leu Ser Ala Ala Asp Gly Xaa Asp Xaa Ser Xaa Asp Ser
        -10                 -5                  1

Trp Asp Val Glu Arg Val Ala Glu Trp Pro Trp Leu Ser Gly Thr Ile
  5                  10                  15                  20

Arg Ala Val Ser His Thr Asp Val Thr Lys Lys Asp Leu Lys
                25                  30

(2) INFORMATION FOR SEQ ID NO: 417:

    (i) SEQUENCE CHARACTERISTICS:
        (A) LENGTH: 23 amino acids
        (B) TYPE: AMINO ACID
        (D) TOPOLOGY: LINEAR

    (ii) MOLECULE TYPE: PROTEIN

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: sig_peptide
        (B) LOCATION: -17..-1

(C) IDENTIFICATION METHOD: Von Heijne matrix
(D) OTHER INFORMATION:  score 4.4
                        seq LTSVFQAMIWSQG/VS

(xi) SEQUENCE DESCRIPTION: SEQ ID NO: 417:

Met Val Ile Glu Leu Thr Ser Val Phe Gln Ala Met Ile Trp Ser Gln
        -15                 -10                 -5

Gly Val Ser Asp Ser Ser Lys
    1               5

(2) INFORMATION FOR SEQ ID NO: 418:

(i) SEQUENCE CHARACTERISTICS:
(A) LENGTH: 68 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -50..-1
(C) IDENTIFICATION METHOD: Von Heijne matrix
(D) OTHER INFORMATION:  score 4.4
                        seq ILFLFYFPAAYYA/SR

(xi) SEQUENCE DESCRIPTION: SEQ ID NO: 418:

Met Glu Ser Thr Leu Gly Ala Gly Ile Val Ile Ala Glu Ala Leu Gln
-50                 -45                 -40                 -35

Asn Gln Leu Ala Trp Leu Glu Asn Val Trp Leu Trp Xaa Xaa Leu Xaa
                -30                 -25                 -20

Xaa Xaa Ile Pro Xaa Ile Leu Phe Leu Phe Tyr Phe Pro Ala Ala Tyr
            -15                 -10                 -5

Tyr Ala Ser Arg Arg Val Gly Ile Ala Val Leu Trp Ile Ser Leu Ile
        1               5                   10

Thr Glu Trp Leu
15

(2) INFORMATION FOR SEQ ID NO: 419:

(i) SEQUENCE CHARACTERISTICS:
(A) LENGTH: 31 amino acids
(B) TYPE: AMINO ACID

(D) TOPOLOGY: LINEAR

(ii) MOLECULE TYPE: PROTEIN .

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -25..-1
(C) IDENTIFICATION METHOD: Von Heijne matrix
(D) OTHER INFORMATION: score 4.4
seq VLVGVFLSTFLYC/EC

(xi) SEQUENCE DESCRIPTION: SEQ ID NO: 419:

(2) INFORMATION FOR SEQ ID NO: 420:

(i) SEQUENCE CHARACTERISTICS:
(A) LENGTH: 37 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -22..-1
(C) IDENTIFICATION METHOD: Von Heijne matrix
(D) OTHER INFORMATION: score 4.4
seq GFLLCPLVCGLRR/WT

(xi) SEQUENCE DESCRIPTION: SEQ ID NO: 420:

(2) INFORMATION FOR SEQ ID NO: 421:

(i) SEQUENCE CHARACTERISTICS:
(A) LENGTH: 21 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION: score 4.4
seq RGLLLGLAVAAAA/VR

(xi) SEQUENCE DESCRIPTION: SEQ ID NO: 421:

Met Leu Arg Cys Gly Gly Arg Gly Leu Leu Leu Gly Leu Ala Val Ala
-15 -10 -5

Ala Ala Ala Val Arg
1

(2) INFORMATION FOR SEQ ID NO: 422:

(i) SEQUENCE CHARACTERISTICS:
(A) LENGTH: 27 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -14..-1
(C) IDENTIFICATION METHOD: Von Heijne matrix
(D) OTHER INFORMATION: score 4.4
seq ILLMIVFSIFLLL/CN

(xi) SEQUENCE DESCRIPTION: SEQ ID NO: 422:

(2) INFORMATION FOR SEQ ID NO: 423:

(i) SEQUENCE CHARACTERISTICS:
(A) LENGTH: 22 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -14..-1
(C) IDENTIFICATION METHOD: Von Heijne matrix
(D) OTHER INFORMATION: score 4.4
seq SLLFIFRSILISC/FS

(xi) SEQUENCE DESCRIPTION: SEQ ID NO: 423:

Met Ser Leu Leu Phe Ile Phe Arg Ser Ile Leu Ile Ser Cys Phe Ser
-10 -5 1

Gly Asp Phe Phe Phe Phe
                5

(2) INFORMATION FOR SEQ ID NO: 424:

(i) SEQUENCE CHARACTERISTICS:
(A) LENGTH: 46 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -17..-1
(C) IDENTIFICATION METHOD: Von Heijne matrix
(D) OTHER INFORMATION: score 4.3
seq SKVLIQLSQAFWA/SP

(xi) SEQUENCE DESCRIPTION: SEQ ID NO: 424:

(2) INFORMATION FOR SEQ ID NO: 425:

(i) SEQUENCE CHARACTERISTICS:
(A) LENGTH: 108 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -29..-1
(C) IDENTIFICATION METHOD: Von Heijne matrix
(D) OTHER INFORMATION: score 4.3
seq VLLGSTAMATSLT/NV

(xi) SEQUENCE DESCRIPTION: SEQ ID NO: 425:

Met Asp Thr Ser Ser Val Gly Gly Leu Glu Leu Thr Asp Gln Thr Pro
                -25                 -20                 -15

Val Leu Leu Gly Ser Thr Ala Met Ala Thr Ser Leu Thr Asn Val Gly
            -10                 -5                  1

Asn Ser Phe Ser Gly Pro Ala Asn Pro Leu Val Ser Arg Ser Asn Lys
    5                   10                  15

Phe Gln Asn Ser Ser Val Glu Asp Asp Asp Asp Val Val Phe Ile Glu
20                  25                  30                  35

Pro Val Gln Pro Pro Pro Pro Ser Val Pro Val Val Ala Asp Gln Arg
                40                  45                  50

Thr Ile Thr Phe Thr Ser Ser Lys Asn Xaa Glu Leu Gln Gly Asn Asp
            55                  60                  65

Ser Lys Ile Thr Pro Ser Ser Lys Glu Leu Ala Ser
        70                  75

(2) INFORMATION FOR SEQ ID NO: 426:

(i) SEQUENCE CHARACTERISTICS:
(A) LENGTH: 51 amino acids
(B) TYPE: 'AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: -31..-1
(C) IDENTIFICATION METHOD: Von Heijne matrix
(D) OTHER INFORMATION: score 4.3
seq ILLLTHVPPWILE/NP

(xi) SEQUENCE DESCRIPTION: SEQ ID NO: 426:

Met Asp Thr Gly Glu Ser Phe Ser Pro His Thr Ser Cys Arg Gly His
-30 -25 -20

Trp Arg Ile Leu Leu Leu Thr His Val Pro Pro Trp Ile Leu Glu Asn
-15 -10 -5 1

Pro Ser Cys His Thr Arg Pro Ala Val Asp Thr Gly Glu Ser Phe Ser
                5                   10                  15

Pro Gln Arg
20

(2) INFORMATION FOR SEQ ID NO: 427:

(i) SEQUENCE CHARACTERISTICS:
(A) LENGTH: 100 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -31..-1
(C) IDENTIFICATION METHOD: Von Heijne matrix
(D) OTHER INFORMATION: score 4.3
seq LVLLSVLKEPVSR/SI

(xi) SEQUENCE DESCRIPTION: SEQ ID NO: 427:

Met Pro Tyr Leu Asp Pro Tyr Ile Thr Gln Pro Ile Ile Gln Ile Glu
-30 -25 -20

Arg Lys Leu Val Leu Leu Ser Val Leu Lys Glu Pro Val Ser Arg Ser
-15 -10 -5 1

Ile Phe Asp Tyr Ala Leu Arg Ser Lys Asp Ile Thr Ser Leu Phe Arg
                5                   10                  15

His Leu His Met Arg Gln Lys Lys Arg Asn Gly Ser Leu Pro Asp Cys
        20                  25                  30

Pro Pro Pro Glu Asp Pro Ala Ile Ala Gln Leu Leu Lys Lys Leu Leu
    35                  40                  45

Ser Gln Gly Met Thr Glu Glu Glu Glu Asp Lys Leu Leu Ala Leu Lys
50                  55                  60                  65

Asp Phe Met Met

(2) INFORMATION FOR SEQ ID NO: 428:

(i) SEQUENCE CHARACTERISTICS:
(A) LENGTH: 44 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -29..-1
(C) IDENTIFICATION METHOD: Von Heijne matrix
(D) OTHER INFORMATION: score 4.3
seq VLLGSTAMATSLT/NV

(xi) SEQUENCE DESCRIPTION: SEQ ID NO: 428:

Met Asp Thr Ser Ser Val Gly Gly Leu Glu Leu Thr Asp Gln Thr Pro
                -25                 -20                 -15

Val Leu Leu Gly Ser Thr Ala Met Ala Thr Ser Leu Thr Asn Val Gly
            -10                 -5                  1

Asn Ser Phe Ser Gly Pro Ala Asn Pro Leu Val Ser
    5                   10                  15

(2) INFORMATION FOR SEQ ID NO: 429:

(i) SEQUENCE CHARACTERISTICS:
(A) LENGTH: 50 amino acids
(B) TYPE: AMINO ACID
(D) TOPOLOGY: LINEAR

(ii) MOLECULE TYPE: PROTEIN

(vi) ORIGINAL SOURCE:

(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -28..-1
(C) IDENTIFICATION METHOD: Von Heijne matrix
(D) OTHER INFORMATION: score 4.2
seq FGLLDFVVQCCDS/LR

(xi) SEQUENCE DESCRIPTION: SEQ ID NO: 429:

Met His Val Leu Phe Asn Ile Val Thr Thr Asn Xaa Xaa Asn His Phe
-25 -20 -15

Gly Leu Leu Asp Phe Val Val Gln Cys Cys Asp Ser Leu Arg Asn His
-10 -5 1

Xaa Xaa Ser Phe Gln Ser Ser Tyr Leu Arg Leu Asn His Ser Xaa His
5 10 15 20

Thr Cys

(2) INFORMATION FOR SEQ ID NO: 430:

(i) SEQUENCE CHARACTERISTICS:
(A) LENGTH: 66 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -22..-1
(C) IDENTIFICATION METHOD: Von Heijne matrix
(D) OTHER INFORMATION: score 4.2
seq TAYWLSFMSWAQS/SS

(xi) SEQUENCE DESCRIPTION: SEQ ID NO: 430:

Met Pro Pro Gln Ser Cys Cys Ser Lys Thr Ala Tyr Trp Leu Ser Phe
-20 -15 -10

Met Ser Trp Ala Gln Ser Ser Ser Phe Gly Ser Arg Xaa Glu Ser Thr
-5 1 5 10

Ser Pro Cys Thr Asp His Cys Ser Gly Pro Arg Glu Glu Gln Leu Cys
15 20 25

Ser Ser Arg Val Phe His Cys Ile Thr His Pro Asn Gly Arg Ile His

30 35 40

Arg Trp

(2) INFORMATION FOR SEQ ID NO: 431:

(i) SEQUENCE CHARACTERISTICS:
(A) LENGTH: 25 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -14..-1
(C) IDENTIFICATION METHOD: Von Heijne matrix
(D) OTHER INFORMATION: score 4.2
seq SCVFFHFLQGGLG/FG

(xi) SEQUENCE DESCRIPTION: SEQ ID NO: 431:

Met Ser Cys Val Phe Phe His Phe Leu Gln Gly Gly Leu Gly Phe Gly
-10 -5 1

Ser Ala Gly Arg Cys Ala Gly Asp Arg
5 10

(2) INFORMATION FOR SEQ ID NO: 432:

(i) SEQUENCE CHARACTERISTICS:
(A) LENGTH: 54 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 4.2
seq LILLPIWINMAQI/QQ

(xi) SEQUENCE DESCRIPTION: SEQ ID NO: 432:

Met Ser Ile Ser Leu Ser Ser Leu Ile Leu Leu Pro Ile Trp Ile Asn
-20                 -15                 -10                 -5

Met Ala Gln Ile Gln Gln Gly Gly Pro Asp Glu Lys Glu Lys Thr Thr
                1               5                   10

Ala Leu Lys Asp Leu Leu Ser Arg Ile Asp Leu Asp Glu Leu Met Lys
        15                  20                  25

Lys Asp Glu Pro Pro Gly
    30

(2) INFORMATION FOR SEQ ID NO: 433:

(i) SEQUENCE CHARACTERISTICS:
(A) LENGTH: 52 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -34..-1
(C) IDENTIFICATION METHOD: Von Heijne matrix
(D) OTHER INFORMATION: score 4.2
seq SFCNAVVLSPVFQ/EE

(xi) SEQUENCE DESCRIPTION: SEQ ID NO: 433:

(2) INFORMATION FOR SEQ ID NO: 434:

(i) SEQUENCE CHARACTERISTICS:
(A) LENGTH: 39 amino acids
(B) TYPE: AMINO ACID
(D) TOPOLOGY: LINEAR

(ii) MOLECULE TYPE: PROTEIN

(vi) ORIGINAL SOURCE:

(A) ORGANISM: Homo Sapiens
(F) TISSUE TYPE: Kidney

(ix) FEATURE:
(A) NAME/KEY: sig_peptide
(B) LOCATION: -17..-1
(C) IDENTIFICATION METHOD: Von Heijne matrix
(D) OTHER INFORMATION: score 4.2
seq PVQVLGLLATCQH/AP

(xi) SEQUENCE DESCRIPTION: SEQ ID NO: 434:

(2) INFORMATION FOR SEQ ID NO: 435:

(i) SEQUENCE CHARACTERISTICS:
(A) LENGTH: 28 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -24..-1
(C) IDENTIFICATION METHOD: Von Heijne matrix
(D) OTHER INFORMATION: score 4.2
seq SLNQILLFLLISC/RT

(xi) SEQUENCE DESCRIPTION: SEQ ID NO: 435:

Met Arg Tyr Arg Leu Arg Ile Gln Ile Thr Thr Ser Leu Asn Gln Ile
-20 -15 -10

Leu Leu Phe Leu Leu Ile Ser Cys Arg Thr Leu Ser
-5 1

(2) INFORMATION FOR SEQ ID NO: 436:

(i) SEQUENCE CHARACTERISTICS:
(A) LENGTH: 35 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -25..-1
(C) IDENTIFICATION METHOD: Von Heijne matrix
(D) OTHER INFORMATION: score 4.2
seq VLLFFCCSPLYSP/LF

(xi) SEQUENCE DESCRIPTION: SEQ ID NO: 436:

Met Pro Phe Phe Ser Asn Gln Pro Thr Gln Val Ser Val Leu Leu Phe
-25 -20 -15 -10

Phe Cys Cys Ser Pro Leu Tyr Ser Pro Leu Phe Leu Leu Xaa Leu Ile
-5 1 5

Pro His Gln
10

(2) INFORMATION FOR SEQ ID NO: 437:

(i) SEQUENCE CHARACTERISTICS:
(A) LENGTH: 115 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -44..-1
(C) IDENTIFICATION METHOD: Von Heijne matrix
(D) OTHER INFORMATION: score 4.1
seq IAVGLTCQHVSHA/IS

(xi) SEQUENCE DESCRIPTION: SEQ ID NO: 437:

Met Arg Val Lys Asp Pro Thr Lys Ala Leu Pro Glu Lys Ala Lys Arg
-40 -35 -30

Ser Lys Arg Pro Thr Val Pro His Asp Glu Asp Ser Ser Asp Asp Ile
-25 -20 -15

Ala Val Gly Leu Thr Cys Gln His Val Ser His Ala Ile Ser Val Asn
-10 -5 1

His Val Lys Arg Ala Ile Ala Glu Asn Leu Trp Ser Val Cys Ser Glu
  5                   10                  15                  20

Cys Leu Lys Glu Arg Arg Phe Tyr Asp Gly Gln Leu Val Leu Thr Ser
                25                  30                  35

Asp Ile Trp Leu Cys Leu Lys Cys Gly Phe Gln Gly Cys Gly Lys Asn
            40                  45                  50

Ser Glu Ser Gln His Ser Leu Lys His Phe Lys Ser Ser Arg Thr Glu
        55                  60                  65

Pro Leu Arg
    70

(2) INFORMATION FOR SEQ ID NO: 438:

(i) SEQUENCE CHARACTERISTICS:
(A) LENGTH: 48 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -44..-1
(C) IDENTIFICATION METHOD: Von Heijne matrix
(D) OTHER INFORMATION: score 4.1
seq GTYLTSSSPLCQL/QP

(xi) SEQUENCE DESCRIPTION: SEQ ID NO: 438:

(2) INFORMATION FOR SEQ ID NO: 439:

(i) SEQUENCE CHARACTERISTICS:
(A) LENGTH: 44 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -35..-1
(C) IDENTIFICATION METHOD: Von Heijne matrix
(D) OTHER INFORMATION: score 4.1
seq VLCCLLIATPTFF/LL

(xi) SEQUENCE DESCRIPTION: SEQ ID NO: 439:

Met Ser Leu Thr Ser Arg Xaa Xaa Ile Met Xaa Thr Ile Lys Ile Gln
-35 -30 -25 -20

Asn Ile Ser Ile Thr Lys Val Leu Cys Cys Leu Leu Ile Ala Thr Pro
-15 -10 -5

Thr Phe Phe Leu Leu Leu Pro Ser Ser Ile Pro Arg
1 5

(2) INFORMATION FOR SEQ ID NO: 440:

(i) SEQUENCE CHARACTERISTICS:
(A) LENGTH: 27 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -18..-1
(C) IDENTIFICATION METHOD: Von Heijne matrix
(D) OTHER INFORMATION: score 4.1
seq AGVVSTSVAAAVA/AV

(xi) SEQUENCE DESCRIPTION: SEQ ID NO: 440:

Met Xaa Ala Glu Ala Ala Gly Val Val Ser Thr Ser Val Ala Ala Ala
-15 -10 -5

Val Ala Ala Val Ala Ala Pro Ala Gly Ala Gly
1 5

(2) INFORMATION FOR SEQ ID NO: 441:

(i) SEQUENCE CHARACTERISTICS:

(A) LENGTH: 32 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -15..-1
(C) IDENTIFICATION METHOD: Von Heijne matrix
(D) OTHER INFORMATION: score 4
seq IMSSCLALTYTNS/IS

(xi) SEQUENCE DESCRIPTION: SEQ ID NO: 441:

Met Trp Ile Met Ser Ser Cys Leu Ala Leu Thr Tyr Thr Asn Ser Ile
-15 -10 -5 1

Ser His Ser Leu Cys Leu Glu Arg Ala Tyr Ser Leu Phe Lys Val Asp
                5                   10                  15

(2) INFORMATION FOR SEQ ID NO: 442:

(i) SEQUENCE CHARACTERISTICS:
(A) LENGTH: 50 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 4
seq SNALVLVTRGSSS/LP

(xi) SEQUENCE DESCRIPTION: SEQ ID NO: 442:

(2) INFORMATION FOR SEQ ID NO: 443:

    (i) SEQUENCE CHARACTERISTICS:
        (A) LENGTH: 48 amino acids
        (B) TYPE: AMINO ACID
        (D) TOPOLOGY: LINEAR

    (ii) MOLECULE TYPE: PROTEIN

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: sig_peptide
        (B) LOCATION: -44..-1
        (C) IDENTIFICATION METHOD: Von Heijne matrix
        (D) OTHER INFORMATION: score 4
                               seq FLLPCVHPFSVIA/VY

    (xi) SEQUENCE DESCRIPTION: SEQ ID NO: 443:

Met Ile Glu Pro Cys Glu Lys Met Lys His Tyr Asp Met Asn Trp Phe
                -40                 -35                 -30

Leu Cys Met Tyr Glu Cys Phe Phe Phe His Leu Leu Glu Thr Glu Phe
            -25                 -20                 -15

Leu Leu Pro Cys Val His Pro Phe Ser Val Ile Ala Val Tyr Val Phe
        -10                 -5                  1

(2) INFORMATION FOR SEQ ID NO: 444:

    (i) SEQUENCE CHARACTERISTICS:
        (A) LENGTH: 58 amino acids
        (B) TYPE: AMINO ACID
        (D) TOPOLOGY: LINEAR

    (ii) MOLECULE TYPE: PROTEIN

    (vi) ORIGINAL SOURCE:
        (A) ORGANISM: Homo Sapiens
        (F) TISSUE TYPE: Muscle

    (ix) FEATURE:
        (A) NAME/KEY: sig_peptide
        (B) LOCATION: -55..-1
        (C) IDENTIFICATION METHOD: Von Heijne matrix
        (D) OTHER INFORMATION: score 4
                               seq AALCGISLSQXFP/EP

    (xi) SEQUENCE DESCRIPTION: SEQ ID NO: 444:

Met Ala Met Trp Asn Arg Pro Cys Gln Xaa Leu Pro Gln Gln Pro Leu
-55                 -50                 -45                 -40

Val Ala Glu Pro Thr Ala Glu Gly Glu Pro His Leu Pro Thr Gly Arg
                -35                 -30                 -25

Glu Leu Thr Glu Ala Asn Arg Phe Ala Tyr Ala Ala Leu Cys Gly Ile
            -20                 -15                 -10

Ser Leu Ser Gln Xaa Phe Pro Glu Pro Gly
        -5                  1

(2) INFORMATION FOR SEQ ID NO: 445:

(i) SEQUENCE CHARACTERISTICS:
(A) LENGTH: 20 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -17..-1
(C) IDENTIFICATION METHOD: Von Heijne matrix
(D) OTHER INFORMATION: score 4
seq CLLVSYAVDSAAG/RF

(xi) SEQUENCE DESCRIPTION: SEQ ID NO: 445:

Met Glu Gln Val Cys Leu Leu Val Ser Tyr Ala Val Asp Ser Ala Ala
    -15                 -10                 -5

Gly Arg Phe Gly
    1

(2) INFORMATION FOR SEQ ID NO: 446:

(i) SEQUENCE CHARACTERISTICS:
(A) LENGTH: 115 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -28..-1
(C) IDENTIFICATION METHOD: Von Heijne matrix
(D) OTHER INFORMATION: score 4
seq ATLRCWASTPVSG/RL

(xi) SEQUENCE DESCRIPTION: SEQ ID NO: 446:

Met Arg Lys Ile Ser His Cys Leu His Cys Trp Pro Glu Ser Gly Ala
-25 -20 -15

Thr Leu Arg Cys Trp Ala Ser Thr Pro Val Ser Gly Arg Leu Ser Ser
-10 -5 1

Met Ala Val Xaa Xaa Xaa Gly Glu Xaa Pro Pro Gln Asp Ala Phe Thr
5 10 15 20

Thr Gln Trp Leu Val Arg Asp Leu Arg Gly Lys Thr Glu Lys Glu Phe
25 30 35

Lys Ala Tyr Val Ser Leu Phe Met Arg His Leu Cys Glu Pro Gly Ala
40 45 50

Asp Gly Ser Glu Thr Phe Ala Asp Gly Val Pro Arg Glu Gly Leu Ser
55 60 65

Arg Gln Gln Val Leu Thr Arg Ile Gly Val Met Ser Leu Val Lys Lys
70 75 80

Lys Gly Gln
85

(2) INFORMATION FOR SEQ ID NO: 447:

(i) SEQUENCE CHARACTERISTICS:
(A) LENGTH: 26 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -22..-1
(C) IDENTIFICATION METHOD: Von Heijne matrix
(D) OTHER INFORMATION: score 4
seq LLHPCGSITLTSS/ST

(xi) SEQUENCE DESCRIPTION: SEQ ID NO: 447:

(2) INFORMATION FOR SEQ ID NO: 448:

(i) SEQUENCE CHARACTERISTICS:
(A) LENGTH: 45 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -17..-1
(C) IDENTIFICATION METHOD: Von Heijne matrix
(D) OTHER INFORMATION:  score 4
                        seq VALQCGLTIPALX/LP

(xi) SEQUENCE DESCRIPTION: SEQ ID NO: 448:

Met Arg Cys Arg Val Ala Leu Gln Cys Gly Leu Thr Ile Pro Ala Leu
    -15                 -10                 -5

Xaa Leu Pro Gln Gly Asp Glu Ala Gly Asp Ala Gln Asp Leu Arg Gly
    1               5                   10                  15

Pro Ala Gln Ala Glu Tyr Leu Tyr Ile Ile Ser Pro Ser
                20                  25

(2) INFORMATION FOR SEQ ID NO: 449:

(i) SEQUENCE CHARACTERISTICS:
(A) LENGTH: 118 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -93..-1
(C) IDENTIFICATION METHOD: Von Heijne matrix
(D) OTHER INFORMATION:  score 3.9

seq LTSAFLWLPRLHI/SV

(xi) SEQUENCE DESCRIPTION: SEQ ID NO: 449:

Met Thr Val Arg Tyr Gly Lys Phe Leu Ser Leu Leu Lys Asp Gly Ala
            -90                 -85                 -80

Glu Asn Asp Leu Thr Trp Val Leu Lys His Cys Glu Arg Phe Leu Lys
        -75                 -70                 -65

Gln Gln Gln Thr Ser Ile Lys Ser Ser Leu Leu Cys Leu Gln Gly Asn
    -60                 -55                 -50

Tyr Ala Gly His Asp Trp Phe Val Ser Ser Leu Phe Met Ile Met Leu
-45                 -40                 -35                 -30

Gly Asp Lys Glu Lys Thr Phe Gln Phe Leu His Gln Phe Ser Arg Leu
                -25                 -20                 -15

Leu Thr Ser Ala Phe Leu Trp Leu Pro Arg Leu His Ile Ser Val Arg
            -10                 -5                  1

Leu Gln Ser Val Phe Lys Gly Gly Phe Xaa Ile Leu Arg Thr Leu Tyr
    5                   10                  15

Leu His Ser Xaa Gly Arg
 20                 25

(2) INFORMATION FOR SEQ ID NO: 450:

(i) SEQUENCE CHARACTERISTICS:
(A) LENGTH: 34 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION:  score 3.9
seq FFWVVLFSAGCKV/IT

(xi) SEQUENCE DESCRIPTION: SEQ ID NO: 450:

Met Ala Phe Asp Val Ser Cys Phe Phe Trp Val Val Leu Phe Ser Ala
-20                 -15                 -10                 -5

Gly Cys Lys Val Ile Thr Ser Trp Asp Gln Met Cys Ile Glu Lys Glu
                1                   5                   10

Ala Thr

(2) INFORMATION FOR SEQ ID NO: 451:

(i) SEQUENCE CHARACTERISTICS:
(A) LENGTH: 41 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -22..-1
(C) IDENTIFICATION METHOD: Von Heijne matrix
(D) OTHER INFORMATION: score 3.9
seq HLSSTTSPPWTHA/AI

(xi) SEQUENCE DESCRIPTION: SEQ ID NO: 451:

Met Leu Thr Arg Leu Val Leu Ser Ala His Leu Ser Ser Thr Thr Ser
-20 -15 -10

Pro Pro Trp Thr His Ala Ala Ile Ser Trp Glu Leu Asp Asn Val Leu
-5 1 5 10

Met Pro Ser Pro Arg Ile Trp Pro Leu
15

(2) INFORMATION FOR SEQ ID NO: 452:

(i) SEQUENCE CHARACTERISTICS:
(A) LENGTH: 51 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -40..-1
(C) IDENTIFICATION METHOD: Von Heijne matrix
(D) OTHER INFORMATION: score 3.9
seq CVNLLLGFEPVIS/RS

(xi) SEQUENCE DESCRIPTION: SEQ ID NO: 452:

Met Arg Tyr Phe Gln Gly Pro Ser Pro Tyr Ser Glu Ile Glu Ile Glu
-40                 -35                 -30                 -25

Leu Cys Asp His Val Tyr Ser Phe Gln Gly Leu Cys Val Asn Leu Leu
                -20                 -15                 -10

Leu Gly Phe Glu Pro Val Ile Ser Arg Ser Arg Xaa Ser Ser Leu Ala
            -5                  1               5

Val Glu Ser
    10

(2) INFORMATION FOR SEQ ID NO: 453:

(i) SEQUENCE CHARACTERISTICS:
(A) LENGTH: 70 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -41..-1
(C) IDENTIFICATION METHOD: Von Heijne matrix
(D) OTHER INFORMATION: score 3.9
seq LASLECYVPSTNQ/WQ

(xi) SEQUENCE DESCRIPTION: SEQ ID NO: 453:

Met Xaa Xaa Lys Arg Thr His Xaa Xaa Xaa Ser Val Phe Asn Gly Leu
    -40                 -35                 -30

Val Tyr Ala Ala Gly Gly Arg Asn Ala Glu Gly Ser Leu Ala Ser Leu
-25                 -20                 -15                 -10

Glu Cys Tyr Val Pro Ser Thr Asn Gln Trp Gln Pro Lys Xaa Xaa Leu
                -5                  1               5

Glu Val Ala Arg Cys Cys His Ala Ser Ala Val Ala Asp Gly Arg Val
        10                  15                  20

Leu Val Thr Gly Gly Leu
    25

(2) INFORMATION FOR SEQ ID NO: 454:

(i) SEQUENCE CHARACTERISTICS:
(A) LENGTH: 43 amino acids

(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -38..-1
(C) IDENTIFICATION METHOD: Von Heijne matrix
(D) OTHER INFORMATION: score 3.9
seq LLFFHLLLNDFFT/FY

(xi) SEQUENCE DESCRIPTION: SEQ ID NO: 454:

Met Phe Leu Lys Val Gln Ser Gln Ser Phe Tyr Xaa Pro Tyr Arg Asp
-35 -30 -25

Cys Leu Asn Phe His Lys Ser Thr Tyr Leu Leu Phe Phe His Leu Leu
-20 -15 -10

Leu Asn Asp Phe Phe Thr Phe Tyr Xaa Ala Lys
-5 1 5

(2) INFORMATION FOR SEQ ID NO: 455:

(i) SEQUENCE CHARACTERISTICS:
(A) LENGTH: 36 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -27..-1
(C) IDENTIFICATION METHOD: Von Heijne matrix
(D) OTHER INFORMATION: score 3.9
seq WIILIIYTFQCNS/SL

(xi) SEQUENCE DESCRIPTION: SEQ ID NO: 455:

Met Gln Pro Leu Lys Ile Ile Phe Tyr Leu Ser Val Ser Ile Trp Ile
-25 -20 -15

Ile Leu Ile Ile Tyr Thr Phe Gln Cys Asn Ser Ser Leu Ser Ile Leu
-10 -5 1 5

Leu Leu Glu Leu

(2) INFORMATION FOR SEQ ID NO: 456:

(i) SEQUENCE CHARACTERISTICS:
(A) LENGTH: 61 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -19..-1
(C) IDENTIFICATION METHOD: Von Heijne matrix
(D) OTHER INFORMATION: score 3.9
seq RVAACTAAAPLQA/HG

(xi) SEQUENCE DESCRIPTION: SEQ ID NO: 456:

Met Met Arg Thr Thr Ala Arg Val Ala Ala Cys Thr Ala Ala Ala Pro
-15 -10 -5

Leu Gln Ala His Gly Ala Xaa Ile Gln Gln Xaa Pro Asp Xaa Leu Xaa
1 5 10

Ser Xaa Arg Leu Ser Arg Xaa Gly Leu Ser Ala Gly Arg Leu His Gln
15 20 25

Ser Glu Thr Glu Ala Glu Leu Glu Ala Pro Gly Arg Ala
30 35 40

(2) INFORMATION FOR SEQ ID NO: 457:

(i) SEQUENCE CHARACTERISTICS:
(A) LENGTH: 45 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: -34..-1
(C) IDENTIFICATION METHOD: Von Heijne matrix
(D) OTHER INFORMATION: score 3.8
seq RWASSCLHPSARS/SN

(xi) SEQUENCE DESCRIPTION: SEQ ID NO: 457:

Met Glu Ala Ala Thr Thr Leu His Pro Gly Pro Arg Pro Ala Leu Pro
-30 -25 -20

Leu Gly Ala Arg Ala Arg Trp Ala Ser Ser Cys Leu His Pro Ser Ala
-15 -10 -5

Arg Ser Ser Asn Pro Ala Gly Lys Ser Ser Arg Thr Pro
1               5                   10

(2) INFORMATION FOR SEQ ID NO: 458:

(i) SEQUENCE CHARACTERISTICS:
(A) LENGTH: 35 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: -29..-1
(C) IDENTIFICATION METHOD: Von Heijne matrix
(D) OTHER INFORMATION: score 3.8
seq LCPVIFFPSNCWK/EY

(xi) SEQUENCE DESCRIPTION: SEQ ID NO: 458:

(2) INFORMATION FOR SEQ ID NO: 459:

(i) SEQUENCE CHARACTERISTICS:
(A) LENGTH: 20 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -18..-1
(C) IDENTIFICATION METHOD: Von Heijne matrix
(D) OTHER INFORMATION: score 3.8
seq FXLLFXXFXFFRQ/XG

(xi) SEQUENCE DESCRIPTION: SEQ ID NO: 459:

Met Xaa Xaa Phe Ser Phe Xaa Leu Leu Phe Xaa Xaa Phe Xaa Phe Phe
-15 -10 -5

Arg Gln Xaa Gly
1

(2) INFORMATION FOR SEQ ID NO: 460:

(i) SEQUENCE CHARACTERISTICS:
(A) LENGTH: 31 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -23..-1
(C) IDENTIFICATION METHOD: Von Heijne matrix
(D) OTHER INFORMATION: score 3.8
seq SVRLLFRFSVIMA/SE

(xi) SEQUENCE DESCRIPTION: SEQ ID NO: 460:

Met Leu Leu Leu Ser Glu Ala Leu Ser Glu Ser Val Arg Leu Leu Phe
-20 -15 -10

Arg Phe Ser Val Ile Met Ala Ser Glu Lys Gln Ser Phe Gln Ile
-5 1 5

(2) INFORMATION FOR SEQ ID NO: 461:

(i) SEQUENCE CHARACTERISTICS:
(A) LENGTH: 27 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(D) DEVELOPMENTAL STAGE: Fetal
(F) TISSUE TYPE: kidney

(ix) FEATURE:
(A) NAME/KEY: sig_peptide
(B) LOCATION: -17..-1
(C) IDENTIFICATION METHOD: Von Heijne matrix
(D) OTHER INFORMATION: score 3.8
seq SLPCTTAFPLLSS/KV

(xi) SEQUENCE DESCRIPTION: SEQ ID NO: 461:

Met Ala Leu Ile Ser Leu Pro Cys Thr Thr Ala Phe Pro Leu Leu Ser
-15 -10 -5

Ser Lys Val Ser Gln Leu Leu Leu Pro Leu Ser
1               5                   10

(2) INFORMATION FOR SEQ ID NO: 462:

(i) SEQUENCE CHARACTERISTICS:
(A) LENGTH: 57 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -37..-1
(C) IDENTIFICATION METHOD: Von Heijne matrix
(D) OTHER INFORMATION: score 3.8
seq RVVALPLVRATCT/AV

(xi) SEQUENCE DESCRIPTION: SEQ ID NO: 462:

Met Ser Glu Glu Glu Ala Ala Gln Ile Pro Arg Ser Ser Val Trp Glu
-35 -30 -25

Gln Asp Gln Gln Asn Val Val Gln Arg Val Val Ala Leu Pro Leu Val
-20 -15 -10

Arg Ala Thr Cys Thr Ala Val Cys Asp Val Tyr Ser Ala Ala Lys Asp
-5 1 5 10

Arg His Pro Leu Leu Gly Ser Ala Trp
15 20

(2) INFORMATION FOR SEQ ID NO: 463:

(i) SEQUENCE CHARACTERISTICS:

(A) LENGTH: 97 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -72..-1
(C) IDENTIFICATION METHOD: Von Heijne matrix
(D) OTHER INFORMATION: score 3.8
seq LAELTVDPQGALA/IR

(xi) SEQUENCE DESCRIPTION: SEQ ID NO: 463:

Met Ala Ala Ala Ala Ala Ala Gly Ala Ala Ser Gly Leu Pro Gly Pro
-70 -65 -60

Val Ala Gln Gly Leu Lys Glu Ala Leu Val Asp Thr Leu Thr Gly Ile
-55 -50 -45

Leu Ser Pro Val Gln Glu Val Arg Ala Ala Ala Glu Glu Gln Ile Lys
-40 -35 -30 -25

Val Leu Glu Val Thr Glu Glu Phe Gly Val His Leu Ala Glu Leu Thr
-20 -15 -10

Val Asp Pro Gln Gly Ala Leu Ala Ile Arg Gln Leu Ala Ser Val Ile
-5 1 5

Leu Lys Gln Tyr Val Glu Thr His Trp Cys Ala Gln Ser Glu Lys Phe
10 15 20

Arg
25

(2) INFORMATION FOR SEQ ID NO: 464:

(i) SEQUENCE CHARACTERISTICS:
(A) LENGTH: 130 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -117..-1

(C) IDENTIFICATION METHOD: Von Heijne matrix
(D) OTHER INFORMATION: score 3.8
seq XXXYLNFCPVCYC/FS

(xi) SEQUENCE DESCRIPTION: SEQ ID NO: 464:

Met Asn Ser Gly Gly Gly Phe Gly Leu Gly Leu Gly Phe Gly Leu Thr
-115 -110 -105

Pro Thr Ser Val Ile Gln Val Thr Asn Leu Ser Ser Ala Val Thr Ser
-100 -95 -90

Glu Gln Met Arg Thr Leu Phe Ser Phe Leu Gly Glu Ile Glu Glu Leu
-85 -80 -75 -70

Arg Leu Tyr Pro Pro Asp Asn Ala Pro Leu Ala Phe Ser Ser Xaa Val
-65 -60 -55

Cys Tyr Val Lys Phe Arg Asp Pro Ser Ser Val Gly Val Ala Gln His
-50 -45 -40

Leu Thr Asn Thr Val Phe Ile Asp Arg Xaa Leu Xaa Ser Cys Ser Leu
-35 -30 -25

Cys Arg Arg Leu Val Ser Arg Phe Xaa Xaa Xaa Tyr Leu Asn Phe Cys
-20 -15 -10

Pro Val Cys Tyr Cys Phe Ser Phe Pro Arg Asp Trp Gln Val Asp Ser
-5 1 5 10

Thr Leu

(2) INFORMATION FOR SEQ ID NO: 465:

(i) SEQUENCE CHARACTERISTICS:
(A) LENGTH: 43 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -13..-1
(C) IDENTIFICATION METHOD: Von Heijne matrix
(D) OTHER INFORMATION: score 3.7
seq MIEMLIFLDCVLS/SK

(xi) SEQUENCE DESCRIPTION: SEQ ID NO: 465:

Met Ile Glu Met Leu Ile Phe Leu Asp Cys Val Leu Ser Ser Lys Asp
-10 -5 1

Thr Ile Thr Met Phe Val Lys Phe Ile Pro Ile Phe Pro Phe Pro Leu
     5                   10                  15

Gln Phe Tyr Leu Pro Ser Phe Leu Leu Leu Glu
20                  25                  30

(2) INFORMATION FOR SEQ ID NO: 466:

     (i) SEQUENCE CHARACTERISTICS:
          (A) LENGTH: 81 amino acids
          (B) TYPE: AMINO ACID
          (D) TOPOLOGY: LINEAR

     (ii) MOLECULE TYPE: PROTEIN

     (vi) ORIGINAL SOURCE:
          (A) ORGANISM: Homo Sapiens
          (D) DEVELOPMENTAL STAGE: Fetal
          (F) TISSUE TYPE: kidney

     (ix) FEATURE:
          (A) NAME/KEY: sig_peptide
          (B) LOCATION: -79..-1
          (C) IDENTIFICATION METHOD: Von Heijne matrix
          (D) OTHER INFORMATION:  score 3.7
                                  seq VIGSLLVLTMLTC/RR

     (xi) SEQUENCE DESCRIPTION: SEQ ID NO: 466:

Met His Pro Phe Leu Ala Ala His Gly Pro Ala Phe His Lys Gly Tyr
                -75                 -70                 -65

Lys His Ser Thr Ile Asn Ile Val Asp Ile Tyr Pro Met Met Cys His
            -60                 -55                 -50

Ile Leu Gly Leu Lys Pro His Pro Asn Asn Gly Thr Phe Gly His Thr
        -45                 -40                 -35

Lys Cys Leu Leu Val Asp Gln Trp Cys Ile Asn Leu Pro Glu Ala Ile
    -30                 -25                 -20

Ala Ile Val Ile Gly Ser Leu Leu Val Leu Thr Met Leu Thr Cys Arg
-15                 -10                 -5                   1

Arg

(2) INFORMATION FOR SEQ ID NO: 467:

     (i) SEQUENCE CHARACTERISTICS:
          (A) LENGTH: 30 amino acids
          (B) TYPE: AMINO ACID
          (D) TOPOLOGY: LINEAR

     (ii) MOLECULE TYPE: PROTEIN

     (vi) ORIGINAL SOURCE:

(A) ORGANISM: Homo Sapiens
(F) TISSUE TYPE: Kidney

(ix) FEATURE:
(A) NAME/KEY: sig_peptide
(B) LOCATION: -14..-1
(C) IDENTIFICATION METHOD: Von Heijne matrix
(D) OTHER INFORMATION: score 3.7
seq IWPMSASVATLWS/FT

(xi) SEQUENCE DESCRIPTION: SEQ ID NO: 467:

(2) INFORMATION FOR SEQ ID NO: 468:

(i) SEQUENCE CHARACTERISTICS:
(A) LENGTH: 85 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -31..-1
(C) IDENTIFICATION METHOD: Von Heijne matrix
(D) OTHER INFORMATION: score 3.6
seq LFIYLVFVECLLC/TR

(xi) SEQUENCE DESCRIPTION: SEQ ID NO: 468:

Met Gly Ile Asp Ile Phe Tyr Pro Ser His Ile Pro Asp Phe His Pro
-30 -25 -20

Ile His Leu Phe Ile Tyr Leu Val Phe Val Glu Cys Leu Leu Cys Thr
-15 -10 -5 1

Arg Asn Xaa Xaa Xaa Leu Ser Xaa Phe Asn Cys Asp Asn Ala Gln Ile
                5                   10                  15

Ile Phe Thr Thr Gly Ser Ser Ser Ser Gly Gly Asn Lys Pro Phe Lys
            20                  25                  30

Ser Ser Leu Cys Thr Val His Arg Gly Gln Glu Arg Glu Arg Ile Glu
        35                  40                  45

Cys Gln Gly Asn Gly

    50

(2) INFORMATION FOR SEQ ID NO: 469:

(i) SEQUENCE CHARACTERISTICS:
(A) LENGTH: 116 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -87..-1
(C) IDENTIFICATION METHOD: Von Heijne matrix
(D) OTHER INFORMATION: score 3.6
seq LILQASLKGELEA/SQ

(xi) SEQUENCE DESCRIPTION: SEQ ID NO: 469:

Met Lys Glu Leu Asn Gln Lys Leu Thr Asn Lys Asn Asn Lys Ile Glu
-85 -80 -75

Asp Leu Glu Gln Glu Ile Lys Ile Gln Lys Gln Lys Gln Glu Thr Leu
-70 -65 -60

Gln Glu Glu Ile Thr Ser Leu Gln Ser Ser Val Gln Glu Tyr Glu Glu
-55 -50 -45 -40

Lys Asn Xaa Lys Ile Lys Gln Leu Leu Val Lys Thr Lys Lys Glu Leu
-35 -30 -25

Ala Asp Ser Lys Gln Ala Glu Thr Asp His Leu Ile Leu Gln Ala Ser
-20 -15 -10

Leu Lys Gly Glu Leu Glu Ala Ser Gln Gln Gln Val Glu Val Tyr Lys
-5 1 5

Val Arg Val Leu Leu Phe Lys Ile Lys Lys Met Phe Phe His Val Glu
10 15 20 25

Val Arg Asn Gly

(2) INFORMATION FOR SEQ ID NO: 470:

(i) SEQUENCE CHARACTERISTICS:
(A) LENGTH: 117 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -113..-1
(C) IDENTIFICATION METHOD: Von Heijne matrix
(D) OTHER INFORMATION: score 3.6
seq RLLLCILIIVCYI/LF

(xi) SEQUENCE DESCRIPTION: SEQ ID NO: 470:

Met Gly Asn Thr Leu Lys Glu Met Gln Asp Val Gln Gly Ala Leu Gln
-110 -105 -100

Cys Tyr Thr Arg Ala Ile Gln Ile Asn Pro Ala Phe Ala Asp Ala His
-95 -90 -85

Ser Asn Leu Ala Ser Ile His Lys Asp Ser Gly Asn Ile Pro Glu Ala
-80 -75 -70

Ile Ala Ser Tyr Arg Thr Ala Leu Lys Leu Lys Pro Asp Phe Pro Asp
-65 -60 -55 -50

Ala Tyr Cys Asn Leu Ala His Cys Leu Gln Ile Val Cys Asp Trp Thr
-45 -40 -35

Asp Tyr Asp Glu Arg Met Lys Lys Leu Val Ser Ile Val Ala Asp Gln
-30 -25 -20

Leu Glu Lys Asn Arg Leu Leu Leu Cys Ile Leu Ile Ile Val Cys Tyr
-15 -10 -5

Ile Leu Phe Leu Met
1

(2) INFORMATION FOR SEQ ID NO: 471:

(i) SEQUENCE CHARACTERISTICS:
(A) LENGTH: 42 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -39..-1
(C) IDENTIFICATION METHOD: Von Heijne matrix
(D) OTHER INFORMATION: score 3.6
seq VAYAIPSIPSLFC/QR

(xi) SEQUENCE DESCRIPTION: SEQ ID NO: 471:

(2) INFORMATION FOR SEQ ID NO: 472:

(i) SEQUENCE CHARACTERISTICS:
(A) LENGTH: 36 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 3.6
seq CVFLFPLISNTSS/YK

(xi) SEQUENCE DESCRIPTION: SEQ ID NO: 472:

(2) INFORMATION FOR SEQ ID NO: 473:

(i) SEQUENCE CHARACTERISTICS:
(A) LENGTH: 67 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(D) DEVELOPMENTAL STAGE: Fetal
(F) TISSUE TYPE: kidney

(ix) FEATURE:
(A) NAME/KEY: sig_peptide
(B) LOCATION: -42..-1
(C) IDENTIFICATION METHOD: Von Heijne matrix
(D) OTHER INFORMATION: score 3.5
seq LLLQGACPCLIFL/RP

(xi) SEQUENCE DESCRIPTION: SEQ ID NO: 473:

Met Phe Leu Ala Pro Ser Leu Leu Ile Thr Lys Leu Leu Thr Gly Ser
-40 -35 -30

Glu Ser Pro Asp Gly Asn Pro Pro Ala Leu Gly Arg Pro Leu Leu Leu
-25 -20 -15

Gln Gly Ala Cys Pro Cys Leu Ile Phe Leu Arg Pro Asp Glu Asn Lys
-10 -5 1 5

Lys Glu Gly Xaa Glu Glu Lys Lys Asn His Lys Leu Pro Leu Lys Thr
10 15 20

Ser Leu Gly
25

(2) INFORMATION FOR SEQ ID NO: 474:

(i) SEQUENCE CHARACTERISTICS:
(A) LENGTH: 24 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -18..-1
(C) IDENTIFICATION METHOD: Von Heijne matrix
(D) OTHER INFORMATION: score 3.5
seq SKSCLFYLQKVSG/IP

(xi) SEQUENCE DESCRIPTION: SEQ ID NO: 474:

Met Asp Pro Ser Ala Ser Lys Ser Cys Leu Phe Tyr Leu Gln Lys Val
-15 -10 -5

Ser Gly Ile Pro Gly Leu Leu Thr
1               5

(2) INFORMATION FOR SEQ ID NO: 475:

(i) SEQUENCE CHARACTERISTICS:
(A) LENGTH: 66 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -46..-1
(C) IDENTIFICATION METHOD: Von Heijne matrix
(D) OTHER INFORMATION: score 3.5
seq RWLCLQAYLASFS/LE

(xi) SEQUENCE DESCRIPTION: SEQ ID NO: 475:

(2) INFORMATION FOR SEQ ID NO: 476:

(i) SEQUENCE CHARACTERISTICS:
(A) LENGTH: 42 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -22..-1
(C) IDENTIFICATION METHOD: Von Heijne matrix
(D) OTHER INFORMATION: score 3.5
seq AQLASPLLPGATP/VA

(xi) SEQUENCE DESCRIPTION: SEQ ID NO: 476:

Met Lys Tyr Gln Met Val Ser Gly Ser Ala Gln Leu Ala Ser Pro Leu
-20 -15 -10

Leu Pro Gly Ala Thr Pro Val Ala Gly Thr Ile Leu Lys Ser Leu Leu
-5 1 5 10

Leu Arg Thr Val Lys Met Met Arg Val Met
15 20

(2) INFORMATION FOR SEQ ID NO: 477:

(i) SEQUENCE CHARACTERISTICS:
(A) LENGTH: 38 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -35..-1
(C) IDENTIFICATION METHOD: Von Heijne matrix
(D) OTHER INFORMATION: score 3.5
seq CFWGLMYXWLLLG/SX

(xi) SEQUENCE DESCRIPTION: SEQ ID NO: 477:

Met Asn Gly Thr Phe Pro Gly Thr Tyr Val Tyr Leu Val Ala Tyr Gly
-35 -30 -25 -20

Asp Leu Arg Ile Phe Gly Cys Phe Trp Gly Leu Met Tyr Xaa Trp Leu
-15 -10 -5

Leu Leu Gly Ser Xaa Gly
1

(2) INFORMATION FOR SEQ ID NO: 478:

(i) SEQUENCE CHARACTERISTICS:
(A) LENGTH: 97 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens

(F) TISSUE TYPE: Muscle

(ix) FEATURE:
(A) NAME/KEY: sig_peptide
(B) LOCATION: -21..-1
(C) IDENTIFICATION METHOD: Von Heijne matrix
(D) OTHER INFORMATION: score 12.7
seq ILFLLSWSGPLQG/QQ

(xi) SEQUENCE DESCRIPTION: SEQ ID NO: 478:

Met Gly Pro Ser Thr Pro Leu Leu Ile Leu Phe Leu Leu Ser Trp Ser
-20 -15 -10

Gly Pro Leu Gln Gly Gln Gln His His Leu Val Glu Tyr Met Glu Arg
-5 1 5 10

Arg Leu Ala Ala Leu Glu Glu Arg Leu Ala Gln Cys Gln Asp Gln Ser
15 20 25

Ser Arg His Ala Ala Glu Leu Arg Asn Phe Lys Asn Lys Met Leu Pro
30 35 40

Leu Leu Glu Val Ala Glu Lys Glu Arg Glu Ala Leu Arg Thr Glu Ala
45 50 55

Xaa Thr Ile Ser Xaa Gly Val Asp Arg Leu Glu Arg Glu Val Asp Tyr
60 65 70 75

Leu

(2) INFORMATION FOR SEQ ID NO: 479:

(i) SEQUENCE CHARACTERISTICS:
(A) LENGTH: 82 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -22..-1
(C) IDENTIFICATION METHOD: Von Heijne matrix
(D) OTHER INFORMATION: score 10.5
seq LMLLVSSLSPVQG/VL

(xi) SEQUENCE DESCRIPTION: SEQ ID NO: 479:

Met Lys Phe Ile Ser Thr Ser Leu Leu Leu Met Leu Leu Val Ser Ser
-20 -15 -10

Leu Ser Pro Val Gln Gly Val Leu Glu Val Tyr Tyr Thr Ser Leu Arg
     -5                   1           5                    10

Cys Arg Cys Val Gln Glu Ser Ser Val Phe Ile Pro Arg Arg Phe Ile
                15                  20                  25

Asp Arg Ile Gln Ile Leu Pro Arg Gly Asn Gly Cys Pro Arg Lys Glu
            30                  35                  40

Ile Ile Val Trp Lys Lys Asn Lys Ser Ile Val Cys Val Asp Leu Lys
        45                  50                  55

His Arg
     60

(2) INFORMATION FOR SEQ ID NO: 480:

     (i) SEQUENCE CHARACTERISTICS:
          (A) LENGTH: 137 amino acids
          (B) TYPE: AMINO ACID
          (D) TOPOLOGY: LINEAR

     (ii) MOLECULE TYPE: PROTEIN

     (vi) ORIGINAL SOURCE:
          (A) ORGANISM: Homo Sapiens
          (D) DEVELOPMENTAL STAGE: Fetal
          (F) TISSUE TYPE: kidney

     (ix) FEATURE:
          (A) NAME/KEY: sig_peptide
          (B) LOCATION: -47..-1
          (C) IDENTIFICATION METHOD: Von Heijne matrix
          (D) OTHER INFORMATION:  score 8
                                  seq VLELLAAVCLVRG/GH

     (xi) SEQUENCE DESCRIPTION: SEQ ID NO: 480:

Met Asn Tyr Gln Tyr Gly Phe Asn Met Val Met Ser His Pro His Ala
        -45                 -40                 -35

Val Asn Glu Ile Ala Leu Ser Leu Asn Asn Lys Asn Pro Arg Thr Lys
    -30                 -25                 -20

Ala Leu Val Leu Glu Leu Leu Ala Ala Val Cys Leu Val Arg Gly Gly
-15                 -10                  -5                   1

His Glu Ile Ile Leu Ser Ala Phe Asp Asn Phe Lys Glu Val Cys Gly
            5                   10                  15

Glu Lys Gln Arg Phe Glu Lys Leu Met Glu His Phe Arg Asn Glu Asp
        20                  25                  30

Asn Asn Ile Asp Phe Met Val Ala Ser Met Gln Phe Ile Asn Ile Val
     35                  40                  45

Val His Ser Val Glu Asp Met Asn Phe Arg Val His Leu Gln Tyr Glu
 50                  55                  60                  65

Phe Thr Lys Leu Gly Leu Xaa Glu Tyr Leu Xaa Lys Leu Lys His Thr
70 75 80

Glu Ser Asp Lys Leu Gln Val Gln Ile
85 90

(2) INFORMATION FOR SEQ ID NO: 481:

(i) SEQUENCE CHARACTERISTICS:
(A) LENGTH: 61 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -28..-1
(C) IDENTIFICATION METHOD: Von Heijne matrix
(D) OTHER INFORMATION: score 7.7
seq LVMCFLSYFGTFA/VE

(xi) SEQUENCE DESCRIPTION: SEQ ID NO: 481:

Met Ala Gln Ser Ile His Met Tyr Ala Ala Arg Val Gln Trp Gly Leu
-25 -20 -15

Val Met Cys Phe Leu Ser Tyr Phe Gly Thr Phe Ala Val Glu Phe Arg
-10 -5 1

His Tyr Arg Tyr Glu Ile Val Cys Ser Glu Tyr Gln Glu Asn Phe Leu
5 10 15 20

Ser Phe Ser Glu Ser Leu Ser Glu Ala Ser Glu Tyr Gln
25 30

(2) INFORMATION FOR SEQ ID NO: 482:

(i) SEQUENCE CHARACTERISTICS:
(A) LENGTH: 88 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -21..-1
(C) IDENTIFICATION METHOD: Von Heijne matrix
(D) OTHER INFORMATION: score 7.1
seq LHLFHLLIRPXQG/WX

(xi) SEQUENCE DESCRIPTION: SEQ ID NO: 482:

Met Gly Ser Gly Tyr Ser His Ser Leu His Leu Phe His Leu Leu Ile
-20 -15 -10

Arg Pro Xaa Gln Gly Trp Xaa Xaa Ile Val Pro Ala Cys Phe Trp Arg
-5 1 5 10

Lys Lys Ile Leu Thr Pro Ser Thr Gly Thr Met Glu Leu Leu Gln Val
15 20 25

Thr Ile Leu Phe Leu Leu Pro Ser Ile Cys Ser Ser Asn Ser Thr Gly
30 35 40

Val Leu Glu Ala Ala Asn Asn Ser Leu Val Val Thr Thr Thr Lys Pro
45 50 55

Ser Ile Thr Thr Pro Asn Thr Trp
60 65

(2) INFORMATION FOR SEQ ID NO: 483:

(i) SEQUENCE CHARACTERISTICS:
(A) LENGTH: 69 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -16..-1
(C) IDENTIFICATION METHOD: Von Heijne matrix
(D) OTHER INFORMATION: score 6.7
seq CFSLVLLLTSIWT/TR

(xi) SEQUENCE DESCRIPTION: SEQ ID NO: 483:

Met Ala Arg Cys Phe Ser Leu Val Leu Leu Leu Thr Ser Ile Trp Thr
-15 -10 -5

Thr Arg Leu Leu Val Gln Gly Ser Leu Arg Ala Glu Glu Leu Ser Ile
1               5                   10                  15

Gln Val Ser Cys Arg Ile Met Xaa Xaa Thr Leu Val Ser Lys Lys Ala
            20                  25                  30

Asn Gln Gln Leu Asn Phe Thr Glu Xaa Xaa Gly Gly Xaa Xaa Ala Ala
35 40 . 45

Gly Thr Lys Phe Gly
50

(2) INFORMATION FOR SEQ ID NO: 484:

(i) SEQUENCE CHARACTERISTICS:
(A) LENGTH: 40 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -33..-1
(C) IDENTIFICATION METHOD: Von Heijne matrix
(D) OTHER INFORMATION: score 6.7
seq MTCLSVLFGYATS/HP

(xi) SEQUENCE DESCRIPTION: SEQ ID NO: 484:

Met Ala Met Arg Tyr Asn Arg Leu Thr Val Leu Ala Gly Ala Met Leu
-30 -25 -20

Ala Leu Gly Leu Met Thr Cys Leu Ser Val Leu Phe Gly Tyr Ala Thr
-15 -10 -5

Ser His Pro Gln Gly Leu Tyr Ile
1               5

(2) INFORMATION FOR SEQ ID NO: 485:

(i) SEQUENCE CHARACTERISTICS:
(A) LENGTH: 53 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -26..-1
(C) IDENTIFICATION METHOD: Von Heijne matrix

(D) OTHER INFORMATION:  score 6.3
                           seq RQLLLPLPPFSFP/AP

(xi) SEQUENCE DESCRIPTION: SEQ ID NO: 485:

Met Pro Gln Gln Pro Val Glu Gln Gly Ser Pro Leu Leu Arg Gln Leu
    -25                 -20                 -15

Leu Leu Pro Leu Pro Pro Phe Ser Phe Pro Ala Pro Ser Pro Cys Pro
-10                 -5                  1               5

Ser Trp Pro Val Ala Leu Gly Ser His Gly Val Ala Tyr Trp Gly Ser
            10                  15                  20

Cys Ser Leu Gly His
        25

(2) INFORMATION FOR SEQ ID NO: 486:

(i) SEQUENCE CHARACTERISTICS:
    (A) LENGTH: 90 amino acids
    (B) TYPE: AMINO ACID
    (D) TOPOLOGY: LINEAR

(ii) MOLECULE TYPE: PROTEIN

(vi) ORIGINAL SOURCE:
    (A) ORGANISM: Homo Sapiens
    (D) DEVELOPMENTAL STAGE: Fetal
    (F) TISSUE TYPE: kidney

(ix) FEATURE:
    (A) NAME/KEY: sig_peptide
    (B) LOCATION: -80..-1
    (C) IDENTIFICATION METHOD: Von Heijne matrix
    (D) OTHER INFORMATION:  score 6.2
                            seq RASLLPMLLGSWA/FL

(xi) SEQUENCE DESCRIPTION: SEQ ID NO: 486:

Met Pro Ser Arg Ser Pro Phe Thr Trp Ser His Leu Cys Trp Arg Ala
-80                 -75                 -70                 -65

Gly Arg Cys Pro Arg Trp Arg Ala Cys Leu Ser Ser Ser Ser Val Arg
                -60                 -55                 -50

Met Cys Ser Pro Ala Ala Pro Ser Arg Phe Gly Ala Leu Gly Xaa Ser
            -45                 -40                 -35

Ala Arg Arg Trp Pro Arg Arg Asp Ala Asp Thr Trp Cys Ala Pro Gln
        -30                 -25                 -20

Gly Val Met Arg Ala Ser Leu Leu Pro Met Leu Leu Gly Ser Trp Ala
    -15                 -10                 -5

Phe Leu Pro Pro Ser Cys Ser Pro Arg Ala
1               5                   10

(2) INFORMATION FOR SEQ ID NO: 487:

(i) SEQUENCE CHARACTERISTICS:
(A) LENGTH: 95 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -40..-1
(C) IDENTIFICATION METHOD: Von Heijne matrix
(D) OTHER INFORMATION: score 6
seq LTYGIILTHGASG/DM

(xi) SEQUENCE DESCRIPTION: SEQ ID NO: 487:

Met Ser His Thr Glu Val Lys Leu Lys Ile Pro Phe Gly Asn Lys Leu
-40 -35 -30 -25

Leu Asp Ala Val Cys Leu Val Pro Asn Lys Ser Leu Thr Tyr Gly Ile
-20 -15 -10

Ile Leu Thr His Gly Ala Ser Gly Asp Met Asn Leu Pro His Leu Met
-5 1 5

Ser Leu Ala Ser His Leu Ala Ser His Gly Phe Phe Cys Leu Arg Phe
10 15 20

Thr Cys Lys Gly Leu Asn Ile Val His Arg Ile Lys Ala Tyr Lys Ser
25 30 35 40

Val Leu Asn Tyr Leu Lys Thr Ser Gly Xaa Tyr Lys Leu Ala Gly
45 50 55

(2) INFORMATION FOR SEQ ID NO: 488:

(i) SEQUENCE CHARACTERISTICS:
(A) LENGTH: 76 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:

(A) NAME/KEY: sig_peptide
(B) LOCATION: -40..-1
(C) IDENTIFICATION METHOD: Von Heijne matrix
(D) OTHER INFORMATION:  score 6
                        seq LCXEFXSVASCDA/AV

(xi) SEQUENCE DESCRIPTION: SEQ ID NO: 488:

Met Glu Leu Gly Ser Cys Leu Glu Gly Gly Arg Glu Ala Ala Glu Glu
-40                 -35                 -30                 -25

Glu Gly Glu Pro Glu Val Lys Lys Arg Arg Leu Leu Cys Xaa Glu Phe
                -20                 -15                 -10

Xaa Ser Val Ala Ser Cys Asp Ala Ala Val Ala Gln Cys Phe Leu Ala
            -5                   1               5

Xaa Asn Asp Trp Glu Met Glu Arg Ala Leu Asn Ser Tyr Phe Glu Pro
     10                  15                  20

Pro Val Glu Glu Ser Ala Leu Glu Arg Arg Pro Xaa
 25                  30                  35

(2) INFORMATION FOR SEQ ID NO: 489:

(i) SEQUENCE CHARACTERISTICS:
(A) LENGTH: 57 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -36..-1
(C) IDENTIFICATION METHOD: Von Heijne matrix
(D) OTHER INFORMATION:  score 5.8
                        seq AFVSGLLIGQCSS/QK

(xi) SEQUENCE DESCRIPTION: SEQ ID NO: 489:

Met Gly Arg Thr Tyr Ile Val Glu Glu Thr Val Gly Gln Tyr Leu Ser
    -35                 -30                 -25

Asn Ile Asn Leu Gln Gly Lys Ala Phe Val Ser Gly Leu Leu Ile Gly
-20                 -15                 -10                  -5

Gln Cys Ser Ser Gln Lys Asp Tyr Val Ile Leu Ala Thr Arg Thr Pro
                 1               5                  10

Pro Lys Glu Glu Gln Ser Glu Asn Leu
        15                  20

(2) INFORMATION FOR SEQ ID NO: 490:

(i) SEQUENCE CHARACTERISTICS:
(A) LENGTH: 122 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -21..-1
(C) IDENTIFICATION METHOD: Von Heijne matrix
(D) OTHER INFORMATION: score 5.6
seq CLSCLLIPLALWS/II

(xi) SEQUENCE DESCRIPTION: SEQ ID NO: 490:

Met Gly Ser Arg Lys Cys Gly Gly Cys Leu Ser Cys Leu Leu Ile Pro
-20 -15 -10

Leu Ala Leu Trp Ser Ile Ile Val Asn Ile Leu Leu Tyr Phe Pro Asn
-5 1 5 10

Gly Gln Thr Ser Tyr Ala Ser Ser Asn Lys Leu Thr Asn Tyr Val Trp
15 20 25

Tyr Phe Glu Gly Ile Cys Phe Ser Gly Ile Met Met Leu Ile Val Thr
30 35 40

Thr Val Leu Leu Val Leu Glu Asn Asn Asn Asn Tyr Lys Cys Cys Gln
45 50 55

Ser Glu Asn Cys Ser Lys Lys Tyr Val Thr Leu Leu Ser Ile Ile Phe
60 65 70 75

Ser Ser Leu Gly Ile Ala Phe Ser Gly Tyr Cys Leu Val Ile Ser Ala
80 85 90

Leu Gly Leu Val Gln Gly Pro Tyr Cys Arg
95 100

(2) INFORMATION FOR SEQ ID NO: 491:

(i) SEQUENCE CHARACTERISTICS:
(A) LENGTH: 150 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -21..-1
(C) IDENTIFICATION METHOD: Von Heijne matrix
(D) OTHER INFORMATION: score 5.6
seq CLSCLLIPLALWS/II

(xi) SEQUENCE DESCRIPTION: SEQ ID NO: 491:

Met Gly Ser Arg Lys Cys Gly Gly Cys Leu Ser Cys Leu Leu Ile Pro
-20 -15 -10

Leu Ala Leu Trp Ser Ile Ile Val Asn Ile Leu Leu Tyr Phe Pro Asn
-5 1 5 10

Gly Gln Thr Ser Tyr Ala Ser Ser Asn Lys Leu Thr Asn Tyr Val Trp
15 20 25

Tyr Phe Glu Gly Ile Cys Phe Ser Gly Ile Met Met Leu Ile Val Thr
30 35 40

Thr Val Leu Leu Val Leu Glu Asn Asn Asn Asn Tyr Lys Cys Cys Gln
45 50 55

Ser Glu Asn Cys Ser Lys Lys Tyr Val Thr Leu Leu Ser Ile Ile Phe
60 65 70 75

Ser Ser Leu Gly Ile Ala Phe Ser Gly Tyr Cys Leu Val Ile Ser Ala
80 85 90

Leu Gly Leu Val Gln Gly Pro Tyr Cys Arg Thr Leu Asp Gly Trp Glu
95 100 105

Tyr Ala Phe Glu Gly Thr Xaa Gly Arg Phe Leu Thr Asp Ser Ser Ile
110 115 120

Trp Ile Gln Cys Leu Glu
125

(2) INFORMATION FOR SEQ ID NO: 492:

(i) SEQUENCE CHARACTERISTICS:
(A) LENGTH: 36 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -21..-1
(C) IDENTIFICATION METHOD: Von Heijne matrix
(D) OTHER INFORMATION: score 5.5
seq SFLPSALVIWTSA/AF

(xi) SEQUENCE DESCRIPTION: SEQ ID NO: 492:

Met Trp Trp Phe Gln Gln Gly Leu Ser Phe Leu Pro Ser Ala Leu Val
-20 -15 -10

Ile Trp Thr Ser Ala Ala Phe Ile Phe Ser Tyr Ile Thr Ala Val Thr
-5 1 5 10

Leu His His Ile
15

(2) INFORMATION FOR SEQ ID NO: 493:

(i) SEQUENCE CHARACTERISTICS:
(A) LENGTH: 59 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -41..-1
(C) IDENTIFICATION METHOD: Von Heijne matrix
(D) OTHER INFORMATION: score 5.4
seq PLIFSLWCSGVLL/HI

(xi) SEQUENCE DESCRIPTION: SEQ ID NO: 493:

(2) INFORMATION FOR SEQ ID NO: 494:

(i) SEQUENCE CHARACTERISTICS:
(A) LENGTH: 37 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -29..-1
(C) IDENTIFICATION METHOD: Von Heijne matrix
(D) OTHER INFORMATION: score 13.4
seq SLLLVQLLTPCSA/QF

(xi) SEQUENCE DESCRIPTION: SEQ ID NO: 494:

Met Lys Met Ala Ser Ser Leu Ala Phe Leu Leu Leu Asn Phe His Val
-25 -20 -15

Ser Leu Leu Leu Val Gln Leu Leu Thr Pro Cys Ser Ala Gln Phe Ser
-10 -5 1

Val Leu Gly Pro Leu
                5

(2) INFORMATION FOR SEQ ID NO: 495:

(i) SEQUENCE CHARACTERISTICS:
(A) LENGTH: 47 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -42..-1
(C) IDENTIFICATION METHOD: Von Heijne matrix
(D) OTHER INFORMATION: score 5.2
seq LLFDLVCHEFCQS/DD

(xi) SEQUENCE DESCRIPTION: SEQ ID NO: 495:

Met His Ile Leu Gln Leu Leu Thr Thr Val Asp Asp Gly Ile Gln Ala
-40 -35 -30

Ile Val His Cys Pro Asp Thr Gly Lys Asp Ile Trp Asn Leu Leu Phe
    -25                 -20                 -15

Asp Leu Val Cys His Glu Phe Cys Gln Ser Asp Asp Pro Ala Arg
-10                 -5                  1               5

(2) INFORMATION FOR SEQ ID NO: 496:

    (i) SEQUENCE CHARACTERISTICS:
        (A) LENGTH: 102 amino acids
        (B) TYPE: AMINO ACID
        (D) TOPOLOGY: LINEAR

    (ii) MOLECULE TYPE: PROTEIN

    (vi) ORIGINAL SOURCE:
        (A) ORGANISM: Homo Sapiens
        (D) DEVELOPMENTAL STAGE: Fetal
        (F) TISSUE TYPE: kidney

    (ix) FEATURE:
        (A) NAME/KEY: sig_peptide
        (B) LOCATION: -43..-1
        (C) IDENTIFICATION METHOD: Von Heijne matrix
        (D) OTHER INFORMATION:  score 5.2
                                seq PMQLLQVLSDVLA/EI

    (xi) SEQUENCE DESCRIPTION: SEQ ID NO: 496:

Met Ser Asp Gln Ile Lys Phe Ile Met Asp Ser Leu Asn Lys Glu Pro
            -40                 -35                 -30

Phe Arg Lys Asn Tyr Asn Leu Ile Thr Phe Xaa Ser Leu Glu Pro Met
        -25                 -20                 -15

Gln Leu Leu Gln Val Leu Ser Asp Val Leu Ala Glu Ile Asp Pro Lys
    -10                 -5                  1               5

Gln Leu Val Asp Ile Arg Glu Glu Met Pro Glu Gln Thr Ala Lys Arg
                10                  15                  20

Met Leu Ser Leu Leu Gly Ile Leu Lys Tyr Lys Pro Ser Gly Asn Ala
            25                  30                  35

Thr Asp Met Ser Thr Phe Arg Gln Gly Leu Val Ile Gly Ser Lys Pro
        40                  45                  50

Val Ile Tyr Pro Val Leu
    55

(2) INFORMATION FOR SEQ ID NO: 497:

    (i) SEQUENCE CHARACTERISTICS:
        (A) LENGTH: 93 amino acids
        (B) TYPE: AMINO ACID
        (D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -79..-1
(C) IDENTIFICATION METHOD: Von Heijne matrix
(D) OTHER INFORMATION: score 4.6
seq IIHAXGLVRECLA/XT

(xi) SEQUENCE DESCRIPTION: SEQ ID NO: 497:

Met Ala Thr Ser Ser Gln Xaa Arg Gln Leu Leu Ser Asp Tyr Gly Pro
-75 -70 -65

Pro Ser Leu Gly Tyr Thr Gln Gly Thr Gly Asn Ser Gln Xaa Pro Gln
-60 -55 -50

Ser Lys Tyr Ala Glu Leu Leu Ala Ile Ile Xaa Glu Leu Gly Lys Glu
-45 -40 -35

Ile Arg Pro Met Tyr Ala Gly Ser Lys Ser Ala Met Glu Arg Leu Lys
-30 -25 -20

Arg Gly Ile Ile His Ala Xaa Gly Leu Val Arg Glu Cys Leu Ala Xaa
-15 -10 -5 1

Thr Glu Arg Met Pro Asp Pro Ser Cys Leu Val Gly Phe
                5                   10

(2) INFORMATION FOR SEQ ID NO: 498:

(i) SEQUENCE CHARACTERISTICS:
(A) LENGTH: 59 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -15..-1
(C) IDENTIFICATION METHOD: Von Heijne matrix
(D) OTHER INFORMATION: score 4.5
seq LLGAAAVAALGRG/RA

(xi) SEQUENCE DESCRIPTION: SEQ ID NO: 498:

Met Arg Leu Leu Gly Ala Ala Ala Val Ala Ala Leu Gly Arg Gly Arg
-15                 -10             .       -5                   1

Ala Pro Ala Ser Leu Gly Trp Gln Arg Lys Gln Val Asn Trp Lys Ala
            5                   10                  15

Cys Arg Trp Ser Ser Ser Gly Val Ile Pro Asn Glu Lys Ile Arg Asn
        20                  25                  30

Ile Gly Ile Ser Ala His Ile Asp Ser Gly Lys
    35                  40

(2) INFORMATION FOR SEQ ID NO: 499:

(i) SEQUENCE CHARACTERISTICS:
(A) LENGTH: 51 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -16..-1
(C) IDENTIFICATION METHOD: Von Heijne matrix
(D) OTHER INFORMATION: score 4.5
seq RLLLRRFLASVIS/RK

(xi) SEQUENCE DESCRIPTION: SEQ ID NO: 499:

Met Ala Gln Arg Leu Leu Leu Arg Arg Phe Leu Ala Ser Val Ile Ser
    -15                 -10                 -5

Arg Lys Pro Ser Gln Gly Gln Trp Pro Pro Leu Thr Ser Arg Ala Leu
 1              5                   10                  15

Gln Thr Pro Gln Cys Ser Pro Gly Gly Leu Thr Val Thr Pro Asn Pro
            20                  25                  30

Ala Pro Gly
        35

(2) INFORMATION FOR SEQ ID NO: 500:

(i) SEQUENCE CHARACTERISTICS:
(A) LENGTH: 32 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -16..-1
(C) IDENTIFICATION METHOD: Von Heijne matrix
(D) OTHER INFORMATION: score 4.4
seq LNSLSALAELAVG/SR

(xi) SEQUENCE DESCRIPTION: SEQ ID NO: 500:

Met Phe Arg Leu Asn Ser Leu Ser Ala Leu Ala Glu Leu Ala Val Gly
-15 -10 -5

Ser Arg Trp Tyr His Gly Gly Ser Gln Pro Ile Gln Ile Arg Leu Ala
1               5                   10                  15

(2) INFORMATION FOR SEQ ID NO: 501:

(i) SEQUENCE CHARACTERISTICS:
(A) LENGTH: 90 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -61..-1
(C) IDENTIFICATION METHOD: Von Heijne matrix
(D) OTHER INFORMATION: score 3.9
seq YTAVSVLAGPRWA/DP

(xi) SEQUENCE DESCRIPTION: SEQ ID NO: 501:

Met Ser Gly Ser Asn Gly Ser Lys Glu Asn Ser His Asn Lys Ala Arg
-60 -55 -50

Thr Ser Pro Tyr Pro Gly Ser Lys Val Glu Arg Ser Gln Val Pro Asn
-45 -40 -35 -30

Glu Lys Val Gly Trp Leu Val Glu Trp Gln Asp Tyr Lys Pro Val Glu
-25 -20 -15

Tyr Thr Ala Val Ser Val Leu Ala Gly Pro Arg Trp Ala Asp Pro Gln
-10 -5 1

Ile Ser Glu Ser Asn Phe Ser Pro Lys Phe Asn Glu Lys Asp Gly His
                5                   10                  15

Val Glu Arg Lys Ser Lys Asn Gly Leu Tyr
 20                    25

(2) INFORMATION FOR SEQ ID NO: 502:

      (i) SEQUENCE CHARACTERISTICS:
            (A) LENGTH: 21 amino acids
            (B) TYPE: AMINO ACID
            (D) TOPOLOGY: LINEAR

      (ii) MOLECULE TYPE: PROTEIN

      (vi) ORIGINAL SOURCE:
            (A) ORGANISM: Homo Sapiens
            (F) TISSUE TYPE: Dystrophic muscle

      (ix) FEATURE:
            (A) NAME/KEY: sig_peptide
            (B) LOCATION: -16..-1
            (C) IDENTIFICATION METHOD: Von Heijne matrix
            (D) OTHER INFORMATION:  score 3.5
                                    seq TLMFSLTAQWXTS/RS

      (xi) SEQUENCE DESCRIPTION: SEQ ID NO: 502:

Met Arg Thr Thr Leu Met Phe Ser Leu Thr Ala Gln Trp Xaa Thr Ser
    -15                 -10                 -5

Arg Ser Ser Phe Gln
  1               5

(2) INFORMATION FOR SEQ ID NO: 503:

      (i) SEQUENCE CHARACTERISTICS:
            (A) LENGTH: 104 amino acids
            (B) TYPE: AMINO ACID
            (D) TOPOLOGY: LINEAR

      (ii) MOLECULE TYPE: PROTEIN

      (vi) ORIGINAL SOURCE:
            (A) ORGANISM: Homo Sapiens
            (D) DEVELOPMENTAL STAGE: Fetal
            (F) TISSUE TYPE: kidney

      (ix) FEATURE:
            (A) NAME/KEY: sig_peptide
            (B) LOCATION: -25..-1
            (C) IDENTIFICATION METHOD: Von Heijne matrix
            (D) OTHER INFORMATION:  score 14.1
                                    seq LTLLLLLTLLAFA/GY

      (xi) SEQUENCE DESCRIPTION: SEQ ID NO: 503:

Met Ser Asp Leu Leu Leu Leu Gly Leu Ile Gly Gly Leu Thr Leu Leu
-25                 -20                 -15                 -10

Leu Leu Leu Thr Leu Leu Ala Phe Ala Gly Tyr Ser Gly Leu Leu Ala
                -5                  1               5

Gly Val Glu Val Ser Ala Gly Ser Pro Pro Ile Arg Asn Val Thr Val
        10                  15                  20

Ala Tyr Lys Phe His Met Gly Leu Tyr Gly Glu Thr Gly Arg Leu Phe
    25                  30                  35

Thr Glu Ser Cys Ser Ile Ser Pro Lys Leu Arg Ser Ile Ala Val Tyr
40                  45                  50                  55

Tyr Asp Asn Pro His Met Val Pro Pro Asp Lys Cys Arg Cys Ala Val
                60                  65                  70

Gly Ser Ile Leu Ser Glu Gly Glu
            75

(2) INFORMATION FOR SEQ ID NO: 504:

(i) SEQUENCE CHARACTERISTICS:
(A) LENGTH: 78 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -32..-1
(C) IDENTIFICATION METHOD: Von Heijne matrix
(D) OTHER INFORMATION:  score 11.4
seq LWSLALWLPLALS/VS

(xi) SEQUENCE DESCRIPTION: SEQ ID NO: 504:

Met Glu Gly Thr Glu Met Gly Ala Arg Pro Gly Gly His Pro Xaa Lys
        -30                 -25                 -20

Trp Ser Phe Leu Trp Ser Leu Ala Leu Trp Leu Pro Leu Ala Leu Ser
    -15                 -10                 -5

Val Ser Leu Phe Leu Gly Leu Ser Leu Ser Pro Pro Gln Pro Gly Leu
1               5                   10                  15

Ser Leu Trp Cys Thr Leu Ser Tyr Cys Cys Glu Gln Trp Lys Phe Lys
            20                  25                  30

Gly Thr Pro Ser Pro Ala Leu Leu Asn Leu Gly Thr Arg Gly
        35                  40                  45

(2) INFORMATION FOR SEQ ID NO: 505:

(i) SEQUENCE CHARACTERISTICS:
(A) LENGTH: 86 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -55..-1
(C) IDENTIFICATION METHOD: Von Heijne matrix
(D) OTHER INFORMATION: score 11.2
seq LLFALGSLGLIFA/LI

(xi) SEQUENCE DESCRIPTION: SEQ ID NO: 505:

Met Xaa Phe Leu Arg Lys Val Xaa Ser Ile Leu Ser Leu Gln Val Leu
-55 -50 -45 -40

Leu Thr Thr Val Thr Ser Thr Val Phe Leu Tyr Phe Glu Ser Val Arg
-35 -30 -25

Thr Phe Val Xaa Glu Ser Pro Ala Leu Ile Leu Leu Phe Ala Leu Gly
-20 -15 -10

Ser Leu Gly Leu Ile Phe Ala Leu Ile Leu Asn Xaa His Lys Tyr Pro
-5 1 5

Leu Asn Leu Tyr Leu Leu Phe Gly Phe Thr Leu Leu Xaa Ala Leu Thr
10 15 20 25

Val Ala Val Val Val Thr
30

(2) INFORMATION FOR SEQ ID NO: 506:

(i) SEQUENCE CHARACTERISTICS:
(A) LENGTH: 45 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide

(B) LOCATION: -38..-1
(C) IDENTIFICATION METHOD: Von Heijne matrix
(D) OTHER INFORMATION: score 10.8
seq MLLLLLLLGSGQG/PQ

(xi) SEQUENCE DESCRIPTION: SEQ ID NO: 506:

Met Ala Ala Thr Leu Gly Pro Leu Gly Ser Trp Gln Gln Trp Arg Arg
-35 -30 -25

Cys Leu Ser Ala Arg Asp Gly Ser Arg Met Leu Leu Leu Leu Leu Leu
-20 -15 -10

Leu Gly Ser Gly Gln Gly Pro Gln Gln Val Gly Ala Gly
-5 1 5

(2) INFORMATION FOR SEQ ID NO: 507:

(i) SEQUENCE CHARACTERISTICS:
(A) LENGTH: 53 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -41..-1
(C) IDENTIFICATION METHOD: Von Heijne matrix
(D) OTHER INFORMATION: score 9.9
seq ILPFLLFPFPVNA/RS

(xi) SEQUENCE DESCRIPTION: SEQ ID NO: 507:

Met Ser Ser Trp Met Tyr Leu Gly Tyr Pro Ile Val Thr Ser Asn Thr
-40 -35 -30

Thr Cys Leu Lys Leu Ile Ser Ser Ser Phe Pro Gln Ile Leu Pro Phe
-25 -20 -15 -10

Leu Leu Phe Pro Phe Pro Val Asn Ala Arg Ser His Xaa Val Ala Gln
-5 1 5

Thr Lys Ser Pro Arg
10

(2) INFORMATION FOR SEQ ID NO: 508:

(i) SEQUENCE CHARACTERISTICS:
(A) LENGTH: 38 amino acids

(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -21..-1
(C) IDENTIFICATION METHOD: Von Heijne matrix
(D) OTHER INFORMATION: score 9.7
seq QLCLLLLPSCSLS/VS

(xi) SEQUENCE DESCRIPTION: SEQ ID NO: 508:

(2) INFORMATION FOR SEQ ID NO: 509:

(i) SEQUENCE CHARACTERISTICS:
(A) LENGTH: 110 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -93..-1
(C) IDENTIFICATION METHOD: Von Heijne matrix
(D) OTHER INFORMATION: score 9.6
seq LSLSLGASAPVQC/QQ

(xi) SEQUENCE DESCRIPTION: SEQ ID NO: 509:

Met Arg His Gly Phe Ile Gln Gln Gln Phe Ser Leu Thr Ala Phe Ser
-90 -85 -80

Xaa Xaa Xaa Xaa Ile Phe Thr Leu Xaa Xaa Leu Ser Gln Leu Leu Ser
-75 -70 -65

Ser Ala Ala Pro Lys His Thr Ala Ala Pro Thr Ala Leu Pro Cys Leu

-60                    -55                    -50

Gln Gly Gln Gln Leu Asn Ser Leu Ser Leu Gly Thr Ser Glu Leu Ser
-45                 -40                 -35                 -30

Cys Val Leu Ala Ser Ser Cys Leu Ser Thr Lys Thr Asp Pro Ser Gly
                -25                 -20                 -15

Leu Ser Leu Ser Leu Gly Ala Ser Ala Pro Val Gln Cys Gln Gln Asp
            -10                 -5                  1

Asn Tyr Thr Phe Cys Xaa Gln Tyr Trp Leu Arg Ala Arg His
    5                   10                  15

(2) INFORMATION FOR SEQ ID NO: 510:

(i) SEQUENCE CHARACTERISTICS:
(A) LENGTH: 77 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -41..-1
(C) IDENTIFICATION METHOD: Von Heijne matrix
(D) OTHER INFORMATION: score 9.5
seq LIIFLSFLPFINS/SF

(xi) SEQUENCE DESCRIPTION: SEQ ID NO: 510:

Met Phe Gln Asn Ile Gln Lys Cys Leu Asn Val Pro Phe Val Arg Gly
    -40                 -35                 -30

Tyr His Val Phe Tyr Ile Asn Leu Asn Ala Val Ile Leu Ile Ile Phe
-25                 -20                 -15                 -10

Leu Ser Phe Leu Pro Phe Ile Asn Ser Ser Phe Val Tyr Lys Thr Asn
                -5                  1                   5

Pro Leu Tyr Asp Ala Ile Ser Asn Tyr Val Phe Ser Phe Arg Tyr Pro
        10                  15                  20

Asn Leu Xaa Xaa Phe Ala Leu Asp Val Arg Leu Val Phe
    25                  30                  35

(2) INFORMATION FOR SEQ ID NO: 511:

(i) SEQUENCE CHARACTERISTICS:
(A) LENGTH: 27 amino acids

(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 9.2
seq FPVLALFLSGSLA/LF

(xi) SEQUENCE DESCRIPTION: SEQ ID NO: 511:

Met Ser Leu Ser Gln Arg Gly Phe Pro Val Leu Ala Leu Phe Leu Ser
-20 -15 -10 -5

Gly Ser Leu Ala Leu Phe His His Thr Ser Gly
1 5

(2) INFORMATION FOR SEQ ID NO: 512:

(i) SEQUENCE CHARACTERISTICS:
(A) LENGTH: 70 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -29..-1
(C) IDENTIFICATION METHOD: Von Heijne matrix
(D) OTHER INFORMATION: score 8.9
seq ALLIVCDVPSASA/QR

(xi) SEQUENCE DESCRIPTION: SEQ ID NO: 512:

Met Ala Ala Arg Trp Arg Phe Trp Cys Val Ser Val Thr Met Val Val
-25 -20 -15

Ala Leu Leu Ile Val Cys Asp Val Pro Ser Ala Ser Ala Gln Arg Lys
-10 -5 1

Lys Glu Met Val Leu Ser Glu Lys Val Ser Gln Leu Met Glu Trp Thr
                5                   10                  15

Asn Lys Arg Pro Val Ile Arg Met Asn Gly Asp Lys Phe Arg Arg Leu
20                  25                  30                  35

Val Lys Xaa Pro Pro Arg
                40

(2) INFORMATION FOR SEQ ID NO: 513:

(i) SEQUENCE CHARACTERISTICS:
(A) LENGTH: 46 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -32..-1
(C) IDENTIFICATION METHOD: Von Heijne matrix
(D) OTHER INFORMATION:  score 8.8
                        seq VPMLLLIVGGSFG/LR

(xi) SEQUENCE DESCRIPTION: SEQ ID NO: 513:

Met Phe Ala Pro Ala Val Met Arg Ala Phe Arg Lys Asn Lys Thr Leu
        -30                 -25                 -20

Gly Tyr Gly Val Pro Met Leu Leu Leu Ile Val Gly Gly Ser Phe Gly
    -15                 -10                 -5

Leu Arg Glu Phe Ser Xaa Ile Arg Tyr Asp Ala Val Lys Gly
1               5                   10

(2) INFORMATION FOR SEQ ID NO: 514:

(i) SEQUENCE CHARACTERISTICS:
(A) LENGTH: 103 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: -37..-1
(C) IDENTIFICATION METHOD: Von Heijne matrix
(D) OTHER INFORMATION:  score 8.5

seq LLVLLLYAPVGFC/LL

(xi) SEQUENCE DESCRIPTION: SEQ ID NO: 514:

Met Glu Leu Pro Ser Gly Pro Gly Pro Glu Arg Leu Phe Asp Ser His
        -35                 -30                 -25

Arg Leu Pro Gly Asp Cys Phe Leu Leu Leu Val Leu Leu Leu Tyr Ala
    -20                 -15                 -10

Pro Val Gly Phe Cys Leu Leu Val Leu Xaa Leu Phe Leu Gly Ile His
 -5                  1               5                  10

Val Phe Leu Val Ser Cys Ala Leu Pro Asp Ser Val Leu Arg Arg Phe
            15                  20                  25

Val Val Arg Thr Met Cys Ala Val Leu Gly Leu Val Ala Arg Gln Glu
        30                  35                  40

Asp Ser Gly Leu Arg Asp His Ser Val Arg Val Leu Ile Ser Asn His
    45                  50                  55

Val Thr Pro Phe Asp His Gln
 60                  65

(2) INFORMATION FOR SEQ ID NO: 515:

(i) SEQUENCE CHARACTERISTICS:
(A) LENGTH: 92 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -90..-1
(C) IDENTIFICATION METHOD: Von Heijne matrix
(D) OTHER INFORMATION: score 8.4
seq SLVLLTVTPSXRQ/QE

(xi) SEQUENCE DESCRIPTION: SEQ ID NO: 515:

Met Ala Gln Ser Gln Gly Trp Val Xaa Arg Tyr Xaa Lys Ala Phe Cys
-90                 -85                 -80                 -75

Lys Gly Phe Phe Val Ala Val Pro Val Ala Val Thr Phe Leu Asp Arg
                -70                 -65                 -60

Val Ala Cys Val Ala Arg Val Glu Gly Ala Ser Met Gln Pro Ser Leu
            -55                 -50                 -45

Asn Pro Gly Gly Ser Xaa Ser Ser Asp Val Val Xaa Xaa Asn His Trp
     -40                 -35                 -30

Lys Val Arg Asn Phe Glu Val His Arg Gly Asp Ile Val Ser Leu Val
    -25                 -20                 -15

Leu Leu Thr Val Thr Pro Ser Xaa Arg Gln Gln Glu
-10                 -5                   1

(2) INFORMATION FOR SEQ ID NO: 516:

(i) SEQUENCE CHARACTERISTICS:
(A) LENGTH: 85 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -23..-1
(C) IDENTIFICATION METHOD: Von Heijne matrix
(D) OTHER INFORMATION: score 8.1
seq WLLVLSFVFGCNV/LR

(xi) SEQUENCE DESCRIPTION: SEQ ID NO: 516:

Met Ser Ser Ala Ala Ala Asp His Trp Ala Trp Leu Leu Val Leu Ser
            -20                 -15                 -10

Phe Val Phe Gly Cys Asn Val Leu Arg Ile Leu Xaa Pro Xaa Xaa Xaa
        -5                   1               5

Ile Xaa Xaa Val Gln Gly Ala Ala Glu Gly Arg Gly Xaa Glu Ser Gln
 10                  15                  20                  25

Met Arg Ala Glu Ile Gln Asp Met Lys Gln Glu Leu Ser Thr Val Asn
                 30                  35                  40

Met Met Asp Glu Phe Ala Arg Tyr Ala Arg Leu Xaa Arg Lys Ile Asn
             45                  50                  55

Lys Met Thr Asp Lys
        60

(2) INFORMATION FOR SEQ ID NO: 517:

(i) SEQUENCE CHARACTERISTICS:
(A) LENGTH: 34 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 8
seq HVFFLLLLAHIIA/LE

(xi) SEQUENCE DESCRIPTION: SEQ ID NO: 517:

Met Asn Leu Phe Lys Thr Asn His Val Phe Phe Leu Leu Leu Leu Ala
-20 -15 -10 -5

His Ile Ile Ala Leu Glu Ser Ile Ala Trp Phe Thr Val Phe Tyr Phe
1 5 10

Gly Asn

(2) INFORMATION FOR SEQ ID NO: 518:

(i) SEQUENCE CHARACTERISTICS:
(A) LENGTH: 43 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -24..-1
(C) IDENTIFICATION METHOD: Von Heijne matrix
(D) OTHER INFORMATION: score 7.9
seq LLLPRVLLTMASG/SP

(xi) SEQUENCE DESCRIPTION: SEQ ID NO: 518:

Met Pro Ala Leu Leu Pro Val Ala Ser Arg Leu Leu Leu Leu Pro Arg
-20 -15 -10

Val Leu Leu Thr Met Ala Ser Gly Ser Pro Pro Thr Gln Pro Ser Pro
-5 1 5

Ala Ser Asp Ser Gly Ser Gly Tyr Val Pro Gly
10 15

(2) INFORMATION FOR SEQ ID NO: 519:

(i) SEQUENCE CHARACTERISTICS:
(A) LENGTH: 96 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -66..-1
(C) IDENTIFICATION METHOD: Von Heijne matrix
(D) OTHER INFORMATION: score 7.9
seq LLLPRVLLTMASG/SP

(xi) SEQUENCE DESCRIPTION: SEQ ID NO: 519:

Met Ile Gly Ser Gly Leu Ala Gly Ser Gly Gly Ala Gly Gly Pro Ser
-65 -60 -55

Ser Thr Val Thr Trp Cys Ala Leu Phe Ser Asn His Val Ala Ala Thr
-50 -45 -40 -35

Gln Ala Ser Leu Leu Leu Ser Phe Val Trp Met Pro Ala Leu Leu Pro
-30 -25 -20

Val Ala Ser Arg Leu Leu Leu Leu Pro Arg Val Leu Leu Thr Met Ala
-15 -10 -5

Ser Gly Ser Pro Pro Thr Gln Pro Ser Pro Ala Ser Asp Ser Gly Ser
1 5 10

Gly Tyr Val Pro Gly Ser Val Ser Ala Ala Phe Val Thr Cys Pro Arg
15 20 25 30

(2) INFORMATION FOR SEQ ID NO: 520:

(i) SEQUENCE CHARACTERISTICS:
(A) LENGTH: 104 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide

(B) LOCATION: -24..-1
(C) IDENTIFICATION METHOD: Von Heijne matrix
(D) OTHER INFORMATION:. score 7.9
seq LLLPRVLLTMASG/SP

(xi) SEQUENCE DESCRIPTION: SEQ ID NO: 520:

Met Pro Ala Leu Leu Pro Val Ala Ser Arg Leu Leu Leu Leu Pro Arg
-20 -15 -10

Val Leu Leu Thr Met Ala Ser Gly Ser Pro Pro Thr Gln Pro Ser Pro
-5 1 5

Ala Ser Asp Ser Gly Ser Gly Tyr Val Pro Gly Ser Val Ser Ala Ala
10 15 20

Phe Val Thr Cys Pro Asn Glu Lys Val Ala Lys Glu Ile Ala Arg Ala
25 30 35 40

Val Val Glu Lys Arg Leu Ala Ala Cys Val Asn Leu Ile Pro Gln Ile
45 50 55

Thr Ser Ile Tyr Glu Trp Lys Gly Xaa Ile Glu Glu Asp Ser Glu Val
60 65 70

Leu Met Met Ile Lys Thr Gln Ala
75 80

(2) INFORMATION FOR SEQ ID NO: 521:

(i) SEQUENCE CHARACTERISTICS:
(A) LENGTH: 121 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -92..-1
(C) IDENTIFICATION METHOD: Von Heijne matrix
(D) OTHER INFORMATION: score 7.6
seq FLLLTVALLASYS/VH

(xi) SEQUENCE DESCRIPTION: SEQ ID NO: 521:

Met Glu Ala Ser Trp Gly Ser Phe Asn Ala Glu Arg Gly Trp Tyr Val
-90 -85 -80

Ser Val Gln Gln Pro Glu Glu Ala Glu Ala Glu Glu Leu Ser Pro Leu
-75 -70 -65

Leu Ser Asn Glu Leu His Arg Gln Arg Ser Pro Gly Val Ser Phe Gly
-60                 -55                 -50                 -45

Leu Ser Val Phe Asn Leu Met Asn Ala Ile Met Gly Ser Gly Ile Leu
                -40                 -35                 -30

Gly Leu Ala Tyr Val Met Ala Asn Thr Gly Val Phe Gly Phe Ser Phe
            -25                 -20                 -15

Leu Leu Leu Thr Val Ala Leu Leu Ala Ser Tyr Ser Val His Leu Leu
        -10                 -5                  1

Leu Ser Met Cys Ile Gln Thr Ala Val Thr Ser Tyr Glu Asp Leu Gly
  5                 10                  15                  20

Leu Phe Ala Phe Gly Leu Pro Gly Leu
                25

(2) INFORMATION FOR SEQ ID NO: 522:

(i) SEQUENCE CHARACTERISTICS:
(A) LENGTH: 19 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -17..-1
(C) IDENTIFICATION METHOD: Von Heijne matrix
(D) OTHER INFORMATION:  score 7.6
                        seq FFLLLRFFLRIDG/VP

(xi) SEQUENCE DESCRIPTION: SEQ ID NO: 522:

Met Pro Ser Ser Phe Phe Leu Leu Leu Arg Phe Phe Leu Arg Ile Asp
        -15                 -10                 -5

Gly Val Pro
      1

(2) INFORMATION FOR SEQ ID NO: 523:

(i) SEQUENCE CHARACTERISTICS:
(A) LENGTH: 46 amino acids
(B) TYPE: AMINO ACID
(D) TOPOLOGY: LINEAR

(ii) MOLECULE TYPE: PROTEIN

(vi) ORIGINAL SOURCE:

(A) ORGANISM: Homo Sapiens
(F) TISSUE TYPE: Dystrophic muscle

(ix) FEATURE:
(A) NAME/KEY: sig_peptide
(B) LOCATION: -19..-1
(C) IDENTIFICATION METHOD: Von Heijne matrix
(D) OTHER INFORMATION: score 7.6
seq FIVGIYFLSSCRA/EE

(xi) SEQUENCE DESCRIPTION: SEQ ID NO: 523:

Met Lys Arg Thr His Leu Phe Ile Val Gly Ile Tyr Phe Leu Ser Ser
-15 -10 -5

Cys Arg Ala Glu Glu Gly Leu Asn Phe Pro Thr Tyr Asp Gly Lys Asp
1 5 10

Arg Val Val Ser Leu Ser Glu Lys Asn Phe Lys Gln Val Leu
15 20 25

(2) INFORMATION FOR SEQ ID NO: 524:

(i) SEQUENCE CHARACTERISTICS:
(A) LENGTH: 61 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -23..-1
(C) IDENTIFICATION METHOD: Von Heijne matrix
(D) OTHER INFORMATION: score 7.4
seq VLLLAALPPVLLP/GA

(xi) SEQUENCE DESCRIPTION: SEQ ID NO: 524:

Met Gly Asp Lys Ile Trp Leu Pro Phe Pro Val Leu Leu Leu Ala Ala
-20 -15 -10

Leu Pro Pro Val Leu Leu Pro Gly Ala Ala Gly Phe Thr Pro Ser Leu
-5 1 5

Asp Ser Asp Phe Thr Phe Thr Leu Pro Ala Gly Gln Lys Glu Cys Phe
10 15 20 25

Tyr Gln Pro Met Pro Leu Xaa Ala Ser Leu Glu Ile Glu
30 35

(2) INFORMATION FOR SEQ ID NO: 525:

(i) SEQUENCE CHARACTERISTICS:
(A) LENGTH: 57 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -37..-1
(C) IDENTIFICATION METHOD: Von Heijne matrix
(D) OTHER INFORMATION: score 7.3
seq LLSACLVTLWGLG/EP

(xi) SEQUENCE DESCRIPTION: SEQ ID NO: 525:

Met Pro His Ser Ser Leu His Pro Ser Ile Pro Cys Pro Arg Gly His
-35 -30 -25

Gly Ala Gln Lys Ala Ala Leu Val Leu Leu Ser Ala Cys Leu Val Thr
-20 -15 -10

Leu Trp Gly Leu Gly Glu Pro Pro Glu His Thr Leu Arg Tyr Leu Val
-5 1 5 10

Leu Xaa Leu Ala Ser Leu Gln Leu Gly
15 20

(2) INFORMATION FOR SEQ ID NO: 526:

(i) SEQUENCE CHARACTERISTICS:
(A) LENGTH: 54 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -29..-1
(C) IDENTIFICATION METHOD: Von Heijne matrix
(D) OTHER INFORMATION: score 7.3
seq HLLLLLLPAPTLK/GL

(xi) SEQUENCE DESCRIPTION: SEQ ID NO: 526:

Met Gly Ala Trp Gly Arg Gly Trp Pro Trp Glu Glu Arg Gln Gly His
                -25                 -20                 -15

His Leu Leu Leu Leu Leu Leu Pro Ala Pro Thr Leu Lys Gly Leu Gly
            -10                 -5                  1

Ala Ala Gln Leu Pro Leu Cys Pro Ser Gly Gly Leu Ser Pro Leu Leu
    5                   10                  15

Thr Leu Leu Gln Ser Gly
20                  25

(2) INFORMATION FOR SEQ ID NO: 527:

(i) SEQUENCE CHARACTERISTICS:
(A) LENGTH: 124 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -75..-1
(C) IDENTIFICATION METHOD: Von Heijne matrix
(D) OTHER INFORMATION: score 7.2
seq LLFIIGLIGCCAT/IR

(xi) SEQUENCE DESCRIPTION: SEQ ID NO: 527:

Met Gly Gln Cys Gly Ile Thr Ser Ser Lys Thr Val Leu Val Phe Leu
-75                 -70                 -65                 -60

Asn Leu Ile Phe Trp Gly Ala Ala Gly Ile Leu Cys Tyr Val Gly Ala
                -55                 -50                 -45

Tyr Val Phe Ile Thr Tyr Asp Asp Tyr Asp His Phe Phe Glu Asp Val
            -40                 -35                 -30

Tyr Thr Leu Ile Pro Ala Val Val Ile Ile Ala Val Arg Ala Leu Leu
        -25                 -20                 -15

Phe Ile Ile Gly Leu Ile Gly Cys Cys Ala Thr Ile Arg Glu Ser Arg
    -10                 -5                  1                   5

Cys Gly Leu Ala Thr Phe Val Ile Ile Leu Leu Leu Val Phe Val Thr
                10                  15                  20

Glu Val Val Val Val Val Leu Gly Tyr Val Tyr Arg Ala Lys Val Glu
            25                  30                  35

Asn Glu Val Asp Arg Ser Ile Gln Lys Val Tyr Lys
        40                  45

(2) INFORMATION FOR SEQ ID NO: 528:

(i) SEQUENCE CHARACTERISTICS:
(A) LENGTH: 115 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -65..-1
(C) IDENTIFICATION METHOD: Von Heijne matrix
(D) OTHER INFORMATION: score 7
seq IGHFLCLVILVYC/AE

(xi) SEQUENCE DESCRIPTION: SEQ ID NO: 528:

Met Pro Xaa Ala Phe Ser Val Ser Ser Phe Pro Val Ser Ile Pro Ala
-65 -60 -55 -50

Val Leu Thr Gln Thr Asp Trp Thr Glu Pro Trp Leu Met Gly Leu Ala
-45 -40 -35

Thr Phe His Ala Leu Cys Val Leu Leu Thr Cys Leu Ser Ser Arg Ser
-30 -25 -20

Tyr Arg Leu Gln Ile Gly His Phe Leu Cys Leu Val Ile Leu Val Tyr
-15 -10 -5

Cys Ala Glu Tyr Ile Asn Glu Ala Ala Ala Met Asn Trp Arg Leu Phe
1               5                   10                  15

Ser Xaa Tyr Gln Tyr Phe Asp Ser Arg Gly Met Phe Ile Ser Ile Val
            20                  25                  30

Phe Ser Ala Pro Leu Leu Val Asn Ala Met Ile Ile Val Val Met Trp
        35                  40                  45

Val Trp Lys
    50

(2) INFORMATION FOR SEQ ID NO: 529:

(i) SEQUENCE CHARACTERISTICS:
(A) LENGTH: 26 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -14..-1
(C) IDENTIFICATION METHOD: Von Heijne matrix
(D) OTHER INFORMATION: score 6.7
seq LLLSLFFPLRISL/SP

(xi) SEQUENCE DESCRIPTION: SEQ ID NO: 529:

Met Leu Leu Leu Ser Leu Phe Phe Pro Leu Arg Ile Ser Leu Ser Pro
-10 -5 1

Ser Asn His Leu Trp Ser Ala Ser Ser Gly
                5                   10

(2) INFORMATION FOR SEQ ID NO: 530:

(i) SEQUENCE CHARACTERISTICS:
(A) LENGTH: 80 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -23..-1
(C) IDENTIFICATION METHOD: Von Heijne matrix
(D) OTHER INFORMATION: score 6.6
seq LILVLQLLLRIRR/NR

(xi) SEQUENCE DESCRIPTION: SEQ ID NO: 530:

Met Glu Thr Gly Glu Arg Ala Arg Leu Ile Leu Ile Leu Val Leu Gln
-20 -15 -10

Leu Leu Leu Arg Ile Arg Arg Asn Arg Gln Gln Arg Cys Xaa Ala Ser
-5 1 5

Ser Ala Thr Ala Pro Ser Ser His Gly Cys Asp Leu Arg Gly Gly Lys
10 15 20 25

Leu Asn Phe Lys Thr Thr Pro Met Asp Ala Asp Ser Asp Val Ala Leu
30 35 40

Asp Ile Leu Ile Thr Asn Val Val Cys Val Phe Arg Thr Arg Cys Arg

45                    50                    55

(2) INFORMATION FOR SEQ ID NO: 531:

(i) SEQUENCE CHARACTERISTICS:
(A) LENGTH: 66 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -41..-1
(C) IDENTIFICATION METHOD: Von Heijne matrix
(D) OTHER INFORMATION: score 6.4
seq ILGCSSVCQLCTG/RQ

(xi) SEQUENCE DESCRIPTION: SEQ ID NO: 531:

Met Cys Gly Xaa Xaa Phe Ser Leu Pro Cys Leu Arg Leu Phe Leu Val
-40                 -35                 -30

Val Thr Cys Tyr Xaa Leu Leu Leu Leu His Lys Glu Ile Leu Gly Cys
-25                 -20                 -15                 -10

Ser Ser Val Cys Gln Leu Cys Thr Gly Arg Gln Ile Asn Cys Arg Asn
                -5                  1               5

Leu Gly Leu Ser Ser Ile Leu Arg Ile Phe Leu Lys Val Gln Phe Phe
        10                  15                  20

Cys Ile
    25

(2) INFORMATION FOR SEQ ID NO: 532:

(i) SEQUENCE CHARACTERISTICS:
(A) LENGTH: 119 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide

(B) LOCATION: -73..-1
(C) IDENTIFICATION METHOD: Von Heijne matrix
(D) OTHER INFORMATION: score 6.4
seq ACCFLSAFSPTLT/KS

(xi) SEQUENCE DESCRIPTION: SEQ ID NO: 532:

Met Asn Pro Val Thr Glu Ser Pro Ser Cys Leu Phe Ser Pro Pro Ser
-70 -65 -60

Glu Ser Ala Leu Ala Ser Gln Leu Ala Leu Ser Ala Ser Cys Asp Gln
-55 -50 -45

Arg Ala Pro Phe Ser Leu Ala Gly Val Xaa Ser Xaa Xaa Pro Arg Leu
-40 -35 -30

Ala Ser Arg Gln Val Ala Pro Pro Phe Gly Ser Arg Ala Cys Cys Phe
-25 -20 -15 -10

Leu Ser Ala Phe Ser Pro Thr Leu Thr Lys Ser Ala Ala Ala Thr Ser
-5 1 5

Thr Ala His Thr Phe Leu Ala Asn Gln Leu Ser Cys Leu Phe Thr Lys
10 15 20

Cys Leu His Asn Asn Tyr Ser Ser Ser Leu Arg Leu Thr Lys Lys Gln
25 30 35

Glu Lys Ser Thr Thr Pro Gln
40 45

(2) INFORMATION FOR SEQ ID NO: 533:

(i) SEQUENCE CHARACTERISTICS:
(A) LENGTH: 30 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -21..-1
(C) IDENTIFICATION METHOD: Von Heijne matrix
(D) OTHER INFORMATION: score 6.3
seq LGLSVLLTAATVA/GV

(xi) SEQUENCE DESCRIPTION: SEQ ID NO: 533:

Met Ser Arg Ser Ser Lys Val Val Leu Gly Leu Ser Val Leu Leu Thr
-20 -15 -10

Ala Ala Thr Val Ala Gly Val His Val Lys Gln Gln Trp Asp

-5                    1                    5

(2) INFORMATION FOR SEQ ID NO: 534:

(i) SEQUENCE CHARACTERISTICS:
(A) LENGTH: 58 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -26..-1
(C) IDENTIFICATION METHOD: Von Heijne matrix
(D) OTHER INFORMATION:  score 6.3
seq GVGLVTLLGLAVG/SY

(xi) SEQUENCE DESCRIPTION: SEQ ID NO: 534:

Met Gly Ile Gln Thr Ser Pro Val Leu Leu Ala Ser Leu Gly Val Gly
    -25                 -20                 -15

Leu Val Thr Leu Leu Gly Leu Ala Val Gly Ser Tyr Leu Val Arg Arg
-10                 -5                  1               5

Ser Arg Arg Pro Gln Val Thr Leu Leu Asp Pro Ser Glu Lys Tyr Leu
            10                  15                  20

Leu Arg Leu Leu Asp Lys Thr Thr Pro Gly
        25                  30

(2) INFORMATION FOR SEQ ID NO: 535:

(i) SEQUENCE CHARACTERISTICS:
(A) LENGTH: 58 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -51..-1
(C) IDENTIFICATION METHOD: Von Heijne matrix
(D) OTHER INFORMATION:  score 6.2
seq VLLLSSAXLVXXS/SP

(xi) SEQUENCE DESCRIPTION: SEQ ID NO: 535:

Met Tyr Pro Ser Tyr Leu Leu Ile Xaa Pro Pro Ile Pro Ser Gln Phe
    -50                 -45                 -40

Leu Lys Gln Cys Xaa Pro Pro Thr Leu Ser Asp Pro Phe Leu Pro Leu
-35                 -30                 -25                 -20

Ala Leu Arg Ser Leu Asp Val Leu Leu Leu Ser Ser Ala Xaa Leu Val
                -15                 -10                  -5

Xaa Xaa Ser Ser Pro Leu Glu Phe Ile Arg
            1               5

(2) INFORMATION FOR SEQ ID NO: 536:

(i) SEQUENCE CHARACTERISTICS:
(A) LENGTH: 58 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -33..-1
(C) IDENTIFICATION METHOD: Von Heijne matrix
(D) OTHER INFORMATION: score 6.2
seq ILLLXTFQTWCLR/IS

(xi) SEQUENCE DESCRIPTION: SEQ ID NO: 536:

Met Glu Gln Lys His Arg Xaa Glu Leu Glu Gln Leu Lys Leu Xaa Thr
            -30                 -25                 -20

Lys Glu Asn Lys Ile Leu Leu Leu Xaa Thr Phe Gln Thr Trp Cys Leu
        -15                 -10                  -5

Arg Ile Ser His Leu Gly Tyr Gln Lys His Xaa Arg Xaa Gly Cys Leu
    1               5                   10                  15

Asp Xaa Arg Ser Ser Leu Cys Cys Pro Trp
                20                  25

(2) INFORMATION FOR SEQ ID NO: 537:

(i) SEQUENCE CHARACTERISTICS:
(A) LENGTH: 115 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -23..-1
(C) IDENTIFICATION METHOD: Von Heijne matrix
(D) OTHER INFORMATION: score 5.9
seq TLKFLTLLQKSNA/KR

(xi) SEQUENCE DESCRIPTION: SEQ ID NO: 537:

Met Met Thr Ala Pro Val Leu Ala Ala Gln Thr Leu Lys Phe Leu Thr
-20 -15 -10

Leu Leu Gln Lys Ser Asn Ala Lys Arg Xaa Asn Leu Asp Arg Leu His
-5 1 5

Asp Glu Leu Trp Tyr Asn Asp Pro Gly Gln Met Asn Asp Gly Pro Leu
10 15 20 25

Cys Lys Cys Ser Ala Lys Ala Arg Arg Thr Gly Ile Arg His Ser Ile
30 35 40

Tyr Pro Gly Glu Glu Ala Ile Lys Pro Cys Arg Pro Met Thr Asn Asn
45 50 55

Ala Gly Arg Leu Phe His Tyr Arg Ile Thr Val Ser Pro Pro Thr Asn
60 65 70

Phe Leu Thr Asp Arg Pro Thr Val Ile Glu Tyr Asp Asp His Glu Tyr
75 80 85

Ile Phe Glu
90

(2) INFORMATION FOR SEQ ID NO: 538:

(i) SEQUENCE CHARACTERISTICS:
(A) LENGTH: 102 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -27..-1

(C) IDENTIFICATION METHOD: Von Heijne matrix
(D) OTHER INFORMATION:  score 5.9
                        seq ALALAXAPDLAQA/PL

(xi) SEQUENCE DESCRIPTION: SEQ ID NO: 538:

Met Asp Ser Ala Ala Cys Ala Ala Ala Ala Thr Pro Val Pro Ala Leu
    -25                 -20                 -15

Ala Leu Ala Xaa Ala Pro Asp Leu Ala Gln Ala Pro Leu Ala Leu Pro
    -10                 -5                  1               5

Gly Leu Leu Ser Pro Ser Cys Leu Leu Ser Ser Gly Gln Glu Val Asn
                10                  15                  20

Gly Ser Glu Arg Gly Thr Cys Leu Trp Arg Pro Trp Leu Ser Ser Thr
            25                  30                  35

Asn Asp Ser Pro Arg Gln Met Arg Lys Leu Val Asp Leu Ala Ala Gly
        40                  45                  50

Gly Ala Thr Ala Ala Glu Val Thr Lys Ala Glu Ser Xaa Xaa His His
    55                  60                  65

Pro Val Arg Leu Phe Trp
70                  75

(2) INFORMATION FOR SEQ ID NO: 539:

(i) SEQUENCE CHARACTERISTICS:
(A) LENGTH: 114 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -24..-1
(C) IDENTIFICATION METHOD: Von Heijne matrix
(D) OTHER INFORMATION:  score 5.7
                        seq ILGLLGLLGTLVA/ML

(xi) SEQUENCE DESCRIPTION: SEQ ID NO: 539:

Met Ala Ser Leu Gly Leu Gln Leu Val Gly Tyr Ile Leu Gly Leu Leu
                -20                 -15                 -10

Gly Leu Leu Gly Thr Leu Val Ala Met Leu Leu Pro Ser Trp Lys Thr
            -5                  1               5

Ser Ser Tyr Val Gly Ala Ser Ile Val Thr Ala Val Gly Phe Ser Lys

10                  15                  20

Gly Leu Trp Met Glu Cys Ala Thr Xaa Ser Thr Gly Ile Thr Gln Cys
 25                  30                  35                  40

Asp Ile Tyr Ser Thr Leu Leu Gly Leu Pro Ala Asp Ile Gln Ala Ala
                45                  50                  55

Gln Ala Met Met Val Thr Ser Ser Ala Ile Ser Ser Leu Ala Cys Ile
            60                  65                  70

Ile Ser Val Val Gly Met Arg Cys Thr Val Phe Cys Gln Glu Ser Arg
        75                  80                  85

Ala Arg
    90

(2) INFORMATION FOR SEQ ID NO: 540:

(i) SEQUENCE CHARACTERISTICS:
(A) LENGTH: 55 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -24..-1
(C) IDENTIFICATION METHOD: Von Heijne matrix
(D) OTHER INFORMATION:  score 5.7
seq ILGLLGLLGTLVA/ML

(xi) SEQUENCE DESCRIPTION: SEQ ID NO: 540:

Met Ala Ser Leu Gly Leu Gln Leu Val Gly Tyr Ile Leu Gly Leu Leu
                -20                 -15                 -10

Gly Leu Leu Gly Thr Leu Val Ala Met Leu Leu Pro Ser Trp Lys Thr
            -5                  1               5

Ser Ser Tyr Val Gly Ala Ser Ile Val Thr Ala Val Gly Phe Ser Lys
    10                  15                  20

Gly Leu Trp Met Glu Cys Ala
 25                  30

(2) INFORMATION FOR SEQ ID NO: 541:

(i) SEQUENCE CHARACTERISTICS:
(A) LENGTH: 42 amino acids

(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -18..-1
(C) IDENTIFICATION METHOD: Von Heijne matrix
(D) OTHER INFORMATION: score 5.6
seq LLCECLLLVAGYA/HD

(xi) SEQUENCE DESCRIPTION: SEQ ID NO: 541:

Met Leu Cys Ser Leu Leu Leu Cys Glu Cys Leu Leu Leu Val Ala Gly
-15 -10 -5

Tyr Ala His Asp Asp Asp Trp Ile Asp Pro Thr Asp Met Leu Asn Tyr
1 5 10

Asp Ala Ala Ser Gly Thr Met Arg Lys Ser
15 20

(2) INFORMATION FOR SEQ ID NO: 542:

(i) SEQUENCE CHARACTERISTICS:
(A) LENGTH: 25 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -22..-1
(C) IDENTIFICATION METHOD: Von Heijne matrix
(D) OTHER INFORMATION: score 5.5
seq LWYVCPCPSGAWM/VP

(xi) SEQUENCE DESCRIPTION: SEQ ID NO: 542:

Met Ala Ser Arg Leu Cys Gly Gly Ala Leu Trp Tyr Val Cys Pro Cys
-20 -15 -10

Pro Ser Gly Ala Trp Met Val Pro Gly
-5 1

(2) INFORMATION FOR SEQ ID NO: 543:

(i) SEQUENCE CHARACTERISTICS:
(A) LENGTH: 63 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -28..-1
(C) IDENTIFICATION METHOD: Von Heijne matrix
(D) OTHER INFORMATION: score 5.5
seq LGYLVLSEGAVLA/SS

(xi) SEQUENCE DESCRIPTION: SEQ ID NO: 543:

Met Thr Ser Ala Leu Thr Gln Gly Leu Glu Arg Ile Pro Asp Gln Leu
-25 -20 -15

Gly Tyr Leu Val Leu Ser Glu Gly Ala Val Leu Ala Ser Ser Gly Asp
-10 -5 1

Leu Glu Asn Asp Glu Gln Ala Xaa Ser Ala Ile Ser Glu Leu Val Ser
5 10 15 20

Thr Ala Cys Gly Phe Arg Leu His Arg Gly Met Asn Val Pro Arg
25 30 35

(2) INFORMATION FOR SEQ ID NO: 544:

(i) SEQUENCE CHARACTERISTICS:
(A) LENGTH: 77 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -42..-1
(C) IDENTIFICATION METHOD: Von Heijne matrix
(D) OTHER INFORMATION: score 5.4
seq ITGVILLAVGIWG/KV

(xi) SEQUENCE DESCRIPTION: SEQ ID NO: 544:

Met Ala Ser Pro Ser Arg Arg Leu Gln Thr Lys Pro Val Ile Thr Cys
     -40                 -35                 -30

Phe Lys Ser Val Leu Leu Ile Xaa Thr Xaa Ile Xaa Trp Ile Thr Gly
    -25                 -20                 -15

Val Ile Leu Leu Ala Val Gly Ile Trp Gly Lys Val Ser Leu Glu Asn
-10                 -5                  1               5

Tyr Phe Xaa Leu Leu Asn Glu Lys Ala Thr Asn Val Pro Phe Xaa Leu
            10                  15                  20

Ile Ala Thr Gly Thr Val Xaa Ile Leu Leu Gly Tyr Arg
        25                  30                  35

(2) INFORMATION FOR SEQ ID NO: 545:

(i) SEQUENCE CHARACTERISTICS:
(A) LENGTH: 61 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 5.3
seq VLLGSGLTILSQP/LM

(xi) SEQUENCE DESCRIPTION: SEQ ID NO: 545:

Met Ala Asp Ala Ala Ser Gln Val Leu Leu Gly Ser Gly Leu Thr Ile
-20                 -15                 -10                 -5

Leu Ser Gln Pro Leu Met Tyr Val Lys Val Leu Ile Gln Val Gly Tyr
                1               5                   10

Glu Pro Leu Pro Pro Thr Ile Gly Arg Asn Ile Phe Gly Arg Gln Val
        15                  20                  25

Xaa Xaa Leu Pro Xaa Leu Phe Ser Tyr Ala Gln His Gly
    30                  35                  40

(2) INFORMATION FOR SEQ ID NO: 546:

(i) SEQUENCE CHARACTERISTICS:
(A) LENGTH: 31 amino acids

(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: -20..-1
(C) IDENTIFICATION METHOD: Von Heijne matrix
(D) OTHER INFORMATION: score 5.3
seq ALIFGGFISLIGA/AF

(xi) SEQUENCE DESCRIPTION: SEQ ID NO: 546:

Met Ser Arg Asn Leu Arg Thr Ala Leu Ile Phe Gly Gly Phe Ile Ser
-20 -15 -10 -5

Leu Ile Gly Ala Ala Phe Tyr Pro Ile Tyr Phe Arg Pro His Gly
1 5 10

(2) INFORMATION FOR SEQ ID NO: 547:

(i) SEQUENCE CHARACTERISTICS:
(A) LENGTH: 23 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -17..-1
(C) IDENTIFICATION METHOD: Von Heijne matrix
(D) OTHER INFORMATION: score 5.1
seq LWCFHLVVLSLYS/SV

(xi) SEQUENCE DESCRIPTION: SEQ ID NO: 547:

(2) INFORMATION FOR SEQ ID NO: 548:

(i) SEQUENCE CHARACTERISTICS:
(A) LENGTH: 35 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -14..-1
(C) IDENTIFICATION METHOD: Von Heijne matrix
(D) OTHER INFORMATION: score 5
seq SLVAVFLSCGLIS/KN

(xi) SEQUENCE DESCRIPTION: SEQ ID NO: 548:

Met Ser Leu Val Ala Val Phe Leu Ser Cys Gly Leu Ile Ser Lys Asn
-10 -5 1

His Met Leu Leu Asn Leu Pro Gly Ile Leu Ile Pro His Asn Ala Asn
                5                   10                  15

His Leu Leu
20

(2) INFORMATION FOR SEQ ID NO: 549:

(i) SEQUENCE CHARACTERISTICS:
(A) LENGTH: 42 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Kidney

(ix) FEATURE:
(A) NAME/KEY: sig_peptide
(B) LOCATION: -24..-1
(C) IDENTIFICATION METHOD: Von Heijne matrix
(D) OTHER INFORMATION: score 5
seq GALAVGAVPVVLS/AM

(xi) SEQUENCE DESCRIPTION: SEQ ID NO: 549:

Met Met Lys Arg Ala Ala Ala Ala Ala Val Gly Gly Ala Leu Ala Val
-20 -15 -10

Gly Ala Val Pro Val Val Leu Ser Ala Met Gly Phe Thr Gly Ala Gly
-5 1 5

Ile Ala Ala Ser Ser Ile Ala Ala His Gly
10 15

(2) INFORMATION FOR SEQ ID NO: 550:

(i) SEQUENCE CHARACTERISTICS:
(A) LENGTH: 137 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -81..-1
(C) IDENTIFICATION METHOD: Von Heijne matrix
(D) OTHER INFORMATION: score 4.9
seq LISFSWFANYIRA/GT

(xi) SEQUENCE DESCRIPTION: SEQ ID NO: 550:

Met Ala Val Ile Val Asp Lys Pro Trp Phe Tyr Asp Met Lys Lys Val
-80 -75 -70

Trp Glu Gly Tyr Pro Ile Gln Ser Thr Ile Pro Ser Gln Tyr Trp Tyr
-65 -60 -55 -50

Tyr Met Ile Glu Leu Ser Phe Tyr Trp Ser Leu Leu Phe Ser Ile Ala
-45 -40 -35

Ser Asp Val Lys Arg Lys Asp Phe Lys Glu Gln Ile Ile His His Val
-30 -25 -20

Ala Thr Ile Ile Leu Ile Ser Phe Ser Trp Phe Ala Asn Tyr Ile Arg
-15 -10 -5

Ala Gly Thr Leu Ile Met Ala Leu His Asp Ser Ser Asp Tyr Leu Leu
1               5                   10                  15

Glu Ser Ala Lys Met Phe Asn Tyr Ala Gly Trp Lys Asn Thr Cys Asn
            20                  25                  30

Asn Ile Phe Thr Val Phe Ala Ile Val Phe Ile Ile Thr Arg Leu Val
        35                  40                  45

Ile Leu Pro Phe Trp Ile Leu His Cys
    50                  55

(2) INFORMATION FOR SEQ ID NO: 551:

(i) SEQUENCE CHARACTERISTICS:
(A) LENGTH: 78 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -16..-1
(C) IDENTIFICATION METHOD: Von Heijne matrix
(D) OTHER INFORMATION: score 4.8
seq SLFIYIFLTCSNT/SP

(xi) SEQUENCE DESCRIPTION: SEQ ID NO: 551:

Met Ile Ile Ser Leu Phe Ile Tyr Ile Phe Leu Thr Cys Ser Asn Thr
-15 -10 -5

Ser Pro Ser Tyr Gln Gly Thr Gln Leu Gly Leu Gly Leu Pro Ser Ala
1               5                   10                  15

Gln Trp Trp Pro Leu Thr Gly Arg Arg Met Gln Cys Cys Arg Leu Phe
            20                  25                  30

Cys Phe Leu Leu Gln Asn Cys Leu Phe Pro Phe Pro Leu His Leu Ile
        35                  40                  45

Gln His Asp Pro Cys Glu Leu Val Leu Thr Ile Ser Gly Thr
    50                  55                  60

(2) INFORMATION FOR SEQ ID NO: 552:

(i) SEQUENCE CHARACTERISTICS:
(A) LENGTH: 86 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -32..-1
(C) IDENTIFICATION METHOD: Von Heijne matrix
(D) OTHER INFORMATION: score 4.7
seq LQMLLGFVGRSKS/GL

(xi) SEQUENCE DESCRIPTION: SEQ ID NO: 552:

Met Ala Ala Glu Leu Val Glu Ala Lys Asn Met Val Met Ser Phe Arg
-30 -25 -20

Val Ser Asp Leu Gln Met Leu Leu Gly Phe Val Gly Arg Ser Lys Ser
-15 -10 -5

Gly Leu Lys His Glu Leu Val Thr Arg Ala Leu Gln Leu Val Gln Phe
1               5                   10                  15

Asp Cys Ser Pro Glu Leu Phe Lys Lys Ile Lys Glu Leu Tyr Glu Thr
            20                  25                  30

Arg Tyr Ala Lys Lys Asn Ser Glu Pro Ala Pro Gln Pro His Arg Pro
        35                  40                  45

Leu Asp Pro Leu Thr Gly
    50

(2) INFORMATION FOR SEQ ID NO: 553:

(i) SEQUENCE CHARACTERISTICS:
(A) LENGTH: 67 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -60..-1
(C) IDENTIFICATION METHOD: Von Heijne matrix
(D) OTHER INFORMATION: score 4.7
seq VHALCPLSPLVTT/GC

(xi) SEQUENCE DESCRIPTION: SEQ ID NO: 553:

Met Thr Gly Leu Ser Met Xaa Gly Gly Gly Ser Xaa Xaa Gly Asp Val
-60 -55 -50 -45

Xaa Pro Xaa Tyr Tyr Gly Lys Xaa Gly Pro Leu Arg Xaa Leu Pro Glu
-40 -35 -30

Pro Ser Gly Pro Leu Pro Pro Ser Ser Gly Leu Ser Gln Pro Gln Val
-25 -20 -15

His Ala Leu Cys Pro Leu Ser Pro Leu Val Thr Thr Gly Cys Cys Gly
-10 -5 1

Gln Ala Ala
5

(2) INFORMATION FOR SEQ ID NO: 554:

(i) SEQUENCE CHARACTERISTICS:
(A) LENGTH: 33 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -31..-1
(C) IDENTIFICATION METHOD: Von Heijne matrix
(D) OTHER INFORMATION: score 4.6
seq GLLGXGLXXXSLT/AG

(xi) SEQUENCE DESCRIPTION: SEQ ID NO: 554:

Met Gln Met Tyr Ser Arg Gln Leu Ala Ser Xaa Glu Trp Leu Thr Ile
-30 -25 -20

Gln Gly Gly Leu Leu Gly Xaa Gly Leu Xaa Xaa Xaa Ser Leu Thr Ala
-15 -10 -5 1

Gly

(2) INFORMATION FOR SEQ ID NO: 555:

(i) SEQUENCE CHARACTERISTICS:
(A) LENGTH: 122 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -54..-1
(C) IDENTIFICATION METHOD: Von Heijne matrix
(D) OTHER INFORMATION: score 4.3
seq LIVWLLVKSFSES/GI

(xi) SEQUENCE DESCRIPTION: SEQ ID NO: 555:

Met Ala Ser Leu Glu Val Ser Arg Ser Pro Arg Arg Ser Arg Arg Glu

-50                    -45                    -40

Leu Glu Val Arg Ser Pro Arg Gln Asn Lys Tyr Ser Val Leu Leu Pro
            -35                    -30                    -25

Thr Tyr Asn Glu Arg Glu Asn Leu Pro Leu Ile Val Trp Leu Leu Val
        -20                    -15                    -10

Lys Ser Phe Ser Glu Ser Gly Ile Asn Tyr Glu Ile Ile Ile Ile Asp
    -5                      1               5                  10

Asp Gly Ser Pro Asp Gly Thr Arg Asp Val Ala Glu Gln Leu Glu Lys
                15                  20                  25

Ile Tyr Gly Ser Asp Arg Ile Leu Leu Arg Pro Arg Glu Lys Lys Leu
            30                  35                  40

Gly Leu Gly Thr Ala Tyr Ile Xaa Xaa Met Lys His Ala Gln Glu Thr
        45                  50                  55

Thr Ser Leu Leu Trp Xaa Leu Ile Ser His
    60                  65

(2) INFORMATION FOR SEQ ID NO: 556:

(i) SEQUENCE CHARACTERISTICS:
(A) LENGTH: 42 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -20..-1
(C) IDENTIFICATION METHOD: Von Heijne matrix
(D) OTHER INFORMATION:  score 4.3
seq LLDSSLMASGTAS/RS

(xi) SEQUENCE DESCRIPTION: SEQ ID NO: 556:

Met Asp Lys Asp Ser Gln Gly Leu Leu Asp Ser Ser Leu Met Ala Ser
-20                 -15                 -10                 -5

Gly Thr Ala Ser Arg Ser Glu Asp Glu Glu Ser Leu Ala Gly Gln Lys
                1               5                   10

Arg Ala Ser Ser Gln Ala Leu Gly Thr Gly
        15                  20

(2) INFORMATION FOR SEQ ID NO: 557:

(i) SEQUENCE CHARACTERISTICS:
(A) LENGTH: 83 amino acids
(B) TYPE: AMINO ACID .
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -36..-1
(C) IDENTIFICATION METHOD: Von Heijne matrix
(D) OTHER INFORMATION: score 4.2
seq CLAVSWEAAGCHG/AG

(xi) SEQUENCE DESCRIPTION: SEQ ID NO: 557:

Met Gly Leu Leu Thr Phe Gly Tyr Ile Glu Xaa Xaa Xaa Lys Thr Glu
-35 -30 -25

His Asn Pro Asp His His Ser Cys Leu Ala Val Ser Trp Glu Ala Ala
-20 -15 -10 -5

Gly Cys His Gly Ala Gly Thr Gln Gln Ser Pro Leu Gly Val Ala Gly
1 5 10

Pro Trp Arg Pro Arg Pro Pro Cys Val Gly Ser Leu Leu Ala Ala Arg
15 20 25

Ser Leu His Lys Gln Val Ile Leu Phe Gly Leu Leu Gly Phe Ala Tyr
30 35 40

Asp His Trp
45

(2) INFORMATION FOR SEQ ID NO: 558:

(i) SEQUENCE CHARACTERISTICS:
(A) LENGTH: 65 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -16..-1
(C) IDENTIFICATION METHOD: Von Heijne matrix
(D) OTHER INFORMATION: score 4.1
seq YAAVAGVLAGVES/RQ

(xi) SEQUENCE DESCRIPTION: SEQ ID NO: 558:

Met Gly Leu Tyr Ala Ala Val Ala Gly Val Leu Ala Gly Val Glu Ser
    -15                 -10                 -5

Arg Gln Gly Ser Asn Gln Gly Ala Gly Val Leu Gln Gln Leu Pro Glu
  1               5                  10                  15

Arg Glu Xaa Ala Val Arg Ala Gly Val Arg Xaa Ala Ala Leu Leu Arg
            20                  25                  30

Arg Ala Gly Xaa Arg Asp Leu Gln Arg Arg Pro Pro Gln Cys Glu Glu
        35                  40                  45

Ala

(2) INFORMATION FOR SEQ ID NO: 559:

(i) SEQUENCE CHARACTERISTICS:
(A) LENGTH: 94 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -62..-1
(C) IDENTIFICATION METHOD: Von Heijne matrix
(D) OTHER INFORMATION:  score 4.1
                        seq LDAVIASAGLLRA/EK

(xi) SEQUENCE DESCRIPTION: SEQ ID NO: 559:

Met Gly Leu Tyr Ala Ala Ala Ala Gly Val Leu Ala Gly Val Glu Ser
        -60                 -55                 -50

Arg Gln Gly Ser Ile Lys Gly Leu Val Tyr Ser Ser Asn Phe Gln Asn
    -45                 -40                 -35

Val Lys Gln Leu Tyr Ala Leu Val Cys Glu Thr Gln Arg Tyr Ser Ala
-30                 -25                 -20                 -15

Val Leu Asp Ala Val Ile Ala Ser Ala Gly Leu Leu Arg Ala Glu Lys
                -10                 -5                   1

Lys Leu Arg Pro His Leu Ala Lys Val Leu Val Tyr Glu Leu Leu Leu
        5                  10                  15

Gly Lys Gly Phe Arg Gly Gly Gly Gly Arg Trp Lys Ala Arg
    20                  25                  30

(2) INFORMATION FOR SEQ ID NO: 560:

(i) SEQUENCE CHARACTERISTICS:
(A) LENGTH: 151 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -64..-1
(C) IDENTIFICATION METHOD: Von Heijne matrix
(D) OTHER INFORMATION: score 4.1
seq WLLRLAYLADIFT/KL

(xi) SEQUENCE DESCRIPTION: SEQ ID NO: 560:

Met Gly Ala Gln His Thr Ala Leu Leu Leu Asn Thr Glu Val Arg Trp
-60 -55 -50

Leu Ser Arg Gly Lys Val Leu Val Arg Leu Phe Glu Leu Arg Arg Glu
-45 -40 -35

Leu Leu Val Phe Met Asp Ser Ala Phe Arg Leu Ser Asp Cys Leu Thr
-30 -25 -20

Asn Ser Ser Trp Leu Leu Arg Leu Ala Tyr Leu Ala Asp Ile Phe Thr
-15 -10 -5

Lys Leu Asn Glu Val Asn Leu Ser Met Gln Gly Lys Asn Val Thr Val
1               5                   10                  15

Phe Thr Val Phe Asp Lys Met Ser Ser Leu Leu Arg Lys Leu Glu Phe
            20                  25                  30

Trp Ala Ser Ser Val Glu Glu Glu Asn Phe Asp Cys Phe Pro Thr Leu
        35                  40                  45

Ser Asp Phe Leu Thr Glu Ile Asn Ser Thr Val Asp Lys Asp Ile Cys
    50                  55                  60

Ser Ala Ile Val Gln His Leu Arg Gly Leu Arg Ala Thr Leu Leu Lys
65                  70                  75                  80

Tyr Phe Pro Val Thr Asn Asp
                85

(2) INFORMATION FOR SEQ ID NO: 561:

(i) SEQUENCE CHARACTERISTICS:

(A) LENGTH: 44 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -25..-1
(C) IDENTIFICATION METHOD: Von Heijne matrix
(D) OTHER INFORMATION: score 4
seq LVVMVPLVGLIHL/GW

(xi) SEQUENCE DESCRIPTION: SEQ ID NO: 561:

Met Ser Leu Arg Asn Leu Trp Arg Asp Tyr Lys Val Leu Val Val Met
-25 -20 -15 -10

Val Pro Leu Val Gly Leu Ile His Leu Gly Trp Tyr Arg Ile Lys Ser
-5 1 5

Ser Pro Val Phe Gln Ile Pro Lys Asn Asp Asn Met
10 15

(2) INFORMATION FOR SEQ ID NO: 562:

(i) SEQUENCE CHARACTERISTICS:
(A) LENGTH: 105 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -51..-1
(C) IDENTIFICATION METHOD: Von Heijne matrix
(D) OTHER INFORMATION: score 3.9
seq GKLLQLVLGCAIS/CE

(xi) SEQUENCE DESCRIPTION: SEQ ID NO: 562:

Met Val Leu Arg Ser Leu Val Glu Tyr Ser Gln Asp Val Leu Ala His
-50 -45 -40

Pro Val Ser Glu Glu His Leu Pro Asp Val Ser Leu Ile Gly Glu Phe
-35 -30 -25 -20

Ser Asp Pro Ala Glu Leu Gly Lys Leu Leu Gln Leu Val Leu Gly Cys

-15                    -10                    -5

Ala Ile Ser Cys Glu Lys Lys Gln Asp His Ile Gln Arg Ile Met Thr
              1               5                  10

Leu Glu Glu Ser Val Gln His Val Val Met Glu Ala Ile Gln Glu Leu
     15                  20                  25

Met Thr Lys Asp Thr Pro Asp Ser Leu Ser Pro Glu Thr Tyr Gly Asn
 30                  35                  40                  45

Phe Asp Ser Gln Ser Arg Ser Thr Gly
                    50

(2) INFORMATION FOR SEQ ID NO: 563:

(i) SEQUENCE CHARACTERISTICS:
(A) LENGTH: 16 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -13..-1
(C) IDENTIFICATION METHOD: Von Heijne matrix
(D) OTHER INFORMATION:  score 3.9
                        seq MIHGFCLAPTTSA/KN

(xi) SEQUENCE DESCRIPTION: SEQ ID NO: 563:

Met Ile His Gly Phe Cys Leu Ala Pro Thr Thr Ser Ala Lys Asn Ala
            -10                 -5                  1

(2) INFORMATION FOR SEQ ID NO: 564:

(i) SEQUENCE CHARACTERISTICS:
(A) LENGTH: 26 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide

(B) LOCATION: -17..-1
(C) IDENTIFICATION METHOD: Von Heijne matrix
(D) OTHER INFORMATION: . score 3.7
seq RTWCLACVEASPG/QP

(xi) SEQUENCE DESCRIPTION: SEQ ID NO: 564:

Met Xaa Cys Pro Arg Thr Trp Cys Leu Ala Cys Val Glu Ala Ser Pro
-15 -10 -5

Gly Gln Pro Phe Leu Pro Pro Arg Pro Gly
1 5

(2) INFORMATION FOR SEQ ID NO: 565:

(i) SEQUENCE CHARACTERISTICS:
(A) LENGTH: 67 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Dystrophic muscle

(ix) FEATURE:
(A) NAME/KEY: sig_peptide
(B) LOCATION: -21..-1
(C) IDENTIFICATION METHOD: Von Heijne matrix
(D) OTHER INFORMATION: score 3.7
seq ETCALASHSGSSG/SK

(xi) SEQUENCE DESCRIPTION: SEQ ID NO: 565:

Met Ala Asp Val Glu Asp Gly Glu Glu Thr Cys Ala Leu Ala Ser His
-20 -15 -10

Ser Gly Ser Ser Gly Ser Lys Ser Gly Gly Asp Lys Met Phe Ser Leu
-5 1 5 10

Lys Lys Trp Asn Ala Val Ala Met Trp Ser Trp Asp Val Glu Cys Asp
15 20 25

Thr Cys Ala Ile Cys Arg Val Gln Val Met Asp Ala Cys Xaa Arg Cys
30 35 40

Gln Ala Gly
45

(2) INFORMATION FOR SEQ ID NO: 566:

(i) SEQUENCE CHARACTERISTICS:
(A) LENGTH: 28 amino acids
(B) TYPE: AMINO ACID

(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -26..-1
(C) IDENTIFICATION METHOD: Von Heijne matrix
(D) OTHER INFORMATION: score 3.7
seq IIMFLLIIVCGSP/RP

(xi) SEQUENCE DESCRIPTION: SEQ ID NO: 566:

Met Phe Lys Val Ala Ala Pro Pro Met Leu Ile Xaa Xaa Ile Ile Met
-25 -20 -15

Phe Leu Leu Ile Ile Val Cys Gly Ser Pro Arg Pro
-10 -5 1

(2) INFORMATION FOR SEQ ID NO: 567:

(i) SEQUENCE CHARACTERISTICS:
(A) LENGTH: 51 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Muscle

(ix) FEATURE:
(A) NAME/KEY: sig_peptide
(B) LOCATION: -21..-1
(C) IDENTIFICATION METHOD: Von Heijne matrix
(D) OTHER INFORMATION: score 3.6
seq FXMCLWSLRNLFS/RC

(xi) SEQUENCE DESCRIPTION: SEQ ID NO: 567:

Met Asp Phe Trp Asp Pro Ala Val Phe Xaa Met Cys Leu Trp Ser Leu
-20 -15 -10

Arg Asn Leu Phe Ser Arg Cys Ser Pro Cys Leu Thr Glu Ile Ser Leu
-5 1 5 10

His Leu Val His Leu Thr Ala Glu Lys Lys Gln His Gly Ser Asn Asn
15 20 25

Gly Ser Ala
30

(2) INFORMATION FOR SEQ ID NO: 568:

(i) SEQUENCE CHARACTERISTICS:
(A) LENGTH: 38 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -34..-1
(C) IDENTIFICATION METHOD: Von Heijne matrix
(D) OTHER INFORMATION: score 3.6
seq SVPLLSLSHSIGI/SP

(xi) SEQUENCE DESCRIPTION: SEQ ID NO: 568:

(2) INFORMATION FOR SEQ ID NO: 569:

(i) SEQUENCE CHARACTERISTICS:
(A) LENGTH: 47 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart

(ix) FEATURE:
(A) NAME/KEY: sig_peptide
(B) LOCATION: -17..-1
(C) IDENTIFICATION METHOD: Von Heijne matrix
(D) OTHER INFORMATION: score 3.5
seq LVCVGLHTEGPWG/RP

(xi) SEQUENCE DESCRIPTION: SEQ ID NO: 569:

(2) INFORMATION FOR SEQ ID NO: 570:

(i) SEQUENCE CHARACTERISTICS:
(A) LENGTH: 79 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -72..-1
(C) IDENTIFICATION METHOD: Von Heijne matrix
(D) OTHER INFORMATION: score 3.5
seq WFYIGSSLNGTRG/KR

(xi) SEQUENCE DESCRIPTION: SEQ ID NO: 570:

Met Phe Gly Ala Ala Ala Arg Ser Ala Asp Leu Val Leu Leu Glu Lys
-70 -65 -60

Asn Leu Gln Ala Ala His Gly Tyr Ala Gln Glu Asp Arg Glu Arg Met
-55 -50 -45

His Arg Xaa Ile Val Ser Leu Xaa Gln Asn Leu Leu Asn Phe Met Ile
-40 -35 -30 -25

Gly Ser Ile Leu Asp Leu Trp Gln Cys Phe Leu Trp Phe Tyr Ile Gly
-20 -15 -10

Ser Ser Leu Asn Gly Thr Arg Gly Lys Arg Val Pro Ala His Phe
-5 1 5

(2) INFORMATION FOR SEQ ID NO: 571:

(i) SEQUENCE CHARACTERISTICS:
(A) LENGTH: 30 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(F) TISSUE TYPE: Heart .

(ix) FEATURE:
(A) NAME/KEY: sig_peptide
(B) LOCATION: -27..-1
(C) IDENTIFICATION METHOD: Von Heijne matrix
(D) OTHER INFORMATION: score 3.5
seq VVALLIVCDVPSA/SA

(xi) SEQUENCE DESCRIPTION: SEQ ID NO: 571:

Met Ala Ala Arg Trp Arg Phe Trp Cys Val Ser Val Thr Met Val Val
-25 -20 -15

Ala Leu Leu Ile Val Cys Asp Val Pro Ser Ala Ser Ala Arg
-10 -5 1

(2) INFORMATION FOR SEQ ID NO: 572:

(i) SEQUENCE CHARACTERISTICS:
(A) LENGTH: 64 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -16..-1
(C) IDENTIFICATION METHOD: Von Heijne matrix
(D) OTHER INFORMATION: score 3.5
seq LLLQPSMIQEVWT/XY

(xi) SEQUENCE DESCRIPTION: SEQ ID NO: 572:

Met Val Val Leu Leu Leu Gln Pro Ser Met Ile Gln Glu Val Trp Thr
-15 -10 -5

Xaa Tyr Ala Asn Leu Phe His Ser Phe Phe Val Asp Asn Pro Phe Gln
1               5                   10                  15

Lys Glu Cys Phe His Gln Lys Asn Trp Tyr His Ile Thr Leu Met Gln
            20                  25                  30

Arg Thr Val Gly Thr Trp Arg Ile Leu Pro Asn Phe Leu Lys His Asp
        35                  40                  45

(2) INFORMATION FOR SEQ ID NO: 573:

(i) SEQUENCE CHARACTERISTICS:
(A) LENGTH: 86 amino acids
(B) TYPE: AMINO ACID
(D) TOPOLOGY: LINEAR

(ii) MOLECULE TYPE: PROTEIN

(vi) ORIGINAL SOURCE:
(A) ORGANISM: Homo Sapiens
(D) DEVELOPMENTAL STAGE: Fetal
(F) TISSUE TYPE: kidney

(ix) FEATURE:
(A) NAME/KEY: sig_peptide
(B) LOCATION: -31..-1
(C) IDENTIFICATION METHOD: Von Heijne matrix
(D) OTHER INFORMATION: score 10.5
seq LAVLLSLAPSASS/DI

(xi) SEQUENCE DESCRIPTION: SEQ ID NO: 573:

Met Leu His Leu His Xaa Ser Cys Leu Cys Phe Arg Ser Trp Leu Pro
-30 -25 -20

Ala Met Leu Ala Val Leu Leu Ser Leu Ala Pro Ser Ala Ser Ser Asp
-15 -10 -5 1

Ile Ser Ala Ser Arg Pro Asn Ile Leu Leu Leu Met Ala Asp Asp Leu
                5                   10                  15

Gly Ile Gly Asp Ile Gly Cys Tyr Gly Asn Asn Thr Met Arg Thr Pro
            20                  25                  30

Xaa Ile Asp Arg Leu Ala Glu Asp Gly Val Lys Leu Thr Gln His Ile
        35                  40                  45

Ser Ala Ala Ser Leu Cys
50 55

## Claims

1. A signal peptide having the sequence of amino acids -22 to -1 of SEQ ID NO: 309.

2. A signal sequence encoding the signal peptide of claim 1.

3. The signal sequence of claim 2 having the sequence of nucleotides 69 to 134 of SEQ ID NO: 41.

4. A purified and isolated nucleic acid encoding a polypeptide comprising the signal peptide of claim 1 at the 5' end of the coding sequence.

5. The nucleic acid of claim 4, wherein said nucleic acid comprises a signal sequence having the sequence of nucleotides 69 to 134 of SEQ ID NO: 41.

6. The nucleic acid of claim 5, wherein the amino terminus of said polypeptide is encoded by nucleotides 69 to 161 of SEQ ID NO: 41.

7. The nucleic acid of claim 4, wherein said signal sequence is fused in frame to the 5' end of a sequence encoding a polypeptide that is heterologous to a polypeptide comprising amino acids 1 to 9 of SEQ ID NO: 309.

8. An expression vector comprising the signal sequence of claim 2 or 3 operably linked to a promoter.

9. An expression vector comprising the nucleic acid of any one of the nucleic acids of any one of claims 4 to 7 operably linked to a promoter.

10. The expression vector of claim 8 or 9, wherein said vector is a secretion vector.

11. The expression vector of claim 8 or 9, wherein said vector is a gene therapy vector.

12. A polypeptide encoded by the polynucleotide of any one of claims 4 to 7.

13. The polypeptide of claim 12, wherein said polypeptide is a human secreted protein.

14. The polypeptide of claim 13, wherein said polypeptide comprises amino acids 1 to 9 of SEQ ID NO: 309.

15. A fusion protein encoded by the nucleic acid of claim 7.

16. Use of the signal peptide of claim 1 for directing the extracellular secretion of a polypeptide.

17. Use of the signal peptide of claim 1 for simplifying protein purification of a desired polypeptide.

18. Use of the vector of any one of claims 8 to 11 for directing the extracellular secretion of a polypeptide.

19. Use of the vector of any one of claims 8 to 10 for simplifying protein purification of a desired polypeptide.

20. Use of any one of claims 16 to 19, wherein said polypeptide is the polypeptide of any one of claims 12 to 15.

21. A method of making a secreted protein comprising the step of inserting a gene encoding a non-secreted protein in frame with the signal sequence of claim 2 or 3 into a vector such that the protein encoded by the inserted gene is expressed from the transcribed mRNA.

22. The method of claim 21, further comprising the step of introducing said vector into a host cell, tissue or organism.

23. A method of making a secreted protein comprising the step of introducing the vector of claim 9 into a host cell, tissue or organism.

24. The method of any one of claims 21 to 23 further comprising the step of isolating the secreted protein.

25. The method of claim 24, wherein the step of isolating the secreted protein comprises purifying the secreted protein from the supernatant, the culture medium or the cell extract of said host cell.

26. An isolated polypeptide obtainable by any one of the methods of claims 21 to 25.

27. A method of making a cDNA encoding a human secreted protein, wherein said method comprises the steps of:
a) contacting a collection of mRNA molecules from human cells with a primer comprising at least 15 consecutive nucleotides of a sequence complementary to a sequence of SEQ ID NO: 41.
b) hybridizing said primer to an mRNA in said collection;
c) reverse transcribing said hybridized primer to make a first cDNA strand from said mRNA;
d) making a second cDNA strand complementary to said first cDNA strand; and
e) isolating the resulting cDNA encoding said polypeptide comprising said first cDNA strand and said second cDNA strand.

28. A method of making a cDNA encoding a human secreted protein, wherein said method comprises the steps of:
a) obtaining a cDNA comprising a sequence of SEQ ID NO: 41;
b) contacting said cDNA with a detectable probe comprising at least 15 consecutive nucleotides of said sequence of SEQ ID NO: 41 or a sequence complementary thereto under conditions which permit said probe to hybridize to said cDNA;
c) identifying a cDNA which hybridizes to said detectable probe; and
d) isolating said cDNA which hybridizes to said probe.

29. A method of making a cDNA encoding a human secreted protein, wherein said method comprises the steps of:
a) contacting a collection of mRNA molecules from human cells with a first primer capable of hybridizing to the polyA tail of said mRNA;
b) hybridizing said first primer to said polyA tail;
c) reverse transcribing said mRNA to make a first cDNA strand;
d) making a second cDNA strand complementary to said first cDNA strand using at least one primer comprising at least 15 nucleotides of a sequence SEQ ID NO: 41; and
e) isolating the resulting cDNA comprising said first cDNA strand and said second cDNA strand.
